# EUROPEAN PATENT SPECIFICATION

(11) **EP 1 954 812 B1**
(45) Date of publication and mention of the grant of the patent: **21.11.2012**
(21) Application number: 06807517.5
(22) Date of filing: 24.10.2006
(51) Int. Cl.: C12N 15/80

(54) **DNA BINDING SITE OF A TRANSCRIPTIONAL ACTIVATOR USEFUL IN GENE EXPRESSION**
DNA-BINDUNGSSTELLE EINES TRANSKRIPTIONSAKTIVATORS MIT EIGNUNG BEI DER GENEXPRESSION
SITE DE LIAISON A L'ADN D'UN ACTIVATEUR TRANSCRIPTIONNEL UTILE DANS L'EXPRESSION GENETIQUE

(30) Priority: 29.11.2005 EP 05111442
(43) Date of publication of application: 13.08.2008
(60) Divisional application: 11194875.8
(73) Proprietor: DSM IP Assets B.V., 6411 TE Heerlen (NL)
(72) Inventor: PARENICOVA, Lucie, NL-2497 ZJ Den Haag (NL); PEIJ, VAN, Noël Nicolaas Maria Elisabeth, NL-2622 KA Delft (NL)
(74) Representative: Elkenbracht, Johan Christiaan
(86) International application number: PCT/EP2006/067734
(87) International publication number: WO 2007/062936

(56) References cited:
- EP-A- 0 238 023
- DATABASE REGISTRY [Online] 5 November 2002 (2002-11-05), "DNA ( ... apaf-1 promoter region ...)" XP002421401 retrieved from STN Database accession no. 470486-77-2
- DATABASE REGISTRY [Online] 8 December 2005 (2005-12-08), "DNA ( ... SPRD TRANSCRIPTION FACTOR ADPA BINDING SITE ...)" XP002421402 retrieved from STN Database accession no. 869595-71-1

### SEQUENCE LISTING

<110> DSM IP Assets B.V.
<120> DNA BINDING SITE OF A TRANSCRIPTIONAL ACTIVATOR USEFUL IN GENE EXPRESSION
<130> 25148-EP-EPT
<160> 139
<170> PatentIn version 3.5
<210> 1
   <211> 30
   <212> DNA
   <213> Artificial sequence
<220>
   <223> Primer
<400> 1
   ggggctcgag gcttttggct tgtgatcttg 30
<210> 2
   <211> 36
   <212> DNA
   <213> Artificial sequence
<220>
   <223> Primer
<400> 2
   ctgaagacga ccattttgac cacgagaatg tcaagc 36
<210> 3
   <211> 30
   <212> DNA
   <213> Artificial sequence
<220>
   <223> Primer
<400> 3
   ggggctcgag gcgcaatcta aggacatccg 30
<210> 4
<400> 4
   000
<210> 5
   <211> 38
   <212> DNA
   <213> Artificial sequence
<220>
   <223> Primer
<400> 5
   cattctcgtg gtcaaaatgg tcgtcttcag caaaaccg 38
<210> 6
   <211> 34
   <212> DNA
   <213> Artificial sequence
<220>
   <223> Primer
<400> 6
   ggggggcgcg ccctaagcct gagcggcgaa tccc 34
<210> 7
   <211> 32
   <212> DNA
   <213> Artificial sequence
<220>
   <223> Primer
<400> 7
   ggcgtctccg gaggccagac tgaccctgcg cg 32
<210> 8
   <211> 32
   <212> DNA
   <213> Artificial sequence
<220>
   <223> Primer
<400> 8
   cgcgcagggt cagtctggcc tccggagacg cc 32
<210> 9
   <211> 32
   <212> DNA
   <213> Artificial sequence
<220>
   <223> Primer
<400> 9
   ggcgtctccg gaggcaagac ttaccctgcg cg 32
<210> 10
   <211> 32
   <212> DNA
   <213> Artificial sequence
<220>
   <223> Primer
<400> 10
   cgcgcagggt aagtcttgcc tccggagacg cc 32
<210> 11
   <211> 29
   <212> DNA
   <213> Artificial sequence
<220>
   <223> Primer
<400> 11
   cggcttcgaa tggctaggtg tcgattttg 29
<210> 12
   <211> 41
   <212> DNA
   <213> Artificial sequence
<220>
   <223> Primer
<400> 12
   ggtagtggat cgtgtggaat tggcaggcaa tctggagagt a 41
<210> 13
   <211> 21
   <212> DNA
   <213> Artificial sequence
<220>
   <223> Primer
<400> 13
   aattccacac gatccactac c 21
<210> 14
   <211> 32
   <212> DNA
   <213> Artificial sequence
<220>
   <223> Primer
<400> 14
   actaggcgcg cctttgtttg gtcgatcgga ga 32
<210> 15
   <211> 31
   <212> DNA
   <213> Artificial sequence
<220>
   <223> Primer
<400> 15
   ttgaggtacc aattccacac gatccactac c 31
<210> 16
   <211> 30
   <212> DNA
   <213> Artificial sequence
<220>
   <223> Primer
<400> 16
   tcatcccggg cgtccatcgc attgattctt 30
<210> 17
   <211> 1001
   <212> DNA
   <213> Aspergillus niger
<400> 17
   gcttttggct tgtgatcttg attgctagag atgtatatcc tcacggatac cgccggagtg      60
   cgccatttct ggttaccttc tctttccctt tttgtctcga tcgtgaggcg gaacgcagga     120
   tgaagacacg gcttctccat cgcggcccac caaccaacaa tgtccttgga cgcccaactc     180
   tccatctact ggtcattggt ccaatgcaga gactccgtcg agctcaaatg ggccggccaa     240
   ccccgagtcg tcaggggcag cggcagcgac gagctaaatt agaccactga taagacgcga     300
   tagtccaaag tctgaccgtc acattgtgcc aggcagataa gttgaatcgt gtgactggat     360
   gttggctaac gtatggcgtc tccggaggcc cgacggaccc tgcgcgatcg gcggtggagc     420
   gcaatctaag gacatccgcg cctaagatat ctacccttca gcagttcagc ctagccctgc     480
   agacttgtcg gaccagtgct atcgtgatcg gcccccacgg tcgaatgagc tcttgtctct     540
   ttccgtcaga ccctgccagt taatctgcta tctactccgc ggtaacatcg tgcctgtctc     600
   cactaaggca gggtccaggg ctgtatgtct tactttgcac cgagtcggcc gccggttggc     660
   tctgtcttgg caattgcgaa tatcctcacg ggcgacggac gacacggatt tggacggaca     720
   tgcggagatc ttcgtcggtt tattcctgga agggacatca tctccttcca tcatgacggc     780
   tgccatagcg gggactctga gacattttgc tctgaagagc atggtcgact tggatgatgg     840
   gaggagttga tcgaggtcaa tgaggagagg cttgcaagta taagaagaga ctgctcgacc     900
   agcagaatgg atcttcttgt tcatcaacca agagtccaag gcttctttgt ctggttctat     960
   ctcttctccg aactctcttg cttgacattc tcgtggtcaa a                        1001
<210> 18
   <211> 582
   <212> DNA
   <213> Aspergillus niger
<400> 18
   cgcaatctaa ggacatccgc gcctaagata tctacccttc agcagttcag cctagccctg      60
   cagacttgtc ggaccagtgc tatcgtgatc ggcccccacg gtcgaatgag ctcttgtctc     120
   tttccgtcag accctgccag ttaatctgct atctactccg cggtaacatc gtgcctgtct     180
   ccactaaggc agggtccagg gctgtatgtc ttactttgca ccgagtcggc cgccggttgg     240
   ctctgtcttg gcaattgcga atatcctcac gggcgacgga cgacacggat ttggacggac     300
   atgcggagat cttcgtcggt ttattcctgg aagggacatc atctccttcc atcatgacgg     360
   ctgccatagc ggggactctg agacattttt gctctgaaga gcatggtcga cttggatgat     420
   ggaggagttg atcgaggtca atgaggagag gcttgcaagt ataagaagag actgctcgac     480
   cagcagaatg gatcttcttg ttcatcaacc aagagtccaa ggcttctttg tctggttcta     540
   tctcttctcc gaactctctt gcttgacatt ctcgtggtca aa                        582
<210> 19
<400> 19
   000
<210> 20
<400> 20
   000
<210> 21
   <211> 1185
   <212> DNA
   <213> Aspergillus niger
<400> 21
   atggtcgtct tcagcaaaac cgctgccctc gttctgggtc tgtcctccgc cgtctctgcg      60
   gcgccggctc ctactcgcaa gggcttcacc atcaaccaga ttgcccggcc tgccaacaag     120
   acccgcacca tcaacctgcc aggcatgtac gcccgttccc tggccaagtt tggcggtacg     180
   gtgccccaga gcgtgaagga ggctgccagc aagggtagtg ccgtgaccac gccccagaac     240
   aatgacgagg agtacctgac tcccgtcact gtcggaaagt ccaccctcca tctggacttt     300
   gacaccggat ctgcagatct ctgggtcttc tcggacgagc tcccttcctc ggagcagacc     360
   ggtcacgatc tgtacacgcc tagctccagc gcgaccaagc tgagcggcta cacttgggac     420
   atctcctacg gtgacggcag ctcggccagc ggagacgtgt accgggatac tgtcactgtc     480
   ggcggtgtca ccaccaacaa gcaggctgtt gaagcagcca gcaagatcag ctccgagttc     540
   gttcagaaca cggccaatga cggccttttg ggactggcct ttagctccat caacactgtc     600
   cagcccaagg cgcagaccac cttcttcgac accgtcaagt cccagctgga ctctcccctt     660
   ttcgccgtgc agctgaagca cgacgccccc ggtgtttacg actttggcta catcgatgac     720
   tccaagtaca ccggttctat cacctacacg gatgccgata gctcccaggg ttactggggc     780
   ttcagcaccg acggctacag tatcggtgac ggcagctcca gctccagcgg cttcagcgcc     840
   attgctgaca ccggtaccac cctcatcctc ctcgatgacg aaatcgtctc cgcctactac     900
   gagcaggttt ctggcgctca ggagagcgag gaagccggtg gctacgtttt ctcttgctcg     960
   accaaccccc ctgacttcac tgtcgtgatt ggcgactaca aggccgttgt tccgggcaag    1020
   tacatcaact acgctcccat ctcgactggc agctccacct gctttggcgg tatccagagc    1080
   aacagcggtc tgggactgtc catcctgggt gatgttttct tgaagagcca gtacgtggtc    1140
   ttcaactctg agggccctaa gctgggattc gccgctcagg cttag                    1185
<210> 22
   <211> 33
   <212> DNA
   <213> Aspergillus niger
<220>
   <221> misc_feature
   <222> (2) .. (6)
   <223> n is a, c, g, or t
<220>
   <221> misc_feature
   <222> (14) .. (32)
   <223> n is a, c, g, or t
<400> 22
   snnnnnccgw cggnnnnnnn nnnnnnnnnn nns 33
<210> 23
   <211> 3174
   <212> DNA
   <213> Aspergillus niger
<400> 23
   tcggggagca tgtcggagga aaattcaagt cctggcaaga gcacggaagc ccttgctgtc        60
   aacatctcta cccgctttaa ttatctagta tgtatggtta tcgattgctc atatttgggt       120
   acaagagaag gggaaagtgg gctgccgaga gctgaggtcc attgcagatc ggactattta       180
   tagttgactg gtggcagaat tgtgggttcc ggagaagaca ataacagatg atgggggcta       240
   atgggagaag attcggacag tgaccagcat acaatagata ctggttccca gtgttgctct       300
   gatgatggca tctttgactt cacagcgcca gtgccattct atccatcctg ttccgacttg       360
   cttccccaca ccaagaagag gaggatccta cacccgacgg agagccgggg agagcatcgg      420
   agaagttgat ccttgactga gaccatgaca gcagaatact acattgtaga ttgacgataa       480
   catggagaga gcatgtgcct gatcatgcag ccccagtgac ctgcctcttc aggatagccc       540
   aatgttgcag aaattacggc attggcccga ttagctagtg gcttatcaag aaagcggggg       600
   agaagctacc aaagtgacga aataggagca gaactatcaa ttctgctagc cgtgccaaga       660
   accaggaaag ggagaaggaa ggcgggtgga gccggtcagg ctgggcggtc cctgaggttg       720
   cactcgagtg gcacccgact tgttctcccg ccttccgccc agccaatcat aagaaacccg       780
   gtcctggcaa ggtcttggcg gggatggtgc aagccagcca aacaaccggt ttgttggacg       840
   ccctgcggta cactgaaatc ttgggctgtt cgacaaaaac aaagcaggta acacaaagat       900
   ataatacggc ggaatgatag gatatccctg tcgatcagcc ggacaggatg gcgctagtgt       960
   cacctccagc ttcggctacc ctcgcagcgg acccaatcag cgtccctccc agcccccgat      1020
   acagtaatgg tatgcacatc gcagtcttaa tcgcctgcag cggcagtaat gatagtcctg      1080
   ccggtgaata ataccccata acaaacaaat aaataatact acttatctct cctcgtccct      1140
   ttcactttcc ctttgccgtc ttcaatcccc tcatcttggt ctcttcggca gcctttcacc      1200
   atgctgtcgt ctctccttag ccagggagca gccgtatccc tcgcggtgtt gtcgctgctc      1260
   ccttcgcctg tagccgcgga gatcttcgaa aagctatccg gcgtccccaa tggtgagtta 1320
   tagaccccaa ttcttcattt tgagccacat actgacgtga ttccttcgaa tactaccagg 1380
   ctggagatac gccaacaatc ctcaaggcaa cgaggtcatt cgcttgcaaa tcgcccttca 1440
   gcagcatgat gtcgctggtt tcgaacaagc cgtgatggat atgtccaccc ccggacacgc 1500
   cgactatgga aagcatttcc gcacccacga tgagatgaag cgcatgttgc tccccagcga 1560
   gactgccgtc gactcagtcc gcgactggct ggaatccgcc ggtgtccaca atatccaggt 1620
   cgacgccgac tgggtcaagt tccataccac cgtaaacaag gccaatgccc tgctggatgc 1680
   cgacttcaag tggtatgtca gcgacgccaa gcatattcgt cgtctgcgca ccctgcaata 1740
   ctccatcccc gacgccctgg tctcgcacat caacatgatc cagcccacca cccgctttgg 1800
   ccagatccag cccaaccgtg ccaccatgcg cagcaagccc aagcacgccg atgagacatt 1860
   cctcaccgca gccaccctgg cccagaacac ctcccactgc gactccatca tcacaccgca 1920
   ctgtctgaag cagctgtaca acatcggtga ctaccaggcc gatcccaagt ccggcagcaa 1980
   gatcggcttt gccagctacc ttgaggaata cgcccggtat gccgatctcg agaggttcga 2040
   gcagcacctg gctcccaatg ccatcggcca gaacttcagc gtcgtccaat tcaacggcgg 2100
   cctcaacgat cagctttcat cgagtgacag cggcgaagcc aacctcgacc tgcagtacat 2160
   cctgggcgtc agcgctcccg tccccatcac cgagtacagc accggcggac gcggcgaact 2220
   agtccccgac ctgagctccc ccgaccccaa cgacaacagc aacgagccct accttgactt 2280
   ccttcaggga atcctcaagc ttaacaactc cgacctccca caagtcatct ctacctccta 2340
   cggtgaagac gaacaggtat gcacctcacc tgacccattc cattttacat ccctcacctc 2400
   tctcaaccaa actaacaaca ccaacagact atccccgtcc cctacgcccg caccgtctgc 2460
   aacctctacg cccaactcgg cagccgcggc gtctctgtaa tcttctccag cggcgactcc 2520
   ggcgtcggcg ccgcctgcct caccaacgac ggcaccaacc gcacgcactt ccctcctcaa 2580
   ttccccgcct cctgcccctg ggtaacctcc gtcggcgcaa cctccaagac ctcccccgag 2640
   caagccgtct ccttctcctc cggcggcttc tccgacctct ggccccgccc ctcctaccaa 2700
   cacgccgccg tgcaaaccta cctcaccaag cacctgggca acaagttctc ggggcttttc 2760
   aacgcctccg gccgcgcctt ccccgacgtc tccgcgcagg gcgtcaacta cgctgtttac 2820
   gacaagggca tgcttggcca gttcgacggg acgagttgct ccgcgccgac gttcagtggc 2880
   gtcatcgcgt tgttgaacga tgcgagactg agggccgggt tgcctgtgat ggggttcttg 2940
   aatccgttcc tgtatggtgt cggaagtgag aagggtgcgt tgaatgatat tgtgaacggc 3000
   gggagtgtgg gttgtgatgg gaggaatcgg ttcgggggca cgcctaatgg tagtcctgtt 3060
   gtgccgtttg ctagttggaa tgccacgacc gggtgggatc ctgtgtcggg gttgggaacg 3120
   ccggattttg cgaagttgaa aggggtggcg ttgggtgagg agggtggtaa ttaa 3174
<210> 24
   <211> 1836
   <212> DNA
   <213> Aspergillus niger
<220>
   <221> CDS
   <222> (1)..(1836)
<400> 24
   atg ctg tcg tct ctc ctt agc cag gga gca gcc gta tcc ctc gcg gtg      48
   Met Leu Ser Ser Leu Leu Ser Gln Gly Ala Ala Val Ser Leu Ala Val
   1               5                   10                  15
   ttg tcg ctg ctc cct tcg cct gta gcc gcg gag atc ttc gaa aag cta      96
   Leu Ser Leu Leu Pro Ser Pro Val Ala Ala Glu Ile Phe Glu Lys Leu
               20                  25                  30
   tcc ggc gtc ccc aat ggc tgg aga tac gcc aac aat cct caa ggc aac     144
   Ser Gly Val Pro Asn Gly Trp Arg Tyr Ala Asn Asn Pro Gln Gly Asn
           35                  40                  45
   gag gtc att cgc ttg caa atc gcc ctt cag cag cat gat gtc gct ggt     192
   Glu Val Ile Arg Leu Gln Ile Ala Leu Gln Gln His Asp Val Ala Gly
       50                  55                  60
   ttc gaa caa gcc gtg atg gat atg tcc acc ccc gga cac gcc gac tat     240
   Phe Glu Gln Ala Val Met Asp Met Ser Thr Pro Gly His Ala Asp Tyr
   65                  70                  75                  80
   gga aag cat ttc cgc acc cac gat gag atg aag cgc atg ttg ctc ccc     288
   Gly Lys His Phe Arg Thr His Asp Glu Met Lys Arg Met Leu Leu Pro
                   85                  90                  95
   agc gag act gcc gtc gac tca gtc cgc gac tgg ctg gaa tcc gcc ggt     336
   Ser Glu Thr Ala Val Asp Ser Val Arg Asp Trp Leu Glu Ser Ala Gly
               100                 105                 110
   gtc cac aat atc cag gtc gac gcc gac tgg gtc aag ttc cat acc acc     384
   Val His Asn Ile Gln Val Asp Ala Asp Trp Val Lys Phe His Thr Thr
           115                 120                 125
   gta aac aag gcc aat gcc ctg ctg gat gcc gac ttc aag tgg tat gtc     432
   Val Asn Lys Ala Asn Ala Leu Leu Asp Ala Asp Phe Lys Trp Tyr Val
       130                 135                 140
   agc gac gcc aag cat att cgt cgt ctg cgc acc ctg caa tac tcc atc     480
   Ser Asp Ala Lys His Ile Arg Arg Leu Arg Thr Leu Gln Tyr Ser Ile
   145                 150                 155                 160
   ccc gac gcc ctg gtc tcg cac atc aac atg atc cag ccc acc acc cgc     528
   Pro Asp Ala Leu Val Ser His Ile Asn Met Ile Gln Pro Thr Thr Arg
                   165                 170                 175
   ttt ggc cag atc cag ccc aac cgt gcc acc atg cgc agc aag ccc aag     576
   Phe Gly Gln Ile Gln Pro Asn Arg Ala Thr Met Arg Ser Lys Pro Lys
               180                 185                 190
   cac gcc gat gag aca ttc ctc acc gca gcc acc ctg gcc cag aac acc     624
   His Ala Asp Glu Thr Phe Leu Thr Ala Ala Thr Leu Ala Gln Asn Thr
           195                 200                 205
   tcc cac tgc gac tcc atc atc aca ccg cac tgt ctg aag cag ctg tac     672
   Ser His Cys Asp Ser Ile Ile Thr Pro His Cys Leu Lys Gln Leu Tyr
       210                 215                 220
   aac atc ggt gac tac cag gcc gat ccc aag tcc ggc agc aag atc ggc       720
   Asn Ile Gly Asp Tyr Gln Ala Asp Pro Lys Ser Gly Ser Lys Ile Gly
   225                 230                 235                 240
   ttt gcc agc tac ctt gag gaa tac gcc cgg tat gcc gat ctc gag agg       768
   Phe Ala Ser Tyr Leu Glu Glu Tyr Ala Arg Tyr Ala Asp Leu Glu Arg
                   245                 250                 255
   ttc gag cag cac ctg gct ccc aat gcc atc ggc cag aac ttc agc gtc       816
   Phe Glu Gln His Leu Ala Pro Asn Ala Ile Gly Gln Asn Phe Ser Val
               260                 265                 270
   gtc caa ttc aac ggc ggc ctc aac gat cag ctt tca tcg agt gac agc       864
   Val Gln Phe Asn Gly Gly Leu Asn Asp Gln Leu Ser Ser Ser Asp Ser
           275                 280                 285
   ggc gaa gcc aac ctc gac ctg cag tac atc ctg ggc gtc agc gct ccc       912
   Gly Glu Ala Asn Leu Asp Leu Gln Tyr Ile Leu Gly Val Ser Ala Pro
       290                 295                 300
   gtc ccc atc acc gag tac agc acc ggc gga cgc ggc gaa cta gtc ccc       960
   Val Pro Ile Thr Glu Tyr Ser Thr Gly Gly Arg Gly Glu Leu Val Pro
   305                 310                 315                 320
   gac ctg agc tcc ccc gac ccc aac gac aac agc aac gag ccc tac ctt      1008
   Asp Leu Ser Ser Pro Asp Pro Asn Asp Asn Ser Asn Glu Pro Tyr Leu
                   325                 330                 335
   gac ttc ctt cag gga atc ctc aag ctt aac aac tcc gac ctc cca caa      1056
   Asp Phe Leu Gln Gly Ile Leu Lys Leu Asn Asn Ser Asp Leu Pro Gln
               340                 345                 350
   gtc atc tct acc tcc tac ggt gaa gac gaa cag act atc ccc gtc ccc      1104
   Val Ile Ser Thr Ser Tyr Gly Glu Asp Glu Gln Thr Ile Pro Val Pro
           355                 360                 365
   tac gcc cgc acc gtc tgc aac ctc tac gcc caa ctc ggc agc cgc ggc      1152
   Tyr Ala Arg Thr Val Cys Asn Leu Tyr Ala Gln Leu Gly Ser Arg Gly
       370                 375                 380
   gtc tct gta atc ttc tcc agc ggc gac tcc ggc gtc ggc gcc gcc tgc      1200
   Val Ser Val Ile Phe Ser Ser Gly Asp Ser Gly Val Gly Ala Ala Cys
   385                 390                 395                 400
   ctc acc aac gac ggc acc aac cgc acg cac ttc cct cct caa ttc ccc      1248
   Leu Thr Asn Asp Gly Thr Asn Arg Thr His Phe Pro Pro Gln Phe Pro
                   405                 410                 415
   gcc tcc tgc ccc tgg gta acc tcc gtc ggc gca acc tcc aag acc tcc      1296
   Ala Ser Cys Pro Trp Val Thr Ser Val Gly Ala Thr Ser Lys Thr Ser
               420                 425                 430
   ccc gag caa gcc gtc tcc ttc tcc tcc ggc ggc ttc tcc gac ctc tgg      1344
   Pro Glu Gln Ala Val Ser Phe Ser Ser Gly Gly Phe Ser Asp Leu Trp
           435                 440                 445
   ccc cgc ccc tcc tac caa cac gcc gcc gtg caa acc tac ctc acc aag      1392
   Pro Arg Pro Ser Tyr Gln His Ala Ala Val Gln Thr Tyr Leu Thr Lys
       450                 455                 460
   cac ctg ggc aac aag ttc tcg ggg ctt ttc aac gcc tcc ggc cgc gcc      1440
   His Leu Gly Asn Lys Phe Ser Gly Leu Phe Asn Ala Ser Gly Arg Ala
   465                 470                 475                 480
   ttc ccc gac gtc tcc gcg cag ggc gtc aac tac gct gtt tac gac aag        1488
   Phe Pro Asp Val Ser Ala Gln Gly Val Asn Tyr Ala Val Tyr Asp Lys
                   485                 490                 495
   ggc atg ctt ggc cag ttc gac ggg acg agt tgc tcc gcg ccg acg ttc        1536
   Gly Met Leu Gly Gln Phe Asp Gly Thr Ser Cys Ser Ala Pro Thr Phe
               500                 505                 510
   agt ggc gtc atc gcg ttg ttg aac gat gcg aga ctg agg gcc ggg ttg        1584
   Ser Gly Val Ile Ala Leu Leu Asn Asp Ala Arg Leu Arg Ala Gly Leu
           515                 520                 525
   cct gtg atg ggg ttc ttg aat ccg ttc ctg tat ggt gtc gga agt gag        1632
   Pro Val Met Gly Phe Leu Asn Pro Phe Leu Tyr Gly Val Gly Ser Glu
       530                 535                 540
   aag ggt gcg ttg aat gat att gtg aac ggc ggg agt gtg ggt tgt gat        1680
   Lys Gly Ala Leu Asn Asp Ile Val Asn Gly Gly Ser Val Gly Cys Asp
   545                 550                 555                 560
   ggg agg aat cgg ttc ggg ggc acg cct aat ggt agt cct gtt gtg ccg        1728
   Gly Arg Asn Arg Phe Gly Gly Thr Pro Asn Gly Ser Pro Val Val Pro
                   565                 570                 575
   ttt gct agt tgg aat gcc acg acc ggg tgg gat cct gtg tcg ggg ttg        1776
   Phe Ala Ser Trp Asn Ala Thr Thr Gly Trp Asp Pro Val Ser Gly Leu
               580                 585                 590
   gga acg ccg gat ttt gcg aag ttg aaa ggg gtg gcg ttg ggt gag gag        1824
   Gly Thr Pro Asp Phe Ala Lys Leu Lys Gly Val Ala Leu Gly Glu Glu
           595                 600                 605
   ggt ggt aat taa                                                        1836
   Gly Gly Asn
       610
<210> 25
   <211> 611
   <212> PRT
   <213> Aspergillus niger
<400> 25
   Met Leu Ser Ser Leu Leu Ser Gln Gly Ala Ala Val Ser Leu Ala Val
   1 5 10 15
   Leu Ser Leu Leu Pro Ser Pro Val Ala Ala Glu Ile Phe Glu Lys Leu
   20 25 30
   Ser Gly Val Pro Asn Gly Trp Arg Tyr Ala Asn Asn Pro Gln Gly Asn
   35 40 45
   Glu Val Ile Arg Leu Gln Ile Ala Leu Gln Gln His Asp Val Ala Gly
   50 55 60
   Phe Glu Gln Ala Val Met Asp Met Ser Thr Pro Gly His Ala Asp Tyr
   65 70 75 80
   Gly Lys His Phe Arg Thr His Asp Glu Met Lys Arg Met Leu Leu Pro
   85 90 95
   Ser Glu Thr Ala Val Asp Ser Val Arg Asp Trp Leu Glu Ser Ala Gly
   100 105 110
   Val His Asn Ile Gln Val Asp Ala Asp Trp Val Lys Phe His Thr Thr
   115 120 125
   Val Asn Lys Ala Asn Ala Leu Leu Asp Ala Asp Phe Lys Trp Tyr Val
   130 135 140
   Ser Asp Ala Lys His Ile Arg Arg Leu Arg Thr Leu Gln Tyr Ser Ile
   145 150 155 160
   Pro Asp Ala Leu Val Ser His Ile Asn Met Ile Gln Pro Thr Thr Arg
   165 170 175
   Phe Gly Gln Ile Gln Pro Asn Arg Ala Thr Met Arg Ser Lys Pro Lys
   180 185 190
   His Ala Asp Glu Thr Phe Leu Thr Ala Ala Thr Leu Ala Gln Asn Thr
   195 200 205
   Ser His Cys Asp Ser Ile Ile Thr Pro His Cys Leu Lys Gln Leu Tyr
   210 215 220
   Asn Ile Gly Asp Tyr Gln Ala Asp Pro Lys Ser Gly Ser Lys Ile Gly
   225 230 235 240
   Phe Ala Ser Tyr Leu Glu Glu Tyr Ala Arg Tyr Ala Asp Leu Glu Arg
   245 250 255
   Phe Glu Gln His Leu Ala Pro Asn Ala Ile Gly Gln Asn Phe Ser Val
   260 265 270
   Val Gln Phe Asn Gly Gly Leu Asn Asp Gln Leu Ser Ser Ser Asp Ser
   275 280 285
   Gly Glu Ala Asn Leu Asp Leu Gln Tyr Ile Leu Gly Val Ser Ala Pro
   290 295 300
   Val Pro Ile Thr Glu Tyr Ser Thr Gly Gly Arg Gly Glu Leu Val Pro
   305 310 315 320
   Asp Leu Ser Ser Pro Asp Pro Asn Asp Asn Ser Asn Glu Pro Tyr Leu
   325 330 335
   Asp Phe Leu Gln Gly Ile Leu Lys Leu Asn Asn Ser Asp Leu Pro Gln
   340 345 350
   Val Ile Ser Thr Ser Tyr Gly Glu Asp Glu Gln Thr Ile Pro Val Pro
   355 360 365
   Tyr Ala Arg Thr Val Cys Asn Leu Tyr Ala Gln Leu Gly Ser Arg Gly
   370 375 380
   Val Ser Val Ile Phe Ser Ser Gly Asp Ser Gly Val Gly Ala Ala Cys
   385 390 395 400
   Leu Thr Asn Asp Gly Thr Asn Arg Thr His Phe Pro Pro Gln Phe Pro
   405 410 415
   Ala Ser Cys Pro Trp Val Thr Ser Val Gly Ala Thr Ser Lys Thr Ser
   420 425 430
   Pro Glu Gln Ala Val Ser Phe Ser Ser Gly Gly Phe Ser Asp Leu Trp
   435 440 445
   Pro Arg Pro Ser Tyr Gln His Ala Ala Val Gln Thr Tyr Leu Thr Lys
   450 455 460
   His Leu Gly Asn Lys Phe Ser Gly Leu Phe Asn Ala Ser Gly Arg Ala
   465 470 475 480
   Phe Pro Asp Val Ser Ala Gln Gly Val Asn Tyr Ala Val Tyr Asp Lys
   485 490 495
   Gly Met Leu Gly Gln Phe Asp Gly Thr Ser Cys Ser Ala Pro Thr Phe
   500 505 510
   Ser Gly Val Ile Ala Leu Leu Asn Asp Ala Arg Leu Arg Ala Gly Leu
   515 520 525
   Pro Val Met Gly Phe Leu Asn Pro Phe Leu Tyr Gly Val Gly Ser Glu
   530 535 540
   Lys Gly Ala Leu Asn Asp Ile Val Asn Gly Gly Ser Val Gly Cys Asp
   545 550 555 560
   Gly Arg Asn Arg Phe Gly Gly Thr Pro Asn Gly Ser Pro Val Val Pro
   565 570 575
   Phe Ala Ser Trp Asn Ala Thr Thr Gly Trp Asp Pro Val Ser Gly Leu
   580 585 590
   Gly Thr Pro Asp Phe Ala Lys Leu Lys Gly Val Ala Leu Gly Glu Glu
   595 600 605
   Gly Gly Asn
   610
<210> 26
   <211> 2547
   <212> DNA
   <213> Aspergillus niger
<400> 26
   gcgaattcga catgctggag gtgcttctaa atgaacagta tgacggtcat gaggggatag 60
   agtcggctgg cggagggaac cttccgtcac agtttttgca atcgtgatca tcgatggtct 120
   catgaatgta cgaatttacg cgtcgtagct tcttcctctt tttgcttgga ttcttgctag 180
   ttcctttctg tacacttccg cttttggctt gtgatcttga ttgctagaga tgtatatcct 240
   cacggatacc gccggagtgc gccatttctg gttaccttct ctttcccttt ttgtctcgat 300
   cgtgaggcgg aacgcaggat gaagacacgg cttctccatc gcggcccacc aaccaacaat 360
   gtccttggac gcccaactct ccatctactg gtcattggtc caatgcagag actccgtcga 420
   gctcaaatgg gccggccaac cccgagtcgt caggggcagc ggcagcgacg agctaaatta 480
   gaccactgat aagacgcgat agtccaaagt ctgaccgtca cattgtgcca ggcagataag 540
   ttgaatcgtg tgactggatg ttggctaacg tatggcgtct ccggaggccc gacggaccct 600
   gcgcgatcgg cggtggagcg caatctaagg acatccgcgc ctaagatatc tacccttcag 660
   cagttcagcc tagccctgca gacttgtcgg accagtgcta tcgtgatcgg cccccacggt 720
   cgaatgagct cttgtctctt tccgtcagac cctgccagtt aatctgctat ctactccgcg 780
   gtaacatcgt gcctgtctcc actaaggcag ggtccagggc tgtatgtctt actttgcacc 840
   gagtcggccg ccggttggct ctgtcttggc aattgcgaat atcctcacgg gcgacggacg 900
   acacggattt ggacggacat gcggagatct tcgtcggttt attcctggaa gggacatcat 960
   ctccttccat catgacggct gccatagcgg ggactctgag acatttttgc tctgaagagc 1020
   atggtcgact tggatgatgg aggagttgat cgaggtcaat gaggagaggc ttgcaagtat 1080
   aagaagagac tgctcgacca gcagaatgga tcttcttgtt catcaaccaa gagtccaagg 1140
   cttctttgtc tggttctatc tcttctccga actctcttgc ttgacattct cgtggtcaaa 1200
   atggtcgtct tcagcaaaac cgctgccctc gttctgggtc tgtcctccgc cgtctctgcg 1260
   gcgccggctc ctactcgcaa gggcttcacc atcaaccaga ttgcccggcc tgccaacaag 1320
   acccgcacca tcaacctgcc aggcatgtac gcccgttccc tggccaagtt tggcggtacg 1380
   gtgccccaga gcgtgaagga ggctgccagc aagggtagtg ccgtgaccac gccccagaac 1440
   aatgacgagg agtacctgac tcccgtcact gtcggaaagt ccaccctcca tctggacttt 1500
   gacaccggat ctgcagatct gtaagcttcc ctgctcgggt gttcgggcaa atcgtgacta 1560
   acctggacta gctgggtctt ctcggacgag ctcccttcct cggagcagac cggtcacgat      1620
   ctgtacacgc ctagctccag cgcgaccaag ctgagcggct acacttggga catctcctac      1680
   ggtgacggca gctcggccag cggagacgtg taccgggata ctgtcactgt cggcggtgtc      1740
   accaccaaca agcaggctgt tgaagcagcc agcaagatca gctccgagtt cgttcagaac      1800
   acggccaatg acggcctttt gggactggcc tttagctcca tcaacactgg tgagtcaatc      1860
   ctacatcagc cgggttgacc tacctgctga ccgatacaca gtccagccca aggcgcagac      1920
   caccttcttc gacaccgtca agtcccagct ggactctccc cttttcgccg tgcagctgaa      1980
   gcacgacgcc cccggtgttt acgactttgg ctacatcgat gactccaagt acaccggttc      2040
   tatcacctac acggatgccg atagctccca gggttactgg ggcttcagca ccgacggcta      2100
   cagtatcggt gacggcagct ccagctccag cggcttcagc gccattgctg gtaagaaccg      2160
   ccttcattta acacacaact tgtccacctc tttactaact agtgtataga caccggtacc      2220
   accctcatcc tcctcgatga cgaaatcgtc tccgcctact acgagcaggt ttctggcgct      2280
   caggagagcg aggaagccgg tggctacgtt ttctcttgct cgaccaaccc ccctgacttc      2340
   actgtcgtga ttggcgacta caaggccgtt gttccgggca agtacatcaa ctacgctccc      2400
   atctcgactg gcagctccac ctgctttggc ggtatccaga gcaacagcgg tctgggactg      2460
   tccatcctgg gtgatgtttt cttgaagagc cagtacgtgg tcttcaactc tgagggccct      2520
   aagctgggat tcgccgctca ggcttag                                          2547
<210> 27
   <211> 1185
   <212> DNA
   <213> Aspergillus niger
<220>
   <221> CDS
   <222> (1)..(1185)
<400> 27
   atg gtc gtc ttc agc aaa acc gct gcc ctc gtt ctg ggt ctg tcc tcc        48
   Met Val Val Phe Ser Lys Thr Ala Ala Leu Val Leu Gly Leu Ser Ser
   1               5                   10                  15
   gcc gtc tct gcg gcg ccg gct cct act cgc aag ggc ttc acc atc aac        96
   Ala Val Ser Ala Ala Pro Ala Pro Thr Arg Lys Gly Phe Thr Ile Asn
               20                  25                  30
   cag att gcc cgg cct gcc aac aag acc cgc acc atc aac ctg cca ggc       144
   Gln Ile Ala Arg Pro Ala Asn Lys Thr Arg Thr Ile Asn Leu Pro Gly
           35                  40                  45
   atg tac gcc cgt tcc ctg gcc aag ttt ggc ggt acg gtg ccc cag agc       192
   Met Tyr Ala Arg Ser Leu Ala Lys Phe Gly Gly Thr Val Pro Gln Ser
       50                  55                  60
   gtg aag gag gct gcc agc aag ggt agt gcc gtg acc acg ccc cag aac       240
   Val Lys Glu Ala Ala Ser Lys Gly Ser Ala Val Thr Thr Pro Gln Asn
   65                  70                  75                  80
   aat gac gag gag tac ctg act ccc gtc act gtc gga aag tcc acc ctc      288
   Asn Asp Glu Glu Tyr Leu Thr Pro Val Thr Val Gly Lys Ser Thr Leu
                   85                  90                  95
   cat ctg gac ttt gac acc gga tct gca gat ctc tgg gtc ttc tcg gac      336
   His Leu Asp Phe Asp Thr Gly Ser Ala Asp Leu Trp Val Phe Ser Asp
               100                 105                 110
   gag ctc cct tcc tcg gag cag acc ggt cac gat ctg tac acg cct agc      384
   Glu Leu Pro Ser Ser Glu Gln Thr Gly His Asp Leu Tyr Thr Pro Ser
           115                 120                 125
   tcc agc gcg acc aag ctg agc ggc tac act tgg gac atc tcc tac ggt      432
   Ser Ser Ala Thr Lys Leu Ser Gly Tyr Thr Trp Asp Ile Ser Tyr Gly
       130                 135                 140
   gac ggc agc tcg gcc agc gga gac gtg tac cgg gat act gtc act gtc      480
   Asp Gly Ser Ser Ala Ser Gly Asp Val Tyr Arg Asp Thr Val Thr Val
   145                 150                 155                 160
   ggc ggt gtc acc acc aac aag cag gct gtt gaa gca gcc agc aag atc      528
   Gly Gly Val Thr Thr Asn Lys Gln Ala Val Glu Ala Ala Ser Lys Ile
                   165                 170                 175
   agc tcc gag ttc gtt cag aac acg gcc aat gac ggc ctt ttg gga ctg      576
   Ser Ser Glu Phe Val Gln Asn Thr Ala Asn Asp Gly Leu Leu Gly Leu
               180                 185                 190
   gcc ttt agc tcc atc aac act gtc cag ccc aag gcg cag acc acc ttc      624
   Ala Phe Ser Ser Ile Asn Thr Val Gln Pro Lys Ala Gln Thr Thr Phe
           195                 200                 205
   ttc gac acc gtc aag tcc cag ctg gac tct ccc ctt ttc gcc gtg cag      672
   Phe Asp Thr Val Lys Ser Gln Leu Asp Ser Pro Leu Phe Ala Val Gln
       210                 215                 220
   ctg aag cac gac gcc ccc ggt gtt tac gac ttt ggc tac atc gat gac      720
   Leu Lys His Asp Ala Pro Gly Val Tyr Asp Phe Gly Tyr Ile Asp Asp
   225                 230                 235                 240
   tcc aag tac acc ggt tct atc acc tac acg gat gcc gat agc tcc cag      768
   Ser Lys Tyr Thr Gly Ser Ile Thr Tyr Thr Asp Ala Asp Ser Ser Gln
                   245                 250                 255
   ggt tac tgg ggc ttc agc acc gac ggc tac agt atc ggt gac ggc agc      816
   Gly Tyr Trp Gly Phe Ser Thr Asp Gly Tyr Ser Ile Gly Asp Gly Ser
               260                 265                 270
   tcc agc tcc agc ggc ttc agc gcc att gct gac acc ggt acc acc ctc      864
   Ser Ser Ser Ser Gly Phe Ser Ala Ile Ala Asp Thr Gly Thr Thr Leu
           275                 280                 285
   atc ctc ctc gat gac gaa atc gtc tcc gcc tac tac gag cag gtt tct      912
   Ile Leu Leu Asp Asp Glu Ile Val Ser Ala Tyr Tyr Glu Gln Val Ser
       290                 295                 300
   ggc gct cag gag agc gag gaa gcc ggt ggc tac gtt ttc tct tgc tcg      960
   Gly Ala Gln Glu Ser Glu Glu Ala Gly Gly Tyr Val Phe Ser Cys Ser
   305                 310                 315                 320
   acc aac ccc cct gac ttc act gtc gtg att ggc gac tac aag gcc gtt     1008
   Thr Asn Pro Pro Asp Phe Thr Val Val Ile Gly Asp Tyr Lys Ala Val
                   325                 330                 335
   gtt ccg ggc aag tac atc aac tac gct ccc atc tcg act ggc agc tcc        1056
   Val Pro Gly Lys Tyr Ile Asn Tyr Ala Pro Ile Ser Thr Gly Ser Ser
               340                 345                 350
   acc tgc ttt ggc ggt atc cag agc aac agc ggt ctg gga ctg tcc atc        1104
   Thr Cys Phe Gly Gly Ile Gln Ser Asn Ser Gly Leu Gly Leu Ser Ile
           355                 360                 365
   ctg ggt gat gtt ttc ttg aag agc cag tac gtg gtc ttc aac tct gag        1152
   Leu Gly Asp Val Phe Leu Lys Ser Gln Tyr Val Val Phe Asn Ser Glu
       370                 375                 380
   ggc cct aag ctg gga ttc gcc gct cag gct tag                            1185
   Gly Pro Lys Leu Gly Phe Ala Ala Gln Ala
   385                 390
<210> 28
   <211> 394
   <212> PRT
   <213> Aspergillus niger
<400> 28
   Met Val Val Phe Ser Lys Thr Ala Ala Leu Val Leu Gly Leu Ser Ser
   1               5                   10                  15
   Ala Val Ser Ala Ala Pro Ala Pro Thr Arg Lys Gly Phe Thr Ile Asn
               20                  25                  30
   Gln Ile Ala Arg Pro Ala Asn Lys Thr Arg Thr Ile Asn Leu Pro Gly
           35                  40                  45
   Met Tyr Ala Arg Ser Leu Ala Lys Phe Gly Gly Thr Val Pro Gln Ser
       50                  55                  60
   Val Lys Glu Ala Ala Ser Lys Gly Ser Ala Val Thr Thr Pro Gln Asn
   65                  70                  75                  80
   Asn Asp Glu Glu Tyr Leu Thr Pro Val Thr Val Gly Lys Ser Thr Leu
                   85                  90                  95
   His Leu Asp Phe Asp Thr Gly Ser Ala Asp Leu Trp Val Phe Ser Asp
               100                 105                 110
   Glu Leu Pro Ser Ser Glu Gln Thr Gly His Asp Leu Tyr Thr Pro Ser
           115                 120                 125
   Ser Ser Ala Thr Lys Leu Ser Gly Tyr Thr Trp Asp Ile Ser Tyr Gly
       130                 135                 140
   Asp Gly Ser Ser Ala Ser Gly Asp Val Tyr Arg Asp Thr Val Thr Val
   145                 150                 155                 160
   Gly Gly Val Thr Thr Asn Lys Gln Ala Val Glu Ala Ala Ser Lys Ile
   165 170 175
   Ser Ser Glu Phe Val Gln Asn Thr Ala Asn Asp Gly Leu Leu Gly Leu
   180 185 190
   Ala Phe Ser Ser Ile Asn Thr Val Gln Pro Lys Ala Gln Thr Thr Phe
   195 200 205
   Phe Asp Thr Val Lys Ser Gln Leu Asp Ser Pro Leu Phe Ala Val Gln
   210 215 220
   Leu Lys His Asp Ala Pro Gly Val Tyr Asp Phe Gly Tyr Ile Asp Asp
   225 230 235 240
   Ser Lys Tyr Thr Gly Ser Ile Thr Tyr Thr Asp Ala Asp Ser Ser Gln
   245 250 255
   Gly Tyr Trp Gly Phe Ser Thr Asp Gly Tyr Ser Ile Gly Asp Gly Ser
   260 265 270
   Ser Ser Ser Ser Gly Phe Ser Ala Ile Ala Asp Thr Gly Thr Thr Leu
   275 280 285
   Ile Leu Leu Asp Asp Glu Ile Val Ser Ala Tyr Tyr Glu Gln Val Ser
   290 295 300
   Gly Ala Gln Glu Ser Glu Glu Ala Gly Gly Tyr Val Phe Ser Cys Ser
   305 310 315 320
   Thr Asn Pro Pro Asp Phe Thr Val Val Ile Gly Asp Tyr Lys Ala Val
   325 330 335
   Val Pro Gly Lys Tyr Ile Asn Tyr Ala Pro Ile Ser Thr Gly Ser Ser
   340 345 350
   Thr Cys Phe Gly Gly Ile Gln Ser Asn Ser Gly Leu Gly Leu Ser Ile
   355 360 365
   Leu Gly Asp Val Phe Leu Lys Ser Gln Tyr Val Val Phe Asn Ser Glu
   370 375 380
   Gly Pro Lys Leu Gly Phe Ala Ala Gln Ala
   385 390
<210> 29
   <211> 2049
   <212> DNA
   <213> Aspergillus niger
<400> 29
   cacacctttc ctctcccctt tctcaatcta tctagtatag tactacggag tactaacctt 60
   gtactatact ctttccatcc caacctgcgc ccctccccta attgcttatc attcgcccac 120
   aaggcttgga cgaagcttgt tgtgcctgcg ccggtgccgc ttccctcccc tcctcccctg 180
   ccttttcggg cgacgccatc cgcgcactaa ccctccacgt attccaatat accaaatctg 240
   cccaaagcgc cagccagctt cctcaagcct tgcggtcaga taaggccctg tacctagcta 300
   gttgccgctg ctcccggcgc tgggccaagc cgtcggacgt ccgtcccccc tctttccccc 360
   tcctctcccc tctccactgg tggaacgatg tctggctgtt gccatcgttc tcagaagcaa 420
   cgccccctgg atcgggtggc tgtcgtacta ttgcatgttc gtccgcgcta ctaggaaagt 480
   ttttttccca cccggagtat ccgtgtttag tccgcgggct ggctgaccgg ctagctggcc 540
   gtgccagttg ggtaaggttc caagggagga ccttactagg tagaaacggg atccaacaat 600
   gaggggaaaa gggcggatat ggcttgccgg gggttcattg cggcctggac gaagaaaggg 660
   agatgatcac taatgcaaca caatcttggc ttgcaaggaa ttgcgctcca accagaatgt 720
   ctctgcgtag ggatgccaat tcgtgcgggc catgctggat ggatagtacg ctgctccact 780
   ctcgctcgac cttttgcagt ccacaatcgt ttccccgtat cgttgggcgg gggcgttttt 840
   ctgcagctat ggttgctgct gccccgacgg tgaacctttc tgcatccccg gttttagtcg 900
   attttagttg gcgggcctgg agattaaact ccgtcggacg aagaggagca gtggtgtcat 960
   cgtcggcgga ttgcatgcta tcggaagagc atggaagagg gaaaacatca acttcatttg 1020
   caaaacgctc gagcataaat agaggcctgg attccgccgt tctggtgtct tttcttcttc 1080
   atccagcatc gcaagtctct caagcatcgc ctggttcgtt cttctcactc ttccaccacc 1140
   agccttgtca ataagttagc tcttcatctt ttcgaagaaa ccaattctcc aaacgtcaaa 1200
   atgaagttct ctaccatcct taccggctcc ctcttcgcca ctgccgctct ggctgctcct 1260
   ctcactgaga agcgccgtgc tcgcaaggag gcccgcgccg ctggcaagcg ccacagcaac 1320
   cctccctaca tccctggttc cgacaaggag atcctcaagc tgaacggcac ctccaacgag 1380
   gattacagct ccaactgggc tggtgccgtc ctgatcggcg acggctacac caaggtcact 1440
   ggcgagttca ctgtccccag tgtctctgct ggatctagca gctccagtgg ctacggcggt 1500
   ggctacggct actacaagaa caagagacaa tccgaggagt actgcgcctc cgcttgggtt 1560
   ggtatcgacg gtgacacctg cgagaccgct attctccaga ctggtgtcga cttctgctac 1620
   gaggatggcc agacttccta cgatgcctgg tacgagtggt accccgacta cgcctacgac 1680
   ttcaacgaca tcaccatctc cgagggtgac accatcaagg tcactgtcga ggccaccagc 1740
   aagagcagcg gtagcgccac cgttgagaac ctgaccactg gccagtccgt cacccacacc 1800
   ttcagcggca acgtcgaggg tgacctttgc gagaccaacg ccgagtggat cgtcgaggac 1860
   ttcgagtctg gtgactctct tgtggctttc gctgacttcg gctccgttac cttcaccaat 1920
   gctgaggcta ccagcgacgg ttccactgtc ggcccctctg acgctaccgt tatggacatt 1980
   gagcaggatg gcaccgtcct caccgagacc tccgtctctg gcgacagcgt cactgtcacc 2040
   tacgtttaa 2049
<210> 30
   <211> 849
   <212> DNA
   <213> Aspergillus niger
<220>
   <221> CDS
   <222> (1) .. (849)
<400> 30
   atg aag ttc tct acc atc ctt acc ggc tcc ctc ttc gcc act gcc gct         48
   Met Lys Phe Ser Thr Ile Leu Thr Gly Ser Leu Phe Ala Thr Ala Ala
   1               5                   10                  15
   ctg gct gct cct ctc act gag aag cgc cgt gct cgc aag gag gcc cgc         96
   Leu Ala Ala Pro Leu Thr Glu Lys Arg Arg Ala Arg Lys Glu Ala Arg
               20                  25                  30
   gcc gct ggc aag cgc cac agc aac cct ccc tac atc cct ggt tcc gac        144
   Ala Ala Gly Lys Arg His Ser Asn Pro Pro Tyr Ile Pro Gly Ser Asp
           35                  40                  45
   aag gag atc ctc aag ctg aac ggc acc tcc aac gag gat tac agc tcc        192
   Lys Glu Ile Leu Lys Leu Asn Gly Thr Ser Asn Glu Asp Tyr Ser Ser
       50                  55                  60
   aac tgg gct ggt gcc gtc ctg atc ggc gac ggc tac acc aag gtc act        240
   Asn Trp Ala Gly Ala Val Leu Ile Gly Asp Gly Tyr Thr Lys Val Thr
   65                  70                  75                  80
   ggc gag ttc act gtc ccc agt gtc tct gct gga tct agc agc tcc agt        288
   Gly Glu Phe Thr Val Pro Ser Val Ser Ala Gly Ser Ser Ser Ser Ser
                   85                  90                  95
   ggc tac ggc ggt ggc tac ggc tac tac aag aac aag aga caa tcc gag        336
   Gly Tyr Gly Gly Gly Tyr Gly Tyr Tyr Lys Asn Lys Arg Gln Ser Glu
               100                 105                 110
   gag tac tgc gcc tcc gct tgg gtt ggt atc gac ggt gac acc tgc gag        384
   Glu Tyr Cys Ala Ser Ala Trp Val Gly Ile Asp Gly Asp Thr Cys Glu
           115                 120                 125
   acc gct att ctc cag act ggt gtc gac ttc tgc tac gag gat ggc cag        432
   Thr Ala Ile Leu Gln Thr Gly Val Asp Phe Cys Tyr Glu Asp Gly Gln
       130                 135                 140
   act tcc tac gat gcc tgg tac gag tgg tac ccc gac tac gcc tac gac        480
   Thr Ser Tyr Asp Ala Trp Tyr Glu Trp Tyr Pro Asp Tyr Ala Tyr Asp
   145                 150                 155                 160
   ttc aac gac atc acc atc tcc gag ggt gac acc atc aag gtc act gtc        528
   Phe Asn Asp Ile Thr Ile Ser Glu Gly Asp Thr Ile Lys Val Thr Val
                   165                 170                 175
   gag gcc acc agc aag agc agc ggt agc gcc acc gtt gag aac ctg acc        576
   Glu Ala Thr Ser Lys Ser Ser Gly Ser Ala Thr Val Glu Asn Leu Thr
               180                 185                 190
   act ggc cag tcc gtc acc cac acc ttc agc ggc aac gtc gag ggt gac          624
   Thr Gly Gln Ser Val Thr His Thr Phe Ser Gly Asn Val Glu Gly Asp
           195                 200                 205
   ctt tgc gag acc aac gcc gag tgg atc gtc gag gac ttc gag tct ggt          672
   Leu Cys Glu Thr Asn Ala Glu Trp Ile Val Glu Asp Phe Glu Ser Gly
       210                 215                 220
   gac tct ctt gtg gct ttc gct gac ttc ggc tcc gtt acc ttc acc aat          720
   Asp Ser Leu Val Ala Phe Ala Asp Phe Gly Ser Val Thr Phe Thr Asn
   225                 230                 235                 240
   gct gag gct acc agc gac ggt tcc act gtc ggc ccc tct gac gct acc          768
   Ala Glu Ala Thr Ser Asp Gly Ser Thr Val Gly Pro Ser Asp Ala Thr
                   245                 250                 255
   gtt atg gac att gag cag gat ggc acc gtc ctc acc gag acc tcc gtc          816
   Val Met Asp Ile Glu Gln Asp Gly Thr Val Leu Thr Glu Thr Ser Val
               260                 265                 270
   tct ggc gac agc gtc act gtc acc tac gtt taa                              849
   Ser Gly Asp Ser Val Thr Val Thr Tyr Val
           275                 280
<210> 31
   <211> 282
   <212> PRT
   <213> Aspergillus niger
<400> 31
   Met Lys Phe Ser Thr Ile Leu Thr Gly Ser Leu Phe Ala Thr Ala Ala
   1 5 10 15
   Leu Ala Ala Pro Leu Thr Glu Lys Arg Arg Ala Arg Lys Glu Ala Arg
   20 25 30
   Ala Ala Gly Lys Arg His Ser Asn Pro Pro Tyr Ile Pro Gly Ser Asp
   35 40 45
   Lys Glu Ile Leu Lys Leu Asn Gly Thr Ser Asn Glu Asp Tyr Ser Ser
   50 55 60
   Asn Trp Ala Gly Ala Val Leu Ile Gly Asp Gly Tyr Thr Lys Val Thr
   65 70 75 80
   Gly Glu Phe Thr Val Pro Ser Val Ser Ala Gly Ser Ser Ser Ser Ser
   85 90 95
   Gly Tyr Gly Gly Gly Tyr Gly Tyr Tyr Lys Asn Lys Arg Gln Ser Glu
   100 105 110
   Glu Tyr Cys Ala Ser Ala Trp Val Gly Ile Asp Gly Asp Thr Cys Glu
   115 120 125
   Thr Ala Ile Leu Gln Thr Gly Val Asp Phe Cys Tyr Glu Asp Gly Gln
   130 135 140
   Thr Ser Tyr Asp Ala Trp Tyr Glu Trp Tyr Pro Asp Tyr Ala Tyr Asp
   145 150 155 160
   Phe Asn Asp Ile Thr Ile Ser Glu Gly Asp Thr Ile Lys Val Thr Val
   165 170 175
   Glu Ala Thr Ser Lys Ser Ser Gly Ser Ala Thr Val Glu Asn Leu Thr
   180 185 190
   Thr Gly Gln Ser Val Thr His Thr Phe Ser Gly Asn Val Glu Gly Asp
   195 200 205
   Leu Cys Glu Thr Asn Ala Glu Trp Ile Val Glu Asp Phe Glu Ser Gly
   210 215 220
   Asp Ser Leu Val Ala Phe Ala Asp Phe Gly Ser Val Thr Phe Thr Asn
   225 230 235 240
   Ala Glu Ala Thr Ser Asp Gly Ser Thr Val Gly Pro Ser Asp Ala Thr
   245 250 255
   Val Met Asp Ile Glu Gln Asp Gly Thr Val Leu Thr Glu Thr Ser Val
   260 265 270
   Ser Gly Asp Ser Val Thr Val Thr Tyr Val
   275 280
<210> 32
   <211> 3201
   <212> DNA
   <213> Aspergillus niger
<400> 32
   atgtgatcta tctagctgac cgattattct tccgtagaaa caccgcatgc accggaagct      60
   acgacacggt gggataccgg agcgactgct atcagaatag ccggttcttt cagaactttg     120
   gtacgataag tggaagcatt tattgctatc cttcttaggg gacgatgggc ggtcgagact     180
   tgtctgaaga gtctgtggat ggactgtacg caatactatt atgactgtgg gggcatgctt     240
   atctaggtag cgaatgaaat tgacttgcta ttgctgagca gtataaatgc gacataaatt     300
   agtgatgaga caagacagat ttggggaaat tggcagctac tcttccagcg tgattaaatt     360
   tcccccaatt cgggcaaggc agctgctact gctattcgat tattctgagc atgtagccta     420
   gtatcggcaa ttcccgacaa tagattgggc accccacgtt gagcgaaggc catcaacagc     480
   ggtaagctga agcgattgtt ggggaaaggg acgcagccga gagaagccgc gagaattctc     540
   cgctggtaac ctgagttcag acccacggcg ccagaaagcg actattggat gcgacggtac     600
   agtagtgggt tttagtttgg atgtatctgg agacatcgat gcttgttcgc cggcgctaat 660
   ttggggccat cgcttaggga agaaaaagaa aggaaggatt tcatctgccg cggcattttt 720
   ggtcaggcat tggcaatact ccaagcagct tgccaaagtg agaaatgtgc ttatgaataa 780
   tattgagtaa tagttaatca attgtcttac aggataatat gggaatagca ttggcattgc 840
   gtggttggca ggcagcatgt tgcaaattgc aacaacgcga tcccggcgat cgccgtcgga 900
   ctccgggtgg gaatcagggt tggagaccca ttgcttatca acaacagccg acgccagtcg 960
   ttctggggtg catttgcaga atctttggac gaatcagcct tttgtcagta tcggccggtc 1020
   cgaattgacc cagcttgcta gccgttggag acatgcaatg atgccattgt ctcgtccgat 1080
   aagcaagtgc ggccaatccc tcgttccttc tggctgtctc agaaaagtta taatgtccgt 1140
   ctcgcctgcc tagcgaacaa ttcgaactct gtgtgtgttg tccctctcaa ggtctccagc 1200
   atgcgttctt ccggtctcta cacagcactc ctgtgctccc tggccgcctc gaccaacgcg 1260
   attgtccatg aaaagctcgc cgcggtcccc tccggctggc atcatgtcga agatgctggc 1320
   tccgaccacc agataagctt gtcgatcgcg ctggcacgca agaacctcga tcagcttgaa 1380
   tccaagctga aagacttgtc aacacctggc gaatcgcaat acggccagtg gctggaccag 1440
   gaggatgtcg acacgctgtt cccggtggcc agcgacaagg ctgtgattaa ctggctgcgc 1500
   agcgccaaca tcacccatat ttcccgccag ggcagcttgg tgaactttgc gaccacggtc 1560
   gataaggtga acaagcttct caacgccacc tttgcctact accaaagcgg ctcttcccag 1620
   agattgcgca caacagagta ctccatcccg gatgatctgg tcgactcaat cgacctcatc 1680
   tccccaacga ccttcttcgg caaggaaaag accactgctg gtctgaacca gcgggcgcaa 1740
   aagattgaca cccatgtggc caaacgctcc aacagctcgt cctgtgccga tgtcatcacg 1800
   ctgtcctgcc tgaaggagat gtacaatttt ggcaactaca ctcccagcgc ctcgtcgggc 1860
   agcaagctgg gcttcggcag cttcctgaac gaatccgcct cgtattctga ccttgccaag 1920
   ttcgagaagc tgtttaacct gccctcccag agcttttccg tggagttggt caacggcggt 1980
   gtcaatgatc agaatcaatc gacggcttcc ttgaccgagg cggacctcga tgtggaattg 2040
   ctcgtcggag ttgctcatcc cctcccggtg actgagttca tcacttctgg cgaaccgtga 2100
   gtattgaatt cctagacaga gcttgaatcg aagctaattg tgtagtcctt tcattcccga 2160
   ccccgatgag ccgagtgccg ccgacaacga gaacgagcct tacctccagt actatgagta 2220
   ccttctctcc aagcccaact cggctctgcc ccaagtgatt tccaactcct atggtgacga 2280
   cgaacaggta cgaccccaca ttcccccttc cctgtatgta gatactaacc ggaccagacc 2340
   gttccagagt actacgccaa gcgagtctgc aacctgatcg gacttgttgg cctgcgcggc 2400
   atcagtgtcc tcgagtcgtc cggtgacgaa ggtacactta cttcccctgt cctattcctt 2460
   gcatcgaaaa taccctaaca gcagcacagg tatcggatct ggctgccgaa ccaccgacgg 2520
   caccaaccga acccaattca accccatctt cccggccacc tgtccctacg tgactgccgt 2580
   gggaggaaca atgtcctatg cccccgaaat cgcctgggaa gccagttccg gcggattcag 2640
   caactacttc gagcgggcgt ggttccagaa ggaagctgtg cagaactacc tggcgcacca 2700
   catcaccaac gagaccaagc agtactactc gcaattcgcc aactttagcg gtcgcggatt 2760
   tcctgacgtt gctgcccata gctttgagcc ttcgtgagtc cattcccagc atcatgataa 2820
   tacgaaatag ggtctaatga catctgcaga tatgaggtta tcttctacgg cgcccgctac 2880
   ggctccggcg gtacctcagc cgcgtgtccc cttttctctg cgctagtggg catgctgaac 2940
   gatgctcgtc tgcgggcggg caagtccacg ctgggtttct tgaaccccct gctctatagc 3000
   aaggggtaca gagcgttgac tgatgtgacg gggggccagt cgatcggatg caatggcatt 3060
   gatccgcaga atgatgagac tgttgccggc gcgggcatta tcccgtgggc gcactggaat 3120
   gccacggtcg gatgggatcc ggtgactgga ttgggacttc ctgactttga gaagttgagg 3180
   cagttggtgc tgtcgttgta g 3201
<210> 33
   <211> 1788
   <212> DNA
   <213> Aspergillus niger
<220>
   <221> CDS
   <222> (1)..(1788)
<400> 33
   atg cgt tct tcc ggt ctc tac aca gca ctc ctg tgc tcc ctg gcc gcc      48
   Met Arg Ser Ser Gly Leu Tyr Thr Ala Leu Leu Cys Ser Leu Ala Ala
   1               5                   10                  15
   tcg acc aac gcg att gtc cat gaa aag ctc gcc gcg gtc ccc tcc ggc      96
   Ser Thr Asn Ala Ile Val His Glu Lys Leu Ala Ala Val Pro Ser Gly
               20                  25                  30
   tgg cat cat gtc gaa gat gct ggc tcc gac cac cag ata agc ttg tcg     144
   Trp His His Val Glu Asp Ala Gly Ser Asp His Gln Ile Ser Leu Ser
           35                  40                  45
   atc gcg ctg gca cgc aag aac ctc gat cag ctt gaa tcc aag ctg aaa     192
   Ile Ala Leu Ala Arg Lys Asn Leu Asp Gln Leu Glu Ser Lys Leu Lys
       50                  55                  60
   gac ttg tca aca cct ggc gaa tcg caa tac ggc cag tgg ctg gac cag     240
   Asp Leu Ser Thr Pro Gly Glu Ser Gln Tyr Gly Gln Trp Leu Asp Gln
   65                  70                  75                  80
   gag gat gtc gac acg ctg ttc ccg gtg gcc agc gac aag gct gtg att     288
   Glu Asp Val Asp Thr Leu Phe Pro Val Ala Ser Asp Lys Ala Val Ile
                   85                  90                  95
   aac tgg ctg cgc agc gcc aac atc acc cat att tcc cgc cag ggc agc     336
   Asn Trp Leu Arg Ser Ala Asn Ile Thr His Ile Ser Arg Gln Gly Ser
               100                 105                 110
   ttg gtg aac ttt gcg acc acg gtc gat aag gtg aac aag ctt ctc aac     384
   Leu Val Asn Phe Ala Thr Thr Val Asp Lys Val Asn Lys Leu Leu Asn
           115                 120                 125
   gcc acc ttt gcc tac tac caa agc ggc tct tcc cag aga ttg cgc aca        432
   Ala Thr Phe Ala Tyr Tyr Gln Ser Gly Ser Ser Gln Arg Leu Arg Thr
       130                 135                 140
   aca gag tac tcc atc ccg gat gat ctg gtc gac tca atc gac ctc atc        480
   Thr Glu Tyr Ser Ile Pro Asp Asp Leu Val Asp Ser Ile Asp Leu Ile
   145                 150                 155                 160
   tcc cca acg acc ttc ttc ggc aag gaa aag acc act gct ggt ctg aac        528
   Ser Pro Thr Thr Phe Phe Gly Lys Glu Lys Thr Thr Ala Gly Leu Asn
                   165                 170                 175
   cag cgg gcg caa aag att gac acc cat gtg gcc aaa cgc tcc aac agc        576
   Gln Arg Ala Gln Lys Ile Asp Thr His Val Ala Lys Arg Ser Asn Ser
               180                 185                 190
   tcg tcc tgt gcc gat gtc atc acg ctg tcc tgc ctg aag gag atg tac        624
   Ser Ser Cys Ala Asp Val Ile Thr Leu Ser Cys Leu Lys Glu Met Tyr
           195                 200                 205
   aat ttt ggc aac tac act ccc agc gcc tcg tcg ggc agc aag ctg ggc        672
   Asn Phe Gly Asn Tyr Thr Pro Ser Ala Ser Ser Gly Ser Lys Leu Gly
       210                 215                 220
   ttc ggc agc ttc ctg aac gaa tcc gcc tcg tat tct gac ctt gcc aag        720
   Phe Gly Ser Phe Leu Asn Glu Ser Ala Ser Tyr Ser Asp Leu Ala Lys
   225                 230                 235                 240
   ttc gag aag ctg ttt aac ctg ccc tcc cag agc ttt tcc gtg gag ttg        768
   Phe Glu Lys Leu Phe Asn Leu Pro Ser Gln Ser Phe Ser Val Glu Leu
                   245                 250                 255
   gtc aac ggc ggt gtc aat gat cag aat caa tcg acg gct tcc ttg acc        816
   Val Asn Gly Gly Val Asn Asp Gln Asn Gln Ser Thr Ala Ser Leu Thr
               260                 265                 270
   gag gcg gac ctc gat gtg gaa ttg ctc gtc gga gtt gct cat ccc ctc        864
   Glu Ala Asp Leu Asp Val Glu Leu Leu Val Gly Val Ala His Pro Leu
           275                 280                 285
   ccg gtg act gag ttc atc act tct ggc gaa cct cct ttc att ccc gac        912
   Pro Val Thr Glu Phe Ile Thr Ser Gly Glu Pro Pro Phe Ile Pro Asp
       290                 295                 300
   ccc gat gag ccg agt gcc gcc gac aac gag aac gag cct tac ctc cag        960
   Pro Asp Glu Pro Ser Ala Ala Asp Asn Glu Asn Glu Pro Tyr Leu Gln
   305                 310                 315                 320
   tac tat gag tac ctt ctc tcc aag ccc aac tcg gct ctg ccc caa gtg       1008
   Tyr Tyr Glu Tyr Leu Leu Ser Lys Pro Asn Ser Ala Leu Pro Gln Val
                   325                 330                 335
   att tcc aac tcc tat ggt gac gac gaa cag acc gtt cca gag tac tac       1056
   Ile Ser Asn Ser Tyr Gly Asp Asp Glu Gln Thr Val Pro Glu Tyr Tyr
               340                 345                 350
   gcc aag cga gtc tgc aac ctg atc gga ctt gtt ggc ctg cgc ggc atc       1104
   Ala Lys Arg Val Cys Asn Leu Ile Gly Leu Val Gly Leu Arg Gly Ile
           355                 360                 365
   agt gtc ctc gag tcg tcc ggt gac gaa ggt atc gga tct ggc tgc cga       1152
   Ser Val Leu Glu Ser Ser Gly Asp Glu Gly Ile Gly Ser Gly Cys Arg
       370                 375                 380
   acc acc gac ggc acc aac cga acc caa ttc aac ccc atc ttc ccg gcc 1200
   Thr Thr Asp Gly Thr Asn Arg Thr Gln Phe Asn Pro Ile Phe Pro Ala
   385 390 395 400
   acc tgt ccc tac gtg act gcc gtg gga gga aca atg tcc tat gcc ccc 1248
   Thr Cys Pro Tyr Val Thr Ala Val Gly Gly Thr Met Ser Tyr Ala Pro
   405 410 415
   gaa atc gcc tgg gaa gcc agt tcc ggc gga ttc agc aac tac ttc gag 1296
   Glu Ile Ala Trp Glu Ala Ser Ser Gly Gly Phe Ser Asn Tyr Phe Glu
   420 425 430
   cgg gcg tgg ttc cag aag gaa gct gtg cag aac tac ctg gcg cac cac 1344
   Arg Ala Trp Phe Gln Lys Glu Ala Val Gln Asn Tyr Leu Ala His His
   435 440 445
   atc acc aac gag acc aag cag tac tac tcg caa ttc gcc aac ttt agc 1392
   Ile Thr Asn Glu Thr Lys Gln Tyr Tyr Ser Gln Phe Ala Asn Phe Ser
   450 455 460
   ggt cgc gga ttt cct gac gtt gct gcc cat agc ttt gag cct tca tat 1440
   Gly Arg Gly Phe Pro Asp Val Ala Ala His Ser Phe Glu Pro Ser Tyr
   465 470 475 480
   gag gtt atc ttc tac ggc gcc cgc tac ggc tcc ggc ggt acc tca gcc 1488
   Glu Val Ile Phe Tyr Gly Ala Arg Tyr Gly Ser Gly Gly Thr Ser Ala
   485 490 495
   gcg tgt ccc ctt ttc tct gcg cta gtg ggc atg ctg aac gat gct cgt 1536
   Ala Cys Pro Leu Phe Ser Ala Leu Val Gly Met Leu Asn Asp Ala Arg
   500 505 510
   ctg cgg gcg ggc aag tcc acg ctg ggt ttc ttg aac ccc ctg ctc tat 1584
   Leu Arg Ala Gly Lys Ser Thr Leu Gly Phe Leu Asn Pro Leu Leu Tyr
   515 520 525
   agc aag ggg tac aga gcg ttg act gat gtg acg ggg ggc cag tcg atc 1632
   Ser Lys Gly Tyr Arg Ala Leu Thr Asp Val Thr Gly Gly Gln Ser Ile
   530 535 540
   gga tgc aat ggc att gat ccg cag aat gat gag act gtt gcc ggc gcg 1680
   Gly Cys Asn Gly Ile Asp Pro Gln Asn Asp Glu Thr Val Ala Gly Ala
   545 550 555 560
   ggc att atc ccg tgg gcg cac tgg aat gcc acg gtc gga tgg gat ccg 1728
   Gly Ile Ile Pro Trp Ala His Trp Asn Ala Thr Val Gly Trp Asp Pro
   565 570 575
   gtg act gga ttg gga ctt cct gac ttt gag aag ttg agg cag ttg gtg 1776
   Val Thr Gly Leu Gly Leu Pro Asp Phe Glu Lys Leu Arg Gln Leu Val
   580 585 590
   ctg tcg ttg tag 1788
   Leu Ser Leu
   595
<210> 34
   <211> 595
   <212> PRT
   <213> Aspergillus niger
<400> 34
   Met Arg Ser Ser Gly Leu Tyr Thr Ala Leu Leu Cys Ser Leu Ala Ala
   1               5                   10                  15
   Ser Thr Asn Ala Ile Val His Glu Lys Leu Ala Ala Val Pro Ser Gly
               20                  25                  30
   Trp His His Val Glu Asp Ala Gly Ser Asp His Gln Ile Ser Leu Ser
           35                  40                  45
   Ile Ala Leu Ala Arg Lys Asn Leu Asp Gln Leu Glu Ser Lys Leu Lys
       50                  55                  60
   Asp Leu Ser Thr Pro Gly Glu Ser Gln Tyr Gly Gln Trp Leu Asp Gln
   65                  70                  75                  80
   Glu Asp Val Asp Thr Leu Phe Pro Val Ala Ser Asp Lys Ala Val Ile
                   85                  90                  95
   Asn Trp Leu Arg Ser Ala Asn Ile Thr His Ile Ser Arg Gln Gly Ser
               100                 105                 110
   Leu Val Asn Phe Ala Thr Thr Val Asp Lys Val Asn Lys Leu Leu Asn
           115                 120                 125
   Ala Thr Phe Ala Tyr Tyr Gln Ser Gly Ser Ser Gln Arg Leu Arg Thr
       130                 135                 140
   Thr Glu Tyr Ser Ile Pro Asp Asp Leu Val Asp Ser Ile Asp Leu Ile
   145                 150                 155                 160
   Ser Pro Thr Thr Phe Phe Gly Lys Glu Lys Thr Thr Ala Gly Leu Asn
                   165                 170                 175
   Gln Arg Ala Gln Lys Ile Asp Thr His Val Ala Lys Arg Ser Asn Ser
               180                 185                 190
   Ser Ser Cys Ala Asp Val Ile Thr Leu Ser Cys Leu Lys Glu Met Tyr
           195                 200                 205
   Asn Phe Gly Asn Tyr Thr Pro Ser Ala Ser Ser Gly Ser Lys Leu Gly
       210                 215                 220
   Phe Gly Ser Phe Leu Asn Glu Ser Ala Ser Tyr Ser Asp Leu Ala Lys
   225                 230                 235                 240
   Phe Glu Lys Leu Phe Asn Leu Pro Ser Gln Ser Phe Ser Val Glu Leu
                   245                 250                 255
   Val Asn Gly Gly Val Asn Asp Gln Asn Gln Ser Thr Ala Ser Leu Thr
   260 265 270
   Glu Ala Asp Leu Asp Val Glu Leu Leu Val Gly Val Ala His Pro Leu
   275 280 285
   Pro Val Thr Glu Phe Ile Thr Ser Gly Glu Pro Pro Phe Ile Pro Asp
   290 295 300
   Pro Asp Glu Pro Ser Ala Ala Asp Asn Glu Asn Glu Pro Tyr Leu Gln
   305 310 315 320
   Tyr Tyr Glu Tyr Leu Leu Ser Lys Pro Asn Ser Ala Leu Pro Gln Val
   325 330 335
   Ile Ser Asn Ser Tyr Gly Asp Asp Glu Gln Thr Val Pro Glu Tyr Tyr
   340 345 350
   Ala Lys Arg Val Cys Asn Leu Ile Gly Leu Val Gly Leu Arg Gly Ile
   355 360 365
   Ser Val Leu Glu Ser Ser Gly Asp Glu Gly Ile Gly Ser Gly Cys Arg
   370 375 380
   Thr Thr Asp Gly Thr Asn Arg Thr Gln Phe Asn Pro Ile Phe Pro Ala
   385 390 395 400
   Thr Cys Pro Tyr Val Thr Ala Val Gly Gly Thr Met Ser Tyr Ala Pro
   405 410 415
   Glu Ile Ala Trp Glu Ala Ser Ser Gly Gly Phe Ser Asn Tyr Phe Glu
   420 425 430
   Arg Ala Trp Phe Gln Lys Glu Ala Val Gln Asn Tyr Leu Ala His His
   435 440 445
   Ile Thr Asn Glu Thr Lys Gln Tyr Tyr Ser Gln Phe Ala Asn Phe Ser
   450 455 460
   Gly Arg Gly Phe Pro Asp Val Ala Ala His Ser Phe Glu Pro Ser Tyr
   465 470 475 480
   Glu Val Ile Phe Tyr Gly Ala Arg Tyr Gly Ser Gly Gly Thr Ser Ala
   485 490 495
   Ala Cys Pro Leu Phe Ser Ala Leu Val Gly Met Leu Asn Asp Ala Arg
   500 505 510
   Leu Arg Ala Gly Lys Ser Thr Leu Gly Phe Leu Asn Pro Leu Leu Tyr
   515 520 525
   Ser Lys Gly Tyr Arg Ala Leu Thr Asp Val Thr Gly Gly Gln Ser Ile
   530 535 540
   Gly Cys Asn Gly Ile Asp Pro Gln Asn Asp Glu Thr Val Ala Gly Ala
   545 550 555 560
   Gly Ile Ile Pro Trp Ala His Trp Asn Ala Thr Val Gly Trp Asp Pro
   565 570 575
   Val Thr Gly Leu Gly Leu Pro Asp Phe Glu Lys Leu Arg Gln Leu Val
   580 585 590
   Leu Ser Leu
   595
<210> 35
   <211> 3027
   <212> DNA
   <213> Aspergillus niger
<400> 35
   gcagacatca atttcacaag cattacgaac atcccctgga aatatcaaat gtctctcggt 60
   taattactgc atgcaggcct ctctatcaca tctctagagg aatggttctg cgtcacagtg 120
   ctccgtgact gccccggatc agtacaagtc ctgcctccca agatgtttca gacagctaca 180
   atctacagcg acatcagaga atacagccgg tggttaccaa cttctacaga atctgcatag 240
   aagtattttt gccttactag ctaagcttat gggtgtgctc ttacagtatg cgctcgaagt 300
   tgcctggatc caacctgttc tgactatttg tcggagaatt gttcatatag tgcgttggag 360
   accattgttg ttccccattc ttcgactgcc tagctcgtgt gctatggagt gctgaagctt 420
   atagttgaga caaggttcca catacattag gttctcggcc ggtgattggt acgctcatgg 480
   gccacgggtt gtctcggtgg tccgcggatg cagcccaacc ctaagggacc gacaacgaag 540
   aagggacggg atgatcttga acttttcagt atcggcagga tgccattgca aagccgacgc 600
   gaagcgtggt tgtggcgtca taacaatatg gaagtggagg gagcctgcgt cggttacatg 660
   gtatccctgc atgttcccct gagtggcttc agcacatcat catttgccat gataatggcc 720
   agtcaattcg acgtcgtcct gagcaagcag ctaccggtaa tctgagacct aatcctggta 780
   agtggatcga gttcatttac ctcatcatct aacctcgctt catgattcgc aggcgctttc 840
   cgcgactagt cgatgccagt atccatttgc ctgcagatcc gatgcttcca ccgaccaccg 900
   ttacttctct ccagaccccc atccggcttt ccgacggcct cctctgcatc ccctcacaaa 960
   gcaagcggag acctgcctga atagggagag ttttttcaat gagcccacac gcagttacct 1020
   cgcttccgca tgagatgggt ccattcttga caatgctgcc taatgcgcag aagggccgtc 1080
   atgcgcctgg agaactacat aaatagggac cacgcatgcc ccgacgatgt atgaattgtt 1140
   caaacttctc cgcccaagtg agttctcttt tcattccatt ccgaagggag aatcaccagt 1200
   atgcgggtta ccacggcaat tgcttcatta ctactggtcg gctcggccac cagtctccaa 1260
   aatcctcatc gtcgggctgt tccgccccct ctctcgcatc gcagcgtagc gtctcgctcc 1320
   gtgcccgttg agcgccgaac caccgacttt gagtatttga ctaacaagac tgcaagtgcg 1380
   tgattccgtt ttttaactac cgcatttatc gttctaagat caattgcagg attcctggtc 1440
   aatggcacaa gcatccccga agtcgatttc gacgtcggcg agtcctacgc cggccttctc 1500
   cccaatacgc ccactggcaa ttctagccta ttcttctggt tcttcccctc gcaaaatcca 1560
   gaggccagcg atgaggttag tggtcgctct gttttttccg gtcatgcgtc agccagctaa 1620
   caattaacaa agatcaccat ctggctcaac ggcggccccg gatgtagctc cctagacggc 1680
   ctgcttcaag agaacggccc attcctctgg cagcctggca cttacaagcc cgttcctaat 1740
   ccatactcat ggaccaacct caccaatgtg gtttacatcg accaacccgc cggcacaggc 1800
   ttctccccgg gcccctcgac cgtaaataac gaggaagacg tggctgccca gttcaacagc 1860
   tggttcaagc acttcgtcga caccttcgac ctgcacggcc gcaaggtcta catcaccggt 1920
   gaaagctacg cgggcatgta cgtcccctac attgccgatg ccatgctgaa cgaggaggat 1980
   acaacctact tcaacttgaa gggtatccag atcaacgacc cgtccatcaa cagcgactcg 2040
   gtcatgatgt actgtatgtt tcccttcata tacctccacc tccaccacca ccaccactaa 2100
   caacatcacc caccagcccc cgccgtccgc catctgaacc actacaacaa catcttccag 2160
   ctaaactcca ctttcctctc ctacatcaac gccaaagccg acaagtgcgg ctacaacgcc 2220
   ttcctcgaca aagccatcac ctacccaccc cccagtccct tccccaccgc ccctgaaatc 2280
   accgaagact gccaagtctg ggacgaagtc gtcatggccg cctacgacat caacccctgc 2340
   ttcaattact accacctgat cgacttctgc ccctacctct gggacgtgct cggcttcccc 2400
   tccctcgcct ccggcccaaa caactacttc aaccgctccg acgtccagaa gatcctgcac 2460
   gtccctccaa cggactactc cgtgtgctcg gagaccgtca tcttcgcgaa cggcgacggc 2520
   agcgacccca gctcctgggg tcccctaccc agcgtcatcg aacgcactaa caacactatc 2580
   atcggccacg gctggctcga ttacctcctc ttcttgaacg gctcgctcgc cacaatccag 2640
   aacatgacct ggaacggtaa gcaagggttc cagcgtcctc ccgtggaacc gctcttcgtc 2700
   ccttaccatt atggtctggc tgagctgtac tggggcgatg agcctgaccc gtataacctt 2760
   gatgctggcg ctggatacct gggtacagcg cataccgagc gcgggttgac tttcagctcg 2820
   gtgtatttgt ctggtcatgg taagtttatt atatcccctt ggaagcggta tgatgaacgt 2880
   tagagagtgc tgactgttgc ttcctcctcg tgatagaaat cccgcagtat gttcctggtg 2940
   cggcttaccg ccagttggag ttcctgctcg gtaggattag tagtctttcg gcgaagggga 3000
   actatacctc ttgatttcag cggatga 3027
<210> 36
   <211> 1563
   <212> DNA
   <213> Aspergillus niger
<220>
   <221> CDS
   <222> (1)..(1563)
<400> 36
   atg cgg gtt acc acg gca att gct tca tta cta ctg gtc ggc tcg gcc        48
   Met Arg Val Thr Thr Ala Ile Ala Ser Leu Leu Leu Val Gly Ser Ala
   1               5                   10                  15
   acc agt ctc caa aat cct cat cgt cgg gct gtt ccg ccc cct ctc tcg        96
   Thr Ser Leu Gln Asn Pro His Arg Arg Ala Val Pro Pro Pro Leu Ser
               20                  25                  30
   cat cgc agc gta gcg tct cgc tcc gtg ccc gtt gag cgc cga acc acc       144
   His Arg Ser Val Ala Ser Arg Ser Val Pro Val Glu Arg Arg Thr Thr
           35                  40                  45
   gac ttt gag tat ttg act aac aag act gca aga ttc ctg gtc aat ggc       192
   Asp Phe Glu Tyr Leu Thr Asn Lys Thr Ala Arg Phe Leu Val Asn Gly
       50                  55                  60
   aca agc atc ccc gaa gtc gat ttc gac gtc ggc gag tcc tac gcc ggc       240
   Thr Ser Ile Pro Glu Val Asp Phe Asp Val Gly Glu Ser Tyr Ala Gly
   65                  70                  75                  80
   ctt ctc ccc aat acg ccc act ggc aat tct agc cta ttc ttc tgg ttc       288
   Leu Leu Pro Asn Thr Pro Thr Gly Asn Ser Ser Leu Phe Phe Trp Phe
                   85                  90                  95
   ttc ccc tcg caa aat cca gag gcc agc gat gag atc acc atc tgg ctc       336
   Phe Pro Ser Gln Asn Pro Glu Ala Ser Asp Glu Ile Thr Ile Trp Leu
               100                 105                 110
   aac ggc ggc ccc gga tgt agc tcc cta gac ggc ctg ctt caa gag aac       384
   Asn Gly Gly Pro Gly Cys Ser Ser Leu Asp Gly Leu Leu Gln Glu Asn
           115                 120                 125
   ggc cca ttc ctc tgg cag cct ggc act tac aag ccc gtt cct aat cca       432
   Gly Pro Phe Leu Trp Gln Pro Gly Thr Tyr Lys Pro Val Pro Asn Pro
       130                 135                 140
   tac tca tgg acc aac ctc acc aat gtg gtt tac atc gac caa ccc gcc       480
   Tyr Ser Trp Thr Asn Leu Thr Asn Val Val Tyr Ile Asp Gln Pro Ala
   145                 150                 155                 160
   ggc aca ggc ttc tcc ccg ggc ccc tcg acc gta aat aac gag gaa gac       528
   Gly Thr Gly Phe Ser Pro Gly Pro Ser Thr Val Asn Asn Glu Glu Asp
                   165                 170                 175
   gtg gct gcc cag ttc aac agc tgg ttc aag cac ttc gtc gac acc ttc       576
   Val Ala Ala Gln Phe Asn Ser Trp Phe Lys His Phe Val Asp Thr Phe
               180                 185                 190
   gac ctg cac ggc cgc aag gtc tac atc acc ggt gaa agc tac gcg ggc       624
   Asp Leu His Gly Arg Lys Val Tyr Ile Thr Gly Glu Ser Tyr Ala Gly
           195                 200                 205
   atg tac gtc ccc tac att gcc gat gcc atg ctg aac gag gag gat aca       672
   Met Tyr Val Pro Tyr Ile Ala Asp Ala Met Leu Asn Glu Glu Asp Thr
       210                 215                 220
   acc tac ttc aac ttg aag ggt atc cag atc aac gac ccg tcc atc aac      720
   Thr Tyr Phe Asn Leu Lys Gly Ile Gln Ile Asn Asp Pro Ser Ile Asn
   225                 230                 235                 240
   agc gac tcg gtc atg atg tac tcc ccc gcc gtc cgc cat ctg aac cac      768
   Ser Asp Ser Val Met Met Tyr Ser Pro Ala Val Arg His Leu Asn His
                   245                 250                 255
   tac aac aac atc ttc cag cta aac tcc act ttc ctc tcc tac atc aac      816
   Tyr Asn Asn Ile Phe Gln Leu Asn Ser Thr Phe Leu Ser Tyr Ile Asn
               260                 265                 270
   gcc aaa gcc gac aag tgc ggc tac aac gcc ttc ctc gac aaa gcc atc      864
   Ala Lys Ala Asp Lys Cys Gly Tyr Asn Ala Phe Leu Asp Lys Ala Ile
           275                 280                 285
   acc tac cca ccc ccc agt ccc ttc ccc acc gcc cct gaa atc acc gaa      912
   Thr Tyr Pro Pro Pro Ser Pro Phe Pro Thr Ala Pro Glu Ile Thr Glu
       290                 295                 300
   gac tgc caa gtc tgg gac gaa gtc gtc atg gcc gcc tac gac atc aac      960
   Asp Cys Gln Val Trp Asp Glu Val Val Met Ala Ala Tyr Asp Ile Asn
   305                 310                 315                 320
   ccc tgc ttc aat tac tac cac ctg atc gac ttc tgc ccc tac ctc tgg     1008
   Pro Cys Phe Asn Tyr Tyr His Leu Ile Asp Phe Cys Pro Tyr Leu Trp
                   325                 330                 335
   gac gtg ctc ggc ttc ccc tcc ctc gcc tcc ggc cca aac aac tac ttc     1056
   Asp Val Leu Gly Phe Pro Ser Leu Ala Ser Gly Pro Asn Asn Tyr Phe
               340                 345                 350
   aac cgc tcc gac gtc cag aag atc ctg cac gtc cct cca acg gac tac     1104
   Asn Arg Ser Asp Val Gln Lys Ile Leu His Val Pro Pro Thr Asp Tyr
           355                 360                 365
   tcc gtg tgc tcg gag acc gtc atc ttc gcg aac ggc gac ggc agc gac     1152
   Ser Val Cys Ser Glu Thr Val Ile Phe Ala Asn Gly Asp Gly Ser Asp
       370                 375                 380
   ccc agc tcc tgg ggt ccc cta ccc agc gtc atc gaa cgc act aac aac     1200
   Pro Ser Ser Trp Gly Pro Leu Pro Ser Val Ile Glu Arg Thr Asn Asn
   385                 390                 395                 400
   act atc atc ggc cac ggc tgg ctc gat tac ctc ctc ttc ttg aac ggc     1248
   Thr Ile Ile Gly His Gly Trp Leu Asp Tyr Leu Leu Phe Leu Asn Gly
                   405                 410                 415
   tcg ctc gcc aca atc cag aac atg acc tgg aac ggt aag caa ggg ttc     1296
   Ser Leu Ala Thr Ile Gln Asn Met Thr Trp Asn Gly Lys Gln Gly Phe
               420                 425                 430
   cag cgt cct ccc gtg gaa ccg ctc ttc gtc cct tac cat tat ggt ctg     1344
   Gln Arg Pro Pro Val Glu Pro Leu Phe Val Pro Tyr His Tyr Gly Leu
           435                 440                 445
   gct gag ctg tac tgg ggc gat gag cct gac ccg tat aac ctt gat gct     1392
   Ala Glu Leu Tyr Trp Gly Asp Glu Pro Asp Pro Tyr Asn Leu Asp Ala
       450                 455                 460
   ggc gct gga tac ctg ggt aca gcg cat acc gag cgc ggg ttg act ttc     1440
   Gly Ala Gly Tyr Leu Gly Thr Ala His Thr Glu Arg Gly Leu Thr Phe
   465                 470                 475                 480
   agc tcg gtg tat ttg tct ggt cat gaa atc ccg cag tat gtt cct ggt           1488
   Ser Ser Val Tyr Leu Ser Gly His Glu Ile Pro Gln Tyr Val Pro Gly
                   485                 490                 495
   gcg gct tac cgc cag ttg gag ttc ctg ctc ggt agg att agt agt ctt           1536
   Ala Ala Tyr Arg Gln Leu Glu Phe Leu Leu Gly Arg Ile Ser Ser Leu
               500                 505                 510
   tcg gcg aag ggg aac tat acc tct tga                                       1563
   Ser Ala Lys Gly Asn Tyr Thr Ser
           515                 520
<210> 37
   <211> 520
   <212> PRT
   <213> Aspergillus niger
<400> 37
   Met Arg Val Thr Thr Ala Ile Ala Ser Leu Leu Leu Val Gly Ser Ala
   1               5                   10                  15
   Thr Ser Leu Gln Asn Pro His Arg Arg Ala Val Pro Pro Pro Leu Ser
               20                  25                  30
   His Arg Ser Val Ala Ser Arg Ser Val Pro Val Glu Arg Arg Thr Thr
           35                  40                  45
   Asp Phe Glu Tyr Leu Thr Asn Lys Thr Ala Arg Phe Leu Val Asn Gly
       50                  55                  60
   Thr Ser Ile Pro Glu Val Asp Phe Asp Val Gly Glu Ser Tyr Ala Gly
   65                  70                  75                  80
   Leu Leu Pro Asn Thr Pro Thr Gly Asn Ser Ser Leu Phe Phe Trp Phe
                   85                  90                  95
   Phe Pro Ser Gln Asn Pro Glu Ala Ser Asp Glu Ile Thr Ile Trp Leu
               100                 105                 110
   Asn Gly Gly Pro Gly Cys Ser Ser Leu Asp Gly Leu Leu Gln Glu Asn
           115                 120                 125
   Gly Pro Phe Leu Trp Gln Pro Gly Thr Tyr Lys Pro Val Pro Asn Pro
       130                 135                 140
   Tyr Ser Trp Thr Asn Leu Thr Asn Val Val Tyr Ile Asp Gln Pro Ala
   145                 150                 155                 160
   Gly Thr Gly Phe Ser Pro Gly Pro Ser Thr Val Asn Asn Glu Glu Asp
                   165                 170                 175
   Val Ala Ala Gln Phe Asn Ser Trp Phe Lys His Phe Val Asp Thr Phe
   180 185 190
   Asp Leu His Gly Arg Lys Val Tyr Ile Thr Gly Glu Ser Tyr Ala Gly
   195 200 205
   Met Tyr Val Pro Tyr Ile Ala Asp Ala Met Leu Asn Glu Glu Asp Thr
   210 215 220
   Thr Tyr Phe Asn Leu Lys Gly Ile Gln Ile Asn Asp Pro Ser Ile Asn
   225 230 235 240
   Ser Asp Ser Val Met Met Tyr Ser Pro Ala Val Arg His Leu Asn His
   245 250 255
   Tyr Asn Asn Ile Phe Gln Leu Asn Ser Thr Phe Leu Ser Tyr Ile Asn
   260 265 270
   Ala Lys Ala Asp Lys Cys Gly Tyr Asn Ala Phe Leu Asp Lys Ala Ile
   275 280 285
   Thr Tyr Pro Pro Pro Ser Pro Phe Pro Thr Ala Pro Glu Ile Thr Glu
   290 295 300
   Asp Cys Gln Val Trp Asp Glu Val Val Met Ala Ala Tyr Asp Ile Asn
   305 310 315 320
   Pro Cys Phe Asn Tyr Tyr His Leu Ile Asp Phe Cys Pro Tyr Leu Trp
   325 330 335
   Asp Val Leu Gly Phe Pro Ser Leu Ala Ser Gly Pro Asn Asn Tyr Phe
   340 345 350
   Asn Arg Ser Asp Val Gln Lys Ile Leu His Val Pro Pro Thr Asp Tyr
   355 360 365
   Ser Val Cys Ser Glu Thr Val Ile Phe Ala Asn Gly Asp Gly Ser Asp
   370 375 380
   Pro Ser Ser Trp Gly Pro Leu Pro Ser Val Ile Glu Arg Thr Asn Asn
   385 390 395 400
   Thr Ile Ile Gly His Gly Trp Leu Asp Tyr Leu Leu Phe Leu Asn Gly
   405 410 415
   Ser Leu Ala Thr Ile Gln Asn Met Thr Trp Asn Gly Lys Gln Gly Phe
   420 425 430
   Gln Arg Pro Pro Val Glu Pro Leu Phe Val Pro Tyr His Tyr Gly Leu
   435 440 445
   Ala Glu Leu Tyr Trp Gly Asp Glu Pro Asp Pro Tyr Asn Leu Asp Ala
   450 455 460
   Gly Ala Gly Tyr Leu Gly Thr Ala His Thr Glu Arg Gly Leu Thr Phe
   465 470 475 480
   Ser Ser Val Tyr Leu Ser Gly His Glu Ile Pro Gln Tyr Val Pro Gly
   485 490 495
   Ala Ala Tyr Arg Gln Leu Glu Phe Leu Leu Gly Arg Ile Ser Ser Leu
   500 505 510
   Ser Ala Lys Gly Asn Tyr Thr Ser
   515 520
<210> 38
   <211> 3399
   <212> DNA
   <213> Aspergillus niger
<400> 38
   cctgccgccg atcggtggag tagacgagga ggagggcaag agcttggaag aggagatgac      60
   gattctcagc gagtctaagc ggcaggagct gacggtgcgg ttcaagaagg ctgcggatgg     120
   agtgtacgtg gaggccaagc gcagcgccat cggcggcatg acgcaggtgc ccctgtactt     180
   ttatggcatt cttctggcgc ttggctggaa cgagattgtg gctggtaagt ttcgcactat     240
   gaatttcggg ttctgggttg atcatggcta actgtgcaca gttcttcgta accctgccta     300
   cttcttcctc ttgtttgtgt gcctcgttgc agggtacgtg acgtaccagc tcaatctgtg     360
   gggaccgatc atgaagatga cggaggctgc atcgaaccag gcactcgtgg agggtaagaa     420
   gcgactgcgc gagtttctcg agtcttcgga tacgggacga caggcaattg cgatgtcgac     480
   ggccggcggt gctggccggg gggagcaggt ggagatgtcg cgcctcaaca agcagggaaa     540
   gacaacggcc gaggatgagg acggtgactt gtaagagagg cagaaggagt catttatcac     600
   ttgtattcca atgtattttc catttataga tactgcattc aaatgcaccg tttagcatag     660
   catcccacat tctatttcat tccaatctca tgccattgcc atccccggta ttaatttact     720
   tctccgcctt atcttgcaat cttgcaatct ctttctcctc gttatcacgc gttcctgcag     780
   gcgcacctcc gatggcactg cagccggagt ccccgcggcg ccggcactac taaagactaa     840
   agtgtctagt ctagcctcca atgtgctcac ctccatcagc atctcatcca tttatcttct     900
   gacgatgtca tctgcaggct ccaccccctc cggccgcccc gacgctctcc gacggtgcac     960
   aacaatcaat tctgcagtca cgctcaagat tcgtccctgc cggactcctc atgccgtgcc    1020
   tggtttaatc tatgcaatgg agtaaggtag tatcgcctag caggagcgga gttcctgctg    1080
   cgctcacgcc atggtgccgg cgcagacata aatcgctcgt ttcctccggc gctggccgtt 1140
   ctctcgagcc agtttgtctg ttgtggttgt aggatcctct gttcccctcg acagctcaca 1200
   atgcgttcct tctccgttgt cgctgccgcg tcactggcgc tctcttgggc gtctctggcc 1260
   caggctgctc gcccccgtct tgtgcccaag cctatctctc ggccagcttc gagtaagtcg 1320
   gctgcgacta cgggtgaggc ttattttgag cagctgctgg accatcacaa cccggagaag 1380
   ggaacgtttt cccagcggta ctggtggagt actgaatact ggggtggacc tgggtcaccg 1440
   gtgcgtctct gacatttggt cttatgaccg gccatattga aacttagccg gtggcaaggt 1500
   ccgcaatcat gaggaacatt gctgattaaa ctaggtggtc ctctttaacc ctggagaggt 1560
   ctctgccgat ggctatgagg ggtatctcac caacgatact ctcactggtg tctatgcgca 1620
   ggagatccag ggtgccgtca ttctcattga acgtgagtgt cactgctacc atggaaaaaa 1680
   gacattcgct gatcgacccc aatctagacc gctactgggg cgactcttcg ccttatgagg 1740
   tgctcaatgc cgaaacactt cagtatctca cactggatca gtccattctg gacatgacct 1800
   acttcgccga gacggtaaag ctgcagttcg ataatagcag ccgcagcaat gcgcagaatg 1860
   ctgtatgtta ccttcaccgc tctatgtttc tgataggtac tgacaacgta gccctgggtc 1920
   atggtcggtg gctcatacag cggtgccttg acggcttgga ccgagtctat cgcgcctgga 1980
   acgttctggg cttaccatgc caccagtgcg cctgtggagg ctatctatga ctttgtaggt 2040
   gtagcctgct cttgttatct atacttgcag ctaaccaagc cagtggcaat acttctaccc 2100
   cattcagcaa ggtatggcac agaactgcag caaggatgtg tctctggtag ccgagtatgt 2160
   cgacaaaatt gggaagaatg gaactgccaa ggaacagcag gagctcaaag aattgtttgg 2220
   tctgggagct gttgagcatt acgatgactt tgccgcgtga gtacttcaaa gtctatagac 2280
   gagcttttct gacaggaaca gtgtcctgcc caacggaccg tacctctggc aagacaacga 2340
   ctttgtcaca ggatactctt ccttcttcca gttctgtgat gctgtcgagg tgagttacca 2400
   ccagattcct cttgattgaa gcaatatact aacggacaca gggtgtcgaa gccggcgcgg 2460
   cagtgacccc cggccccgag ggcgtcggac ttgaaaaggc cctggccaac tacgcaaact 2520
   ggttcaattc aaccatactc cctaactgta tttcaccatc tcttgtctcg ttcctctccc 2580
   ttatcctccc agactaacct agtgacagac tgcgcaagct acggctactg gaccgacgaa 2640
   tggagcgtcg cctgtttcga cagctataat gcctcgagcc ccatcttcac cgacacctcc 2700
   gtgggtaacc ctgtcgaccg ccaatgggaa tggttcctct gcaacgagcc tttcttctgg 2760
   tggcaggagt gcgtacccct tacctcattc atgataacac acgaacaatt ccactaacaa 2820
   agatccagcg gtgcccccga gggaacctcc actattgtgc cccggctcgt cagcgcctcc 2880
   tactggcaac gccaatgccc gctctacttc cccgaagtta acggctacac gtacggcagc 2940
   gcgaagggta aaaactccgc tacggtgaac agctggacgg gtggatggga tatgacccgc 3000
   aacacgacgc ggttgatctg gacgaacggg taggtctccc cctaatttcc gttgaatgtg 3060
   atgtgaagat aaactcaatg ctaataaatt gagaaggcaa tatgacccct ggcgcgactc 3120
   cggtgtgtcg agcactttcc ggcccggtgg tccgctggtt agcacggcga acgaacccgt 3180
   gcagattatt ccgggcgggt tccattgctc ggacttgtat atggaggatt actatgcgaa 3240
   tgagggtgtg aggaaggtgg ttgataatga ggtgaagcag attaaggagt gggtggagga 3300
   gtattatgct tgatgaagat actggtggac atatggagtg tacataagat gaatggtcat 3360
   aaaatgatga tggtagatac ggctatggct gttgattag 3399
<210> 39
   <211> 1536
   <212> DNA
   <213> Aspergillus niger
<220>
   <221> CDS
   <222> (1) .. (1536)
<400> 39
   atg cgt tcc ttc tcc gtt gtc gct gcc gcg tca ctg gcg ctc tct tgg        48
   Met Arg Ser Phe Ser Val Val Ala Ala Ala Ser Leu Ala Leu Ser Trp
   1               5                   10                  15
   gcg tct ctg gcc cag gct gct cgc ccc cgt ctt gtg ccc aag cct atc        96
   Ala Ser Leu Ala Gln Ala Ala Arg Pro Arg Leu Val Pro Lys Pro Ile
               20                  25                  30
   tct cgg cca gct tcg agt aag tcg gct gcg act acg ggt gag gct tat       144
   Ser Arg Pro Ala Ser Ser Lys Ser Ala Ala Thr Thr Gly Glu Ala Tyr
           35                  40                  45
   ttt gag cag ctg ctg gac cat cac aac ccg gag aag gga acg ttt tcc       192
   Phe Glu Gln Leu Leu Asp His His Asn Pro Glu Lys Gly Thr Phe Ser
       50                  55                  60
   cag cgg tac tgg tgg agt act gaa tac tgg ggt gga cct ggg tca ccg       240
   Gln Arg Tyr Trp Trp Ser Thr Glu Tyr Trp Gly Gly Pro Gly Ser Pro
   65                  70                  75                  80
   gtg gtc ctc ttt aac cct gga gag gtc tct gcc gat ggc tat gag ggg       288
   Val Val Leu Phe Asn Pro Gly Glu Val Ser Ala Asp Gly Tyr Glu Gly
                   85                  90                  95
   tat ctc acc aac gat act ctc act ggt gtc tat gcg cag gag atc cag       336
   Tyr Leu Thr Asn Asp Thr Leu Thr Gly Val Tyr Ala Gln Glu Ile Gln
               100                 105                 110
   ggt gcc gtc att ctc att gaa cac cgc tac tgg ggc gac tct tcg cct       384
   Gly Ala Val Ile Leu Ile Glu His Arg Tyr Trp Gly Asp Ser Ser Pro
           115                 120                 125
   tat gag gtg ctc aat gcc gaa aca ctt cag tat ctc aca ctg gat cag       432
   Tyr Glu Val Leu Asn Ala Glu Thr Leu Gln Tyr Leu Thr Leu Asp Gln
       130                 135                 140
   tcc att ctg gac atg acc tac ttc gcc gag acg gta aag ctg cag ttc       480
   Ser Ile Leu Asp Met Thr Tyr Phe Ala Glu Thr Val Lys Leu Gln Phe
   145                 150                 155                 160
   gat aat agc agc cgc agc aat gcg cag aat gct ccc tgg gtc atg gtc      528
   Asp Asn Ser Ser Arg Ser Asn Ala Gln Asn Ala Pro Trp Val Met Val
                   165                 170                 175
   ggt ggc tca tac agc ggt gcc ttg acg gct tgg acc gag tct atc gcg      576
   Gly Gly Ser Tyr Ser Gly Ala Leu Thr Ala Trp Thr Glu Ser Ile Ala
               180                 185                 190
   cct gga acg ttc tgg gct tac cat gcc acc agt gcg cct gtg gag gct      624
   Pro Gly Thr Phe Trp Ala Tyr His Ala Thr Ser Ala Pro Val Glu Ala
           195                 200                 205
   atc tat gac ttt tgg caa tac ttc tac ccc att cag caa ggt atg gca      672
   Ile Tyr Asp Phe Trp Gln Tyr Phe Tyr Pro Ile Gln Gln Gly Met Ala
       210                 215                 220
   cag aac tgc agc aag gat gtg tct ctg gta gcc gag tat gtc gac aaa      720
   Gln Asn Cys Ser Lys Asp Val Ser Leu Val Ala Glu Tyr Val Asp Lys
   225                 230                 235                 240
   att ggg aag aat gga act gcc aag gaa cag cag gag ctc aaa gaa ttg      768
   Ile Gly Lys Asn Gly Thr Ala Lys Glu Gln Gln Glu Leu Lys Glu Leu
                   245                 250                 255
   ttt ggt ctg gga gct gtt gag cat tac gat gac ttt gcc gct gtc ctg      816
   Phe Gly Leu Gly Ala Val Glu His Tyr Asp Asp Phe Ala Ala Val Leu
               260                 265                 270
   ccc aac gga ccg tac ctc tgg caa gac aac gac ttt gtc aca gga tac      864
   Pro Asn Gly Pro Tyr Leu Trp Gln Asp Asn Asp Phe Val Thr Gly Tyr
           275                 280                 285
   tct tcc ttc ttc cag ttc tgt gat gct gtc gag ggt gtc gaa gcc ggc      912
   Ser Ser Phe Phe Gln Phe Cys Asp Ala Val Glu Gly Val Glu Ala Gly
       290                 295                 300
   gcg gca gtg acc ccc ggc ccc gag ggc gtc gga ctt gaa aag gcc ctg      960
   Ala Ala Val Thr Pro Gly Pro Glu Gly Val Gly Leu Glu Lys Ala Leu
   305                 310                 315                 320
   gcc aac tac gca aac tgg ttc aat tca acc ata ctc cct aac tac tgc     1008
   Ala Asn Tyr Ala Asn Trp Phe Asn Ser Thr Ile Leu Pro Asn Tyr Cys
                   325                 330                 335
   gca agc tac ggc tac tgg acc gac gaa tgg agc gtc gcc tgt ttc gac     1056
   Ala Ser Tyr Gly Tyr Trp Thr Asp Glu Trp Ser Val Ala Cys Phe Asp
               340                 345                 350
   agc tat aat gcc tcg agc ccc atc ttc acc gac acc tcc gtg ggt aac     1104
   Ser Tyr Asn Ala Ser Ser Pro Ile Phe Thr Asp Thr Ser Val Gly Asn
           355                 360                 365
   cct gtc gac cgc caa tgg gaa tgg ttc ctc tgc aac gag cct ttc ttc     1152
   Pro Val Asp Arg Gln Trp Glu Trp Phe Leu Cys Asn Glu Pro Phe Phe
       370                 375                 380
   tgg tgg cag gac ggt gcc ccc gag gga acc tcc act att gtg ccc cgg     1200
   Trp Trp Gln Asp Gly Ala Pro Glu Gly Thr Ser Thr Ile Val Pro Arg
   385                 390                 395                 400
   ctc gtc agc gcc tcc tac tgg caa cgc caa tgc ccg ctc tac ttc ccc     1248
   Leu Val Ser Ala Ser Tyr Trp Gln Arg Gln Cys Pro Leu Tyr Phe Pro
                   405                 410                 415
   gaa gtt aac ggc tac acg tac ggc agc gcg aag ggt aaa aac tcc gct        1296
   Glu Val Asn Gly Tyr Thr Tyr Gly Ser Ala Lys Gly Lys Asn Ser Ala
               420                 425                 430
   acg gtg aac agc tgg acg ggt gga tgg gat atg acc cgc aac acg acg        1344
   Thr Val Asn Ser Trp Thr Gly Gly Trp Asp Met Thr Arg Asn Thr Thr
           435                 440                 445
   cgg ttg atc tgg acg aac ggg caa tat gac ccc tgg cgc gac tcc ggt        1392
   Arg Leu Ile Trp Thr Asn Gly Gln Tyr Asp Pro Trp Arg Asp Ser Gly
       450                 455                 460
   gtg tcg agc act ttc cgg ccc ggt ggt ccg ctg gtt agc acg gcg aac        1440
   Val Ser Ser Thr Phe Arg Pro Gly Gly Pro Leu Val Ser Thr Ala Asn
   465                 470                 475                 480
   gaa ccc gtg cag att att ccg ggc ggg ttc cat tgc tcg gac ttg tat        1488
   Glu Pro Val Gln Ile Ile Pro Gly Gly Phe His Cys Ser Asp Leu Tyr
                   485                 490                 495
   atg gag gat tac tat gcg aat gag gat acg gct atg gct gtt gat tag        1536
   Met Glu Asp Tyr Tyr Ala Asn Glu Asp Thr Ala Met Ala Val Asp
               500                 505                 510
<210> 40
   <211> 511
   <212> PRT
   <213> Aspergillus niger
<400> 40
   Met Arg Ser Phe Ser Val Val Ala Ala Ala Ser Leu Ala Leu Ser Trp
   1               5                   10                  15
   Ala Ser Leu Ala Gln Ala Ala Arg Pro Arg Leu Val Pro Lys Pro Ile
               20                  25                  30
   Ser Arg Pro Ala Ser Ser Lys Ser Ala Ala Thr Thr Gly Glu Ala Tyr
           35                  40                  45
   Phe Glu Gln Leu Leu Asp His His Asn Pro Glu Lys Gly Thr Phe Ser
       50                  55                  60
   Gln Arg Tyr Trp Trp Ser Thr Glu Tyr Trp Gly Gly Pro Gly Ser Pro
   65                  70                  75                  80
   Val Val Leu Phe Asn Pro Gly Glu Val Ser Ala Asp Gly Tyr Glu Gly
                   85                  90                  95
   Tyr Leu Thr Asn Asp Thr Leu Thr Gly Val Tyr Ala Gln Glu Ile Gln
               100                 105                 110
   Gly Ala Val Ile Leu Ile Glu His Arg Tyr Trp Gly Asp Ser Ser Pro
           115                 120                 125
   Tyr Glu Val Leu Asn Ala Glu Thr Leu Gln Tyr Leu Thr Leu Asp Gln
   130 135 140
   Ser Ile Leu Asp Met Thr Tyr Phe Ala Glu Thr Val Lys Leu Gln Phe
   145 150 155 160
   Asp Asn Ser Ser Arg Ser Asn Ala Gln Asn Ala Pro Trp Val Met Val
   165 170 175
   Gly Gly Ser Tyr Ser Gly Ala Leu Thr Ala Trp Thr Glu Ser Ile Ala
   180 185 190
   Pro Gly Thr Phe Trp Ala Tyr His Ala Thr Ser Ala Pro Val Glu Ala
   195 200 205
   Ile Tyr Asp Phe Trp Gln Tyr Phe Tyr Pro Ile Gln Gln Gly Met Ala
   210 215 220
   Gln Asn Cys Ser Lys Asp Val Ser Leu Val Ala Glu Tyr Val Asp Lys
   225 230 235 240
   Ile Gly Lys Asn Gly Thr Ala Lys Glu Gln Gln Glu Leu Lys Glu Leu
   245 250 255
   Phe Gly Leu Gly Ala Val Glu His Tyr Asp Asp Phe Ala Ala Val Leu
   260 265 270
   Pro Asn Gly Pro Tyr Leu Trp Gln Asp Asn Asp Phe Val Thr Gly Tyr
   275 280 285
   Ser Ser Phe Phe Gln Phe Cys Asp Ala Val Glu Gly Val Glu Ala Gly
   290 295 300
   Ala Ala Val Thr Pro Gly Pro Glu Gly Val Gly Leu Glu Lys Ala Leu
   305 310 315 320
   Ala Asn Tyr Ala Asn Trp Phe Asn Ser Thr Ile Leu Pro Asn Tyr Cys
   325 330 335
   Ala Ser Tyr Gly Tyr Trp Thr Asp Glu Trp Ser Val Ala Cys Phe Asp
   340 345 350
   Ser Tyr Asn Ala Ser Ser Pro Ile Phe Thr Asp Thr Ser Val Gly Asn
   355 360 365
   Pro Val Asp Arg Gln Trp Glu Trp Phe Leu Cys Asn Glu Pro Phe Phe
   370 375 380
   Trp Trp Gln Asp Gly Ala Pro Glu Gly Thr Ser Thr Ile Val Pro Arg
   385 390 395 400
   Leu Val Ser Ala Ser Tyr Trp Gln Arg Gln Cys Pro Leu Tyr Phe Pro
   405 410 415
   Glu Val Asn Gly Tyr Thr Tyr Gly Ser Ala Lys Gly Lys Asn Ser Ala
   420 425 430
   Thr Val Asn Ser Trp Thr Gly Gly Trp Asp Met Thr Arg Asn Thr Thr
   435 440 445
   Arg Leu Ile Trp Thr Asn Gly Gln Tyr Asp Pro Trp Arg Asp Ser Gly
   450 455 460
   Val Ser Ser Thr Phe Arg Pro Gly Gly Pro Leu Val Ser Thr Ala Asn
   465 470 475 480
   Glu Pro Val Gln Ile Ile Pro Gly Gly Phe His Cys Ser Asp Leu Tyr
   485 490 495
   Met Glu Asp Tyr Tyr Ala Asn Glu Asp Thr Ala Met Ala Val Asp
   500 505 510
<210> 41
   <211> 3009
   <212> DNA
   <213> Aspergillus niger
<400> 41
   atggtctcct gcggacctac gtgaaccatc cagccatctg gtgccacaca atcggcgatc 60
   tatcatatca gaaaggcgcc ttgttggagc cactaagtgt cgcactcacc gcagttactc 120
   gatctggggt cagaattgga gatccggttt tgatttgtgg cgcgggaccg attgggttgg 180
   tggtgctgca atgctgccgc gctgctgggg cgtatcccac tgtgatcagc gatgttaacc 240
   cggcgcggtt gcaatttgcg cgacagttcg tgcctgctgc tcgaacactg caggttcggc 300
   cggaggagac cgacaaggaa tttgcagctc gggtggtgca gttgatgggt ggggaggatt 360
   gcgagcccgt ggttgccctg gaatgcacgg gagtcgaaag ttcgatcgcc ggtgctattc 420
   agagtgtcaa gtttgggggc aaagtgtttg tggttggcgt gggcaaggat aagcttcagt 480
   ttccattcat gcgactgagc gagcgcgaga tcgatctgca gttccagcag cgctatgtca 540
   acatgtggcc ccgcgccatc cgggtgatgt ccagcggagc catcgatcta gaccagatga 600
   tcactcatgt ctatgccttt gaagacgctc tcaaggcgtt tcagacggcg tcagatccga 660
   gtagcggctc aattaaggtg ctgattgagg ggccacaagg atgagagatg gcggatattg 720
   gacagtgaag ccatgactgt tataaggtat agcagaacag aacatgtatc ttgtcaacga 780
   attgattgat ttcagcacgg aaatgttcaa ggcatggaac aattgcttct gacccggaac 840
   cgcggattgc aaggatggat atgtggatca gatggtgtga agaagatctg tctgttagca 900
   tacttcataa gttccaagga ggagtaaatg tggagtcata agagtaatat cgaatatcat 960
   agaatggggt tgtcttgcta acggtgaatc ggactccgtc ggctctgcca gcgatcggca 1020
   ggtcacgcgt ctgcgtaact tggtacttat cttcccagtc acctaagcac tgttactcca 1080
   ctcttctctc tttgacgata agaagatagc agagactctt gataaaaagg gttgggagca 1140
   tgctttgaat ttcttcactc cgagagcttt ctgtccccat ctggtctctt gttgtccccg 1200
   atgtactact ctctctgggt tgctgccttg gtggccgcgc tgcccgtctc ccgggcccag 1260
   tttgtggctc cgcccacgga tctcattccc accaagggat atctcgacat ccccgtccgc 1320
   tacaaacagg tccccaccgg catttgtgag actgatccca gtgtcaagag cttctccggt 1380
   tacgtcgatg tcgctgagca tgagcacatc ttcttctggt tcttcgaggc gcgcaaccaa 1440
   gatcccaccg aggctccctt gaccgtctgg atcaatggag gcatgtctga ccccggtcat 1500
   tatttccttc caattgctaa ccgttcgtag gtcctggttc ctcctccatg atcggcttgt 1560
   tccaagagca cggcccatgc ggcattgacg ccaatggctc cgtctacaac aacccctact 1620
   cctggaacaa cgccagcaac atgctctaca tcgaccagcc cgtgcagacc ggcttctcct 1680
   acagcattcc ggttcccggc tatgtggatt cttccacaga caatgttatt gctctgccct 1740
   cccccgcctg ccccgactat gcagcggata tgttctgtgg cacttactcc taccccaacg 1800
   tgagccttac ggctaattcc accgacaacg ccgcccccaa cttctaccgc gccctacagg 1860
   gttttatggg cgcatttcct cagtactcgc gcgaaacctt ccacttcacc acggagagtt 1920
   atggcggcca ctacgggccc gtcttcaacg agtacatcga ggagcagaac gcccatctcc 1980
   agccgggagc caagaagatc caactgggca gtgtgatgat cggcaatggc tggtatgacc     2040
   cgattattca ataccaggcc tactacaact ttacggtaca ctacctggtt tctgatcctt     2100
   atccttacag cctggacact aatctatcta caggtatatc cgggcaacac atacgactac 2160
   ctgccattca acaagtccat cagctcgctg atgtacaaca acctctatgg ccccggaaac 2220
   tgcctcgacc agctctacga ctgcgccgcc cgaggcatcg acgagatctg cagcactgcc 2280
   gacgattttt gcgccaacga ggtcgaaaac gtctacgaca tttactccgg tcgggatgag 2340
   tatgactttc gtgaactcac tccggacccg ttcccttacg agttctacgt tgactacctg 2400
   aacaaagcgt ccgtgcaggc cgccatcggc gcatacatca attacacgga gagcaacaac 2460
   gctgttggac tcgccttttc gtccaccggt gacgacgggc gactcatgaa caccatccag 2520
   gatgtgggca agctgctcaa acagggtgtc acggtggtca tgtacgccgg ggatgccgac 2580
   tataactgca actggctggg tggggaagcc gtgtcgttgc aggtcaaggc cgccaacttc 2640
   agtagtgcgg gttacaccaa cattgtcacc tcggatggag tgacacacgg ccaggtgcgc 2700
   caggcggggc aatttgcctt tgtgcgagtg tatgagagtg gacatgaggt tcccttctat 2760
   caacccttgc ttgcgctgga gatgtttgag cgcgtcattg gcggcaagga tgtggcgacg 2820
   ggaaagattc ccatctcgtc gagtttacag acggtgggca cgcccaagag ttactaccgg 2880
   gagggcaaca gcacgattca gtgggaggtg ttggattctc tggcgacgta caacacaacc 2940
   acgaatgctc cgaacccggt gagccggagg ctgaagcgga tgggaccagc tttgcggttt 3000
   cagatgtag 3009
<210> 42
   <211> 1701
   <212> DNA
   <213> Aspergillus niger
<220>
   <221> CDS
   <222> (1)..(1701)
<400> 42
   atg tac tac tct ctc tgg gtt gct gcc ttg gtg gcc gcg ctg ccc gtc      48
   Met Tyr Tyr Ser Leu Trp Val Ala Ala Leu Val Ala Ala Leu Pro Val
   1               5                   10                  15
   tcc cgg gcc cag ttt gtg gct ccg ccc acg gat ctc att ccc acc aag      96
   Ser Arg Ala Gln Phe Val Ala Pro Pro Thr Asp Leu Ile Pro Thr Lys
               20                  25                  30
   gga tat ctc gac atc ccc gtc cgc tac aaa cag gtc ccc acc ggc att     144
   Gly Tyr Leu Asp Ile Pro Val Arg Tyr Lys Gln Val Pro Thr Gly Ile
           35                  40                  45
   tgt gag act gat ccc agt gtc aag agc ttc tcc ggt tac gtc gat gtc     192
   Cys Glu Thr Asp Pro Ser Val Lys Ser Phe Ser Gly Tyr Val Asp Val
       50                  55                  60
   gct gag cat gag cac atc ttc ttc tgg ttc ttc gag gcg cgc aac caa     240
   Ala Glu His Glu His Ile Phe Phe Trp Phe Phe Glu Ala Arg Asn Gln
   65                  70                  75                  80
   gat ccc acc gag gct ccc ttg acc gtc tgg atc aat gga ggt cct ggt     288
   Asp Pro Thr Glu Ala Pro Leu Thr Val Trp Ile Asn Gly Gly Pro Gly
                   85                  90                  95
   tcc tcc tcc atg atc ggc ttg ttc caa gag cac ggc cca tgc ggc att     336
   Ser Ser Ser Met Ile Gly Leu Phe Gln Glu His Gly Pro Cys Gly Ile
               100                 105                 110
   gac gcc aat ggc tcc gtc tac aac aac ccc tac tcc tgg aac aac gcc     384
   Asp Ala Asn Gly Ser Val Tyr Asn Asn Pro Tyr Ser Trp Asn Asn Ala
           115                 120                 125
   agc aac atg ctc tac atc gac cag ccc gtg cag acc ggc ttc tcc tac     432
   Ser Asn Met Leu Tyr Ile Asp Gln Pro Val Gln Thr Gly Phe Ser Tyr
       130                 135                 140
   agc att ccg gtt ccc ggc tat gtg gat tct tcc aca gac aat gtt att     480
   Ser Ile Pro Val Pro Gly Tyr Val Asp Ser Ser Thr Asp Asn Val Ile
   145                 150                 155                 160
   gct ctg ccc tcc ccc gcc tgc ccc gac tat gca gcg gat atg ttc tgt     528
   Ala Leu Pro Ser Pro Ala Cys Pro Asp Tyr Ala Ala Asp Met Phe Cys
                   165                 170                 175
   ggc act tac tcc tac ccc aac gtg agc ctt acg gct aat tcc acc gac     576
   Gly Thr Tyr Ser Tyr Pro Asn Val Ser Leu Thr Ala Asn Ser Thr Asp
               180                 185                 190
   aac gcc gcc ccc aac ttc tac cgc gcc cta cag ggt ttt atg ggc gca      624
   Asn Ala Ala Pro Asn Phe Tyr Arg Ala Leu Gln Gly Phe Met Gly Ala
           195                 200                 205
   ttt cct cag tac tcg cgc gaa acc ttc cac ttc acc acg gag agt tat      672
   Phe Pro Gln Tyr Ser Arg Glu Thr Phe His Phe Thr Thr Glu Ser Tyr
       210                 215                 220
   ggc ggc cac tac ggg ccc gtc ttc aac gag tac atc gag gag cag aac      720
   Gly Gly His Tyr Gly Pro Val Phe Asn Glu Tyr Ile Glu Glu Gln Asn
   225                 230                 235                 240
   gcc cat ctc cag ccg gga gcc aag aag atc caa ctg ggc agt gtg atg      768
   Ala His Leu Gln Pro Gly Ala Lys Lys Ile Gln Leu Gly Ser Val Met
                   245                 250                 255
   atc ggc aat ggc tgg tat gac ccg att att caa tac cag gcc tac tac      816
   Ile Gly Asn Gly Trp Tyr Asp Pro Ile Ile Gln Tyr Gln Ala Tyr Tyr
               260                 265                 270
   aac ttt acg gta tat ccg ggc aac aca tac gac tac ctg cca ttc aac      864
   Asn Phe Thr Val Tyr Pro Gly Asn Thr Tyr Asp Tyr Leu Pro Phe Asn
           275                 280                 285
   aag tcc atc agc tcg ctg atg tac aac aac ctc tat ggc ccc gga aac      912
   Lys Ser Ile Ser Ser Leu Met Tyr Asn Asn Leu Tyr Gly Pro Gly Asn
       290                 295                 300
   tgc ctc gac cag ctc tac gac tgc gcc gcc cga ggc atc gac gag atc      960
   Cys Leu Asp Gln Leu Tyr Asp Cys Ala Ala Arg Gly Ile Asp Glu Ile
   305                 310                 315                 320
   tgc agc act gcc gac gat ttt tgc gcc aac gag gtc gaa aac gtc tac     1008
   Cys Ser Thr Ala Asp Asp Phe Cys Ala Asn Glu Val Glu Asn Val Tyr
                   325                 330                 335
   gac att tac tcc ggt cgg gat gag tat gac ttt cgt gaa ctc act ccg     1056
   Asp Ile Tyr Ser Gly Arg Asp Glu Tyr Asp Phe Arg Glu Leu Thr Pro
               340                 345                 350
   gac ccg ttc cct tac gag ttc tac gtt gac tac ctg aac aaa gcg tcc     1104
   Asp Pro Phe Pro Tyr Glu Phe Tyr Val Asp Tyr Leu Asn Lys Ala Ser
           355                 360                 365
   gtg cag gcc gcc atc ggc gca tac atc aat tac acg gag agc aac aac     1152
   Val Gln Ala Ala Ile Gly Ala Tyr Ile Asn Tyr Thr Glu Ser Asn Asn
       370                 375                 380
   gct gtt gga ctc gcc ttt tcg tcc acc ggt gac gac ggg cga ctc atg     1200
   Ala Val Gly Leu Ala Phe Ser Ser Thr Gly Asp Asp Gly Arg Leu Met
   385                 390                 395                 400
   aac acc atc cag gat gtg ggc aag ctg ctc aaa cag ggt gtc acg gtg     1248
   Asn Thr Ile Gln Asp Val Gly Lys Leu Leu Lys Gln Gly Val Thr Val
                   405                 410                 415
   gtc atg tac gcc ggg gat gcc gac tat aac tgc aac tgg ctg ggt ggg     1296
   Val Met Tyr Ala Gly Asp Ala Asp Tyr Asn Cys Asn Trp Leu Gly Gly
               420                 425                 430
   gaa gcc gtg tcg ttg cag gtc aag gcc gcc aac ttc agt agt gcg ggt     1344
   Glu Ala Val Ser Leu Gln Val Lys Ala Ala Asn Phe Ser Ser Ala Gly
           435                     440                     445
   tac acc aac att gtc acc tcg gat gga gtg aca cac ggc cag gtg cgc        1392
   Tyr Thr Asn Ile Val Thr Ser Asp Gly Val Thr His Gly Gln Val Arg
       450                     455                     460
   cag gcg ggg caa ttt gcc ttt gtg cga gtg tat gag agt gga cat gag        1440
   Gln Ala Gly Gln Phe Ala Phe Val Arg Val Tyr Glu Ser Gly His Glu
   465                     470                     475                     480
   gtt ccc ttc tat caa ccc ttg ctt gcg ctg gag atg ttt gag cgc gtc        1488
   Val Pro Phe Tyr Gln Pro Leu Leu Ala Leu Glu Met Phe Glu Arg Val
                   485                     490                     495
   att ggc ggc aag gat gtg gcg acg gga aag att ccc atc tcg tcg agt        1536
   Ile Gly Gly Lys Asp Val Ala Thr Gly Lys Ile Pro Ile Ser Ser Ser
               500                     505                     510
   tta cag acg gtg ggc acg ccc aag agt tac tac cgg gag ggc aac agc        1584
   Leu Gln Thr Val Gly Thr Pro Lys Ser Tyr Tyr Arg Glu Gly Asn Ser
           515                     520                     525
   acg att cag tgg gag gtg ttg gat tct ctg gcg acg tac aac aca acc        1632
   Thr Ile Gln Trp Glu Val Leu Asp Ser Leu Ala Thr Tyr Asn Thr Thr
       530                     535                     540
   acg aat gct ccg aac ccg gtg agc cgg agg ctg aag cgg atg gga cca        1680
   Thr Asn Ala Pro Asn Pro Val Ser Arg Arg Leu Lys Arg Met Gly Pro
   545                     550                     555                     560
   gct ttg cgg ttt cag atg tag                                            1701
   Ala Leu Arg Phe Gln Met
                   565
<210> 43
   <211> 566
   <212> PRT
   <213> Aspergillus niger
<400> 43
   Met Tyr Tyr Ser Leu Trp Val Ala Ala Leu Val Ala Ala Leu Pro Val
   1               5                   10                  15
   Ser Arg Ala Gln Phe Val Ala Pro Pro Thr Asp Leu Ile Pro Thr Lys
               20                  25                  30
   Gly Tyr Leu Asp Ile Pro Val Arg Tyr Lys Gln Val Pro Thr Gly Ile
           35                  40                  45
   Cys Glu Thr Asp Pro Ser Val Lys Ser Phe Ser Gly Tyr Val Asp Val
       50                  55                  60
   Ala Glu His Glu His Ile Phe Phe Trp Phe Phe Glu Ala Arg Asn Gln
   65                  70                  75                  80
   Asp Pro Thr Glu Ala Pro Leu Thr Val Trp Ile Asn Gly Gly Pro Gly
                   85                  90                  95
   Ser Ser Ser Met Ile Gly Leu Phe Gln Glu His Gly Pro Cys Gly Ile
               100                 105                 110
   Asp Ala Asn Gly Ser Val Tyr Asn Asn Pro Tyr Ser Trp Asn Asn Ala
           115                 120                 125
   Ser Asn Met Leu Tyr Ile Asp Gln Pro Val Gln Thr Gly Phe Ser Tyr
       130                 135                 140
   Ser Ile Pro Val Pro Gly Tyr Val Asp Ser Ser Thr Asp Asn Val Ile
   145                 150                 155                 160
   Ala Leu Pro Ser Pro Ala Cys Pro Asp Tyr Ala Ala Asp Met Phe Cys
                   165                 170                 175
   Gly Thr Tyr Ser Tyr Pro Asn Val Ser Leu Thr Ala Asn Ser Thr Asp
               180                 185                 190
   Asn Ala Ala Pro Asn Phe Tyr Arg Ala Leu Gln Gly Phe Met Gly Ala
           195                 200                 205
   Phe Pro Gln Tyr Ser Arg Glu Thr Phe His Phe Thr Thr Glu Ser Tyr
       210                 215                 220
   Gly Gly His Tyr Gly Pro Val Phe Asn Glu Tyr Ile Glu Glu Gln Asn
   225                 230                 235                 240
   Ala His Leu Gln Pro Gly Ala Lys Lys Ile Gln Leu Gly Ser Val Met
                   245                 250                 255
   Ile Gly Asn Gly Trp Tyr Asp Pro Ile Ile Gln Tyr Gln Ala Tyr Tyr
               260                 265                 270
   Asn Phe Thr Val Tyr Pro Gly Asn Thr Tyr Asp Tyr Leu Pro Phe Asn
           275                 280                 285
   Lys Ser Ile Ser Ser Leu Met Tyr Asn Asn Leu Tyr Gly Pro Gly Asn
       290                 295                 300
   Cys Leu Asp Gln Leu Tyr Asp Cys Ala Ala Arg Gly Ile Asp Glu Ile
   305                 310                 315                 320
   Cys Ser Thr Ala Asp Asp Phe Cys Ala Asn Glu Val Glu Asn Val Tyr
                   325                 330                 335
   Asp Ile Tyr Ser Gly Arg Asp Glu Tyr Asp Phe Arg Glu Leu Thr Pro
               340                 345                 350
   Asp Pro Phe Pro Tyr Glu Phe Tyr Val Asp Tyr Leu Asn Lys Ala Ser
   355 360 365
   Val Gln Ala Ala Ile Gly Ala Tyr Ile Asn Tyr Thr Glu Ser Asn Asn
   370 375 380
   Ala Val Gly Leu Ala Phe Ser Ser Thr Gly Asp Asp Gly Arg Leu Met
   385 390 395 400
   Asn Thr Ile Gln Asp Val Gly Lys Leu Leu Lys Gln Gly Val Thr Val
   405 410 415
   Val Met Tyr Ala Gly Asp Ala Asp Tyr Asn Cys Asn Trp Leu Gly Gly
   420 425 430
   Glu Ala Val Ser Leu Gln Val Lys Ala Ala Asn Phe Ser Ser Ala Gly
   435 440 445
   Tyr Thr Asn Ile Val Thr Ser Asp Gly Val Thr His Gly Gln Val Arg
   450 455 460
   Gln Ala Gly Gln Phe Ala Phe Val Arg Val Tyr Glu Ser Gly His Glu
   465 470 475 480
   Val Pro Phe Tyr Gln Pro Leu Leu Ala Leu Glu Met Phe Glu Arg Val
   485 490 495
   Ile Gly Gly Lys Asp Val Ala Thr Gly Lys Ile Pro Ile Ser Ser Ser
   500 505 510
   Leu Gln Thr Val Gly Thr Pro Lys Ser Tyr Tyr Arg Glu Gly Asn Ser
   515 520 525
   Thr Ile Gln Trp Glu Val Leu Asp Ser Leu Ala Thr Tyr Asn Thr Thr
   530 535 540
   Thr Asn Ala Pro Asn Pro Val Ser Arg Arg Leu Lys Arg Met Gly Pro
   545 550 555 560
   Ala Leu Arg Phe Gln Met
   565
<210> 44
   <211> 3133
   <212> DNA
   <213> Aspergillus niger
<400> 44
   tctacaacaa attcacaagt cgcgaataag ttctttcaag tatcaaacgg agtactagaa 60
   gacccacgca ccatgtcctc caccacgttc gcctccgaag ccaccacctt cgaatcggcc 120
   ctaaccagcc tcttcagcgg ccctcctgag accaccgaag ccgatctagc caagctcttc 180
   acacctacct tcacacagcg tgacgatgag acaacgcgcg acttccctgc ctttgtcgcg 240
   cacatcaagt ggctgcgggg gattctccca ccaggaagcg tgacactcaa aattacccag 300
   ttcctgcggg acgggaatca atttgcggat agacacagct cgaccatgaa aatgccggat 360
   ggaaaagtgg gcgccgcgga gacgtttatg tttggggagg tggcagagga tggacgaatt 420
   gagtgggtgg tggagacagt gaagcggaag tagtgaaagt gaggactggg ataattccgg 480
   cgggatgagg cattaaattg ttgtgattgc taagagacat cacattgctc atatgagttt 540
   gattgcgact gttaatatta atgtccgact gggaggaaat gagtggcgtt atccaggcag 600
   ctgcatgcaa tactacattc ccctcacgag ggaaatctgg ccatctcgga aatcaatggt 660
   gtcatccgag ttgattcccc tcaataatgg tcctgataag ccactattgc tgcagaacgc 720
   ctatctgtgc cgttagtgca tgcatagtgt ccccagttct ttttctgccg attaatctat 780
   tgccagacgg actccgtcgc gcttccgaat gccgggaacc gcggctaagc tttgctgtag 840
   ctttgctgca ggaacatctg catctttcaa aatatcttca gaggagccga tcgctgagtt 900
   tctcaagaga ttagatgaat gttcgtactt ggccgcaata taagcttcat ggcggcttca 960
   accttccatg agaaataaga ataaggctgc cggggatggc ctcaccgtgt ccctgcatgc 1020
   aagggatgca taatgcaccg tgacctgttc caccacgccg gagcccgccc gccctccgac 1080
   ggcccaaacc cactgcagaa tggcttgatt aagttcccct tctataacta accggttgtt 1140
   cctcgtcgtt gtctgctctg ctctccaccc agggtcgttt ttgcttttgc ctccttaacc 1200
   atggtcgcct tttcccgcat ctcggcaggc ttcgcccttg ccgcccctgc cctggccagc 1260
   gtcgtcctgg agaccgtcaa gtctgttccc agcgactgga agctcgtgga ggctgctgat 1320
   accagctcca caatttcttt gtccgttgct ctggcgcgtc agaacctgga ccagttggag 1380
   gagaagctcc tggccgtgtc cacccctggc aaggacacct acggccagtt cttggatctg 1440
   gacgacatca atgagcagtt tcctctcgca gatgacgctg ctgttgtggc ttggctgaag 1500
   aaggcaggcg tcacccagat ccataaggag ggtggtctgc tgaactttgc gaccactgtg 1560
   ggcacagcca accagcttct caacaccacc ttctcggtgt acaagagcgg atctacccag 1620
   aagctgcgca caacgcaata ctctgttccg gatgagctga ccgggtccat tgatctcatc 1680
   tcgccgactg ttttctttgg aaagtccaac gctgcgcgct cggcggccgt gcgtgcttcg 1740
   cagactacca aggagaccag cagaaagaag agcagtaatg tgtgcgagta catcactccg 1800
   gattgcctca aagagcagta tagcattgac tatacgcccg aggcatcgtc gggaagtcgt 1860
   gttgggtttg gcagtttctt gaacgagtcg gccttgtact cggatttgga tctgttcacc 1920
   cagtactttg acattcccca gcagagtttc actgttgaga ctatcaacgg gggaatcaac 1980
   aaccaggaga atgatccgga tggtgaagcc gatctcgatg tccagaacat cgtgggcatc 2040
   tcgcatccct tgccggtgac ggagtacatt accggaggat ctccgtgagt gttcccaaag 2100
   atgcaattga attaaagcta atggttcagt ccattcattc ccgacgtcga gactactacc 2160
   gacgagaacg agccttacct gcagtactac gagtatctgc tggccaagac caacgacgag 2220
   ctgccactgg ttatcagcaa ctcgtacggc gatgacgaag atgtaagcat ccctgcctcc 2280
   cacacaaatc gcctgctgac agaatagacc gttcccattg cctacgccac ccgcgtatgc 2340
   aacctcatcg gcctgatggg cacacgtggt atctccatcc tcgagtcttc cggcgactct 2400
   ggtacgttgt accccatata tattgcatca agtcccgact gacaaataca ggtgtgggcg 2460
   gcgcatgcat gtccaacgac ggcaccgaca agaccgaatt cacccccatg ttcccaggaa 2520
   catgcccgta catcaccgcg gtcggcggca cccaagacgt gcccgaagtc gcctgggtgg 2580
   acagctccgg cggcttcagc aactacttct cgcagccgtc gtaccagtcg gatcaggtgg 2640
   agacctacct ggacaagtac atctctgcct cgacgaagaa gtactacgag cagtacacca 2700
   acttcagcgg tcgcgcgttc cctgacgtgt ctgcgtttgc aggttctcct tagtatgtat 2760
   ccatcccaga tgattgtatg gacatctgct aatgtccgac agctacgaaa cttatattga 2820
   tggtcagctc ggccttgtgg cgggtacttc tggcgctagc cctgtgtttg cggggatcgt 2880
   cgcgctgctg aacgatgccc gtctgcgggc caacaagaca tccttgggct tcctgaaccc 2940
   ttggctgtac tcgagcggct acaagagcct gaatgacatt accagtggcg aggcagtggg 3000
   ctgccaaggc gatgtggagg gcgctggagt cattccttgg gcgagctgga atgccacgac 3060
   gggatgggat ccggcgacag ggctgggaac gcctaatttt gccaagctga aggaggcggt 3120
   tcttgcgttg taa 3133
<210> 45
   <211> 1743
   <212> DNA
   <213> Aspergillus niger
<220>
   <221> CDS
   <222> (1)..(1743)
<400> 45
   atg gtc gcc ttt tcc cgc atc tcg gca ggc ttc gcc ctt gcc gcc cct      48
   Met Val Ala Phe Ser Arg Ile Ser Ala Gly Phe Ala Leu Ala Ala Pro
   1               5                   10                  15
   gcc ctg gcc agc gtc gtc ctg gag acc gtc aag tct gtt ccc agc gac      96
   Ala Leu Ala Ser Val Val Leu Glu Thr Val Lys Ser Val Pro Ser Asp
               20                  25                  30
   tgg aag ctc gtg gag gct gct gat acc agc tcc aca att tct ttg tcc     144
   Trp Lys Leu Val Glu Ala Ala Asp Thr Ser Ser Thr Ile Ser Leu Ser
           35                  40                  45
   gtt gct ctg gcg cgt cag aac ctg gac cag ttg gag gag aag ctc ctg     192
   Val Ala Leu Ala Arg Gln Asn Leu Asp Gln Leu Glu Glu Lys Leu Leu
       50                  55                  60
   gcc gtg tcc acc cct ggc aag gac acc tac ggc cag ttc ttg gat ctg        240
   Ala Val Ser Thr Pro Gly Lys Asp Thr Tyr Gly Gln Phe Leu Asp Leu
   65                  70                  75                  80
   gac gac atc aat gag cag ttt cct ctc gca gat gac gct gct gtt gtg        288
   Asp Asp Ile Asn Glu Gln Phe Pro Leu Ala Asp Asp Ala Ala Val Val
                   85                  90                  95
   gct tgg ctg aag aag gca ggc gtc acc cag atc cat aag gag ggt ggt        336
   Ala Trp Leu Lys Lys Ala Gly Val Thr Gln Ile His Lys Glu Gly Gly
               100                 105                 110
   ctg ctg aac ttt gcg acc act gtg ggc aca gcc aac cag ctt ctc aac        384
   Leu Leu Asn Phe Ala Thr Thr Val Gly Thr Ala Asn Gln Leu Leu Asn
           115                 120                 125
   acc acc ttc tcg gtg tac aag agc gga tct acc cag aag ctg cgc aca        432
   Thr Thr Phe Ser Val Tyr Lys Ser Gly Ser Thr Gln Lys Leu Arg Thr
       130                 135                 140
   acg caa tac tct gtt ccg gat gag ctg acc ggg tcc att gat ctc atc        480
   Thr Gln Tyr Ser Val Pro Asp Glu Leu Thr Gly Ser Ile Asp Leu Ile
   145                 150                 155                 160
   tcg ccg act gtt ttc ttt gga aag tcc aac gct gcg cgc tcg gcg gcc        528
   Ser Pro Thr Val Phe Phe Gly Lys Ser Asn Ala Ala Arg Ser Ala Ala
                   165                 170                 175
   gtg cgt gct tcg cag act acc aag gag acc agc aga aag aag agc agt        576
   Val Arg Ala Ser Gln Thr Thr Lys Glu Thr Ser Arg Lys Lys Ser Ser
               180                 185                 190
   aat gtg tgc gag tac atc act ccg gat tgc ctc aaa gag cag tat agc        624
   Asn Val Cys Glu Tyr Ile Thr Pro Asp Cys Leu Lys Glu Gln Tyr Ser
           195                 200                 205
   att gac tat acg ccc gag gca tcg tcg gga agt cgt gtt ggg ttt ggc        672
   Ile Asp Tyr Thr Pro Glu Ala Ser Ser Gly Ser Arg Val Gly Phe Gly
       210                 215                 220
   agt ttc ttg aac gag tcg gcc ttg tac tcg gat ttg gat ctg ttc acc        720
   Ser Phe Leu Asn Glu Ser Ala Leu Tyr Ser Asp Leu Asp Leu Phe Thr
   225                 230                 235                 240
   cag tac ttt gac att ccc cag cag agt ttc act gtt gag act atc aac        768
   Gln Tyr Phe Asp Ile Pro Gln Gln Ser Phe Thr Val Glu Thr Ile Asn
                   245                 250                 255
   ggg gga atc aac aac cag gag aat gat ccg gat ggt gaa gcc gat ctc        816
   Gly Gly Ile Asn Asn Gln Glu Asn Asp Pro Asp Gly Glu Ala Asp Leu
               260                 265                 270
   gat gtc cag aac atc gtg ggc atc tcg cat ccc ttg ccg gtg acg gag        864
   Asp Val Gln Asn Ile Val Gly Ile Ser His Pro Leu Pro Val Thr Glu
           275                 280                 285
   tac att acc gga gga tct cct cca ttc att ccc gac gtc gag act act        912
   Tyr Ile Thr Gly Gly Ser Pro Pro Phe Ile Pro Asp Val Glu Thr Thr
       290                 295                 300
   acc gac gag aac gag cct tac ctg cag tac tac gag tat ctg ctg gcc        960
   Thr Asp Glu Asn Glu Pro Tyr Leu Gln Tyr Tyr Glu Tyr Leu Leu Ala
   305                 310                 315                 320
   aag acc aac gac gag ctg cca ctg gtt atc agc aac tcg tac ggc gat        1008
   Lys Thr Asn Asp Glu Leu Pro Leu Val Ile Ser Asn Ser Tyr Gly Asp
                   325                 330                 335
   gac gaa gat acc gtt ccc att gcc tac gcc acc cgc gta tgc aac ctc        1056
   Asp Glu Asp Thr Val Pro Ile Ala Tyr Ala Thr Arg Val Cys Asn Leu
               340                 345                 350
   atc ggc ctg atg ggc aca cgt ggt atc tcc atc ctc gag tct tcc ggc        1104
   Ile Gly Leu Met Gly Thr Arg Gly Ile Ser Ile Leu Glu Ser Ser Gly
           355                 360                 365
   gac tct ggt gtg ggc ggc gca tgc atg tcc aac gac ggc acc gac aag        1152
   Asp Ser Gly Val Gly Gly Ala Cys Met Ser Asn Asp Gly Thr Asp Lys
       370                 375                 380
   acc gaa ttc acc ccc atg ttc cca gga aca tgc ccg tac atc acc gcg        1200
   Thr Glu Phe Thr Pro Met Phe Pro Gly Thr Cys Pro Tyr Ile Thr Ala
   385                 390                 395                 400
   gtc ggc ggc acc caa gac gtg ccc gaa gtc gcc tgg gtg gac agc tcc        1248
   Val Gly Gly Thr Gln Asp Val Pro Glu Val Ala Trp Val Asp Ser Ser
                   405                 410                 415
   ggc ggc ttc agc aac tac ttc tcg cag ccg tcg tac cag tcg gat cag        1296
   Gly Gly Phe Ser Asn Tyr Phe Ser Gln Pro Ser Tyr Gln Ser Asp Gln
               420                 425                 430
   gtg gag acc tac ctg gac aag tac atc tct gcc tcg acg aag aag tac        1344
   Val Glu Thr Tyr Leu Asp Lys Tyr Ile Ser Ala Ser Thr Lys Lys Tyr
           435                 440                 445
   tac gag cag tac acc aac ttc agc ggt cgc gcg ttc cct gac gtg tct        1392
   Tyr Glu Gln Tyr Thr Asn Phe Ser Gly Arg Ala Phe Pro Asp Val Ser
       450                 455                 460
   gcg ttt gca ggt tct cct tac tac gaa act tat att gat ggt cag ctc        1440
   Ala Phe Ala Gly Ser Pro Tyr Tyr Glu Thr Tyr Ile Asp Gly Gln Leu
   465                 470                 475                 480
   ggc ctt gtg gcg ggt act tct ggc gct agc cct gtg ttt gcg ggg atc        1488
   Gly Leu Val Ala Gly Thr Ser Gly Ala Ser Pro Val Phe Ala Gly Ile
                   485                 490                 495
   gtc gcg ctg ctg aac gat gcc cgt ctg cgg gcc aac aag aca tcc ttg        1536
   Val Ala Leu Leu Asn Asp Ala Arg Leu Arg Ala Asn Lys Thr Ser Leu
               500                 505                 510
   ggc ttc ctg aac cct tgg ctg tac tcg agc ggc tac aag agc ctg aat        1584
   Gly Phe Leu Asn Pro Trp Leu Tyr Ser Ser Gly Tyr Lys Ser Leu Asn
           515                 520                 525
   gac att acc agt ggc gag gca gtg ggc tgc caa ggc gat gtg gag ggc        1632
   Asp Ile Thr Ser Gly Glu Ala Val Gly Cys Gln Gly Asp Val Glu Gly
       530                 535                 540
   gct gga gtc att cct tgg gcg agc tgg aat gcc acg acg gga tgg gat        1680
   Ala Gly Val Ile Pro Trp Ala Ser Trp Asn Ala Thr Thr Gly Trp Asp
   545                 550                 555                 560
   ccg gcg aca ggg ctg gga acg cct aat ttt gcc aag ctg aag gag gcg        1728
   Pro Ala Thr Gly Leu Gly Thr Pro Asn Phe Ala Lys Leu Lys Glu Ala
                   565                 570                 575
   gtt ctt gcg ttg taa                                                    1743
   Val Leu Ala Leu
   580
<210> 46
   <211> 580
   <212> PRT
   <213> Aspergillus niger
<400> 46
   Met Val Ala Phe Ser Arg Ile Ser Ala Gly Phe Ala Leu Ala Ala Pro
   1 5 10 15
   Ala Leu Ala Ser Val Val Leu Glu Thr Val Lys Ser Val Pro Ser Asp
   20 25 30
   Trp Lys Leu Val Glu Ala Ala Asp Thr Ser Ser Thr Ile Ser Leu Ser
   35 40 45
   Val Ala Leu Ala Arg Gln Asn Leu Asp Gln Leu Glu Glu Lys Leu Leu
   50 55 60
   Ala Val Ser Thr Pro Gly Lys Asp Thr Tyr Gly Gln Phe Leu Asp Leu
   65 70 75 80
   Asp Asp Ile Asn Glu Gln Phe Pro Leu Ala Asp Asp Ala Ala Val Val
   85 90 95
   Ala Trp Leu Lys Lys Ala Gly Val Thr Gln Ile His Lys Glu Gly Gly
   100 105 110
   Leu Leu Asn Phe Ala Thr Thr Val Gly Thr Ala Asn Gln Leu Leu Asn
   115 120 125
   Thr Thr Phe Ser Val Tyr Lys Ser Gly Ser Thr Gln Lys Leu Arg Thr
   130 135 140
   Thr Gln Tyr Ser Val Pro Asp Glu Leu Thr Gly Ser Ile Asp Leu Ile
   145 150 155 160
   Ser Pro Thr Val Phe Phe Gly Lys Ser Asn Ala Ala Arg Ser Ala Ala
   165 170 175
   Val Arg Ala Ser Gln Thr Thr Lys Glu Thr Ser Arg Lys Lys Ser Ser
   180 185 190
   Asn Val Cys Glu Tyr Ile Thr Pro Asp Cys Leu Lys Glu Gln Tyr Ser
   195 200 205
   Ile Asp Tyr Thr Pro Glu Ala Ser Ser Gly Ser Arg Val Gly Phe Gly
   210 215 220
   Ser Phe Leu Asn Glu Ser Ala Leu Tyr Ser Asp Leu Asp Leu Phe Thr
   225                 230                 235                 240
   Gln Tyr Phe Asp Ile Pro Gln Gln Ser Phe Thr Val Glu Thr Ile Asn
                   245                 250                 255
   Gly Gly Ile Asn Asn Gln Glu Asn Asp Pro Asp Gly Glu Ala Asp Leu
               260                 265                 270
   Asp Val Gln Asn Ile Val Gly Ile Ser His Pro Leu Pro Val Thr Glu
           275                 280                 285
   Tyr Ile Thr Gly Gly Ser Pro Pro Phe Ile Pro Asp Val Glu Thr Thr
       290                 295                 300
   Thr Asp Glu Asn Glu Pro Tyr Leu Gln Tyr Tyr Glu Tyr Leu Leu Ala
   305                 310                 315                 320
   Lys Thr Asn Asp Glu Leu Pro Leu Val Ile Ser Asn Ser Tyr Gly Asp
                   325                 330                 335
   Asp Glu Asp Thr Val Pro Ile Ala Tyr Ala Thr Arg Val Cys Asn Leu
               340                 345                 350
   Ile Gly Leu Met Gly Thr Arg Gly Ile Ser Ile Leu Glu Ser Ser Gly
           355                 360                 365
   Asp Ser Gly Val Gly Gly Ala Cys Met Ser Asn Asp Gly Thr Asp Lys
       370                 375                 380
   Thr Glu Phe Thr Pro Met Phe Pro Gly Thr Cys Pro Tyr Ile Thr Ala
   385                 390                 395                 400
   Val Gly Gly Thr Gln Asp Val Pro Glu Val Ala Trp Val Asp Ser Ser
                   405                 410                 415
   Gly Gly Phe Ser Asn Tyr Phe Ser Gln Pro Ser Tyr Gln Ser Asp Gln
               420                 425                 430
   Val Glu Thr Tyr Leu Asp Lys Tyr Ile Ser Ala Ser Thr Lys Lys Tyr
           435                 440                 445
   Tyr Glu Gln Tyr Thr Asn Phe Ser Gly Arg Ala Phe Pro Asp Val Ser
       450                 455                 460
   Ala Phe Ala Gly Ser Pro Tyr Tyr Glu Thr Tyr Ile Asp Gly Gln Leu
   465                 470                 475                 480
   Gly Leu Val Ala Gly Thr Ser Gly Ala Ser Pro Val Phe Ala Gly Ile
   485 490 495
   Val Ala Leu Leu Asn Asp Ala Arg Leu Arg Ala Asn Lys Thr Ser Leu
   500 505 510
   Gly Phe Leu Asn Pro Trp Leu Tyr Ser Ser Gly Tyr Lys Ser Leu Asn
   515 520 525
   Asp Ile Thr Ser Gly Glu Ala Val Gly Cys Gln Gly Asp Val Glu Gly
   530 535 540
   Ala Gly Val Ile Pro Trp Ala Ser Trp Asn Ala Thr Thr Gly Trp Asp
   545 550 555 560
   Pro Ala Thr Gly Leu Gly Thr Pro Asn Phe Ala Lys Leu Lys Glu Ala
   565 570 575
   Val Leu Ala Leu
   580
<210> 47
   <211> 3591
   <212> DNA
   <213> Aspergillus niger
<400> 47
   ctgagtcata cttttctacc acccgcacaa gccgaatttc agccgagtta catatactaa      60
   cttatgtgcg tcttagtgtg aattttccct actgaaatcc tatttctata actaggataa     120
   atgttcgaat ctaattgata ttttcaggtg ctatgatggt tactgtactt tcgtatattc     180
   attttacacg ttgaagtatt taacatactt cgataaaata gactttgagc tctatagaag     240
   atcgcttagg cgttctcctt tgtggccgga acagttactc ttgccacgca cctttatcca     300
   caatccacct cccaagctgt tgccaggcag caaaaagtgg cgccaaattc ggcctggact     360
   acggcggtct caaaaatcag atttgccaga tgcccgctaa tacccggcgt actagagcag     420
   aaagggaagg cgactaaagt ctgctggaaa tacaccgtaa tacccatttc gatgctaact     480
   gtccgggaat catttctagg tttctcgtac agatgggtag acggtggata gaaatcccta     540
   tgctcaacac atacatacag gtcatgcgtc cttaaaccct cccattggcc ccttcccctc     600
   cacaagatga catccaattt tcctagcaaa ttcccgggct tgatcctccg ttatcgcttg     660
   cgcattagat gactttctat tttcggccaa gtctttcttc cgataaggat gaatagcggt     720
   tgagcccgtc aagcgggcag ccaacataaa ttgatctgca agtggtcggg tttgcaaagc     780
   tggttattag ggctgtgtgt gatagttctt atcccgtgca ggcacagata cccctctctg     840
   cttaattaac cacccgtcag gtgtccgcac ttacatggcg ccattaatgt ctaaaagcaa     900
   gacattaatt catccttcac caatcaatgg ccatcattgg ttgcaatgac ggataagata 960
   catttagtta cataactata tattgaccat ggtctgataa gggtccgttc tcctcacgga 1020
   agtgagacgt tgctagttcc cgtgccctaa catgaattgg atgccgaaag gatgcaaccc 1080
   cgtcggagtg tatacggaga tgcgtcggag tctatttcat tataaatatc agggtagcag 1140
   tatgtgatgt tcacagtact cttgcactga agtagggtac agacgggata aggagtcaga 1200
   atgaagttct tctcgacaat ctgcagtttg acccttgcgg tctctgcgtt ggccttgcca 1260
   acttctgacc atgttattca tgagaagcga tctgctaccc catctcgatg ggagaagatc 1320
   agtcgggtca atggtactga gcatgtcttg gtgcgcatcg gtctgacgca gaacaatctt 1380
   gatcgagcct atgagtatct catgagcgtg tacgttgctt cctttaccct atattgttcc 1440
   gactgactag tcaggtctga ctccgtgtcg cctaactacg gcaaattctg gactccagaa 1500
   gaggtgcaca gtacctttgc gccgtcgaat gagactgtca acgctgtgcg caactggctt 1560
   attgaatccg gtgtggatga gtcgcgcttg gttcacacca aaaaccaagg ctggattgtg 1620
   tttgatgcaa ccaccaaaga agcggaaaat ctcctccaca ccaagtatta ccactacact 1680
   gaccggattt ctggcttcaa gacactcgca gcggaagagt atgtctccca ttcattctca 1740
   gagatatagt atactaacaa gacgaccaga taccgcgtcc cccagaagat ccaacaacac 1800
   atcgacttca ttaaacccgg cgttcttctc ccattgacct ccaagggccc atctgccaag 1860
   cacaccaaga agtacaaacc cttgaagcag acatcagtca acgccacgtc tctcaccacg 1920
   tgcgacgagg tcatcactcc agcttgtgtc gccgctctgt acaagatccc tcacgcaagc 1980
   ggcaacgtca gtgcaagcaa ttcgcttgga atcttcgaag aaggagacta ctacgcccag 2040
   gaagatctgg atctcttttt ccgcaacttc accccgtaca ttccgaaggg aacacaccct 2100
   aagccggcat ttatcgatgg agcctcagcg cccgtgagcg ttgccgatgc tggtgcggag 2160
   tcggatttgg actttcagct tgcgtatcca attgtttacc ctcagaccat cacgctgtat 2220
   cagacagatg actatgacta tgcaagtggg gaggttgaga ctgatggatt ctttaacacc 2280
   tttcttgatg ctgttgatgg ggtaagttgg cctctggaat tttcggattt agctaatgat 2340
   tcagtcatac tgcacctact gtgcctatgg ggaatgcgga gacagcccga ctctcgatcc 2400
   cacttaccca gataactcca ccggagggta caagggacag ttgatgtgcg gtgtttataa 2460
   gcccacgaat gtcatctccg tctcttatgg tggccaggag gccgacctcc cggcttacta 2520
   ccagcagcga caatgcaatg agtatgccat ccccgcctct gtttatacag ccccaaatta 2580
   acaacaggaa tagattcctc aagctcggtc tccagggaat ttccatcctc ttcgcctctg 2640
   gcgatgatgg cgtcgcaggg cccccaggcg acgactcgac taacgggtgt ctcggaaatg 2700
   gaaccatctt cagtcctgcg ttccccaatt cgtatgtttt cttctcttgc aatatccttg 2760
   caacaaccac atctaacttc tctagttgcc cctgggtgac taatgtcgga gccaccaaac 2820
   tctaccccgg aaagaccatc gcagacgggg aaagcgccgt cgtcgacccg gccggccacc 2880
   cctactcggt cgcattctcc tctggtggtg gcttcagcaa catctatact attccagact 2940
   atcaagccga agcagtagca gagtaggttc ttttctatca tcctatacgc gcaatctaac 3000
   aacccccatt cagatacttc aaaaagcaca acccgcccta tccttactac gaaggcaacg 3060
   ccagcttcgg caaaaacggc ggtgtctaca accgtcttgg acgtgggtac cccgacgtgg 3120
   cagcgaacgg cgacaacatc gcggagtaca acgcgggaga attcatactt gagggtggaa 3180
   ctagtgctag tacgttacta ccctactaca tccacccaac tccgttatat ttcaattact 3240
   aatgacaata atcaaaaggt accccgatct tctcctccgt gattaaccgc atcattgaga 3300
   agcgcatcgc ggcaggaaag ggcccactag gcttcctgaa cccggttctg tatcggaatg 3360
   cgtgggcgtt gaatgatatt acgaacgggt cgaatccggg ttgtggaacg gaggggttct 3420
   atactgctcc tgggtatgta ttttcctctc gatcttttat tcctttatta ccgtgcttcg 3480
   atatatggag ccatgctaac aatcggtgaa caaaatagat gggatcccgt caccggtctc 3540
   ggaacgccta acttcccgaa attgctggac gtgttcctga acctcccgta a 3591
<210> 48
   <211> 1941
   <212> DNA
   <213> Aspergillus niger
<220>
   <221> CDS
   <222> (1) .. (1941)
<400> 48
   atg aag ttc ttc tcg aca atc tgc agt ttg acc ctt gcg gtc tct gcg          48
   Met Lys Phe Phe Ser Thr Ile Cys Ser Leu Thr Leu Ala Val Ser Ala
   1               5                   10                  15
   ttg gcc ttg cca act tct gac cat gtt att cat gag aag cga tct gct          96
   Leu Ala Leu Pro Thr Ser Asp His Val Ile His Glu Lys Arg Ser Ala
               20                  25                  30
   acc cca tct cga tgg gag aag atc agt cgg gtc aat ggt act gag cat          144
   Thr Pro Ser Arg Trp Glu Lys Ile Ser Arg Val Asn Gly Thr Glu His
           35                  40                  45
   gtc ttg gtg cgc atc ggt ctg acg cag aac aat ctt gat cga gcc tat          192
   Val Leu Val Arg Ile Gly Leu Thr Gln Asn Asn Leu Asp Arg Ala Tyr
       50                  55                  60
   gag tat ctc atg agc gtg tct gac tcc gtg tcg cct aac tac ggc aaa          240
   Glu Tyr Leu Met Ser Val Ser Asp Ser Val Ser Pro Asn Tyr Gly Lys
   65                  70                  75                  80
   ttc tgg act cca gaa gag gtg cac agt acc ttt gcg ccg tcg aat gag          288
   Phe Trp Thr Pro Glu Glu Val His Ser Thr Phe Ala Pro Ser Asn Glu
                   85                  90                  95
   act gtc aac gct gtg cgc aac tgg ctt att gaa tcc ggt gtg gat gag          336
   Thr Val Asn Ala Val Arg Asn Trp Leu Ile Glu Ser Gly Val Asp Glu
               100                 105                 110
   tcg cgc ttg gtt cac acc aaa aac caa ggc tgg att gtg ttt gat gca         384
   Ser Arg Leu Val His Thr Lys Asn Gln Gly Trp Ile Val Phe Asp Ala
           115                 120                 125
   acc acc aaa gaa gcg gaa aat ctc ctc cac acc aag tat tac cac tac         432
   Thr Thr Lys Glu Ala Glu Asn Leu Leu His Thr Lys Tyr Tyr His Tyr
       130                 135                 140
   act gac cgg att tct ggc ttc aag aca ctc gca gcg gaa gaa tac cgc         480
   Thr Asp Arg Ile Ser Gly Phe Lys Thr Leu Ala Ala Glu Glu Tyr Arg
   145                 150                 155                 160
   gtc ccc cag aag atc caa caa cac atc gac ttc att aaa ccc ggc gtt         528
   Val Pro Gln Lys Ile Gln Gln His Ile Asp Phe Ile Lys Pro Gly Val
                   165                 170                 175
   ctt ctc cca ttg acc tcc aag ggc cca tct gcc aag cac acc aag aag         576
   Leu Leu Pro Leu Thr Ser Lys Gly Pro Ser Ala Lys His Thr Lys Lys
               180                 185                 190
   tac aaa ccc ttg aag cag aca tca gtc aac gcc acg tct ctc acc acg         624
   Tyr Lys Pro Leu Lys Gln Thr Ser Val Asn Ala Thr Ser Leu Thr Thr
           195                 200                 205
   tgc gac gag gtc atc act cca gct tgt gtc gcc gct ctg tac aag atc         672
   Cys Asp Glu Val Ile Thr Pro Ala Cys Val Ala Ala Leu Tyr Lys Ile
       210                 215                 220
   cct cac gca agc ggc aac gtc agt gca agc aat tcg ctt gga atc ttc         720
   Pro His Ala Ser Gly Asn Val Ser Ala Ser Asn Ser Leu Gly Ile Phe
   225                 230                 235                 240
   gaa gaa gga gac tac tac gcc cag gaa gat ctg gat ctc ttt ttc cgc         768
   Glu Glu Gly Asp Tyr Tyr Ala Gln Glu Asp Leu Asp Leu Phe Phe Arg
                   245                 250                 255
   aac ttc acc ccg tac att ccg aag gga aca cac cct aag ccg gca ttt         816
   Asn Phe Thr Pro Tyr Ile Pro Lys Gly Thr His Pro Lys Pro Ala Phe
               260                 265                 270
   atc gat gga gcc tca gcg ccc gtg agc gtt gcc gat gct ggt gcg gag         864
   Ile Asp Gly Ala Ser Ala Pro Val Ser Val Ala Asp Ala Gly Ala Glu
           275                 280                 285
   tcg gat ttg gac ttt cag ctt gcg tat cca att gtt tac cct cag acc         912
   Ser Asp Leu Asp Phe Gln Leu Ala Tyr Pro Ile Val Tyr Pro Gln Thr
       290                 295                 300
   atc acg ctg tat cag aca gat gac tat gac tat gca agt ggg gag gtt         960
   Ile Thr Leu Tyr Gln Thr Asp Asp Tyr Asp Tyr Ala Ser Gly Glu Val
   305                 310                 315                 320
   gag act gat gga ttc ttt aac acc ttt ctt gat gct gtt gat ggg tca        1008
   Glu Thr Asp Gly Phe Phe Asn Thr Phe Leu Asp Ala Val Asp Gly Ser
                   325                 330                 335
   tac tgc acc tac tgt gcc tat ggg gaa tgc gga gac agc ccg act ctc        1056
   Tyr Cys Thr Tyr Cys Ala Tyr Gly Glu Cys Gly Asp Ser Pro Thr Leu
               340                 345                 350
   gat ccc act tac cca gat aac tcc acc gga ggg tac aag gga cag ttg        1104
   Asp Pro Thr Tyr Pro Asp Asn Ser Thr Gly Gly Tyr Lys Gly Gln Leu
           355                 360                 365
   atg tgc ggt gtt tat aag ccc acg aat gtc atc tcc gtc tct tat ggt        1152
   Met Cys Gly Val Tyr Lys Pro Thr Asn Val Ile Ser Val Ser Tyr Gly
       370                 375                 380
   ggc cag gag gcc gac ctc ccg gct tac tac cag cag cga caa tgc aat        1200
   Gly Gln Glu Ala Asp Leu Pro Ala Tyr Tyr Gln Gln Arg Gln Cys Asn
   385                 390                 395                 400
   gaa ttc ctc aag ctc ggt ctc cag gga att tcc atc ctc ttc gcc tct        1248
   Glu Phe Leu Lys Leu Gly Leu Gln Gly Ile Ser Ile Leu Phe Ala Ser
                   405                 410                 415
   ggc gat gat ggc gtc gca ggg ccc cca ggc gac gac tcg act aac ggg        1296
   Gly Asp Asp Gly Val Ala Gly Pro Pro Gly Asp Asp Ser Thr Asn Gly
               420                 425                 430
   tgt ctc gga aat gga acc atc ttc agt cct gcg ttc ccc aat tct tgc        1344
   Cys Leu Gly Asn Gly Thr Ile Phe Ser Pro Ala Phe Pro Asn Ser Cys
           435                 440                 445
   ccc tgg gtg act aat gtc gga gcc acc aaa ctc tac ccc gga aag acc        1392
   Pro Trp Val Thr Asn Val Gly Ala Thr Lys Leu Tyr Pro Gly Lys Thr
       450                 455                 460
   atc gca gac ggg gaa agc gcc gtc gtc gac ccg gcc ggc cac ccc tac        1440
   Ile Ala Asp Gly Glu Ser Ala Val Val Asp Pro Ala Gly His Pro Tyr
   465                 470                 475                 480
   tcg gtc gca ttc tcc tct ggt ggt ggc ttc agc aac atc tat act att        1488
   Ser Val Ala Phe Ser Ser Gly Gly Gly Phe Ser Asn Ile Tyr Thr Ile
                   485                 490                 495
   cca gac tat caa gcc gaa gca gta gca gaa tac ttc aaa aag cac aac        1536
   Pro Asp Tyr Gln Ala Glu Ala Val Ala Glu Tyr Phe Lys Lys His Asn
               500                 505                 510
   ccg ccc tat cct tac tac gaa ggc aac gcc agc ttc ggc aaa aac ggc        1584
   Pro Pro Tyr Pro Tyr Tyr Glu Gly Asn Ala Ser Phe Gly Lys Asn Gly
           515                 520                 525
   ggt gtc tac aac cgt ctt gga cgt ggg tac ccc gac gtg gca gcg aac        1632
   Gly Val Tyr Asn Arg Leu Gly Arg Gly Tyr Pro Asp Val Ala Ala Asn
       530                 535                 540
   ggc gac aac atc gcg gag tac aac gcg gga gaa ttc ata ctt gag ggt        1680
   Gly Asp Asn Ile Ala Glu Tyr Asn Ala Gly Glu Phe Ile Leu Glu Gly
   545                 550                 555                 560
   gga act agt gct agt acc ccg atc ttc tcc tcc gtg att aac cgc atc        1728
   Gly Thr Ser Ala Ser Thr Pro Ile Phe Ser Ser Val Ile Asn Arg Ile
                   565                 570                 575
   att gag aag cgc atc gcg gca gga aag ggc cca cta ggc ttc ctg aac        1776
   Ile Glu Lys Arg Ile Ala Ala Gly Lys Gly Pro Leu Gly Phe Leu Asn
               580                 585                 590
   ccg gtt ctg tat cgg aat gcg tgg gcg ttg aat gat att acg aac ggg        1824
   Pro Val Leu Tyr Arg Asn Ala Trp Ala Leu Asn Asp Ile Thr Asn Gly
           595                 600                 605
   tcg aat ccg ggt tgt gga acg gag ggg ttc tat act gct cct gga tgg        1872
   Ser Asn Pro Gly Cys Gly Thr Glu Gly Phe Tyr Thr Ala Pro Gly Trp
       610                 615                 620
   gat ccc gtc acc ggt ctc gga acg cct aac ttc ccg aaa ttg ctg gac        1920
   Asp Pro Val Thr Gly Leu Gly Thr Pro Asn Phe Pro Lys Leu Leu Asp
   625 630 635 640
   gtg ttc ctg aac ctc ccg taa 1941
   Val Phe Leu Asn Leu Pro
   645
<210> 49
   <211> 646
   <212> PRT
   <213> Aspergillus niger
<400> 49
   Met Lys Phe Phe Ser Thr Ile Cys Ser Leu Thr Leu Ala Val Ser Ala
   1               5                   10                  15
   Leu Ala Leu Pro Thr Ser Asp His Val Ile His Glu Lys Arg Ser Ala
               20                  25                  30
   Thr Pro Ser Arg Trp Glu Lys Ile Ser Arg Val Asn Gly Thr Glu His
           35                  40                  45
   Val Leu Val Arg Ile Gly Leu Thr Gln Asn Asn Leu Asp Arg Ala Tyr
       50                  55                  60
   Glu Tyr Leu Met Ser Val Ser Asp Ser Val Ser Pro Asn Tyr Gly Lys
   65                  70                  75                  80
   Phe Trp Thr Pro Glu Glu Val His Ser Thr Phe Ala Pro Ser Asn Glu
                   85                  90                  95
   Thr Val Asn Ala Val Arg Asn Trp Leu Ile Glu Ser Gly Val Asp Glu
               100                 105                 110
   Ser Arg Leu Val His Thr Lys Asn Gln Gly Trp Ile Val Phe Asp Ala
           115                 120                 125
   Thr Thr Lys Glu Ala Glu Asn Leu Leu His Thr Lys Tyr Tyr His Tyr
       130                 135                 140
   Thr Asp Arg Ile Ser Gly Phe Lys Thr Leu Ala Ala Glu Glu Tyr Arg
   145                 150                 155                 160
   Val Pro Gln Lys Ile Gln Gln His Ile Asp Phe Ile Lys Pro Gly Val
                   165                 170                 175
   Leu Leu Pro Leu Thr Ser Lys Gly Pro Ser Ala Lys His Thr Lys Lys
               180                 185                 190
   Tyr Lys Pro Leu Lys Gln Thr Ser Val Asn Ala Thr Ser Leu Thr Thr
           195                 200                 205
   Cys Asp Glu Val Ile Thr Pro Ala Cys Val Ala Ala Leu Tyr Lys Ile
   210 215 220
   Pro His Ala Ser Gly Asn Val Ser Ala Ser Asn Ser Leu Gly Ile Phe
   225 230 235 240
   Glu Glu Gly Asp Tyr Tyr Ala Gln Glu Asp Leu Asp Leu Phe Phe Arg
   245 250 255
   Asn Phe Thr Pro Tyr Ile Pro Lys Gly Thr His Pro Lys Pro Ala Phe
   260 265 270
   Ile Asp Gly Ala Ser Ala Pro Val Ser Val Ala Asp Ala Gly Ala Glu
   275 280 285
   Ser Asp Leu Asp Phe Gln Leu Ala Tyr Pro Ile Val Tyr Pro Gln Thr
   290 295 300
   Ile Thr Leu Tyr Gln Thr Asp Asp Tyr Asp Tyr Ala Ser Gly Glu Val
   305 310 315 320
   Glu Thr Asp Gly Phe Phe Asn Thr Phe Leu Asp Ala Val Asp Gly Ser
   325 330 335
   Tyr Cys Thr Tyr Cys Ala Tyr Gly Glu Cys Gly Asp Ser Pro Thr Leu
   340 345 350
   Asp Pro Thr Tyr Pro Asp Asn Ser Thr Gly Gly Tyr Lys Gly Gln Leu
   355 360 365
   Met Cys Gly Val Tyr Lys Pro Thr Asn Val Ile Ser Val Ser Tyr Gly
   370 375 380
   Gly Gln Glu Ala Asp Leu Pro Ala Tyr Tyr Gln Gln Arg Gln Cys Asn
   385 390 395 400
   Glu Phe Leu Lys Leu Gly Leu Gln Gly Ile Ser Ile Leu Phe Ala Ser
   405 410 415
   Gly Asp Asp Gly Val Ala Gly Pro Pro Gly Asp Asp Ser Thr Asn Gly
   420 425 430
   Cys Leu Gly Asn Gly Thr Ile Phe Ser Pro Ala Phe Pro Asn Ser Cys
   435 440 445
   Pro Trp Val Thr Asn Val Gly Ala Thr Lys Leu Tyr Pro Gly Lys Thr
   450 455 460
   Ile Ala Asp Gly Glu Ser Ala Val Val Asp Pro Ala Gly His Pro Tyr
   465 470 475 480
   Ser Val Ala Phe Ser Ser Gly Gly Gly Phe Ser Asn Ile Tyr Thr Ile
   485 490 495
   Pro Asp Tyr Gln Ala Glu Ala Val Ala Glu Tyr Phe Lys Lys His Asn
   500 505 510
   Pro Pro Tyr Pro Tyr Tyr Glu Gly Asn Ala Ser Phe Gly Lys Asn Gly
   515 520 525
   Gly Val Tyr Asn Arg Leu Gly Arg Gly Tyr Pro Asp Val Ala Ala Asn
   530 535 540
   Gly Asp Asn Ile Ala Glu Tyr Asn Ala Gly Glu Phe Ile Leu Glu Gly
   545 550 555 560
   Gly Thr Ser Ala Ser Thr Pro Ile Phe Ser Ser Val Ile Asn Arg Ile
   565 570 575
   Ile Glu Lys Arg Ile Ala Ala Gly Lys Gly Pro Leu Gly Phe Leu Asn
   580 585 590
   Pro Val Leu Tyr Arg Asn Ala Trp Ala Leu Asn Asp Ile Thr Asn Gly
   595 600 605
   Ser Asn Pro Gly Cys Gly Thr Glu Gly Phe Tyr Thr Ala Pro Gly Trp
   610 615 620
   Asp Pro Val Thr Gly Leu Gly Thr Pro Asn Phe Pro Lys Leu Leu Asp
   625 630 635 640
   Val Phe Leu Asn Leu Pro
   645
<210> 50
   <211> 3468
   <212> DNA
   <213> Aspergillus niger
<400> 50
   ggtgcgaaag ggagcatgct cccggcgcaa aacaatctca gcatgcgttc gatcactcct      60
   gattacagct tgaacacatt cccgtctcta gcacctgcca ctcgggcaaa atctccccca     120
   acgccatcat ttcctactct gcagccttcg ccttcttcat cgtggagcat accaaacccc     180
   gcaactaatc agcaatcgac tcagagatct aatgtttcgg gtatttctct gggagcgtta     240
   gcgaacatga agacacaggc tcctatgcct agccaagcca ctcaagctgc tcccaattac     300
   tctgcatttt caatccctcc gcctccgtcg ggccacagct ccgttggatc gtttggcagt     360
   cccggaggac tagctaagcc ggggggctcg gcttttggta caactgcctc ctccaatgcg 420
   ttcctaaaca acagtgcgcc acaagcaaca ccaaagcaag gcctcgataa gtacgagagc 480
   ttgctgtgaa ccccgggggt aaggttatac aatggtttca accgcatatt ttacaaatta 540
   tagccatcag gctctgatac tctttgggat ttccccttta tttcgttttg tctttctagc 600
   atagtcaccg gttatgttgt gtgtacagca tcatgcatga ttcccccatg gtttcaggcg 660
   tagttcaaaa tgggcttcaa aaagttttca tttcctccat tgtctaagtt ggctccatat 720
   ggagcaaaag aagggatgtg acatatctag tatactacat catcagacta catcgtggta 780
   atggagcatg aggagagggc agtgttggtg tgagttgtct gtggtggagc aagagtcatg 840
   gaagcgataa gtgtaggctc cacttttttt gttgccaagc aacccgatag tagattgata 900
   ctattccatt cgactgcaat ctaggttcct ttccccgacg gtgacccgac gtagcagtga 960
   cggagattct gggtacccac ggccaccaac agctctccag gaatgcaacc tcctcttcga 1020
   ggcgccttga gacgctttcc gtcggctcag cgttttgtcc tcccccaccg cccctcgtcc 1080
   ctgctgcccc tccgtcatcg tctgcctcct ctccacctca cccgctgctc ttcctcgtct 1140
   gtagcaaata cccttccttc atcctccgat tccctgattc acccccctca ccaagccaca 1200
   atggacgccc aggtcaagca gcactacctg gccgactctc cgccaaccgt ggttcgcttg 1260
   gaagtcaagt ctcactttga caatctcacg gacccgaagc tgcggaaata cgcccattat 1320
   ttgagcaggt gcgcctaaat ccctatagcc catacatttg ctagtatttc ggtgtcatgt 1380
   ggcgtctcgc taacatgcat gtgtgtgtag agctgccttt gagggaactc gtatcaccct 1440
   ccgtcaggtc tcgccggagt ccgagcctat ttacgacctc attctcgagc ttcatcgggc 1500
   ctgtgatggg aactggtcag aacttgctca gaagaccaat gtcagcgatg agcatctacg 1560
   ctacttcttg gagtatgcaa cccaatttct tgggaattgt ggtaactaca agggtttcgg 1620
   agactcgaaa ttcattcccc gactcccggt tgaggctttc caggctttag cttcggccac 1680
   gccaaagaca aaggctgcct ttgacttggc caactctact ggtggtggca tctacgagac 1740
   tgatgagcag tcccgtatgc acctcggcta cccggagggg ggacacatga caacatacta 1800
   ccccgactca ccgtctatca caaaggatga aatcacggct atcggagatc tgatggaggc 1860
   gaaaggattg ccgttggaga acacaagact caagaagacc gagtccggtg acttccaact 1920
   attgattgca tcgggtgttt cctcaccacc cgtgcgggat agagatcttg gcgatgccga 1980
   gatcttcgaa ttggatggaa agctcaaggg aaagacgctt cgtctcgtct ttggtgacta 2040
   tcgggaggaa atggcgaagg tcgctcacag cgtaaagcag gcgggactca atgcagcaaa 2100
   tgagaaccag aagcggatgt tggatgcata tgccatgtca ttcggctctg gctcgattga 2160
   agcattcaag gagagtcaga ggatctgggt gaaggaccag aagccggctc tagagacgaa 2220
   tctgggcttt gtcgaaacct acagagaccc ccacggtgtg agaggagaat gggaaggatt 2280
   tgttgctctg gtaagcgtca cttcagatct caagacatat cgctcggaca gctaacagag 2340
   ctcccaaaac aggtcaactt ggagcggaca cgtgcttttg gaaaattggt ggacagtgcc 2400
   gaagccatga tccccaagct tccttggggt aaggattttg agaaggacaa gttcctgagt 2460
   cctgacttca cctccctgga agtgctgagc ttccagtctt ctggtatccc ggctggtatc 2520
   aatttgccta attacgatga tatccggcaa aaccttggat ttaagaacgt ctccctcggc 2580
   aatgtcctta gcgcaaaggc tcctaatgag cctgtgcctt tcattgcgga gaaggatctg 2640
   gaggtctacc gtagatgccg tgatgctgcg tttgaggttc aggtcggcat tcatgaattg 2700
   cttggtcatg gaaccggaaa gttgctccag gaaacagccc ctggggagta caatttcgat 2760
   gtctccaatc cccccattag cccagtgacg aacaagccta tctcatcctg gtacaagcct 2820
   gggcagactt ggggctcggt gtttggagca atgtcttctt cctatgaaga gtgccgcgcg 2880
   gagtgtgtcg ccatggcgct cagctgcgac tttagcatcc tgcagctgtt tggtttcggc 2940
   gacggtaagg aagatctggc gaacgaggcc ggtgacgttc tttttgcagg atacctgcag 3000
   atggctcgtg ctggtctcgt ggctctggag ttctgggatc ccaagacgca gaagtgggga 3060
   caggcccata tgcaggcccg atacagtatc ctacgtacct tcctcgatgc tggaggcgaa 3120
   tttgtcaagc tctcatacac taaggatgac ctatccgatc tggagatcca cctggaccgt 3180
   tccaagattc tgactcatgg tcgtcctgct gtggagaagt acctgcagaa attgcatgtc 3240
   tacaagagta ctgcagactt tgaagcaggc aagaagctct atgacgatat cacatcagtc 3300
   gatgagtggt ggggtaccaa ggtgcgcgag attgttctca agaacaaaat ccctcgcaag 3360
   gtcttcgtgc aaggcaacac tatcttgaac ggggatgaag taaccctcaa ggagtacgag 3420
   cctacacttg aaggaatgat ccagagcttt gtggagcgta atgtctaa 3468
<210> 51
   <211> 2124
   <212> DNA
   <213> Aspergillus niger
<220>
   <221> CDS
   <222> (1) .. (2124)
<400> 51
   atg gac gcc cag gtc aag cag cac tac ctg gcc gac tct ccg cca acc        48
   Met Asp Ala Gln Val Lys Gln His Tyr Leu Ala Asp Ser Pro Pro Thr
   1               5                   10                  15
   gtg gtt cgc ttg gaa gtc aag tct cac ttt gac aat ctc acg gac ccg        96
   Val Val Arg Leu Glu Val Lys Ser His Phe Asp Asn Leu Thr Asp Pro
               20                  25                  30
   aag ctg cgg aaa tac gcc cat tat ttg agc aga gct gcc ttt gag gga       144
   Lys Leu Arg Lys Tyr Ala His Tyr Leu Ser Arg Ala Ala Phe Glu Gly
           35                  40                  45
   act cgt atc acc ctc cgt cag gtc tcg ccg gag tcc gag cct att tac       192
   Thr Arg Ile Thr Leu Arg Gln Val Ser Pro Glu Ser Glu Pro Ile Tyr
       50                  55                  60
   gac ctc att ctc gag ctt cat cgg gcc tgt gat ggg aac tgg tca gaa      240
   Asp Leu Ile Leu Glu Leu His Arg Ala Cys Asp Gly Asn Trp Ser Glu
   65                  70                  75                  80
   ctt gct cag aag acc aat gtc agc gat gag cat cta cgc tac ttc ttg      288
   Leu Ala Gln Lys Thr Asn Val Ser Asp Glu His Leu Arg Tyr Phe Leu
                   85                  90                  95
   gag tat gca acc caa ttt ctt ggg aat tgt ggt aac tac aag ggt ttc      336
   Glu Tyr Ala Thr Gln Phe Leu Gly Asn Cys Gly Asn Tyr Lys Gly Phe
               100                 105                 110
   gga gac tcg aaa ttc att ccc cga ctc ccg gtt gag gct ttc cag gct      384
   Gly Asp Ser Lys Phe Ile Pro Arg Leu Pro Val Glu Ala Phe Gln Ala
           115                 120                 125
   tta gct tcg gcc acg cca aag aca aag gct gcc ttt gac ttg gcc aac      432
   Leu Ala Ser Ala Thr Pro Lys Thr Lys Ala Ala Phe Asp Leu Ala Asn
       130                 135                 140
   tct act ggt ggt ggc atc tac gag act gat gag cag tcc cgt atg cac      480
   Ser Thr Gly Gly Gly Ile Tyr Glu Thr Asp Glu Gln Ser Arg Met His
   145                 150                 155                 160
   ctc ggc tac ccg gag ggg gga cac atg aca aca tac tac ccc gac tca      528
   Leu Gly Tyr Pro Glu Gly Gly His Met Thr Thr Tyr Tyr Pro Asp Ser
                   165                 170                 175
   ccg tct atc aca aag gat gaa atc acg gct atc gga gat ctg atg gag      576
   Pro Ser Ile Thr Lys Asp Glu Ile Thr Ala Ile Gly Asp Leu Met Glu
               180                 185                 190
   gcg aaa gga ttg ccg ttg gag aac aca aga ctc aag aag acc gag tcc      624
   Ala Lys Gly Leu Pro Leu Glu Asn Thr Arg Leu Lys Lys Thr Glu Ser
           195                 200                 205
   ggt gac ttc caa cta ttg att gca tcg ggt gtt tcc tca cca ccc gtg      672
   Gly Asp Phe Gln Leu Leu Ile Ala Ser Gly Val Ser Ser Pro Pro Val
       210                 215                 220
   cgg gat aga gat ctt ggc gat gcc gag atc ttc gaa ttg gat gga aag      720
   Arg Asp Arg Asp Leu Gly Asp Ala Glu Ile Phe Glu Leu Asp Gly Lys
   225                 230                 235                 240
   ctc aag gga aag acg ctt cgt ctc gtc ttt ggt gac tat cgg gag gaa      768
   Leu Lys Gly Lys Thr Leu Arg Leu Val Phe Gly Asp Tyr Arg Glu Glu
                   245                 250                 255
   atg gcg aag gtc gct cac agc gta aag cag gcg gga ctc aat gca gca      816
   Met Ala Lys Val Ala His Ser Val Lys Gln Ala Gly Leu Asn Ala Ala
               260                 265                 270
   aat gag aac cag aag cgg atg ttg gat gca tat gcc atg tca ttc ggc      864
   Asn Glu Asn Gln Lys Arg Met Leu Asp Ala Tyr Ala Met Ser Phe Gly
           275                 280                 285
   tct ggc tcg att gaa gca ttc aag gag agt cag agg atc tgg gtg aag      912
   Ser Gly Ser Ile Glu Ala Phe Lys Glu Ser Gln Arg Ile Trp Val Lys
       290                 295                 300
   gac cag aag ccg gct cta gag acg aat ctg ggc ttt gtc gaa acc tac      960
   Asp Gln Lys Pro Ala Leu Glu Thr Asn Leu Gly Phe Val Glu Thr Tyr
   305                 310                 315                 320
   aga gac ccc cac ggt gtg aga gga gaa tgg gaa gga ttt gtt gct ctg      1008
   Arg Asp Pro His Gly Val Arg Gly Glu Trp Glu Gly Phe Val Ala Leu
                   325                 330                 335
   gtc aac ttg gag cgg aca cgt gct ttt gga aaa ttg gtg gac agt gcc      1056
   Val Asn Leu Glu Arg Thr Arg Ala Phe Gly Lys Leu Val Asp Ser Ala
               340                 345                 350
   gaa gcc atg atc ccc aag ctt cct tgg ggt aag gat ttt gag aag gac      1104
   Glu Ala Met Ile Pro Lys Leu Pro Trp Gly Lys Asp Phe Glu Lys Asp
           355                 360                 365
   aag ttc ctg agt cct gac ttc acc tcc ctg gaa gtg ctg agc ttc cag      1152
   Lys Phe Leu Ser Pro Asp Phe Thr Ser Leu Glu Val Leu Ser Phe Gln
       370                 375                 380
   tct tct ggt atc ccg gct ggt atc aat ttg cct aat tac gat gat atc      1200
   Ser Ser Gly Ile Pro Ala Gly Ile Asn Leu Pro Asn Tyr Asp Asp Ile
   385                 390                 395                 400
   cgg caa aac ctt gga ttt aag aac gtc tcc ctc ggc aat gtc ctt agc      1248
   Arg Gln Asn Leu Gly Phe Lys Asn Val Ser Leu Gly Asn Val Leu Ser
                   405                 410                 415
   gca aag gct cct aat gag cct gtg cct ttc att gcg gag aag gat ctg      1296
   Ala Lys Ala Pro Asn Glu Pro Val Pro Phe Ile Ala Glu Lys Asp Leu
               420                 425                 430
   gag gtc tac cgt aga tgc cgt gat gct gcg ttt gag gtt cag gtc ggc      1344
   Glu Val Tyr Arg Arg Cys Arg Asp Ala Ala Phe Glu Val Gln Val Gly
           435                 440                 445
   att cat gaa ttg ctt ggt cat gga acc gga aag ttg ctc cag gaa aca      1392
   Ile His Glu Leu Leu Gly His Gly Thr Gly Lys Leu Leu Gln Glu Thr
       450                 455                 460
   gcc cct ggg gag tac aat ttc gat gtc tcc aat ccc ccc att agc cca      1440
   Ala Pro Gly Glu Tyr Asn Phe Asp Val Ser Asn Pro Pro Ile Ser Pro
   465                 470                 475                 480
   gtg acg aac aag cct atc tca tcc tgg tac aag cct ggg cag act tgg      1488
   Val Thr Asn Lys Pro Ile Ser Ser Trp Tyr Lys Pro Gly Gln Thr Trp
                   485                 490                 495
   ggc tcg gtg ttt gga gca atg tct tct tcc tat gaa gag tgc cgc gcg      1536
   Gly Ser Val Phe Gly Ala Met Ser Ser Ser Tyr Glu Glu Cys Arg Ala
               500                 505                 510
   gag tgt gtc gcc atg gcg ctc agc tgc gac ttt agc atc ctg cag ctg      1584
   Glu Cys Val Ala Met Ala Leu Ser Cys Asp Phe Ser Ile Leu Gln Leu
           515                 520                 525
   ttt ggt ttc ggc gac ggt aag gaa gat ctg gcg aac gag gcc ggt gac      1632
   Phe Gly Phe Gly Asp Gly Lys Glu Asp Leu Ala Asn Glu Ala Gly Asp
       530                 535                 540
   gtt ctt ttt gca gga tac ctg cag atg gct cgt gct ggt ctc gtg gct      1680
   Val Leu Phe Ala Gly Tyr Leu Gln Met Ala Arg Ala Gly Leu Val Ala
   545                 550                 555                 560
   ctg gag ttc tgg gat ccc aag acg cag aag tgg gga cag gcc cat atg      1728
   Leu Glu Phe Trp Asp Pro Lys Thr Gln Lys Trp Gly Gln Ala His Met
                   565                 570                 575
   cag gcc cga tac agt atc cta cgt acc ttc ctc gat gct gga ggc gaa      1776
   Gln Ala Arg Tyr Ser Ile Leu Arg Thr Phe Leu Asp Ala Gly Gly Glu
               580                 585                 590
   ttt gtc aag ctc tca tac act aag gat gac cta tcc gat ctg gag atc      1824
   Phe Val Lys Leu Ser Tyr Thr Lys Asp Asp Leu Ser Asp Leu Glu Ile
           595                 600                 605
   cac ctg gac cgt tcc aag att ctg act cat ggt cgt cct gct gtg gag      1872
   His Leu Asp Arg Ser Lys Ile Leu Thr His Gly Arg Pro Ala Val Glu
       610                 615                 620
   aag tac ctg cag aaa ttg cat gtc tac aag agt act gca gac ttt gaa      1920
   Lys Tyr Leu Gln Lys Leu His Val Tyr Lys Ser Thr Ala Asp Phe Glu
   625                 630                 635                 640
   gca ggc aag aag ctc tat gac gat atc aca tca gtc gat gag tgg tgg      1968
   Ala Gly Lys Lys Leu Tyr Asp Asp Ile Thr Ser Val Asp Glu Trp Trp
                   645                 650                 655
   ggt acc aag gtg cgc gag att gtt ctc aag aac aaa atc cct cgc aag      2016
   Gly Thr Lys Val Arg Glu Ile Val Leu Lys Asn Lys Ile Pro Arg Lys
               660                 665                 670
   gtc ttc gtg caa ggc aac act atc ttg aac ggg gat gaa gta acc ctc      2064
   Val Phe Val Gln Gly Asn Thr Ile Leu Asn Gly Asp Glu Val Thr Leu
           675                 680                 685
   aag gag tac gag cct aca ctt gaa gga atg atc cag agc ttt gtg gag      2112
   Lys Glu Tyr Glu Pro Thr Leu Glu Gly Met Ile Gln Ser Phe Val Glu
       690                 695                 700
   cgt aat gtc taa                                                      2124
   Arg Asn Val
   705
<210> 52
   <211> 707
   <212> PRT
   <213> Aspergillus niger
<400> 52
   Met Asp Ala Gln Val Lys Gln His Tyr Leu Ala Asp Ser Pro Pro Thr
   1               5                   10                  15
   Val Val Arg Leu Glu Val Lys Ser His Phe Asp Asn Leu Thr Asp Pro
               20                  25                  30
   Lys Leu Arg Lys Tyr Ala His Tyr Leu Ser Arg Ala Ala Phe Glu Gly
           35                  40                  45
   Thr Arg Ile Thr Leu Arg Gln Val Ser Pro Glu Ser Glu Pro Ile Tyr
       50                  55                  60
   Asp Leu Ile Leu Glu Leu His Arg Ala Cys Asp Gly Asn Trp Ser Glu
   65                  70                  75                  80
   Leu Ala Gln Lys Thr Asn Val Ser Asp Glu His Leu Arg Tyr Phe Leu
   85 90 95
   Glu Tyr Ala Thr Gln Phe Leu Gly Asn Cys Gly Asn Tyr Lys Gly Phe
   100 105 110
   Gly Asp Ser Lys Phe Ile Pro Arg Leu Pro Val Glu Ala Phe Gln Ala
   115 120 125
   Leu Ala Ser Ala Thr Pro Lys Thr Lys Ala Ala Phe Asp Leu Ala Asn
   130 135 140
   Ser Thr Gly Gly Gly Ile Tyr Glu Thr Asp Glu Gln Ser Arg Met His
   145 150 155 160
   Leu Gly Tyr Pro Glu Gly Gly His Met Thr Thr Tyr Tyr Pro Asp Ser
   165 170 175
   Pro Ser Ile Thr Lys Asp Glu Ile Thr Ala Ile Gly Asp Leu Met Glu
   180 185 190
   Ala Lys Gly Leu Pro Leu Glu Asn Thr Arg Leu Lys Lys Thr Glu Ser
   195 200 205
   Gly Asp Phe Gln Leu Leu Ile Ala Ser Gly Val Ser Ser Pro Pro Val
   210 215 220
   Arg Asp Arg Asp Leu Gly Asp Ala Glu Ile Phe Glu Leu Asp Gly Lys
   225 230 235 240
   Leu Lys Gly Lys Thr Leu Arg Leu Val Phe Gly Asp Tyr Arg Glu Glu
   245 250 255
   Met Ala Lys Val Ala His Ser Val Lys Gln Ala Gly Leu Asn Ala Ala
   260 265 270
   Asn Glu Asn Gln Lys Arg Met Leu Asp Ala Tyr Ala Met Ser Phe Gly
   275 280 285
   Ser Gly Ser Ile Glu Ala Phe Lys Glu Ser Gln Arg Ile Trp Val Lys
   290 295 300
   Asp Gln Lys Pro Ala Leu Glu Thr Asn Leu Gly Phe Val Glu Thr Tyr
   305 310 315 320
   Arg Asp Pro His Gly Val Arg Gly Glu Trp Glu Gly Phe Val Ala Leu
   325 330 335
   Val Asn Leu Glu Arg Thr Arg Ala Phe Gly Lys Leu Val Asp Ser Ala
   340 345 350
   Glu Ala Met Ile Pro Lys Leu Pro Trp Gly Lys Asp Phe Glu Lys Asp
   355 360 365
   Lys Phe Leu Ser Pro Asp Phe Thr Ser Leu Glu Val Leu Ser Phe Gln
   370 375 380
   Ser Ser Gly Ile Pro Ala Gly Ile Asn Leu Pro Asn Tyr Asp Asp Ile
   385 390 395 400
   Arg Gln Asn Leu Gly Phe Lys Asn Val Ser Leu Gly Asn Val Leu Ser
   405 410 415
   Ala Lys Ala Pro Asn Glu Pro Val Pro Phe Ile Ala Glu Lys Asp Leu
   420 425 430
   Glu Val Tyr Arg Arg Cys Arg Asp Ala Ala Phe Glu Val Gln Val Gly
   435 440 445
   Ile His Glu Leu Leu Gly His Gly Thr Gly Lys Leu Leu Gln Glu Thr
   450 455 460
   Ala Pro Gly Glu Tyr Asn Phe Asp Val Ser Asn Pro Pro Ile Ser Pro
   465 470 475 480
   Val Thr Asn Lys Pro Ile Ser Ser Trp Tyr Lys Pro Gly Gln Thr Trp
   485 490 495
   Gly Ser Val Phe Gly Ala Met Ser Ser Ser Tyr Glu Glu Cys Arg Ala
   500 505 510
   Glu Cys Val Ala Met Ala Leu Ser Cys Asp Phe Ser Ile Leu Gln Leu
   515 520 525
   Phe Gly Phe Gly Asp Gly Lys Glu Asp Leu Ala Asn Glu Ala Gly Asp
   530 535 540
   Val Leu Phe Ala Gly Tyr Leu Gln Met Ala Arg Ala Gly Leu Val Ala
   545 550 555 560
   Leu Glu Phe Trp Asp Pro Lys Thr Gln Lys Trp Gly Gln Ala His Met
   565 570 575
   Gln Ala Arg Tyr Ser Ile Leu Arg Thr Phe Leu Asp Ala Gly Gly Glu
   580 585 590
   Phe Val Lys Leu Ser Tyr Thr Lys Asp Asp Leu Ser Asp Leu Glu Ile
   595 600 605
   His Leu Asp Arg Ser Lys Ile Leu Thr His Gly Arg Pro Ala Val Glu
   610 615 620
   Lys Tyr Leu Gln Lys Leu His Val Tyr Lys Ser Thr Ala Asp Phe Glu
   625 630 635 640
   Ala Gly Lys Lys Leu Tyr Asp Asp Ile Thr Ser Val Asp Glu Trp Trp
   645 650 655
   Gly Thr Lys Val Arg Glu Ile Val Leu Lys Asn Lys Ile Pro Arg Lys
   660 665 670
   Val Phe Val Gln Gly Asn Thr Ile Leu Asn Gly Asp Glu Val Thr Leu
   675 680 685
   Lys Glu Tyr Glu Pro Thr Leu Glu Gly Met Ile Gln Ser Phe Val Glu
   690 695 700
   Arg Asn Val
   705
<210> 53
   <211> 4009
   <212> DNA
   <213> Aspergillus niger
<400> 53
   ggagggaagg gaaaaaggga atgattgtta gggaagaaat gggtttgttt tgatagactt 60
   gctgtctttc ttcgagatac ctcttgtgtt ttattactag atttaaagct aaagctcctt 120
   tcaacaatcg acgagatatt gctgaatact gttcttgaac atagaatcat aaatgtgcca 180
   ttgatataga tttcttaaat aattattgta tagaatatgt atgtcccagc cctctatgga 240
   agcccattca ccggaagaaa gcatcagatg cctctaatca tgaacccctc aaactggctg 300
   gtggaccgtc ttgcaagcca ctgctcccga aggagcagct cgacagccaa ctgccagata 360
   gaggaatatc tacccttgaa ggcaaacctg gcacccggct gtatggagaa gacctgcgtg 420
   aaagaatgta atatgtatgg agaaaaagaa ggaaaaggaa aagggacgca gaaatgggag 480
   aaaagatggg tgacatgtga tgcggagaaa ccctggtcga gtagccctta ttgacttcca 540
   gaagaggaaa ggcgttgggc caggggggag ctgattgcct gatttagtag gctactcagt 600
   tttggctgac tagttggtca gatgactgga tcgtgttagt catagtaaga ttaccgcctg 660
   ccggattcag acggacgacc gacggaattc ccgcggcaaa aagggaactt ttctttccct 720
   tcgagattgg atgaattgtt gatggattga ttgaaggaga gaataacctc attgtatcag 780
   taatcttttt ggtcattgtt gtcttttgat ttgtctacta tccgctgtat cattttgggt 840
   tgtttatctg ggtggggact gcgtgtgtgt gtctcttctt tgtcaatctg tcttttctct 900
   ctatcaagac tcgttcccat gcgtcgcttt ccctcctccg cacggctccc gctgagcaac 960
   agcagcaaca gaagggcagc tgctgctccc ctccgcttcc accgactctc tgctcttccc 1020
   cgcccctctt ctacttctac ttttaacttc cccactgctt ctttatctac tactactacg 1080
   ccctcctctc tcaccttctt ctcctcccct tcttcttcta ctgtccctcc tctccatcgc 1140
   tcttctcttc gtcgtgctcc cctctctcaa gcttccaaac gttactgttc ctatcgcagg 1200
   atgtgtggtg ctcgacgcgg agaggtgacg ggctcgacca acgtccccgg tggtcgtgag 1260
   gtcttgccca ccaacgtcaa gccccttcac tatgacctga ccctcgaacc taactttgcc 1320
   aacttctcct atgacggtac cgtcgtcatt gagtacgttc atgctcttcc ctacccaccc 1380
   atgaacccta gctggttacc ccgccatctg gagcagtagc taactctccg ggaatatagt 1440
   ctcgacgtcg ctgaagatac cacctccatc tccctcaact ccaacgagat caagatccac 1500
   aatgccgtcg tctcctccca aggcgccgtc gtggcctcca accccgagat caccctcaac 1560
   caggaccagc aggttgcgac catcaagttc gccgacacta tccccgccgg ttcctccgcc 1620
   cagctgaagc tgaccttcac tggtgagctc aacgacaaca tggccggttt ctaccgctcc 1680
   tcctacaagg ctgccgatgg tcagactaag tacattgcca cgacccagat ggagcctact 1740
   gatgcccgcc gcgccttccc ctgcttcgac gagcccgctt tgaaggccaa gttcaccgtg 1800
   accctggtgg cagacaagag catgacatgt ctgagcaaca tggacgtcgc cagtgagacc 1860
   gaggttgccg gcggcaagaa ggctgtgaag ttcaacacct ccccggtcat gtcgacttac 1920
   ctgctcgcct tcattgtcgg ccacctcaac tacatcgaga cgaaggcttt ccgcgtcccc 1980
   atccgtgtct atgctacccc ggatcaggat attgagcacg gtcgcttctc gctggacctg 2040
   gccgcccgca ctctggcctt ttacgagaag gcgttcgata acgagttccc tctgcccaag 2100
   atggacatgg tcgccgtgcc cgacttcagc gctggtgcca tggagaactg gggtctgatc 2160
   acctaccgtg tcgttgacgt gctgttggat gagaagacca gcggtgccgc ccgcaaggag 2220
   cgtattgccg agaccgtcca gcacgagctg gcccaccagt ggttcggtaa cttggtcacc 2280
   atggacttct gggatggcct ctggctcaac gagggtttcg ccacctggat gtcctggtac 2340
   tcttgcaaca gcttcttccc cgagtggaag gtctggcaga cgtatgtcat cgacaccctg 2400
   cagggcgccc tgtccttgga ttcgctgcgc agcagtcacc ctattgaggt gcccgtgaag 2460
   cgtgccgacg agatcaacca gatcttcgac gccatctcct actccaaggg atcctccgtt 2520
   ctgcgcatga tctctaagta catgggtgag gatgtcttca tccagggtgt gcgcgactac 2580
   atcaagaagc acgcctacgg aaacacccag acgggtgatc tgtgggccgc cctggctaac 2640
   gccagtggca agcccgtgga ggaggtcatg gacatctgga ccaagaaagt cggtttcccc 2700
   gttgtgactg tctcggagaa ccccagcaac tccaccatca agctgaagca gaaccgtttc 2760
   ctgcgcaccg gcgatgtccg ccccgacgag gacaccacgc tctaccccgt catgctgggt 2820
   ctgcgtacca agaatggcat cgacgaggac accatgctga ccgagcgcga gggcgaattt      2880
   aaggtgcccg accttgactt cttcaagctc aacgcggacc actccgccat ctaccgcacc      2940
   tcgtacaccc cggagcgtct gtccaagctg ggtgaggcgg ccaagggcgg acttttgagc      3000
   gtcgaggacc gcgctggtat gattgccgat gccggtgcct tggccgcgtc cggcttccag      3060
   agcacctccg gcctgctgtc gctgctgaag ggcttcgaca gcgaggctga gttcatcgta      3120
   tggaacgaga tcctgactcg tgttggctct ctgcgggctg cttggctgtt cgaggatgtt      3180
   tccaccaggg acgccttgaa ggccttccag cgctctctgg tcagccacaa ggcgcatgag      3240
   ctcggatggg aattctcgga gaaggatggc cacatcctgc agcaattcaa ggctctgatg      3300
   ttcggcagtg ctggtatggc tgaggacccg gtcgtcgtca aggccgccca ggacatgttc      3360
   cagcagttcg ccgccggcga cgagaacgcc atccacccca acattcgcgg cagcgtattc      3420
   agcattgtgc tgaagaacgg tggcgagaag gagtacaacg tcgtcctcga ccgcttccgc      3480
   aatgccccca cttccgatga gaagaccacg gccctccgct gcctgggtgc cgccgaagac      3540
   cccgccctga tccagcgcac tctcgacctg gccagcggcg acgaggtcaa gaaccaggac      3600
   atctacatgc ccctgggtgg tctccgcagc caccctgcgg gtatcgaggc tcgctggtct      3660
   tggttgaaga acaactggga tgccatctac aagcgtctgc ctccgtctct cggtatgctg      3720
   ggcacggtgg tgcagctcag cacagccagc ttctgcactg aggagcagtt gaaggatgtg      3780
   caggactttt tccagagcaa ggacactaag gtaggcccca tcccttcctt tcattagtta      3840
   cacctgtgtt gttcaagttt tatgtgctaa ctattttctc gtatagggct tcgaccgtgc      3900
   cgttgagcag agtctcgact cgatccgggc caaggtcaac tgggtcaagc gtgaccgggc      3960
   ggacgtggag tcgtggctca aggccaacag ctacaatggt aagctgtaa                  4009
<210> 54
   <211> 2646
   <212> DNA
   <213> Aspergillus niger
<220>
   <221> CDS
   <222> (1)..(2646)
<400> 54
   atg tgt ggt gct cga cgc gga gag gtg acg ggc tcg acc aac gtc ccc      48
   Met Cys Gly Ala Arg Arg Gly Glu Val Thr Gly Ser Thr Asn Val Pro
   1               5                   10                  15
   ggt ggt cgt gag gtc ttg ccc acc aac gtc aag ccc ctt cac tat gac      96
   Gly Gly Arg Glu Val Leu Pro Thr Asn Val Lys Pro Leu His Tyr Asp
               20                  25                  30
   ctg acc ctc gaa cct aac ttt gcc aac ttc tcc tat gac ggt acc gtc     144
   Leu Thr Leu Glu Pro Asn Phe Ala Asn Phe Ser Tyr Asp Gly Thr Val
           35                  40                  45
   gtc att gat ctc gac gtc gct gaa gat acc acc tcc atc tcc ctc aac     192
   Val Ile Asp Leu Asp Val Ala Glu Asp Thr Thr Ser Ile Ser Leu Asn
       50                  55                  60
   tcc aac gag atc aag atc cac aat gcc gtc gtc tcc tcc caa ggc gcc      240
   Ser Asn Glu Ile Lys Ile His Asn Ala Val Val Ser Ser Gln Gly Ala
   65                  70                  75                  80
   gtc gtg gcc tcc aac ccc gag atc acc ctc aac cag gac cag cag gtt      288
   Val Val Ala Ser Asn Pro Glu Ile Thr Leu Asn Gln Asp Gln Gln Val
                   85                  90                  95
   gcg acc atc aag ttc gcc gac act atc ccc gcc ggt tcc tcc gcc cag      336
   Ala Thr Ile Lys Phe Ala Asp Thr Ile Pro Ala Gly Ser Ser Ala Gln
               100                 105                 110
   ctg aag ctg acc ttc act ggt gag ctc aac gac aac atg gcc ggt ttc      384
   Leu Lys Leu Thr Phe Thr Gly Glu Leu Asn Asp Asn Met Ala Gly Phe
           115                 120                 125
   tac cgc tcc tcc tac aag gct gcc gat ggt cag act aag tac att gcc      432
   Tyr Arg Ser Ser Tyr Lys Ala Ala Asp Gly Gln Thr Lys Tyr Ile Ala
       130                 135                 140
   acg acc cag atg gag cct act gat gcc cgc cgc gcc ttc ccc tgc ttc      480
   Thr Thr Gln Met Glu Pro Thr Asp Ala Arg Arg Ala Phe Pro Cys Phe
   145                 150                 155                 160
   gac gag ccc gct ttg aag gcc aag ttc acc gtg acc ctg gtg gca gac      528
   Asp Glu Pro Ala Leu Lys Ala Lys Phe Thr Val Thr Leu Val Ala Asp
                   165                 170                 175
   aag agc atg aca tgt ctg agc aac atg gac gtc gcc agt gag acc gag      576
   Lys Ser Met Thr Cys Leu Ser Asn Met Asp Val Ala Ser Glu Thr Glu
               180                 185                 190
   gtt gcc ggc ggc aag aag gct gtg aag ttc aac acc tcc ccg gtc atg      624
   Val Ala Gly Gly Lys Lys Ala Val Lys Phe Asn Thr Ser Pro Val Met
           195                 200                 205
   tcg act tac ctg ctc gcc ttc att gtc ggc cac ctc aac tac atc gag      672
   Ser Thr Tyr Leu Leu Ala Phe Ile Val Gly His Leu Asn Tyr Ile Glu
       210                 215                 220
   acg aag gct ttc cgc gtc ccc atc cgt gtc tat gct acc ccg gat cag      720
   Thr Lys Ala Phe Arg Val Pro Ile Arg Val Tyr Ala Thr Pro Asp Gln
   225                 230                 235                 240
   gat att gag cac ggt cgc ttc tcg ctg gac ctg gcc gcc cgc act ctg      768
   Asp Ile Glu His Gly Arg Phe Ser Leu Asp Leu Ala Ala Arg Thr Leu
                   245                 250                 255
   gcc ttt tac gag aag gcg ttc gat aac gag ttc cct ctg ccc aag atg      816
   Ala Phe Tyr Glu Lys Ala Phe Asp Asn Glu Phe Pro Leu Pro Lys Met
               260                 265                 270
   gac atg gtc gcc gtg ccc gac ttc agc gct ggt gcc atg gag aac tgg      864
   Asp Met Val Ala Val Pro Asp Phe Ser Ala Gly Ala Met Glu Asn Trp
           275                 280                 285
   ggt ctg atc acc tac cgt gtc gtt gac gtg ctg ttg gat gag aag acc      912
   Gly Leu Ile Thr Tyr Arg Val Val Asp Val Leu Leu Asp Glu Lys Thr
       290                 295                 300
   agc ggt gcc gcc cgc aag gag cgt att gcc gag acc gtc cag cac gag      960
   Ser Gly Ala Ala Arg Lys Glu Arg Ile Ala Glu Thr Val Gln His Glu
   305 310 315 320
   ctg gcc cac cag tgg ttc ggt aac ttg gtc acc atg gac ttc tgg gat 1008
   Leu Ala His Gln Trp Phe Gly Asn Leu Val Thr Met Asp Phe Trp Asp
   325 330 335
   ggc ctc tgg ctc aac gag ggt ttc gcc acc tgg atg tcc tgg tac tct 1056
   Gly Leu Trp Leu Asn Glu Gly Phe Ala Thr Trp Met Ser Trp Tyr Ser
   340 345 350
   tgc aac agc ttc ttc ccc gag tgg aag gtc tgg cag acg tat gtc atc 1104
   Cys Asn Ser Phe Phe Pro Glu Trp Lys Val Trp Gln Thr Tyr Val Ile
   355 360 365
   gac acc ctg cag ggc gcc ctg tcc ttg gat tcg ctg cgc agc agt cac 1152
   Asp Thr Leu Gln Gly Ala Leu Ser Leu Asp Ser Leu Arg Ser Ser His
   370 375 380
   cct att gag gtg ccc gtg aag cgt gcc gac gag atc aac cag atc ttc 1200
   Pro Ile Glu Val Pro Val Lys Arg Ala Asp Glu Ile Asn Gln Ile Phe
   385 390 395 400
   gac gcc atc tcc tac tcc aag gga tcc tcc gtt ctg cgc atg atc tct 1248
   Asp Ala Ile Ser Tyr Ser Lys Gly Ser Ser Val Leu Arg Met Ile Ser
   405 410 415
   aag tac atg ggt gag gat gtc ttc atc cag ggt gtg cgc gac tac atc 1296
   Lys Tyr Met Gly Glu Asp Val Phe Ile Gln Gly Val Arg Asp Tyr Ile
   420 425 430
   aag aag cac gcc tac gga aac acc cag acg ggt gat ctg tgg gcc gcc 1344
   Lys Lys His Ala Tyr Gly Asn Thr Gln Thr Gly Asp Leu Trp Ala Ala
   435 440 445
   ctg gct aac gcc agt ggc aag ccc gtg gag gag gtc atg gac atc tgg 1392
   Leu Ala Asn Ala Ser Gly Lys Pro Val Glu Glu Val Met Asp Ile Trp
   450 455 460
   acc aag aaa gtc ggt ttc ccc gtt gtg act gtc tcg gag aac ccc agc 1440
   Thr Lys Lys Val Gly Phe Pro Val Val Thr Val Ser Glu Asn Pro Ser
   465 470 475 480
   aac tcc acc atc aag ctg aag cag aac cgt ttc ctg cgc acc ggc gat 1488
   Asn Ser Thr Ile Lys Leu Lys Gln Asn Arg Phe Leu Arg Thr Gly Asp
   485 490 495
   gtc cgc ccc gac gag gac acc acg ctc tac ccc gtc atg ctg ggt ctg 1536
   Val Arg Pro Asp Glu Asp Thr Thr Leu Tyr Pro Val Met Leu Gly Leu
   500 505 510
   cgt acc aag aat ggc atc gac gag gac acc atg ctg acc gag cgc gag 1584
   Arg Thr Lys Asn Gly Ile Asp Glu Asp Thr Met Leu Thr Glu Arg Glu
   515 520 525
   ggc gaa ttt aag gtg ccc gac ctt gac ttc ttc aag ctc aac gcg gac 1632
   Gly Glu Phe Lys Val Pro Asp Leu Asp Phe Phe Lys Leu Asn Ala Asp
   530 535 540
   cac tcc gcc atc tac cgc acc tcg tac acc ccg gag cgt ctg tcc aag 1680
   His Ser Ala Ile Tyr Arg Thr Ser Tyr Thr Pro Glu Arg Leu Ser Lys
   545 550 555 560
   ctg ggt gag gcg gcc aag ggc gga ctt ttg agc gtc gag gac cgc gct 1728
   Leu Gly Glu Ala Ala Lys Gly Gly Leu Leu Ser Val Glu Asp Arg Ala
   565 570 575
   ggt atg att gcc gat gcc ggt gcc ttg gcc gcg tcc ggc ttc cag agc      1776
   Gly Met Ile Ala Asp Ala Gly Ala Leu Ala Ala Ser Gly Phe Gln Ser
               580                 585                 590
   acc tcc ggc ctg ctg tcg ctg ctg aag ggc ttc gac agc gag gct gag      1824
   Thr Ser Gly Leu Leu Ser Leu Leu Lys Gly Phe Asp Ser Glu Ala Glu
           595                 600                 605
   ttc atc gta tgg aac gag atc ctg act cgt gtt ggc tct ctg cgg gct      1872
   Phe Ile Val Trp Asn Glu Ile Leu Thr Arg Val Gly Ser Leu Arg Ala
       610                 615                 620
   gct tgg ctg ttc gag gat gtt tcc acc agg gac gcc ttg aag gcc ttc      1920
   Ala Trp Leu Phe Glu Asp Val Ser Thr Arg Asp Ala Leu Lys Ala Phe
   625                 630                 635                 640
   cag cgc tct ctg gtc agc cac aag gcg cat gag ctc gga tgg gaa ttc      1968
   Gln Arg Ser Leu Val Ser His Lys Ala His Glu Leu Gly Trp Glu Phe
                   645                 650                 655
   tcg gag aag gat ggc cac atc ctg cag caa ttc aag gct ctg atg ttc      2016
   Ser Glu Lys Asp Gly His Ile Leu Gln Gln Phe Lys Ala Leu Met Phe
               660                 665                 670
   ggc agt gct ggt atg gct gag gac ccg gtc gtc gtc aag gcc gcc cag      2064
   Gly Ser Ala Gly Met Ala Glu Asp Pro Val Val Val Lys Ala Ala Gln
           675                 680                 685
   gac atg ttc cag cag ttc gcc gcc ggc gac gag aac gcc atc cac ccc      2112
   Asp Met Phe Gln Gln Phe Ala Ala Gly Asp Glu Asn Ala Ile His Pro
       690                 695                 700
   aac att cgc ggc agc gta ttc agc att gtg ctg aag aac ggt ggc gag      2160
   Asn Ile Arg Gly Ser Val Phe Ser Ile Val Leu Lys Asn Gly Gly Glu
   705                 710                 715                 720
   aag gag tac aac gtc gtc ctc gac cgc ttc cgc aat gcc ccc act tcc      2208
   Lys Glu Tyr Asn Val Val Leu Asp Arg Phe Arg Asn Ala Pro Thr Ser
                   725                 730                 735
   gat gag aag acc acg gcc ctc cgc tgc ctg ggt gcc gcc gaa gac ccc      2256
   Asp Glu Lys Thr Thr Ala Leu Arg Cys Leu Gly Ala Ala Glu Asp Pro
               740                 745                 750
   gcc ctg atc cag cgc act ctc gac ctg gcc agc ggc gac gag gtc aag      2304
   Ala Leu Ile Gln Arg Thr Leu Asp Leu Ala Ser Gly Asp Glu Val Lys
           755                 760                 765
   aac cag gac atc tac atg ccc ctg ggt ggt ctc cgc agc cac cct gcg      2352
   Asn Gln Asp Ile Tyr Met Pro Leu Gly Gly Leu Arg Ser His Pro Ala
       770                 775                 780
   ggt atc gag gct cgc tgg tct tgg ttg aag aac aac tgg gat gcc atc      2400
   Gly Ile Glu Ala Arg Trp Ser Trp Leu Lys Asn Asn Trp Asp Ala Ile
   785                 790                 795                 800
   tac aag cgt ctg cct ccg tct ctc ggt atg ctg ggc acg gtg gtg cag      2448
   Tyr Lys Arg Leu Pro Pro Ser Leu Gly Met Leu Gly Thr Val Val Gln
                   805                 810                 815
   ctc agc aca gcc agc ttc tgc act gag gag cag ttg aag gat gtg cag      2496
   Leu Ser Thr Ala Ser Phe Cys Thr Glu Glu Gln Leu Lys Asp Val Gln
               820                 825                 830
   gac ttt ttc cag agc aag gac act aag ggc ttc gac cgt gcc gtt gag        2544
   Asp Phe Phe Gln Ser Lys Asp Thr Lys Gly Phe Asp Arg Ala Val Glu
           835                 840                 845
   cag agt ctc gac tcg atc cgg gcc aag gtc aac tgg gtc aag cgt gac        2592
   Gln Ser Leu Asp Ser Ile Arg Ala Lys Val Asn Trp Val Lys Arg Asp
       850                 855                 860
   cgg gcg gac gtg gag tcg tgg ctc aag gcc aac agc tac aat ggt aag        2640
   Arg Ala Asp Val Glu Ser Trp Leu Lys Ala Asn Ser Tyr Asn Gly Lys
   865                 870                 875                 880
   ctg taa                                                                2646
   Leu
<210> 55
   <211> 881
   <212> PRT
   <213> Aspergillus niger
<400> 55
   Met Cys Gly Ala Arg Arg Gly Glu Val Thr Gly Ser Thr Asn Val Pro
   1               5                   10                  15
   Gly Gly Arg Glu Val Leu Pro Thr Asn Val Lys Pro Leu His Tyr Asp
               20                  25                  30
   Leu Thr Leu Glu Pro Asn Phe Ala Asn Phe Ser Tyr Asp Gly Thr Val
           35                  40                  45
   Val Ile Asp Leu Asp Val Ala Glu Asp Thr Thr Ser Ile Ser Leu Asn
       50                  55                  60
   Ser Asn Glu Ile Lys Ile His Asn Ala Val Val Ser Ser Gln Gly Ala
   65                  70                  75                  80
   Val Val Ala Ser Asn Pro Glu Ile Thr Leu Asn Gln Asp Gln Gln Val
                   85                  90                  95
   Ala Thr Ile Lys Phe Ala Asp Thr Ile Pro Ala Gly Ser Ser Ala Gln
               100                 105                 110
   Leu Lys Leu Thr Phe Thr Gly Glu Leu Asn Asp Asn Met Ala Gly Phe
           115                 120                 125
   Tyr Arg Ser Ser Tyr Lys Ala Ala Asp Gly Gln Thr Lys Tyr Ile Ala
       130                 135                 140
   Thr Thr Gln Met Glu Pro Thr Asp Ala Arg Arg Ala Phe Pro Cys Phe
   145                 150                 155                 160
   Asp Glu Pro Ala Leu Lys Ala Lys Phe Thr Val Thr Leu Val Ala Asp
   165 170 175
   Lys Ser Met Thr Cys Leu Ser Asn Met Asp Val Ala Ser Glu Thr Glu
   180 185 190
   Val Ala Gly Gly Lys Lys Ala Val Lys Phe Asn Thr Ser Pro Val Met
   195 200 205
   Ser Thr Tyr Leu Leu Ala Phe Ile Val Gly His Leu Asn Tyr Ile Glu
   210 215 220
   Thr Lys Ala Phe Arg Val Pro Ile Arg Val Tyr Ala Thr Pro Asp Gln
   225 230 235 240
   Asp Ile Glu His Gly Arg Phe Ser Leu Asp Leu Ala Ala Arg Thr Leu
   245 250 255
   Ala Phe Tyr Glu Lys Ala Phe Asp Asn Glu Phe Pro Leu Pro Lys Met
   260 265 270
   Asp Met Val Ala Val Pro Asp Phe Ser Ala Gly Ala Met Glu Asn Trp
   275 280 285
   Gly Leu Ile Thr Tyr Arg Val Val Asp Val Leu Leu Asp Glu Lys Thr
   290 295 300
   Ser Gly Ala Ala Arg Lys Glu Arg Ile Ala Glu Thr Val Gln His Glu
   305 310 315 320
   Leu Ala His Gln Trp Phe Gly Asn Leu Val Thr Met Asp Phe Trp Asp
   325 330 335
   Gly Leu Trp Leu Asn Glu Gly Phe Ala Thr Trp Met Ser Trp Tyr Ser
   340 345 350
   Cys Asn Ser Phe Phe Pro Glu Trp Lys Val Trp Gln Thr Tyr Val Ile
   355 360 365
   Asp Thr Leu Gln Gly Ala Leu Ser Leu Asp Ser Leu Arg Ser Ser His
   370 375 380
   Pro Ile Glu Val Pro Val Lys Arg Ala Asp Glu Ile Asn Gln Ile Phe
   385 390 395 400
   Asp Ala Ile Ser Tyr Ser Lys Gly Ser Ser Val Leu Arg Met Ile Ser
   405 410 415
   Lys Tyr Met Gly Glu Asp Val Phe Ile Gln Gly Val Arg Asp Tyr Ile
   420 425 430
   Lys Lys His Ala Tyr Gly Asn Thr Gln Thr Gly Asp Leu Trp Ala Ala
   435 440 445
   Leu Ala Asn Ala Ser Gly Lys Pro Val Glu Glu Val Met Asp Ile Trp
   450 455 460
   Thr Lys Lys Val Gly Phe Pro Val Val Thr Val Ser Glu Asn Pro Ser
   465 470 475 480
   Asn Ser Thr Ile Lys Leu Lys Gln Asn Arg Phe Leu Arg Thr Gly Asp
   485 490 495
   Val Arg Pro Asp Glu Asp Thr Thr Leu Tyr Pro Val Met Leu Gly Leu
   500 505 510
   Arg Thr Lys Asn Gly Ile Asp Glu Asp Thr Met Leu Thr Glu Arg Glu
   515 520 525
   Gly Glu Phe Lys Val Pro Asp Leu Asp Phe Phe Lys Leu Asn Ala Asp
   530 535 540
   His Ser Ala Ile Tyr Arg Thr Ser Tyr Thr Pro Glu Arg Leu Ser Lys
   545 550 555 560
   Leu Gly Glu Ala Ala Lys Gly Gly Leu Leu Ser Val Glu Asp Arg Ala
   565 570 575
   Gly Met Ile Ala Asp Ala Gly Ala Leu Ala Ala Ser Gly Phe Gln Ser
   580 585 590
   Thr Ser Gly Leu Leu Ser Leu Leu Lys Gly Phe Asp Ser Glu Ala Glu
   595 600 605
   Phe Ile Val Trp Asn Glu Ile Leu Thr Arg Val Gly Ser Leu Arg Ala
   610 615 620
   Ala Trp Leu Phe Glu Asp Val Ser Thr Arg Asp Ala Leu Lys Ala Phe
   625 630 635 640
   Gln Arg Ser Leu Val Ser His Lys Ala His Glu Leu Gly Trp Glu Phe
   645 650 655
   Ser Glu Lys Asp Gly His Ile Leu Gln Gln Phe Lys Ala Leu Met Phe
   660 665 670
   Gly Ser Ala Gly Met Ala Glu Asp Pro Val Val Val Lys Ala Ala Gln
   675 680 685
   Asp Met Phe Gln Gln Phe Ala Ala Gly Asp Glu Asn Ala Ile His Pro
       690                 695                 700
   Asn Ile Arg Gly Ser Val Phe Ser Ile Val Leu Lys Asn Gly Gly Glu
   705                 710                 715                 720
   Lys Glu Tyr Asn Val Val Leu Asp Arg Phe Arg Asn Ala Pro Thr Ser
                   725                 730                 735
   Asp Glu Lys Thr Thr Ala Leu Arg Cys Leu Gly Ala Ala Glu Asp Pro
               740                 745                 750
   Ala Leu Ile Gln Arg Thr Leu Asp Leu Ala Ser Gly Asp Glu Val Lys
           755                 760                 765
   Asn Gln Asp Ile Tyr Met Pro Leu Gly Gly Leu Arg Ser His Pro Ala
       770                 775                 780
   Gly Ile Glu Ala Arg Trp Ser Trp Leu Lys Asn Asn Trp Asp Ala Ile
   785                 790                 795                 800
   Tyr Lys Arg Leu Pro Pro Ser Leu Gly Met Leu Gly Thr Val Val Gln
                   805                 810                 815
   Leu Ser Thr Ala Ser Phe Cys Thr Glu Glu Gln Leu Lys Asp Val Gln
               820                 825                 830
   Asp Phe Phe Gln Ser Lys Asp Thr Lys Gly Phe Asp Arg Ala Val Glu
           835                 840                 845
   Gln Ser Leu Asp Ser Ile Arg Ala Lys Val Asn Trp Val Lys Arg Asp
       850                 855                 860
   Arg Ala Asp Val Glu Ser Trp Leu Lys Ala Asn Ser Tyr Asn Gly Lys
   865                 870                 875                 880
   Leu
<210> 56
   <211> 2910
   <212> DNA
   <213> Aspergillus niger
<400> 56
   tcgttggggc gaaagggggc atgatgtaca cagcctgcct ggatgagtgg aggcgaagtg 60
   atgcgtagca ttggatatgt ccagttcctt ccaacttggt gagggaggag agtggctgcg 120
   ttgaaagcat ggtcgtagtc atgctggggg agagcaaatg gaagctcgcg tgcgacggtg 180
   agagttgggg gcggccccaa tttagcattc ctggttgtac cgaagtttcc tagcattggg 240
   ttaactggaa gtttttgtag attagggggt tcgtatctgt cgcatagtga agaggtacca 300
   acacgttctt tgtagagtct ggtgtcattg ggtgatactt gatgcttttc gacgactcct 360
   gtcagcccta actgacgctt tgggtaagac atgaaccaga aactgttgtc ctgatcatga 420
   tgtgcatttt tatggaatat actgcatatt gggagcttcg acacgattgc aatataaacg 480
   gtcggttccc atgcataata gcttcgatga tcctggtcag gataggaccg ggtttctgca 540
   ttggtggtga agctgcagaa tgcggccccc acacagcaac agggggtgca accattcact 600
   aagaatagtt gactgactcc agatcccggc tttacccgtc gagtcacgtg atagaacacg 660
   gagtatccca ggttcgatcc ctgctctgag cgaattgacc ttttgtgatt gtcaaccaag 720
   tatattgatc acgacattac tacacagtgc tgataccacc gggtggaact gtgttaaaat 780
   caatgggtga ccgaggcgtg atccgcccta aaaattcaac ggcgcagcga gtacccgcaa 840
   atgtcacgcc atcctgggct gtatgtcgga gcgaatggtt tttgcggtca ctctttctga 900
   gcgacaccag ttggctcatg agctgggaat gacgagtaga ccgaggaggt gagcacaatg 960
   gaggacacgg ggaataattg tcaattattg gcaagtgcag aaaccagcca cgccgggaga 1020
   ccgacggata acgcgacgat cgccgtccca acctcatcac accttgctgg ctggctgggc 1080
   tgattggcaa tcgccagttc caccgcttag ataaagctcg tcttgcaaag aaaccccgca 1140
   taagggtcta ggagtaataa actcggggga gtcccccgac tcttccatcg cacgaccatc 1200
   atgttccttt cgcttcccct tctggggacc ttgattgcct ccacggttgc cctcgactcc 1260
   agccccctgg agttgcgggt accacatctg caccaactca gccagctcag caagtctcac 1320
   caggcttctc tcaaccagga ttcagcaagc ttcccggtgt acaacctttc tgtgcctata 1380
   gaccatttcc acgatgaatc acgctatgag ccacacacca atgccacctt tggcctgcgc 1440
   tactggctcg acactagcca ttaccagcct ggaggacccg tttttgtgat tgccgcaggc 1500
   gagaccgatg gaagcgaccg tattcccttc ctttcacaag gggttgttac tcaattagcg 1560
   gcagcctacc atggaattgg cctcattctc gaacatcgct attacggcga gagctaccca 1620
   ttcaccaacc ttactaccga gaacatccga ttcttgacta ccgagcaggc gctcgccgac 1680
   tatgcttact ttgcctcgaa cattgtcttc cccggtctgg aggatctcga tcttacggca 1740
   gccaccactc catggatcgc ctatggcggc tcttatgctg gtgctttcgt cgctttcctc 1800
   cgcaaactct acccggagct ctactgggga gcggtgtctt cttctggcgt cacggaagcc 1860
   atcattgatt actgggaata ctacgaaccg atccgactgt atggcccatc tcagtgcatc 1920
   tccaccatac agacgtcaat cgatattgtg gatcgcattc tcatcgacca tgccgacaat 1980
   aaaaccctgg cccagcaact gaaatccgct tttgggggct cagcgaccaa cctcagtaac 2040
   caggactttg tctcctccct ctcctatggt ctggactcgt ttcagtcccg caactgggat 2100
   aagaccatcg ggacccctct cttccgcgct ttctgcaaca acatcaccaa ttcggacctg 2160
   ctgtaccccg aggccgccga acccatctct gcatccgtca agcaattggt ccaaattgca      2220
   ggctacgatc cctccaacac gaccctggtc aacaacttcc tcaactggat tggattccta      2280
   agcacctcga aagttttcgc cgactcatcc tcggagtcct catcatccaa catcacgaac      2340
   ttcaccaccc cgcagcccct cagcaaggac tccggcacca gctggaacta ccaagtctgc      2400
   gttgaatggg ggtatttcac tacaggctcc tctgtgcctt cgaccactaa gccccttatt      2460
   tctcgtcttc tcgacctccc ctacctttcg tctttctgcc cgaacacatt cggaatcaag      2520
   acaccgccag atgttcagcg aatcaaccaa ttcggcgggt tcaatttctc gtatccgcgc      2580
   gttgcgataa taggcgggct ggcggatccg tggcgcgacg cgacaccact agcggacgga      2640
   gtcgactggc agagtcgaaa gtcgtctgat agtgagccga ttattttgct ggatattccg      2700
   gcggcggatg tgtgggatgg tgtgagaggt gcggtccatc actgggatca gaacggattg      2760
   tcgggtcagc agaagaagga gggggagaag gcgcctaaag agattgttag tgtgcatgag      2820
   gaggtggtga gatttgtggg ggtgtggttg aaggagtgga aggggaaaca gtctgatgca      2880
   gatgagaggg tgtatcagac tgttttgtag                                        2910
<210> 57
   <211> 1710
   <212> DNA
   <213> Aspergillus niger
<220>
   <221> CDS
   <222> (1)..(1710)
<400> 57
   atg ttc ctt tcg ctt ccc ctt ctg ggg acc ttg att gcc tcc acg gtt        48
   Met Phe Leu Ser Leu Pro Leu Leu Gly Thr Leu Ile Ala Ser Thr Val
   1               5                   10                  15
   gcc ctc gac tcc agc ccc ctg gag ttg cgg gta cca cat ctg cac caa        96
   Ala Leu Asp Ser Ser Pro Leu Glu Leu Arg Val Pro His Leu His Gln
               20                  25                  30
   ctc agc cag ctc agc aag tct cac cag gct tct ctc aac cag gat tca       144
   Leu Ser Gln Leu Ser Lys Ser His Gln Ala Ser Leu Asn Gln Asp Ser
           35                  40                  45
   gca agc ttc ccg gtg tac aac ctt tct gtg cct ata gac cat ttc cac       192
   Ala Ser Phe Pro Val Tyr Asn Leu Ser Val Pro Ile Asp His Phe His
       50                  55                  60
   gat gaa tca cgc tat gag cca cac acc aat gcc acc ttt ggc ctg cgc       240
   Asp Glu Ser Arg Tyr Glu Pro His Thr Asn Ala Thr Phe Gly Leu Arg
   65                  70                  75                  80
   tac tgg ctc gac act agc cat tac cag cct gga gga ccc gtt ttt gtg       288
   Tyr Trp Leu Asp Thr Ser His Tyr Gln Pro Gly Gly Pro Val Phe Val
                   85                  90                  95
   att gcc gca ggc gag acc gat gga agc gac cgt att ccc ttc ctt tca       336
   Ile Ala Ala Gly Glu Thr Asp Gly Ser Asp Arg Ile Pro Phe Leu Ser
                   100                 105                 110
   caa ggg gtt gtt act caa tta gcg gca gcc tac cat gga att ggc ctc        384
   Gln Gly Val Val Thr Gln Leu Ala Ala Ala Tyr His Gly Ile Gly Leu
           115                 120                 125
   att ctc gaa cat cgc tat tac ggc gag agc tac cca ttc acc aac ctt        432
   Ile Leu Glu His Arg Tyr Tyr Gly Glu Ser Tyr Pro Phe Thr Asn Leu
       130                 135                 140
   act acc gag aac atc cga ttc ttg act acc gag cag gcg ctc gcc gac        480
   Thr Thr Glu Asn Ile Arg Phe Leu Thr Thr Glu Gln Ala Leu Ala Asp
   145                 150                 155                 160
   tat gct tac ttt gcc tcg aac att gtc ttc ccc ggt ctg gag gat ctc        528
   Tyr Ala Tyr Phe Ala Ser Asn Ile Val Phe Pro Gly Leu Glu Asp Leu
                   165                 170                 175
   gat ctt acg gca gcc acc act cca tgg atc gcc tat ggc ggc tct tat        576
   Asp Leu Thr Ala Ala Thr Thr Pro Trp Ile Ala Tyr Gly Gly Ser Tyr
               180                 185                 190
   gct ggt gct ttc gtc gct ttc ctc cgc aaa ctc tac ccg gag ctc tac        624
   Ala Gly Ala Phe Val Ala Phe Leu Arg Lys Leu Tyr Pro Glu Leu Tyr
           195                 200                 205
   tgg gga gcg gtg tct tct tct ggc gtc acg gaa gcc atc att gat tac        672
   Trp Gly Ala Val Ser Ser Ser Gly Val Thr Glu Ala Ile Ile Asp Tyr
       210                 215                 220
   tgg gaa tac tac gaa ccg atc cga ctg tat ggc cca tct cag tgc atc        720
   Trp Glu Tyr Tyr Glu Pro Ile Arg Leu Tyr Gly Pro Ser Gln Cys Ile
   225                 230                 235                 240
   tcc acc ata cag acg tca atc gat att gtg gat cgc att ctc atc gac        768
   Ser Thr Ile Gln Thr Ser Ile Asp Ile Val Asp Arg Ile Leu Ile Asp
                   245                 250                 255
   cat gcc gac aat aaa acc ctg gcc cag caa ctg aaa tcc gct ttt ggg        816
   His Ala Asp Asn Lys Thr Leu Ala Gln Gln Leu Lys Ser Ala Phe Gly
               260                 265                 270
   ggc tca gcg acc aac ctc agt aac cag gac ttt gtc tcc tcc ctc tcc        864
   Gly Ser Ala Thr Asn Leu Ser Asn Gln Asp Phe Val Ser Ser Leu Ser
           275                 280                 285
   tat ggt ctg gac tcg ttt cag tcc cgc aac tgg gat aag acc atc ggg        912
   Tyr Gly Leu Asp Ser Phe Gln Ser Arg Asn Trp Asp Lys Thr Ile Gly
       290                 295                 300
   acc cct ctc ttc cgc gct ttc tgc aac aac atc acc aat tcg gac ctg        960
   Thr Pro Leu Phe Arg Ala Phe Cys Asn Asn Ile Thr Asn Ser Asp Leu
   305                 310                 315                 320
   ctg tac ccc gag gcc gcc gaa ccc atc tct gca tcc gtc aag caa ttg       1008
   Leu Tyr Pro Glu Ala Ala Glu Pro Ile Ser Ala Ser Val Lys Gln Leu
                   325                 330                 335
   gtc caa att gca ggc tac gat ccc tcc aac acg acc ctg gtc aac aac       1056
   Val Gln Ile Ala Gly Tyr Asp Pro Ser Asn Thr Thr Leu Val Asn Asn
               340                 345                 350
   ttc ctc aac tgg att gga ttc cta agc acc tcg aaa gtt ttc gcc gac       1104
   Phe Leu Asn Trp Ile Gly Phe Leu Ser Thr Ser Lys Val Phe Ala Asp
           355                 360                 365
   tca tcc tcg gag tcc tca tca tcc aac atc acg aac ttc acc acc ccg       1152
   Ser Ser Ser Glu Ser Ser Ser Ser Asn Ile Thr Asn Phe Thr Thr Pro
       370                 375                 380
   cag ccc ctc agc aag gac tcc ggc acc agc tgg aac tac caa gtc tgc       1200
   Gln Pro Leu Ser Lys Asp Ser Gly Thr Ser Trp Asn Tyr Gln Val Cys
   385                 390                 395                 400
   gtt gaa tgg ggg tat ttc act aca ggc tcc tct gtg cct tcg acc act       1248
   Val Glu Trp Gly Tyr Phe Thr Thr Gly Ser Ser Val Pro Ser Thr Thr
                   405                 410                 415
   aag ccc ctt att tct cgt ctt ctc gac ctc ccc tac ctt tcg tct ttc       1296
   Lys Pro Leu Ile Ser Arg Leu Leu Asp Leu Pro Tyr Leu Ser Ser Phe
               420                 425                 430
   tgc ccg aac aca ttc gga atc aag aca ccg cca gat gtt cag cga atc       1344
   Cys Pro Asn Thr Phe Gly Ile Lys Thr Pro Pro Asp Val Gln Arg Ile
           435                 440                 445
   aac caa ttc ggc ggg ttc aat ttc tcg tat ccg cgc gtt gcg ata ata       1392
   Asn Gln Phe Gly Gly Phe Asn Phe Ser Tyr Pro Arg Val Ala Ile Ile
       450                 455                 460
   ggc ggg ctg gcg gat ccg tgg cgc gac gcg aca cca cta gcg gac gga       1440
   Gly Gly Leu Ala Asp Pro Trp Arg Asp Ala Thr Pro Leu Ala Asp Gly
   465                 470                 475                 480
   gtc gac tgg cag agt cga aag tcg tct gat agt gag ccg att att ttg       1488
   Val Asp Trp Gln Ser Arg Lys Ser Ser Asp Ser Glu Pro Ile Ile Leu
                   485                 490                 495
   ctg gat att ccg gcg gcg gat gtg tgg gat ggt gtg aga ggt gcg gtc       1536
   Leu Asp Ile Pro Ala Ala Asp Val Trp Asp Gly Val Arg Gly Ala Val
               500                 505                 510
   cat cac tgg gat cag aac gga ttg tcg ggt cag cag aag aag gag ggg       1584
   His His Trp Asp Gln Asn Gly Leu Ser Gly Gln Gln Lys Lys Glu Gly
           515                 520                 525
   gag aag gcg cct aaa gag att gtt agt gtg cat gag gag gtg gtg aga       1632
   Glu Lys Ala Pro Lys Glu Ile Val Ser Val His Glu Glu Val Val Arg
       530                 535                 540
   ttt gtg ggg gtg tgg ttg aag gag tgg aag ggg aaa cag tct gat gca       1680
   Phe Val Gly Val Trp Leu Lys Glu Trp Lys Gly Lys Gln Ser Asp Ala
   545                 550                 555                 560
   gat gag agg gtg tat cag act gtt ttg tag                               1710
   Asp Glu Arg Val Tyr Gln Thr Val Leu
                   565
<210> 58
   <211> 569
   <212> PRT
   <213> Aspergillus niger
<400> 58
   Met Phe Leu Ser Leu Pro Leu Leu Gly Thr Leu Ile Ala Ser Thr Val
   1               5                   10                  15
   Ala Leu Asp Ser Ser Pro Leu Glu Leu Arg Val Pro His Leu His Gln
   20 25 30
   Leu Ser Gln Leu Ser Lys Ser His Gln Ala Ser Leu Asn Gln Asp Ser
   35 40 45
   Ala Ser Phe Pro Val Tyr Asn Leu Ser Val Pro Ile Asp His Phe His
   50 55 60
   Asp Glu Ser Arg Tyr Glu Pro His Thr Asn Ala Thr Phe Gly Leu Arg
   65 70 75 80
   Tyr Trp Leu Asp Thr Ser His Tyr Gln Pro Gly Gly Pro Val Phe Val
   85 90 95
   Ile Ala Ala Gly Glu Thr Asp Gly Ser Asp Arg Ile Pro Phe Leu Ser
   100 105 110
   Gln Gly Val Val Thr Gln Leu Ala Ala Ala Tyr His Gly Ile Gly Leu
   115 120 125
   Ile Leu Glu His Arg Tyr Tyr Gly Glu Ser Tyr Pro Phe Thr Asn Leu
   130 135 140
   Thr Thr Glu Asn Ile Arg Phe Leu Thr Thr Glu Gln Ala Leu Ala Asp
   145 150 155 160
   Tyr Ala Tyr Phe Ala Ser Asn Ile Val Phe Pro Gly Leu Glu Asp Leu
   165 170 175
   Asp Leu Thr Ala Ala Thr Thr Pro Trp Ile Ala Tyr Gly Gly Ser Tyr
   180 185 190
   Ala Gly Ala Phe Val Ala Phe Leu Arg Lys Leu Tyr Pro Glu Leu Tyr
   195 200 205
   Trp Gly Ala Val Ser Ser Ser Gly Val Thr Glu Ala Ile Ile Asp Tyr
   210 215 220
   Trp Glu Tyr Tyr Glu Pro Ile Arg Leu Tyr Gly Pro Ser Gln Cys Ile
   225 230 235 240
   Ser Thr Ile Gln Thr Ser Ile Asp Ile Val Asp Arg Ile Leu Ile Asp
   245 250 255
   His Ala Asp Asn Lys Thr Leu Ala Gln Gln Leu Lys Ser Ala Phe Gly
   260 265 270
   Gly Ser Ala Thr Asn Leu Ser Asn Gln Asp Phe Val Ser Ser Leu Ser
   275 280 285
   Tyr Gly Leu Asp Ser Phe Gln Ser Arg Asn Trp Asp Lys Thr Ile Gly
   290 295 300
   Thr Pro Leu Phe Arg Ala Phe Cys Asn Asn Ile Thr Asn Ser Asp Leu
   305 310 315 320
   Leu Tyr Pro Glu Ala Ala Glu Pro Ile Ser Ala Ser Val Lys Gln Leu
   325 330 335
   Val Gln Ile Ala Gly Tyr Asp Pro Ser Asn Thr Thr Leu Val Asn Asn
   340 345 350
   Phe Leu Asn Trp Ile Gly Phe Leu Ser Thr Ser Lys Val Phe Ala Asp
   355 360 365
   Ser Ser Ser Glu Ser Ser Ser Ser Asn Ile Thr Asn Phe Thr Thr Pro
   370 375 380
   Gln Pro Leu Ser Lys Asp Ser Gly Thr Ser Trp Asn Tyr Gln Val Cys
   385 390 395 400
   Val Glu Trp Gly Tyr Phe Thr Thr Gly Ser Ser Val Pro Ser Thr Thr
   405 410 415
   Lys Pro Leu Ile Ser Arg Leu Leu Asp Leu Pro Tyr Leu Ser Ser Phe
   420 425 430
   Cys Pro Asn Thr Phe Gly Ile Lys Thr Pro Pro Asp Val Gln Arg Ile
   435 440 445
   Asn Gln Phe Gly Gly Phe Asn Phe Ser Tyr Pro Arg Val Ala Ile Ile
   450 455 460
   Gly Gly Leu Ala Asp Pro Trp Arg Asp Ala Thr Pro Leu Ala Asp Gly
   465 470 475 480
   Val Asp Trp Gln Ser Arg Lys Ser Ser Asp Ser Glu Pro Ile Ile Leu
   485 490 495
   Leu Asp Ile Pro Ala Ala Asp Val Trp Asp Gly Val Arg Gly Ala Val
   500 505 510
   His His Trp Asp Gln Asn Gly Leu Ser Gly Gln Gln Lys Lys Glu Gly
   515 520 525
   Glu Lys Ala Pro Lys Glu Ile Val Ser Val His Glu Glu Val Val Arg
   530 535 540
   Phe Val Gly Val Trp Leu Lys Glu Trp Lys Gly Lys Gln Ser Asp Ala
   545 550 555 560
   Asp Glu Arg Val Tyr Gln Thr Val Leu
   565
<210> 59
   <211> 2852
   <212> DNA
   <213> Aspergillus niger
<400> 59
   cccgtgcacg caaccttcca gatgatcgga gaagagccat cgcgggctac ttagatgtca      60
   tccgaggagg aaattcgagc ggaaagcgtg ttatctcaag cggcctctgc atccacacat     120
   gtctcgcagc ctcgaaattc ttaaaccgta cttcgctcaa actacgttgg tggaccagga     180
   aacatttgga gttcagagca agcgcaacgt cttctttctt tgctgcccga caaggggttg     240
   aacgaatcct taaggaagag atgggagtct tcgcccgacc gcagcagtgc caataagtgg     300
   gcggatatcg acgccttggc caagacaggc aagagcagca ctctcaaccc tactacactg     360
   cgagatgcca agcaagacat tgttctcgag tacacatatc cgcgcctgga ttctgaagtt     420
   agcaaaaaga tgattcactt gctaaagagt ccatttgtca ttcaccctgg cactggtcgc     480
   gtgtgtgttc cgatagacgc ccgaaaggcg gaacaattcg accccctctc ggtacctaca     540
   gtttcggaac tgcttgcgga gatcgatgcg tgggatgcgg agcatccaag caccaatgca     600
   ggtccggagg tagcagatgg cgaaggtagt gtacccgact ccgatgctag gggtagtcgc     660
   agactgcaag actatgaaaa gacgagtctg aagccgtacg tcgactattt ccgctcattc     720
   attgccgggc ttctgaagga agagcgtacg ggcaagcggg aaaggaacga cgatgcgact     780
   gaggtcaaga ccgagagcat ggagttctag acaattccaa gtctgacaat tttgtgttgt     840
   tgcgcgtttg actttggtca ttgatttgga ttagcacgtc ggatatgata tattcatggg     900
   caacgggatt ggcgtcattg tgcattgcaa aggttggaca tgctggagtt acttttcttc     960
   ttttacgtta ctagaacctt attcatcatt ctacgtaaat aatctgtctg tttttccgcc    1020
   atatcctcaa atcagtcttc ggcaaataag cctgattatc actctaagct tccttgattg    1080
   gcatgcggag ctccgacggg gaaactcagc atgtcagcct caaccccgcg cagacaatat    1140
   attaaagtaa gagagaggct ttgactcggc catctgcttc ttccatgaag ccaggcaaac    1200
   atggcagcag accacaccaa caccaacccg ttagatgagc ggttcgaaac cctcgtccag    1260
   gagacattga attactggca cgttccaggc atttcaatcg cagtagtcga tggagacaac    1320
   acctgggcca aggcaggtgt cccatacatc tccaatgatc aagataatag acagaactcc    1380
   catactaaca aacaagcaca ggggtacggc attgcgacct tcccctctga gcccgtaacc    1440
   ccttcaacgc tcttctatgc cggaagcacc acgaaagctt tcaccgcagc cttgatggcc    1500
   accttcgtcg aagacaacga cacccatccc caagtacact gggacacgcc catcagccac 1560
   ctcatccgcg acgacttcgt cctacacgac gagcacacaa ccgaacacac caccatcgaa 1620
   gatgccctat cccatcgctc aggcctcccc cgtcacgatc aagcctacgg caaccgtctc 1680
   acccgcgaca aagccgtacg aacaatagtg cgtcagttgc gccatctccc actaaccgct 1740
   cctccgcgca ccaaactcca atactgcaat ctcatgtacg tcgtcgtctc tcacgtcatc 1800
   gagaccctca ccggcggaga atggctaggc gacaccctag ctcgcaccat ctggcaaccc 1860
   cttgacatga aatccacatt ctttgacctc aacaacgcca aagcatcccc tcaccaccta 1920
   gcacacggat attaccatac cccaagcgac aagggcagcg acagccacta ccacgaagtc 1980
   ccctggatgc cactagacga aatctccggc gccggaagca tcatcaccaa cgtcctcgat 2040
   tacgcaaaat gggcgcgcgc actacttaac caaaccgagc ctttatcgcc cgccgtatac 2100
   aaagcaatct tcgaaccgcg aactgttctg cctcccgagg cgccgttcac gggcccgcga 2160
   gcatacgctc tgggctggta tacgggcgta taccaaggcg tgcagttctt cgagcataca 2220
   ggggggatga atgcgttcgg ggccgagata atcttattcc cgagcttgaa gtatgcggtt 2280
   gtcatgttgg cgaatacagc cgggacgtcg aattacgcgc agaaggcgct ggcatttcgg 2340
   ttggtggatg agaaacttgg tgtgccggtt gagaggaggt ttgactggaa tgcgaagtat 2400
   gtttgggatc agggctcttt tcagggaaag gtggttgatg ctaactgttt gatgttagga 2460
   atatggcaca catagaagag gagaagaaaa aggccctcga tgctattcat aaggtccccg 2520
   cgcatgtgat tccgcactcg ttagacctac acgaatatgt tgggacgtat gtgcatccgg 2580
   gctatcagac tgttgagatt tatcttgatc ggtcgaaaag tacactacgg gctgaccggg 2640
   atcgtacaac ttggccggag tatctgagct tcaaacatgt caatggggaa cattttctcg 2700
   ctatatcgga gcatgttgga gacttggggg ctttctttcc tgatgtgtat gcggcggaat 2760
   ttcgaattgg ggaaaagggg aagccgtccg cgcttggagt ggcgtgggag aagactatga 2820
   aggagaagat atggtttacg cggtcggatt aa 2852
<210> 60
   <211> 1521
   <212> DNA
   <213> Aspergillus niger
<220>
   <221> CDS
   <222> (1)..(1521)
<400> 60
   atg gca gca gac cac acc aac acc aac ccg tta gat gag cgg ttc gaa        48
   Met Ala Ala Asp His Thr Asn Thr Asn Pro Leu Asp Glu Arg Phe Glu
   1               5                   10                  15
   acc ctc gtc cag gag aca ttg aat tac tgg cac gtt cca ggc att tca        96
   Thr Leu Val Gln Glu Thr Leu Asn Tyr Trp His Val Pro Gly Ile Ser
           20                  25                  30
   atc gca gta gtc gat gga gac aac acc tgg gcc aag ggg tac ggc att        144
   Ile Ala Val Val Asp Gly Asp Asn Thr Trp Ala Lys Gly Tyr Gly Ile
               35                  40                  45
   gcg acc ttc ccc tct gag ccc gta acc cct tca acg ctc ttc tat gcc        192
   Ala Thr Phe Pro Ser Glu Pro Val Thr Pro Ser Thr Leu Phe Tyr Ala
       50                  55                  60
   gga agc acc acg aaa gct ttc acc gca gcc ttg atg gcc acc ttc gtc        240
   Gly Ser Thr Thr Lys Ala Phe Thr Ala Ala Leu Met Ala Thr Phe Val
   65                  70                  75                  80
   gaa gac aac gac acc cat ccc caa gta cac tgg gac acg ccc atc agc        288
   Glu Asp Asn Asp Thr His Pro Gln Val His Trp Asp Thr Pro Ile Ser
                   85                  90                  95
   cac ctc atc cgc gac gac ttc gtc cta cac gac gag cac aca acc gaa        336
   His Leu Ile Arg Asp Asp Phe Val Leu His Asp Glu His Thr Thr Glu
               100                 105                 110
   cac acc acc atc gaa gat gcc cta tcc cat cgc tca ggc ctc ccc cgt        384
   His Thr Thr Ile Glu Asp Ala Leu Ser His Arg Ser Gly Leu Pro Arg
           115                 120                 125
   cac gat caa gcc tac ggc aac cgt ctc acc cgc gac aaa gcc gta cga        432
   His Asp Gln Ala Tyr Gly Asn Arg Leu Thr Arg Asp Lys Ala Val Arg
       130                 135                 140
   aca ata gtg cgt cag ttg cgc cat ctc cca cta acc gct cct ccg cgc        480
   Thr Ile Val Arg Gln Leu Arg His Leu Pro Leu Thr Ala Pro Pro Arg
   145                 150                 155                 160
   acc aaa ctc caa tac tgc aat ctc atg tac gtc gtc gtc tct cac gtc        528
   Thr Lys Leu Gln Tyr Cys Asn Leu Met Tyr Val Val Val Ser His Val
                   165                 170                 175
   atc gag acc ctc acc ggc gga gaa tgg cta ggc gac acc cta gct cgc        576
   Ile Glu Thr Leu Thr Gly Gly Glu Trp Leu Gly Asp Thr Leu Ala Arg
               180                 185                 190
   acc atc tgg caa ccc ctt gac atg aaa tcc aca ttc ttt gac ctc aac        624
   Thr Ile Trp Gln Pro Leu Asp Met Lys Ser Thr Phe Phe Asp Leu Asn
           195                 200                 205
   aac gcc aaa gca tcc cct cac cac cta gca cac gga tat tac cat acc        672
   Asn Ala Lys Ala Ser Pro His His Leu Ala His Gly Tyr Tyr His Thr
       210                 215                 220
   cca agc gac aag ggc agc gac agc cac tac cac gaa gtc ccc tgg atg        720
   Pro Ser Asp Lys Gly Ser Asp Ser His Tyr His Glu Val Pro Trp Met
   225                 230                 235                 240
   cca cta gac gaa atc tcc ggc gcc gga agc atc atc acc aac gtc ctc        768
   Pro Leu Asp Glu Ile Ser Gly Ala Gly Ser Ile Ile Thr Asn Val Leu
                   245                 250                 255
   gat tac gca aaa tgg gcg cgc gca cta ctt aac caa acc gag cct tta        816
   Asp Tyr Ala Lys Trp Ala Arg Ala Leu Leu Asn Gln Thr Glu Pro Leu
               260                 265                 270
   tcg ccc gcc gta tac aaa gca atc ttc gaa ccg cga act gtt ctg cct        864
   Ser Pro Ala Val Tyr Lys Ala Ile Phe Glu Pro Arg Thr Val Leu Pro
           275                 280                 285
   ccc gag gcg ccg ttc acg ggc ccg cga gca tac gct ctg ggc tgg tat          912
   Pro Glu Ala Pro Phe Thr Gly Pro Arg Ala Tyr Ala Leu Gly Trp Tyr
       290                 295                 300
   acg ggc gta tac caa ggc gtg cag ttc ttc gag cat aca ggg ggg atg          960
   Thr Gly Val Tyr Gln Gly Val Gln Phe Phe Glu His Thr Gly Gly Met
   305                 310                 315                 320
   aat gcg ttc ggg gcc gag ata atc tta ttc ccg agc ttg aag tat gcg         1008
   Asn Ala Phe Gly Ala Glu Ile Ile Leu Phe Pro Ser Leu Lys Tyr Ala
                   325                 330                 335
   gtt gtc atg ttg gcg aat aca gcc ggg acg tcg aat tac gcg cag aag         1056
   Val Val Met Leu Ala Asn Thr Ala Gly Thr Ser Asn Tyr Ala Gln Lys
               340                 345                 350
   gcg ctg gca ttt cgg ttg gtg gat gag aaa ctt ggt gtg ccg gtt gag         1104
   Ala Leu Ala Phe Arg Leu Val Asp Glu Lys Leu Gly Val Pro Val Glu
           355                 360                 365
   agg agg ttt gac tgg aat gcg aag aat atg gca cac ata gaa gag gag         1152
   Arg Arg Phe Asp Trp Asn Ala Lys Asn Met Ala His Ile Glu Glu Glu
       370                 375                 380
   aag aaa aag gcc ctc gat gct att cat aag gtc ccc gcg cat gtg att         1200
   Lys Lys Lys Ala Leu Asp Ala Ile His Lys Val Pro Ala His Val Ile
   385                 390                 395                 400
   ccg cac tcg tta gac cta cac gaa tat gtt ggg acg tat gtg cat ccg         1248
   Pro His Ser Leu Asp Leu His Glu Tyr Val Gly Thr Tyr Val His Pro
                   405                 410                 415
   ggc tat cag act gtt gag att tat ctt gat cgg tcg aaa agt aca cta         1296
   Gly Tyr Gln Thr Val Glu Ile Tyr Leu Asp Arg Ser Lys Ser Thr Leu
               420                 425                 430
   cgg gct gac cgg gat cgt aca act tgg ccg gag tat ctg agc ttc aaa         1344
   Arg Ala Asp Arg Asp Arg Thr Thr Trp Pro Glu Tyr Leu Ser Phe Lys
           435                 440                 445
   cat gtc aat ggg gaa cat ttt ctc gct ata tcg gag cat gtt gga gac         1392
   His Val Asn Gly Glu His Phe Leu Ala Ile Ser Glu His Val Gly Asp
       450                 455                 460
   ttg ggg gct ttc ttt cct gat gtg tat gcg gcg gaa ttt cga att ggg         1440
   Leu Gly Ala Phe Phe Pro Asp Val Tyr Ala Ala Glu Phe Arg Ile Gly
   465                 470                 475                 480
   gaa aag ggg aag ccg tcc gcg ctt gga gtg gcg tgg gag aag act atg         1488
   Glu Lys Gly Lys Pro Ser Ala Leu Gly Val Ala Trp Glu Lys Thr Met
                   485                 490                 495
   aag gag aag ata tgg ttt acg cgg tcg gat taa                             1521
   Lys Glu Lys Ile Trp Phe Thr Arg Ser Asp
               500                 505
<210> 61
   <211> 506
   <212> PRT
   <213> Aspergillus niger
<400> 61
   Met Ala Ala Asp His Thr Asn Thr Asn Pro Leu Asp Glu Arg Phe Glu
   1               5                   10                  15
   Thr Leu Val Gln Glu Thr Leu Asn Tyr Trp His Val Pro Gly Ile Ser
               20                  25                  30
   Ile Ala Val Val Asp Gly Asp Asn Thr Trp Ala Lys Gly Tyr Gly Ile
           35                  40                  45
   Ala Thr Phe Pro Ser Glu Pro Val Thr Pro Ser Thr Leu Phe Tyr Ala
       50                  55                  60
   Gly Ser Thr Thr Lys Ala Phe Thr Ala Ala Leu Met Ala Thr Phe Val
   65                  70                  75                  80
   Glu Asp Asn Asp Thr His Pro Gln Val His Trp Asp Thr Pro Ile Ser
                   85                  90                  95
   His Leu Ile Arg Asp Asp Phe Val Leu His Asp Glu His Thr Thr Glu
               100                 105                 110
   His Thr Thr Ile Glu Asp Ala Leu Ser His Arg Ser Gly Leu Pro Arg
           115                 120                 125
   His Asp Gln Ala Tyr Gly Asn Arg Leu Thr Arg Asp Lys Ala Val Arg
       130                 135                 140
   Thr Ile Val Arg Gln Leu Arg His Leu Pro Leu Thr Ala Pro Pro Arg
   145                 150                 155                 160
   Thr Lys Leu Gln Tyr Cys Asn Leu Met Tyr Val Val Val Ser His Val
                   165                 170                 175
   Ile Glu Thr Leu Thr Gly Gly Glu Trp Leu Gly Asp Thr Leu Ala Arg
               180                 185                 190
   Thr Ile Trp Gln Pro Leu Asp Met Lys Ser Thr Phe Phe Asp Leu Asn
           195                 200                 205
   Asn Ala Lys Ala Ser Pro His His Leu Ala His Gly Tyr Tyr His Thr
       210                 215                 220
   Pro Ser Asp Lys Gly Ser Asp Ser His Tyr His Glu Val Pro Trp Met
   225                 230                 235                 240
   Pro Leu Asp Glu Ile Ser Gly Ala Gly Ser Ile Ile Thr Asn Val Leu
                   245                 250                 255
   Asp Tyr Ala Lys Trp Ala Arg Ala Leu Leu Asn Gln Thr Glu Pro Leu
   260 265 270
   Ser Pro Ala Val Tyr Lys Ala Ile Phe Glu Pro Arg Thr Val Leu Pro
   275 280 285
   Pro Glu Ala Pro Phe Thr Gly Pro Arg Ala Tyr Ala Leu Gly Trp Tyr
   290 295 300
   Thr Gly Val Tyr Gln Gly Val Gln Phe Phe Glu His Thr Gly Gly Met
   305 310 315 320
   Asn Ala Phe Gly Ala Glu Ile Ile Leu Phe Pro Ser Leu Lys Tyr Ala
   325 330 335
   Val Val Met Leu Ala Asn Thr Ala Gly Thr Ser Asn Tyr Ala Gln Lys
   340 345 350
   Ala Leu Ala Phe Arg Leu Val Asp Glu Lys Leu Gly Val Pro Val Glu
   355 360 365
   Arg Arg Phe Asp Trp Asn Ala Lys Asn Met Ala His Ile Glu Glu Glu
   370 375 380
   Lys Lys Lys Ala Leu Asp Ala Ile His Lys Val Pro Ala His Val Ile
   385 390 395 400
   Pro His Ser Leu Asp Leu His Glu Tyr Val Gly Thr Tyr Val His Pro
   405 410 415
   Gly Tyr Gln Thr Val Glu Ile Tyr Leu Asp Arg Ser Lys Ser Thr Leu
   420 425 430
   Arg Ala Asp Arg Asp Arg Thr Thr Trp Pro Glu Tyr Leu Ser Phe Lys
   435 440 445
   His Val Asn Gly Glu His Phe Leu Ala Ile Ser Glu His Val Gly Asp
   450 455 460
   Leu Gly Ala Phe Phe Pro Asp Val Tyr Ala Ala Glu Phe Arg Ile Gly
   465 470 475 480
   Glu Lys Gly Lys Pro Ser Ala Leu Gly Val Ala Trp Glu Lys Thr Met
   485 490 495
   Lys Glu Lys Ile Trp Phe Thr Arg Ser Asp
   500 505
<210> 62
   <211> 2782
   <212> DNA
   <213> Aspergillus niger
<400> 62
   cagtgcgaag gctcccccaa gccgatagtg gtagaacggt agagattcgg caactcgacg 60
   agcaactgca aagctttctt tcagaacgga taaatacagc taccgcggaa attggccatg 120
   attgcagtcc tatagcatct gccgtgaggt atgtcagccg taatctccct gccttgtgac 180
   ccgaaaggga atggatatcc ctagtctgaa ggaccgcgcc actcggctgc agctaactga 240
   ttctgccagg gcgctttgcc tattacatca ggaagtcata gagtctcctt ccaacaccgt 300
   tgacttgcag agtctgcaat actcacaggc ggctctagga atggtatcta atgtagtggt 360
   ggatgtcgcg cgccaccaca aagcgcaaat tgcgtgccaa aattcccatg ccgatctact 420
   acccctgagc tgctcgtaca ctctgcacat ggcaatgaga catattcggg attgcaggaa 480
   gctagtctgc ccatacagcc gctctagcga cctgaactct ctagtgcagc tggatcaaga 540
   attttccgac agatggaaag cctgaatccc ctcttcctgc agtgggcatg agcgattttc 600
   gtgataggaa aagcggctac ggggttggtc caatccaagg gcaaagcaga ggcccaccgc 660
   caagcgaatg gccgcaaatt ccgtccgcaa cagcccaatg aaggtgcatc gtcgttatgt 720
   cttaaattgc gatggagacg gattagattg ctcgaatcag gatggaggcc cgacggccat 780
   caaccgccga acatccggct gctcgaccga attcggagac atggtcggag ctaggcggga 840
   atagccgtcc aaagtcacga taggtcgctt gtactgtgta gaagtcagac cctttggatc 900
   cagcgttcgt tgtgaatggc tccacgcagc ctttgctttg accaatttgg ggctgagtca 960
   cttggtcgat gatgactggc tataccagtg caaagtgtga gcaccatgct tacactattg 1020
   ccttagcctg gccgttgaag ctgacatgag aggaacaggc agaagagaaa tgctgccttg 1080
   cctcttgggg gccaatttaa cctccccaga ctgcggcaca tccgtcttgg acttgtgctt 1140
   ctttgcaact tacgttataa cgagtctgtg aacaggaaaa cagacgcccg gtcagtcaag 1200
   atgaaaggtg cggcgctaat tcctcttgcg gcgggcattc cttttgccca tggcctgtct 1260
   ctccataaac gcgacgggcc tgccgtcgtt cgtatgccca ttgagcgcag gagcgcccag 1320
   tccttgcaga aacgagattc tacggtcggt gtgactttgc agaactgggt atgttaagct 1380
   atacggccgt gaagtataga tcatgctgac aatcgctagg atgcgaccta ttacgcagtc 1440
   aacctgacgt taggaacacc tgcgcaaaag gtatcattag ctttggacac tggcagcagc 1500
   gacctctggg tgaacaccgg caactcaact tactgctcaa tcgacaatct atgcacccct 1560
   tatggcttgt acaatgccag cgaatcgtct actgtaaaga ccgtgggcac acacctcaac 1620
   gatacatatg cggacggcac aaacctttac ggtccttatg tgaccgataa gctcacgatc 1680
   ggcaacacaa caatcgataa tatgcagttt gggatcgccg agtcaacgac tagtaaacgt 1740
   gggtgaaccg tttgtccatg aatgatcgtc gctgactagg tctactatag gcgggatcgc 1800
   cggcgtcggt tacaagattt cgacctacca agccgagcat gacgacaaag tctacgccaa 1860
   cctccctcag gccctcgtcg acagcggtgc cattaagtct gctgcgtaca gcatatggct 1920
   agatagtttg gaggcgtcga ctggctccct ccttttcgga ggtgtcaata cagccaagta 1980
   caagggcgat ctgcagactc ttccgatcat tcctgtgtat ggcaaatact actccctcgc 2040
   catcgccctt acggagctca gcgttgcgac cgactccaac tccagtagct tcaccgacag 2100
   tctccccctc tctgtgtcac tcgatactgg caccaccatg acggcactgc ccagcgacct 2160
   ggtcaacaag gtctacgatg cgctcaacgc aacctacgac aagacatacg acatggccta 2220
   catcgactgc gacactagag aggcggatta caatgtaaca tacagtttct ccggggcaac 2280
   gatcaccgtg agcatgagtg agctgattat ccccgcaacg gagccggggt ggcccgacaa 2340
   cacgtgtgtc ttgggcctcg tgcctagcca gccgggcgtg aacctgctcg gtgatacatt 2400
   cctgcgcagt gcgtacgtcg tgtatgatct cgagaacaac gaaatctctc tcgccaatac 2460
   caatttcaat ccaggcgacg atgatatcct cgaaatcgga acgggaacgt ctgctgtgcc 2520
   aggagccaca ccggttccct ctgctgtctc ttctgcaact ggaaatggac tgatctcgtc 2580
   tggcaccgca gtgcccacgc tgtcgggtgt cacaataact gctacagcca cagcaaccgg 2640
   ctcaaccggc actggctcta gcggtggttc gtcggctgaa gccacgagta cttcctcgga 2700
   gggcgctgcg gcgcaagcta cgagcaaccc gatgaacctg ctcccaggac ttgcgggtat 2760
   cggcctactt ctcgctctgt aa 2782
<210> 63
   <211> 1479
   <212> DNA
   <213> Aspergillus niger
<220>
   <221> CDS
   <222> (1)..(1479)
<400> 63
   atg aaa ggt gcg gcg cta att cct ctt gcg gcg ggc att cct ttt gcc        48
   Met Lys Gly Ala Ala Leu Ile Pro Leu Ala Ala Gly Ile Pro Phe Ala
   1               5                   10                  15
   cat ggc ctg tct ctc cat aaa cgc gac ggg cct gcc gtc gtt cgt atg        96
   His Gly Leu Ser Leu His Lys Arg Asp Gly Pro Ala Val Val Arg Met
               20                  25                  30
   ccc att gag cgc agg agc gcc cag tcc ttg cag aaa cga gat tct acg       144
   Pro Ile Glu Arg Arg Ser Ala Gln Ser Leu Gln Lys Arg Asp Ser Thr
           35                  40                  45
   gtc ggt gtg act ttg cag aac tgg gat gcg acc tat tac gca gtc aac       192
   Val Gly Val Thr Leu Gln Asn Trp Asp Ala Thr Tyr Tyr Ala Val Asn
       50                  55                  60
   ctg acg tta gga aca cct gcg caa aag gta tca tta gct ttg gac act       240
   Leu Thr Leu Gly Thr Pro Ala Gln Lys Val Ser Leu Ala Leu Asp Thr
   65                  70                  75                  80
   ggc agc agc gac ctc tgg gtg aac acc ggc aac tca act tac tgc tca        288
   Gly Ser Ser Asp Leu Trp Val Asn Thr Gly Asn Ser Thr Tyr Cys Ser
                   85                  90                  95
   atc gac aat cta tgc acc cct tat ggc ttg tac aat gcc agc gaa tcg        336
   Ile Asp Asn Leu Cys Thr Pro Tyr Gly Leu Tyr Asn Ala Ser Glu Ser
               100                 105                 110
   tct act gta aag acc gtg ggc aca cac ctc aac gat aca tat gcg gac        384
   Ser Thr Val Lys Thr Val Gly Thr His Leu Asn Asp Thr Tyr Ala Asp
           115                 120                 125
   ggc aca aac ctt tac ggt cct tat gtg acc gat aag ctc acg atc ggc        432
   Gly Thr Asn Leu Tyr Gly Pro Tyr Val Thr Asp Lys Leu Thr Ile Gly
       130                 135                 140
   aac aca aca atc gat aat atg cag ttt ggg atc gcc gag tca acg act        480
   Asn Thr Thr Ile Asp Asn Met Gln Phe Gly Ile Ala Glu Ser Thr Thr
   145                 150                 155                 160
   agt aaa cgc ggg atc gcc ggc gtc ggt tac aag att tcg acc tac caa        528
   Ser Lys Arg Gly Ile Ala Gly Val Gly Tyr Lys Ile Ser Thr Tyr Gln
                   165                 170                 175
   gcc gag cat gac gac aaa gtc tac gcc aac ctc cct cag gcc ctc gtc        576
   Ala Glu His Asp Asp Lys Val Tyr Ala Asn Leu Pro Gln Ala Leu Val
               180                 185                 190
   gac agc ggt gcc att aag tct gct gcg tac agc ata tgg cta gat agt        624
   Asp Ser Gly Ala Ile Lys Ser Ala Ala Tyr Ser Ile Trp Leu Asp Ser
           195                 200                 205
   ttg gag gcg tcg act ggc tcc ctc ctt ttc gga ggt gtc aat aca gcc        672
   Leu Glu Ala Ser Thr Gly Ser Leu Leu Phe Gly Gly Val Asn Thr Ala
       210                 215                 220
   aag tac aag ggc gat ctg cag act ctt ccg atc att cct gtg tat ggc        720
   Lys Tyr Lys Gly Asp Leu Gln Thr Leu Pro Ile Ile Pro Val Tyr Gly
   225                 230                 235                 240
   aaa tac tac tcc ctc gcc atc gcc ctt acg gag ctc agc gtt gcg acc        768
   Lys Tyr Tyr Ser Leu Ala Ile Ala Leu Thr Glu Leu Ser Val Ala Thr
                   245                 250                 255
   gac tcc aac tcc agt agc ttc acc gac agt ctc ccc ctc tct gtg tca        816
   Asp Ser Asn Ser Ser Ser Phe Thr Asp Ser Leu Pro Leu Ser Val Ser
               260                 265                 270
   ctc gat act ggc acc acc atg acg gca ctg ccc agc gac ctg gtc aac        864
   Leu Asp Thr Gly Thr Thr Met Thr Ala Leu Pro Ser Asp Leu Val Asn
           275                 280                 285
   aag gtc tac gat gcg ctc aac gca acc tac gac aag aca tac gac atg        912
   Lys Val Tyr Asp Ala Leu Asn Ala Thr Tyr Asp Lys Thr Tyr Asp Met
       290                 295                 300
   gcc tac atc gac tgc gac act aga gag gcg gat tac aat gta aca tac        960
   Ala Tyr Ile Asp Cys Asp Thr Arg Glu Ala Asp Tyr Asn Val Thr Tyr
   305                 310                 315                 320
   agt ttc tcc ggg gca acg atc acc gtg agc atg agt gag ctg att atc       1008
   Ser Phe Ser Gly Ala Thr Ile Thr Val Ser Met Ser Glu Leu Ile Ile
                   325                 330                 335
   ccc gca acg gag ccg ggg tgg ccc gac aac acg tgt gtc ttg ggc ctc       1056
   Pro Ala Thr Glu Pro Gly Trp Pro Asp Asn Thr Cys Val Leu Gly Leu
               340                 345                 350
   gtg cct agc cag ccg ggc gtg aac ctg ctc ggt gat aca ttc ctg cgc      1104
   Val Pro Ser Gln Pro Gly Val Asn Leu Leu Gly Asp Thr Phe Leu Arg
           355                 360                 365
   agt gcg tac gtc gtg tat gat ctc gag aac aac gaa atc tct ctc gcc      1152
   Ser Ala Tyr Val Val Tyr Asp Leu Glu Asn Asn Glu Ile Ser Leu Ala
       370                 375                 380
   aat acc aat ttc aat cca ggc gac gat gat atc ctc gaa atc gga acg      1200
   Asn Thr Asn Phe Asn Pro Gly Asp Asp Asp Ile Leu Glu Ile Gly Thr
   385                 390                 395                 400
   gga acg tct gct gtg cca gga gcc aca ccg gtt ccc tct gct gtc tct      1248
   Gly Thr Ser Ala Val Pro Gly Ala Thr Pro Val Pro Ser Ala Val Ser
                   405                 410                 415
   tct gca act gga aat gga ctg atc tcg tct ggc acc gca gtg ccc acg      1296
   Ser Ala Thr Gly Asn Gly Leu Ile Ser Ser Gly Thr Ala Val Pro Thr
               420                 425                 430
   ctg tcg ggt gtc aca ata act gct aca gcc aca gca acc ggc tca acc      1344
   Leu Ser Gly Val Thr Ile Thr Ala Thr Ala Thr Ala Thr Gly Ser Thr
           435                 440                 445
   ggc act ggc tct agc ggt ggt tcg tcg gct gaa gcc acg agt act tcc      1392
   Gly Thr Gly Ser Ser Gly Gly Ser Ser Ala Glu Ala Thr Ser Thr Ser
       450                 455                 460
   tcg gag ggc gct gcg gcg caa gct acg agc aac ccg atg aac ctg ctc      1440
   Ser Glu Gly Ala Ala Ala Gln Ala Thr Ser Asn Pro Met Asn Leu Leu
   465                 470                 475                 480
   cca gga ctt gcg ggt atc ggc cta ctt ctc gct ctg taa                  1479
   Pro Gly Leu Ala Gly Ile Gly Leu Leu Leu Ala Leu
                   485                 490
<210> 64
   <211> 492
   <212> PRT
   <213> Aspergillus niger
<400> 64
   Met Lys Gly Ala Ala Leu Ile Pro Leu Ala Ala Gly Ile Pro Phe Ala
   1 5 10 15
   His Gly Leu Ser Leu His Lys Arg Asp Gly Pro Ala Val Val Arg Met
   20 25 30
   Pro Ile Glu Arg Arg Ser Ala Gln Ser Leu Gln Lys Arg Asp Ser Thr
   35 40 45
   Val Gly Val Thr Leu Gln Asn Trp Asp Ala Thr Tyr Tyr Ala Val Asn
   50 55 60
   Leu Thr Leu Gly Thr Pro Ala Gln Lys Val Ser Leu Ala Leu Asp Thr
   65 70 75 80
   Gly Ser Ser Asp Leu Trp Val Asn Thr Gly Asn Ser Thr Tyr Cys Ser
                   85                  90                  95
   Ile Asp Asn Leu Cys Thr Pro Tyr Gly Leu Tyr Asn Ala Ser Glu Ser
               100                 105                 110
   Ser Thr Val Lys Thr Val Gly Thr His Leu Asn Asp Thr Tyr Ala Asp
           115                 120                 125
   Gly Thr Asn Leu Tyr Gly Pro Tyr Val Thr Asp Lys Leu Thr Ile Gly
       130                 135                 140
   Asn Thr Thr Ile Asp Asn Met Gln Phe Gly Ile Ala Glu Ser Thr Thr
   145                 150                 155                 160
   Ser Lys Arg Gly Ile Ala Gly Val Gly Tyr Lys Ile Ser Thr Tyr Gln
                   165                 170                 175
   Ala Glu His Asp Asp Lys Val Tyr Ala Asn Leu Pro Gln Ala Leu Val
               180                 185                 190
   Asp Ser Gly Ala Ile Lys Ser Ala Ala Tyr Ser Ile Trp Leu Asp Ser
           195                 200                 205
   Leu Glu Ala Ser Thr Gly Ser Leu Leu Phe Gly Gly Val Asn Thr Ala
       210                 215                 220
   Lys Tyr Lys Gly Asp Leu Gln Thr Leu Pro Ile Ile Pro Val Tyr Gly
   225                 230                 235                 240
   Lys Tyr Tyr Ser Leu Ala Ile Ala Leu Thr Glu Leu Ser Val Ala Thr
                   245                 250                 255
   Asp Ser Asn Ser Ser Ser Phe Thr Asp Ser Leu Pro Leu Ser Val Ser
               260                 265                 270
   Leu Asp Thr Gly Thr Thr Met Thr Ala Leu Pro Ser Asp Leu Val Asn
           275                 280                 285
   Lys Val Tyr Asp Ala Leu Asn Ala Thr Tyr Asp Lys Thr Tyr Asp Met
       290                 295                 300
   Ala Tyr Ile Asp Cys Asp Thr Arg Glu Ala Asp Tyr Asn Val Thr Tyr
   305                 310                 315                 320
   Ser Phe Ser Gly Ala Thr Ile Thr Val Ser Met Ser Glu Leu Ile Ile
                   325                 330                 335
   Pro Ala Thr Glu Pro Gly Trp Pro Asp Asn Thr Cys Val Leu Gly Leu
   340 345 350
   Val Pro Ser Gln Pro Gly Val Asn Leu Leu Gly Asp Thr Phe Leu Arg
   355 360 365
   Ser Ala Tyr Val Val Tyr Asp Leu Glu Asn Asn Glu Ile Ser Leu Ala
   370 375 380
   Asn Thr Asn Phe Asn Pro Gly Asp Asp Asp Ile Leu Glu Ile Gly Thr
   385 390 395 400
   Gly Thr Ser Ala Val Pro Gly Ala Thr Pro Val Pro Ser Ala Val Ser
   405 410 415
   Ser Ala Thr Gly Asn Gly Leu Ile Ser Ser Gly Thr Ala Val Pro Thr
   420 425 430
   Leu Ser Gly Val Thr Ile Thr Ala Thr Ala Thr Ala Thr Gly Ser Thr
   435 440 445
   Gly Thr Gly Ser Ser Gly Gly Ser Ser Ala Glu Ala Thr Ser Thr Ser
   450 455 460
   Ser Glu Gly Ala Ala Ala Gln Ala Thr Ser Asn Pro Met Asn Leu Leu
   465 470 475 480
   Pro Gly Leu Ala Gly Ile Gly Leu Leu Leu Ala Leu
   485 490
<210> 65
   <211> 1998
   <212> DNA
   <213> Aspergillus niger
<400> 65
   ccatgctttg aagcggggct gaagatgttg gcgccgggga tgcagtggat atcccgacgg 60
   actccggccc gttgcctttt agccacgcct gacccctcat gccggtagtt acgcctcttc 120
   aggggatgta attcagactg ctaacgcgtt tttcacctgg ggacagactg aggtgcgtga 180
   aataagtggc atttttcgca taaaagcagt gttctactgc tccccaatat ccaacatttg 240
   acgtccagcc caagtcctta cagtggacca aaacccactt cccttggtgt ttgattgcat 300
   ctcgttgccg atagacaagt ggatggtgtg aaggctaatt taattgattt ctccggagtt 360
   tgcaggttcg gcaaggcacg agccattcaa tgacccccat tcttcatcct aaatgaagtt 420
   tatctgccac acacgggacg atccccgcag agccaataga ttgtttctct cgttatcatc 480
   acgccatgac cacacgtcgt caccgttgag gatgatagtc tgatggccat tccgcttgag 540
   gcaaagtgtg ccccaatgtc acgcggtcag ctagtcttct gcaggtctgc agttggacag    600
   gggaacttga gtgctattat cgccaaggtg aatgttctct accgctttga taccagagtg    660
   atcgtccctt ttgccgtcac tcttggtgtt gcgaagcaga tgtcgttcgt ttgcatatgg    720
   gagccgaaga tccacgcagg gtgcttgaaa agacatactg cgatcctccc gtttccaaga    780
   atgcttgtac acttgaagtt tttgtcaaga tccccccgcc tggccagtcc cgagctaagc    840
   gaaatgagct ttccctcaat gctgcagaca ggagacacca tcgctttggc gccttggcac    900
   catcagccac cgtcggtcct atcatcatcg ccctggtcaa tagggaacgc gtcaattgcg    960
   tatctgggta gtgttatctg ctcgatattg caccggcaga gcaggaatcg ggattggcgg   1020
   atgtagagaa aattcagctt caaggagggg ggaagggaga cgctgaaatg gtataaaacc   1080
   acgtccaatt ccctaccaga tagccctcat ccagcagcat caaaagcatc ttccactcag   1140
   actccaagca gctcccagtc cctcttcaat tcattacctt ccaaacatca tcccatcaag   1200
   atgaagacta ctgctctctt gaccgccggc ctgctggcca ccactgctat ggccgctcct   1260
   ctgacggcca agcgccaggc tgctcgggcc aagcgctcca cgaaccgcca gagcaaccct   1320
   cccttcaagc ctggcaccaa cgaggtcctc gcccttaacg gcaccaagaa tgtggagtac   1380
   agctccaact gggccggtgc cgtcctcatt ggcactggtt acactgccgt gaccgccgag   1440
   ttcgtcgtgc ccaccccctc cgtgccctcc ggtggctcga gccgcgagga gtactgtgcc   1500
   tccgcctggg tgggcattga cggtgacacc tgtgacactg ctatcctcca gaccggtgtg   1560
   gacttttgtg tccagggcag cgaggtgagc ttcgatgcct ggtacgagtg gtaccccgac   1620
   tacgcctacg acttcagcgg catctccatc tcggccggtg ataccatcaa ggtcaccgtc   1680
   gatgccagca gcgacaccac cggtactgcc acgattgaga acgtgagcac tggtaccacg   1740
   gtcacccaca gcttcacggg cggtgttgat ggtgatctgt gtgagtacaa cgctgagtgg   1800
   atcgtcgagg acttcgagga ggatgactcc ctcgttccct ttgccgactt tggcaccgtg   1860
   actttcacca gctgctccgc taccaaggat ggttcctctg ttggccctga ggatgctacc   1920
   atcatcgaca tcgagcagaa tgaggtgctg acctccgttt ccgtctccag tagcgaggtc   1980
   gttgtcaagt acgtctaa                                                 1998
<210> 66
   <211> 798
   <212> DNA
   <213> Aspergillus niger
<220>
   <221> CDS
   <222> (1) .. (798)
<400> 66
   atg aag act act gct ctc ttg acc gcc ggc ctg ctg gcc acc act gct          48
   Met Lys Thr Thr Ala Leu Leu Thr Ala Gly Leu Leu Ala Thr Thr Ala
   1               5                   10                  15
   atg gcc gct cct ctg acg gcc aag cgc cag gct gct cgg gcc aag cgc        96
   Met Ala Ala Pro Leu Thr Ala Lys Arg Gln Ala Ala Arg Ala Lys Arg
               20                  25                  30
   tcc acg aac cgc cag agc aac cct ccc ttc aag cct ggc acc aac gag       144
   Ser Thr Asn Arg Gln Ser Asn Pro Pro Phe Lys Pro Gly Thr Asn Glu
           35                  40                  45
   gtc ctc gcc ctt aac ggc acc aag aat gtg gag tac agc tcc aac tgg       192
   Val Leu Ala Leu Asn Gly Thr Lys Asn Val Glu Tyr Ser Ser Asn Trp
       50                  55                  60
   gcc ggt gcc gtc ctc att ggc act ggt tac act gcc gtg acc gcc gag       240
   Ala Gly Ala Val Leu Ile Gly Thr Gly Tyr Thr Ala Val Thr Ala Glu
   65                  70                  75                  80
   ttc gtc gtg ccc acc ccc tcc gtg ccc tcc ggt ggc tcg agc cgc gag       288
   Phe Val Val Pro Thr Pro Ser Val Pro Ser Gly Gly Ser Ser Arg Glu
                   85                  90                  95
   gag tac tgt gcc tcc gcc tgg gtg ggc att gac ggt gac acc tgt gac       336
   Glu Tyr Cys Ala Ser Ala Trp Val Gly Ile Asp Gly Asp Thr Cys Asp
               100                 105                 110
   act gct atc ctc cag acc ggt gtg gac ttt tgt gtc cag ggc agc gag       384
   Thr Ala Ile Leu Gln Thr Gly Val Asp Phe Cys Val Gln Gly Ser Glu
           115                 120                 125
   gtg agc ttc gat gcc tgg tac gag tgg tac ccc gac tac gcc tac gac       432
   Val Ser Phe Asp Ala Trp Tyr Glu Trp Tyr Pro Asp Tyr Ala Tyr Asp
       130                 135                 140
   ttc agc ggc atc tcc atc tcg gcc ggt gat acc atc aag gtc acc gtc       480
   Phe Ser Gly Ile Ser Ile Ser Ala Gly Asp Thr Ile Lys Val Thr Val
   145                 150                 155                 160
   gat gcc agc agc gac acc acc ggt act gcc acg att gag aac gtg agc       528
   Asp Ala Ser Ser Asp Thr Thr Gly Thr Ala Thr Ile Glu Asn Val Ser
                   165                 170                 175
   act ggt acc acg gtc acc cac agc ttc acg ggc ggt gtt gat ggt gat       576
   Thr Gly Thr Thr Val Thr His Ser Phe Thr Gly Gly Val Asp Gly Asp
               180                 185                 190
   ctg tgt gag tac aac gct gag tgg atc gtc gag gac ttc gag gag gat       624
   Leu Cys Glu Tyr Asn Ala Glu Trp Ile Val Glu Asp Phe Glu Glu Asp
           195                 200                 205
   gac tcc ctc gtt ccc ttt gcc gac ttt ggc acc gtg act ttc acc agc       672
   Asp Ser Leu Val Pro Phe Ala Asp Phe Gly Thr Val Thr Phe Thr Ser
       210                 215                 220
   tgc tcc gct acc aag gat ggt tcc tct gtt ggc cct gag gat gct acc       720
   Cys Ser Ala Thr Lys Asp Gly Ser Ser Val Gly Pro Glu Asp Ala Thr
   225                 230                 235                 240
   atc atc gac atc gag cag aat gag gtg ctg acc tcc gtt tcc gtc tcc       768
   Ile Ile Asp Ile Glu Gln Asn Glu Val Leu Thr Ser Val Ser Val Ser
                   245                 250                 255
   agt agc gag gtc gtt gtc aag tac gtc taa                               798
   Ser Ser Glu Val Val Val Lys Tyr Val
               260                 265
<210> 67
   <211> 265
   <212> PRT
   <213> Aspergillus niger
<400> 67
   Met Lys Thr Thr Ala Leu Leu Thr Ala Gly Leu Leu Ala Thr Thr Ala
   1               5                   10                  15
   Met Ala Ala Pro Leu Thr Ala Lys Arg Gln Ala Ala Arg Ala Lys Arg
               20                  25                  30
   Ser Thr Asn Arg Gln Ser Asn Pro Pro Phe Lys Pro Gly Thr Asn Glu
           35                  40                  45
   Val Leu Ala Leu Asn Gly Thr Lys Asn Val Glu Tyr Ser Ser Asn Trp
       50                  55                  60
   Ala Gly Ala Val Leu Ile Gly Thr Gly Tyr Thr Ala Val Thr Ala Glu
   65                  70                  75                  80
   Phe Val Val Pro Thr Pro Ser Val Pro Ser Gly Gly Ser Ser Arg Glu
                   85                  90                  95
   Glu Tyr Cys Ala Ser Ala Trp Val Gly Ile Asp Gly Asp Thr Cys Asp
               100                 105                 110
   Thr Ala Ile Leu Gln Thr Gly Val Asp Phe Cys Val Gln Gly Ser Glu
           115                 120                 125
   Val Ser Phe Asp Ala Trp Tyr Glu Trp Tyr Pro Asp Tyr Ala Tyr Asp
       130                 135                 140
   Phe Ser Gly Ile Ser Ile Ser Ala Gly Asp Thr Ile Lys Val Thr Val
   145                 150                 155                 160
   Asp Ala Ser Ser Asp Thr Thr Gly Thr Ala Thr Ile Glu Asn Val Ser
                   165                 170                 175
   Thr Gly Thr Thr Val Thr His Ser Phe Thr Gly Gly Val Asp Gly Asp
               180                 185                 190
   Leu Cys Glu Tyr Asn Ala Glu Trp Ile Val Glu Asp Phe Glu Glu Asp
           195                 200                 205
   Asp Ser Leu Val Pro Phe Ala Asp Phe Gly Thr Val Thr Phe Thr Ser
       210                 215                 220
   Cys Ser Ala Thr Lys Asp Gly Ser Ser Val Gly Pro Glu Asp Ala Thr
   225 230 235 240
   Ile Ile Asp Ile Glu Gln Asn Glu Val Leu Thr Ser Val Ser Val Ser
   245 250 255
   Ser Ser Glu Val Val Val Lys Tyr Val
   260 265
<210> 68
   <211> 3610
   <212> DNA
   <213> Aspergillus niger
<400> 68
   tgactctcat aagcagtctt ctgcggagca ggagtggagg gagaaggggt atccggaaat 60
   tgactgggat acgttcgatg aacgaatcac tcggcatttc gcggacctgg agacgatcat 120
   ggtgccggag agtgcctcgt tctaccggaa tagcctggat acggcgttga agaccggaca 180
   ggctaagaac ttccgtctgt cgctggacgg agacggattg gaaaagatct cgtgtggata 240
   ttacgggact cgaggggctg ggaaaatgta cgccccaccc ttccatctac ttgatatggc 300
   tctatacggg taggctaatt gaaacaggct acaagcgatc acagcgcgat atgggcggaa 360
   gctccgtcgg ttagcggccg atgatcagat tgtgaagatg gccgggcccg tcggatatgc 420
   gcaggcggtg ctggtgccag agttggcggt gcgactggtc aaagaggaca tgggagtgaa 480
   tgaggagacg gcacggcaaa ttctccgtga gacaattgat ctcggggaga aggtgaactt 540
   cgccttgaat gacgaggtgc cagtgccgga ggagtctgac gcagaagtgc agtgagatgc 600
   aggtaggagt agtggtggtg atgtgttttg gaagggatta ttattcttat actggtcgga 660
   ctctgttcat tggactgtcc agtgaccaat gctatgttgt atataaggcg atgagtgata 720
   cctctctgtt agctagtctg ttgtgttgtg ttgtaattta gccgggagaa ttgtctcata 780
   tggcgcttgc tggagacgaa ttgacaccat tacgactcca cagttggtga tgcttcaagt 840
   ccggccgagg tggagccggc gataagatag cggagactgc tgattagctg atagccttgg 900
   ttacgtcgtg cggagctgcc cgaaaccctt ccttccattg caagtaagta gctggtctag 960
   tcactactaa gtactactgc ttactgcagt aagttactcc tcacctccat ctctctctct 1020
   ctctctctct ctgactctct gactcttact cttactcttg ctcttccttc atccacactt 1080
   ccatctcccc tctttcatga gatggatagt attccattct atctcttatc cctctaatcc 1140
   cttatcccaa tcatgccccc cgaaatgcgg gaaaaggccc atgcctatca ctactccaca 1200
   atgcccagcg aatccacgcc tctcctcacc acggtcggca tccgtcccca tcgtcgtcgc 1260
   tacccacacc acactctccg ccgtttctgc accatcattc tcacctccat tcttaccctc 1320
   tccctctgtc tcttcttgac ccccaccgcc ctccttcccc gcgcccacgg ctccatctgg 1380
   agctacctcc cctgggcaaa ccccttcccc cactcctcct ggcctgaagg ccacggcctg    1440
   agctacgctg aactccaagc catcttgaaa gaaaccccca gcgccgaaaa ggctcgcgaa 1500
   tggagcactt actacaccgc tggtccgcac ctggccggga agaacctcag ccaggcagtc 1560
   tggaccaagg aacgctgggc ggagttcgga gtcccggaga ccgatatcgt agcatatgat 1620
   atctttctca attaccctgt cgatcatcgg ttggcattgc tgaagaaaga cgaggtcgtg 1680
   tttgaggcaa ccttggaaga ggacgtgctc gaggaggacc ataccagcgg attgccgaac 1740
   cgcgtccctg tgtttcacgg ctactcggcc agtggaaatg tcacggcgcc atatgtgttt 1800
   gtgaactatg gcactgtgga cgatttccag gacctcgtgg accggaatgt cagtctcgga 1860
   ggtaaaattg ctattgccaa gtatggacgc atcttccgcg gattgaaggt caagagagcg 1920
   caggaattgg gcatgtctgg agtgattctg tatgatgatc ccgagtcgga tggggagatt 1980
   atcgaggaaa atggctatgc tccgtatccg gaggggccgg ccaggaaccc gagtgctgtt 2040
   cagaggggaa gtacccagtt tttgagtaag ttatcctatc ttccgcctgc ttgttggatg 2100
   gagaagctaa actgagtagg tatcgcaccg ggtgacccaa ctacccccgg atacccatcc 2160
   aagccgggct gtgatcgcca ggacccgtat gagtccatcc ccttgattcc ttctctgccc 2220
   atctcatacc aggaggtggt gccgttgcta aaggccctga atggccacgg tcccaaggcg 2280
   tctgacttca acgaatactg gcaaggcggt aagctgggca gcaagggcgt cgagtactac 2340
   attggcccga ccccggatga tgttcaaatc aatctgtaca atgaacagga gtacgtgacg 2400
   actcctctgt ggaacgtcat tggcattatc aagggtgcca tcccggacga ggtcgtgatc 2460
   atgggcaacc accgcgatgc ctggattgcc ggtggcgctg gagatccgaa cagcggatct 2520
   gccgctctca acgaagttgt gcgcagcttc ggcgaagcgc ttaaggccgg atggaagccg 2580
   ctccgaacca ttgtctttgc cagctgggac ggtgaagagt acggattgct gggttctacc 2640
   gagtgggtgg aagagcatct gccctggttg tccaaggcca acattgccta tctcaacgtg 2700
   gacgtagcgg cgtcgggaac gagcttgcgc ccgagggcca gcccgctgct gaacaagctg 2760
   atctacgagg tcactggtct agtgaagtcg cccaaccaga ctgtgcctgg acagacagtc 2820
   cgggacctct gggacggcga cattgccacc atgggcagtg gcagtgactt tacggccttc 2880
   caggactttg cgggtgtggc aagctacgac ctcggattca gccgcggtcc gaaggacccg 2940
   gtttaccact accactcgaa ctacgacagc ttcgactgga tggaccggtt cggcgacccg 3000
   ggctggaagt accatgaagc aaccaccaag atctgggctc tggcagcggc caagctggtg 3060
   gagacgccgg tggtggcctt caatgctacg gactacgcgc tgggcctgga gaagtatctt 3120
   gacaagatca agccggcggc gaagcaactc cccgagggga catcgttcga ctttggtccg 3180
   ctcgacgcgg cgatcgcgga gtttcaagcg gtgtccgcga aatttgacgc ttacgcggcg 3240
   gatctgagcg cgcagctggg cgacgatgtg ccgtggtacc actggtggaa gaaggtgcgg 3300
   ctctacttcc agatccgcat cgtcaacgac aagtacaagg gcattgaacg acagttcctg 3360
   taccagccgg gcctggacgg gcgcagctgg tttaagcacg tagtgtttgc accgggcatc 3420
   tggacgggat actcaggcgc gacgtatccc ggattggtgg agagcttgga agcgggtgat      3480
   ttgctgaatt ctcaggtgag taacccagta cctacctcgc tgatttaatt ttcagcggct      3540
   aatggggttc tagaaatggc gcgcgatcat catcgagcgc attgaggctg cgacgaagct      3600
   actgcagtga                                                              3610
<210> 69
   <211> 2298
   <212> DNA
   <213> Aspergillus niger
<220>
   <221> CDS
   <222> (1)..(2298)
<400> 69
   atg ccc agc gaa tcc acg cct ctc ctc acc acg gtc ggc atc cgt ccc      48
   Met Pro Ser Glu Ser Thr Pro Leu Leu Thr Thr Val Gly Ile Arg Pro
   1               5                   10                  15
   cat cgt cgt cgc tac cca cac cac act ctc cgc cgt ttc tgc acc atc      96
   His Arg Arg Arg Tyr Pro His His Thr Leu Arg Arg Phe Cys Thr Ile
               20                  25                  30
   att ctc acc tcc att ctt acc ctc tcc ctc tgt ctc ttc ttg acc ccc     144
   Ile Leu Thr Ser Ile Leu Thr Leu Ser Leu Cys Leu Phe Leu Thr Pro
           35                  40                  45
   acc gcc ctc ctt ccc cgc gcc cac ggc tcc atc tgg agc tac ctc ccc     192
   Thr Ala Leu Leu Pro Arg Ala His Gly Ser Ile Trp Ser Tyr Leu Pro
       50                  55                  60
   tgg gca aac ccc ttc ccc cac tcc tcc tgg cct gaa ggc cac ggc ctg     240
   Trp Ala Asn Pro Phe Pro His Ser Ser Trp Pro Glu Gly His Gly Leu
   65                  70                  75                  80
   agc tac gct gaa ctc caa gcc atc ttg aaa gaa acc ccc agc gcc gaa     288
   Ser Tyr Ala Glu Leu Gln Ala Ile Leu Lys Glu Thr Pro Ser Ala Glu
                   85                  90                  95
   aag gct cgc gaa tgg agc act tac tac acc gct ggt ccg cac ctg gcc     336
   Lys Ala Arg Glu Trp Ser Thr Tyr Tyr Thr Ala Gly Pro His Leu Ala
               100                 105                 110
   ggg aag aac ctc agc cag gca gtc tgg acc aag gaa cgc tgg gcg gag     384
   Gly Lys Asn Leu Ser Gln Ala Val Trp Thr Lys Glu Arg Trp Ala Glu
           115                 120                 125
   ttc gga gtc ccg gag acc gat atc gta gca tat gat atc ttt ctc aat     432
   Phe Gly Val Pro Glu Thr Asp Ile Val Ala Tyr Asp Ile Phe Leu Asn
       130                 135                 140
   tac cct gtc gat cat cgg ttg gca ttg ctg aag aaa gac gag gtc gtg     480
   Tyr Pro Val Asp His Arg Leu Ala Leu Leu Lys Lys Asp Glu Val Val
   145                 150                 155                 160
   ttt gag gca acc ttg gaa gag gac gtg ctc gag gag gac cat acc agc     528
   Phe Glu Ala Thr Leu Glu Glu Asp Val Leu Glu Glu Asp His Thr Ser
                   165                 170                 175
   gga ttg ccg aac cgc gtc cct gtg ttt cac ggc tac tcg gcc agt gga        576
   Gly Leu Pro Asn Arg Val Pro Val Phe His Gly Tyr Ser Ala Ser Gly
               180                 185                 190
   aat gtc acg gcg cca tat gtg ttt gtg aac tat ggc act gtg gac gat        624
   Asn Val Thr Ala Pro Tyr Val Phe Val Asn Tyr Gly Thr Val Asp Asp
           195                 200                 205
   ttc cag gac ctc gtg gac cgg aat gtc agt ctc gga ggt aaa att gct        672
   Phe Gln Asp Leu Val Asp Arg Asn Val Ser Leu Gly Gly Lys Ile Ala
       210                 215                 220
   att gcc aag tat gga cgc atc ttc cgc gga ttg aag gtc aag aga gcg        720
   Ile Ala Lys Tyr Gly Arg Ile Phe Arg Gly Leu Lys Val Lys Arg Ala
   225                 230                 235                 240
   cag gaa ttg ggc atg tct gga gtg att ctg tat gat gat ccc gag tcg        768
   Gln Glu Leu Gly Met Ser Gly Val Ile Leu Tyr Asp Asp Pro Glu Ser
                   245                 250                 255
   gat ggg gag att atc gag gaa aat ggc tat gct ccg tat ccg gag ggg        816
   Asp Gly Glu Ile Ile Glu Glu Asn Gly Tyr Ala Pro Tyr Pro Glu Gly
               260                 265                 270
   ccg gcc agg aac ccg agt gct gtt cag agg gga agt acc cag ttt ttg        864
   Pro Ala Arg Asn Pro Ser Ala Val Gln Arg Gly Ser Thr Gln Phe Leu
           275                 280                 285
   agt atc gca ccg ggt gac cca act acc ccc gga tac cca tcc aag ccg        912
   Ser Ile Ala Pro Gly Asp Pro Thr Thr Pro Gly Tyr Pro Ser Lys Pro
       290                 295                 300
   ggc tgt gat cgc cag gac ccg tat gag tcc atc ccc ttg att cct tct        960
   Gly Cys Asp Arg Gln Asp Pro Tyr Glu Ser Ile Pro Leu Ile Pro Ser
   305                 310                 315                 320
   ctg ccc atc tca tac cag gag gtg gtg ccg ttg cta aag gcc ctg aat       1008
   Leu Pro Ile Ser Tyr Gln Glu Val Val Pro Leu Leu Lys Ala Leu Asn
                   325                 330                 335
   ggc cac ggt ccc aag gcg tct gac ttc aac gaa tac tgg caa ggc ggt       1056
   Gly His Gly Pro Lys Ala Ser Asp Phe Asn Glu Tyr Trp Gln Gly Gly
               340                 345                 350
   aag ctg ggc agc aag ggc gtc gag tac tac att ggc ccg acc ccg gat       1104
   Lys Leu Gly Ser Lys Gly Val Glu Tyr Tyr Ile Gly Pro Thr Pro Asp
           355                 360                 365
   gat gtt caa atc aat ctg tac aat gaa cag gag tac gtg acg act cct       1152
   Asp Val Gln Ile Asn Leu Tyr Asn Glu Gln Glu Tyr Val Thr Thr Pro
       370                 375                 380
   ctg tgg aac gtc att ggc att atc aag ggt gcc atc ccg gac gag gtc       1200
   Leu Trp Asn Val Ile Gly Ile Ile Lys Gly Ala Ile Pro Asp Glu Val
   385                 390                 395                 400
   gtg atc atg ggc aac cac cgc gat gcc tgg att gcc ggt ggc gct gga       1248
   Val Ile Met Gly Asn His Arg Asp Ala Trp Ile Ala Gly Gly Ala Gly
                   405                 410                 415
   gat ccg aac agc gga tct gcc gct ctc aac gaa gtt gtg cgc agc ttc       1296
   Asp Pro Asn Ser Gly Ser Ala Ala Leu Asn Glu Val Val Arg Ser Phe
               420                 425                 430
   ggc gaa gcg ctt aag gcc gga tgg aag ccg ctc cga acc att gtc ttt       1344
   Gly Glu Ala Leu Lys Ala Gly Trp Lys Pro Leu Arg Thr Ile Val Phe
   435 440 445
   gcc agc tgg gac ggt gaa gag tac gga ttg ctg ggt tct acc gag tgg 1392
   Ala Ser Trp Asp Gly Glu Glu Tyr Gly Leu Leu Gly Ser Thr Glu Trp
   450 455 460
   gtg gaa gag cat ctg ccc tgg ttg tcc aag gcc aac att gcc tat ctc 1440
   Val Glu Glu His Leu Pro Trp Leu Ser Lys Ala Asn Ile Ala Tyr Leu
   465 470 475 480
   aac gtg gac gta gcg gcg tcg gga acg agc ttg cgc ccg agg gcc agc 1488
   Asn Val Asp Val Ala Ala Ser Gly Thr Ser Leu Arg Pro Arg Ala Ser
   485 490 495
   ccg ctg ctg aac aag ctg atc tac gag gtc act ggt cta gtg aag tcg 1536
   Pro Leu Leu Asn Lys Leu Ile Tyr Glu Val Thr Gly Leu Val Lys Ser
   500 505 510
   ccc aac cag act gtg cct gga cag aca gtc cgg gac ctc tgg gac ggc 1584
   Pro Asn Gln Thr Val Pro Gly Gln Thr Val Arg Asp Leu Trp Asp Gly
   515 520 525
   gac att gcc acc atg ggc agt ggc agt gac ttt acg gcc ttc cag gac 1632
   Asp Ile Ala Thr Met Gly Ser Gly Ser Asp Phe Thr Ala Phe Gln Asp
   530 535 540
   ttt gcg ggt gtg gca agc tac gac ctc gga ttc agc cgc ggt ccg aag 1680
   Phe Ala Gly Val Ala Ser Tyr Asp Leu Gly Phe Ser Arg Gly Pro Lys
   545 550 555 560
   gac ccg gtt tac cac tac cac tcg aac tac gac agc ttc gac tgg atg 1728
   Asp Pro Val Tyr His Tyr His Ser Asn Tyr Asp Ser Phe Asp Trp Met
   565 570 575
   gac cgg ttc ggc gac ccg ggc tgg aag tac cat gaa gca acc acc aag 1776
   Asp Arg Phe Gly Asp Pro Gly Trp Lys Tyr His Glu Ala Thr Thr Lys
   580 585 590
   atc tgg gct ctg gca gcg gcc aag ctg gtg gag acg ccg gtg gtg gcc 1824
   Ile Trp Ala Leu Ala Ala Ala Lys Leu Val Glu Thr Pro Val Val Ala
   595 600 605
   ttc aat gct acg gac tac gcg ctg ggc ctg gag aag tat ctt gac aag 1872
   Phe Asn Ala Thr Asp Tyr Ala Leu Gly Leu Glu Lys Tyr Leu Asp Lys
   610 615 620
   atc aag ccg gcg gcg aag caa ctc ccc gag ggg aca tcg ttc gac ttt 1920
   Ile Lys Pro Ala Ala Lys Gln Leu Pro Glu Gly Thr Ser Phe Asp Phe
   625 630 635 640
   ggt ccg ctc gac gcg gcg atc gcg gag ttt caa gcg gtg tcc gcg aaa 1968
   Gly Pro Leu Asp Ala Ala Ile Ala Glu Phe Gln Ala Val Ser Ala Lys
   645 650 655
   ttt gac gct tac gcg gcg gat ctg agc gcg cag ctg ggc gac gat gtg 2016
   Phe Asp Ala Tyr Ala Ala Asp Leu Ser Ala Gln Leu Gly Asp Asp Val
   660 665 670
   ccg tgg tac cac tgg tgg aag aag gtg cgg ctc tac ttc cag atc cgc 2064
   Pro Trp Tyr His Trp Trp Lys Lys Val Arg Leu Tyr Phe Gln Ile Arg
   675 680 685
   atc gtc aac gac aag tac aag ggc att gaa cga cag ttc ctg tac cag 2112
   Ile Val Asn Asp Lys Tyr Lys Gly Ile Glu Arg Gln Phe Leu Tyr Gln
       690                 695                 700
   ccg ggc ctg gac ggg cgc agc tgg ttt aag cac gta gtg ttt gca ccg        2160
   Pro Gly Leu Asp Gly Arg Ser Trp Phe Lys His Val Val Phe Ala Pro
   705                 710                 715                 720
   ggc atc tgg acg gga tac tca ggc gcg acg tat ccc gga ttg gtg gag        2208
   Gly Ile Trp Thr Gly Tyr Ser Gly Ala Thr Tyr Pro Gly Leu Val Glu
                   725                 730                 735
   agc ttg gaa gcg ggt gat ttg ctg aat tct cag aaa tgg cgc gcg atc        2256
   Ser Leu Glu Ala Gly Asp Leu Leu Asn Ser Gln Lys Trp Arg Ala Ile
               740                 745                 750
   atc atc gag cgc att gag gct gcg acg aag cta ctg cag tga                2298
   Ile Ile Glu Arg Ile Glu Ala Ala Thr Lys Leu Leu Gln
           755                 760                 765
<210> 70
   <211> 765
   <212> PRT
   <213> Aspergillus niger
<400> 70
   Met Pro Ser Glu Ser Thr Pro Leu Leu Thr Thr Val Gly Ile Arg Pro
   1               5                   10                  15
   His Arg Arg Arg Tyr Pro His His Thr Leu Arg Arg Phe Cys Thr Ile
               20                  25                  30
   Ile Leu Thr Ser Ile Leu Thr Leu Ser Leu Cys Leu Phe Leu Thr Pro
           35                  40                  45
   Thr Ala Leu Leu Pro Arg Ala His Gly Ser Ile Trp Ser Tyr Leu Pro
       50                  55                  60
   Trp Ala Asn Pro Phe Pro His Ser Ser Trp Pro Glu Gly His Gly Leu
   65                  70                  75                  80
   Ser Tyr Ala Glu Leu Gln Ala Ile Leu Lys Glu Thr Pro Ser Ala Glu
                   85                  90                  95
   Lys Ala Arg Glu Trp Ser Thr Tyr Tyr Thr Ala Gly Pro His Leu Ala
               100                 105                 110
   Gly Lys Asn Leu Ser Gln Ala Val Trp Thr Lys Glu Arg Trp Ala Glu
           115                 120                 125
   Phe Gly Val Pro Glu Thr Asp Ile Val Ala Tyr Asp Ile Phe Leu Asn
       130                 135                 140
   Tyr Pro Val Asp His Arg Leu Ala Leu Leu Lys Lys Asp Glu Val Val
   145                 150                 155                 160
   Phe Glu Ala Thr Leu Glu Glu Asp Val Leu Glu Glu Asp His Thr Ser
   165 170 175
   Gly Leu Pro Asn Arg Val Pro Val Phe His Gly Tyr Ser Ala Ser Gly
   180 185 190
   Asn Val Thr Ala Pro Tyr Val Phe Val Asn Tyr Gly Thr Val Asp Asp
   195 200 205
   Phe Gln Asp Leu Val Asp Arg Asn Val Ser Leu Gly Gly Lys Ile Ala
   210 215 220
   Ile Ala Lys Tyr Gly Arg Ile Phe Arg Gly Leu Lys Val Lys Arg Ala
   225 230 235 240
   Gln Glu Leu Gly Met Ser Gly Val Ile Leu Tyr Asp Asp Pro Glu Ser
   245 250 255
   Asp Gly Glu Ile Ile Glu Glu Asn Gly Tyr Ala Pro Tyr Pro Glu Gly
   260 265 270
   Pro Ala Arg Asn Pro Ser Ala Val Gln Arg Gly Ser Thr Gln Phe Leu
   275 280 285
   Ser Ile Ala Pro Gly Asp Pro Thr Thr Pro Gly Tyr Pro Ser Lys Pro
   290 295 300
   Gly Cys Asp Arg Gln Asp Pro Tyr Glu Ser Ile Pro Leu Ile Pro Ser
   305 310 315 320
   Leu Pro Ile Ser Tyr Gln Glu Val Val Pro Leu Leu Lys Ala Leu Asn
   325 330 335
   Gly His Gly Pro Lys Ala Ser Asp Phe Asn Glu Tyr Trp Gln Gly Gly
   340 345 350
   Lys Leu Gly Ser Lys Gly Val Glu Tyr Tyr Ile Gly Pro Thr Pro Asp
   355 360 365
   Asp Val Gln Ile Asn Leu Tyr Asn Glu Gln Glu Tyr Val Thr Thr Pro
   370 375 380
   Leu Trp Asn Val Ile Gly Ile Ile Lys Gly Ala Ile Pro Asp Glu Val
   385 390 395 400
   Val Ile Met Gly Asn His Arg Asp Ala Trp Ile Ala Gly Gly Ala Gly
   405 410 415
   Asp Pro Asn Ser Gly Ser Ala Ala Leu Asn Glu Val Val Arg Ser Phe
   420 425 430
   Gly Glu Ala Leu Lys Ala Gly Trp Lys Pro Leu Arg Thr Ile Val Phe
   435 440 445
   Ala Ser Trp Asp Gly Glu Glu Tyr Gly Leu Leu Gly Ser Thr Glu Trp
   450 455 460
   Val Glu Glu His Leu Pro Trp Leu Ser Lys Ala Asn Ile Ala Tyr Leu
   465 470 475 480
   Asn Val Asp Val Ala Ala Ser Gly Thr Ser Leu Arg Pro Arg Ala Ser
   485 490 495
   Pro Leu Leu Asn Lys Leu Ile Tyr Glu Val Thr Gly Leu Val Lys Ser
   500 505 510
   Pro Asn Gln Thr Val Pro Gly Gln Thr Val Arg Asp Leu Trp Asp Gly
   515 520 525
   Asp Ile Ala Thr Met Gly Ser Gly Ser Asp Phe Thr Ala Phe Gln Asp
   530 535 540
   Phe Ala Gly Val Ala Ser Tyr Asp Leu Gly Phe Ser Arg Gly Pro Lys
   545 550 555 560
   Asp Pro Val Tyr His Tyr His Ser Asn Tyr Asp Ser Phe Asp Trp Met
   565 570 575
   Asp Arg Phe Gly Asp Pro Gly Trp Lys Tyr His Glu Ala Thr Thr Lys
   580 585 590
   Ile Trp Ala Leu Ala Ala Ala Lys Leu Val Glu Thr Pro Val Val Ala
   595 600 605
   Phe Asn Ala Thr Asp Tyr Ala Leu Gly Leu Glu Lys Tyr Leu Asp Lys
   610 615 620
   Ile Lys Pro Ala Ala Lys Gln Leu Pro Glu Gly Thr Ser Phe Asp Phe
   625 630 635 640
   Gly Pro Leu Asp Ala Ala Ile Ala Glu Phe Gln Ala Val Ser Ala Lys
   645 650 655
   Phe Asp Ala Tyr Ala Ala Asp Leu Ser Ala Gln Leu Gly Asp Asp Val
   660 665 670
   Pro Trp Tyr His Trp Trp Lys Lys Val Arg Leu Tyr Phe Gln Ile Arg
   675 680 685
   Ile Val Asn Asp Lys Tyr Lys Gly Ile Glu Arg Gln Phe Leu Tyr Gln
   690 695 700
   Pro Gly Leu Asp Gly Arg Ser Trp Phe Lys His Val Val Phe Ala Pro
   705 710 715 720
   Gly Ile Trp Thr Gly Tyr Ser Gly Ala Thr Tyr Pro Gly Leu Val Glu
   725 730 735
   Ser Leu Glu Ala Gly Asp Leu Leu Asn Ser Gln Lys Trp Arg Ala Ile
   740 745 750
   Ile Ile Glu Arg Ile Glu Ala Ala Thr Lys Leu Leu Gln
   755 760 765
<210> 71
   <211> 2694
   <212> DNA
   <213> Aspergillus niger
<400> 71
   tagatgatgg ctagtatccc atgtcctgca tcaaaatccg tcatatggtc aagataacaa 60
   gtccagtgca agtccaatac ccctttcaag gcgagcatga tagcaaaagt tttgcttaat 120
   ataacggtag cactagcccc tcgatccacc ctatacgaag tggatatcgc agttcacggc 180
   caggatgcga atcagggtct tgccaagcgc tctttgatct ctaggcgaac gggccgataa 240
   ccacgaagga ggcgctattg tcagggatta aattgcattg tactgtgcaa aggcccgttg 300
   aactaggctg tgaaaccgac aaactcaaat gccccaaatg ctaacctgga atagtagctg 360
   ttctgttctt gttccagtcg aaggatggtt agctaaccgc gacggcttat taactaactc 420
   tagtcgaccg cgcggatgac agcttgtcat tggataaggg ggaacaagtc cgcggtgact 480
   aacaaagaag ttttagcgct ggacgaagaa tcgggtgcct ccgcctgcat ccgaggatgc 540
   gaggccacgc catcccactt tgggtttgtc gtcactctgg ctgggtgtgc agtgttgaac 600
   agcatttagt tgtgatctca atcgcaaatg gacaggagat gatattcatg tcataatcag 660
   agtgccatgt cagcgaccat gtcttgccaa gagtcatcat ttccggtaaa accgacggag 720
   gtaaaacccc ggtcattcac tagttcgagg gagggaatag tctcgcaggt ctcttcttct 780
   ttcttcctcg ggtggcggga tggatcactt cttcatttga tggttgcctg ccaacgaccc 840
   acttgactag gtttccccct tgatgggatt cgttggtttt accagacaga gcctcttcac 900
   agcccagcgc gtgttccgca aaagcaacag cccaagggtc tgaaggctga cattggagcg 960
   ggtctgacgc aggatggctt gctgttaaaa aaactcagga acaggccaag acgaagcgac 1020
   cgcgcaaggc tgagcagttc tccgatttcc ccgtgcccct cctgcgcctc ccatgggcga 1080
   cttttgatcc cagatccatc gtttctgcac cgaccatata agaagctccg cacccccaaa 1140
   ctcccctggc aagttcctgc ccccaaatta cgtgagacag ggaaccatca cagcaacacc 1200
   atgaagagca ccactcttct ttccttggcc tgggctgccc agtccgccta ttccctctct 1260
   atccacgagc gcgatgaacc cgctactctt cagttcaact ttgaacgtcg tcagatcgcc 1320
   gaccggtccc gtcggaagcg atcgacggcc tcggccgacc tcgttaacct ggtatgttcc 1380
   catcccgagt ctcaaatcag gggaattatg acggtcgcta atgcaggttt ctaggctacg 1440
   aatcttggct acacgatgaa cctcacactc ggcactcccg gccaggaagt cagtgtgacg 1500
   ttggacaccg gcagcagcga tctctgggtc aatggggcca actcgtccgt ctgcccctgt 1560
   accgattacg gctcttacaa ctcaagcgct tcttccacct acaccttcgt gaacgatgag 1620
   ttttatatcc agtatgtcga cggcagtgaa gccacaggcg actatgtcaa cgatactcta 1680
   aagttctcca atgtgacttt gacgaacttt caatttgccg tcgcatatga cggcgactcc 1740
   gagggtaagt cttcgctatt ccctcacttt cattttacac tttgctaacg gttttaccat 1800
   gcagaggggg tcctcggtat cggatacgcc agcaatgaag ccagccaggc caccgtcggt 1860
   ggtggtgaat acaccaactt ccccgaagcc ctcgtcgatc aaggcgcgat caactggccg 1920
   gcctacagtc tatggctcga tgacctcgac gaaggaaaag gcaccattct gttcggcgga 1980
   gtcaacaccg ccaagtacta cggcagcctg cagaccctgc ctatcgtctc catcgaagac 2040
   atgtacgtcg agttcgcggt caacctgacg gccgtgcacc ttgagaagaa cggcaactcc 2100
   gtctcggtca acaacagcgc cacgcaattc cccatccccg ccgtgctgga cagcggcacg 2160
   gccctgacct acatcccgac ctccgccgca gccagcatct acgaggccgt cggtgcccaa 2220
   tacctgagcg agtacgggta cggagtgatc gagtgcgacg tcaaggacga agacttcacc 2280
   ttcctgttcg actttggatc cttcaacatg agcgttgaca tcagcgagat gatcctcgag 2340
   gccagttccg acatgaccga catgaacgtt tgtacgtttg gcctcgcagt gatcgaaaat 2400
   gaggccctgc tgggcgatac cttcctgcgc agcgcatacg tcgtctacga tctcggaaac 2460
   aacgagatct ccctggccaa ggccaacttc aaccccggcg aggaccacgt cctggagatc 2520
   ggcaccggat cggatgccgt gcccaaggcg acgggggcga cggcgaccgg cgcggcagcc 2580
   acatccacgg cctcgagcga caagtcggac aaggagagtt cggctacagt gccgcgcagc 2640
   cagattgtct cgctggtggc gggagtcttg gtcggtgttt tcttggttct gtaa 2694
<210> 72
   <211> 1371
   <212> DNA
   <213> Aspergillus niger
<220>
   <221> CDS
   <222> (1)..(1371)
<400> 72
   atg aag agc acc act ctt ctt tcc ttg gcc tgg gct gcc cag tcc gcc       48
   Met Lys Ser Thr Thr Leu Leu Ser Leu Ala Trp Ala Ala Gln Ser Ala
   1               5                   10                  15
   tat tcc ctc tct atc cac gag cgc gat gaa ccc gct act ctt cag ttc       96
   Tyr Ser Leu Ser Ile His Glu Arg Asp Glu Pro Ala Thr Leu Gln Phe
               20                  25                  30
   aac ttt gaa cgt cgt cag atc gcc gac cgg tcc cgt cgg aag cga tcg      144
   Asn Phe Glu Arg Arg Gln Ile Ala Asp Arg Ser Arg Arg Lys Arg Ser
           35                  40                  45
   acg gcc tcg gcc gac ctc gtt aac ctg gct acg aat ctt ggc tac acg      192
   Thr Ala Ser Ala Asp Leu Val Asn Leu Ala Thr Asn Leu Gly Tyr Thr
       50                  55                  60
   atg aac ctc aca ctc ggc act ccc ggc cag gaa gtc agt gtg acg ttg      240
   Met Asn Leu Thr Leu Gly Thr Pro Gly Gln Glu Val Ser Val Thr Leu
   65                  70                  75                  80
   gac acc ggc agc agc gat ctc tgg gtc aat ggg gcc aac tcg tcc gtc      288
   Asp Thr Gly Ser Ser Asp Leu Trp Val Asn Gly Ala Asn Ser Ser Val
                   85                  90                  95
   tgc ccc tgt acc gat tac ggc tct tac aac tca agc gct tct tcc acc      336
   Cys Pro Cys Thr Asp Tyr Gly Ser Tyr Asn Ser Ser Ala Ser Ser Thr
               100                 105                 110
   tac acc ttc gtg aac gat gag ttt tat atc cag tat gtc gac ggc agt      384
   Tyr Thr Phe Val Asn Asp Glu Phe Tyr Ile Gln Tyr Val Asp Gly Ser
           115                 120                 125
   gaa gcc aca ggc gac tat gtc aac gat act cta aag ttc tcc aat gtg      432
   Glu Ala Thr Gly Asp Tyr Val Asn Asp Thr Leu Lys Phe Ser Asn Val
       130                 135                 140
   act ttg acg aac ttt caa ttt gcc gtc gca tat gac ggc gac tcc gag      480
   Thr Leu Thr Asn Phe Gln Phe Ala Val Ala Tyr Asp Gly Asp Ser Glu
   145                 150                 155                 160
   gag ggg gtc ctc ggt atc gga tac gcc agc aat gaa gcc agc cag gcc      528
   Glu Gly Val Leu Gly Ile Gly Tyr Ala Ser Asn Glu Ala Ser Gln Ala
                   165                 170                 175
   acc gtc ggt ggt ggt gaa tac acc aac ttc ccc gaa gcc ctc gtc gat      576
   Thr Val Gly Gly Gly Glu Tyr Thr Asn Phe Pro Glu Ala Leu Val Asp
               180                 185                 190
   caa ggc gcg atc aac tgg ccg gcc tac agt cta tgg ctc gat gac ctc      624
   Gln Gly Ala Ile Asn Trp Pro Ala Tyr Ser Leu Trp Leu Asp Asp Leu
           195                 200                 205
   gac gaa gga aaa ggc acc att ctg ttc ggc gga gtc aac acc gcc aag      672
   Asp Glu Gly Lys Gly Thr Ile Leu Phe Gly Gly Val Asn Thr Ala Lys
       210                 215                 220
   tac tac ggc agc ctg cag acc ctg cct atc gtc tcc atc gaa gac atg      720
   Tyr Tyr Gly Ser Leu Gln Thr Leu Pro Ile Val Ser Ile Glu Asp Met
   225                 230                 235                 240
   tac gtc gag ttc gcg gtc aac ctg acg gcc gtg cac ctt gag aag aac      768
   Tyr Val Glu Phe Ala Val Asn Leu Thr Ala Val His Leu Glu Lys Asn
                   245                 250                 255
   ggc aac tcc gtc tcg gtc aac aac agc gcc acg caa ttc ccc atc ccc        816
   Gly Asn Ser Val Ser Val Asn Asn Ser Ala Thr Gln Phe Pro Ile Pro
               260                 265                 270
   gcc gtg ctg gac agc ggc acg gcc ctg acc tac atc ccg acc tcc gcc        864
   Ala Val Leu Asp Ser Gly Thr Ala Leu Thr Tyr Ile Pro Thr Ser Ala
           275                 280                 285
   gca gcc agc atc tac gag gcc gtc ggt gcc caa tac ctg agc gag tac        912
   Ala Ala Ser Ile Tyr Glu Ala Val Gly Ala Gln Tyr Leu Ser Glu Tyr
       290                 295                 300
   ggg tac gga gtg atc gag tgc gac gtc aag gac gaa gac ttc acc ttc        960
   Gly Tyr Gly Val Ile Glu Cys Asp Val Lys Asp Glu Asp Phe Thr Phe
   305                 310                 315                 320
   ctg ttc gac ttt gga tcc ttc aac atg agc gtt gac atc agc gag atg       1008
   Leu Phe Asp Phe Gly Ser Phe Asn Met Ser Val Asp Ile Ser Glu Met
                   325                 330                 335
   atc ctc gag gcc agt tcc gac atg acc gac atg aac gtt tgt acg ttt       1056
   Ile Leu Glu Ala Ser Ser Asp Met Thr Asp Met Asn Val Cys Thr Phe
               340                 345                 350
   ggc ctc gca gtg atc gaa aat gag gcc ctg ctg ggc gat acc ttc ctg       1104
   Gly Leu Ala Val Ile Glu Asn Glu Ala Leu Leu Gly Asp Thr Phe Leu
           355                 360                 365
   cgc agc gca tac gtc gtc tac gat ctc gga aac aac gag atc tcc ctg       1152
   Arg Ser Ala Tyr Val Val Tyr Asp Leu Gly Asn Asn Glu Ile Ser Leu
       370                 375                 380
   gcc aag gcc aac ttc aac ccc ggc gag gac cac gtc ctg gag atc ggc       1200
   Ala Lys Ala Asn Phe Asn Pro Gly Glu Asp His Val Leu Glu Ile Gly
   385                 390                 395                 400
   acc gga tcg gat gcc gtg ccc aag gcg acg ggg gcg acg gcg acc ggc       1248
   Thr Gly Ser Asp Ala Val Pro Lys Ala Thr Gly Ala Thr Ala Thr Gly
                   405                 410                 415
   gcg gca gcc aca tcc acg gcc tcg agc gac aag tcg gac aag gag agt       1296
   Ala Ala Ala Thr Ser Thr Ala Ser Ser Asp Lys Ser Asp Lys Glu Ser
               420                 425                 430
   tcg gct aca gtg ccg cgc agc cag att gtc tcg ctg gtg gcg gga gtc       1344
   Ser Ala Thr Val Pro Arg Ser Gln Ile Val Ser Leu Val Ala Gly Val
           435                 440                 445
   ttg gtc ggt gtt ttc ttg gtt ctg taa                                   1371
   Leu Val Gly Val Phe Leu Val Leu
       450                 455
<210> 73
   <211> 456
   <212> PRT
   <213> Aspergillus niger
<400> 73
   Met Lys Ser Thr Thr Leu Leu Ser Leu Ala Trp Ala Ala Gln Ser Ala
   1               5                   10                  15
   Tyr Ser Leu Ser Ile His Glu Arg Asp Glu Pro Ala Thr Leu Gln Phe
   20 25 30
   Asn Phe Glu Arg Arg Gln Ile Ala Asp Arg Ser Arg Arg Lys Arg Ser
   35 40 45
   Thr Ala Ser Ala Asp Leu Val Asn Leu Ala Thr Asn Leu Gly Tyr Thr
   50 55 60
   Met Asn Leu Thr Leu Gly Thr Pro Gly Gln Glu Val Ser Val Thr Leu
   65 70 75 80
   Asp Thr Gly Ser Ser Asp Leu Trp Val Asn Gly Ala Asn Ser Ser Val
   85 90 95
   Cys Pro Cys Thr Asp Tyr Gly Ser Tyr Asn Ser Ser Ala Ser Ser Thr
   100 105 110
   Tyr Thr Phe Val Asn Asp Glu Phe Tyr Ile Gln Tyr Val Asp Gly Ser
   115 120 125
   Glu Ala Thr Gly Asp Tyr Val Asn Asp Thr Leu Lys Phe Ser Asn Val
   130 135 140
   Thr Leu Thr Asn Phe Gln Phe Ala Val Ala Tyr Asp Gly Asp Ser Glu
   145 150 155 160
   Glu Gly Val Leu Gly Ile Gly Tyr Ala Ser Asn Glu Ala Ser Gln Ala
   165 170 175
   Thr Val Gly Gly Gly Glu Tyr Thr Asn Phe Pro Glu Ala Leu Val Asp
   180 185 190
   Gln Gly Ala Ile Asn Trp Pro Ala Tyr Ser Leu Trp Leu Asp Asp Leu
   195 200 205
   Asp Glu Gly Lys Gly Thr Ile Leu Phe Gly Gly Val Asn Thr Ala Lys
   210 215 220
   Tyr Tyr Gly Ser Leu Gln Thr Leu Pro Ile Val Ser Ile Glu Asp Met
   225 230 235 240
   Tyr Val Glu Phe Ala Val Asn Leu Thr Ala Val His Leu Glu Lys Asn
   245 250 255
   Gly Asn Ser Val Ser Val Asn Asn Ser Ala Thr Gln Phe Pro Ile Pro
   260 265 270
   Ala Val Leu Asp Ser Gly Thr Ala Leu Thr Tyr Ile Pro Thr Ser Ala
   275 280 285
   Ala Ala Ser Ile Tyr Glu Ala Val Gly Ala Gln Tyr Leu Ser Glu Tyr
       290                 295                 300
   Gly Tyr Gly Val Ile Glu Cys Asp Val Lys Asp Glu Asp Phe Thr Phe
   305                 310                 315                 320
   Leu Phe Asp Phe Gly Ser Phe Asn Met Ser Val Asp Ile Ser Glu Met
                   325                 330                 335
   Ile Leu Glu Ala Ser Ser Asp Met Thr Asp Met Asn Val Cys Thr Phe
               340                 345                 350
   Gly Leu Ala Val Ile Glu Asn Glu Ala Leu Leu Gly Asp Thr Phe Leu
           355                 360                 365
   Arg Ser Ala Tyr Val Val Tyr Asp Leu Gly Asn Asn Glu Ile Ser Leu
       370                 375                 380
   Ala Lys Ala Asn Phe Asn Pro Gly Glu Asp His Val Leu Glu Ile Gly
   385                 390                 395                 400
   Thr Gly Ser Asp Ala Val Pro Lys Ala Thr Gly Ala Thr Ala Thr Gly
                   405                 410                 415
   Ala Ala Ala Thr Ser Thr Ala Ser Ser Asp Lys Ser Asp Lys Glu Ser
               420                 425                 430
   Ser Ala Thr Val Pro Arg Ser Gln Ile Val Ser Leu Val Ala Gly Val
           435                 440                 445
   Leu Val Gly Val Phe Leu Val Leu
       450                 455
<210> 74
   <211> 5057
   <212> DNA
   <213> Aspergillus niger
<400> 74
   gtaagtctac ttgcgcgccc ttgaacagaa aaagaagacg aagagtgtct gccaaaatag      60
   agcacagcta acgtaacgag caaaacaggc aatacacgaa agtcccatcc cagctccggg     120
   accacgaagt gctgccgggc ttttcggaga taccttcaat tataaccacg atacggcatc     180
   cccgacgacg cgctatttcg atcggtccgc agtcagaagc tgatgcgtat gagacactgg     240
   cctatcgcga gaaacggaga cgggaaagtc tttcaacagc caaccaagca tctccagtgc     300
   cggaagaagg tggaccggtt tcggagtccg agccacggaa actgtcgcga aagccgtcta     360
   agttacacga gtatgagaat atgatgggca caggtgcccc tgctgtggcg agttctgtcg     420
   atgttagctc acaagaagct tcttcgaccg tgccctttcc tccgtttgag tcggaaattg 480
   aagccgacga gaaggaagtg ttttcgcaga tcaaacagcc ccgggtgcgc tatgatgtag 540
   aggttgtaac gaaacttgtt gtatattccg gtaggtcaac ccctgtgcgt cgagttataa 600
   agttgcggat gctgactgtg tttgactagg cattgcctgg attgtgatgg agggagcgcc 660
   ttggttcttc gatcggatag ggctggcgcc atagtagctt agtagtcgct gggtgggtcc 720
   gacggccatc acccatttcg aattctgtaa accttttgct atattctcta cggttcagcg 780
   ggctggacta accaaagtcc ttggtgtata acatagctgc cgttcttgta catacaccgt 840
   tgatgatctc ttttctattt gttcttaaaa ggtgggatat ttagccatta gtggaggcga 900
   atgagagttg atactagtag tagtatttct agtagggagt ctaaaatttt aaggtggtgg 960
   tttagtctag ttggctatta ttgatccttg ataaggaacg agaacggact agtagcgatc 1020
   tactggtagc taattgtgga agttgagcca agttgccgat ggatgtggat gtggatgtgg 1080
   atgaatagtg tgatatatgt gtataggcag aacaggaaga gtagtcaata gtctaagcac 1140
   tgtactgata tcctataccg agtcctagag aatgaagata tgacgagttg aggaggaacc 1200
   atggatcgca tgttcactgt ttggtgggaa tacccgcaga aagtgaacag caaatgcata 1260
   gtccatccgc cagccggcac acaccacgtg acatgctgga tgaggaaaag tggccgccaa 1320
   gctccctcgg aagttgaact gatctgctgc tgctccatta cttgcttcgt ctcttcctca 1380
   acttctcgcg gcttcatcgc ctcccctccg tctttgcgcc ttcctcgccc ctcgcccacc 1440
   ccgcgctccc cccgcgcgtt cctatggagc tccaattcac tgcggcgacg cgcgcgactt 1500
   ccaccatcac tatggcagtc gcccatggct gccctcagtg aatacatttg ttttggttct 1560
   tccttccctt tcgctcaccc cggcgccgct cttgttttct tcttcaccct gctcttcctt 1620
   ctcttcttct tcttcttctt cttctttccc ttctctccta tcacccatct tcccattacc 1680
   ttcagacgcg tttacgagct tagcgaatac agcaactttc cattcctcgc gatcgaactc 1740
   gtaccgcttc tttcaccact tctttcaccc aaagaacttg tttctagatt acttctcacc 1800
   atgtctgggg ttttctcgac tatgagaccc agcgtggagg accacccaat gcctgacgta 1860
   tgctatatgc cctcagattc ggcgccccac gaggaggccg tcgataatac cgcccatgac 1920
   cccatggatt gtgctcccga ttcccccgca tctaacatcc ctcaggaaac tgccaaagcc 1980
   ccctccgcag agattccaaa gtatcagctc cgatccgtgc atgttcccgg caatccaaag 2040
   gctgtgagac tcaatggaaa acatgaagtc aagccctcct tccccctccg caccccgctc 2100
   atcagacccg atcatcgttt ctccccgaat agtcgcgcgg cctcattcct cggcgttgat 2160
   gccctccaga agagcggtcg taatgcgcat tcatctcatc ttttaagcca tctgggtacc 2220
   agcaaatatc tatctgccgg cctgaaccac gtctcctggt cgaataacaa acctctcagc 2280
   ctcccttttg gtccagacag gtcaaatgaa accggttacc ttcttggagg caagtctatt 2340
   ggtatagaca agaaaacttt tcatatgaat gtcaataaac attctggctt tactacgcct 2400
   tctcgcaaga gatcaatgga aggtgatggc tatgagcaag ctcaacccat cacttcacct 2460
   tctgctaaat tccgtcgtgt gagggctggt agctttgcac gcagtgctgc tactgaactt 2520
   gaggccactg ccaacgaaga tgattggtac cctggttccc ccatggacat cgacactcct 2580
   gaacacaaca acgaaccaaa catggttaca gcccttgcgg cagttgaatc tcatctcgct 2640
   cttcctgaaa cctctcctaa gtcgcgtcga ctggacgctt ccaaagtcaa gcctgtcacc 2700
   gctcttgcgg tctcgaccac ccctagcact ccagagaacc atatgatgcc tggagcgtgg 2760
   cccgaatcag tgccgtctca ctggtccacc gatgccgcca ccgatatcga agatagccct 2820
   atgatttccg atgaagagat cctatccaac aatacggaca tggccgacgc tggagagccg 2880
   actattcccg tgaattcgga cgccgaacca cagccttcct caccttggta tgcttactgg 2940
   gggacgtggc atagctactt gcagacagtt gtctccattc ccactggaat tactcagaac 3000
   ttttcctatg cggcaaggtc cgctgcgaat gcggttggtg ctgctaggcg acgcgtgtgc 3060
   gcactttgga atcgccgcgg tggacgtcgt cttccttcgc ctcctggatc gcccacgcgc 3120
   ctcaaccttc gcaacctttc tccggaacag caggagcgtg tggatcgtga taggagaatc 3180
   cgaagcggac aatccagccc ccgtaccgac aaatctttcc ccgacttctc caacgttcct 3240
   aatttctccc ataacaacaa cacggctcct acagtcgagt cttctcagat tttgcaggac 3300
   cttgtgtctc aggagcagat gccccaggca ctcagtgact ctgtatctgt tcagcgacgt 3360
   gctcatgagc caaagaatgt gggaactcgc aaacaccctg ggatgccgcc agtgaaacag 3420
   ccagctcgga gatcccctcc acgctcggct cagcgttcag ctcaacgttc aactcaagac 3480
   acagcccaaa agcctctccg tcggcaagga agcccgggta ttcgcaagca aaagcacatt 3540
   acgcgtgctg agcgcgccga gaaccgtgag cgatctgctg cgttcagagc cctcttcgtt 3600
   ggggacatcg ccggcatgaa gaaagctatg gaggctcagg ccgcgcgtga ccgtcaagcc 3660
   tccgccgcag gtcgccgtcc ggttgaacta aacttgatga gaagcagcta tgaactagac 3720
   agagctcaag acttgaacat gttggcgaat gttcagtctc gctcgtttac tgcagttgat 3780
   caactgggtg caactgaaaa ggttctctca tcatcaatca ctgttgcacc caagacaatt 3840
   ctcaagataa gaggaaggcc cagcatttct gggaatagaa ctagatctcg ccgcgccact 3900
   ccgcgcaggg ttcgcttccg cgagcctgtg gtagccgagt tcattgacga cacgcgagat 3960
   tggggtgagg tggaggacaa cctggcaccc catctcggag atacgcttga tgtcaatgag 4020
   agaactaccg aaaccagtgt tggcacgaaa gccgagtaca cagaaaagga aaacgtgccg 4080
   ccagccctgg agaacgagga agagaatgag cccatcgttg acccctggtc ccaaccaacc 4140
   tttgagttcc cgctgggtcg agcagtatca gctgtcagtc tattttatcc cgagccaaaa 4200
   cccattccag agggcagaac cgagtctgtg tacgccgacc gttggagaca gatccaagaa 4260
   gaagagaggc agaaagcgct cccatcacgt attaaaccgg atggtccagc tgtacggcct 4320
   ctctctcccg aatgggaagc tcaaataaaa aaaaacttca agatgggcgc gtatccggag 4380
   gaaaaaccgt cgcgaccacc ctctccggtg accctctaac gaagaggagc ctcgccacct 4440
   gttacacgcg cggcgagtgg cttaacgacg aggtgatcaa cggatacctt gccctcatcg 4500
   ttgattacct ccgacgcaag aaccacaacg ccggccgcaa cgacaagccc cgattccatg 4560
   ccttcaattc gtttttcttt tcgaatctca gagacaaagg ctacgagtcc gtggcccggt 4620
   gggccaaacg ggccaagatc ggcggctcac tcctcctcga cgtagacacc gtctatatcc 4680
   cggtccacaa cagccagcac tggacgctgg ttgttgtgcg accgggggaa cgctcaatcg 4740
   agcatttcga ttctcttggg gcccggtctc gccgccacat cgcggtagtc caaacttggc 4800
   tccgcggcga gctgggcccc aagtacgtcg aggaggagtg gcgcgtgttg ccgtccctgt 4860
   ccccccagca agacaatggt tcagattgcg gggtcttcct cctcaccacg gccaaggctg 4920
   tggccatcgg cctcgagcca ctgtcttacg gcgcgcaaga cacaccgctc ttgcgccgta 4980
   agatagtggc cgagctgatg gccggcggcc tggaggggga atttgacccc gcccagcaag 5040
   accatgtctt gctatga 5057
<210> 75
   <211> 3783
   <212> DNA
   <213> Aspergillus niger
<220>
   <221> CDS
   <222> (1)..(3783)
<400> 75
   atg gat cgc atg ttc act gtt tgg tgg gaa tac ccg cag aaa gtg aac          48
   Met Asp Arg Met Phe Thr Val Trp Trp Glu Tyr Pro Gln Lys Val Asn
   1               5                   10                  15
   agc aaa tgc ata gtc cat ccg cca gcc ggc aca cac cac gtg aca tgc          96
   Ser Lys Cys Ile Val His Pro Pro Ala Gly Thr His His Val Thr Cys
               20                  25                  30
   tgg atg agg aaa agt ggc cgc caa gct ccc tcg gaa gtt gaa ctg atc          144
   Trp Met Arg Lys Ser Gly Arg Gln Ala Pro Ser Glu Val Glu Leu Ile
           35                  40                  45
   tgc tgc tgc tcc att act tgc ttc gtc tct tcc tca act tct cgc ggc          192
   Cys Cys Cys Ser Ile Thr Cys Phe Val Ser Ser Ser Thr Ser Arg Gly
       50                  55                  60
   ttc atc gcc tcc cct ccg tct ttg cgc ctt cct cgc ccc tcg ccc acc          240
   Phe Ile Ala Ser Pro Pro Ser Leu Arg Leu Pro Arg Pro Ser Pro Thr
   65                  70                  75                  80
   ccg cgc tcc ccc cgc gcg ttc cta tgg agc tcc aat tca ctg cgg cga          288
   Pro Arg Ser Pro Arg Ala Phe Leu Trp Ser Ser Asn Ser Leu Arg Arg
                   85                  90                  95
   cgc gcg cga ctt cca cca tca cta tgg cag tcg ccc atg gct gcc ctc          336
   Arg Ala Arg Leu Pro Pro Ser Leu Trp Gln Ser Pro Met Ala Ala Leu
                   100                 105                 110
   agt gaa tac att tgt ttt ggt tct tcc ttc cct ttc gct cac ccc ggc      384
   Ser Glu Tyr Ile Cys Phe Gly Ser Ser Phe Pro Phe Ala His Pro Gly
               115                 120                 125
   gcc gct ctt gtt ttc ttc ttc acc ctg ctc ttc ctt ctc ttc ttc ttc      432
   Ala Ala Leu Val Phe Phe Phe Thr Leu Leu Phe Leu Leu Phe Phe Phe
       130                 135                 140
   ttc ttc ttc ttc ttt ccc ttc tct cct atc acc cat ctt ccc att acc      480
   Phe Phe Phe Phe Phe Pro Phe Ser Pro Ile Thr His Leu Pro Ile Thr
   145                 150                 155                 160
   ttc aga cgc gtt tac gag ctt agc gaa tac agc aac ttt cca ttc ctc      528
   Phe Arg Arg Val Tyr Glu Leu Ser Glu Tyr Ser Asn Phe Pro Phe Leu
                   165                 170                 175
   gcg atc gaa ctc gta ccg ctt ctt tca cca ctt ctt tca ccc aaa gaa      576
   Ala Ile Glu Leu Val Pro Leu Leu Ser Pro Leu Leu Ser Pro Lys Glu
               180                 185                 190
   ctt gtt tct aga tta ctt ctc acc atg tct ggg gtt ttc tcg act atg      624
   Leu Val Ser Arg Leu Leu Leu Thr Met Ser Gly Val Phe Ser Thr Met
           195                 200                 205
   aga ccc agc gtg gag gac cac cca atg cct gac gta tgc tat atg ccc      672
   Arg Pro Ser Val Glu Asp His Pro Met Pro Asp Val Cys Tyr Met Pro
       210                 215                 220
   tca gat tcg gcg ccc cac gag gag gcc gtc gat aat acc gcc cat gac      720
   Ser Asp Ser Ala Pro His Glu Glu Ala Val Asp Asn Thr Ala His Asp
   225                 230                 235                 240
   ccc atg gat tgt gct ccc gat tcc ccc gca tct aac atc cct cag gaa      768
   Pro Met Asp Cys Ala Pro Asp Ser Pro Ala Ser Asn Ile Pro Gln Glu
                   245                 250                 255
   act gcc aaa gcc ccc tcc gca gag att cca aag tat cag ctc cga tcc      816
   Thr Ala Lys Ala Pro Ser Ala Glu Ile Pro Lys Tyr Gln Leu Arg Ser
               260                 265                 270
   gtg cat gtt ccc ggc aat cca aag gct gtg aga ctc aat gga aaa cat      864
   Val His Val Pro Gly Asn Pro Lys Ala Val Arg Leu Asn Gly Lys His
           275                 280                 285
   gaa gtc aag ccc tcc ttc ccc ctc cgc acc ccg ctc atc aga ccc gat      912
   Glu Val Lys Pro Ser Phe Pro Leu Arg Thr Pro Leu Ile Arg Pro Asp
       290                 295                 300
   cat cgt ttc tcc ccg aat agt cgc gcg gcc tca ttc ctc ggc gtt gat      960
   His Arg Phe Ser Pro Asn Ser Arg Ala Ala Ser Phe Leu Gly Val Asp
   305                 310                 315                 320
   gcc ctc cag aag agc ggt cgt aat gcg cat tca tct cat ctt tta agc     1008
   Ala Leu Gln Lys Ser Gly Arg Asn Ala His Ser Ser His Leu Leu Ser
                   325                 330                 335
   cat ctg ggt acc agc aaa tat cta tct gcc ggc ctg aac cac gtc tcc     1056
   His Leu Gly Thr Ser Lys Tyr Leu Ser Ala Gly Leu Asn His Val Ser
               340                 345                 350
   tgg tcg aat aac aaa cct ctc agc ctc cct ttt ggt cca gac agg tca     1104
   Trp Ser Asn Asn Lys Pro Leu Ser Leu Pro Phe Gly Pro Asp Arg Ser
           355                 360                 365
   aat gaa acc ggt tac ctt ctt gga ggc aag tct att ggt ata gac aag      1152
   Asn Glu Thr Gly Tyr Leu Leu Gly Gly Lys Ser Ile Gly Ile Asp Lys
       370                 375                 380
   aaa act ttt cat atg aat gtc aat aaa cat tct ggc ttt act acg cct      1200
   Lys Thr Phe His Met Asn Val Asn Lys His Ser Gly Phe Thr Thr Pro
   385                 390                 395                 400
   tct cgc aag aga tca atg gaa ggt gat ggc tat gag caa gct caa ccc      1248
   Ser Arg Lys Arg Ser Met Glu Gly Asp Gly Tyr Glu Gln Ala Gln Pro
                   405                 410                 415
   atc act tca cct tct gct aaa ttc cgt cgt gtg agg gct ggt agc ttt      1296
   Ile Thr Ser Pro Ser Ala Lys Phe Arg Arg Val Arg Ala Gly Ser Phe
               420                 425                 430
   gca cgc agt gct gct act gaa ctt gag gcc act gcc aac gaa gat gat      1344
   Ala Arg Ser Ala Ala Thr Glu Leu Glu Ala Thr Ala Asn Glu Asp Asp
           435                 440                 445
   tgg tac cct ggt tcc ccc atg gac atc gac act cct gaa cac aac aac      1392
   Trp Tyr Pro Gly Ser Pro Met Asp Ile Asp Thr Pro Glu His Asn Asn
       450                 455                 460
   gaa cca aac atg gtt aca gcc ctt gcg gca gtt gaa tct cat ctc gct      1440
   Glu Pro Asn Met Val Thr Ala Leu Ala Ala Val Glu Ser His Leu Ala
   465                 470                 475                 480
   ctt cct gaa acc tct cct aag tcg cgt cga ctg gac gct tcc aaa gtc      1488
   Leu Pro Glu Thr Ser Pro Lys Ser Arg Arg Leu Asp Ala Ser Lys Val
                   485                 490                 495
   aag cct gtc acc gct ctt gcg gtc tcg acc acc cct agc act cca gag      1536
   Lys Pro Val Thr Ala Leu Ala Val Ser Thr Thr Pro Ser Thr Pro Glu
               500                 505                 510
   aac cat atg atg cct gga gcg tgg ccc gaa tca gtg ccg tct cac tgg      1584
   Asn His Met Met Pro Gly Ala Trp Pro Glu Ser Val Pro Ser His Trp
           515                 520                 525
   tcc acc gat gcc gcc acc gat atc gaa gat agc cct atg att tcc gat      1632
   Ser Thr Asp Ala Ala Thr Asp Ile Glu Asp Ser Pro Met Ile Ser Asp
       530                 535                 540
   gaa gag atc cta tcc aac aat acg gac atg gcc gac gct gga gag ccg      1680
   Glu Glu Ile Leu Ser Asn Asn Thr Asp Met Ala Asp Ala Gly Glu Pro
   545                 550                 555                 560
   act att ccc gtg aat tcg gac gcc gaa cca cag cct tcc tca cct tgg      1728
   Thr Ile Pro Val Asn Ser Asp Ala Glu Pro Gln Pro Ser Ser Pro Trp
                   565                 570                 575
   tat gct tac tgg ggg acg tgg cat agc tac ttg cag aca gtt gtc tcc      1776
   Tyr Ala Tyr Trp Gly Thr Trp His Ser Tyr Leu Gln Thr Val Val Ser
               580                 585                 590
   att ccc act gga att act cag aac ttt tcc tat gcg gca agg tcc gct      1824
   Ile Pro Thr Gly Ile Thr Gln Asn Phe Ser Tyr Ala Ala Arg Ser Ala
           595                 600                 605
   gcg aat gcg gtt ggt gct gct agg cga cgc gtg tgc gca ctt tgg aat      1872
   Ala Asn Ala Val Gly Ala Ala Arg Arg Arg Val Cys Ala Leu Trp Asn
       610                 615                 620
   cgc cgc ggt gga cgt cgt ctt cct tcg cct cct gga tcg ccc acg cgc      1920
   Arg Arg Gly Gly Arg Arg Leu Pro Ser Pro Pro Gly Ser Pro Thr Arg
   625                 630                 635                 640
   ctc aac ctt cgc aac ctt tct ccg gaa cag cag gag cgt gtg gat cgt      1968
   Leu Asn Leu Arg Asn Leu Ser Pro Glu Gln Gln Glu Arg Val Asp Arg
                   645                 650                 655
   gat agg aga atc cga agc gga caa tcc agc ccc cgt acc gac aaa tct      2016
   Asp Arg Arg Ile Arg Ser Gly Gln Ser Ser Pro Arg Thr Asp Lys Ser
               660                 665                 670
   ttc ccc gac ttc tcc aac gtt cct aat ttc tcc cat aac aac aac acg      2064
   Phe Pro Asp Phe Ser Asn Val Pro Asn Phe Ser His Asn Asn Asn Thr
           675                 680                 685
   gct cct aca gtc gag tct tct cag att ttg cag gac ctt gtg tct cag      2112
   Ala Pro Thr Val Glu Ser Ser Gln Ile Leu Gln Asp Leu Val Ser Gln
       690                 695                 700
   gag cag atg ccc cag gca ctc agt gac tct gta tct gtt cag cga cgt      2160
   Glu Gln Met Pro Gln Ala Leu Ser Asp Ser Val Ser Val Gln Arg Arg
   705                 710                 715                 720
   gct cat gag cca aag aat gtg gga act cgc aaa cac cct ggg atg ccg      2208
   Ala His Glu Pro Lys Asn Val Gly Thr Arg Lys His Pro Gly Met Pro
                   725                 730                 735
   cca gtg aaa cag cca gct cgg aga tcc cct cca cgc tcg gct cag cgt      2256
   Pro Val Lys Gln Pro Ala Arg Arg Ser Pro Pro Arg Ser Ala Gln Arg
               740                 745                 750
   tca gct caa cgt tca act caa gac aca gcc caa aag cct ctc cgt cgg      2304
   Ser Ala Gln Arg Ser Thr Gln Asp Thr Ala Gln Lys Pro Leu Arg Arg
           755                 760                 765
   caa gga agc ccg ggt att cgc aag caa aag cac att acg cgt gct gag      2352
   Gln Gly Ser Pro Gly Ile Arg Lys Gln Lys His Ile Thr Arg Ala Glu
       770                 775                 780
   cgc gcc gag aac cgt gag cga tct gct gcg ttc aga gcc ctc ttc gtt      2400
   Arg Ala Glu Asn Arg Glu Arg Ser Ala Ala Phe Arg Ala Leu Phe Val
   785                 790                 795                 800
   ggg gac atc gcc ggc atg aag aaa gct atg gag gct cag gcc gcg cgt      2448
   Gly Asp Ile Ala Gly Met Lys Lys Ala Met Glu Ala Gln Ala Ala Arg
                   805                 810                 815
   gac cgt caa gcc tcc gcc gca ggt cgc cgt ccg gtt gaa cta aac ttg      2496
   Asp Arg Gln Ala Ser Ala Ala Gly Arg Arg Pro Val Glu Leu Asn Leu
               820                 825                 830
   atg aga agc agc tat gaa cta gac aga gct caa gac ttg aac atg ttg      2544
   Met Arg Ser Ser Tyr Glu Leu Asp Arg Ala Gln Asp Leu Asn Met Leu
           835                 840                 845
   gcg aat gtt cag tct cgc tcg ttt act gca gtt gat caa ctg ggt gca      2592
   Ala Asn Val Gln Ser Arg Ser Phe Thr Ala Val Asp Gln Leu Gly Ala
       850                 855                 860
   act gaa aag gtt ctc tca tca tca atc act gtt gca ccc aag aca att      2640
   Thr Glu Lys Val Leu Ser Ser Ser Ile Thr Val Ala Pro Lys Thr Ile
   865                 870                 875                 880
   ctc aag ata aga gga agg ccc agc att tct ggg aat aga act aga tct      2688
   Leu Lys Ile Arg Gly Arg Pro Ser Ile Ser Gly Asn Arg Thr Arg Ser
                   885                 890                 895
   cgc cgc gcc act ccg cgc agg gtt cgc ttc cgc gag cct gtg gta gcc      2736
   Arg Arg Ala Thr Pro Arg Arg Val Arg Phe Arg Glu Pro Val Val Ala
               900                 905                 910
   gag ttc att gac gac acg cga gat tgg ggt gag gtg gag gac aac ctg      2784
   Glu Phe Ile Asp Asp Thr Arg Asp Trp Gly Glu Val Glu Asp Asn Leu
           915                 920                 925
   gca ccc cat ctc gga gat acg ctt gat gtc aat gag aga act acc gaa      2832
   Ala Pro His Leu Gly Asp Thr Leu Asp Val Asn Glu Arg Thr Thr Glu
       930                 935                 940
   acc agt gtt ggc acg aaa gcc gag tac aca gaa aag gaa aac gtg ccg      2880
   Thr Ser Val Gly Thr Lys Ala Glu Tyr Thr Glu Lys Glu Asn Val Pro
   945                 950                 955                 960
   cca gcc ctg gag aac gag gaa gag aat gag ccc atc gtt gac ccc tgg      2928
   Pro Ala Leu Glu Asn Glu Glu Glu Asn Glu Pro Ile Val Asp Pro Trp
                   965                 970                 975
   tcc caa cca acc ttt gag ttc ccg ctg ggt cga gca gta tca gct gtc      2976
   Ser Gln Pro Thr Phe Glu Phe Pro Leu Gly Arg Ala Val Ser Ala Val
               980                 985                 990
   agt cta ttt tat ccc gag cca aaa  ccc att cca gag ggc  aga acc gag    3024
   Ser Leu Phe Tyr Pro Glu Pro Lys  Pro Ile Pro Glu Gly  Arg Thr Glu
           995                 1000                1005
   tct gtg  tac gcc gac cgt tgg  aga cag atc caa gaa  gaa gag agg      3069
   Ser Val  Tyr Ala Asp Arg Trp  Arg Gln Ile Gln Glu  Glu Glu Arg
       1010                 1015                 1020
   cag aaa  gcg ctc cca tca cat  ggg cgc gta tcc gga  gga aaa acc      3114
   Gln Lys  Ala Leu Pro Ser His  Gly Arg Val Ser Gly  Gly Lys Thr
       1025                 1030                 1035
   gtc gcg  acc acc ctc tcc ggt  gac cct cta acg aag  agg agc ctc      3159
   Val Ala  Thr Thr Leu Ser Gly  Asp Pro Leu Thr Lys  Arg Ser Leu
       1040                 1045                 1050
   gcc acc  tgt tac acg cgc ggc  gag tgg ctt aac gac  gag gtg atc      3204
   Ala Thr  Cys Tyr Thr Arg Gly  Glu Trp Leu Asn Asp  Glu Val Ile
       1055                 1060                 1065
   aac gga  tac ctt gcc ctc atc  gtt gat tac ctc cga  cgc aag aac      3249
   Asn Gly  Tyr Leu Ala Leu Ile  Val Asp Tyr Leu Arg  Arg Lys Asn
       1070                 1075                 1080
   cac aac  gcc ggc cgc aac gac  aag ccc cga ttc cat  gcc ttc aat      3294
   His Asn  Ala Gly Arg Asn Asp  Lys Pro Arg Phe His  Ala Phe Asn
       1085                 1090                 1095
   tcg ttt  ttc ttt tcg aat ctc  aga gac aaa ggc tac  gag tcc gtg      3339
   Ser Phe  Phe Phe Ser Asn Leu  Arg Asp Lys Gly Tyr  Glu Ser Val
       1100                 1105                 1110
   gcc cgg  tgg gcc aaa cgg gcc  aag atc ggc ggc tca  ctc ctc ctc      3384
   Ala Arg  Trp Ala Lys Arg Ala  Lys Ile Gly Gly Ser  Leu Leu Leu
       1115                 1120                 1125
   gac gta  gac acc gtc tat atc  ccg gtc cac aac agc  cag cac tgg      3429
   Asp Val  Asp Thr Val Tyr Ile  Pro Val His Asn Ser  Gln His Trp
           1130                1135                1140
   acg ctg  gtt gtt gtg cga ccg  ggg gaa cgc tca atc  gag cat ttc        3474
   Thr Leu  Val Val Val Arg Pro  Gly Glu Arg Ser Ile  Glu His Phe
       1145                1150                1155
   gat tct  ctt ggg gcc cgg tct  cgc cgc cac atc gcg  gta gtc caa        3519
   Asp Ser  Leu Gly Ala Arg Ser  Arg Arg His Ile Ala  Val Val Gln
       1160                1165                1170
   act tgg  ctc cgc ggc gag ctg  ggc ccc aag tac gtc  gag gag gag        3564
   Thr Trp  Leu Arg Gly Glu Leu  Gly Pro Lys Tyr Val  Glu Glu Glu
       1175                1180                1185
   tgg cgc  gtg ttg ccg tcc ctg  tcc ccc cag caa gac  aat ggt tca        3609
   Trp Arg  Val Leu Pro Ser Leu  Ser Pro Gln Gln Asp  Asn Gly Ser
       1190                1195                1200
   gat tgc  ggg gtc ttc ctc ctc  acc acg gcc aag gct  gtg gcc atc        3654
   Asp Cys  Gly Val Phe Leu Leu  Thr Thr Ala Lys Ala  Val Ala Ile
       1205                1210                1215
   ggc ctc  gag cca ctg tct tac  ggc gcg caa gac aca  ccg ctc ttg        3699
   Gly Leu  Glu Pro Leu Ser Tyr  Gly Ala Gln Asp Thr  Pro Leu Leu
       1220                1225                1230
   cgc cgt  aag ata gtg gcc gag  ctg atg gcc ggc ggc  ctg gag ggg        3744
   Arg Arg  Lys Ile Val Ala Glu  Leu Met Ala Gly Gly  Leu Glu Gly
       1235                1240                1245
   gaa ttt  gac ccc gcc cag caa  gac cat gtc ttg cta  tga               3783
   Glu Phe  Asp Pro Ala Gln Gln  Asp His Val Leu Leu
       1250                1255                1260
<210> 76
   <211> 1260
   <212> PRT
   <213> Aspergillus niger
<400> 76
   Met Asp Arg Met Phe Thr Val Trp Trp Glu Tyr Pro Gln Lys Val Asn
   1               5                   10                  15
   Ser Lys Cys Ile Val His Pro Pro Ala Gly Thr His His Val Thr Cys
               20                  25                  30
   Trp Met Arg Lys Ser Gly Arg Gln Ala Pro Ser Glu Val Glu Leu Ile
           35                  40                  45
   Cys Cys Cys Ser Ile Thr Cys Phe Val Ser Ser Ser Thr Ser Arg Gly
       50                  55                  60
   Phe Ile Ala Ser Pro Pro Ser Leu Arg Leu Pro Arg Pro Ser Pro Thr
   65                  70                  75                  80
   Pro Arg Ser Pro Arg Ala Phe Leu Trp Ser Ser Asn Ser Leu Arg Arg
                   85                  90                  95
   Arg Ala Arg Leu Pro Pro Ser Leu Trp Gln Ser Pro Met Ala Ala Leu
   100 105 110
   Ser Glu Tyr Ile Cys Phe Gly Ser Ser Phe Pro Phe Ala His Pro Gly
   115 120 125
   Ala Ala Leu Val Phe Phe Phe Thr Leu Leu Phe Leu Leu Phe Phe Phe
   130 135 140
   Phe Phe Phe Phe Phe Pro Phe Ser Pro Ile Thr His Leu Pro Ile Thr
   145 150 155 160
   Phe Arg Arg Val Tyr Glu Leu Ser Glu Tyr Ser Asn Phe Pro Phe Leu
   165 170 175
   Ala Ile Glu Leu Val Pro Leu Leu Ser Pro Leu Leu Ser Pro Lys Glu
   180 185 190
   Leu Val Ser Arg Leu Leu Leu Thr Met Ser Gly Val Phe Ser Thr Met
   195 200 205
   Arg Pro Ser Val Glu Asp His Pro Met Pro Asp Val Cys Tyr Met Pro
   210 215 220
   Ser Asp Ser Ala Pro His Glu Glu Ala Val Asp Asn Thr Ala His Asp
   225 230 235 240
   Pro Met Asp Cys Ala Pro Asp Ser Pro Ala Ser Asn Ile Pro Gln Glu
   245 250 255
   Thr Ala Lys Ala Pro Ser Ala Glu Ile Pro Lys Tyr Gln Leu Arg Ser
   260 265 270
   Val His Val Pro Gly Asn Pro Lys Ala Val Arg Leu Asn Gly Lys His
   275 280 285
   Glu Val Lys Pro Ser Phe Pro Leu Arg Thr Pro Leu Ile Arg Pro Asp
   290 295 300
   His Arg Phe Ser Pro Asn Ser Arg Ala Ala Ser Phe Leu Gly Val Asp
   305 310 315 320
   Ala Leu Gln Lys Ser Gly Arg Asn Ala His Ser Ser His Leu Leu Ser
   325 330 335
   His Leu Gly Thr Ser Lys Tyr Leu Ser Ala Gly Leu Asn His Val Ser
   340 345 350
   Trp Ser Asn Asn Lys Pro Leu Ser Leu Pro Phe Gly Pro Asp Arg Ser
   355 360 365
   Asn Glu Thr Gly Tyr Leu Leu Gly Gly Lys Ser Ile Gly Ile Asp Lys
   370 375 380
   Lys Thr Phe His Met Asn Val Asn Lys His Ser Gly Phe Thr Thr Pro
   385 390 395 400
   Ser Arg Lys Arg Ser Met Glu Gly Asp Gly Tyr Glu Gln Ala Gln Pro
   405 410 415
   Ile Thr Ser Pro Ser Ala Lys Phe Arg Arg Val Arg Ala Gly Ser Phe
   420 425 430
   Ala Arg Ser Ala Ala Thr Glu Leu Glu Ala Thr Ala Asn Glu Asp Asp
   435 440 445
   Trp Tyr Pro Gly Ser Pro Met Asp Ile Asp Thr Pro Glu His Asn Asn
   450 455 460
   Glu Pro Asn Met Val Thr Ala Leu Ala Ala Val Glu Ser His Leu Ala
   465 470 475 480
   Leu Pro Glu Thr Ser Pro Lys Ser Arg Arg Leu Asp Ala Ser Lys Val
   485 490 495
   Lys Pro Val Thr Ala Leu Ala Val Ser Thr Thr Pro Ser Thr Pro Glu
   500 505 510
   Asn His Met Met Pro Gly Ala Trp Pro Glu Ser Val Pro Ser His Trp
   515 520 525
   Ser Thr Asp Ala Ala Thr Asp Ile Glu Asp Ser Pro Met Ile Ser Asp
   530 535 540
   Glu Glu Ile Leu Ser Asn Asn Thr Asp Met Ala Asp Ala Gly Glu Pro
   545 550 555 560
   Thr Ile Pro Val Asn Ser Asp Ala Glu Pro Gln Pro Ser Ser Pro Trp
   565 570 575
   Tyr Ala Tyr Trp Gly Thr Trp His Ser Tyr Leu Gln Thr Val Val Ser
   580 585 590
   Ile Pro Thr Gly Ile Thr Gln Asn Phe Ser Tyr Ala Ala Arg Ser Ala
   595 600 605
   Ala Asn Ala Val Gly Ala Ala Arg Arg Arg Val Cys Ala Leu Trp Asn
   610 615 620
   Arg Arg Gly Gly Arg Arg Leu Pro Ser Pro Pro Gly Ser Pro Thr Arg
   625 630 635 640
   Leu Asn Leu Arg Asn Leu Ser Pro Glu Gln Gln Glu Arg Val Asp Arg
   645 650 655
   Asp Arg Arg Ile Arg Ser Gly Gln Ser Ser Pro Arg Thr Asp Lys Ser
   660 665 670
   Phe Pro Asp Phe Ser Asn Val Pro Asn Phe Ser His Asn Asn Asn Thr
   675 680 685
   Ala Pro Thr Val Glu Ser Ser Gln Ile Leu Gln Asp Leu Val Ser Gln
   690 695 700
   Glu Gln Met Pro Gln Ala Leu Ser Asp Ser Val Ser Val Gln Arg Arg
   705 710 715 720
   Ala His Glu Pro Lys Asn Val Gly Thr Arg Lys His Pro Gly Met Pro
   725 730 735
   Pro Val Lys Gln Pro Ala Arg Arg Ser Pro Pro Arg Ser Ala Gln Arg
   740 745 750
   Ser Ala Gln Arg Ser Thr Gln Asp Thr Ala Gln Lys Pro Leu Arg Arg
   755 760 765
   Gln Gly Ser Pro Gly Ile Arg Lys Gln Lys His Ile Thr Arg Ala Glu
   770 775 780
   Arg Ala Glu Asn Arg Glu Arg Ser Ala Ala Phe Arg Ala Leu Phe Val
   785 790 795 800
   Gly Asp Ile Ala Gly Met Lys Lys Ala Met Glu Ala Gln Ala Ala Arg
   805 810 815
   Asp Arg Gln Ala Ser Ala Ala Gly Arg Arg Pro Val Glu Leu Asn Leu
   820 825 830
   Met Arg Ser Ser Tyr Glu Leu Asp Arg Ala Gln Asp Leu Asn Met Leu
   835 840 845
   Ala Asn Val Gln Ser Arg Ser Phe Thr Ala Val Asp Gln Leu Gly Ala
   850 855 860
   Thr Glu Lys Val Leu Ser Ser Ser Ile Thr Val Ala Pro Lys Thr Ile
   865 870 875 880
   Leu Lys Ile Arg Gly Arg Pro Ser Ile Ser Gly Asn Arg Thr Arg Ser
   885 890 895
   Arg Arg Ala Thr Pro Arg Arg Val Arg Phe Arg Glu Pro Val Val Ala
   900 905 910
   Glu Phe Ile Asp Asp Thr Arg Asp Trp Gly Glu Val Glu Asp Asn Leu
   915 920 925
   Ala Pro His Leu Gly Asp Thr Leu Asp Val Asn Glu Arg Thr Thr Glu
   930 935 940
   Thr Ser Val Gly Thr Lys Ala Glu Tyr Thr Glu Lys Glu Asn Val Pro
   945 950 955 960
   Pro Ala Leu Glu Asn Glu Glu Glu Asn Glu Pro Ile Val Asp Pro Trp
   965 970 975
   Ser Gln Pro Thr Phe Glu Phe Pro Leu Gly Arg Ala Val Ser Ala Val
   980 985 990
   Ser Leu Phe Tyr Pro Glu Pro Lys Pro Ile Pro Glu Gly Arg Thr Glu
   995 1000 1005
   Ser Val Tyr Ala Asp Arg Trp Arg Gln Ile Gln Glu Glu Glu Arg
   1010 1015 1020
   Gln Lys Ala Leu Pro Ser His Gly Arg Val Ser Gly Gly Lys Thr
   1025 1030 1035
   Val Ala Thr Thr Leu Ser Gly Asp Pro Leu Thr Lys Arg Ser Leu
   1040 1045 1050
   Ala Thr Cys Tyr Thr Arg Gly Glu Trp Leu Asn Asp Glu Val Ile
   1055 1060 1065
   Asn Gly Tyr Leu Ala Leu Ile Val Asp Tyr Leu Arg Arg Lys Asn
   1070 1075 1080
   His Asn Ala Gly Arg Asn Asp Lys Pro Arg Phe His Ala Phe Asn
   1085 1090 1095
   Ser Phe Phe Phe Ser Asn Leu Arg Asp Lys Gly Tyr Glu Ser Val
   1100 1105 1110
   Ala Arg Trp Ala Lys Arg Ala Lys Ile Gly Gly Ser Leu Leu Leu
   1115 1120 1125
   Asp Val Asp Thr Val Tyr Ile Pro Val His Asn Ser Gln His Trp
   1130 1135 1140
   Thr Leu Val Val Val Arg Pro Gly Glu Arg Ser Ile Glu His Phe
   1145 1150 1155
   Asp Ser Leu Gly Ala Arg Ser Arg Arg His Ile Ala Val Val Gln
   1160 1165 1170
   Thr Trp Leu Arg Gly Glu Leu Gly Pro Lys Tyr Val Glu Glu Glu
   1175 1180 1185
   Trp Arg Val Leu Pro Ser Leu Ser Pro Gln Gln Asp Asn Gly Ser
   1190 1195 1200
   Asp Cys Gly Val Phe Leu Leu Thr Thr Ala Lys Ala Val Ala Ile
   1205 1210 1215
   Gly Leu Glu Pro Leu Ser Tyr Gly Ala Gln Asp Thr Pro Leu Leu
   1220 1225 1230
   Arg Arg Lys Ile Val Ala Glu Leu Met Ala Gly Gly Leu Glu Gly
   1235 1240 1245
   Glu Phe Asp Pro Ala Gln Gln Asp His Val Leu Leu
   1250 1255 1260
<210> 77
   <211> 2664
   <212> DNA
   <213> Aspergillus niger
<400> 77
   acttgctcga agcgctcttt gtgtagtttg ctcaatgcgt tgtgctcctc tcggagaaag 60
   gcgaggcagc gattcagtgg gtagctaatg cgcagttcgt tgtggtcaag ctggtactgt 120
   ccgttcgcct tttcatcgac taaggacttc tccatatgct ggattgcttc gatgaggttc 180
   tgtgcctcat cctccatatc ttttttctca ctacaaaaga ttagccctag gtcataagga 240
   cattggaatg taggcgaggc gcatacttat ccacaagttt gaggtggttg ttcagcgttt 300
   cagacaacgc gctgaagagc tgctttacag ttagtcgcga ggggtgctga gcatatcaga 360
   aacaaccaac ctctgcttct ctggactcgc gctcatggtt cggaacgcca atgtcatcaa 420
   agattccatg caattgagtg acgatgttat tgacctgagt ggtcaagtag cttgtatcga 480
   cggccatagt gttgtgtttg tttcccacgc ccaagccagg gggggttatt ggtgtattgt 540
   ggagctgatt ggcctgaagg aggttgtttg agtgcgcgat cgaagacaaa ctcgccttta 600
   aatggcgggg gtcagcgtca tgtacgctcg gggggcggcg atgcgcctgg agagattaga 660
   aagaggagct gcgagttatc caaacaggca ggatgaacaa cgcgagactg cgattgacag 720
   cgaagcgcct cacgggaaga tgagtggcat ctgcgaggac aagggaagac ccgccagatt 780
   tatgaccagc tcagcgagat gcgggtatat gcaagggtga ctgggatgga ggggatggag 840
   acaggcagcg gtgatggagg gaaagacaat aacaatatcc ctttggggat tctgcagccg 900
   caatcccgat ctgctcgccg ccttgttgct ctgtttgctg ccgctttcag ctgtgacccc 960
   gtcggcccgg taaccgctga ctcagcgccg atctccgccg cgatctgccg ggaagttgct 1020
   caccgcccgg agtcacctca ctccatctct ctatctacta tccagatctc attatctact 1080
   gctccgcttg accttttcat tccaataatc aaaatctact tctgatttca gccaatccgt 1140
   gttaatcact tatcccattc ccttgctatt ccaattacat cttctactct accatccacc 1200
   atgcaggctg cccagcagtc ctggggtacg tccatcccca accccatccc cacccccact 1260
   ccatctcaca gcactctaac cccataacca cagaattcga aaatgccgtc accctcatag 1320
   acccccaccg agacgcgctc tacaaatacg atgaagagac acacaaagct cttagcgctc 1380
   agcgcccttg gtccaaagac ccacactact tcaaatcgat ccgaatctcc gccgtcgcac 1440
   tcctcaagat ggtaatgcac gcgcggtccg gcggctccct cgaagtaatg ggcctaatgc 1500
   aaggctatgt cctgcccgaa accttcgtgg taaccgacgc cttccgactt cccgtagagg 1560
   gaaccgaaac gcgagtcaac gcccaagacg aagccaacga atacatggtc tcatacctac 1620
   agtcgtgccg ggatgcagga cggatggaaa atgcagtggg ctggtaccac agtcatcccg 1680
   ggtacgggtg ctggttatcg ggcattgatg tgacgacgca ggatatgcag cagctcgggg 1740
   gaccgtttgt ggcggtggtg gttgatccag agaggacgat ctcagcaggg aaggtggata 1800
   ttggggcatt taggacgttt ccgaaggatt atacgccgcc gaaaggggag gggggtagtg 1860
   gtgctgggga ggatgactat cagacggtgc cgttgaacaa ggcggaggac tttggggcac 1920
   atgcgtcaca ttattattcg ctggaggtgt cggtgtttaa gagtgcgttg gatacggaga 1980
   ttctgtcgtt gctgtggaat aagtattggg ttgcgacgtt gagtcagagt ccgttgttta 2040
   cgacgaggga ttatgggagt aagcagatgg tggatttgag tcagaagatt aagcgggcca 2100
   cgaggggcgt ggagagtagt gcttcgcgag ggaatctggc gcaagttaag gatccgcagt 2160
   tggagagggt ggcgagggat gggcagcgga ttgtctcgga agaagtgaag ggtttgttgg 2220
   cggcggaagt gaagatgaag ctgtttcaaa atgttgggga ggggaataga tcggagaaat 2280
   catgagcttg tgctcaactt ctgttcttat gaaaggctta tgaaggttca tttgagaaca 2340
   actctactat gaatgacgag atgacttaat ggatacattt gctgcttctt tctttttgtt 2400
   gtctactcag tagcaatttg tgctatcatt gtatttggtc gtattacgtc tcgttaccgc 2460
   tattcctgtg cactgatgac cattgatgca tgtaatatgt gctttaatat gtactaacac 2520
   actgcagcag gtgtatatgt ccataggaga acagatcagc acaaggggac gacaagaggg 2580
   gtaatcatga atgaaaatgc ccaaagcaaa gaccaatccg tcaatagtag cctgcgtcgt 2640
   gataaatcat catccgaacc gtaa 2664
<210> 78
   <211> 1080
   <212> DNA
   <213> Aspergillus niger
<220>
   <221> CDS
   <222> (1)..(1080)
<400> 78
   atg cag gct gcc cag cag tcc tgg gaa ttc gaa aat gcc gtc acc ctc        48
   Met Gln Ala Ala Gln Gln Ser Trp Glu Phe Glu Asn Ala Val Thr Leu
   1               5                   10                  15
   ata gac ccc cac cga gac gcg ctc tac aaa tac gat gaa gag aca cac        96
   Ile Asp Pro His Arg Asp Ala Leu Tyr Lys Tyr Asp Glu Glu Thr His
               20                  25                  30
   aaa gct ctt agc gct cag cgc cct tgg tcc aaa gac cca cac tac ttc       144
   Lys Ala Leu Ser Ala Gln Arg Pro Trp Ser Lys Asp Pro His Tyr Phe
           35                  40                  45
   aaa tcg atc cga atc tcc gcc gtc gca ctc ctc aag atg gta atg cac       192
   Lys Ser Ile Arg Ile Ser Ala Val Ala Leu Leu Lys Met Val Met His
       50                  55                  60
   gcg cgg tcc ggc ggc tcc ctc gaa gta atg ggc cta atg caa ggc tat       240
   Ala Arg Ser Gly Gly Ser Leu Glu Val Met Gly Leu Met Gln Gly Tyr
   65                  70                  75                  80
   gtc ctg ccc gaa acc ttc gtg gta acc gac gcc ttc cga ctt ccc gta       288
   Val Leu Pro Glu Thr Phe Val Val Thr Asp Ala Phe Arg Leu Pro Val
                   85                  90                  95
   gag gga acc gaa acg cga gtc aac gcc caa gac gaa gcc aac gaa tac       336
   Glu Gly Thr Glu Thr Arg Val Asn Ala Gln Asp Glu Ala Asn Glu Tyr
               100                 105                 110
   atg gtc tca tac cta cag tcg tgc cgg gat gca gga cgg atg gaa aat       384
   Met Val Ser Tyr Leu Gln Ser Cys Arg Asp Ala Gly Arg Met Glu Asn
           115                 120                 125
   gca gtg ggc tgg tac cac agt cat ccc ggg tac ggg tgc tgg tta tcg       432
   Ala Val Gly Trp Tyr His Ser His Pro Gly Tyr Gly Cys Trp Leu Ser
       130                 135                 140
   ggc att gat gtg acg acg cag gat atg cag cag ctc ggg gga ccg ttt       480
   Gly Ile Asp Val Thr Thr Gln Asp Met Gln Gln Leu Gly Gly Pro Phe
   145                 150                 155                 160
   gtg gcg gtg gtg gtt gat cca gag agg acg atc tca gca ggg aag gtg       528
   Val Ala Val Val Val Asp Pro Glu Arg Thr Ile Ser Ala Gly Lys Val
                   165                 170                 175
   gat att ggg gca ttt agg acg ttt ccg aag gat tat acg ccg ccg aaa       576
   Asp Ile Gly Ala Phe Arg Thr Phe Pro Lys Asp Tyr Thr Pro Pro Lys
               180                 185                 190
   ggg gag ggg ggt agt ggt gct ggg gag gat gac tat cag acg gtg ccg       624
   Gly Glu Gly Gly Ser Gly Ala Gly Glu Asp Asp Tyr Gln Thr Val Pro
           195                 200                 205
   ttg aac aag gcg gag gac ttt ggg gca cat gcg tca cat tat tat tcg      672
   Leu Asn Lys Ala Glu Asp Phe Gly Ala His Ala Ser His Tyr Tyr Ser
       210                 215                 220
   ctg gag gtg tcg gtg ttt aag agt gcg ttg gat acg gag att ctg tcg      720
   Leu Glu Val Ser Val Phe Lys Ser Ala Leu Asp Thr Glu Ile Leu Ser
   225                 230                 235                 240
   ttg ctg tgg aat aag tat tgg gtt gcg acg ttg agt cag agt ccg ttg      768
   Leu Leu Trp Asn Lys Tyr Trp Val Ala Thr Leu Ser Gln Ser Pro Leu
                   245                 250                 255
   ttt acg acg agg gat tat ggg agt aag cag atg gtg gat ttg agt cag      816
   Phe Thr Thr Arg Asp Tyr Gly Ser Lys Gln Met Val Asp Leu Ser Gln
               260                 265                 270
   aag att aag cgg gcc acg agg ggc gtg gag agt agt gct tcg cga ggg      864
   Lys Ile Lys Arg Ala Thr Arg Gly Val Glu Ser Ser Ala Ser Arg Gly
           275                 280                 285
   aat ctg gcg caa gtt aag gat ccg cag ttg gag agg gtg gcg agg gat      912
   Asn Leu Ala Gln Val Lys Asp Pro Gln Leu Glu Arg Val Ala Arg Asp
       290                 295                 300
   ggg cag cgg att gtc tcg gaa gaa gtg aag gca ggt gta tat gtc cat      960
   Gly Gln Arg Ile Val Ser Glu Glu Val Lys Ala Gly Val Tyr Val His
   305                 310                 315                 320
   agg aga aca gat cag cac aag ggg acg aca aga ggg gta atc atg aat      1008
   Arg Arg Thr Asp Gln His Lys Gly Thr Thr Arg Gly Val Ile Met Asn
                   325                 330                 335
   gaa aat gcc caa agc aaa gac caa tcc gtc aat agt agc ctg cgt cgt      1056
   Glu Asn Ala Gln Ser Lys Asp Gln Ser Val Asn Ser Ser Leu Arg Arg
               340                 345                 350
   gat aaa tca tca tcc gaa ccg taa                                      1080
   Asp Lys Ser Ser Ser Glu Pro
           355
<210> 79
   <211> 359
   <212> PRT
   <213> Aspergillus niger
<400> 79
   Met Gln Ala Ala Gln Gln Ser Trp Glu Phe Glu Asn Ala Val Thr Leu
   1               5                   10                  15
   Ile Asp Pro His Arg Asp Ala Leu Tyr Lys Tyr Asp Glu Glu Thr His
               20                  25                  30
   Lys Ala Leu Ser Ala Gln Arg Pro Trp Ser Lys Asp Pro His Tyr Phe
           35                  40                  45
   Lys Ser Ile Arg Ile Ser Ala Val Ala Leu Leu Lys Met Val Met His
       50                  55                  60
   Ala Arg Ser Gly Gly Ser Leu Glu Val Met Gly Leu Met Gln Gly Tyr
   65 70 75 80
   Val Leu Pro Glu Thr Phe Val Val Thr Asp Ala Phe Arg Leu Pro Val
   85 90 95
   Glu Gly Thr Glu Thr Arg Val Asn Ala Gln Asp Glu Ala Asn Glu Tyr
   100 105 110
   Met Val Ser Tyr Leu Gln Ser Cys Arg Asp Ala Gly Arg Met Glu Asn
   115 120 125
   Ala Val Gly Trp Tyr His Ser His Pro Gly Tyr Gly Cys Trp Leu Ser
   130 135 140
   Gly Ile Asp Val Thr Thr Gln Asp Met Gln Gln Leu Gly Gly Pro Phe
   145 150 155 160
   Val Ala Val Val Val Asp Pro Glu Arg Thr Ile Ser Ala Gly Lys Val
   165 170 175
   Asp Ile Gly Ala Phe Arg Thr Phe Pro Lys Asp Tyr Thr Pro Pro Lys
   180 185 190
   Gly Glu Gly Gly Ser Gly Ala Gly Glu Asp Asp Tyr Gln Thr Val Pro
   195 200 205
   Leu Asn Lys Ala Glu Asp Phe Gly Ala His Ala Ser His Tyr Tyr Ser
   210 215 220
   Leu Glu Val Ser Val Phe Lys Ser Ala Leu Asp Thr Glu Ile Leu Ser
   225 230 235 240
   Leu Leu Trp Asn Lys Tyr Trp Val Ala Thr Leu Ser Gln Ser Pro Leu
   245 250 255
   Phe Thr Thr Arg Asp Tyr Gly Ser Lys Gln Met Val Asp Leu Ser Gln
   260 265 270
   Lys Ile Lys Arg Ala Thr Arg Gly Val Glu Ser Ser Ala Ser Arg Gly
   275 280 285
   Asn Leu Ala Gln Val Lys Asp Pro Gln Leu Glu Arg Val Ala Arg Asp
   290 295 300
   Gly Gln Arg Ile Val Ser Glu Glu Val Lys Ala Gly Val Tyr Val His
   305 310 315 320
   Arg Arg Thr Asp Gln His Lys Gly Thr Thr Arg Gly Val Ile Met Asn
   325 330 335
   Glu Asn Ala Gln Ser Lys Asp Gln Ser Val Asn Ser Ser Leu Arg Arg
   340 345 350
   Asp Lys Ser Ser Ser Glu Pro
   355
<210> 80
   <211> 2678
   <212> DNA
   <213> Aspergillus niger
<400> 80
   aatacttgtc ccagtccgac atcccgggcc agggttgctt cgtccttcaa cttaagatcg 60
   tctaattcgg gtgtcacccc gggaggaggg tcgttatgct gcttctccat ggtggtttga 120
   ttgcgtgagg gaagccacgg catgatggca cggaactgtt ttactgttta tactgctaca 180
   ggtaagtaca gatgaggtcc atcggttata agataaccag ttaactttaa ccaagagcac 240
   ccacggaatc aagcgcacaa cttccgcgat cagcgactga tacacagtac cttcaccggg 300
   taatttgacg agccgatacc gctatgatgc cctgtctgta ctcatcgcga tgaaggcgag 360
   gtcgggagct tgggcgtgag tcaaatgccg ggcattgact tttcctatct agaaggcaac 420
   tatacccgtg ggaaggacga cccaatgaga ttcgtgaagc catgttggtc tcgaaacagg 480
   aatacgagat ctgtgcggtc actcgggcag gttcagaatc cggggtcgcg atatggcagc 540
   agatcaaggg aagccgtcgg cagatgcgcc aatacgaagc gtcggtcggc aagatgacaa 600
   ccaggttatc gttcatcgca atgccggcag ctgaaccggc aactctccac cacgcttatc 660
   cttgggtttc cactggttcg agaagtcaat gctgggtcca tcttccggga aactctcaac 720
   tgcgcctccc ccacagattc gatcgtgcgg agaaacgcct cgtgttggtc agggacgagg 780
   gactcattta atacttgatc cactgttatc tgccgagcta gtcaccctga taatacctga 840
   tagtgtttga tagcttgata gcggaggtat tattaatggc ttatgcgctg gatttcccgc 900
   ggctgtcggg gcggatttga taacgacaat cttccgcgat gcggggtgca gatggagtca 960
   ccgttgtaac tgacactggt tcgtaatgat ggttacccga gtgatcctat catggctttc 1020
   atctgaatgt ccgggatttg ccccattgcg tcggccgagg ttctccacgc ttatcccttg 1080
   ccgcgatagc agggcgatga tggggagggg agggcatata tgacgccccg gtagacaacg 1140
   tcgggagctc ttacttgatt ttcagccagc agagaacaag attggcagac catcgccaca 1200
   atgaccgcaa cgcagaccat tacccaaacc gtgccaacga tcgacgaggt caaggtagca 1260
   gttgatgcag cgttggagtc tgcgcaaaca tcgctacggg agctcaaccg agaggtaggc 1320
   acctaaccct cctttcgctt catatgatac tgatcatcga ctttaccaca gatctggtgt 1380
   aatccagaaa cagcctacca agagcatcga gcccatgaca ccatctgcga cttcctcgag 1440
   aagcaaggct tcaccgtaac ccgtcacgca tacggactgg acacctcttt tgaggcaatc 1500
   agcggatctg gtggacgact tatcaacttc aatgccgaat acgatgcgct acctgacatc 1560
   ggtcacgcct gtggccataa tctcatcact accagcagtg tggctgcctt cctagcgctg 1620
   tccacggtcc tgaaacaata cggtatcccc ggtcgcactc aacttctggg caccccggca 1680
   gaggagaatg gcggtggcaa ggccaaactg attgacttgg gtgcatacaa gggtgtagat 1740
   gtgtcattga tggcgtacgt attgcctagc cctttatgtg gcttatcatc ttcgagagtt 1800
   gacttttcct attcgcatac tgaccttcct cagtcacgca ggacctaaag aactattccc 1860
   tggcgttgta acagacagcg ttggtggtgt gcgaatgaac gcccgaaaag agttacactg 1920
   cgagttcaca ggtcggagtg cccatgctgg cggcaatcct tgggaaggaa tcaatgccct 1980
   cgacgcccta gtaacatcat acaacaacgt ggccgtactg cgccagcagc tccgcccaga 2040
   tgagcgcata cactgtgctt tcttggatac acccacggtg gccaacatta ttccctcgta 2100
   caccaaagcg tactggcagg ttcgcagccc gacattgaag gggttgaacc aactcatggt 2160
   caaagtgcgg aactgcattg aggcaggtgc attggctaca ggttgtgagg tgaagattat 2220
   cgagtaaggc tgttgatccc tggaccaaca gaaggtgagc taactggacc aggaacgagc 2280
   tgtataccga catcaagatc aatgacacac tgtgcgaccg ttataaagaa cacatgtcca 2340
   agtacgaccg aaatgttatc gcgcgtcttg agcaggtcat gacaggatca tcggatatcg 2400
   gtaggtgccc ttgcttgtgc aatcacagta ctttgctgat ttctccaggc aatgtcagct 2460
   acattgtccc gacactacac accatgttcg gtatcacgga tgaaccaggc tccttccctc 2520
   atcaccccaa atttgctgca gctgctggaa ccgattacgc gcatcaagaa gcggtgattg 2580
   taggcaagtc gctggcgctg attgggtggg agatggtgac gagcgatgag ttgtttggga 2640
   ttgcgcagaa gcagttgaag gaatgtcttg aggagtag 2678
<210> 81
   <211> 1227
   <212> DNA
   <213> Aspergillus niger
<220>
   <221> CDS
   <222> (1)..(1227)
<400> 81
   atg acc gca acg cag acc att acc caa acc gtg cca acg atc gac gag        48
   Met Thr Ala Thr Gln Thr Ile Thr Gln Thr Val Pro Thr Ile Asp Glu
   1               5                   10                  15
   gtc aag gta gca gtt gat gca gcg ttg gag tct gcg caa aca tcg cta        96
   Val Lys Val Ala Val Asp Ala Ala Leu Glu Ser Ala Gln Thr Ser Leu
               20                  25                  30
   cgg gag ctc aac cga gag atc tgg tgt aat cca gaa aca gcc tac caa       144
   Arg Glu Leu Asn Arg Glu Ile Trp Cys Asn Pro Glu Thr Ala Tyr Gln
           35                  40                  45
   gag cat cga gcc cat gac acc atc tgc gac ttc ctc gag aag caa ggc      192
   Glu His Arg Ala His Asp Thr Ile Cys Asp Phe Leu Glu Lys Gln Gly
       50                  55                  60
   ttc acc gta acc cgt cac gca tac gga ctg gac acc tct ttt gag gca      240
   Phe Thr Val Thr Arg His Ala Tyr Gly Leu Asp Thr Ser Phe Glu Ala
   65                  70                  75                  80
   atc agc gga tct ggt gga cga ctt atc aac ttc aat gcc gaa tac gat      288
   Ile Ser Gly Ser Gly Gly Arg Leu Ile Asn Phe Asn Ala Glu Tyr Asp
                   85                  90                  95
   gcg cta cct gac atc ggt cac gcc tgt ggc cat aat ctc atc act acc      336
   Ala Leu Pro Asp Ile Gly His Ala Cys Gly His Asn Leu Ile Thr Thr
               100                 105                 110
   agc agt gtg gct gcc ttc cta gcg ctg tcc acg gtc ctg aaa caa tac      384
   Ser Ser Val Ala Ala Phe Leu Ala Leu Ser Thr Val Leu Lys Gln Tyr
           115                 120                 125
   ggt atc ccc ggt cgc act caa ctt ctg ggc acc ccg gca gag gag aat      432
   Gly Ile Pro Gly Arg Thr Gln Leu Leu Gly Thr Pro Ala Glu Glu Asn
       130                 135                 140
   ggc ggt ggc aag gcc aaa ctg att gac ttg ggt gca tac aag ggt gta      480
   Gly Gly Gly Lys Ala Lys Leu Ile Asp Leu Gly Ala Tyr Lys Gly Val
   145                 150                 155                 160
   gat gtg tca ttg atg gcg tac gta ttg cct agc cct tta tac agc gtt      528
   Asp Val Ser Leu Met Ala Tyr Val Leu Pro Ser Pro Leu Tyr Ser Val
                   165                 170                 175
   ggt ggt gtg cga atg aac gcc cga aaa gag tta cac tgc gag ttc aca      576
   Gly Gly Val Arg Met Asn Ala Arg Lys Glu Leu His Cys Glu Phe Thr
               180                 185                 190
   ggt cgg agt gcc cat gct ggc ggc aat cct tgg gaa gga atc aat gcc      624
   Gly Arg Ser Ala His Ala Gly Gly Asn Pro Trp Glu Gly Ile Asn Ala
           195                 200                 205
   ctc gac gcc cta gta aca tca tac aac aac gtg gcc gta ctg cgc cag      672
   Leu Asp Ala Leu Val Thr Ser Tyr Asn Asn Val Ala Val Leu Arg Gln
       210                 215                 220
   cag ctc cgc cca gat gag cgc ata cac tgt gct ttc ttg gat aca ccc      720
   Gln Leu Arg Pro Asp Glu Arg Ile His Cys Ala Phe Leu Asp Thr Pro
   225                 230                 235                 240
   acg gtg gcc aac att att ccc tcg tac acc aaa gcg tac tgg cag gtt      768
   Thr Val Ala Asn Ile Ile Pro Ser Tyr Thr Lys Ala Tyr Trp Gln Val
                   245                 250                 255
   cgc agc ccg aca ttg aag ggg ttg aac caa ctc atg gtc aaa gtg cgg      816
   Arg Ser Pro Thr Leu Lys Gly Leu Asn Gln Leu Met Val Lys Val Arg
               260                 265                 270
   aac tgc att gag gca ggt gca ttg gct aca ggt tgt gag gtg aag att      864
   Asn Cys Ile Glu Ala Gly Ala Leu Ala Thr Gly Cys Glu Val Lys Ile
           275                 280                 285
   atc gag aac gag ctg tat acc gac atc aag atc aat gac aca ctg tgc      912
   Ile Glu Asn Glu Leu Tyr Thr Asp Ile Lys Ile Asn Asp Thr Leu Cys
       290                 295                 300
   gac cgt tat aaa gaa cac atg tcc aag tac gac cga aat gtt atc gcg      960
   Asp Arg Tyr Lys Glu His Met Ser Lys Tyr Asp Arg Asn Val Ile Ala
   305                 310                 315                 320
   cgt ctt gag cag gtc atg aca gga tca tcg gat atc ggc aat gtc agc     1008
   Arg Leu Glu Gln Val Met Thr Gly Ser Ser Asp Ile Gly Asn Val Ser
                   325                 330                 335
   tac att gtc ccg aca cta cac acc atg ttc ggt atc acg gat gaa cca     1056
   Tyr Ile Val Pro Thr Leu His Thr Met Phe Gly Ile Thr Asp Glu Pro
               340                 345                 350
   ggc tcc ttc cct cat cac ccc aaa ttt gct gca gct gct gga acc gat     1104
   Gly Ser Phe Pro His His Pro Lys Phe Ala Ala Ala Ala Gly Thr Asp
           355                 360                 365
   tac gcg cat caa gaa gcg gtg att gta ggc aag tcg ctg gcg ctg att     1152
   Tyr Ala His Gln Glu Ala Val Ile Val Gly Lys Ser Leu Ala Leu Ile
       370                 375                 380
   ggg tgg gag atg gtg acg agc gat gag ttg ttt ggg att gcg cag aag     1200
   Gly Trp Glu Met Val Thr Ser Asp Glu Leu Phe Gly Ile Ala Gln Lys
   385                 390                 395                 400
   cag ttg aag gaa tgt ctt gag gag tag                                 1227
   Gln Leu Lys Glu Cys Leu Glu Glu
                   405
<210> 82
   <211> 408
   <212> PRT
   <213> Aspergillus niger
<400> 82
   Met Thr Ala Thr Gln Thr Ile Thr Gln Thr Val Pro Thr Ile Asp Glu
   1               5                   10                  15
   Val Lys Val Ala Val Asp Ala Ala Leu Glu Ser Ala Gln Thr Ser Leu
               20                  25                  30
   Arg Glu Leu Asn Arg Glu Ile Trp Cys Asn Pro Glu Thr Ala Tyr Gln
           35                  40                  45
   Glu His Arg Ala His Asp Thr Ile Cys Asp Phe Leu Glu Lys Gln Gly
       50                  55                  60
   Phe Thr Val Thr Arg His Ala Tyr Gly Leu Asp Thr Ser Phe Glu Ala
   65                  70                  75                  80
   Ile Ser Gly Ser Gly Gly Arg Leu Ile Asn Phe Asn Ala Glu Tyr Asp
                   85                  90                  95
   Ala Leu Pro Asp Ile Gly His Ala Cys Gly His Asn Leu Ile Thr Thr
               100                 105                 110
   Ser Ser Val Ala Ala Phe Leu Ala Leu Ser Thr Val Leu Lys Gln Tyr
   115 120 125
   Gly Ile Pro Gly Arg Thr Gln Leu Leu Gly Thr Pro Ala Glu Glu Asn
   130 135 140
   Gly Gly Gly Lys Ala Lys Leu Ile Asp Leu Gly Ala Tyr Lys Gly Val
   145 150 155 160
   Asp Val Ser Leu Met Ala Tyr Val Leu Pro Ser Pro Leu Tyr Ser Val
   165 170 175
   Gly Gly Val Arg Met Asn Ala Arg Lys Glu Leu His Cys Glu Phe Thr
   180 185 190
   Gly Arg Ser Ala His Ala Gly Gly Asn Pro Trp Glu Gly Ile Asn Ala
   195 200 205
   Leu Asp Ala Leu Val Thr Ser Tyr Asn Asn Val Ala Val Leu Arg Gln
   210 215 220
   Gln Leu Arg Pro Asp Glu Arg Ile His Cys Ala Phe Leu Asp Thr Pro
   225 230 235 240
   Thr Val Ala Asn Ile Ile Pro Ser Tyr Thr Lys Ala Tyr Trp Gln Val
   245 250 255
   Arg Ser Pro Thr Leu Lys Gly Leu Asn Gln Leu Met Val Lys Val Arg
   260 265 270
   Asn Cys Ile Glu Ala Gly Ala Leu Ala Thr Gly Cys Glu Val Lys Ile
   275 280 285
   Ile Glu Asn Glu Leu Tyr Thr Asp Ile Lys Ile Asn Asp Thr Leu Cys
   290 295 300
   Asp Arg Tyr Lys Glu His Met Ser Lys Tyr Asp Arg Asn Val Ile Ala
   305 310 315 320
   Arg Leu Glu Gln Val Met Thr Gly Ser Ser Asp Ile Gly Asn Val Ser
   325 330 335
   Tyr Ile Val Pro Thr Leu His Thr Met Phe Gly Ile Thr Asp Glu Pro
   340 345 350
   Gly Ser Phe Pro His His Pro Lys Phe Ala Ala Ala Ala Gly Thr Asp
   355 360 365
   Tyr Ala His Gln Glu Ala Val Ile Val Gly Lys Ser Leu Ala Leu Ile
   370 375 380
   Gly Trp Glu Met Val Thr Ser Asp Glu Leu Phe Gly Ile Ala Gln Lys
   385 390 395 400
   Gln Leu Lys Glu Cys Leu Glu Glu
   405
<210> 83
   <211> 3545
   <212> DNA
   <213> Aspergillus niger
<400> 83
   tgtactgaca agcgtagtcc tcccgcgccc agccgcaaag cgagccgccg ccctggcatt 60
   catcaactgt ctcagcaatg tgtgccagat ctacatgcca tacctgtacc ccggtaagtc 120
   caaagttttc atgatctcca gtcatataca acatgctaat ccgtgttgtg atcaatagac 180
   tccgcctctc ctcgttatat cgcggccttc agtgtcaata tcgctttgct cgcgatgaca 240
   attgtgattg ccaccatcct tcgcatttat ttgggtagat tgaataagca gctcgacgaa 300
   ttagaaggtg ttactttcgc gcaggctggt gagaatgaag aaaatggctt gcctggccgg 360
   gcagtggcgg agggtttccg gttcttgcta tagtgctcgg agaactctac accattagtt 420
   aaggtttatg gccgataccc cctgtttcgc actatagttt gttatttgta ttacatgcac 480
   tgcaagaggc cacgaggacc ccatccatac tcaggcaatc tctcgccttt ttgtccaggt 540
   tgtcaaatcc tgaacacagc cacaacgcag caaacctttg gatcgagata gccttttgaa 600
   ttgatagtaa ccatgggaaa tagttattcc ctctgccatc tcacttaaga atcttatctt 660
   tactgcagtg gccagctcag gcttatagca cggctcaata gcctacccca atccgaatgg 720
   tatctttagt acagctaaaa atatttcaaa cttagccaac taggacctag tgtataatag 780
   ctagcctatg catagaaggt atatcccacc cagccttctt ctaaaataaa tacgggccat 840
   gcttacattc tcagggacac attgtctagt tctaaaatta ggtcggagtc cgacggaggc 900
   actaaaagcg ccccactgct cagctgccaa ccttatcgaa cgctatcttt ggtatattca 960
   ccatatagag ccgcatattg ctactattat aacacgaagt ggcaggtaag cagcatcttg 1020
   tgcttgaggg tgcatcagca ggggcagtct ataaagtcac ttcctgcgct gcctcccaat 1080
   cgcactgctt gcacttcatt accattcagt ctctcttttc cagctgcata cttcttcagc 1140
   tgctctatcc tcctttggta cctattcctc ctttttcctt ttcagcaagc agggacaagc 1200
   atgtggtaag tgtacatgct ccgaattgtg atagtcttgc atcaccactg acaaatacag 1260
   tggaattttc ggctacgtga attatctcgt agagaagaac cgcaaggaga ttctcgacac 1320
   cctcctcaat ggtataacga ctacatctac tattcttgga ctctacatta ctaaccatga 1380
   acgtaaaggg ctttcgcgtc tagattatcg tggttacgac tcagctgggc tcgcaatcga 1440
   cggtgacaaa aagaaagaag tgttcatcta caaagaagtc gggaaggtcg caggcctgag 1500
   gaagcttatt accgaggaga agcccgatcc cactaagacc ttcgaatctc acgctggtat 1560
   tgcacacact cgttgggcga ctcacggagt accatcgcga accaactgtc atccccatag 1620
   gtatgcaatc agctttggga gcccccactc taaggccgcg taattgattc ctttatctag 1680
   gtctgatcct acctcagagt ttgttgttgt tcacaatggc attacaacaa actacaaaga 1740
   gctgcggaga ctcctcgcga gcaagggctt caagttcgaa actgagaccg atactgaagc 1800
   tattgccaag ttggctaaat acatctacga tgaaaatcca accatcgagt tcaccacgct 1860
   cgccaaggct gttatccagg agcttgaggg cgcattcggt ctgctcataa aatccgttca 1920
   ctaccctcat gaggttatcg ccgcccgcaa aggctccccg ttagttgttg ggatacgatc 1980
   ccagggcaaa atgaaagttg acttcgttga tgtcgaaagt gctcatgagg atatcatgac 2040
   tgccgagacg gcatctcaaa gcctagcagt gaacaagtct cagggactga gtccaggcgg 2100
   cgctgctctt ggcaactcgc tcgcaccccc agacaagggc tttcttcacc actcgcaatc 2160
   gagggcattc ctgtcggacg aaggggtacc catgccagca gagttcttcc tatcttccga 2220
   tccagctgct gtcattgagc acactaagaa ggtattgtat ctcgaagatg atgatatcgc 2280
   tcacatccac gacggccagc tcaacattca ccgtatctcg aaagaccttg ggacgtctag 2340
   tgtccgtgct atccaaacga tcgagctcga attgcaagaa atgatgaaag gtaacttcga 2400
   ctacttcatg cagaaagaga tctacgaaca accagaatcc atcgtcaacg cgatgcgagg 2460
   tcgtctcaat gccgagaaaa agaccgtgac cctcggcggc ttaagacagt acctctcgac 2520
   catcagacgc tcccgcagaa tcatcttcat cgcctgcggc acctcgtacc actcgtgcgt 2580
   cgcagttcgc gggatcttcg aagaattgac cgagacacca attgctgttg aactagcttc 2640
   tgatttcctc gacagacaag ctcccgtctt ccgcgatgat acatgcgttt ttgtctctca 2700
   gtctggtgaa acagccgact ccctcatggc tctacgatac tgtcttagtc gcggtgctct 2760
   aaccgttggt attgtcaatg ttgtcggatc ctcaatctcc ctcttaactc actgcggtgt 2820
   ccacatcaac gctggccctg agatcggcgt tgcgtcgacc aaggcctaca cttcccaatt 2880
   cgtctgcatg gtcatgttcg ccttggcact tagtgaagac cgcacaagca agcgccagcg 2940
   ccgtatagat attatggagg ggctagccaa cacatctgag cagataaagg aggtactcaa 3000
   gctggatgag aagatcaaga acctatgcat gaaattcaag gaccagaaaa gtttgttact 3060
   cctcggtcgc ggtaatcagc atgccactgc tcttgaaggt cagtctaaca ccccgcccct 3120
   ttctctaata atagcccgaa cgctaacaaa tcccttttcc aggcgccctc aagattaaag 3180
   aaatctccta cctccactgt gaagccgtcc tgtccggcga actcaagcac ggtgttttag 3240
   ctcttgttga cgaggcacta cccctcgtca tgattctcac acgcgacgac aacttctcca 3300
   agtcgctgaa tgcctacaac caggttgttg cgcggaacgg tcggcccatc gtcatctgta 3360
   atactggtga ccccgagttt cctagcgaca agaccgatag gattgaggtc cctcgcactg 3420
   tggatgcgtt gcagggtctg ttgaacgtga ttcctctgca gctcatggct tattggctgg      3480
   ctgtcgctga ggggctgaat gttgattttc cgaggaattt ggctaagtct gttacagttg      3540
   agtag                                                                  3545
<210> 84
   <211> 2109
   <212> DNA
   <213> Aspergillus niger
<220>
   <221> CDS
   <222> (1) .. (2109)
<400> 84
   atg tgt gga att ttc ggc tac gtg aat tat ctc gta gag aag aac cgc      48
   Met Cys Gly Ile Phe Gly Tyr Val Asn Tyr Leu Val Glu Lys Asn Arg
   1               5                   10                  15
   aag gag att ctc gac acc ctc ctc aat ggg ctt tcg cgt cta gat tat      96
   Lys Glu Ile Leu Asp Thr Leu Leu Asn Gly Leu Ser Arg Leu Asp Tyr
               20                  25                  30
   cgt ggt tac gac tca gct ggg ctc gca atc gac ggt gac aaa aag aaa     144
   Arg Gly Tyr Asp Ser Ala Gly Leu Ala Ile Asp Gly Asp Lys Lys Lys
           35                  40                  45
   gaa gtg ttc atc tac aaa gaa gtc ggg aag gtc gca ggc ctg agg aag     192
   Glu Val Phe Ile Tyr Lys Glu Val Gly Lys Val Ala Gly Leu Arg Lys
       50                  55                  60
   ctt att acc gag gag aag ccc gat ccc act aag acc ttc gaa tct cac     240
   Leu Ile Thr Glu Glu Lys Pro Asp Pro Thr Lys Thr Phe Glu Ser His
   65                  70                  75                  80
   gct ggt att gca cac act cgt tgg gcg act cac gga gta cca tcg cga     288
   Ala Gly Ile Ala His Thr Arg Trp Ala Thr His Gly Val Pro Ser Arg
                   85                  90                  95
   acc aac tgt cat ccc cat agg tct gat cct acc tca gag ttt gtt gtt     336
   Thr Asn Cys His Pro His Arg Ser Asp Pro Thr Ser Glu Phe Val Val
               100                 105                 110
   gtt cac aat ggc att aca aca aac tac aaa gag ctg cgg aga ctc ctc     384
   Val His Asn Gly Ile Thr Thr Asn Tyr Lys Glu Leu Arg Arg Leu Leu
           115                 120                 125
   gcg agc aag ggc ttc aag ttc gaa act gag acc gat act gaa gct att     432
   Ala Ser Lys Gly Phe Lys Phe Glu Thr Glu Thr Asp Thr Glu Ala Ile
       130                 135                 140
   gcc aag ttg gct aaa tac atc tac gat gaa aat cca acc atc gag ttc     480
   Ala Lys Leu Ala Lys Tyr Ile Tyr Asp Glu Asn Pro Thr Ile Glu Phe
   145                 150                 155                 160
   acc acg ctc gcc aag gct gtt atc cag gag ctt gag ggc gca ttc ggt     528
   Thr Thr Leu Ala Lys Ala Val Ile Gln Glu Leu Glu Gly Ala Phe Gly
                   165                 170                 175
   ctg ctc ata aaa tcc gtt cac tac cct cat gag gtt atc gcc gcc cgc     576
   Leu Leu Ile Lys Ser Val His Tyr Pro His Glu Val Ile Ala Ala Arg
   180 185 190
   aaa ggc tcc ccg tta gtt gtt ggg ata cga tcc cag ggc aaa atg aaa 624
   Lys Gly Ser Pro Leu Val Val Gly Ile Arg Ser Gln Gly Lys Met Lys
   195 200 205
   gtt gac ttc gtt gat gtc gaa agt gct cat gag gat atc atg act gcc 672
   Val Asp Phe Val Asp Val Glu Ser Ala His Glu Asp Ile Met Thr Ala
   210 215 220
   gag acg gca tct caa agc cta gca gtg aac aag tct cag gga ctg agt 720
   Glu Thr Ala Ser Gln Ser Leu Ala Val Asn Lys Ser Gln Gly Leu Ser
   225 230 235 240
   cca ggc ggc gct gct ctt ggc aac tcg ctc gca ccc cca gac aag ggc 768
   Pro Gly Gly Ala Ala Leu Gly Asn Ser Leu Ala Pro Pro Asp Lys Gly
   245 250 255
   ttt ctt cac cac tcg caa tcg agg gca ttc ctg tcg gac gaa ggg gta 816
   Phe Leu His His Ser Gln Ser Arg Ala Phe Leu Ser Asp Glu Gly Val
   260 265 270
   ccc atg cca gca gag ttc ttc cta tct tcc gat cca gct gct gtc att 864
   Pro Met Pro Ala Glu Phe Phe Leu Ser Ser Asp Pro Ala Ala Val Ile
   275 280 285
   gag cac act aag aag gta ttg tat ctc gaa gat gat gat atc gct cac 912
   Glu His Thr Lys Lys Val Leu Tyr Leu Glu Asp Asp Asp Ile Ala His
   290 295 300
   atc cac gac ggc cag ctc aac att cac cgt atc tcg aaa gac ctt ggg 960
   Ile His Asp Gly Gln Leu Asn Ile His Arg Ile Ser Lys Asp Leu Gly
   305 310 315 320
   acg tct agt gtc cgt gct atc caa acg atc gag ctc gaa ttg caa gaa 1008
   Thr Ser Ser Val Arg Ala Ile Gln Thr Ile Glu Leu Glu Leu Gln Glu
   325 330 335
   atg atg aaa ggt aac ttc gac tac ttc atg cag aaa gag atc tac gaa 1056
   Met Met Lys Gly Asn Phe Asp Tyr Phe Met Gln Lys Glu Ile Tyr Glu
   340 345 350
   caa cca gaa tcc atc gtc aac gcg atg cga ggt cgt ctc aat gcc gag 1104
   Gln Pro Glu Ser Ile Val Asn Ala Met Arg Gly Arg Leu Asn Ala Glu
   355 360 365
   aaa aag acc gtg acc ctc ggc ggc tta aga cag tac ctc tcg acc atc 1152
   Lys Lys Thr Val Thr Leu Gly Gly Leu Arg Gln Tyr Leu Ser Thr Ile
   370 375 380
   aga cgc tcc cgc aga atc atc ttc atc gcc tgc ggc acc tcg tac cac 1200
   Arg Arg Ser Arg Arg Ile Ile Phe Ile Ala Cys Gly Thr Ser Tyr His
   385 390 395 400
   tcg tgc gtc gca gtt cgc ggg atc ttc gaa gaa ttg acc gag aca cca 1248
   Ser Cys Val Ala Val Arg Gly Ile Phe Glu Glu Leu Thr Glu Thr Pro
   405 410 415
   att gct gtt gaa cta gct tct gat ttc ctc gac aga caa gct ccc gtc 1296
   Ile Ala Val Glu Leu Ala Ser Asp Phe Leu Asp Arg Gln Ala Pro Val
   420 425 430
   ttc cgc gat gat aca tgc gtt ttt gtc tct cag tct ggt gaa aca gcc 1344
   Phe Arg Asp Asp Thr Cys Val Phe Val Ser Gln Ser Gly Glu Thr Ala
   435 440 445
   gac tcc ctc atg gct cta cga tac tgt ctt agt cgc ggt gct cta acc      1392
   Asp Ser Leu Met Ala Leu Arg Tyr Cys Leu Ser Arg Gly Ala Leu Thr
       450                 455                 460
   gtt ggt att gtc aat gtt gtc gga tcc tca atc tcc ctc tta act cac      1440
   Val Gly Ile Val Asn Val Val Gly Ser Ser Ile Ser Leu Leu Thr His
   465                 470                 475                 480
   tgc ggt gtc cac atc aac gct ggc cct gag atc ggc gtt gcg tcg acc      1488
   Cys Gly Val His Ile Asn Ala Gly Pro Glu Ile Gly Val Ala Ser Thr
                   485                 490                 495
   aag gcc tac act tcc caa ttc gtc tgc atg gtc atg ttc gcc ttg gca      1536
   Lys Ala Tyr Thr Ser Gln Phe Val Cys Met Val Met Phe Ala Leu Ala
               500                 505                 510
   ctt agt gaa gac cgc aca agc aag cgc cag cgc cgt ata gat att atg      1584
   Leu Ser Glu Asp Arg Thr Ser Lys Arg Gln Arg Arg Ile Asp Ile Met
           515                 520                 525
   gag ggg cta gcc aac aca tct gag cag ata aag gag gta ctc aag ctg      1632
   Glu Gly Leu Ala Asn Thr Ser Glu Gln Ile Lys Glu Val Leu Lys Leu
       530                 535                 540
   gat gag aag atc aag aac cta tgc atg aaa ttc aag gac cag aaa agt      1680
   Asp Glu Lys Ile Lys Asn Leu Cys Met Lys Phe Lys Asp Gln Lys Ser
   545                 550                 555                 560
   ttg tta ctc ctc ggt cgc ggt aat cag cat gcc act gct ctt gaa ggc      1728
   Leu Leu Leu Leu Gly Arg Gly Asn Gln His Ala Thr Ala Leu Glu Gly
                   565                 570                 575
   gcc ctc aag att aaa gaa atc tcc tac ctc cac tgt gaa gcc gtc ctg      1776
   Ala Leu Lys Ile Lys Glu Ile Ser Tyr Leu His Cys Glu Ala Val Leu
               580                 585                 590
   tcc ggc gaa ctc aag cac ggt gtt tta gct ctt gtt gac gag gca cta      1824
   Ser Gly Glu Leu Lys His Gly Val Leu Ala Leu Val Asp Glu Ala Leu
           595                 600                 605
   ccc ctc gtc atg att ctc aca cgc gac gac aac ttc tcc aag tcg ctg      1872
   Pro Leu Val Met Ile Leu Thr Arg Asp Asp Asn Phe Ser Lys Ser Leu
       610                 615                 620
   aat gcc tac aac cag gtt gtt gcg cgg aac ggt cgg ccc atc gtc atc      1920
   Asn Ala Tyr Asn Gln Val Val Ala Arg Asn Gly Arg Pro Ile Val Ile
   625                 630                 635                 640
   tgt aat act ggt gac ccc gag ttt cct agc gac aag acc gat agg att      1968
   Cys Asn Thr Gly Asp Pro Glu Phe Pro Ser Asp Lys Thr Asp Arg Ile
                   645                 650                 655
   gag gtc cct cgc act gtg gat gcg ttg cag ggt ctg ttg aac gtg att      2016
   Glu Val Pro Arg Thr Val Asp Ala Leu Gln Gly Leu Leu Asn Val Ile
               660                 665                 670
   cct ctg cag ctc atg gct tat tgg ctg gct gtc gct gag ggg ctg aat      2064
   Pro Leu Gln Leu Met Ala Tyr Trp Leu Ala Val Ala Glu Gly Leu Asn
           675                 680                 685
   gtt gat ttt ccg agg aat ttg gct aag tct gtt aca gtt gag tag          2109
   Val Asp Phe Pro Arg Asn Leu Ala Lys Ser Val Thr Val Glu
       690                 695                 700
<210> 85
   <211> 702
   <212> PRT
   <213> Aspergillus niger
<400> 85
   Met Cys Gly Ile Phe Gly Tyr Val Asn Tyr Leu Val Glu Lys Asn Arg
   1 5 10 15
   Lys Glu Ile Leu Asp Thr Leu Leu Asn Gly Leu Ser Arg Leu Asp Tyr
   20 25 30
   Arg Gly Tyr Asp Ser Ala Gly Leu Ala Ile Asp Gly Asp Lys Lys Lys
   35 40 45
   Glu Val Phe Ile Tyr Lys Glu Val Gly Lys Val Ala Gly Leu Arg Lys
   50 55 60
   Leu Ile Thr Glu Glu Lys Pro Asp Pro Thr Lys Thr Phe Glu Ser His
   65 70 75 80
   Ala Gly Ile Ala His Thr Arg Trp Ala Thr His Gly Val Pro Ser Arg
   85 90 95
   Thr Asn Cys His Pro His Arg Ser Asp Pro Thr Ser Glu Phe Val Val
   100 105 110
   Val His Asn Gly Ile Thr Thr Asn Tyr Lys Glu Leu Arg Arg Leu Leu
   115 120 125
   Ala Ser Lys Gly Phe Lys Phe Glu Thr Glu Thr Asp Thr Glu Ala Ile
   130 135 140
   Ala Lys Leu Ala Lys Tyr Ile Tyr Asp Glu Asn Pro Thr Ile Glu Phe
   145 150 155 160
   Thr Thr Leu Ala Lys Ala Val Ile Gln Glu Leu Glu Gly Ala Phe Gly
   165 170 175
   Leu Leu Ile Lys Ser Val His Tyr Pro His Glu Val Ile Ala Ala Arg
   180 185 190
   Lys Gly Ser Pro Leu Val Val Gly Ile Arg Ser Gln Gly Lys Met Lys
   195 200 205
   Val Asp Phe Val Asp Val Glu Ser Ala His Glu Asp Ile Met Thr Ala
   210 215 220
   Glu Thr Ala Ser Gln Ser Leu Ala Val Asn Lys Ser Gln Gly Leu Ser
   225 230 235 240
   Pro Gly Gly Ala Ala Leu Gly Asn Ser Leu Ala Pro Pro Asp Lys Gly
   245 250 255
   Phe Leu His His Ser Gln Ser Arg Ala Phe Leu Ser Asp Glu Gly Val
   260 265 270
   Pro Met Pro Ala Glu Phe Phe Leu Ser Ser Asp Pro Ala Ala Val Ile
   275 280 285
   Glu His Thr Lys Lys Val Leu Tyr Leu Glu Asp Asp Asp Ile Ala His
   290 295 300
   Ile His Asp Gly Gln Leu Asn Ile His Arg Ile Ser Lys Asp Leu Gly
   305 310 315 320
   Thr Ser Ser Val Arg Ala Ile Gln Thr Ile Glu Leu Glu Leu Gln Glu
   325 330 335
   Met Met Lys Gly Asn Phe Asp Tyr Phe Met Gln Lys Glu Ile Tyr Glu
   340 345 350
   Gln Pro Glu Ser Ile Val Asn Ala Met Arg Gly Arg Leu Asn Ala Glu
   355 360 365
   Lys Lys Thr Val Thr Leu Gly Gly Leu Arg Gln Tyr Leu Ser Thr Ile
   370 375 380
   Arg Arg Ser Arg Arg Ile Ile Phe Ile Ala Cys Gly Thr Ser Tyr His
   385 390 395 400
   Ser Cys Val Ala Val Arg Gly Ile Phe Glu Glu Leu Thr Glu Thr Pro
   405 410 415
   Ile Ala Val Glu Leu Ala Ser Asp Phe Leu Asp Arg Gln Ala Pro Val
   420 425 430
   Phe Arg Asp Asp Thr Cys Val Phe Val Ser Gln Ser Gly Glu Thr Ala
   435 440 445
   Asp Ser Leu Met Ala Leu Arg Tyr Cys Leu Ser Arg Gly Ala Leu Thr
   450 455 460
   Val Gly Ile Val Asn Val Val Gly Ser Ser Ile Ser Leu Leu Thr His
   465 470 475 480
   Cys Gly Val His Ile Asn Ala Gly Pro Glu Ile Gly Val Ala Ser Thr
   485 490 495
   Lys Ala Tyr Thr Ser Gln Phe Val Cys Met Val Met Phe Ala Leu Ala
   500 505 510
   Leu Ser Glu Asp Arg Thr Ser Lys Arg Gln Arg Arg Ile Asp Ile Met
   515 520 525
   Glu Gly Leu Ala Asn Thr Ser Glu Gln Ile Lys Glu Val Leu Lys Leu
   530 535 540
   Asp Glu Lys Ile Lys Asn Leu Cys Met Lys Phe Lys Asp Gln Lys Ser
   545 550 555 560
   Leu Leu Leu Leu Gly Arg Gly Asn Gln His Ala Thr Ala Leu Glu Gly
   565 570 575
   Ala Leu Lys Ile Lys Glu Ile Ser Tyr Leu His Cys Glu Ala Val Leu
   580 585 590
   Ser Gly Glu Leu Lys His Gly Val Leu Ala Leu Val Asp Glu Ala Leu
   595 600 605
   Pro Leu Val Met Ile Leu Thr Arg Asp Asp Asn Phe Ser Lys Ser Leu
   610 615 620
   Asn Ala Tyr Asn Gln Val Val Ala Arg Asn Gly Arg Pro Ile Val Ile
   625 630 635 640
   Cys Asn Thr Gly Asp Pro Glu Phe Pro Ser Asp Lys Thr Asp Arg Ile
   645 650 655
   Glu Val Pro Arg Thr Val Asp Ala Leu Gln Gly Leu Leu Asn Val Ile
   660 665 670
   Pro Leu Gln Leu Met Ala Tyr Trp Leu Ala Val Ala Glu Gly Leu Asn
   675 680 685
   Val Asp Phe Pro Arg Asn Leu Ala Lys Ser Val Thr Val Glu
   690 695 700
<210> 86
   <211> 3214
   <212> DNA
   <213> Aspergillus niger
<400> 86
   aaccagtcga gcaacagccg atcgtctcca ggccacgcgg ccctttgggt ccagcctcgt 60
   ccctccgcgt catcgcccct ccaggcgtca aatcctatcc cgatggcgtt acttcgtcct 120
   ccgagctcca agacatgccc actgtctatc tcgaacacac taaccatatt gatcgcttca 180
   atcgctggca tcataccaat aaggaaactc acaatacccg ttcggaaagt tggtggagtc 240
   tggtcgacca agcatatcct ccgtgcaccc agggttcttc tgccagggtc tttgataatt 300
   gcggagactg tgtgcttatc gatagggaat ctatccacaa tctcccccct aattcacaac 360
   tgaatgggat agataccttg catgcaacag attttttttt tccgtcgtca cccaccttca 420
   acacagtctg tcctccatga cgtatggaga atgtgaaact tgcaaaaggc gacttcgacg 480
   ccttattcgt catccactga catttgcctt gtcaaacatg gaacgcagga gaggcaaagt 540
   tgaaacatcc cgctgtgttg cggggcatcg atactccgaa cggttgatcg gcttacgaag 600
   cttgtcccac caaagttact atagtcaata ctatagttga ctgcaataaa aggctaaata 660
   agggtccttt gggtgctcta acgctggaaa atcccgaccg ttaggctacc taatctgctg 720
   tctttacccc cgttagccca cttacccagc tttagtctct cgaaggaaaa gaaaacaaag 780
   taatcgggag cctataatat tcggccagaa gtggggcaat cacttcaacc gttcatctgt 840
   tattgagtta tcaagtctgt caagttcgtc ggagctggat aaggtagtcg ccatccccat 900
   ttccgctgtg caatgccgca gcgccgaagt gtctgcagct agctctattt tgttcagcta 960
   cgtatctgtc tcttaaccaa atggatgtcg ccattcgccc actaacaacg ccattcccca 1020
   cggccgacgg actccggcgc aagggcctga taagcgttag ctccgactcc gagttagtca 1080
   tgcggcttat tacgcgccct tctcccctca cgatgttgat gctttttctg gtatctctgg 1140
   cataactggc aaagatcagg ctgtggtcat aagagtatca ttgccttcag cttcttcgcc 1200
   atgttgagta gtctgctgct tgggggtctt ctgggtctag cgaccgctca atttcctccc 1260
   gagccggaag gcatcactgt gctcaagtcc aagttgcatg agaatgtgac tatttctttc 1320
   aaagaggtgt gtgcagagta tctagaaata gcttttatgc tcgatgccgt gctgattgtc 1380
   agcctggaat ttgcgaaact acgccgggtg tccgatctta ttcgggctat gtacaccttc 1440
   cccccggttt cctttccgac gggacaggag aagtgcagga ttatcctatc aacacgtaag 1500
   ccaatctcga aacattggag gatgagcaat tactgagcct caaccagctt cttttggttt 1560
   ttcgaagccc gcaaagatcc cagcaatgcg cctctggcca tctggctcaa tggcggtccg 1620
   ggtggctcgt cgctcatggg gctccttgaa gaattaggtc cttgttccat tgcatcagac 1680
   tccaagacca cagtcctcaa tccttggagt tggaacaatg aagtcaatct tctattcctt 1740
   gaccagccaa ctcaagtcgg cttctcatac gatgtcccaa caaatggcac tttggttcgg 1800
   actgcggacg gcgaagaaga gatagtttcc ggtgatttct ccattgatgt tccccagtcc 1860
   aacttcaccc atcatgttgg tacctttgca agccagaagc ttgcacagac agctaatggg 1920
   actgcattcg cggctcacgc tctatggcat ttcgcgcaaa cctggttttt cgagttccca 1980
   cactacaagc caaacgatga tcgtgtcagt ctctgggctg aaagttacgg aggccattat 2040
   ggtccaggca tctttcggtt cttccaacag cagaatgaca aaatcgcaga ggggactgca 2100
   gaagacggtg cacagtattt gcatctcgac acgcttggca ttgtgaacgg cttgatggat      2160
   atggtgatcc aagaagaggc ttacattact tggccataca ataacgtaag gctcgcccct      2220
   tcttcattca actcgcgtaa tgcctaattc agttcagacc tacggcctcg aaatcttcga      2280
   taaacccctc tacgaagaac tgatgtataa ctggacgcat ccaggaggct ttcgcgatca      2340
   ggccctcgcc tgcgaagcgg ctttgaaaga acgcgattcc ggcttgcctc actcagggaa      2400
   gaatatctct gaaatttgcg gaggccttgc actagaatgg ggagatggcc ccatcaccta      2460
   ctaccacacc ttcaatcgcg ggtggtacga catcgcccat cctaagaacg acccattccc      2520
   tgccaagcac atgctcggat atttgacgca ggagtccgtc cttgccgctc ttggggtacc      2580
   agtcaatttc acatcgtctt cgagtgccgt ggctacacag ttcataaaaa cctttgatat      2640
   cgtccacggc ggcttcctgg atgcaattgg ctacctcctc gacagtggtg taaaagtaca      2700
   catgatgtac ggagatcgtg attacgcctg caattgggtc gggggcgaaa aagccagcct      2760
   tgcagttccg tattcccgta tcaccgaatt tgccgacacg ggatactccc cactccttac      2820
   gcccgacggg atcagcggca tgacccgcca gctgggcaac tacagcttca ctcgcgtctt      2880
   ccaagccggg catgaggtcc cctcctacca gcctgtcgcg gcgtatgaga tcttcatgcg      2940
   ggcgacattc aacaaagata tccctactgg cctcttggct gttgatgacg aattccagtc      3000
   ggttggacct aaggatacgt ggcatatcaa gaatatccct cctattatgc caaagccgca      3060
   gtgctatgtt ctaagtcccg gcacgtgtac cccggaggtt tgggagaccg ttttgaacgg      3120
   atccgcgacg gtaaaggatt ggtatgtcgt ggatgatagc gcgggtgttg aggaccacga      3180
   ggggttcagc attcttggag gggatgagtt gtag                                  3214
<210> 87
   <211> 1818
   <212> DNA
   <213> Aspergillus niger
<220>
   <221> CDS
   <222> (1)..(1818)
<400> 87
   atg ttg agt agt ctg ctg ctt ggg ggt ctt ctg ggt cta gcg acc gct       48
   Met Leu Ser Ser Leu Leu Leu Gly Gly Leu Leu Gly Leu Ala Thr Ala
   1               5                   10                  15
   caa ttt cct ccc gag ccg gaa ggc atc act gtg ctc aag tcc aag ttg       96
   Gln Phe Pro Pro Glu Pro Glu Gly Ile Thr Val Leu Lys Ser Lys Leu
               20                  25                  30
   cat gag aat gtg act att tct ttc aaa gag cct gga att tgc gaa act      144
   His Glu Asn Val Thr Ile Ser Phe Lys Glu Pro Gly Ile Cys Glu Thr
           35                  40                  45
   acg ccg ggt gtc cga tct tat tcg ggc tat gta cac ctt ccc ccc ggt      192
   Thr Pro Gly Val Arg Ser Tyr Ser Gly Tyr Val His Leu Pro Pro Gly
       50                  55                  60
   ttc ctt tcc gac ggg aca gga gaa gtg cag gat tat cct atc aac acc 240
   Phe Leu Ser Asp Gly Thr Gly Glu Val Gln Asp Tyr Pro Ile Asn Thr
   65 70 75 80
   ttc ttt tgg ttt ttc gaa gcc cgc aaa gat ccc agc aat gcg cct ctg 288
   Phe Phe Trp Phe Phe Glu Ala Arg Lys Asp Pro Ser Asn Ala Pro Leu
   85 90 95
   gcc atc tgg ctc aat ggc ggt ccg ggt ggc tcg tcg ctc atg ggg ctc 336
   Ala Ile Trp Leu Asn Gly Gly Pro Gly Gly Ser Ser Leu Met Gly Leu
   100 105 110
   ctt gaa gaa tta ggt cct tgt tcc att gca tca gac tcc aag acc aca 384
   Leu Glu Glu Leu Gly Pro Cys Ser Ile Ala Ser Asp Ser Lys Thr Thr
   115 120 125
   gtc ctc aat cct tgg agt tgg aac aat gaa gtc aat ctt cta ttc ctt 432
   Val Leu Asn Pro Trp Ser Trp Asn Asn Glu Val Asn Leu Leu Phe Leu
   130 135 140
   gac cag cca act caa gtc ggc ttc tca tac gat gtc cca aca aat ggc 480
   Asp Gln Pro Thr Gln Val Gly Phe Ser Tyr Asp Val Pro Thr Asn Gly
   145 150 155 160
   act ttg gtt cgg act gcg gac ggc gaa gaa gag ata gtt tcc ggt gat 528
   Thr Leu Val Arg Thr Ala Asp Gly Glu Glu Glu Ile Val Ser Gly Asp
   165 170 175
   ttc tcc att gat gtt ccc cag tcc aac ttc acc cat cat gtt ggt acc 576
   Phe Ser Ile Asp Val Pro Gln Ser Asn Phe Thr His His Val Gly Thr
   180 185 190
   ttt gca agc cag aag ctt gca cag aca gct aat ggg act gca ttc gcg 624
   Phe Ala Ser Gln Lys Leu Ala Gln Thr Ala Asn Gly Thr Ala Phe Ala
   195 200 205
   gct cac gct cta tgg cat ttc gcg caa acc tgg ttt ttc gag ttc cca 672
   Ala His Ala Leu Trp His Phe Ala Gln Thr Trp Phe Phe Glu Phe Pro
   210 215 220
   cac tac aag cca aac gat gat cgt gtc agt ctc tgg gct gaa agt tac 720
   His Tyr Lys Pro Asn Asp Asp Arg Val Ser Leu Trp Ala Glu Ser Tyr
   225 230 235 240
   gga ggc cat tat ggt cca ggc atc ttt cgg ttc ttc caa cag cag aat 768
   Gly Gly His Tyr Gly Pro Gly Ile Phe Arg Phe Phe Gln Gln Gln Asn
   245 250 255
   gac aaa atc gca gag ggg act gca gaa gac ggt gca cag tat ttg cat 816
   Asp Lys Ile Ala Glu Gly Thr Ala Glu Asp Gly Ala Gln Tyr Leu His
   260 265 270
   ctc gac acg ctt ggc att gtg aac ggc ttg atg gat atg gtg atc caa 864
   Leu Asp Thr Leu Gly Ile Val Asn Gly Leu Met Asp Met Val Ile Gln
   275 280 285
   gaa gag gct tac att act tgg cca tac aat aac gta agg ctc gcc cct 912
   Glu Glu Ala Tyr Ile Thr Trp Pro Tyr Asn Asn Val Arg Leu Ala Pro
   290 295 300
   tct tca ttc aac tcg cga ggc ttt cgc gat cag gcc ctc gcc tgc gaa 960
   Ser Ser Phe Asn Ser Arg Gly Phe Arg Asp Gln Ala Leu Ala Cys Glu
   305 310 315 320
   gcg gct ttg aaa gaa cgc gat tcc ggc ttg cct cac tca ggg aag aat        1008
   Ala Ala Leu Lys Glu Arg Asp Ser Gly Leu Pro His Ser Gly Lys Asn
                   325                 330                 335
   atc tct gaa att tgc gga ggc ctt gca cta gaa tgg gga gat ggc ccc        1056
   Ile Ser Glu Ile Cys Gly Gly Leu Ala Leu Glu Trp Gly Asp Gly Pro
               340                 345                 350
   atc acc tac tac cac acc ttc aat cgc ggg tgg tac gac atc gcc cat        1104
   Ile Thr Tyr Tyr His Thr Phe Asn Arg Gly Trp Tyr Asp Ile Ala His
           355                 360                 365
   cct aag aac gac cca ttc cct gcc aag cac atg ctc gga tat ttg acg        1152
   Pro Lys Asn Asp Pro Phe Pro Ala Lys His Met Leu Gly Tyr Leu Thr
       370                 375                 380
   cag gag tcc gtc ctt gcc gct ctt ggg gta cca gtc aat ttc aca tcg        1200
   Gln Glu Ser Val Leu Ala Ala Leu Gly Val Pro Val Asn Phe Thr Ser
   385                 390                 395                 400
   tct tcg agt gcc gtg gct aca cag ttc ata aaa acc ttt gat atc gtc        1248
   Ser Ser Ser Ala Val Ala Thr Gln Phe Ile Lys Thr Phe Asp Ile Val
                   405                 410                 415
   cac ggc ggc ttc ctg gat gca att ggc tac ctc ctc gac agt ggt gta        1296
   His Gly Gly Phe Leu Asp Ala Ile Gly Tyr Leu Leu Asp Ser Gly Val
               420                 425                 430
   aaa gta cac atg atg tac gga gat cgt gat tac gcc tgc aat tgg gtc        1344
   Lys Val His Met Met Tyr Gly Asp Arg Asp Tyr Ala Cys Asn Trp Val
           435                 440                 445
   ggg ggc gaa aaa gcc agc ctt gca gtt ccg tat tcc cgt atc acc gaa        1392
   Gly Gly Glu Lys Ala Ser Leu Ala Val Pro Tyr Ser Arg Ile Thr Glu
       450                 455                 460
   ttt gcc gac acg gga tac tcc cca ctc ctt acg ccc gac ggg atc agc        1440
   Phe Ala Asp Thr Gly Tyr Ser Pro Leu Leu Thr Pro Asp Gly Ile Ser
   465                 470                 475                 480
   ggc atg acc cgc cag ctg ggc aac tac agc ttc act cgc gtc ttc caa        1488
   Gly Met Thr Arg Gln Leu Gly Asn Tyr Ser Phe Thr Arg Val Phe Gln
                   485                 490                 495
   gcc ggg cat gag gtc ccc tcc tac cag cct gtc gcg gcg tat gag atc        1536
   Ala Gly His Glu Val Pro Ser Tyr Gln Pro Val Ala Ala Tyr Glu Ile
               500                 505                 510
   ttc atg cgg gcg aca ttc aac aaa gat atc cct act ggc ctc ttg gct        1584
   Phe Met Arg Ala Thr Phe Asn Lys Asp Ile Pro Thr Gly Leu Leu Ala
           515                 520                 525
   gtt gat gac gaa ttc cag tcg gtt gga cct aag gat acg tgg cat atc        1632
   Val Asp Asp Glu Phe Gln Ser Val Gly Pro Lys Asp Thr Trp His Ile
       530                 535                 540
   aag aat atc cct cct att atg cca aag ccg cag tgc tat gtt cta agt        1680
   Lys Asn Ile Pro Pro Ile Met Pro Lys Pro Gln Cys Tyr Val Leu Ser
   545                 550                 555                 560
   ccc ggc acg tgt acc ccg gag gtt tgg gag acc gtt ttg aac gga tcc        1728
   Pro Gly Thr Cys Thr Pro Glu Val Trp Glu Thr Val Leu Asn Gly Ser
                   565                 570                 575
   gcg acg gta aag gat tgg tat gtc gtg gat gat agc gcg ggt gtt gag        1776
   Ala Thr Val Lys Asp Trp Tyr Val Val Asp Asp Ser Ala Gly Val Glu
               580                 585                 590
   gac cac gag ggg ttc agc att ctt gga ggg gat gag ttg tag                    1818
   Asp His Glu Gly Phe Ser Ile Leu Gly Gly Asp Glu Leu
           595                 600                 605
<210> 88
   <211> 605
   <212> PRT
   <213> Aspergillus niger
<400> 88
   Met Leu Ser Ser Leu Leu Leu Gly Gly Leu Leu Gly Leu Ala Thr Ala
   1               5                   10                  15
   Gln Phe Pro Pro Glu Pro Glu Gly Ile Thr Val Leu Lys Ser Lys Leu
               20                  25                  30
   His Glu Asn Val Thr Ile Ser Phe Lys Glu Pro Gly Ile Cys Glu Thr
           35                  40                  45
   Thr Pro Gly Val Arg Ser Tyr Ser Gly Tyr Val His Leu Pro Pro Gly
       50                  55                  60
   Phe Leu Ser Asp Gly Thr Gly Glu Val Gln Asp Tyr Pro Ile Asn Thr
   65                  70                  75                  80
   Phe Phe Trp Phe Phe Glu Ala Arg Lys Asp Pro Ser Asn Ala Pro Leu
                   85                  90                  95
   Ala Ile Trp Leu Asn Gly Gly Pro Gly Gly Ser Ser Leu Met Gly Leu
               100                 105                 110
   Leu Glu Glu Leu Gly Pro Cys Ser Ile Ala Ser Asp Ser Lys Thr Thr
           115                 120                 125
   Val Leu Asn Pro Trp Ser Trp Asn Asn Glu Val Asn Leu Leu Phe Leu
       130                 135                 140
   Asp Gln Pro Thr Gln Val Gly Phe Ser Tyr Asp Val Pro Thr Asn Gly
   145                 150                 155                 160
   Thr Leu Val Arg Thr Ala Asp Gly Glu Glu Glu Ile Val Ser Gly Asp
                   165                 170                 175
   Phe Ser Ile Asp Val Pro Gln Ser Asn Phe Thr His His Val Gly Thr
               180                 185                 190
   Phe Ala Ser Gln Lys Leu Ala Gln Thr Ala Asn Gly Thr Ala Phe Ala
           195                 200                 205
   Ala His Ala Leu Trp His Phe Ala Gln Thr Trp Phe Phe Glu Phe Pro
   210 215 220
   His Tyr Lys Pro Asn Asp Asp Arg Val Ser Leu Trp Ala Glu Ser Tyr
   225 230 235 240
   Gly Gly His Tyr Gly Pro Gly Ile Phe Arg Phe Phe Gln Gln Gln Asn
   245 250 255
   Asp Lys Ile Ala Glu Gly Thr Ala Glu Asp Gly Ala Gln Tyr Leu His
   260 265 270
   Leu Asp Thr Leu Gly Ile Val Asn Gly Leu Met Asp Met Val Ile Gln
   275 280 285
   Glu Glu Ala Tyr Ile Thr Trp Pro Tyr Asn Asn Val Arg Leu Ala Pro
   290 295 300
   Ser Ser Phe Asn Ser Arg Gly Phe Arg Asp Gln Ala Leu Ala Cys Glu
   305 310 315 320
   Ala Ala Leu Lys Glu Arg Asp Ser Gly Leu Pro His Ser Gly Lys Asn
   325 330 335
   Ile Ser Glu Ile Cys Gly Gly Leu Ala Leu Glu Trp Gly Asp Gly Pro
   340 345 350
   Ile Thr Tyr Tyr His Thr Phe Asn Arg Gly Trp Tyr Asp Ile Ala His
   355 360 365
   Pro Lys Asn Asp Pro Phe Pro Ala Lys His Met Leu Gly Tyr Leu Thr
   370 375 380
   Gln Glu Ser Val Leu Ala Ala Leu Gly Val Pro Val Asn Phe Thr Ser
   385 390 395 400
   Ser Ser Ser Ala Val Ala Thr Gln Phe Ile Lys Thr Phe Asp Ile Val
   405 410 415
   His Gly Gly Phe Leu Asp Ala Ile Gly Tyr Leu Leu Asp Ser Gly Val
   420 425 430
   Lys Val His Met Met Tyr Gly Asp Arg Asp Tyr Ala Cys Asn Trp Val
   435 440 445
   Gly Gly Glu Lys Ala Ser Leu Ala Val Pro Tyr Ser Arg Ile Thr Glu
   450 455 460
   Phe Ala Asp Thr Gly Tyr Ser Pro Leu Leu Thr Pro Asp Gly Ile Ser
   465 470 475 480
   Gly Met Thr Arg Gln Leu Gly Asn Tyr Ser Phe Thr Arg Val Phe Gln
   485 490 495
   Ala Gly His Glu Val Pro Ser Tyr Gln Pro Val Ala Ala Tyr Glu Ile
   500 505 510
   Phe Met Arg Ala Thr Phe Asn Lys Asp Ile Pro Thr Gly Leu Leu Ala
   515 520 525
   Val Asp Asp Glu Phe Gln Ser Val Gly Pro Lys Asp Thr Trp His Ile
   530 535 540
   Lys Asn Ile Pro Pro Ile Met Pro Lys Pro Gln Cys Tyr Val Leu Ser
   545 550 555 560
   Pro Gly Thr Cys Thr Pro Glu Val Trp Glu Thr Val Leu Asn Gly Ser
   565 570 575
   Ala Thr Val Lys Asp Trp Tyr Val Val Asp Asp Ser Ala Gly Val Glu
   580 585 590
   Asp His Glu Gly Phe Ser Ile Leu Gly Gly Asp Glu Leu
   595 600 605
<210> 89
   <211> 4551
   <212> DNA
   <213> Aspergillus niger
<400> 89
   taacgtgtcg gtcggagatc tttctcggtc atagattgtg acagatctat aatcaagatg 60
   agatgacgga ttataccccg ttgtaaagga gtcgtatctc tgaggagcct gcaagcgcga 120
   agacatggaa ctcagctcga accgttatca aaactgagac taactcggct tgaaagtaca 180
   ccctaccttt tccttttccc agcctcgaga agcccttcaa ctgtagagct gattgtaccg 240
   tcggcacctt caactaaggt ttcccatgtt ctcgagacct cccattctgc gcctccacgg 300
   tgctttctcc tcttggagcg ggatgcggac gtagtctctc cgcgagaccc tggtagaaga 360
   ttgttttcat cctcatcctc agagatttct cctatgtact cttcatcgga atcgaccatg 420
   cttgcactaa aaggaagcga gtgcgtcaac ccaagagtgc gtcgggtgtc atgaaagtta 480
   gaatggaaag gtctatctag gcatgtatcc acattgttgg attcggttcc agcagggagg 540
   gtccgagctc cgccggagct ccgttcccta ccttcgattc ctcatgaacc aaaggcctat 600
   ctagcagttg ctattctctt cccttttctc tttatttcct tatcaattca tcctcgcatt 660
   ccttactact atctcacttc tctcaattct ataatactaa ccttcctgtc taggtctttt      720
   tttttttttt tttttttttt tggataccta cccttttatc tactctgagc tcccttgtgg      780
   tcaaagaaag tctgcttcgt aacctgaaga ttaatcagct acacttcgta ttgctacgac 840
   ggtgtactac aataactaat gagctgctcc gagagtaacc gtaggtaggc agacttggct 900
   ccgtcctaaa aaatgtgggg aagaagatgc atcgcgtcaa cccgacgtgg tggtatgaat 960
   gctcatgcag tccgtggtca atctccaatt tatcgtccgg tccgaccagc ttcccacctc 1020
   tgcccctcct cctcctcctc ctccaccagc acttctttct gcccctcctt catctcttcg 1080
   tcacgctccc gctccctttc cggattgccc tactcgacat acctacagtc tacccgtagg 1140
   gtcaaacctg ctgtattgtc acccgtttct gtaaggcact gttcatctga aatccgcgca 1200
   atggcatcta aggacagaga tatcctcccg gacgtgtaag ttaccgatcg ccaacagctg 1260
   agtatcgtgc acccatctgt tggtgtttct ctaacaatcg ctggcagggt caagcctgtt 1320
   cattacaatg tgtctttgtt tgaccttcag ttcggtggct catggggtta taagggtacc 1380
   gtcaaaatcg attccaaggt caaccgtccc acaaaagaga tcgtgttgaa ctcgaaggaa 1440
   atcgaagtcc aggatgccga agtttttgga aatgacggtt agtgacagag ccacactcca 1500
   atttctcctg atccatttgt aatccatgat cgaatgttga gtttgacaaa ttggttgcac 1560
   ttagggacca agctggcaaa ggcatctaat atcgcttacg acaccaaatc ggaaagagtc 1620
   acctttacct ttgcagaaga aatactcccc gcagatgttg tcctgtcaat caatttcacc 1680
   ggtataatga acaatgccat ggctggattt tcccgctcca agtacaagcc ggtggtggat 1740
   ccgaccgacg atactcccaa ggacggagat tcctattaca tgcttagtac ccaattcgaa 1800
   tcttgtgatg ctcgtagggc cttcccttgc tttgatgaac cgaatttgaa ggccacattc 1860
   gacttcgaga tcgaggttcc ccgaggtcaa acagccctta gcaacatgcc tatcaagtct 1920
   gagaggagcg gcagcaggcc agagctcaag ttggtctctt ttgaaactac acctgttatg 1980
   agtacttacg tcagtatgca cgattacttg cttatgatcc tcggcgctaa ctttggagat 2040
   cctccgttca gcttctggca tgggcagtcg gtgactttga atatgttgag gcaatgactc 2100
   aacgcaaata ccaaggcaag agcatacctg ttcgcgtgta tacgaccaag ggcctcaagg 2160
   agcaggctcg ctttgctttg gaatgtgccc atcgtacagt cgattacttc tctgaaatct 2220
   ttgagatcga gtatccgttg cctaaggcgg atctccttgc tgtccatgaa tttgtacgga 2280
   aattgatcct gttgcacacg aagcgaatat gctaaccgca aataatctag gctatgggtg 2340
   ccatggagaa ctgggggctt gttacctacc gaacgactgc tgttcttttc gatgagggca 2400
   agtcagacac cagatacaag aatcgtattg catatgtcgt cgcgcatggt ttgtcgtccc 2460
   ttccaatctg agcggccttg ttccttctta acgggtgtaa tctagaattg gcgcaccaat 2520
   ggtttggaaa cctcgtaacg atggattggt ggaatgagtt gtggctcaat gagggctttg 2580
   caacatgggt tggctggctt gctgtggatc atttctatcc aggtatccga tggccccagg 2640
   cgcttacaga accagaaaag ctgacgattt tcttgcaaat agaatggaac atctggtctc     2700
   agtttgtcgt acgcaattct cgaggtaaat gcagtcctgt gtggcagaat attgactttt     2760
   gcataggctg agagtgtcca acaagcattt caactcgatt cactgcgagc ttcccacccg     2820
   atcgaggtgc ccgtgaggaa cgctctcgaa gtcgatcaaa tttttgatca tatcagctac     2880
   ctcaaaggaa gctcggtcat tcggatgcta agtgatcatc ttggccggga aacattcctg     2940
   cgtggagtag ccgcatatct caaagctcat gcatatggca agtacacatc gaagacgtat     3000
   agggaatcgg gcttgctaat tactgacaac ttcaggtaat gccaccacta atgatctttg     3060
   gagcgctctc agcaaagcgt ctaatcagga tgttactagc tttatggtga gttatctcgt     3120
   tgtacagact ggactactat tctgacttca ttacgaagga tccctggatt cgcaagattg     3180
   gcttccctgt tgtcaccgta accgagcaag ccggccaact cagtgttcgt caaagccgct     3240
   tcttgtcgac gggtgatgtt aagcccgagg aagatgagac ggcctggtgg attccccttg     3300
   gtgtaaaatc aggacctaag atggccgacg taaaacccgg tgctcttgtt tccaaggaag     3360
   atacaatttg ggggctcgga caagattcct attacaagct gaacaaagac ctgtctggct     3420
   tttaccgaac caattaccct gctgatcggc tggcgaaact cgctcagtct ctggaactgc     3480
   tgagcaccga ggataagatt ggattgattg gcgatgccgc tgccctagct gtttctggcg     3540
   acggatccac tgctgctcta ttggctctct tagagggttt caagggcgag aagaattatt     3600
   tgtgagtaac taattatgat gtccttcatt cgttctcggg tttcaacaat ccttatgctt     3660
   catggttggt tcttaacgac ttgcataggg tctggtccca aatctcttcc actattgcga     3720
   acttacgctc tgtgttcgcc ctgaatgagt cggtagcagc aggactcaag aagtttgccc     3780
   tcgagctttc atcccctgct gctaacaaaa tcggctggga atttagctcg gaggatgact     3840
   atctcactat ccagctccgt aagcttctaa tcggaatggc tggccgtgcg ggccacaacg     3900
   agtgggtcta ttaggagatg ttgtacgctt ttcttgcaaa tctaactttt tgctagcata     3960
   atatccgagg ccgaacgacg atttgagctt tggaaatcag gcagcgataa ggatgctgtg     4020
   cataccaacc tccgctcagt gatctttagt atcgtgattt cagagggcgg tcgtgaagaa     4080
   tacaatgctg taaagcaaga gtatctcaag accgactccg ttgacggcaa ggagatttgc     4140
   ttaggagctc ttggacgtac taaagatgct gagctggtaa aggattactt agattttgtt     4200
   ttctcagaca aggttgccat ccaggatatt cacaatggcg cggcttcaat ggctacgaat     4260
   ccctcgactc gccaccttct atgggattat atgaaagaga attgggcggc cgttgaaact     4320
   cgcttgtcag cgaacaatgt cgttttcgag cggtttgtgc gtatgggact gtccaaattc     4380
   gcgaaccacg atatcgcagc cgatattgct tcgtttttcc gggagaagga cactggtgcg     4440
   tacgatcgcg ccctggtaat cgttgctgat agcatccgaa cgaatgcacg ctacaaggaa     4500
   agagatgaga agcaggtctt agagtggctt cggggccatg gctacgcttg a              4551
<210> 90
   <211> 2649
   <212> DNA
   <213> Aspergillus niger
<220>
   <221> CDS
   <222> (1) .. (2649)
<400> 90
   atg gca tct aag gac aga gat atc ctc ccg gac gtg gtc aag cct gtt        48
   Met Ala Ser Lys Asp Arg Asp Ile Leu Pro Asp Val Val Lys Pro Val
   1               5                   10                  15
   cat tac aat gtg tct ttg ttt gac ctt cag ttc ggt ggc tca tgg ggt        96
   His Tyr Asn Val Ser Leu Phe Asp Leu Gln Phe Gly Gly Ser Trp Gly
               20                  25                  30
   tat aag ggt acc gtc aaa atc gat tcc aag gtc aac cgt ccc aca aaa       144
   Tyr Lys Gly Thr Val Lys Ile Asp Ser Lys Val Asn Arg Pro Thr Lys
           35                  40                  45
   gag atc gtg ttg aac tcg aag gaa atc gaa gtc cag gat gcc gaa gtt       192
   Glu Ile Val Leu Asn Ser Lys Glu Ile Glu Val Gln Asp Ala Glu Val
       50                  55                  60
   ttt gga aat gac ggg acc aag ctg gca aag gca tct aat atc gct tac       240
   Phe Gly Asn Asp Gly Thr Lys Leu Ala Lys Ala Ser Asn Ile Ala Tyr
   65                  70                  75                  80
   gac acc aaa tcg gaa aga gtc acc ttt acc ttt gca gaa gaa ata ctc       288
   Asp Thr Lys Ser Glu Arg Val Thr Phe Thr Phe Ala Glu Glu Ile Leu
                   85                  90                  95
   ccc gca gat gtt gtc ctg tca atc aat ttc acc ggt ata atg aac aat       336
   Pro Ala Asp Val Val Leu Ser Ile Asn Phe Thr Gly Ile Met Asn Asn
               100                 105                 110
   gcc atg gct gga ttt tcc cgc tcc aag tac aag ccg gtg gtg gat ccg       384
   Ala Met Ala Gly Phe Ser Arg Ser Lys Tyr Lys Pro Val Val Asp Pro
           115                 120                 125
   acc gac gat act ccc aag gac gga gat tcc tat tac atg ctt agt acc       432
   Thr Asp Asp Thr Pro Lys Asp Gly Asp Ser Tyr Tyr Met Leu Ser Thr
       130                 135                 140
   caa ttc gaa tct tgt gat gct cgt agg gcc ttc cct tgc ttt gat gaa       480
   Gln Phe Glu Ser Cys Asp Ala Arg Arg Ala Phe Pro Cys Phe Asp Glu
   145                 150                 155                 160
   ccg aat ttg aag gcc aca ttc gac ttc gag atc gag gtt ccc cga ggt       528
   Pro Asn Leu Lys Ala Thr Phe Asp Phe Glu Ile Glu Val Pro Arg Gly
                   165                 170                 175
   caa aca gcc ctt agc aac atg cct atc aag tct gag agg agc ggc agc       576
   Gln Thr Ala Leu Ser Asn Met Pro Ile Lys Ser Glu Arg Ser Gly Ser
               180                 185                 190
   agg cca gag ctc aag ttg gtc tct ttt gaa act aca cct gtt atg agt       624
   Arg Pro Glu Leu Lys Leu Val Ser Phe Glu Thr Thr Pro Val Met Ser
           195                 200                 205
   act tac ctt ctg gca tgg gca gtc ggt gac ttt gaa tat gtt gag gca       672
   Thr Tyr Leu Leu Ala Trp Ala Val Gly Asp Phe Glu Tyr Val Glu Ala
       210                 215                 220
   atg act caa cgc aaa tac caa ggc aag agc ata cct gtt cgc gtg tat      720
   Met Thr Gln Arg Lys Tyr Gln Gly Lys Ser Ile Pro Val Arg Val Tyr
   225                 230                 235                 240
   acg acc aag ggc ctc aag gag cag gct cgc ttt gct ttg gaa tgt gcc      768
   Thr Thr Lys Gly Leu Lys Glu Gln Ala Arg Phe Ala Leu Glu Cys Ala
                   245                 250                 255
   cat cgt aca gtc gat tac ttc tct gaa atc ttt gag atc gag tat ccg      816
   His Arg Thr Val Asp Tyr Phe Ser Glu Ile Phe Glu Ile Glu Tyr Pro
               260                 265                 270
   ttg cct aag gcg gat ctc ctt gct gtc cat gaa ttt gct atg ggt gcc      864
   Leu Pro Lys Ala Asp Leu Leu Ala Val His Glu Phe Ala Met Gly Ala
           275                 280                 285
   atg gag aac tgg ggg ctt gtt acc tac cga acg act gct gtt ctt ttc      912
   Met Glu Asn Trp Gly Leu Val Thr Tyr Arg Thr Thr Ala Val Leu Phe
       290                 295                 300
   gat gag ggc aag tca gac acc aga tac aag aat cgt att gca tat gtc      960
   Asp Glu Gly Lys Ser Asp Thr Arg Tyr Lys Asn Arg Ile Ala Tyr Val
   305                 310                 315                 320
   gtc gcg cat gaa ttg gcg cac caa tgg ttt gga aac ctc gta acg atg     1008
   Val Ala His Glu Leu Ala His Gln Trp Phe Gly Asn Leu Val Thr Met
                   325                 330                 335
   gat tgg tgg aat gag ttg tgg ctc aat gag ggc ttt gca aca tgg gtt     1056
   Asp Trp Trp Asn Glu Leu Trp Leu Asn Glu Gly Phe Ala Thr Trp Val
               340                 345                 350
   ggc tgg ctt gct gtg gat cat ttc tat cca gaa tgg aac atc tgg tct     1104
   Gly Trp Leu Ala Val Asp His Phe Tyr Pro Glu Trp Asn Ile Trp Ser
           355                 360                 365
   cag ttt gtc gct gag agt gtc caa caa gca ttt caa ctc gat tca ctg     1152
   Gln Phe Val Ala Glu Ser Val Gln Gln Ala Phe Gln Leu Asp Ser Leu
       370                 375                 380
   cga gct tcc cac ccg atc gag gtg ccc gtg agg aac gct ctc gaa gtc     1200
   Arg Ala Ser His Pro Ile Glu Val Pro Val Arg Asn Ala Leu Glu Val
   385                 390                 395                 400
   gat caa att ttt gat cat atc agc tac ctc aaa gga agc tcg gtc att     1248
   Asp Gln Ile Phe Asp His Ile Ser Tyr Leu Lys Gly Ser Ser Val Ile
                   405                 410                 415
   cgg atg cta agt gat cat ctt ggc cgg gaa aca ttc ctg cgt gga gta     1296
   Arg Met Leu Ser Asp His Leu Gly Arg Glu Thr Phe Leu Arg Gly Val
               420                 425                 430
   gcc gca tat ctc aaa gct cat gca tat ggc aat aat gcc acc act aat     1344
   Ala Ala Tyr Leu Lys Ala His Ala Tyr Gly Asn Asn Ala Thr Thr Asn
           435                 440                 445
   gat ctt tgg agc gct ctc agc aaa gcg tct aat cag gat gtt act agc     1392
   Asp Leu Trp Ser Ala Leu Ser Lys Ala Ser Asn Gln Asp Val Thr Ser
       450                 455                 460
   ttt atg gat ccc tgg att cgc aag att ggc ttc cct gtt gtc acc gta     1440
   Phe Met Asp Pro Trp Ile Arg Lys Ile Gly Phe Pro Val Val Thr Val
   465                 470                 475                 480
   acc gag caa gcc ggc caa ctc agt gtt cgt caa agc cgc ttc ttg tcg       1488
   Thr Glu Gln Ala Gly Gln Leu Ser Val Arg Gln Ser Arg Phe Leu Ser
                   485                 490                 495
   acg ggt gat gtt aag ccc gag gaa gat gag acg gcc tgg tgg att ccc       1536
   Thr Gly Asp Val Lys Pro Glu Glu Asp Glu Thr Ala Trp Trp Ile Pro
               500                 505                 510
   ctt ggt gta aaa tca gga cct aag atg gcc gac gta aaa ccc ggt gct       1584
   Leu Gly Val Lys Ser Gly Pro Lys Met Ala Asp Val Lys Pro Gly Ala
           515                 520                 525
   ctt gtt tcc aag gaa gat aca att tgg ggg ctc gga caa gat tcc tat       1632
   Leu Val Ser Lys Glu Asp Thr Ile Trp Gly Leu Gly Gln Asp Ser Tyr
       530                 535                 540
   tac aag ctg aac aaa gac ctg tct ggc ttt tac cga acc aat tac cct       1680
   Tyr Lys Leu Asn Lys Asp Leu Ser Gly Phe Tyr Arg Thr Asn Tyr Pro
   545                 550                 555                 560
   gct gat cgg ctg gcg aaa ctc gct cag tct ctg gaa ctg ctg agc acc       1728
   Ala Asp Arg Leu Ala Lys Leu Ala Gln Ser Leu Glu Leu Leu Ser Thr
                   565                 570                 575
   gag gat aag att gga ttg att ggc gat gcc gct gcc cta gct gtt tct       1776
   Glu Asp Lys Ile Gly Leu Ile Gly Asp Ala Ala Ala Leu Ala Val Ser
               580                 585                 590
   ggc gac gga tcc act gct gct cta ttg gct ctc tta gag ggt ttc aag       1824
   Gly Asp Gly Ser Thr Ala Ala Leu Leu Ala Leu Leu Glu Gly Phe Lys
           595                 600                 605
   ggc gag aag aat tat ttg gtc tgg tcc caa atc tct tcc act att gcg       1872
   Gly Glu Lys Asn Tyr Leu Val Trp Ser Gln Ile Ser Ser Thr Ile Ala
       610                 615                 620
   aac tta cgc tct gtg ttc gcc ctg aat gag tcg gta gca gca gga ctc       1920
   Asn Leu Arg Ser Val Phe Ala Leu Asn Glu Ser Val Ala Ala Gly Leu
   625                 630                 635                 640
   aag aag ttt gcc ctc gag ctt tca tcc cct gct gct aac aaa atc ggc       1968
   Lys Lys Phe Ala Leu Glu Leu Ser Ser Pro Ala Ala Asn Lys Ile Gly
                   645                 650                 655
   tgg gaa ttt agc tcg gag gat gac tat ctc act atc cag ctc cgt aag       2016
   Trp Glu Phe Ser Ser Glu Asp Asp Tyr Leu Thr Ile Gln Leu Arg Lys
               660                 665                 670
   ctt cta atc gga atg gct ggc cgt gcg ggc cac aac gac ata ata tcc       2064
   Leu Leu Ile Gly Met Ala Gly Arg Ala Gly His Asn Asp Ile Ile Ser
           675                 680                 685
   gag gcc gaa cga cga ttt gag ctt tgg aaa tca ggc agc gat aag gat       2112
   Glu Ala Glu Arg Arg Phe Glu Leu Trp Lys Ser Gly Ser Asp Lys Asp
       690                 695                 700
   gct gtg cat acc aac ctc cgc tca gtg atc ttt agt atc gtg att tca       2160
   Ala Val His Thr Asn Leu Arg Ser Val Ile Phe Ser Ile Val Ile Ser
   705                 710                 715                 720
   gag ggc ggt cgt gaa gaa tac aat gct gta aag caa gag tat ctc aag       2208
   Glu Gly Gly Arg Glu Glu Tyr Asn Ala Val Lys Gln Glu Tyr Leu Lys
                   725                 730                 735
   acc gac tcc gtt gac ggc aag gag att tgc tta gga gct ctt gga cgt       2256
   Thr Asp Ser Val Asp Gly Lys Glu Ile Cys Leu Gly Ala Leu Gly Arg
               740                 745                 750
   act aaa gat gct gag ctg gta aag gat tac tta gat ttt gtt ttc tca       2304
   Thr Lys Asp Ala Glu Leu Val Lys Asp Tyr Leu Asp Phe Val Phe Ser
           755                 760                 765
   gac aag gtt gcc atc cag gat att cac aat ggc gcg gct tca atg gct       2352
   Asp Lys Val Ala Ile Gln Asp Ile His Asn Gly Ala Ala Ser Met Ala
       770                 775                 780
   acg aat ccc tcg act cgc cac ctt cta tgg gat tat atg aaa gag aat       2400
   Thr Asn Pro Ser Thr Arg His Leu Leu Trp Asp Tyr Met Lys Glu Asn
   785                 790                 795                 800
   tgg gcg gcc gtt gaa act cgc ttg tca gcg aac aat gtc gtt ttc gag       2448
   Trp Ala Ala Val Glu Thr Arg Leu Ser Ala Asn Asn Val Val Phe Glu
                   805                 810                 815
   cgg ttt gtg cgt atg gga ctg tcc aaa ttc gcg aac cac gat atc gca       2496
   Arg Phe Val Arg Met Gly Leu Ser Lys Phe Ala Asn His Asp Ile Ala
               820                 825                 830
   gcc gat att gct tcg ttt ttc cgg gag aag gac act ggt gcg tac gat       2544
   Ala Asp Ile Ala Ser Phe Phe Arg Glu Lys Asp Thr Gly Ala Tyr Asp
           835                 840                 845
   cgc gcc ctg gta atc gtt gct gat agc atc cga acg aat gca cgc tac       2592
   Arg Ala Leu Val Ile Val Ala Asp Ser Ile Arg Thr Asn Ala Arg Tyr
       850                 855                 860
   aag gaa aga gat gag aag cag gtc tta gag tgg ctt cgg ggc cat ggc       2640
   Lys Glu Arg Asp Glu Lys Gln Val Leu Glu Trp Leu Arg Gly His Gly
   865                 870                 875                 880
   tac gct tga                                                           2649
   Tyr Ala
<210> 91
   <211> 882
   <212> PRT
   <213> Aspergillus niger
<400> 91
   Met Ala Ser Lys Asp Arg Asp Ile Leu Pro Asp Val Val Lys Pro Val
   1               5                   10                  15
   His Tyr Asn Val Ser Leu Phe Asp Leu Gln Phe Gly Gly Ser Trp Gly
               20                  25                  30
   Tyr Lys Gly Thr Val Lys Ile Asp Ser Lys Val Asn Arg Pro Thr Lys
           35                  40                  45
   Glu Ile Val Leu Asn Ser Lys Glu Ile Glu Val Gln Asp Ala Glu Val
       50                  55                  60
   Phe Gly Asn Asp Gly Thr Lys Leu Ala Lys Ala Ser Asn Ile Ala Tyr
   65 70 75 80
   Asp Thr Lys Ser Glu Arg Val Thr Phe Thr Phe Ala Glu Glu Ile Leu
   85 90 95
   Pro Ala Asp Val Val Leu Ser Ile Asn Phe Thr Gly Ile Met Asn Asn
   100 105 110
   Ala Met Ala Gly Phe Ser Arg Ser Lys Tyr Lys Pro Val Val Asp Pro
   115 120 125
   Thr Asp Asp Thr Pro Lys Asp Gly Asp Ser Tyr Tyr Met Leu Ser Thr
   130 135 140
   Gln Phe Glu Ser Cys Asp Ala Arg Arg Ala Phe Pro Cys Phe Asp Glu
   145 150 155 160
   Pro Asn Leu Lys Ala Thr Phe Asp Phe Glu Ile Glu Val Pro Arg Gly
   165 170 175
   Gln Thr Ala Leu Ser Asn Met Pro Ile Lys Ser Glu Arg Ser Gly Ser
   180 185 190
   Arg Pro Glu Leu Lys Leu Val Ser Phe Glu Thr Thr Pro Val Met Ser
   195 200 205
   Thr Tyr Leu Leu Ala Trp Ala Val Gly Asp Phe Glu Tyr Val Glu Ala
   210 215 220
   Met Thr Gln Arg Lys Tyr Gln Gly Lys Ser Ile Pro Val Arg Val Tyr
   225 230 235 240
   Thr Thr Lys Gly Leu Lys Glu Gln Ala Arg Phe Ala Leu Glu Cys Ala
   245 250 255
   His Arg Thr Val Asp Tyr Phe Ser Glu Ile Phe Glu Ile Glu Tyr Pro
   260 265 270
   Leu Pro Lys Ala Asp Leu Leu Ala Val His Glu Phe Ala Met Gly Ala
   275 280 285
   Met Glu Asn Trp Gly Leu Val Thr Tyr Arg Thr Thr Ala Val Leu Phe
   290 295 300
   Asp Glu Gly Lys Ser Asp Thr Arg Tyr Lys Asn Arg Ile Ala Tyr Val
   305 310 315 320
   Val Ala His Glu Leu Ala His Gln Trp Phe Gly Asn Leu Val Thr Met
   325 330 335
   Asp Trp Trp Asn Glu Leu Trp Leu Asn Glu Gly Phe Ala Thr Trp Val
   340 345 350
   Gly Trp Leu Ala Val Asp His Phe Tyr Pro Glu Trp Asn Ile Trp Ser
   355 360 365
   Gln Phe Val Ala Glu Ser Val Gln Gln Ala Phe Gln Leu Asp Ser Leu
   370 375 380
   Arg Ala Ser His Pro Ile Glu Val Pro Val Arg Asn Ala Leu Glu Val
   385 390 395 400
   Asp Gln Ile Phe Asp His Ile Ser Tyr Leu Lys Gly Ser Ser Val Ile
   405 410 415
   Arg Met Leu Ser Asp His Leu Gly Arg Glu Thr Phe Leu Arg Gly Val
   420 425 430
   Ala Ala Tyr Leu Lys Ala His Ala Tyr Gly Asn Asn Ala Thr Thr Asn
   435 440 445
   Asp Leu Trp Ser Ala Leu Ser Lys Ala Ser Asn Gln Asp Val Thr Ser
   450 455 460
   Phe Met Asp Pro Trp Ile Arg Lys Ile Gly Phe Pro Val Val Thr Val
   465 470 475 480
   Thr Glu Gln Ala Gly Gln Leu Ser Val Arg Gln Ser Arg Phe Leu Ser
   485 490 495
   Thr Gly Asp Val Lys Pro Glu Glu Asp Glu Thr Ala Trp Trp Ile Pro
   500 505 510
   Leu Gly Val Lys Ser Gly Pro Lys Met Ala Asp Val Lys Pro Gly Ala
   515 520 525
   Leu Val Ser Lys Glu Asp Thr Ile Trp Gly Leu Gly Gln Asp Ser Tyr
   530 535 540
   Tyr Lys Leu Asn Lys Asp Leu Ser Gly Phe Tyr Arg Thr Asn Tyr Pro
   545 550 555 560
   Ala Asp Arg Leu Ala Lys Leu Ala Gln Ser Leu Glu Leu Leu Ser Thr
   565 570 575
   Glu Asp Lys Ile Gly Leu Ile Gly Asp Ala Ala Ala Leu Ala Val Ser
   580 585 590
   Gly Asp Gly Ser Thr Ala Ala Leu Leu Ala Leu Leu Glu Gly Phe Lys
   595 600 605
   Gly Glu Lys Asn Tyr Leu Val Trp Ser Gln Ile Ser Ser Thr Ile Ala
   610 615 620
   Asn Leu Arg Ser Val Phe Ala Leu Asn Glu Ser Val Ala Ala Gly Leu
   625 630 635 640
   Lys Lys Phe Ala Leu Glu Leu Ser Ser Pro Ala Ala Asn Lys Ile Gly
   645 650 655
   Trp Glu Phe Ser Ser Glu Asp Asp Tyr Leu Thr Ile Gln Leu Arg Lys
   660 665 670
   Leu Leu Ile Gly Met Ala Gly Arg Ala Gly His Asn Asp Ile Ile Ser
   675 680 685
   Glu Ala Glu Arg Arg Phe Glu Leu Trp Lys Ser Gly Ser Asp Lys Asp
   690 695 700
   Ala Val His Thr Asn Leu Arg Ser Val Ile Phe Ser Ile Val Ile Ser
   705 710 715 720
   Glu Gly Gly Arg Glu Glu Tyr Asn Ala Val Lys Gln Glu Tyr Leu Lys
   725 730 735
   Thr Asp Ser Val Asp Gly Lys Glu Ile Cys Leu Gly Ala Leu Gly Arg
   740 745 750
   Thr Lys Asp Ala Glu Leu Val Lys Asp Tyr Leu Asp Phe Val Phe Ser
   755 760 765
   Asp Lys Val Ala Ile Gln Asp Ile His Asn Gly Ala Ala Ser Met Ala
   770 775 780
   Thr Asn Pro Ser Thr Arg His Leu Leu Trp Asp Tyr Met Lys Glu Asn
   785 790 795 800
   Trp Ala Ala Val Glu Thr Arg Leu Ser Ala Asn Asn Val Val Phe Glu
   805 810 815
   Arg Phe Val Arg Met Gly Leu Ser Lys Phe Ala Asn His Asp Ile Ala
   820 825 830
   Ala Asp Ile Ala Ser Phe Phe Arg Glu Lys Asp Thr Gly Ala Tyr Asp
   835 840 845
   Arg Ala Leu Val Ile Val Ala Asp Ser Ile Arg Thr Asn Ala Arg Tyr
   850 855 860
   Lys Glu Arg Asp Glu Lys Gln Val Leu Glu Trp Leu Arg Gly His Gly
   865 870 875 880
   Tyr Ala
<210> 92
   <211> 4339
   <212> DNA
   <213> Aspergillus niger
<400> 92
   ttgcccatta tgtctgtgcc gaccatgcag cgtgaaacgg ccttccaggt ccggtctctg 60
   gtatgcccac cctgctgaaa ctagattgcg cgaaatccct cctacccttg tatcgcagtg 120
   gcattggggc taaccagtct atctcatgcg gacagttccg ctctttgctc cgtcaatcct 180
   cccagttctc gaactacaac ttccgcgagt atgcccgccg gcgcacgttg gatgccttcc 240
   gcgagcacca gaaggaatcc gaggaccgac ggatacagga gttgatccag gatggactac 300
   agaacttgcg gatgatgaag gtcagtaaat attctcggat ggacatgatt agatgtgccc 360
   acgtttgcct ctactacgca ggctgtgggc tctgagtcaa agcgggggtt gacgctagtg 420
   aacgagctgc taatactttt gcagaggcaa accgttatca gtcagttcta ccagctagat 480
   cggttggttg ttgagggtca gaagactgtg agtatccggg gaacctgtct gtgactgcca 540
   tgaagctaaa atcaatcgat agggtaagca gactggcacg gaagggaaca ttgttcgcca 600
   gaaggatact gggtaagatg cacccttcgt tatttgaatt ttttgggcca tgctgacgtg 660
   taaatagttg ggactaaacg cccaacgggg ttcatttgtt tttttcgcag cgagagtacg 720
   gagttgaggg gaatccagca ccaagacgat atcaatatca tttggggcca tttgagccga 780
   tggcccgaag atgaaggaat agcatagatc gaaggggttc tgtattttta ttgaaagcca 840
   ctgcgcttta ggtagcgatt cttactctga ctcatctgga atactattct tcgctctcca 900
   ttagtccaat ccatcccatc tgaaaccgta gtgggtggta gttatcatgt ttgttcgata 960
   ctatgagagt tctgagtgac cccagtcacg tgcaagggcg gcggcgcggc gtttcgctga 1020
   gcgatcgcat cgcgctgctc cgatcaacca tcacctccaa acagcctcgc cctccagaaa 1080
   gagtcccttt cttttcaatc gggcaaccac acccagctcc gtagatccac tgggtgaggg 1140
   gctctctgcc gttatcctga ttattctccg aagagtgaga ctgcaggtcc ggcgctcatc 1200
   atgccaccta ctgtaagctt ctcgccgcga gccacgaccc cgaccccgtt atcgatcaac 1260
   tcaccattgc accgaagctt gcccatgcgg tatagaacga tgccatcgct gatatgtact 1320
   gacaatcatc cagtttagat cctctcgctc tggtcgccaa ttcggcgatg gcgccaaccg 1380
   taacagaagt cagatcgatc acgatgtttt cgaggggctc cctgtacgac gctggacacg 1440
   tcaaactcag accgtctcgc aagtccccaa ggccgaaggc acagaagctg tcgtgcaggg 1500
   tccggggggg aagtcaacca tcccagaaca cccaatgccc cgagacagtc acctgttgag 1560
   tcccatgagc agagccctcc tgcgagctgc gcgagccgga tgcatctaca tccgccaagc 1620
   gtctaaagac tccgaagacg aagagaaaga agcgactgat ggagaggaac agccacttat 1680
   ccagagcacc gaacgcagtt tcgtcacgcg caaatggtcc gcagtaccga gacacctaga 1740
   atcatcagaa gttgaattcc tcgccaaacg acgaccgggt ttaccatctc tatacggtgc 1800
   tgctgctggg tctacggatg gaaataatgc acctgcgccg atgaggagga ccaagttcaa 1860
   gaaggtcgat ccggcgaccg gaaatatctc gatttatgaa gcctgggtac cggagggaca 1920
   caagattgag ggggagatca ctgatgggtc tcaagtttca gctgaaaaca gcgcggtcac 1980
   tgtgactcct gaggcgccag cgcccggtac ggtcattgag ggagttgggg ttgtcaacgc 2040
   cgagggtgtg gtagtcgcag atacaaactc cgcagctgtc atgactcctc cgaaaagacg 2100
   gcctcctccc ccaaagcgca aggcgaaggg ctttggaaag ggacggagaa agaaggtcat 2160
   gtttgctcct ggcgatggcg cggatgccgc cacggtccat ggttccaatg gctccggagt 2220
   ggatggcgct gcagaatatg ccaaggacgg ggatgcgtcc cgcatgtctg gtgaccagaa 2280
   tggacaagaa gaggacgatg aggagggcga ggagggcgat gagagcgacg agggtgatga 2340
   gtctatgcta gaggccaaga ctccggaaac tccagttcag cgtcccagca ctgagccgcc 2400
   caccgactcg acgcctgtgc ccgcaccggc cccggcaact gaaccagctg ccaaagctga 2460
   ttccacagag gcggcgttgc caacccctga gcttccttca gctgctccag aaccggctcc 2520
   agcaccggct ccagagacag ttgcagagcc agctgcccca ctccctcagc cagatcaacc 2580
   atcagtcgct gctgaacctc cttcacaagc tgctgctatc cagcccacac cagaggctcc 2640
   cgagcagacg agtgaggcat caggacagaa agaagatgtg gagatgaccg aagcttaccc 2700
   ctcaatacct ggtcctgagg aaagtgtatc tgcaacccct cagtcagttc cagctactca 2760
   gcaacaggag cccactcctg gcgctgaaga tgttccgacc ttaccatccc aggaagcccc 2820
   ccaacctccc gttgcggaag catcgtcagt cgtgccaacc tcagagcaac ctgtcgataa 2880
   agagcctgta gatgagccag tgcagccaga taagacaact actgatactg ctcagccgcc 2940
   ggcacagagt gaccagatta tggatacgac catggaggat gttgaacctc tccaacaaac 3000
   cgcacaggat caagcggcgg ttccaccgcc ggcggaacaa ccgatacccg agcaagcagc 3060
   tcagactagt aatgttgtcg cagagaacaa ggaagttgac ctcttgggta ctttggaagc 3120
   gagtctgagc cagccgccgg gcgaggaaca gggtgacaac cgagggacag acattgagat 3180
   ggaaatggat gcaccttccc acattgagca gcctgctgct acggaaacag ctgagcctct 3240
   tgcggataag ccaggggagc ttgagacgca agcagcagag gatactaccg tggctgcaga 3300
   aactgccgtt gaaccagctg cagtgcccgc tgaaccactc gacgaacagc acaccgaaaa 3360
   accgtccgag gcacctgcac cgcagcctat cgaacaactc cccgaagctc cagcagagca 3420
   agtggaagag ccggcaggcg agccggtcat ggagcagcca gtgaagtcgg aatcacctgc 3480
   tcagccagag caacctacgc aattagagcc acctgttcag ccagaacagc ctatgcagga 3540
   agagcctgta caggcagttg aacctgtgca gtcagagcag ccagagcaac ctaagccacc 3600
   tgttcagccg gaacagcctg tgcaggcgga gcctgtagag ccagcggagt ctgcgcagcc 3660
   agagcaaccg gagcaaccag aagggactca gcaatacgag cagcctgcgc aaccattgca 3720
   gcaagagcaa ccccaagagc aagccccatc acaagcttct gaggaagttc cagcccagtc 3780
   tgcagagccg acaccccaga ctgcaaccga gcagcagccc gagcagaccg aacagacaca 3840
   gaccgagcag gcgcctgctg agctaccgtc agagccacaa gcagatgaac caccaaaagc 3900
   actagaggag catgttcccg ttaacgcaga ggaaccagcc gctcccaagg tagaagaaca 3960
   aggaacaccc ctcgtcccac ctcaacaacc agaggaaccc cagccaccca aagaacctac 4020
   gccgcccgca ttacctgcta cagaggcacc acagcaacag ccagagccag taccggcgcc 4080
   acagccccca gctgaagaac ctcaagctga agaaaaacca acccaagaag gtccagctga 4140
   ggctgaatcc aaggagccgg agcctatctc cgaagccgaa cccaccccag ccgaagcccc 4200
   ctctgcacct cccatctctg cgcctgagcc tgagcccgag cccacccccg agcaaccagc 4260
   ccaggagacg accaaacccg aggaggcaga gcagcaggag caacaacaac aaccaccagc 4320
   tgataccaag acagcatga 4339
<210> 93
   <211> 3018
   <212> DNA
   <213> Aspergillus niger
<220>
   <221> CDS
   <222> (1)..(3018)
<400> 93
   atg cca cct act ttt aga tcc tct cgc tct ggt cgc caa ttc ggc gat          48
   Met Pro Pro Thr Phe Arg Ser Ser Arg Ser Gly Arg Gln Phe Gly Asp
   1               5                   10                  15
   ggc gcc aac cgt aac aga agt cag atc gat cac gat gtt ttc gag ggg          96
   Gly Ala Asn Arg Asn Arg Ser Gln Ile Asp His Asp Val Phe Glu Gly
               20                  25                  30
   ctc cct gta cga cgc tgg aca cgt caa act cag acc gtc tcg caa gtc         144
   Leu Pro Val Arg Arg Trp Thr Arg Gln Thr Gln Thr Val Ser Gln Val
           35                  40                  45
   ccc aag gcc gaa ggc aca gaa gct gtc gtg cag ggt ccg ggg ggg aag         192
   Pro Lys Ala Glu Gly Thr Glu Ala Val Val Gln Gly Pro Gly Gly Lys
       50                  55                  60
   tca acc atc cca gaa cac cca atg ccc cga gac agt cac ctg ttg agt         240
   Ser Thr Ile Pro Glu His Pro Met Pro Arg Asp Ser His Leu Leu Ser
   65                  70                  75                  80
   ccc atg agc aga gcc ctc ctg cga gct gcg cga gcc gga tgc atc tac         288
   Pro Met Ser Arg Ala Leu Leu Arg Ala Ala Arg Ala Gly Cys Ile Tyr
                   85                  90                  95
   atc cgc caa gcg tct aaa gac tcc gaa gac gaa gag aaa gaa gcg act         336
   Ile Arg Gln Ala Ser Lys Asp Ser Glu Asp Glu Glu Lys Glu Ala Thr
               100                 105                 110
   gat gga gag gaa cag cca ctt atc cag agc acc gaa cgc agt ttc gtc         384
   Asp Gly Glu Glu Gln Pro Leu Ile Gln Ser Thr Glu Arg Ser Phe Val
           115                 120                 125
   acg cgc aaa tgg tcc gca gta ccg aga cac cta gaa tca tca gaa gtt         432
   Thr Arg Lys Trp Ser Ala Val Pro Arg His Leu Glu Ser Ser Glu Val
       130                 135                 140
   gaa ttc ctc gcc aaa cga cga ccg ggt tta cca tct cta tac ggt gct         480
   Glu Phe Leu Ala Lys Arg Arg Pro Gly Leu Pro Ser Leu Tyr Gly Ala
   145                 150                 155                 160
   gct gct ggg tct acg gat gga aat aat gca cct gcg ccg atg agg agg         528
   Ala Ala Gly Ser Thr Asp Gly Asn Asn Ala Pro Ala Pro Met Arg Arg
                   165                 170                 175
   acc aag ttc aag aag gtc gat ccg gcg acc gga aat atc tcg att tat         576
   Thr Lys Phe Lys Lys Val Asp Pro Ala Thr Gly Asn Ile Ser Ile Tyr
               180                 185                 190
   gaa gcc tgg gta ccg gag gga cac aag att gag ggg gag atc act gat         624
   Glu Ala Trp Val Pro Glu Gly His Lys Ile Glu Gly Glu Ile Thr Asp
           195                 200                 205
   ggg tct caa gtt tca gct gaa aac agc gcg gtc act gtg act cct gag         672
   Gly Ser Gln Val Ser Ala Glu Asn Ser Ala Val Thr Val Thr Pro Glu
       210                 215                 220
   gcg cca gcg ccc ggt acg gtc att gag gga gtt ggg gtt gtc aac gcc         720
   Ala Pro Ala Pro Gly Thr Val Ile Glu Gly Val Gly Val Val Asn Ala
   225                 230                 235                 240
   gag ggt gtg gta gtc gca gat aca aac tcc gca gct gtc atg act cct         768
   Glu Gly Val Val Val Ala Asp Thr Asn Ser Ala Ala Val Met Thr Pro
                   245                 250                 255
   ccg aaa aga cgg cct cct ccc cca aag cgc aag gcg aag ggc ttt gga         816
   Pro Lys Arg Arg Pro Pro Pro Pro Lys Arg Lys Ala Lys Gly Phe Gly
               260                 265                 270
   aag gga cgg aga aag aag gtc atg ttt gct cct ggc gat ggc gcg gat         864
   Lys Gly Arg Arg Lys Lys Val Met Phe Ala Pro Gly Asp Gly Ala Asp
           275                 280                 285
   gcc gcc acg gtc cat ggt tcc aat ggc tcc gga gtg gat ggc gct gca         912
   Ala Ala Thr Val His Gly Ser Asn Gly Ser Gly Val Asp Gly Ala Ala
       290                 295                 300
   gaa tat gcc aag gac ggg gat gcg tcc cgc atg tct ggt gac cag aat         960
   Glu Tyr Ala Lys Asp Gly Asp Ala Ser Arg Met Ser Gly Asp Gln Asn
   305                 310                 315                 320
   gga caa gaa gag gac gat gag gag ggc gag gag ggc gat gag agc gac        1008
   Gly Gln Glu Glu Asp Asp Glu Glu Gly Glu Glu Gly Asp Glu Ser Asp
                   325                 330                 335
   gag ggt gat gag tct atg cta gag gcc aag act ccg gaa act cca gtt      1056
   Glu Gly Asp Glu Ser Met Leu Glu Ala Lys Thr Pro Glu Thr Pro Val
               340                 345                 350
   cag cgt ccc agc act gag ccg ccc acc gac tcg acg cct gtg ccc gca      1104
   Gln Arg Pro Ser Thr Glu Pro Pro Thr Asp Ser Thr Pro Val Pro Ala
           355                 360                 365
   ccg gcc ccg gca act gaa cca gct gcc aaa gct gat tcc aca gag gcg      1152
   Pro Ala Pro Ala Thr Glu Pro Ala Ala Lys Ala Asp Ser Thr Glu Ala
       370                 375                 380
   gcg ttg cca acc cct gag ctt cct tca gct gct cca gaa ccg gct cca      1200
   Ala Leu Pro Thr Pro Glu Leu Pro Ser Ala Ala Pro Glu Pro Ala Pro
   385                 390                 395                 400
   gca ccg gct cca gag aca gtt gca gag cca gct gcc cca ctc cct cag      1248
   Ala Pro Ala Pro Glu Thr Val Ala Glu Pro Ala Ala Pro Leu Pro Gln
                   405                 410                 415
   cca gat caa cca tca gtc gct gct gaa cct cct tca caa gct gct gct      1296
   Pro Asp Gln Pro Ser Val Ala Ala Glu Pro Pro Ser Gln Ala Ala Ala
               420                 425                 430
   atc cag ccc aca cca gag gct ccc gag cag acg agt gag gca tca gga      1344
   Ile Gln Pro Thr Pro Glu Ala Pro Glu Gln Thr Ser Glu Ala Ser Gly
           435                 440                 445
   cag aaa gaa gat gtg gag atg acc gaa gct tac ccc tca ata cct ggt      1392
   Gln Lys Glu Asp Val Glu Met Thr Glu Ala Tyr Pro Ser Ile Pro Gly
       450                 455                 460
   cct gag gaa agt gta tct gca acc cct cag tca gtt cca gct act cag      1440
   Pro Glu Glu Ser Val Ser Ala Thr Pro Gln Ser Val Pro Ala Thr Gln
   465                 470                 475                 480
   caa cag gag ccc act cct ggc gct gaa gat gtt ccg acc tta cca tcc      1488
   Gln Gln Glu Pro Thr Pro Gly Ala Glu Asp Val Pro Thr Leu Pro Ser
                   485                 490                 495
   cag gaa gcc ccc caa cct ccc gtt gcg gaa gca tcg tca gtc gtg cca      1536
   Gln Glu Ala Pro Gln Pro Pro Val Ala Glu Ala Ser Ser Val Val Pro
               500                 505                 510
   acc tca gag caa cct gtc gat aaa gag cct gta gat gag cca gtg cag      1584
   Thr Ser Glu Gln Pro Val Asp Lys Glu Pro Val Asp Glu Pro Val Gln
           515                 520                 525
   cca gat aag aca act act gat act gct cag ccg ccg gca cag agt gac      1632
   Pro Asp Lys Thr Thr Thr Asp Thr Ala Gln Pro Pro Ala Gln Ser Asp
       530                 535                 540
   cag att atg gat acg acc atg gag gat gtt gaa cct ctc caa caa acc      1680
   Gln Ile Met Asp Thr Thr Met Glu Asp Val Glu Pro Leu Gln Gln Thr
   545                 550                 555                 560
   gca cag gat caa gcg gcg gtt cca ccg ccg gcg gaa caa ccg ata ccc      1728
   Ala Gln Asp Gln Ala Ala Val Pro Pro Pro Ala Glu Gln Pro Ile Pro
                   565                 570                 575
   gag caa gca gct cag act agt aat gtt gtc gca gag aac aag gaa gtt      1776
   Glu Gln Ala Ala Gln Thr Ser Asn Val Val Ala Glu Asn Lys Glu Val
               580                 585                 590
   gac ctc ttg ggt act ttg gaa gcg agt ctg agc cag ccg ccg ggc gag      1824
   Asp Leu Leu Gly Thr Leu Glu Ala Ser Leu Ser Gln Pro Pro Gly Glu
           595                 600                 605
   gaa cag ggt gac aac cga ggg aca gac att gag atg gaa atg gat gca      1872
   Glu Gln Gly Asp Asn Arg Gly Thr Asp Ile Glu Met Glu Met Asp Ala
       610                 615                 620
   cct tcc cac att gag cag cct gct gct acg gaa aca gct gag cct ctt      1920
   Pro Ser His Ile Glu Gln Pro Ala Ala Thr Glu Thr Ala Glu Pro Leu
   625                 630                 635                 640
   gcg gat aag cca ggg gag ctt gag acg caa gca gca gag gat act acc      1968
   Ala Asp Lys Pro Gly Glu Leu Glu Thr Gln Ala Ala Glu Asp Thr Thr
                   645                 650                 655
   gtg gct gca gaa act gcc gtt gaa cca gct gca gtg ccc gct gaa cca      2016
   Val Ala Ala Glu Thr Ala Val Glu Pro Ala Ala Val Pro Ala Glu Pro
               660                 665                 670
   ctc gac gaa cag cac acc gaa aaa ccg tcc gag gca cct gca ccg cag      2064
   Leu Asp Glu Gln His Thr Glu Lys Pro Ser Glu Ala Pro Ala Pro Gln
           675                 680                 685
   cct atc gaa caa ctc ccc gaa gct cca gca gag caa gtg gaa gag ccg      2112
   Pro Ile Glu Gln Leu Pro Glu Ala Pro Ala Glu Gln Val Glu Glu Pro
       690                 695                 700
   gca ggc gag ccg gtc atg gag cag cca gtg aag tcg gaa tca cct gct      2160
   Ala Gly Glu Pro Val Met Glu Gln Pro Val Lys Ser Glu Ser Pro Ala
   705                 710                 715                 720
   cag cca gag caa cct acg caa tta gag cca cct gtt cag cca gaa cag      2208
   Gln Pro Glu Gln Pro Thr Gln Leu Glu Pro Pro Val Gln Pro Glu Gln
                   725                 730                 735
   cct atg cag gaa gag cct gta cag gca gtt gaa cct gtg cag tca gag      2256
   Pro Met Gln Glu Glu Pro Val Gln Ala Val Glu Pro Val Gln Ser Glu
               740                 745                 750
   cag cca gag caa cct aag cca cct gtt cag ccg gaa cag cct gtg cag      2304
   Gln Pro Glu Gln Pro Lys Pro Pro Val Gln Pro Glu Gln Pro Val Gln
           755                 760                 765
   gcg gag cct gta gag cca gcg gag tct gcg cag cca gag caa ccg gag      2352
   Ala Glu Pro Val Glu Pro Ala Glu Ser Ala Gln Pro Glu Gln Pro Glu
       770                 775                 780
   caa cca gaa ggg act cag caa tac gag cag cct gcg caa cca ttg cag      2400
   Gln Pro Glu Gly Thr Gln Gln Tyr Glu Gln Pro Ala Gln Pro Leu Gln
   785                 790                 795                 800
   caa gag caa ccc caa gag caa gcc cca tca caa gct tct gag gaa gtt      2448
   Gln Glu Gln Pro Gln Glu Gln Ala Pro Ser Gln Ala Ser Glu Glu Val
                   805                 810                 815
   cca gcc cag tct gca gag ccg aca ccc cag act gca acc gag cag cag      2496
   Pro Ala Gln Ser Ala Glu Pro Thr Pro Gln Thr Ala Thr Glu Gln Gln
               820                 825                 830
   ccc gag cag acc gaa cag aca cag acc gag cag gcg cct gct gag cta      2544
   Pro Glu Gln Thr Glu Gln Thr Gln Thr Glu Gln Ala Pro Ala Glu Leu
           835                 840                 845
   ccg tca gag cca caa gca gat gaa cca cca aaa gca cta gag gag cat      2592
   Pro Ser Glu Pro Gln Ala Asp Glu Pro Pro Lys Ala Leu Glu Glu His
       850                 855                 860
   gtt ccc gtt aac gca gag gaa cca gcc gct ccc aag gta gaa gaa caa      2640
   Val Pro Val Asn Ala Glu Glu Pro Ala Ala Pro Lys Val Glu Glu Gln
   865                 870                 875                 880
   gga aca ccc ctc gtc cca cct caa caa cca gag gaa ccc cag cca ccc      2688
   Gly Thr Pro Leu Val Pro Pro Gln Gln Pro Glu Glu Pro Gln Pro Pro
                   885                 890                 895
   aaa gaa cct acg ccg ccc gca tta cct gct aca gag gca cca cag caa      2736
   Lys Glu Pro Thr Pro Pro Ala Leu Pro Ala Thr Glu Ala Pro Gln Gln
               900                 905                 910
   cag cca gag cca gta ccg gcg cca cag ccc cca gct gaa gaa cct caa      2784
   Gln Pro Glu Pro Val Pro Ala Pro Gln Pro Pro Ala Glu Glu Pro Gln
           915                 920                 925
   gct gaa gaa aaa cca acc caa gaa ggt cca gct gag gct gaa tcc aag      2832
   Ala Glu Glu Lys Pro Thr Gln Glu Gly Pro Ala Glu Ala Glu Ser Lys
       930                 935                 940
   gag ccg gag cct atc tcc gaa gcc gaa ccc acc cca gcc gaa gcc ccc      2880
   Glu Pro Glu Pro Ile Ser Glu Ala Glu Pro Thr Pro Ala Glu Ala Pro
   945                 950                 955                 960
   tct gca cct ccc atc tct gcg cct gag cct gag ccc gag ccc acc ccc      2928
   Ser Ala Pro Pro Ile Ser Ala Pro Glu Pro Glu Pro Glu Pro Thr Pro
                   965                 970                 975
   gag caa cca gcc cag gag acg acc aaa ccc gag gag gca gag cag cag      2976
   Glu Gln Pro Ala Gln Glu Thr Thr Lys Pro Glu Glu Ala Glu Gln Gln
               980                 985                 990
   gag caa caa caa caa cca cca gct  gat acc aag aca gca  tga            3018
   Glu Gln Gln Gln Gln Pro Pro Ala  Asp Thr Lys Thr Ala
           995                 1000                 1005
<210> 94
   <211> 1005
   <212> PRT
   <213> Aspergillus niger
<400> 94
   Met Pro Pro Thr Phe Arg Ser Ser Arg Ser Gly Arg Gln Phe Gly Asp
   1               5                   10                  15
   Gly Ala Asn Arg Asn Arg Ser Gln Ile Asp His Asp Val Phe Glu Gly
               20                  25                  30
   Leu Pro Val Arg Arg Trp Thr Arg Gln Thr Gln Thr Val Ser Gln Val
           35                  40                  45
   Pro Lys Ala Glu Gly Thr Glu Ala Val Val Gln Gly Pro Gly Gly Lys
       50                  55                  60
   Ser Thr Ile Pro Glu His Pro Met Pro Arg Asp Ser His Leu Leu Ser
   65                  70                  75                  80
   Pro Met Ser Arg Ala Leu Leu Arg Ala Ala Arg Ala Gly Cys Ile Tyr
   85 90 95
   Ile Arg Gln Ala Ser Lys Asp Ser Glu Asp Glu Glu Lys Glu Ala Thr
   100 105 110
   Asp Gly Glu Glu Gln Pro Leu Ile Gln Ser Thr Glu Arg Ser Phe Val
   115 120 125
   Thr Arg Lys Trp Ser Ala Val Pro Arg His Leu Glu Ser Ser Glu Val
   130 135 140
   Glu Phe Leu Ala Lys Arg Arg Pro Gly Leu Pro Ser Leu Tyr Gly Ala
   145 150 155 160
   Ala Ala Gly Ser Thr Asp Gly Asn Asn Ala Pro Ala Pro Met Arg Arg
   165 170 175
   Thr Lys Phe Lys Lys Val Asp Pro Ala Thr Gly Asn Ile Ser Ile Tyr
   180 185 190
   Glu Ala Trp Val Pro Glu Gly His Lys Ile Glu Gly Glu Ile Thr Asp
   195 200 205
   Gly Ser Gln Val Ser Ala Glu Asn Ser Ala Val Thr Val Thr Pro Glu
   210 215 220
   Ala Pro Ala Pro Gly Thr Val Ile Glu Gly Val Gly Val Val Asn Ala
   225 230 235 240
   Glu Gly Val Val Val Ala Asp Thr Asn Ser Ala Ala Val Met Thr Pro
   245 250 255
   Pro Lys Arg Arg Pro Pro Pro Pro Lys Arg Lys Ala Lys Gly Phe Gly
   260 265 270
   Lys Gly Arg Arg Lys Lys Val Met Phe Ala Pro Gly Asp Gly Ala Asp
   275 280 285
   Ala Ala Thr Val His Gly Ser Asn Gly Ser Gly Val Asp Gly Ala Ala
   290 295 300
   Glu Tyr Ala Lys Asp Gly Asp Ala Ser Arg Met Ser Gly Asp Gln Asn
   305 310 315 320
   Gly Gln Glu Glu Asp Asp Glu Glu Gly Glu Glu Gly Asp Glu Ser Asp
   325 330 335
   Glu Gly Asp Glu Ser Met Leu Glu Ala Lys Thr Pro Glu Thr Pro Val
   340 345 350
   Gln Arg Pro Ser Thr Glu Pro Pro Thr Asp Ser Thr Pro Val Pro Ala
   355 360 365
   Pro Ala Pro Ala Thr Glu Pro Ala Ala Lys Ala Asp Ser Thr Glu Ala
   370 375 380
   Ala Leu Pro Thr Pro Glu Leu Pro Ser Ala Ala Pro Glu Pro Ala Pro
   385 390 395 400
   Ala Pro Ala Pro Glu Thr Val Ala Glu Pro Ala Ala Pro Leu Pro Gln
   405 410 415
   Pro Asp Gln Pro Ser Val Ala Ala Glu Pro Pro Ser Gln Ala Ala Ala
   420 425 430
   Ile Gln Pro Thr Pro Glu Ala Pro Glu Gln Thr Ser Glu Ala Ser Gly
   435 440 445
   Gln Lys Glu Asp Val Glu Met Thr Glu Ala Tyr Pro Ser Ile Pro Gly
   450 455 460
   Pro Glu Glu Ser Val Ser Ala Thr Pro Gln Ser Val Pro Ala Thr Gln
   465 470 475 480
   Gln Gln Glu Pro Thr Pro Gly Ala Glu Asp Val Pro Thr Leu Pro Ser
   485 490 495
   Gln Glu Ala Pro Gln Pro Pro Val Ala Glu Ala Ser Ser Val Val Pro
   500 505 510
   Thr Ser Glu Gln Pro Val Asp Lys Glu Pro Val Asp Glu Pro Val Gln
   515 520 525
   Pro Asp Lys Thr Thr Thr Asp Thr Ala Gln Pro Pro Ala Gln Ser Asp
   530 535 540
   Gln Ile Met Asp Thr Thr Met Glu Asp Val Glu Pro Leu Gln Gln Thr
   545 550 555 560
   Ala Gln Asp Gln Ala Ala Val Pro Pro Pro Ala Glu Gln Pro Ile Pro
   565 570 575
   Glu Gln Ala Ala Gln Thr Ser Asn Val Val Ala Glu Asn Lys Glu Val
   580 585 590
   Asp Leu Leu Gly Thr Leu Glu Ala Ser Leu Ser Gln Pro Pro Gly Glu
   595 600 605
   Glu Gln Gly Asp Asn Arg Gly Thr Asp Ile Glu Met Glu Met Asp Ala
   610 615 620
   Pro Ser His Ile Glu Gln Pro Ala Ala Thr Glu Thr Ala Glu Pro Leu
   625 630 635 640
   Ala Asp Lys Pro Gly Glu Leu Glu Thr Gln Ala Ala Glu Asp Thr Thr
   645 650 655
   Val Ala Ala Glu Thr Ala Val Glu Pro Ala Ala Val Pro Ala Glu Pro
   660 665 670
   Leu Asp Glu Gln His Thr Glu Lys Pro Ser Glu Ala Pro Ala Pro Gln
   675 680 685
   Pro Ile Glu Gln Leu Pro Glu Ala Pro Ala Glu Gln Val Glu Glu Pro
   690 695 700
   Ala Gly Glu Pro Val Met Glu Gln Pro Val Lys Ser Glu Ser Pro Ala
   705 710 715 720
   Gln Pro Glu Gln Pro Thr Gln Leu Glu Pro Pro Val Gln Pro Glu Gln
   725 730 735
   Pro Met Gln Glu Glu Pro Val Gln Ala Val Glu Pro Val Gln Ser Glu
   740 745 750
   Gln Pro Glu Gln Pro Lys Pro Pro Val Gln Pro Glu Gln Pro Val Gln
   755 760 765
   Ala Glu Pro Val Glu Pro Ala Glu Ser Ala Gln Pro Glu Gln Pro Glu
   770 775 780
   Gln Pro Glu Gly Thr Gln Gln Tyr Glu Gln Pro Ala Gln Pro Leu Gln
   785 790 795 800
   Gln Glu Gln Pro Gln Glu Gln Ala Pro Ser Gln Ala Ser Glu Glu Val
   805 810 815
   Pro Ala Gln Ser Ala Glu Pro Thr Pro Gln Thr Ala Thr Glu Gln Gln
   820 825 830
   Pro Glu Gln Thr Glu Gln Thr Gln Thr Glu Gln Ala Pro Ala Glu Leu
   835 840 845
   Pro Ser Glu Pro Gln Ala Asp Glu Pro Pro Lys Ala Leu Glu Glu His
   850 855 860
   Val Pro Val Asn Ala Glu Glu Pro Ala Ala Pro Lys Val Glu Glu Gln
   865 870 875 880
   Gly Thr Pro Leu Val Pro Pro Gln Gln Pro Glu Glu Pro Gln Pro Pro
   885 890 895
   Lys Glu Pro Thr Pro Pro Ala Leu Pro Ala Thr Glu Ala Pro Gln Gln
   900 905 910
   Gln Pro Glu Pro Val Pro Ala Pro Gln Pro Pro Ala Glu Glu Pro Gln
   915 920 925
   Ala Glu Glu Lys Pro Thr Gln Glu Gly Pro Ala Glu Ala Glu Ser Lys
   930 935 940
   Glu Pro Glu Pro Ile Ser Glu Ala Glu Pro Thr Pro Ala Glu Ala Pro
   945 950 955 960
   Ser Ala Pro Pro Ile Ser Ala Pro Glu Pro Glu Pro Glu Pro Thr Pro
   965 970 975
   Glu Gln Pro Ala Gln Glu Thr Thr Lys Pro Glu Glu Ala Glu Gln Gln
   980 985 990
   Glu Gln Gln Gln Gln Pro Pro Ala Asp Thr Lys Thr Ala
   995 1000 1005
<210> 95
   <211> 4556
   <212> DNA
   <213> Aspergillus niger
<400> 95
   atggagctgg agcgtagcga gaatgtcatc attgtcacgc accaggctgt tctccgctgc      60
   atctatgcct acttcctcaa cacgccgcag gagcaaagtc cttggatgga ggtgccgctc     120
   cacacgctaa tcaagctcac gccgcgcgca tacggcactg aggagcagcg gttcaaggca     180
   gacattcccg ccgtctcgac atggcgtgcg aagggcacgt cggccaaaca ccaggaatac     240
   cccacggaga tcaaggcatg atctgagagg cattcggacg actattatta ttatcctgaa     300
   tagtccgaaa cacaatcgac ctttccggtc atatagttat cctacctatc ggttttgcca     360
   ttctcatatt cccggtcggg aatcggctta cccttcacat tgtacatgcg ccgtgcgttt     420
   tctccattct ttggtcttga tatttgcaaa agtccggatt ccttgtcgct gcggtccgtc     480
   gggctgtgtt atgggttttg atcggtcggt tgggcagtta tggaattgat tcattgcatg     540
   ggatcggagc tttaagccag cggatggcac atccatggat gggatagtag cggtgtgtgc     600
   aggacatcca tccatcggtt agcttagcat aatagactag atactcaaga caacgttgat 660
   gtaatcatta agctacaaga catttcttaa taaatgacac gcaagggatg ctgtcacaag 720
   caaatacaga atgtagttgt agcacagaca gacggggaaa cccatcttgg cgcggcccgg 780
   gggagtgtgc agctgtccgg tccagctgga ctcctccgcc cgtagtacta ggagctccgc 840
   tatcaacctc cgcccaacca ccgcactgca ccgcagcacc acatcactca cctacctacc 900
   tacatacaac aacttccaca ccatgacacc cacctacacc ttctaactcc acctcgaaat 960
   taattgcaca gtaccatagc cacgacatga catgaaccta cccccgatcg cgaacggcgc 1020
   attacccact tacctcacac ctccttcccg agcatccatc ctctacgact taccgagcta 1080
   gctagctagc tagccagcac accgcactat accccaccga taatttgaat atatacacga 1140
   acatacaacc gcttattact tttccgaagg aaagatatta cgaaggcgaa gaagaagaag 1200
   atgctatccg cattcacagc ccgccccctg gtggaactca aaccgcgcga ccgctcacgg 1260
   atcgaatccg tgctggcgta tggggaccgc gtgctggcgg ggctgaataa tgggtcgttg 1320
   aggatatatc gcgtgaatga gcaggaagag gaaccagaag agggagaaga ggggggtgaa 1380
   ggacagcaac atgagcagca tggtcaggat acagggaagg gggggaagaa agataccgat 1440
   caacaaggga atggcaatgg agaggcaggg ataatcacga acggaactac gaacggcacc 1500
   cgggagcaca agaaacccac ggagctacta cgcgaagtgg agaagttctc acgatacaag 1560
   atcgagcagt tggcgctgat taaggaagcc aaggtgcttg tttctctatc tgggggatat 1620
   gtctcgctgc atgatctagg gacgtatgcg ttgcaggagc aattggggaa gacgaagggg 1680
   gcgacgacgt ttgcggtgac gtcgaatatc gagaatgatt ccgaaacggg agtgcctgct 1740
   atcgtgtcga gggtggcggt ggcggtgaag aggaagataa tgctgtgggt gtggcgggat 1800
   atggagatgg aagggggcgt gcctatggag atgacgcttg tcagtgggat taagacgctg 1860
   acgtgggtgt ctgggacgag gttggtggct gggttggggt cggggtttgt gatggttgat 1920
   attgaggggg gtagtggtgg gacggtgacg gatttgactg gtccgtctgg gatagggggg 1980
   ttagggggac aggagagtac ggggaggttg gccggggtgg gggtcgccag tatgagttat 2040
   atggggcttg ggggcagtgc gccgaagccg ttggcgacga ggttgaagga gggccaggtg 2100
   ttgctggcta aggatattaa cacgcatttt attgatgtgc agggaaattc gctggggagg 2160
   agacaaattc cgtggagtca tgcgccggcg gatattgggt actcgtatcc gttcttgttg 2220
   gcgctgcatg attcctcgaa gggagtgttg gaggtacgaa atccggagac gttgtcgctg 2280
   ttgcagtcgg ttccgttgcc atcagcaagt attatgcata tcccgcagcc aacgattagt 2340
   ctggcgcatg ctgggaaggg gttcttggtg gcgagtgatc ggacgatatg gcggatggag 2400
   gcgctgagtt atgatacgca gattgactcg ctggtcgaga aggggtatct ggacgaagcg 2460
   atcagtctgg ccagtatgct ggaggatgcg ctgctgaggg ataagcaggg ccggctgcgc 2520
   cagatcaagc tggagaaggc tgaagggctg ttcaagatgc gaaagtacac cgattcgatg 2580
   gatctgttta cggagatatc tgcgcctccg gagacggtga tacgactgta tccgaagatc 2640
   atcgccgggg agctgtcgtc gattgttgag gagccggagg agtcagaaga tggaacgaca 2700
   gacagccagc ccaagacgca ggagaacaac aacccgacgg acgcgccggc tacggaggaa 2760
   acgccagcgc cgaagacact ctcgcatgct ccgtctgtga tgtcactttt acggacgcgg 2820
   acagatgacg gcagtgatgc gggcagtatt cgtggcaagg ttgtcgagga ggccaagaac 2880
   gacaaggcgc tggaaggtgc cgatctcaag ctggctgttc gtgacctgca gagatatctg 2940
   gcagatgtgc ggaggcggtt ccagcgcttc ttgaacccgg atggcacgct aaaggtgatt 3000
   gatgcgacga cggacggcgc gaacgatgcc ttaacggact cggtcatgaa gctgctgagc 3060
   attgatccgg agggtgagta cgatctcggg gagaagctgc gggaaaaggc caggctcgtg 3120
   gacacgactc tcttccgagc gtacatgtac gctattccag ctctggcagg ctcgttgttc 3180
   cgcattgcca acttctgtga tccagatgtg gtgatggaaa aattggagga gacggggcgt 3240
   cacaacgatc tgattgactt tttgtacgga aagaagctgc accgacaagc gttggagctg 3300
   cttcagaagt tcggacaggc cgacgatgaa gaagagacgg cgccgcagct gcacgggccg 3360
   aagcgaacag tcaattatct acagaatctg tctccagacc atattgactt gatcctcgag 3420
   ttcgctgaat ggccagtacg acaggatcct gagctgggca tggagatatt cctggcagat 3480
   acggagaacg cggagacgct gccgcgggaa cgagtgctag acttcctgca gggcattgat 3540
   gtcaatctgg ccgtccgcta cctggagcat atcattgggg agttgaatga tatgacaccg 3600
   gatctgcatc agaaactgct catcctgtac ttgaaccggc tgaagaagca ccaggctaaa 3660
   gagtgggagt tctctagtct ggatgactat gttaattggc agagcaagtt cttgaatatg 3720
   ctcaggtcca gctctcagta ttctccggct aagattctgg atcgattgga ccgagacggt 3780
   gagtaccctc cttgcgctta cattgcgttg ctgacttgta gaaagatcct gaattcttcg 3840
   aggcacgagc gattgtcttc agcaagatgg ggcaacaccg gcaggcactg gagatttatg 3900
   tattcaagtt ggaagactac gtcaaggctg aagagtgagt tgcattgatc gatatacatg 3960
   agttctgctg accgttcaga tactgtaatc atctccataa agtggaggat acgactgctg 4020
   cggatggatc ggtttcgcgc tgcgtagctc tactaccata cgaagatgac aaaccatcga 4080
   tttatttgac tctattgtct ctatatctat ctccgccgca tggctacaag ccacggtacg 4140
   ggcccgcgct tgaggtgctg gccaaacatg gatcccggct gccacccaat tctgctctgg 4200
   atctgatccc agaatcgctc ccggtcaagg agctcgagtt ctatttcaag ggccgtatgc 4260
   gggccgccaa cacaatcctt aacgagagcc ggatcgtggc caaccttcaa aaggcagaag 4320
   acatcaagac acaggcgcag ttattggtag gagagggaac ggacgggcga tcgacgcgct 4380
   cacgacatgt caccatcacc gaagagcggg tctgcggcat ctgccacaag cggatcggag 4440
   gcagtgtgat taatgtgttt ccggagtaag tatggccaga ttctgttgac ttgagcatgg 4500
   ctaattgagg cagtaatacg gttgtccatc ttggctgtgc caatcgcatg cgttga 4556
<210> 96
   <211> 3216
   <212> DNA
   <213> Aspergillus niger
<220>
   <221> CDS
   <222> (1) .. (3216)
<400> 96
   atg cta tcc gca ttc aca gcc cgc ccc ctg gtg gaa ctc aaa ccg cgc      48
   Met Leu Ser Ala Phe Thr Ala Arg Pro Leu Val Glu Leu Lys Pro Arg
   1               5                   10                  15
   gac cgc tca cgg atc gaa tcc gtg ctg gcg tat ggg gac cgc gtg ctg      96
   Asp Arg Ser Arg Ile Glu Ser Val Leu Ala Tyr Gly Asp Arg Val Leu
               20                  25                  30
   gcg ggg ctg aat aat ggg tcg ttg agg ata tat cgc gtg aat gag cag      144
   Ala Gly Leu Asn Asn Gly Ser Leu Arg Ile Tyr Arg Val Asn Glu Gln
           35                  40                  45
   gaa gag gaa cca gaa gag gga gaa gag ggg ggt gaa gga cag caa cat      192
   Glu Glu Glu Pro Glu Glu Gly Glu Glu Gly Gly Glu Gly Gln Gln His
       50                  55                  60
   gag cag cat ggt cag gat aca ggg aag ggg ggg aag aaa gat acc gat      240
   Glu Gln His Gly Gln Asp Thr Gly Lys Gly Gly Lys Lys Asp Thr Asp
   65                  70                  75                  80
   caa caa ggg aat ggc aat gga gag gca ggg ata atc acg aac gga act      288
   Gln Gln Gly Asn Gly Asn Gly Glu Ala Gly Ile Ile Thr Asn Gly Thr
                   85                  90                  95
   acg aac ggc acc cgg gag cac aag aaa ccc acg gag cta cta cgc gaa      336
   Thr Asn Gly Thr Arg Glu His Lys Lys Pro Thr Glu Leu Leu Arg Glu
               100                 105                 110
   gtg gag aag ttc tca cga tac aag atc gag cag ttg gcg ctg att aag      384
   Val Glu Lys Phe Ser Arg Tyr Lys Ile Glu Gln Leu Ala Leu Ile Lys
           115                 120                 125
   gaa gcc aag gtg ctt gtt tct cta tct ggg gga tat gtc tcg ctg cat      432
   Glu Ala Lys Val Leu Val Ser Leu Ser Gly Gly Tyr Val Ser Leu His
       130                 135                 140
   gat cta ggg acg tat gcg ttg cag gag caa ttg ggg aag acg aag ggg      480
   Asp Leu Gly Thr Tyr Ala Leu Gln Glu Gln Leu Gly Lys Thr Lys Gly
   145                 150                 155                 160
   gcg acg acg ttt gcg gtg acg tcg aat atc gag aat gat tcc gaa acg      528
   Ala Thr Thr Phe Ala Val Thr Ser Asn Ile Glu Asn Asp Ser Glu Thr
                   165                 170                 175
   gga gtg cct gct atc gtg tcg agg gtg gcg gtg gcg gtg aag agg aag      576
   Gly Val Pro Ala Ile Val Ser Arg Val Ala Val Ala Val Lys Arg Lys
               180                 185                 190
   ata atg ctg tgg gtg tgg cgg gat atg gag atg gaa ggg ggc gtg cct      624
   Ile Met Leu Trp Val Trp Arg Asp Met Glu Met Glu Gly Gly Val Pro
           195                 200                 205
   atg gag atg acg ctt gtc agt ggg att aag acg ctg acg tgg gtg tct      672
   Met Glu Met Thr Leu Val Ser Gly Ile Lys Thr Leu Thr Trp Val Ser
       210                 215                 220
   ggg acg agg ttg gtg gct ggg ttg ggg tcg ggg ttt gtg atg gtt gat      720
   Gly Thr Arg Leu Val Ala Gly Leu Gly Ser Gly Phe Val Met Val Asp
   225                 230                 235                 240
   att gag ggg ggt agt ggt ggg acg gtg acg gat ttg act ggt ccg tct      768
   Ile Glu Gly Gly Ser Gly Gly Thr Val Thr Asp Leu Thr Gly Pro Ser
                   245                 250                 255
   ggg ata ggg ggg tta ggg gga cag gag agt acg ggg agg ttg gcc ggg      816
   Gly Ile Gly Gly Leu Gly Gly Gln Glu Ser Thr Gly Arg Leu Ala Gly
               260                 265                 270
   gtg ggg gtc gcc agt atg agt tat atg ggg ctt ggg ggc agt gcg ccg      864
   Val Gly Val Ala Ser Met Ser Tyr Met Gly Leu Gly Gly Ser Ala Pro
           275                 280                 285
   aag ccg ttg gcg acg agg ttg aag gag ggc cag gtg ttg ctg gct aag      912
   Lys Pro Leu Ala Thr Arg Leu Lys Glu Gly Gln Val Leu Leu Ala Lys
       290                 295                 300
   gat att aac acg cat ttt att gat gtg cag gga aat tcg ctg ggg agg      960
   Asp Ile Asn Thr His Phe Ile Asp Val Gln Gly Asn Ser Leu Gly Arg
   305                 310                 315                 320
   aga caa att ccg tgg agt cat gcg ccg gcg gat att ggg tac tcg tat     1008
   Arg Gln Ile Pro Trp Ser His Ala Pro Ala Asp Ile Gly Tyr Ser Tyr
                   325                 330                 335
   ccg ttc ttg ttg gcg ctg cat gat tcc tcg aag gga gtg ttg gag gta     1056
   Pro Phe Leu Leu Ala Leu His Asp Ser Ser Lys Gly Val Leu Glu Val
               340                 345                 350
   cga aat ccg gag acg ttg tcg ctg ttg cag tcg gtt ccg ttg cca tca     1104
   Arg Asn Pro Glu Thr Leu Ser Leu Leu Gln Ser Val Pro Leu Pro Ser
           355                 360                 365
   gca agt att atg cat atc ccg cag cca acg att agt ctg gcg cat gct     1152
   Ala Ser Ile Met His Ile Pro Gln Pro Thr Ile Ser Leu Ala His Ala
       370                 375                 380
   ggg aag ggg ttc ttg gtg gcg agt gat cgg acg ata tgg cgg atg gag     1200
   Gly Lys Gly Phe Leu Val Ala Ser Asp Arg Thr Ile Trp Arg Met Glu
   385                 390                 395                 400
   gcg ctg agt tat gat acg cag att gac tcg ctg gtc gag aag ggg tat     1248
   Ala Leu Ser Tyr Asp Thr Gln Ile Asp Ser Leu Val Glu Lys Gly Tyr
                   405                 410                 415
   ctg gac gaa gcg atc agt ctg gcc agt atg ctg gag gat gcg ctg ctg     1296
   Leu Asp Glu Ala Ile Ser Leu Ala Ser Met Leu Glu Asp Ala Leu Leu
               420                 425                 430
   agg gat aag cag ggc cgg ctg cgc cag atc aag ctg gag aag gct gaa     1344
   Arg Asp Lys Gln Gly Arg Leu Arg Gln Ile Lys Leu Glu Lys Ala Glu
           435                 440                 445
   ggg ctg ttc aag atg cga aag tac acc gat tcg atg gat ctg ttt acg     1392
   Gly Leu Phe Lys Met Arg Lys Tyr Thr Asp Ser Met Asp Leu Phe Thr
       450                 455                 460
   gag ata tct gcg cct ccg gag acg gtg ata cga ctg tat ccg aag atc      1440
   Glu Ile Ser Ala Pro Pro Glu Thr Val Ile Arg Leu Tyr Pro Lys Ile
   465                 470                 475                 480
   atc gcc ggg gag ctg tcg tcg att gtt gag gag ccg gag gag tca gaa      1488
   Ile Ala Gly Glu Leu Ser Ser Ile Val Glu Glu Pro Glu Glu Ser Glu
                   485                 490                 495
   gat gga acg aca gac agc cag ccc aag acg cag gag aac aac aac ccg      1536
   Asp Gly Thr Thr Asp Ser Gln Pro Lys Thr Gln Glu Asn Asn Asn Pro
               500                 505                 510
   acg gac gcg ccg gct acg gag gaa acg cca gcg ccg aag aca ctc tcg      1584
   Thr Asp Ala Pro Ala Thr Glu Glu Thr Pro Ala Pro Lys Thr Leu Ser
           515                 520                 525
   cat gct ccg tct gtg atg tca ctt tta cgg acg cgg aca gat gac ggc      1632
   His Ala Pro Ser Val Met Ser Leu Leu Arg Thr Arg Thr Asp Asp Gly
       530                 535                 540
   agt gat gcg ggc agt att cgt ggc aag gtt gtc gag gag gcc aag aac      1680
   Ser Asp Ala Gly Ser Ile Arg Gly Lys Val Val Glu Glu Ala Lys Asn
   545                 550                 555                 560
   gac aag gcg ctg gaa ggt gcc gat ctc aag ctg gct gtt cgt gac ctg      1728
   Asp Lys Ala Leu Glu Gly Ala Asp Leu Lys Leu Ala Val Arg Asp Leu
                   565                 570                 575
   cag aga tat ctg gca gat gtg cgg agg cgg ttc cag cgc ttc ttg aac      1776
   Gln Arg Tyr Leu Ala Asp Val Arg Arg Arg Phe Gln Arg Phe Leu Asn
               580                 585                 590
   ccg gat ggc acg cta aag gtg att gat gcg acg acg gac ggc gcg aac      1824
   Pro Asp Gly Thr Leu Lys Val Ile Asp Ala Thr Thr Asp Gly Ala Asn
           595                 600                 605
   gat gcc tta acg gac tcg gtc atg aag ctg ctg agc att gat ccg gag      1872
   Asp Ala Leu Thr Asp Ser Val Met Lys Leu Leu Ser Ile Asp Pro Glu
       610                 615                 620
   ggt gag tac gat ctc ggg gag aag ctg cgg gaa aag gcc agg ctc gtg      1920
   Gly Glu Tyr Asp Leu Gly Glu Lys Leu Arg Glu Lys Ala Arg Leu Val
   625                 630                 635                 640
   gac acg act ctc ttc cga gcg tac atg tac gct att cca gct ctg gca      1968
   Asp Thr Thr Leu Phe Arg Ala Tyr Met Tyr Ala Ile Pro Ala Leu Ala
                   645                 650                 655
   ggc tcg ttg ttc cgc att gcc aac ttc tgt gat cca gat gtg gtg atg      2016
   Gly Ser Leu Phe Arg Ile Ala Asn Phe Cys Asp Pro Asp Val Val Met
               660                 665                 670
   gaa aaa ttg gag gag acg ggg cgt cac aac gat ctg att gac ttt ttg      2064
   Glu Lys Leu Glu Glu Thr Gly Arg His Asn Asp Leu Ile Asp Phe Leu
           675                 680                 685
   tac gga aag aag ctg cac cga caa gcg ttg gag ctg ctt cag aag ttc      2112
   Tyr Gly Lys Lys Leu His Arg Gln Ala Leu Glu Leu Leu Gln Lys Phe
       690                 695                 700
   gga cag gcc gac gat gaa gaa gag acg gcg ccg cag ctg cac ggg ccg      2160
   Gly Gln Ala Asp Asp Glu Glu Glu Thr Ala Pro Gln Leu His Gly Pro
   705                 710                 715                 720
   aag cga aca gtc aat tat cta cag aat ctg tct cca gac cat att gac      2208
   Lys Arg Thr Val Asn Tyr Leu Gln Asn Leu Ser Pro Asp His Ile Asp
                   725                 730                 735
   ttg atc ctc gag ttc gct gaa tgg cca gta cga cag gat cct gag ctg       2256
   Leu Ile Leu Glu Phe Ala Glu Trp Pro Val Arg Gln Asp Pro Glu Leu
               740                 745                 750
   ggc atg gag ata ttc ctg gca gat acg gag aac gcg gag acg ctg ccg       2304
   Gly Met Glu Ile Phe Leu Ala Asp Thr Glu Asn Ala Glu Thr Leu Pro
           755                 760                 765
   cgg gaa cga gtg cta gac ttc ctg cag ggc att gat gtc aat ctg gcc       2352
   Arg Glu Arg Val Leu Asp Phe Leu Gln Gly Ile Asp Val Asn Leu Ala
       770                 775                 780
   gtc cgc tac ctg gag cat atc att ggg gag ttg aat gat atg aca ccg       2400
   Val Arg Tyr Leu Glu His Ile Ile Gly Glu Leu Asn Asp Met Thr Pro
   785                 790                 795                 800
   gat ctg cat cag aaa ctg ctc atc ctg tac ttg aac cgg ctg aag aag       2448
   Asp Leu His Gln Lys Leu Leu Ile Leu Tyr Leu Asn Arg Leu Lys Lys
                   805                 810                 815
   cac cag gct aaa gag tgg gag ttc tct agt ctg gat gac tat gtt aat       2496
   His Gln Ala Lys Glu Trp Glu Phe Ser Ser Leu Asp Asp Tyr Val Asn
               820                 825                 830
   tgg cag agc aag ttc ttg aat atg ctc agg tcc agc tct cag tat tct       2544
   Trp Gln Ser Lys Phe Leu Asn Met Leu Arg Ser Ser Ser Gln Tyr Ser
           835                 840                 845
   ccg gct aag att ctg gat cga ttg gac cga gac gat cct gaa ttc ttc       2592
   Pro Ala Lys Ile Leu Asp Arg Leu Asp Arg Asp Asp Pro Glu Phe Phe
       850                 855                 860
   gag gca cga gcg att gtc ttc agc aag atg ggg caa cac cgg cag gca       2640
   Glu Ala Arg Ala Ile Val Phe Ser Lys Met Gly Gln His Arg Gln Ala
   865                 870                 875                 880
   ctg gag att tat gta ttc aag ttg gaa gac tac gtc aag gct gaa gaa       2688
   Leu Glu Ile Tyr Val Phe Lys Leu Glu Asp Tyr Val Lys Ala Glu Glu
                   885                 890                 895
   tac tgt aat cat ctc cat aaa gtg gag gat acg act gct gcg gat gga       2736
   Tyr Cys Asn His Leu His Lys Val Glu Asp Thr Thr Ala Ala Asp Gly
               900                 905                 910
   tcg gtt tcg cgc tgc gta gct cta cta cca tac gaa gat gac aaa cca       2784
   Ser Val Ser Arg Cys Val Ala Leu Leu Pro Tyr Glu Asp Asp Lys Pro
           915                 920                 925
   tcg att tat ttg act cta ttg tct cta tat cta tct ccg ccg cat ggc       2832
   Ser Ile Tyr Leu Thr Leu Leu Ser Leu Tyr Leu Ser Pro Pro His Gly
       930                 935                 940
   tac aag cca cgg tac ggg ccc gcg ctt gag gtg ctg gcc aaa cat gga       2880
   Tyr Lys Pro Arg Tyr Gly Pro Ala Leu Glu Val Leu Ala Lys His Gly
   945                 950                 955                 960
   tcc cgg ctg cca ccc aat tct gct ctg gat ctg atc cca gaa tcg ctc       2928
   Ser Arg Leu Pro Pro Asn Ser Ala Leu Asp Leu Ile Pro Glu Ser Leu
                   965                 970                 975
   ccg gtc aag gag ctc gag ttc tat ttc aag ggc cgt atg cgg gcc gcc       2976
   Pro Val Lys Glu Leu Glu Phe Tyr Phe Lys Gly Arg Met Arg Ala Ala
               980                 985                 990
   aac aca atc ctt aac gag agc cgg  atc gtg gcc aac ctt  caa aag gca      3024
   Asn Thr Ile Leu Asn Glu Ser Arg  Ile Val Ala Asn Leu  Gln Lys Ala
           995                 1000                1005
   gaa gac  atc aag aca cag gcg  cag tta ttg gta gga  gag gga acg         3069
   Glu Asp  Ile Lys Thr Gln Ala  Gln Leu Leu Val Gly  Glu Gly Thr
       1010                1015                1020
   gac ggg  cga tcg acg cgc tca  cga cat gtc acc atc  acc gaa gag         3114
   Asp Gly  Arg Ser Thr Arg Ser  Arg His Val Thr Ile  Thr Glu Glu
       1025                1030                1035
   cgg gtc  tgc ggc atc tgc cac  aag cgg atc gga ggc  agt gtg att         3159
   Arg Val  Cys Gly Ile Cys His  Lys Arg Ile Gly Gly  Ser Val Ile
       1040                1045                1050
   aat gtg  ttt ccg gat aat acg  gtt gtc cat ctt ggc  tgt gcc aat         3204
   Asn Val  Phe Pro Asp Asn Thr  Val Val His Leu Gly  Cys Ala Asn
       1055                1060                1065
   cgc atg  cgt tga                                                       3216
   Arg Met  Arg
       1070
<210> 97
   <211> 1071
   <212> PRT
   <213> Aspergillus niger
<400> 97
   Met Leu Ser Ala Phe Thr Ala Arg Pro Leu Val Glu Leu Lys Pro Arg
   1               5                   10                  15
   Asp Arg Ser Arg Ile Glu Ser Val Leu Ala Tyr Gly Asp Arg Val Leu
               20                  25                  30
   Ala Gly Leu Asn Asn Gly Ser Leu Arg Ile Tyr Arg Val Asn Glu Gln
           35                  40                  45
   Glu Glu Glu Pro Glu Glu Gly Glu Glu Gly Gly Glu Gly Gln Gln His
       50                  55                  60
   Glu Gln His Gly Gln Asp Thr Gly Lys Gly Gly Lys Lys Asp Thr Asp
   65                  70                  75                  80
   Gln Gln Gly Asn Gly Asn Gly Glu Ala Gly Ile Ile Thr Asn Gly Thr
                   85                  90                  95
   Thr Asn Gly Thr Arg Glu His Lys Lys Pro Thr Glu Leu Leu Arg Glu
               100                 105                 110
   Val Glu Lys Phe Ser Arg Tyr Lys Ile Glu Gln Leu Ala Leu Ile Lys
           115                 120                 125
   Glu Ala Lys Val Leu Val Ser Leu Ser Gly Gly Tyr Val Ser Leu His
       130                 135                 140
   Asp Leu Gly Thr Tyr Ala Leu Gln Glu Gln Leu Gly Lys Thr Lys Gly
   145                 150                 155                 160
   Ala Thr Thr Phe Ala Val Thr Ser Asn Ile Glu Asn Asp Ser Glu Thr
                   165                 170                 175
   Gly Val Pro Ala Ile Val Ser Arg Val Ala Val Ala Val Lys Arg Lys
               180                 185                 190
   Ile Met Leu Trp Val Trp Arg Asp Met Glu Met Glu Gly Gly Val Pro
           195                 200                 205
   Met Glu Met Thr Leu Val Ser Gly Ile Lys Thr Leu Thr Trp Val Ser
       210                 215                 220
   Gly Thr Arg Leu Val Ala Gly Leu Gly Ser Gly Phe Val Met Val Asp
   225                 230                 235                 240
   Ile Glu Gly Gly Ser Gly Gly Thr Val Thr Asp Leu Thr Gly Pro Ser
                   245                 250                 255
   Gly Ile Gly Gly Leu Gly Gly Gln Glu Ser Thr Gly Arg Leu Ala Gly
               260                 265                 270
   Val Gly Val Ala Ser Met Ser Tyr Met Gly Leu Gly Gly Ser Ala Pro
           275                 280                 285
   Lys Pro Leu Ala Thr Arg Leu Lys Glu Gly Gln Val Leu Leu Ala Lys
       290                 295                 300
   Asp Ile Asn Thr His Phe Ile Asp Val Gln Gly Asn Ser Leu Gly Arg
   305                 310                 315                 320
   Arg Gln Ile Pro Trp Ser His Ala Pro Ala Asp Ile Gly Tyr Ser Tyr
                   325                 330                 335
   Pro Phe Leu Leu Ala Leu His Asp Ser Ser Lys Gly Val Leu Glu Val
               340                 345                 350
   Arg Asn Pro Glu Thr Leu Ser Leu Leu Gln Ser Val Pro Leu Pro Ser
           355                 360                 365
   Ala Ser Ile Met His Ile Pro Gln Pro Thr Ile Ser Leu Ala His Ala
       370                 375                 380
   Gly Lys Gly Phe Leu Val Ala Ser Asp Arg Thr Ile Trp Arg Met Glu
   385 390 395 400
   Ala Leu Ser Tyr Asp Thr Gln Ile Asp Ser Leu Val Glu Lys Gly Tyr
   405 410 415
   Leu Asp Glu Ala Ile Ser Leu Ala Ser Met Leu Glu Asp Ala Leu Leu
   420 425 430
   Arg Asp Lys Gln Gly Arg Leu Arg Gln Ile Lys Leu Glu Lys Ala Glu
   435 440 445
   Gly Leu Phe Lys Met Arg Lys Tyr Thr Asp Ser Met Asp Leu Phe Thr
   450 455 460
   Glu Ile Ser Ala Pro Pro Glu Thr Val Ile Arg Leu Tyr Pro Lys Ile
   465 470 475 480
   Ile Ala Gly Glu Leu Ser Ser Ile Val Glu Glu Pro Glu Glu Ser Glu
   485 490 495
   Asp Gly Thr Thr Asp Ser Gln Pro Lys Thr Gln Glu Asn Asn Asn Pro
   500 505 510
   Thr Asp Ala Pro Ala Thr Glu Glu Thr Pro Ala Pro Lys Thr Leu Ser
   515 520 525
   His Ala Pro Ser Val Met Ser Leu Leu Arg Thr Arg Thr Asp Asp Gly
   530 535 540
   Ser Asp Ala Gly Ser Ile Arg Gly Lys Val Val Glu Glu Ala Lys Asn
   545 550 555 560
   Asp Lys Ala Leu Glu Gly Ala Asp Leu Lys Leu Ala Val Arg Asp Leu
   565 570 575
   Gln Arg Tyr Leu Ala Asp Val Arg Arg Arg Phe Gln Arg Phe Leu Asn
   580 585 590
   Pro Asp Gly Thr Leu Lys Val Ile Asp Ala Thr Thr Asp Gly Ala Asn
   595 600 605
   Asp Ala Leu Thr Asp Ser Val Met Lys Leu Leu Ser Ile Asp Pro Glu
   610 615 620
   Gly Glu Tyr Asp Leu Gly Glu Lys Leu Arg Glu Lys Ala Arg Leu Val
   625 630 635 640
   Asp Thr Thr Leu Phe Arg Ala Tyr Met Tyr Ala Ile Pro Ala Leu Ala
   645 650 655
   Gly Ser Leu Phe Arg Ile Ala Asn Phe Cys Asp Pro Asp Val Val Met
   660 665 670
   Glu Lys Leu Glu Glu Thr Gly Arg His Asn Asp Leu Ile Asp Phe Leu
   675 680 685
   Tyr Gly Lys Lys Leu His Arg Gln Ala Leu Glu Leu Leu Gln Lys Phe
   690 695 700
   Gly Gln Ala Asp Asp Glu Glu Glu Thr Ala Pro Gln Leu His Gly Pro
   705 710 715 720
   Lys Arg Thr Val Asn Tyr Leu Gln Asn Leu Ser Pro Asp His Ile Asp
   725 730 735
   Leu Ile Leu Glu Phe Ala Glu Trp Pro Val Arg Gln Asp Pro Glu Leu
   740 745 750
   Gly Met Glu Ile Phe Leu Ala Asp Thr Glu Asn Ala Glu Thr Leu Pro
   755 760 765
   Arg Glu Arg Val Leu Asp Phe Leu Gln Gly Ile Asp Val Asn Leu Ala
   770 775 780
   Val Arg Tyr Leu Glu His Ile Ile Gly Glu Leu Asn Asp Met Thr Pro
   785 790 795 800
   Asp Leu His Gln Lys Leu Leu Ile Leu Tyr Leu Asn Arg Leu Lys Lys
   805 810 815
   His Gln Ala Lys Glu Trp Glu Phe Ser Ser Leu Asp Asp Tyr Val Asn
   820 825 830
   Trp Gln Ser Lys Phe Leu Asn Met Leu Arg Ser Ser Ser Gln Tyr Ser
   835 840 845
   Pro Ala Lys Ile Leu Asp Arg Leu Asp Arg Asp Asp Pro Glu Phe Phe
   850 855 860
   Glu Ala Arg Ala Ile Val Phe Ser Lys Met Gly Gln His Arg Gln Ala
   865 870 875 880
   Leu Glu Ile Tyr Val Phe Lys Leu Glu Asp Tyr Val Lys Ala Glu Glu
   885 890 895
   Tyr Cys Asn His Leu His Lys Val Glu Asp Thr Thr Ala Ala Asp Gly
   900 905 910
   Ser Val Ser Arg Cys Val Ala Leu Leu Pro Tyr Glu Asp Asp Lys Pro
           915                 920                 925
   Ser Ile Tyr Leu Thr Leu Leu Ser Leu Tyr Leu Ser Pro Pro His Gly
       930                 935                 940
   Tyr Lys Pro Arg Tyr Gly Pro Ala Leu Glu Val Leu Ala Lys His Gly
   945                 950                 955                 960
   Ser Arg Leu Pro Pro Asn Ser Ala Leu Asp Leu Ile Pro Glu Ser Leu
                   965                 970                 975
   Pro Val Lys Glu Leu Glu Phe Tyr Phe Lys Gly Arg Met Arg Ala Ala
               980                 985                 990
   Asn Thr Ile Leu Asn Glu Ser Arg  Ile Val Ala Asn Leu  Gln Lys Ala
           995                 1000                 1005
   Glu Asp  Ile Lys Thr Gln Ala  Gln Leu Leu Val Gly  Glu Gly Thr
       1010                 1015                 1020
   Asp Gly  Arg Ser Thr Arg Ser  Arg His Val Thr Ile  Thr Glu Glu
       1025                 1030                 1035
   Arg Val  Cys Gly Ile Cys His  Lys Arg Ile Gly Gly  Ser Val Ile
       1040                 1045                 1050
   Asn Val  Phe Pro Asp Asn Thr  Val Val His Leu Gly  Cys Ala Asn
       1055                 1060                 1065
   Arg Met  Arg
       1070
<210> 98
   <211> 5623
   <212> DNA
   <213> Aspergillus niger
<400> 98
   gacggaaatc tgcctccaga agtaaacgta cctcgtccct cgcccaattc ggcaagggtc 60
   tgctctgcgg gaattctgta tgtcttccga agctgctccc gacgctcaaa ctcgtcgata 120
   tcggtgttct tcttgtagcc gaacaacgtt ccaccggtgt attcgaaagc gcccgtggcg 180
   gtggccaaag cgacgccgta gcccaggagg gcggggaatg tgcgagctgc ggacctgtca 240
   gaaccaatgc ttgatcaatt gaacggtcgg atcgcgggca acagacctcg gagacccagg 300
   atggcgccgg agaagaaacc tcccagagcg acgttccagt ggtcttcctt ctcacgaagg 360
   ttggcggatg cggtcttcac gaattcgtag gtacctccca tggcggctgg acgcgaaaat 420
   cgttagcaac cgactaaatc caagacatat tgcccaagcg tccacaattg cattcatacc 480
   gaagataccg acggtaccac cgctccggat gaacacgccc agcgggccga cgttctgtct 540
   ggtgagggtg ttctggacgg ccgaggcgaa aagacccgct ccaccagtga gcatagtggt 600
   cttcatcgat gccgcgatcg catccttggg atggtagtgc tcttcgtggt gcggagccat 660
   ggtggcggtt gaggagctgg ggattaagct gtggctggca ctctcgaatg tcgtccgaac 720
   ggagatgaga ttcgatgaat ttgggacctc gcgctttctg ccccaccaga ccgccttccc 780
   caggttccca gctttcccag tccgtttgac cagagcatct gatctatgcc caaaactctc 840
   gattttctcc cttggcgtgc gggcatcgct ctctatcacc tcctgcacgc tcggtggtcg 900
   tcttttggtg tttctaaagc tgttcgcgtt gagagtggca ggtcgttcgc tgggaacttg 960
   ccgcctgctc tgcctcactt gctgcggtga ctccccgctg ttgagacgat ctaacggtcc 1020
   agctttctgt ttccaataga atcgaatgtg ttcaatacga taacgtggat catccctgca 1080
   agagagtcgc gtcgttatcc tttgtttaaa ttacggactt tatgtgaata acctcacaga 1140
   tagctttaga gttcctttgc taaggttcgc cgacaactgt gagctcataa gagcctactc 1200
   atgtcgtctc cgctgaagcc tggtatgcat cacagtcccc ttgagtccgg atcacttttt 1260
   cagagtccgg agtatctgca gtgaatgcgc atctgaactg atgatggttt cttcgtcttc 1320
   taggcaagac tgccccacgt cttgtgcaag atatccacga ctatgaccct gtccatcctc 1380
   caggctccgg ccgcaacctg ctgtcggagg tccccccggt cttccccgat cactatattg 1440
   gaccatcaga gttcatatcg ccttcagcat gccgtcatac ttatgtgatc aagtcgaaac 1500
   agagcttcct accaaaacca gagcagagaa gcagctcagg caaccccacc aaagtatcag 1560
   cgatatgctc caagtgccga taccatctac agctggtcgt gacatcgaca cctgggatcg 1620
   gcagccagag tttgccggga cacatacacc atctggttta taagtcaggg aggcagagag 1680
   gaggtgcatc ggcagaagaa atcacaccca agggccaggt ggcggagaca ttccattatg 1740
   actgctccta cctcacttgt ccagcggcag tgtccgtcag aattgtctct cctgtgctcc 1800
   atccagaatg ggtacaactc ttgacagatc ctgagttgct gcagcagcga gcagatgagg 1860
   cgattcaaac acaccccgag cgcctggaag ggatagcccg gccgcagccc atcaatgtct 1920
   tggaaaactt gcacgcatat atcagcaatg cctttaatcc cagtcaacag aataaatcta 1980
   tatcagcgat taacaagagg ttcataacgt gcttcggtgt tgaagggaag ccttgcaaag 2040
   acctgttaga atttttgggg ttctccgtca aggtctgtac atgtctgccg attgctgcaa 2100
   tatcatggcc taactctata tagaatgaag gtttttggga gcccccgcgc cccaacgcct 2160
   gggcggaaat cccctaccag gatcaactca aaatcttcct cgatgatgtt ctacatgagc 2220
   ttgctgtact catcgagcag cggccagtgc ttgagaagaa aggccatcag gtggatttct 2280
   caaacaagat agcaagggat gacctcttga atgctctgga agcattagac tgtaggtcaa 2340
   ataatcactc tttcggaggc tgcatgctta ccaagcttct ttaagaccag aaggctctca 2400
   atgtcgacac atttgaaatg ccccctgctc cgtacgtact atacaagctt gacctattga 2460
   cgtggactga cgcaggtgta gttactatga agatctgggc gtcgtggaag atatgtctgc 2520
   gcaattgatt attgaggcgt tcaatcgcca ggtcgccgta gaccctggtc gcatgcctac 2580
   atacctgaaa tgcctcaagg cgataggtag ccttagaggg ggctacgaag ctgacgccat 2640
   tgatcaagca gttcaggtgg cttatgccga cggcaagtac actgatgacg acgtcatcaa 2700
   tgcttacaag tacttcggtt taacacacga cgacccgcgg cttactgagg acagcatcat 2760
   tgggaccttc catgcttact tgagctccac cactcaggag acagactcac gcagagaact 2820
   gtggaagata ggcgacagca ggcgtagtga gcgcattaag tcagcggctg aagatagtga 2880
   gatctcctta cgacgagcgt ttgaagtccg cagctaacag ggtaaaggag tggcaactgt 2940
   tgagcaagcc caagtttacc ttggcgtcag cgaggaaaca agcgacgact ttattatggc 3000
   catgtataca gctaaggtac agcgctgccg attatgagtt tgtgtagatg tgcgctaatg 3060
   ttctttctag gtcaatgaca ctccatcgag cagggatctc gcgcgccgcg ccgttgaatt 3120
   cattgcggag gcgcggaaat cggatgctct tcgacacttc gtcaggactg gcgagatgac 3180
   ggccggtgag atggatatcg gggatgccta ccggcttctt caaatccccg atcgtacggt 3240
   tgatgaagca gcaatcatgg cagcttatac gatttgcgtc gacgaagctc cgggacaggc 3300
   tgaaacgtac aaccaagcct tgcgtataat tgctaaagac aagaatagca ctctgctaag 3360
   cagtatggtc tctggtgatg atacaaagcc ggaccgtaat atgtcggatt ggcctgttgg 3420
   attgcagaac attggcaaca catgctatct caatagtcta ctacaattct atttctctgt 3480
   gcgcccgtac agggagatgg tcttggattt tgagagcttc aagatggagt tgactgatga 3540
   gagcctgagc aagaaacagg tgggatctcg caaggtttcc aagaaggagg tggaacgttc 3600
   acaaaggtgt aagtagcagt atttcacttg atttaccgca tagctaacgc tgattctata 3660
   tagttctcag agaattacgg acgctctttt ccgacatgat tacttcccca tcctctcatg 3720
   tcacgccggg ccgggagttg gcgcggttga cattgatcag tccttccaac gaagcagcga 3780
   tacgtcgtcg gtctacaatc tccgcgtaca ggcccgccgg tgccttaggt gaaatcaacg 3840
   gcatgccggt actaggacct cttgggccgc cgcaaccgat tccggagaag caagtagaga 3900
   atgagacgga gaaccaggcg gttaccgaaa gcgaggtgat taagcgctca acagttagcg 3960
   atgtcgatag cgaggccaca cttgtctctg atggtgtcaa gaatgacatc aaagctcccc 4020
   cggttgacga taaagagaac gagccaccgg ctgatctgga cctgaatgac gtgcagaatg 4080
   agccaagttc tgattccgct gacatagggt cccccggcga tacaacaccc atagacaatg 4140
   tcgaaccttc caatcgacca ccgccagtgc ctccgcggcc caccccgcaa gttgaccccc 4200
   agaagcagct tatagaagag gtagagatcg gagcgcagca ggatgtgacg gaagtcatca 4260
   acaacgtgct tttccaaagc caatgtgcga ttcggcccat tgctcattct ccggagggtg 4320
   agcaaattga tcaagtgaaa gagtaagtta ggagcctttt gggcacgatt atttcaagac 4380
   tgacgccttg tctgtagtct tttttacggc cgaacccgat cttatattgc ctcagaaaaa 4440
   ggcgtccggt cgaaagaaga gtggtggtgt gacatcaaaa tcgatgttgc aactggcccg 4500
   cgcgacattt atgcagccat tgacggtgcc tttgacgtac agaaggtgaa tgtggagaac 4560
   tcggtggctg agcagtatgg tgctatcagc aagctcccgc cagtgcttca gatccaggtt 4620
   cagcgtgtcc agtttgaccc cacgacgaag cgctctttta aatccactca ccatcttgga 4680
   ttgaaggaga cgatttatct cgaccgatac atggactcgc aacaacccca gttcctggac 4740
   cggcgacagc agtgctggca atggaaaaat tcgctcagag agctcgaggt gcgccgcgcg 4800
   gagttactgc gccaagtggt aggttgcgac cgcatcgttc gttgtgagcc cagacatcta 4860
   attcttattc taggattccg acggcatgga tacggcggga ttactcaaca gcgctaagga 4920
   agtactcgaa gatttggcag ctatgagaga agagcccgat tatgcagagg acgcgattga 4980
   ctttgacacg caaataatca ccgaggttga tcagctttct caaatcgcaa aggctgactt 5040
   gaactgtacg tcctcgcttc ccaagattcg gcctctggta cgtgggcgct gacctgagac 5100
   agatgtcgaa aaccaaatcc aggagaccca ggacaagatc tccgctcagt tcgccgacta 5160
   caagtacctc gcctatcgcc tgtatgccgt gttcgtccac cacgggtccg tttccttcgg 5220
   ccattactac atttacatct tcgacttcga acggaacgtc tggagaaaat acaatgacaa 5280
   ctatgtcacc gaagtaaaca acctggacga gatcttcaag gacgagggac agcccaaccc 5340
   gcccacgcca tacttcctgg tatacgtcaa cgacgcgatg aaggatcggc tcgccaaccc 5400
   ggtctgcagg gaaatcttgg aagcttccac ggcgaatacc aatgaagata tggacatgaa 5460
   tccaccagcg tacgacgaga tctggacgga ccagagcgga tcaggtacgg tagtcgatca 5520
   ccccatgacg gaggctaaag acgatgccaa tgcgggacac ggcaagactg cttggggttg 5580
   gccaccgaag acaagcactg acttagatca tgtgaggtgg tag 5623
<210> 99
   <211> 3669
   <212> DNA
   <213> Aspergillus niger
<220>
   <221> CDS
   <222> (1) .. (3669)
<400> 99
   atg tcg tct ccg ctg aag cct ggc aag act gcc cca cgt ctt gtg caa          48
   Met Ser Ser Pro Leu Lys Pro Gly Lys Thr Ala Pro Arg Leu Val Gln
   1               5                   10                  15
   gat atc cac gac tat gac cct gtc cat cct cca ggc tcc ggc cgc aac          96
   Asp Ile His Asp Tyr Asp Pro Val His Pro Pro Gly Ser Gly Arg Asn
               20                  25                  30
   ctg ctg tcg gag gtc ccc ccg gtc ttc ccc gat cac tat att gga cca       144
   Leu Leu Ser Glu Val Pro Pro Val Phe Pro Asp His Tyr Ile Gly Pro
           35                  40                  45
   tca gag ttc ata tcg cct tca gca tgc cgt cat act tat gtg atc aag       192
   Ser Glu Phe Ile Ser Pro Ser Ala Cys Arg His Thr Tyr Val Ile Lys
       50                  55                  60
   tcg aaa cag agc ttc cta cca aaa cca gag cag aga agc agc tca ggc       240
   Ser Lys Gln Ser Phe Leu Pro Lys Pro Glu Gln Arg Ser Ser Ser Gly
   65                  70                  75                  80
   aac ccc acc aaa gta tca gcg ata tgc tcc aag tgc cga tac cat cta       288
   Asn Pro Thr Lys Val Ser Ala Ile Cys Ser Lys Cys Arg Tyr His Leu
                   85                  90                  95
   cag ctg gtc gtg aca tcg aca cct ggg atc ggc agc cag agt ttg ccg       336
   Gln Leu Val Val Thr Ser Thr Pro Gly Ile Gly Ser Gln Ser Leu Pro
               100                 105                 110
   gga cac ata cac cat ctg gtt tat aag tca ggg agg cag aga gga ggt       384
   Gly His Ile His His Leu Val Tyr Lys Ser Gly Arg Gln Arg Gly Gly
           115                 120                 125
   gca tcg gca gaa gaa atc aca ccc aag ggc cag gtg gcg gag aca ttc       432
   Ala Ser Ala Glu Glu Ile Thr Pro Lys Gly Gln Val Ala Glu Thr Phe
       130                 135                 140
   cat tat gac tgc tcc tac ctc act tgt cca gcg gca gtg tcc gtc aga       480
   His Tyr Asp Cys Ser Tyr Leu Thr Cys Pro Ala Ala Val Ser Val Arg
   145                 150                 155                 160
   att gtc tct cct gtg ctc cat cca gaa tgg gta caa ctc ttg aca gat       528
   Ile Val Ser Pro Val Leu His Pro Glu Trp Val Gln Leu Leu Thr Asp
                   165                 170                 175
   cct gag ttg ctg cag cag cga gca gat gag gcg att caa aca cac ccc       576
   Pro Glu Leu Leu Gln Gln Arg Ala Asp Glu Ala Ile Gln Thr His Pro
               180                 185                 190
   gag cgc ctg gaa ggg ata gcc cgg ccg cag ccc atc aat gtc ttg gaa       624
   Glu Arg Leu Glu Gly Ile Ala Arg Pro Gln Pro Ile Asn Val Leu Glu
           195                 200                 205
   aac ttg cac gca tat atc agc aat gcc ttt aat ccc agt caa cag aat       672
   Asn Leu His Ala Tyr Ile Ser Asn Ala Phe Asn Pro Ser Gln Gln Asn
       210                 215                 220
   aaa tct ata tca gcg att aac aag agg ttc ata acg tgc ttc ggt gtt       720
   Lys Ser Ile Ser Ala Ile Asn Lys Arg Phe Ile Thr Cys Phe Gly Val
   225                 230                 235                 240
   gaa ggg aag cct tgc aaa gac ctg tta gaa ttt ttg ggg ttc tcc gtc       768
   Glu Gly Lys Pro Cys Lys Asp Leu Leu Glu Phe Leu Gly Phe Ser Val
                   245                 250                 255
   aag aat gaa ggt ttt tgg gag ccc ccg cgc ccc aac gcc tgg gcg gaa       816
   Lys Asn Glu Gly Phe Trp Glu Pro Pro Arg Pro Asn Ala Trp Ala Glu
               260                 265                 270
   atc ccc tac cag gat caa ctc aaa atc ttc ctc gat gat gtt cta cat       864
   Ile Pro Tyr Gln Asp Gln Leu Lys Ile Phe Leu Asp Asp Val Leu His
           275                 280                 285
   gag ctt gct gta ctc atc gag cag cgg cca gtg ctt gag aag aaa ggc       912
   Glu Leu Ala Val Leu Ile Glu Gln Arg Pro Val Leu Glu Lys Lys Gly
   290 295 300
   cat cag gtg gat ttc tca aac aag ata gca agg gat gac ctc ttg aat 960
   His Gln Val Asp Phe Ser Asn Lys Ile Ala Arg Asp Asp Leu Leu Asn
   305 310 315 320
   gct ctg gaa gca tta gac tac cag aag gct ctc aat gtc gac aca ttt 1008
   Ala Leu Glu Ala Leu Asp Tyr Gln Lys Ala Leu Asn Val Asp Thr Phe
   325 330 335
   gaa atg ccc cct gct cct tac tat gaa gat ctg ggc gtc gtg gaa gat 1056
   Glu Met Pro Pro Ala Pro Tyr Tyr Glu Asp Leu Gly Val Val Glu Asp
   340 345 350
   atg tct gcg caa ttg att att gag gcg ttc aat cgc cag gtc gcc gta 1104
   Met Ser Ala Gln Leu Ile Ile Glu Ala Phe Asn Arg Gln Val Ala Val
   355 360 365
   gac cct ggt cgc atg cct aca tac ctg aaa tgc ctc aag gcg ata ggt 1152
   Asp Pro Gly Arg Met Pro Thr Tyr Leu Lys Cys Leu Lys Ala Ile Gly
   370 375 380
   agc ctt aga ggg ggc tac gaa gct gac gcc att gat caa gca gtt cag 1200
   Ser Leu Arg Gly Gly Tyr Glu Ala Asp Ala Ile Asp Gln Ala Val Gln
   385 390 395 400
   gtg gct tat gcc gac ggc aag tac act gat gac gac gtc atc aat gct 1248
   Val Ala Tyr Ala Asp Gly Lys Tyr Thr Asp Asp Asp Val Ile Asn Ala
   405 410 415
   tac aag tac ttc ggt tta aca cac gac gac ccg cgg ctt act gag gac 1296
   Tyr Lys Tyr Phe Gly Leu Thr His Asp Asp Pro Arg Leu Thr Glu Asp
   420 425 430
   agc atc att ggg acc ttc cat gct tac ttg agc tcc acc act cag gag 1344
   Ser Ile Ile Gly Thr Phe His Ala Tyr Leu Ser Ser Thr Thr Gln Glu
   435 440 445
   aca gac tca cgc aga gaa ctg tgg aag ata ggc gac agc agg cgt agt 1392
   Thr Asp Ser Arg Arg Glu Leu Trp Lys Ile Gly Asp Ser Arg Arg Ser
   450 455 460
   gag cgc att aag tca gcg gct gaa gat aga gtg gca act gtt gag caa 1440
   Glu Arg Ile Lys Ser Ala Ala Glu Asp Arg Val Ala Thr Val Glu Gln
   465 470 475 480
   gcc caa gtt tac ctt ggc gtc agc gag gaa aca agc gac gac ttt att 1488
   Ala Gln Val Tyr Leu Gly Val Ser Glu Glu Thr Ser Asp Asp Phe Ile
   485 490 495
   atg gcc atg tat aca gct aag gtc aat gac act cca tcg agc agg gat 1536
   Met Ala Met Tyr Thr Ala Lys Val Asn Asp Thr Pro Ser Ser Arg Asp
   500 505 510
   ctc gcg cgc cgc gcc gtt gaa ttc att gcg gag gcg cgg aaa tcg gat 1584
   Leu Ala Arg Arg Ala Val Glu Phe Ile Ala Glu Ala Arg Lys Ser Asp
   515 520 525
   gct ctt cga cac ttc gtc agg act ggc gag atg acg gcc ggt gag atg 1632
   Ala Leu Arg His Phe Val Arg Thr Gly Glu Met Thr Ala Gly Glu Met
   530 535 540
   gat atc ggg gat gcc tac cgg ctt ctt caa atc ccc gat cgt acg gtt 1680
   Asp Ile Gly Asp Ala Tyr Arg Leu Leu Gln Ile Pro Asp Arg Thr Val
   545 550 555 560
   gat gaa gca gca atc atg gca gct tat acg att tgc gtc gac gaa gct 1728
   Asp Glu Ala Ala Ile Met Ala Ala Tyr Thr Ile Cys Val Asp Glu Ala
   565 570 575
   ccg gga cag gct gaa acg tac aac caa gcc ttg cgt ata att gct aaa 1776
   Pro Gly Gln Ala Glu Thr Tyr Asn Gln Ala Leu Arg Ile Ile Ala Lys
   580 585 590
   gac aag aat agc act ctg cta agc agt atg gtc tct ggt gat gat aca 1824
   Asp Lys Asn Ser Thr Leu Leu Ser Ser Met Val Ser Gly Asp Asp Thr
   595 600 605
   aag ccg gac cgt aat atg tcg gat tgg cct gtt gga ttg cag aac att 1872
   Lys Pro Asp Arg Asn Met Ser Asp Trp Pro Val Gly Leu Gln Asn Ile
   610 615 620
   ggc aac aca tgc tat ctc aat agt cta cta caa ttc tat ttc tct gtg 1920
   Gly Asn Thr Cys Tyr Leu Asn Ser Leu Leu Gln Phe Tyr Phe Ser Val
   625 630 635 640
   cgc ccg tac agg gag atg gtc ttg gat ttt gag agc ttc aag atg gag 1968
   Arg Pro Tyr Arg Glu Met Val Leu Asp Phe Glu Ser Phe Lys Met Glu
   645 650 655
   ttg act gat gag agc ctg agc aag aaa cag gtg gga tct cgc aag gtt 2016
   Leu Thr Asp Glu Ser Leu Ser Lys Lys Gln Val Gly Ser Arg Lys Val
   660 665 670
   tcc aag aag gag gtg gaa cgt tca caa agg ttt ctc aga gaa tta cgg 2064
   Ser Lys Lys Glu Val Glu Arg Ser Gln Arg Phe Leu Arg Glu Leu Arg
   675 680 685
   acg ctc ttt tcc gac atg att act tcc cca tcc tct cat gtc acg ccg 2112
   Thr Leu Phe Ser Asp Met Ile Thr Ser Pro Ser Ser His Val Thr Pro
   690 695 700
   ggc cgg gag ttg gcg cgg ttg aca ttg atc agt cct tcc aac gaa gca 2160
   Gly Arg Glu Leu Ala Arg Leu Thr Leu Ile Ser Pro Ser Asn Glu Ala
   705 710 715 720
   gcg ata ctt agc gat gtc gat agc gag gcc aca ctt gtc tct gat ggt 2208
   Ala Ile Leu Ser Asp Val Asp Ser Glu Ala Thr Leu Val Ser Asp Gly
   725 730 735
   gtc aag aat gac atc aaa gct ccc ccg gtt gac gat aaa gag aac gag 2256
   Val Lys Asn Asp Ile Lys Ala Pro Pro Val Asp Asp Lys Glu Asn Glu
   740 745 750
   cca ccg gct gat ctg gac ctg aat gac gtg cag aat gag cca agt tct 2304
   Pro Pro Ala Asp Leu Asp Leu Asn Asp Val Gln Asn Glu Pro Ser Ser
   755 760 765
   gat tcc gct gac ata ggg tcc ccc ggc gat aca aca ccc ata gac aat 2352
   Asp Ser Ala Asp Ile Gly Ser Pro Gly Asp Thr Thr Pro Ile Asp Asn
   770 775 780
   gtc gaa cct tcc aat cga cca ccg cca gtg cct ccg cgg ccc acc ccg 2400
   Val Glu Pro Ser Asn Arg Pro Pro Pro Val Pro Pro Arg Pro Thr Pro
   785 790 795 800
   caa gtt gac ccc cag aag cag ctt ata gaa gag gta gag atc gga gcg 2448
   Gln Val Asp Pro Gln Lys Gln Leu Ile Glu Glu Val Glu Ile Gly Ala
   805 810 815
   cag cag gat gtg acg gaa gtc atc aac aac gtg ctt ttc caa agc caa      2496
   Gln Gln Asp Val Thr Glu Val Ile Asn Asn Val Leu Phe Gln Ser Gln
               820                 825                 830
   tgt gcg att cgg ccc att gct cat tct ccg gag ggt gag caa att gat      2544
   Cys Ala Ile Arg Pro Ile Ala His Ser Pro Glu Gly Glu Gln Ile Asp
           835                 840                 845
   caa gtg aaa gat ctt ttt tac ggc cga acc cga tct tat att gcc tca      2592
   Gln Val Lys Asp Leu Phe Tyr Gly Arg Thr Arg Ser Tyr Ile Ala Ser
       850                 855                 860
   gaa aaa ggc gtc cgg tcg aaa gaa gag tgg tgg tgt gac atc aaa atc      2640
   Glu Lys Gly Val Arg Ser Lys Glu Glu Trp Trp Cys Asp Ile Lys Ile
   865                 870                 875                 880
   gat gtt gca act ggc ccg cgc gac att tat gca gcc att gac ggt gcc      2688
   Asp Val Ala Thr Gly Pro Arg Asp Ile Tyr Ala Ala Ile Asp Gly Ala
                   885                 890                 895
   ttt gac gta cag aag gtg aat gtg gag aac tcg gtg gct gag cag tat      2736
   Phe Asp Val Gln Lys Val Asn Val Glu Asn Ser Val Ala Glu Gln Tyr
               900                 905                 910
   ggt gct atc agc aag ctc ccg cca gtg ctt cag atc cag gtt cag cgt      2784
   Gly Ala Ile Ser Lys Leu Pro Pro Val Leu Gln Ile Gln Val Gln Arg
           915                 920                 925
   gtc cag ttt gac ccc acg acg aag cgc tct ttt aaa tcc act cac cat      2832
   Val Gln Phe Asp Pro Thr Thr Lys Arg Ser Phe Lys Ser Thr His His
       930                 935                 940
   ctt gga ttg aag gag acg att tat ctc gac cga tac atg gac tcg caa      2880
   Leu Gly Leu Lys Glu Thr Ile Tyr Leu Asp Arg Tyr Met Asp Ser Gln
   945                 950                 955                 960
   caa ccc cag ttc ctg gac cgg cga cag cag tgc tgg caa tgg aaa aat      2928
   Gln Pro Gln Phe Leu Asp Arg Arg Gln Gln Cys Trp Gln Trp Lys Asn
                   965                 970                 975
   tcg ctc aga gag ctc gag gtg cgc cgc gcg gag tta ctg cgc caa gtg      2976
   Ser Leu Arg Glu Leu Glu Val Arg Arg Ala Glu Leu Leu Arg Gln Val
               980                 985                 990
   gat tcc gac ggc atg gat acg gcg  gga tta ctc aac agc  gct aag gaa    3024
   Asp Ser Asp Gly Met Asp Thr Ala  Gly Leu Leu Asn Ser  Ala Lys Glu
           995                 1000                 1005
   gta ctc  gaa gat ttg gca gct  atg aga gaa gag ccc  gat tat gca      3069
   Val Leu  Glu Asp Leu Ala Ala  Met Arg Glu Glu Pro  Asp Tyr Ala
       1010                 1015                 1020
   gag gac  gcg att gac ttt gac  acg caa ata atc acc  gag gtt gat      3114
   Glu Asp  Ala Ile Asp Phe Asp  Thr Gln Ile Ile Thr  Glu Val Asp
       1025                 1030                 1035
   cag ctt  tct caa atc gca aag  gct gac ttg aac tat  gtc gaa aac      3159
   Gln Leu  Ser Gln Ile Ala Lys  Ala Asp Leu Asn Tyr  Val Glu Asn
       1040                 1045                 1050
   caa atc  cag gag acc cag gac  aag atc tcc gct cag  ttc gcc gac      3204
   Gln Ile  Gln Glu Thr Gln Asp  Lys Ile Ser Ala Gln  Phe Ala Asp
       1055                 1060                 1065
   tac aag  tac ctc gcc tat cgc  ctg tat gcc gtg ttc  gtc cac cac        3249
   Tyr Lys  Tyr Leu Ala Tyr Arg  Leu Tyr Ala Val Phe  Val His His
       1070                 1075                 1080
   ggg tcc  gtt tcc ttc ggc cat  tac tac att tac atc  ttc gac ttc        3294
   Gly Ser  Val Ser Phe Gly His  Tyr Tyr Ile Tyr Ile  Phe Asp Phe
       1085                 1090                 1095
   gaa cgg  aac gtc tgg aga aaa  tac aat gac aac tat  gtc acc gaa        3339
   Glu Arg  Asn Val Trp Arg Lys  Tyr Asn Asp Asn Tyr  Val Thr Glu
       1100                 1105                 1110
   gta aac  aac ctg gac gag atc  ttc aag gac gag gga  cag ccc aac        3384
   Val Asn  Asn Leu Asp Glu Ile  Phe Lys Asp Glu Gly  Gln Pro Asn
       1115                 1120                 1125
   ccg ccc  acg cca tac ttc ctg  gta tac gtc aac gac  gcg atg aag        3429
   Pro Pro  Thr Pro Tyr Phe Leu  Val Tyr Val Asn Asp  Ala Met Lys
       1130                 1135                 1140
   gat cgg  ctc gcc aac ccg gtc  tgc agg gaa atc ttg  gaa gct tcc        3474
   Asp Arg  Leu Ala Asn Pro Val  Cys Arg Glu Ile Leu  Glu Ala Ser
       1145                 1150                 1155
   acg gcg  aat acc aat gaa gat  atg gac atg aat cca  cca gcg tac        3519
   Thr Ala  Asn Thr Asn Glu Asp  Met Asp Met Asn Pro  Pro Ala Tyr
       1160                 1165                 1170
   gac gag  atc tgg acg gac cag  agc gga tca ggt acg  gta gtc gat        3564
   Asp Glu  Ile Trp Thr Asp Gln  Ser Gly Ser Gly Thr  Val Val Asp
       1175                 1180                 1185
   cac ccc  atg acg gag gct aaa  gac gat gcc aat gcg  gga cac ggc        3609
   His Pro  Met Thr Glu Ala Lys  Asp Asp Ala Asn Ala  Gly His Gly
       1190                 1195                 1200
   aag act  gct tgg ggt tgg cca  ccg aag aca agc act  gac tta gat        3654
   Lys Thr  Ala Trp Gly Trp Pro  Pro Lys Thr Ser Thr  Asp Leu Asp
       1205                 1210                 1215
   cat gtg  agg tgg tag                                                  3669
   His Val  Arg Trp
       1220
<210> 100
   <211> 1222
   <212> PRT
   <213> Aspergillus niger
<400> 100
   Met Ser Ser Pro Leu Lys Pro Gly Lys Thr Ala Pro Arg Leu Val Gln
   1               5                   10                  15
   Asp Ile His Asp Tyr Asp Pro Val His Pro Pro Gly Ser Gly Arg Asn
               20                  25                  30
   Leu Leu Ser Glu Val Pro Pro Val Phe Pro Asp His Tyr Ile Gly Pro
           35                  40                  45
   Ser Glu Phe Ile Ser Pro Ser Ala Cys Arg His Thr Tyr Val Ile Lys
   50 55 60
   Ser Lys Gln Ser Phe Leu Pro Lys Pro Glu Gln Arg Ser Ser Ser Gly
   65 70 75 80
   Asn Pro Thr Lys Val Ser Ala Ile Cys Ser Lys Cys Arg Tyr His Leu
   85 90 95
   Gln Leu Val Val Thr Ser Thr Pro Gly Ile Gly Ser Gln Ser Leu Pro
   100 105 110
   Gly His Ile His His Leu Val Tyr Lys Ser Gly Arg Gln Arg Gly Gly
   115 120 125
   Ala Ser Ala Glu Glu Ile Thr Pro Lys Gly Gln Val Ala Glu Thr Phe
   130 135 140
   His Tyr Asp Cys Ser Tyr Leu Thr Cys Pro Ala Ala Val Ser Val Arg
   145 150 155 160
   Ile Val Ser Pro Val Leu His Pro Glu Trp Val Gln Leu Leu Thr Asp
   165 170 175
   Pro Glu Leu Leu Gln Gln Arg Ala Asp Glu Ala Ile Gln Thr His Pro
   180 185 190
   Glu Arg Leu Glu Gly Ile Ala Arg Pro Gln Pro Ile Asn Val Leu Glu
   195 200 205
   Asn Leu His Ala Tyr Ile Ser Asn Ala Phe Asn Pro Ser Gln Gln Asn
   210 215 220
   Lys Ser Ile Ser Ala Ile Asn Lys Arg Phe Ile Thr Cys Phe Gly Val
   225 230 235 240
   Glu Gly Lys Pro Cys Lys Asp Leu Leu Glu Phe Leu Gly Phe Ser Val
   245 250 255
   Lys Asn Glu Gly Phe Trp Glu Pro Pro Arg Pro Asn Ala Trp Ala Glu
   260 265 270
   Ile Pro Tyr Gln Asp Gln Leu Lys Ile Phe Leu Asp Asp Val Leu His
   275 280 285
   Glu Leu Ala Val Leu Ile Glu Gln Arg Pro Val Leu Glu Lys Lys Gly
   290 295 300
   His Gln Val Asp Phe Ser Asn Lys Ile Ala Arg Asp Asp Leu Leu Asn
   305 310 315 320
   Ala Leu Glu Ala Leu Asp Tyr Gln Lys Ala Leu Asn Val Asp Thr Phe
                   325                 330                 335
   Glu Met Pro Pro Ala Pro Tyr Tyr Glu Asp Leu Gly Val Val Glu Asp
               340                 345                 350
   Met Ser Ala Gln Leu Ile Ile Glu Ala Phe Asn Arg Gln Val Ala Val
           355                 360                 365
   Asp Pro Gly Arg Met Pro Thr Tyr Leu Lys Cys Leu Lys Ala Ile Gly
       370                 375                 380
   Ser Leu Arg Gly Gly Tyr Glu Ala Asp Ala Ile Asp Gln Ala Val Gln
   385                 390                 395                 400
   Val Ala Tyr Ala Asp Gly Lys Tyr Thr Asp Asp Asp Val Ile Asn Ala
                   405                 410                 415
   Tyr Lys Tyr Phe Gly Leu Thr His Asp Asp Pro Arg Leu Thr Glu Asp
               420                 425                 430
   Ser Ile Ile Gly Thr Phe His Ala Tyr Leu Ser Ser Thr Thr Gln Glu
           435                 440                 445
   Thr Asp Ser Arg Arg Glu Leu Trp Lys Ile Gly Asp Ser Arg Arg Ser
       450                 455                 460
   Glu Arg Ile Lys Ser Ala Ala Glu Asp Arg Val Ala Thr Val Glu Gln
   465                 470                 475                 480
   Ala Gln Val Tyr Leu Gly Val Ser Glu Glu Thr Ser Asp Asp Phe Ile
                   485                 490                 495
   Met Ala Met Tyr Thr Ala Lys Val Asn Asp Thr Pro Ser Ser Arg Asp
               500                 505                 510
   Leu Ala Arg Arg Ala Val Glu Phe Ile Ala Glu Ala Arg Lys Ser Asp
           515                 520                 525
   Ala Leu Arg His Phe Val Arg Thr Gly Glu Met Thr Ala Gly Glu Met
       530                 535                 540
   Asp Ile Gly Asp Ala Tyr Arg Leu Leu Gln Ile Pro Asp Arg Thr Val
   545                 550                 555                 560
   Asp Glu Ala Ala Ile Met Ala Ala Tyr Thr Ile Cys Val Asp Glu Ala
                   565                 570                 575
   Pro Gly Gln Ala Glu Thr Tyr Asn Gln Ala Leu Arg Ile Ile Ala Lys
               580                 585                 590
   Asp Lys Asn Ser Thr Leu Leu Ser Ser Met Val Ser Gly Asp Asp Thr
           595                 600                 605
   Lys Pro Asp Arg Asn Met Ser Asp Trp Pro Val Gly Leu Gln Asn Ile
       610                 615                 620
   Gly Asn Thr Cys Tyr Leu Asn Ser Leu Leu Gln Phe Tyr Phe Ser Val
   625                 630                 635                 640
   Arg Pro Tyr Arg Glu Met Val Leu Asp Phe Glu Ser Phe Lys Met Glu
                   645                 650                 655
   Leu Thr Asp Glu Ser Leu Ser Lys Lys Gln Val Gly Ser Arg Lys Val
               660                 665                 670
   Ser Lys Lys Glu Val Glu Arg Ser Gln Arg Phe Leu Arg Glu Leu Arg
           675                 680                 685
   Thr Leu Phe Ser Asp Met Ile Thr Ser Pro Ser Ser His Val Thr Pro
       690                 695                 700
   Gly Arg Glu Leu Ala Arg Leu Thr Leu Ile Ser Pro Ser Asn Glu Ala
   705                 710                 715                 720
   Ala Ile Leu Ser Asp Val Asp Ser Glu Ala Thr Leu Val Ser Asp Gly
                   725                 730                 735
   Val Lys Asn Asp Ile Lys Ala Pro Pro Val Asp Asp Lys Glu Asn Glu
               740                 745                 750
   Pro Pro Ala Asp Leu Asp Leu Asn Asp Val Gln Asn Glu Pro Ser Ser
           755                 760                 765
   Asp Ser Ala Asp Ile Gly Ser Pro Gly Asp Thr Thr Pro Ile Asp Asn
       770                 775                 780
   Val Glu Pro Ser Asn Arg Pro Pro Pro Val Pro Pro Arg Pro Thr Pro
   785                 790                 795                 800
   Gln Val Asp Pro Gln Lys Gln Leu Ile Glu Glu Val Glu Ile Gly Ala
                   805                 810                 815
   Gln Gln Asp Val Thr Glu Val Ile Asn Asn Val Leu Phe Gln Ser Gln
               820                 825                 830
   Cys Ala Ile Arg Pro Ile Ala His Ser Pro Glu Gly Glu Gln Ile Asp
   835 840 845
   Gln Val Lys Asp Leu Phe Tyr Gly Arg Thr Arg Ser Tyr Ile Ala Ser
   850 855 860
   Glu Lys Gly Val Arg Ser Lys Glu Glu Trp Trp Cys Asp Ile Lys Ile
   865 870 875 880
   Asp Val Ala Thr Gly Pro Arg Asp Ile Tyr Ala Ala Ile Asp Gly Ala
   885 890 895
   Phe Asp Val Gln Lys Val Asn Val Glu Asn Ser Val Ala Glu Gln Tyr
   900 905 910
   Gly Ala Ile Ser Lys Leu Pro Pro Val Leu Gln Ile Gln Val Gln Arg
   915 920 925
   Val Gln Phe Asp Pro Thr Thr Lys Arg Ser Phe Lys Ser Thr His His
   930 935 940
   Leu Gly Leu Lys Glu Thr Ile Tyr Leu Asp Arg Tyr Met Asp Ser Gln
   945 950 955 960
   Gln Pro Gln Phe Leu Asp Arg Arg Gln Gln Cys Trp Gln Trp Lys Asn
   965 970 975
   Ser Leu Arg Glu Leu Glu Val Arg Arg Ala Glu Leu Leu Arg Gln Val
   980 985 990
   Asp Ser Asp Gly Met Asp Thr Ala Gly Leu Leu Asn Ser Ala Lys Glu
   995 1000 1005
   Val Leu Glu Asp Leu Ala Ala Met Arg Glu Glu Pro Asp Tyr Ala
   1010 1015 1020
   Glu Asp Ala Ile Asp Phe Asp Thr Gln Ile Ile Thr Glu Val Asp
   1025 1030 1035
   Gln Leu Ser Gln Ile Ala Lys Ala Asp Leu Asn Tyr Val Glu Asn
   1040 1045 1050
   Gln Ile Gln Glu Thr Gln Asp Lys Ile Ser Ala Gln Phe Ala Asp
   1055 1060 1065
   Tyr Lys Tyr Leu Ala Tyr Arg Leu Tyr Ala Val Phe Val His His
   1070 1075 1080
   Gly Ser Val Ser Phe Gly His Tyr Tyr Ile Tyr Ile Phe Asp Phe
   1085 1090 1095
   Glu Arg Asn Val Trp Arg Lys Tyr Asn Asp Asn Tyr Val Thr Glu
   1100 1105 1110
   Val Asn Asn Leu Asp Glu Ile Phe Lys Asp Glu Gly Gln Pro Asn
   1115 1120 1125
   Pro Pro Thr Pro Tyr Phe Leu Val Tyr Val Asn Asp Ala Met Lys
   1130 1135 1140
   Asp Arg Leu Ala Asn Pro Val Cys Arg Glu Ile Leu Glu Ala Ser
   1145 1150 1155
   Thr Ala Asn Thr Asn Glu Asp Met Asp Met Asn Pro Pro Ala Tyr
   1160 1165 1170
   Asp Glu Ile Trp Thr Asp Gln Ser Gly Ser Gly Thr Val Val Asp
   1175 1180 1185
   His Pro Met Thr Glu Ala Lys Asp Asp Ala Asn Ala Gly His Gly
   1190 1195 1200
   Lys Thr Ala Trp Gly Trp Pro Pro Lys Thr Ser Thr Asp Leu Asp
   1205 1210 1215
   His Val Arg Trp
   1220
<210> 101
   <211> 4934
   <212> DNA
   <213> Aspergillus niger
<400> 101
   gcgtctttgg agctgctatc ttgaggtttc acgggcggat tctggaggta ctttcggacg      60
   ttcgagtcta ggagcgatat ggaagctctg taagaagaaa gtttcgattc aatagagttt     120
   tccatttggg acaattcgcg aagaatgtgt tgatgtcttg ctttcatttg ggacaaccta     180
   gtctccatct catgtatatc ctcctcaagc ttgcgacttt cctggcgcaa ggcaacggtg     240
   tgctggcttt cccggttatc ctgaatagtc gaaatggcat cttctagccc agcgcgatgg     300
   atattgaagc cattgatatc tgccaacgcc tgtgtccttt catcaatccg gaaggttagt     360
   atctctcgct cttcctcgcg aagcctcaag aggtcgtata tagatctgaa tatccccttt     420
   ctggcagcag acaacccgat tttctctgga gccggttggc ggattggtat agttggagga     480
   cgctgcgaac tgcttgtgtc cgaggtcggt gtataagttc cgtcggagat ggcgccattc     540
   tgcggggatc cgttaagagt agccagaagt ccttcactct gagcatcgat taatatctgt     600
   aaattacgct gaatctgtct ggcctggcgg tcaatgtaga cgatagggtc ctgatgaagg 660
   gaagaggtcc cgataggcag ggccggcagg tgtttgtctc cagcttgcgg aagaatcgac 720
   gacatttgga aggggaactg gatcgcaggg aatcaaatta ccaatacatg agaatcccct 780
   ccttatggat attaaaatga aacaaaaccg acagctcaga acccaagtga cggtgaagtc 840
   ttggaccaga tcacgagtgt ttgtgcaact gccctgtccg gtcccatgcc atcataggtc 900
   acgtgccatc tctaggggtt tctgcaggaa ccacgggaac ctttcgacag cttccattga 960
   cgtgtgtctc gctgctctag aggaagcttc ctaatcactc cgagctcatc tcttccatca 1020
   tctgttattt tgttgccttg actccttcat cgcccttttt ctgtatattt cgttctttga 1080
   ggtgcttaga tctccaataa acaccccatc cattcaccca cgccctaccc tcaatctcac 1140
   agctccgtcg aagctgtcgc ctcactcccc tcgcgacagc acacccgtcc tcccacagcg 1200
   atggttggac tcgcttcggc cgctggcctc gtcggctttc tctcagagcc ggatcctgag 1260
   ttgagagtct ttgctctgaa gacgctggac tcacaaatcg acctgctctg gacggaagtc 1320
   gtggatgctg ttcctcaaat gttcgtactg gcccttgtct ctccatctgc ctcatacctt 1380
   catcataaat accccatcct gtcgaccggc cacaggctgc atcgatgctg acattttgcg 1440
   attcagagaa gctctttacg aagatgaatc gttccccgag cgcgagctcg ccgccctggt 1500
   cgccgcaaag gtgtactacc accttcaaga atacaatgag agtatggtat ttgctctggg 1560
   agctgggaag ctcttccagt tggatagcgg tggcgagttt gaggaaacca ttatcggtaa 1620
   gaacagccgt tccccaggca tttgctcacc cagagtgtca tcttaccggg atatacagcg 1680
   aaatgcgtcg ataccttcgt ctcgctctcg gcggcacagc ggcccgtcgc gggtgaccac 1740
   caagccaatc tgaacacctc tttccccacc tctggcgaag gcgccacaag tacttctgcc 1800
   agtttgacat ccccgatcac acctttctcg caatcagcct tgccatccaa gtcgctcttg 1860
   tctaggcaag aggtgcccgg agtggatgct gcttacccag ggggtgaaga tgcgggtgtg 1920
   aaccatgccg aagaaacccc tctagtcctg aagcgtggtg ttcagggcca ccttcaaacc 1980
   gttatcgatc gtctgttcga gcagtgcttc gtccagaagc gctaccgtca agtcattgga 2040
   atcgctattg aagcaaagaa cctcgatgtg cttagaaaga cgatcctccg ggccagcgag 2100
   gatgagaaga aacagaacgg agagtcgcga cgcagtgagg aactcatgga gtacgtgctc 2160
   gatatctgca tgggaatcgt gcaggaacgc ggtttccgca acgaggtata taattcatta 2220
   agatgatggc aaaacaccca gagctaaccc ttgctttgaa gatcctcaaa ctcatcctcg 2280
   aactcctcaa cgagatcccg gcccccgatt acttctccat cgccaagtgt gttgtatatc 2340
   tcaacgaaca ttccatggcg tcggtcatcc tccgtcaact agttgagaag ggagacgctc 2400
   gttcgctcgc tgtcgcctac caaatctctt tcgatctcta cgataacagt acgcaagaat 2460
   tcctgcagaa ggttcgccag gaaatttcgg agctcgtgcc ggagaatgat gagtccgaac 2520
   aggaggcagc aggtggggac caagaagagt cgaaggaatc cgatcctttg ctggacagcc 2580
   agtctaggtc tattggggat tcgcgcacga agatctcacc cgaagcacgc acggccttca 2640
   agagcattcg tgatattctc gatggcgtca agaccaacca gttgaacgtg gaattcttat 2700
   tccgcaacaa caaggccgat attgccatca tgaacaagct ccgcgatagc ctagaagctc 2760
   gtaactctat cttccactct gcggtttcgc tttcgaatgg tttcatgcat gccggtacag 2820
   cgcacgacaa gttcttccgg gataaccttg actggttgag caaggctgtc aactggtcca 2880
   agttcacggc tacagctgct cttggtgtta tccacagggg caatttgagc caaggacaaa 2940
   agctcctgca gccttacctg cccaaggaac gctctatcac caatgtggca gcgggcggac 3000
   acagcggagc cagctcgatt tacagtcagg gaggatcttt gtacgcttat ggtctgatct 3060
   tcgccaatca tggtggtatg gccgttcctg tgatccggga ttacttcaag aaggctacag 3120
   atgaggtcat ccagcatggt ggagctctgg gtcttggtgt tgcaggcatg gctagtggcg 3180
   atgaggggct ttatgaggac cttcgcaatg tgttgtacac cgactcggct atcaacggtg 3240
   aggctgtcgg tcttgccatg ggtcttatca tgttgggtac aggtaatatg aaggtcctcg 3300
   aggatatgat tcagtatgcc catgacactc aacacgagaa gatcgttcgg ggtttggcta 3360
   tgggcatggc cttgatcatg tacggtcgtc aggaagctgc tgatgagctg attaacggcc 3420
   ttctgggcga ccctgatcct gctctccgct acggtggtat catgactatc gcccttgcct 3480
   actgtggtag cagcagcaac aaggctgtgc gtaagctcct tcatgttgct gtcagcgatg 3540
   tgaacgatga tgttcgacga attgctgttc tgagcttggg attcattctg ttcaggaagc 3600
   atcagagtgt acctcgtatg gttgagctac tgtctgagtc ttacaaccct catgttcgtt 3660
   atggtgcggc aatggcactg ggtatctctt gcgccggaac tggtctggat gaggccattg 3720
   acctgctcga gccgatgctc aaggattcaa cggacttcgt gagacaggga gcgctgatct 3780
   cgttggccat ggtccttgtg caacagaacg aggccatgaa cccccgtgtc agctctctcc 3840
   gcaaggtcat gatgaagatg attggcgata gacacgagga tgcaatggcc aagttcggct 3900
   gcgctgttgc ccttggtatc atcgacgccg gtggtcgtaa ctgcaccatc agcctccaga 3960
   cgcaaaccgg caacctgaac atgcccggta tcgttggcgc tgccgtcttc gtccagtact 4020
   ggtactggtt ccctctcacc cacttcctgt cccttagttt cgccccgacc tccgtcattg 4080
   gtgtcgacca gaagctcgag gtgccctact tcaaattcca cagcaacacg cgacccagcc 4140
   tctttgacta ccccccggag caacaggtta aagccgaaga ggcgcccgag aaggtcaaga 4200
   cagccgtcct ttcgaccacc gcccaggcca agcgccgtgc acagcgtcgg gagaagcagc 4260
   aaaggcgcga gagcatggac attgatcaga cccctacgac tcccaaggtg tcggaccagg 4320
   ctgacaagat ggatacggat gagacagcag tgaagacgga agaagagaac aaggagggtg 4380
   agaaggaggc tggtgagggg cagaagaaga aggttgagcg tgagaaggtc ggatatgagc 4440
   tcgaaaacat gtctcgggtt cttcccgccc agctcaagta cctcacattc cctgaccccc 4500
   ggtatgagcc ggtcaagcgg gtacgtttcc cttcccaatt atttctgtgt tatattgcga    4560
   tgaatgccat gctaactcga atccagccta ccggtggagt tgtggtcgta cttgacaaga    4620
   cccccgagga gccccgcgag acgatcgaga tgacggccag caaggagacc agcagggagg    4680
   ctagacagcc tgcacccgcc gcggatgtcg cggagtccct gcaggaccga ctacaggcca    4740
   ccatgggagc cgctggtctg cagacacctc agcgtggagc tgggcgcttc ccctttggtg    4800
   cggatctggg cccgtttgcg ggcgctgccg ctgctgccgg tgtactgacg gctgtggatg    4860
   aggacgagga aggcggcgag gaggcgccgg tgccgaatga gtttgaatat gaaaccgagc    4920
   cagaagacga gtag                                                      4934
<210> 102
   <211> 3444
   <212> DNA
   <213> Aspergillus niger
<220>
   <221> CDS
   <222> (1)..(3444)
<400> 102
   atg gtt gga ctc gct tcg gcc gct ggc ctc gtc ggc ttt ctc tca gag      48
   Met Val Gly Leu Ala Ser Ala Ala Gly Leu Val Gly Phe Leu Ser Glu
   1               5                   10                  15
   ccg gat cct gag ttg aga gtc ttt gct ctg aag acg ctg gac tca caa      96
   Pro Asp Pro Glu Leu Arg Val Phe Ala Leu Lys Thr Leu Asp Ser Gln
               20                  25                  30
   atc gac ctg ctc tgg acg gaa gtc gtg gat gct gtt cct caa ata gaa      144
   Ile Asp Leu Leu Trp Thr Glu Val Val Asp Ala Val Pro Gln Ile Glu
           35                  40                  45
   gct ctt tac gaa gat gaa tcg ttc ccc gag cgc gag ctc gcc gcc ctg      192
   Ala Leu Tyr Glu Asp Glu Ser Phe Pro Glu Arg Glu Leu Ala Ala Leu
       50                  55                  60
   gtc gcc gca aag gtg tac tac cac ctt caa gaa tac aat gag agt atg      240
   Val Ala Ala Lys Val Tyr Tyr His Leu Gln Glu Tyr Asn Glu Ser Met
   65                  70                  75                  80
   gta ttt gct ctg gga gct ggg aag ctc ttc cag ttg gat agc ggt ggc      288
   Val Phe Ala Leu Gly Ala Gly Lys Leu Phe Gln Leu Asp Ser Gly Gly
                   85                  90                  95
   gag ttt gag gaa acc att atc gcg aaa tgc gtc gat acc ttc gtc tcg      336
   Glu Phe Glu Glu Thr Ile Ile Ala Lys Cys Val Asp Thr Phe Val Ser
               100                 105                 110
   ctc tcg gcg gca cag cgg ccc gtc gcg ggt gac cac caa gcc aat ctg      384
   Leu Ser Ala Ala Gln Arg Pro Val Ala Gly Asp His Gln Ala Asn Leu
           115                 120                 125
   aac acc tct ttc ccc acc tct ggc gaa ggc gcc aca agt act tct gcc      432
   Asn Thr Ser Phe Pro Thr Ser Gly Glu Gly Ala Thr Ser Thr Ser Ala
       130                 135                 140
   agt ttg aca tcc ccg atc aca cct ttc tcg caa tca gcc ttg cca tcc      480
   Ser Leu Thr Ser Pro Ile Thr Pro Phe Ser Gln Ser Ala Leu Pro Ser
   145                 150                 155                 160
   aag tcg ctc ttg tct agg caa gag gtg ccc gga gtg gat gct gct tac        528
   Lys Ser Leu Leu Ser Arg Gln Glu Val Pro Gly Val Asp Ala Ala Tyr
                   165                 170                 175
   cca ggg ggt gaa gat gcg ggt gtg aac cat gcc gaa gaa acc cct cta        576
   Pro Gly Gly Glu Asp Ala Gly Val Asn His Ala Glu Glu Thr Pro Leu
               180                 185                 190
   gtc ctg aag cgt ggt gtt cag ggc cac ctt caa acc gtt atc gat cgt        624
   Val Leu Lys Arg Gly Val Gln Gly His Leu Gln Thr Val Ile Asp Arg
           195                 200                 205
   ctg ttc gag cag tgc ttc gtc cag aag cgc tac cgt caa gtc att gga        672
   Leu Phe Glu Gln Cys Phe Val Gln Lys Arg Tyr Arg Gln Val Ile Gly
       210                 215                 220
   atc gct att gaa gca aag aac ctc gat gtg ctt aga aag acg atc ctc        720
   Ile Ala Ile Glu Ala Lys Asn Leu Asp Val Leu Arg Lys Thr Ile Leu
   225                 230                 235                 240
   cgg gcc agc gag gat gag aag aaa cag aac gga gag tcg cga cgc agt        768
   Arg Ala Ser Glu Asp Glu Lys Lys Gln Asn Gly Glu Ser Arg Arg Ser
                   245                 250                 255
   gag gaa ctc atg gag tac gtg ctc gat atc tgc atg gga atc gtg cag        816
   Glu Glu Leu Met Glu Tyr Val Leu Asp Ile Cys Met Gly Ile Val Gln
               260                 265                 270
   gaa cgc ggt ttc cgc aac gag atc ctc aaa ctc atc ctc gaa ctc ctc        864
   Glu Arg Gly Phe Arg Asn Glu Ile Leu Lys Leu Ile Leu Glu Leu Leu
           275                 280                 285
   aac gag atc ccg gcc ccc gat tac ttc tcc atc gcc aag tgt gtt gta        912
   Asn Glu Ile Pro Ala Pro Asp Tyr Phe Ser Ile Ala Lys Cys Val Val
       290                 295                 300
   tat ctc aac gaa cat tcc atg gcg tcg gtc atc ctc cgt caa cta gtt        960
   Tyr Leu Asn Glu His Ser Met Ala Ser Val Ile Leu Arg Gln Leu Val
   305                 310                 315                 320
   gag aag gga gac gct cgt tcg ctc gct gtc gcc tac caa atc tct ttc       1008
   Glu Lys Gly Asp Ala Arg Ser Leu Ala Val Ala Tyr Gln Ile Ser Phe
                   325                 330                 335
   gat ctc tac gat aac agt acg caa gaa ttc ctg cag aag gtt cgc cag       1056
   Asp Leu Tyr Asp Asn Ser Thr Gln Glu Phe Leu Gln Lys Val Arg Gln
               340                 345                 350
   gaa att tcg gag ctc gtg ccg gag aat gat gag tcc gaa cag gag gca       1104
   Glu Ile Ser Glu Leu Val Pro Glu Asn Asp Glu Ser Glu Gln Glu Ala
           355                 360                 365
   gca ggt ggg gac caa gaa gag tcg aag gaa tcc gat cct ttg ctg gac       1152
   Ala Gly Gly Asp Gln Glu Glu Ser Lys Glu Ser Asp Pro Leu Leu Asp
       370                 375                 380
   agc cag tct agg tct att ggg gat tcg cgc acg aag atc tca ccc gaa       1200
   Ser Gln Ser Arg Ser Ile Gly Asp Ser Arg Thr Lys Ile Ser Pro Glu
   385                 390                 395                 400
   gca cgc acg gcc ttc aag agc att cgt gat att ctc gat ggc gtc aag       1248
   Ala Arg Thr Ala Phe Lys Ser Ile Arg Asp Ile Leu Asp Gly Val Lys
   405 410 415
   acc aac cag ttg aac gtg gaa ttc tta ttc cgc aac aac aag gcc gat 1296
   Thr Asn Gln Leu Asn Val Glu Phe Leu Phe Arg Asn Asn Lys Ala Asp
   420 425 430
   att gcc atc atg aac aag ctc cgc gat agc cta gaa gct cgt aac tct 1344
   Ile Ala Ile Met Asn Lys Leu Arg Asp Ser Leu Glu Ala Arg Asn Ser
   435 440 445
   atc ttc cac tct gcg gtt tcg ctt tcg aat ggt ttc atg cat gcc ggt 1392
   Ile Phe His Ser Ala Val Ser Leu Ser Asn Gly Phe Met His Ala Gly
   450 455 460
   aca gcg cac gac aag ttc ttc cgg gat aac ctt gac tgg ttg agc aag 1440
   Thr Ala His Asp Lys Phe Phe Arg Asp Asn Leu Asp Trp Leu Ser Lys
   465 470 475 480
   gct gtc aac tgg tcc aag ttc acg gct aca gct gct ctt ggt gtt atc 1488
   Ala Val Asn Trp Ser Lys Phe Thr Ala Thr Ala Ala Leu Gly Val Ile
   485 490 495
   cac agg ggc aat ttg agc caa gga caa aag ctc ctg cag cct tac ctg 1536
   His Arg Gly Asn Leu Ser Gln Gly Gln Lys Leu Leu Gln Pro Tyr Leu
   500 505 510
   ccc aag gaa cgc tct atc acc aat gtg gca gcg ggc gga cac agc gga 1584
   Pro Lys Glu Arg Ser Ile Thr Asn Val Ala Ala Gly Gly His Ser Gly
   515 520 525
   gcc agc tcg att tac agt cag gga gga tct ttg tac gct tat ggt ctg 1632
   Ala Ser Ser Ile Tyr Ser Gln Gly Gly Ser Leu Tyr Ala Tyr Gly Leu
   530 535 540
   atc ttc gcc aat cat ggt ggt atg gcc gtt cct gtg atc cgg gat tac 1680
   Ile Phe Ala Asn His Gly Gly Met Ala Val Pro Val Ile Arg Asp Tyr
   545 550 555 560
   ttc aag aag gct aca gat gag gtc atc cag cat ggt gga gct ctg ggt 1728
   Phe Lys Lys Ala Thr Asp Glu Val Ile Gln His Gly Gly Ala Leu Gly
   565 570 575
   ctt ggt gtt gca ggc atg gct agt ggc gat gag ggg ctt tat gag gac 1776
   Leu Gly Val Ala Gly Met Ala Ser Gly Asp Glu Gly Leu Tyr Glu Asp
   580 585 590
   ctt cgc aat gtg ttg tac acc gac tcg gct atc aac ggt gag gct gtc 1824
   Leu Arg Asn Val Leu Tyr Thr Asp Ser Ala Ile Asn Gly Glu Ala Val
   595 600 605
   ggt ctt gcc atg ggt ctt atc atg ttg ggt aca ggt aat atg aag gtc 1872
   Gly Leu Ala Met Gly Leu Ile Met Leu Gly Thr Gly Asn Met Lys Val
   610 615 620
   ctc gag gat atg att cag tat gcc cat gac act caa cac gag aag atc 1920
   Leu Glu Asp Met Ile Gln Tyr Ala His Asp Thr Gln His Glu Lys Ile
   625 630 635 640
   gtt cgg ggt ttg gct atg ggc atg gcc ttg atc atg tac ggt cgt cag 1968
   Val Arg Gly Leu Ala Met Gly Met Ala Leu Ile Met Tyr Gly Arg Gln
   645 650 655
   gaa gct gct gat gag ctg att aac ggc ctt ctg ggc gac cct gat cct 2016
   Glu Ala Ala Asp Glu Leu Ile Asn Gly Leu Leu Gly Asp Pro Asp Pro
   660 665 670
   gct ctc cgc tac ggt ggt atc atg act atc gcc ctt gcc tac tgt ggt      2064
   Ala Leu Arg Tyr Gly Gly Ile Met Thr Ile Ala Leu Ala Tyr Cys Gly
           675                 680                 685
   agc agc agc aac aag gct gtg cgt aag ctc ctt cat gtt gct gtc agc      2112
   Ser Ser Ser Asn Lys Ala Val Arg Lys Leu Leu His Val Ala Val Ser
       690                 695                 700
   gat gtg aac gat gat gtt cga cga att gct gtt ctg agc ttg gga ttc      2160
   Asp Val Asn Asp Asp Val Arg Arg Ile Ala Val Leu Ser Leu Gly Phe
   705                 710                 715                 720
   att ctg ttc agg aag cat cag agt gta cct cgt atg gtt gag cta ctg      2208
   Ile Leu Phe Arg Lys His Gln Ser Val Pro Arg Met Val Glu Leu Leu
                   725                 730                 735
   tct gag tct tac aac cct cat gtt cgt tat ggt gcg gca atg gca ctg      2256
   Ser Glu Ser Tyr Asn Pro His Val Arg Tyr Gly Ala Ala Met Ala Leu
               740                 745                 750
   ggt atc tct tgc gcc gga act ggt ctg gat gag gcc att gac ctg ctc      2304
   Gly Ile Ser Cys Ala Gly Thr Gly Leu Asp Glu Ala Ile Asp Leu Leu
           755                 760                 765
   gag ccg atg ctc aag gat tca acg gac ttc gtg aga cag gga gcg ctg      2352
   Glu Pro Met Leu Lys Asp Ser Thr Asp Phe Val Arg Gln Gly Ala Leu
       770                 775                 780
   atc tcg ttg gcc atg gtc ctt gtg caa cag aac gag gcc atg aac ccc      2400
   Ile Ser Leu Ala Met Val Leu Val Gln Gln Asn Glu Ala Met Asn Pro
   785                 790                 795                 800
   cgt gtc agc tct ctc cgc aag gtc atg atg aag atg att ggc gat aga      2448
   Arg Val Ser Ser Leu Arg Lys Val Met Met Lys Met Ile Gly Asp Arg
                   805                 810                 815
   cac gag gat gca atg gcc aag ttc ggc tgc gct gtt gcc ctt ggt atc      2496
   His Glu Asp Ala Met Ala Lys Phe Gly Cys Ala Val Ala Leu Gly Ile
               820                 825                 830
   atc gac gcc ggt ggt cgt aac tgc acc atc agc ctc cag acg caa acc      2544
   Ile Asp Ala Gly Gly Arg Asn Cys Thr Ile Ser Leu Gln Thr Gln Thr
           835                 840                 845
   ggc aac ctg aac atg ccc ggt atc gtt ggc gct gcc gtc ttc gtc cag      2592
   Gly Asn Leu Asn Met Pro Gly Ile Val Gly Ala Ala Val Phe Val Gln
       850                 855                 860
   tac tgg tac tgg ttc cct ctc acc cac ttc ctg tcc ctt agt ttc gcc      2640
   Tyr Trp Tyr Trp Phe Pro Leu Thr His Phe Leu Ser Leu Ser Phe Ala
   865                 870                 875                 880
   ccg acc tcc gtc att ggt gtc gac cag aag ctc gag gtg ccc tac ttc      2688
   Pro Thr Ser Val Ile Gly Val Asp Gln Lys Leu Glu Val Pro Tyr Phe
                   885                 890                 895
   aaa ttc cac agc aac acg cga ccc agc ctc ttt gac tac ccc ccg gag      2736
   Lys Phe His Ser Asn Thr Arg Pro Ser Leu Phe Asp Tyr Pro Pro Glu
               900                 905                 910
   caa cag gtt aaa gcc gaa gag gcg ccc gag aag gtc aag aca gcc gtc      2784
   Gln Gln Val Lys Ala Glu Glu Ala Pro Glu Lys Val Lys Thr Ala Val
           915                 920                 925
   ctt tcg acc acc gcc cag gcc aag cgc cgt gca cag cgt cgg gag aag      2832
   Leu Ser Thr Thr Ala Gln Ala Lys Arg Arg Ala Gln Arg Arg Glu Lys
       930                 935                 940
   cag caa agg cgc gag agc atg gac att gat cag acc cct acg act ccc      2880
   Gln Gln Arg Arg Glu Ser Met Asp Ile Asp Gln Thr Pro Thr Thr Pro
   945                 950                 955                 960
   aag gtg tcg gac cag gct gac aag atg gat acg gat gag aca gca gtg      2928
   Lys Val Ser Asp Gln Ala Asp Lys Met Asp Thr Asp Glu Thr Ala Val
                   965                 970                 975
   aag acg gaa gaa gag aac aag gag ggt gag aag gag gct ggt gag ggg      2976
   Lys Thr Glu Glu Glu Asn Lys Glu Gly Glu Lys Glu Ala Gly Glu Gly
               980                 985                 990
   cag aag aag aag gtt gag cgt gag  aag gtc gga tat gag  ctc gaa aac    3024
   Gln Lys Lys Lys Val Glu Arg Glu  Lys Val Gly Tyr Glu  Leu Glu Asn
           995                 1000                 1005
   atg tct  cgg gtt ctt ccc gcc  cag ctc aag tac ctc  aca ttc cct       3069
   Met Ser  Arg Val Leu Pro Ala  Gln Leu Lys Tyr Leu  Thr Phe Pro
       1010                 1015                 1020
   gac ccc  cgg tat gag ccg gtc  aag cgg cct acc ggt  gga gtt gtg       3114
   Asp Pro  Arg Tyr Glu Pro Val  Lys Arg Pro Thr Gly  Gly Val Val
       1025                 1030                 1035
   gtc gta  ctt gac aag acc ccc  gag gag ccc cgc gag  acg atc gag       3159
   Val Val  Leu Asp Lys Thr Pro  Glu Glu Pro Arg Glu  Thr Ile Glu
       1040                 1045                 1050
   atg acg  gcc agc aag gag acc  agc agg gag gct aga  cag cct gca       3204
   Met Thr  Ala Ser Lys Glu Thr  Ser Arg Glu Ala Arg  Gln Pro Ala
       1055                 1060                 1065
   ccc gcc  gcg gat gtc gcg gag  tcc ctg cag gac cga  cta cag gcc       3249
   Pro Ala  Ala Asp Val Ala Glu  Ser Leu Gln Asp Arg  Leu Gln Ala
       1070                 1075                 1080
   acc atg  gga gcc gct ggt ctg  cag aca cct cag cgt  gga gct ggg       3294
   Thr Met  Gly Ala Ala Gly Leu  Gln Thr Pro Gln Arg  Gly Ala Gly
       1085                 1090                 1095
   cgc ttc  ccc ttt ggt gcg gat  ctg ggc ccg ttt gcg  ggc gct gcc       3339
   Arg Phe  Pro Phe Gly Ala Asp  Leu Gly Pro Phe Ala  Gly Ala Ala
       1100                 1105                 1110
   gct gct  gcc ggt gta ctg acg  gct gtg gat gag gac  gag gaa ggc       3384
   Ala Ala  Ala Gly Val Leu Thr  Ala Val Asp Glu Asp  Glu Glu Gly
       1115                 1120                 1125
   ggc gag  gag gcg ccg gtg ccg  aat gag ttt gaa tat  gaa acc gag       3429
   Gly Glu  Glu Ala Pro Val Pro  Asn Glu Phe Glu Tyr  Glu Thr Glu
       1130                 1135                 1140
   cca gaa  gac gag tag                                                 3444
   Pro Glu  Asp Glu
       1145
<210> 103
   <211> 1147
   <212> PRT
   <213> Aspergillus niger
<400> 103
   Met Val Gly Leu Ala Ser Ala Ala Gly Leu Val Gly Phe Leu Ser Glu
   1               5                   10                  15
   Pro Asp Pro Glu Leu Arg Val Phe Ala Leu Lys Thr Leu Asp Ser Gln
               20                  25                  30
   Ile Asp Leu Leu Trp Thr Glu Val Val Asp Ala Val Pro Gln Ile Glu
           35                  40                  45
   Ala Leu Tyr Glu Asp Glu Ser Phe Pro Glu Arg Glu Leu Ala Ala Leu
       50                  55                  60
   Val Ala Ala Lys Val Tyr Tyr His Leu Gln Glu Tyr Asn Glu Ser Met
   65                  70                  75                  80
   Val Phe Ala Leu Gly Ala Gly Lys Leu Phe Gln Leu Asp Ser Gly Gly
                   85                  90                  95
   Glu Phe Glu Glu Thr Ile Ile Ala Lys Cys Val Asp Thr Phe Val Ser
               100                 105                 110
   Leu Ser Ala Ala Gln Arg Pro Val Ala Gly Asp His Gln Ala Asn Leu
           115                 120                 125
   Asn Thr Ser Phe Pro Thr Ser Gly Glu Gly Ala Thr Ser Thr Ser Ala
       130                 135                 140
   Ser Leu Thr Ser Pro Ile Thr Pro Phe Ser Gln Ser Ala Leu Pro Ser
   145                 150                 155                 160
   Lys Ser Leu Leu Ser Arg Gln Glu Val Pro Gly Val Asp Ala Ala Tyr
                   165                 170                 175
   Pro Gly Gly Glu Asp Ala Gly Val Asn His Ala Glu Glu Thr Pro Leu
               180                 185                 190
   Val Leu Lys Arg Gly Val Gln Gly His Leu Gln Thr Val Ile Asp Arg
           195                 200                 205
   Leu Phe Glu Gln Cys Phe Val Gln Lys Arg Tyr Arg Gln Val Ile Gly
       210                 215                 220
   Ile Ala Ile Glu Ala Lys Asn Leu Asp Val Leu Arg Lys Thr Ile Leu
   225                 230                 235                 240
   Arg Ala Ser Glu Asp Glu Lys Lys Gln Asn Gly Glu Ser Arg Arg Ser
                   245                 250                 255
   Glu Glu Leu Met Glu Tyr Val Leu Asp Ile Cys Met Gly Ile Val Gln
   260 265 270
   Glu Arg Gly Phe Arg Asn Glu Ile Leu Lys Leu Ile Leu Glu Leu Leu
   275 280 285
   Asn Glu Ile Pro Ala Pro Asp Tyr Phe Ser Ile Ala Lys Cys Val Val
   290 295 300
   Tyr Leu Asn Glu His Ser Met Ala Ser Val Ile Leu Arg Gln Leu Val
   305 310 315 320
   Glu Lys Gly Asp Ala Arg Ser Leu Ala Val Ala Tyr Gln Ile Ser Phe
   325 330 335
   Asp Leu Tyr Asp Asn Ser Thr Gln Glu Phe Leu Gln Lys Val Arg Gln
   340 345 350
   Glu Ile Ser Glu Leu Val Pro Glu Asn Asp Glu Ser Glu Gln Glu Ala
   355 360 365
   Ala Gly Gly Asp Gln Glu Glu Ser Lys Glu Ser Asp Pro Leu Leu Asp
   370 375 380
   Ser Gln Ser Arg Ser Ile Gly Asp Ser Arg Thr Lys Ile Ser Pro Glu
   385 390 395 400
   Ala Arg Thr Ala Phe Lys Ser Ile Arg Asp Ile Leu Asp Gly Val Lys
   405 410 415
   Thr Asn Gln Leu Asn Val Glu Phe Leu Phe Arg Asn Asn Lys Ala Asp
   420 425 430
   Ile Ala Ile Met Asn Lys Leu Arg Asp Ser Leu Glu Ala Arg Asn Ser
   435 440 445
   Ile Phe His Ser Ala Val Ser Leu Ser Asn Gly Phe Met His Ala Gly
   450 455 460
   Thr Ala His Asp Lys Phe Phe Arg Asp Asn Leu Asp Trp Leu Ser Lys
   465 470 475 480
   Ala Val Asn Trp Ser Lys Phe Thr Ala Thr Ala Ala Leu Gly Val Ile
   485 490 495
   His Arg Gly Asn Leu Ser Gln Gly Gln Lys Leu Leu Gln Pro Tyr Leu
   500 505 510
   Pro Lys Glu Arg Ser Ile Thr Asn Val Ala Ala Gly Gly His Ser Gly
   515 520 525
   Ala Ser Ser Ile Tyr Ser Gln Gly Gly Ser Leu Tyr Ala Tyr Gly Leu
   530 535 540
   Ile Phe Ala Asn His Gly Gly Met Ala Val Pro Val Ile Arg Asp Tyr
   545 550 555 560
   Phe Lys Lys Ala Thr Asp Glu Val Ile Gln His Gly Gly Ala Leu Gly
   565 570 575
   Leu Gly Val Ala Gly Met Ala Ser Gly Asp Glu Gly Leu Tyr Glu Asp
   580 585 590
   Leu Arg Asn Val Leu Tyr Thr Asp Ser Ala Ile Asn Gly Glu Ala Val
   595 600 605
   Gly Leu Ala Met Gly Leu Ile Met Leu Gly Thr Gly Asn Met Lys Val
   610 615 620
   Leu Glu Asp Met Ile Gln Tyr Ala His Asp Thr Gln His Glu Lys Ile
   625 630 635 640
   Val Arg Gly Leu Ala Met Gly Met Ala Leu Ile Met Tyr Gly Arg Gln
   645 650 655
   Glu Ala Ala Asp Glu Leu Ile Asn Gly Leu Leu Gly Asp Pro Asp Pro
   660 665 670
   Ala Leu Arg Tyr Gly Gly Ile Met Thr Ile Ala Leu Ala Tyr Cys Gly
   675 680 685
   Ser Ser Ser Asn Lys Ala Val Arg Lys Leu Leu His Val Ala Val Ser
   690 695 700
   Asp Val Asn Asp Asp Val Arg Arg Ile Ala Val Leu Ser Leu Gly Phe
   705 710 715 720
   Ile Leu Phe Arg Lys His Gln Ser Val Pro Arg Met Val Glu Leu Leu
   725 730 735
   Ser Glu Ser Tyr Asn Pro His Val Arg Tyr Gly Ala Ala Met Ala Leu
   740 745 750
   Gly Ile Ser Cys Ala Gly Thr Gly Leu Asp Glu Ala Ile Asp Leu Leu
   755 760 765
   Glu Pro Met Leu Lys Asp Ser Thr Asp Phe Val Arg Gln Gly Ala Leu
   770 775 780
   Ile Ser Leu Ala Met Val Leu Val Gln Gln Asn Glu Ala Met Asn Pro
   785 790 795 800
   Arg Val Ser Ser Leu Arg Lys Val Met Met Lys Met Ile Gly Asp Arg
   805 810 815
   His Glu Asp Ala Met Ala Lys Phe Gly Cys Ala Val Ala Leu Gly Ile
   820 825 830
   Ile Asp Ala Gly Gly Arg Asn Cys Thr Ile Ser Leu Gln Thr Gln Thr
   835 840 845
   Gly Asn Leu Asn Met Pro Gly Ile Val Gly Ala Ala Val Phe Val Gln
   850 855 860
   Tyr Trp Tyr Trp Phe Pro Leu Thr His Phe Leu Ser Leu Ser Phe Ala
   865 870 875 880
   Pro Thr Ser Val Ile Gly Val Asp Gln Lys Leu Glu Val Pro Tyr Phe
   885 890 895
   Lys Phe His Ser Asn Thr Arg Pro Ser Leu Phe Asp Tyr Pro Pro Glu
   900 905 910
   Gln Gln Val Lys Ala Glu Glu Ala Pro Glu Lys Val Lys Thr Ala Val
   915 920 925
   Leu Ser Thr Thr Ala Gln Ala Lys Arg Arg Ala Gln Arg Arg Glu Lys
   930 935 940
   Gln Gln Arg Arg Glu Ser Met Asp Ile Asp Gln Thr Pro Thr Thr Pro
   945 950 955 960
   Lys Val Ser Asp Gln Ala Asp Lys Met Asp Thr Asp Glu Thr Ala Val
   965 970 975
   Lys Thr Glu Glu Glu Asn Lys Glu Gly Glu Lys Glu Ala Gly Glu Gly
   980 985 990
   Gln Lys Lys Lys Val Glu Arg Glu Lys Val Gly Tyr Glu Leu Glu Asn
   995 1000 1005
   Met Ser Arg Val Leu Pro Ala Gln Leu Lys Tyr Leu Thr Phe Pro
   1010 1015 1020
   Asp Pro Arg Tyr Glu Pro Val Lys Arg Pro Thr Gly Gly Val Val
   1025 1030 1035
   Val Val Leu Asp Lys Thr Pro Glu Glu Pro Arg Glu Thr Ile Glu
   1040 1045 1050
   Met Thr Ala Ser Lys Glu Thr Ser Arg Glu Ala Arg Gln Pro Ala
   1055 1060 1065
   Pro Ala Ala Asp Val Ala Glu Ser Leu Gln Asp Arg Leu Gln Ala
   1070 1075 1080
   Thr Met Gly Ala Ala Gly Leu Gln Thr Pro Gln Arg Gly Ala Gly
   1085 1090 1095
   Arg Phe Pro Phe Gly Ala Asp Leu Gly Pro Phe Ala Gly Ala Ala
   1100 1105 1110
   Ala Ala Ala Gly Val Leu Thr Ala Val Asp Glu Asp Glu Glu Gly
   1115 1120 1125
   Gly Glu Glu Ala Pro Val Pro Asn Glu Phe Glu Tyr Glu Thr Glu
   1130 1135 1140
   Pro Glu Asp Glu
   1145
<210> 104
   <211> 3055
   <212> DNA
   <213> Aspergillus niger
<400> 104
   atccgccaga cggcttcata atgatggatc tgttggtgaa agcacagcag cagcagcaga 60
   gggaccagag caccaaaggc gaacccaagc gaggccgaca ggtcggcgat catggcaaat 120
   ataaacccgc gtttgtttcg gacgcccaga tcctcgtcgg tggcttctgc agctccagcc 180
   ccggcgaccg gatactccct ggggaacgag tgagtaacag atccctgatt gcttgagatg 240
   ataataagcg aactcaccca cccgcaccca cgccagcgat tcctcttgcg atgatcagca 300
   tccaaaacat cccatctcga ttcgttccgc tcgcaccagc gctcaaagcg atccctagca 360
   ctaggataac ggtggtcaag actgcgccag tcttccggcc caacctatcg gacaggtatc 420
   cgaaggataa catgccaatg atcataccta ccatgaaggc attggacaga cgagatgcga 480
   cagccgtggt gagagccttt gggtagatga cttccatgat caattcgagg ttgccgatga 540
   tggatgcgtt aaaaccgtcg gagccaatgg ccagaccgga aaacacaatc atcagcaaga 600
   ccgaaagttt ggaagtcttt cgacggcttt gatctggggc cacatcttcg gcggcatctg 660
   atgatatctg cacctgtgtc tgtttggcgg actgagctgc ttccgtcgtc tgtaccgagt 720
   cttgatcctt tttgcgatcc atctcagcgg tcagccttgc gtcttcctcg atggcctaac 780
   agttatctaa acccgactgg accccgccat gatgaggtgt acgtatttat ctctgctgag 840
   ccttgcctat ctgtcccgtc tgtcggccga tccgagcctc ttttctcccg caagcgactg 900
   cgccaacata tctgataagg ccaatgaagc acggcctgtc ccctcctcgg cctgaatggc 960
   ggaccaaccg cgacattcgc cgataagctg cacgtcgcga agcgaatggc cagcgactcc 1020
   aatcaagggg aatccagccg ggcggggaat ctctagctgg catccttcag cgcatcttcc 1080
   cctcttttca aggcctgcag gcgctcggat ctcctccgaa ctcctgcaga agcacggcga 1140
   taaatccatt gtctggaact ctcaaagtta cttgtgcttt cgttcggtct ccgcagcatc 1200
   atgctggaca aacagactga gtctctcccg ctggcggagg agataccgct actccgtcag 1260
   ccaccgcatc aacaccgaaa gactgttctg cgctttataa ctggcgctgg tctactattg 1320
   cttaccatct acacgctctt cccggtgagt atgcttcgcc atcaggatca acggcttgga 1380
   tctaactcca gggcagaggc tatcgccctt tacctccatt aagcaaccct tatccgtctc 1440
   gccatatgaa tccttcgaga gatgttcaat tgagaatctc cgacagacgg gatactattt 1500
   tctggatact gctacgccca tcgcagttga ggagttcgat gagcgccgca accgtctcgc 1560
   gagggctctg gtcgccgatg gggcggacgc ctttgtcgtg gagccggggt atacgtttaa 1620
   atactatgcc aacgtgagcc agccggaatg ggaggtgtgg gaggtcagcg ctctttctcc 1680
   ccagaatgcc atatgtgtac taattccgct cagccggagg aacgcccctt tcttatggtg 1740
   gtgcagcctt atcgtcactc gtcgggccat gttaccgcaa agacaacctt cctagcccca 1800
   tccttcgagg cggagcgagc ccgactgctg aacatgcctt ttctcgaacc gatcgagatc 1860
   attccatggg aggaacactg gaacccatac gacacgctca aaaattccgt catcttctcg 1920
   gctctggatc gagcacctcg actcatggtg gacgaggaaa tgagggactt tatccaacgg 1980
   ggtcttggtc actccgggtt cgaaatcgtc gggctgatgg gagaagtgga gcgagtccgg 2040
   cagatcaaat ccggcaccga agtgggaatc ctccgcgccg tgaacacggg aactgtggaa 2100
   gccgtgcggc agatgcggaa atgttcgtga tccccgactt ggtcgcactg tggacgggct 2160
   gaccctaaat aggcctgtac cccggtctaa ccgaaagtga gatcgcaagt gccctggaca 2220
   atacccttcg ctcggctggt ttggagccat tttttgatat tgtcttgttt ggtatgtgtc 2280
   cgaccgctgc aaatgtgcga ggctgaccgt ggaggtagac gaaaatgccg ccaacccaca 2340
   tggtggtacc aatggctcga aggttctcga gccggaaacc tttgttctga ttgacgtagg 2400
   gtgagcttca tacgcaggat cgcttcccgt ctcgccgttg ttgctgctga ccgatgccag 2460
   cgcccatctc cttgggtact cgtcggatat ttgtcgaaca ttcttcccgc cgttccttga 2520
   gccatcgaaa ccggatatac ccaccccagc acgtttgcga gagaagttgg aggtatacgt 2580
   cattcctcac atgcgcaaga gaggtagttt tattgacttt tgtaggtgtg ggacattgtc 2640
   tttgaagctc aaacccgatc catagggcag ttccaggtga acgccagtgc tgccagcgtc 2700
   gacatcgctg ctcgggaggt gatcacggac ggcgggtatg gggaagcctt cacccatcgt     2760
   gttggccacg gaattggaat caaaggtgag ttgcgacggt ctgtgtgtgg gcattggagg     2820
   tgtgtctgtg gggtcggact catgctgatg tttcgacgta gcccacgaaa gcccctattt     2880
   gaaccagggc aaccatgcca ctctcctcca agccgggatg acgttcacct cggaacctgg     2940
   ggtgtatctg gtggatcgat tcggggtgcg gcacgaggac atctttctgg tgcgggaggg     3000
   tggggaagcc gagctgttga ccggtcgtcg agcatatggg ccttgggacc catag          3055
<210> 105
   <211> 1467
   <212> DNA
   <213> Aspergillus niger
<220>
   <221> CDS
   <222> (1)..(1467)
<400> 105
   atg ctg gac aaa cag act gag tct ctc ccg ctg gcg gag gag ata ccg       48
   Met Leu Asp Lys Gln Thr Glu Ser Leu Pro Leu Ala Glu Glu Ile Pro
   1               5                   10                  15
   cta ctc cgt cag cca ccg cat caa cac cga aag act gtt ctg cgc ttt       96
   Leu Leu Arg Gln Pro Pro His Gln His Arg Lys Thr Val Leu Arg Phe
               20                  25                  30
   ata act ggc gct ggt cta cta ttg ctt acc atc tac acg ctc ttc ccg      144
   Ile Thr Gly Ala Gly Leu Leu Leu Leu Thr Ile Tyr Thr Leu Phe Pro
           35                  40                  45
   agg cta tcg ccc ttt acc tcc att aag caa ccc tta tcc gtc tcg cca      192
   Arg Leu Ser Pro Phe Thr Ser Ile Lys Gln Pro Leu Ser Val Ser Pro
       50                  55                  60
   tat gaa tcc ttc gag aga tgt tca att gag aat ctc cga cag acg gga      240
   Tyr Glu Ser Phe Glu Arg Cys Ser Ile Glu Asn Leu Arg Gln Thr Gly
   65                  70                  75                  80
   tac tat ttt ctg gat act gct acg ccc atc gca gtt gag gag ttc gat      288
   Tyr Tyr Phe Leu Asp Thr Ala Thr Pro Ile Ala Val Glu Glu Phe Asp
                   85                  90                  95
   gag cgc cgc aac cgt ctc gcg agg gct ctg gtc gcc gat ggg gcg gac      336
   Glu Arg Arg Asn Arg Leu Ala Arg Ala Leu Val Ala Asp Gly Ala Asp
               100                 105                 110
   gcc ttt gtc gtg gag ccg ggg tat acg ttt aaa tac tat gcc aac gtg      384
   Ala Phe Val Val Glu Pro Gly Tyr Thr Phe Lys Tyr Tyr Ala Asn Val
           115                 120                 125
   agc cag ccg gaa tgg gag gtg tgg gag ccg gag gaa cgc ccc ttt ctt      432
   Ser Gln Pro Glu Trp Glu Val Trp Glu Pro Glu Glu Arg Pro Phe Leu
       130                 135                 140
   atg gtg gtg cag cct tat cgt cac tcg tcg ggc cat gtt acc gca aag      480
   Met Val Val Gln Pro Tyr Arg His Ser Ser Gly His Val Thr Ala Lys
   145                 150                 155                 160
   aca acc ttc cta gcc cca tcc ttc gag gcg gag cga gcc cga ctg ctg       528
   Thr Thr Phe Leu Ala Pro Ser Phe Glu Ala Glu Arg Ala Arg Leu Leu
                   165                 170                 175
   aac atg cct ttt ctc gaa ccg atc gag atc att cca tgg gag gaa cac       576
   Asn Met Pro Phe Leu Glu Pro Ile Glu Ile Ile Pro Trp Glu Glu His
               180                 185                 190
   tgg aac cca tac gac acg ctc aaa aat tcc gtc atc ttc tcg gct ctg       624
   Trp Asn Pro Tyr Asp Thr Leu Lys Asn Ser Val Ile Phe Ser Ala Leu
           195                 200                 205
   gat cga gca cct cga ctc atg gtg gac gag gaa atg agg gac ttt atc       672
   Asp Arg Ala Pro Arg Leu Met Val Asp Glu Glu Met Arg Asp Phe Ile
       210                 215                 220
   caa cgg ggt ctt ggt cac tcc ggg ttc gaa atc gtc ggg ctg atg gga       720
   Gln Arg Gly Leu Gly His Ser Gly Phe Glu Ile Val Gly Leu Met Gly
   225                 230                 235                 240
   gaa gtg gag cga gtc cgg cag atc aaa tcc ggc acc gaa gtg gga atc       768
   Glu Val Glu Arg Val Arg Gln Ile Lys Ser Gly Thr Glu Val Gly Ile
                   245                 250                 255
   ctc cgc gcc gtg aac acg gga act gtg gaa gcc gtg cgg cag atg cgg       816
   Leu Arg Ala Val Asn Thr Gly Thr Val Glu Ala Val Arg Gln Met Arg
               260                 265                 270
   aaa tgc ctg tac ccc ggt cta acc gaa agt gag atc gca agt gcc ctg       864
   Lys Cys Leu Tyr Pro Gly Leu Thr Glu Ser Glu Ile Ala Ser Ala Leu
           275                 280                 285
   gac aat acc ctt cgc tcg gct ggt ttg gag cca ttt ttt gat att gtc       912
   Asp Asn Thr Leu Arg Ser Ala Gly Leu Glu Pro Phe Phe Asp Ile Val
       290                 295                 300
   ttg ttt gac gaa aat gcc gcc aac cca cat ggt ggt acc aat ggc tcg       960
   Leu Phe Asp Glu Asn Ala Ala Asn Pro His Gly Gly Thr Asn Gly Ser
   305                 310                 315                 320
   aag gtt ctc gag ccg gaa acc ttt gtt ctg att gac gta ggc gcc cat      1008
   Lys Val Leu Glu Pro Glu Thr Phe Val Leu Ile Asp Val Gly Ala His
                   325                 330                 335
   ctc ctt ggg tac tcg tcg gat att tgt cga aca ttc ttc ccg ccg ttc      1056
   Leu Leu Gly Tyr Ser Ser Asp Ile Cys Arg Thr Phe Phe Pro Pro Phe
               340                 345                 350
   ctt gag cca tcg aaa ccg gat ata ccc acc cca gca cgt ttg cga gag      1104
   Leu Glu Pro Ser Lys Pro Asp Ile Pro Thr Pro Ala Arg Leu Arg Glu
           355                 360                 365
   aag ttg gag gtg tgg gac att gtc ttt gaa gct caa acc cga tcc ata      1152
   Lys Leu Glu Val Trp Asp Ile Val Phe Glu Ala Gln Thr Arg Ser Ile
       370                 375                 380
   ggg cag ttc cag gtg aac gcc agt gct gcc agc gtc gac atc gct gct      1200
   Gly Gln Phe Gln Val Asn Ala Ser Ala Ala Ser Val Asp Ile Ala Ala
   385                 390                 395                 400
   cgg gag gtg atc acg gac ggc ggg tat ggg gaa gcc ttc acc cat cgt      1248
   Arg Glu Val Ile Thr Asp Gly Gly Tyr Gly Glu Ala Phe Thr His Arg
                   405                 410                 415
   gtt ggc cac gga att gga atc aaa gcc cac gaa agc ccc tat ttg aac      1296
   Val Gly His Gly Ile Gly Ile Lys Ala His Glu Ser Pro Tyr Leu Asn
               420                 425                 430
   cag ggc aac cat gcc act ctc ctc caa gcc ggg atg acg ttc acc tcg        1344
   Gln Gly Asn His Ala Thr Leu Leu Gln Ala Gly Met Thr Phe Thr Ser
           435                 440                 445
   gaa cct ggg gtg tat ctg gtg gat cga ttc ggg gtg cgg cac gag gac        1392
   Glu Pro Gly Val Tyr Leu Val Asp Arg Phe Gly Val Arg His Glu Asp
       450                 455                 460
   atc ttt ctg gtg cgg gag ggt ggg gaa gcc gag ctg ttg acc ggt cgt        1440
   Ile Phe Leu Val Arg Glu Gly Gly Glu Ala Glu Leu Leu Thr Gly Arg
   465                 470                 475                 480
   cga gca tat ggg cct tgg gac cca tag                                    1467
   Arg Ala Tyr Gly Pro Trp Asp Pro
                   485
<210> 106
   <211> 488
   <212> PRT
   <213> Aspergillus niger
<400> 106
   Met Leu Asp Lys Gln Thr Glu Ser Leu Pro Leu Ala Glu Glu Ile Pro
   1               5                   10                  15
   Leu Leu Arg Gln Pro Pro His Gln His Arg Lys Thr Val Leu Arg Phe
               20                  25                  30
   Ile Thr Gly Ala Gly Leu Leu Leu Leu Thr Ile Tyr Thr Leu Phe Pro
           35                  40                  45
   Arg Leu Ser Pro Phe Thr Ser Ile Lys Gln Pro Leu Ser Val Ser Pro
       50                  55                  60
   Tyr Glu Ser Phe Glu Arg Cys Ser Ile Glu Asn Leu Arg Gln Thr Gly
   65                  70                  75                  80
   Tyr Tyr Phe Leu Asp Thr Ala Thr Pro Ile Ala Val Glu Glu Phe Asp
                   85                  90                  95
   Glu Arg Arg Asn Arg Leu Ala Arg Ala Leu Val Ala Asp Gly Ala Asp
               100                 105                 110
   Ala Phe Val Val Glu Pro Gly Tyr Thr Phe Lys Tyr Tyr Ala Asn Val
           115                 120                 125
   Ser Gln Pro Glu Trp Glu Val Trp Glu Pro Glu Glu Arg Pro Phe Leu
       130                 135                 140
   Met Val Val Gln Pro Tyr Arg His Ser Ser Gly His Val Thr Ala Lys
   145                 150                 155                 160
   Thr Thr Phe Leu Ala Pro Ser Phe Glu Ala Glu Arg Ala Arg Leu Leu
   165 170 175
   Asn Met Pro Phe Leu Glu Pro Ile Glu Ile Ile Pro Trp Glu Glu His
   180 185 190
   Trp Asn Pro Tyr Asp Thr Leu Lys Asn Ser Val Ile Phe Ser Ala Leu
   195 200 205
   Asp Arg Ala Pro Arg Leu Met Val Asp Glu Glu Met Arg Asp Phe Ile
   210 215 220
   Gln Arg Gly Leu Gly His Ser Gly Phe Glu Ile Val Gly Leu Met Gly
   225 230 235 240
   Glu Val Glu Arg Val Arg Gln Ile Lys Ser Gly Thr Glu Val Gly Ile
   245 250 255
   Leu Arg Ala Val Asn Thr Gly Thr Val Glu Ala Val Arg Gln Met Arg
   260 265 270
   Lys Cys Leu Tyr Pro Gly Leu Thr Glu Ser Glu Ile Ala Ser Ala Leu
   275 280 285
   Asp Asn Thr Leu Arg Ser Ala Gly Leu Glu Pro Phe Phe Asp Ile Val
   290 295 300
   Leu Phe Asp Glu Asn Ala Ala Asn Pro His Gly Gly Thr Asn Gly Ser
   305 310 315 320
   Lys Val Leu Glu Pro Glu Thr Phe Val Leu Ile Asp Val Gly Ala His
   325 330 335
   Leu Leu Gly Tyr Ser Ser Asp Ile Cys Arg Thr Phe Phe Pro Pro Phe
   340 345 350
   Leu Glu Pro Ser Lys Pro Asp Ile Pro Thr Pro Ala Arg Leu Arg Glu
   355 360 365
   Lys Leu Glu Val Trp Asp Ile Val Phe Glu Ala Gln Thr Arg Ser Ile
   370 375 380
   Gly Gln Phe Gln Val Asn Ala Ser Ala Ala Ser Val Asp Ile Ala Ala
   385 390 395 400
   Arg Glu Val Ile Thr Asp Gly Gly Tyr Gly Glu Ala Phe Thr His Arg
   405 410 415
   Val Gly His Gly Ile Gly Ile Lys Ala His Glu Ser Pro Tyr Leu Asn
   420 425 430
   Gln Gly Asn His Ala Thr Leu Leu Gln Ala Gly Met Thr Phe Thr Ser
   435 440 445
   Glu Pro Gly Val Tyr Leu Val Asp Arg Phe Gly Val Arg His Glu Asp
   450 455 460
   Ile Phe Leu Val Arg Glu Gly Gly Glu Ala Glu Leu Leu Thr Gly Arg
   465 470 475 480
   Arg Ala Tyr Gly Pro Trp Asp Pro
   485
<210> 107
   <211> 2845
   <212> DNA
   <213> Aspergilllus niger
<400> 107
   cggtccaata gtggcccgct attggatcca cggccggctt ttggcggggc tcttgtcgag 60
   accgtgtatc aaagggtggc cgaaaagcta ttggggtgtg agagacagat taatacggag 120
   tcgttgaggg ccaagtttaa ggagaactga catgtcggtc agtaatggga aaatagataa 180
   aacgagttaa gcgatgtctc agatggtcct gtccaagtct gtccttgtaa tgagtgtggt 240
   gatggaaaga taattgatga tgacgatgtt gatgatgcaa aataagggct tgtccgggtc 300
   tccaggcttt cccctccgac cgaggaacgg gtgaccgagc gatccatgtt tagctggtcg 360
   gtttggtgca gtagctgctg cccccccccc gttgtaccgt aaatgtggta agcgctgctt 420
   ctctccggcc cattgggggg ttcggcgtga aggcgcctag ttgttcaggc cacaaccgta 480
   gctacagctt tccgaatgac taagccccgg ccgccgtgtt cttcccgcaa acacgttcgg 540
   gctcggtttt gaagatcgca taccagccat cgtcttcaga taggctcctc ttagtccaat 600
   ccctcgctgc cgcacccgac ggcttccttt tttgtttgat cacactccct agggtctttt 660
   ttatctagcc agtgcttctt ggagtctgcg agcttgtatt gccgcattta tcctaattct 720
   tttccactgt gaatgagtac ggaattccat ggcaactatt tcccttgcgt gtgaagcgcg 780
   gaattgctga actagccttc tctcaaccct gcagagccag cacagacaac gatcattcta 840
   cgtgactcaa actcgattta ccttttcttc ccctgcgacg ggatgcgaca ttagcttgcc 900
   gccagctaca ctcacgatac tcgctctcaa tcgtatgcga aataggatat ggttacatcc 960
   actccgactc tcaacaactc gtgaagtcaa tccctcggct acaattggca accataagcc 1020
   atattacctc tgatcatctc aatgcgaatg atgatctgta ggaagaatta agttgctttt 1080
   ggagattcgg caaacctcgg cggcaaagac tgccattttt ccagcccgaa aacgtcactc 1140
   tttcgtgctg tgacgatatc atcattccct caacaggtct ctcttttctg aacaatcaca 1200
   atgggacaca tgagctaccc ctcccaaatg gatagcccct ccaggcaact tgtcctggat 1260
   ttagcgcggg accttgaaca gttacgcatg cacaacaccg agttgaagaa ggtcaaagtc 1320
   tacgaacgcc gatccttcta tgagaacctt gaccgaattg atagcgaact tgaggcccag 1380
   cacaatgcgg cccttgacaa ggctgccgct ctgcatgatc gggtactcgt ggaggccgaa 1440
   gagacgttgc gcgcacacca acgagcggta gaggaggagc tccgtcggag agaggaagaa 1500
   gcacggaaag aggcggagcg catagaacga gagaaggcag agaagctacg ccgagagcag 1560
   gaggaggctg cacggcgtga ggctgagcgc agagcagccg aggaggcgaa gaagaaggca 1620
   gaggaagagg cggagaggaa aaagaaggcg gcgcaagaag aacaggagcg gaaagaacga 1680
   gagcgactag aggaagagaa tcgcaagcgc caggaagaga cacaaaaggc agagcaggag 1740
   gcgtcccaac gccaagcgga agccgcgcaa aaggcacagg cggagcgaca gcgacaggtc 1800
   ggaggcgctc gtcttacgga ggaagaaatc aaggtccagg cccgctacgt ggaactgcac 1860
   caacatctca agaagttccg gcagtacttg aaaaatgaag ggaaaacaaa cgccattgtc 1920
   aagcaaaaca tgggcgatat gcggcgaacg attaagaaat gcgttggaca actacgtgaa 1980
   ggcaagggag ccaacaaagc ccaggtaagt gtcttgctgt gcttcccaag taatagaaga 2040
   aggcagcgag ctaacctcgt caactgccaa acagcttcaa gagattcgcg cgacgcttga 2100
   gaaagccgcc tcgattgctg aaccgtcggt ggatgttcgc caattcatcg ctttcccgcc 2160
   cgagaatatt gccaattccg atgacaacaa ggtgcctgcc cttttgatct acgccctgaa 2220
   catcttctcc aagtgtatga tctcttccct cattacggag gcgtccatca acctgggcca 2280
   tgcggagccg gtcgggatcg tcgcggccca gatattctcc gccgatgcct tcatctacaa 2340
   gggctgccac atggtggaca ttctattggc caagtaccgt gttgtttgcc cagcgctgtg 2400
   gggattccat ggcaatgaga agaccgaggg tggccgacgt gccctgggtt ggtggcgcga 2460
   aggaccggac gggccgtaca tcagcgaaca ggcccacgcc gaccgaatga cagctctggg 2520
   tgcgggttat gcggccttga ccctgcgcaa ttttggcaaa accgcgcgca agaacccttt 2580
   ccccaacacc atgttttggt ataccatgca taaaatcctg aatatcccgc cggcagagat 2640
   ccaggatact catgtcaccc tgctttcggc aatgctgaaa tcgtcagccg aaagaattgt 2700
   tggtttcttc gggcatatcg gactggcgct gatgaggaag gctatagtcg aattacccga 2760
   cagcctgccc cggcagagta tgagcgttaa ccaactcaag ctcctcaagg atctgtataa 2820
   acgggagaag aacattttgc tctga 2845
<210> 108
   <211> 1575
   <212> DNA
   <213> Aspergillus niger
<220>
   <221> CDS
   <222> (1)..(1575)
<400> 108
   atg gga cac atg agc tac ccc tcc caa atg gat agc ccc tcc agg caa       48
   Met Gly His Met Ser Tyr Pro Ser Gln Met Asp Ser Pro Ser Arg Gln
   1               5                   10                  15
   ctt gtc ctg gat tta gcg cgg gac ctt gaa cag tta cgc atg cac aac       96
   Leu Val Leu Asp Leu Ala Arg Asp Leu Glu Gln Leu Arg Met His Asn
               20                  25                  30
   acc gag ttg aag aag gtc aaa gtc tac gaa cgc cga tcc ttc tat gag      144
   Thr Glu Leu Lys Lys Val Lys Val Tyr Glu Arg Arg Ser Phe Tyr Glu
           35                  40                  45
   aac ctt gac cga att gat agc gaa ctt gag gcc cag cac aat gcg gcc      192
   Asn Leu Asp Arg Ile Asp Ser Glu Leu Glu Ala Gln His Asn Ala Ala
       50                  55                  60
   ctt gac aag gct gcc gct ctg cat gat cgg gta ctc gtg gag gcc gaa      240
   Leu Asp Lys Ala Ala Ala Leu His Asp Arg Val Leu Val Glu Ala Glu
   65                  70                  75                  80
   gag acg ttg cgc gca cac caa cga gcg gta gag gag gag ctc cgt cgg      288
   Glu Thr Leu Arg Ala His Gln Arg Ala Val Glu Glu Glu Leu Arg Arg
                   85                  90                  95
   aga gag gaa gaa gca cgg aaa gag gcg gag cgc ata gaa cga gag aag      336
   Arg Glu Glu Glu Ala Arg Lys Glu Ala Glu Arg Ile Glu Arg Glu Lys
               100                 105                 110
   gca gag aag cta cgc cga gag cag gag gag gct gca cgg cgt gag gct      384
   Ala Glu Lys Leu Arg Arg Glu Gln Glu Glu Ala Ala Arg Arg Glu Ala
           115                 120                 125
   gag cgc aga gca gcc gag gag gcg aag aag aag gca gag gaa gag gcg      432
   Glu Arg Arg Ala Ala Glu Glu Ala Lys Lys Lys Ala Glu Glu Glu Ala
       130                 135                 140
   gag agg aaa aag aag gcg gcg caa gaa gaa cag gag cgg aaa gaa cga      480
   Glu Arg Lys Lys Lys Ala Ala Gln Glu Glu Gln Glu Arg Lys Glu Arg
   145                 150                 155                 160
   gag cga cta gag gaa gag aat cgc aag cgc cag gaa gag aca caa aag      528
   Glu Arg Leu Glu Glu Glu Asn Arg Lys Arg Gln Glu Glu Thr Gln Lys
                   165                 170                 175
   gca gag cag gag gcg tcc caa cgc caa gcg gaa gcc gcg caa aag gca      576
   Ala Glu Gln Glu Ala Ser Gln Arg Gln Ala Glu Ala Ala Gln Lys Ala
               180                 185                 190
   cag gcg gag cga cag cga cag gtc gga ggc gct cgt ctt acg gag gaa      624
   Gln Ala Glu Arg Gln Arg Gln Val Gly Gly Ala Arg Leu Thr Glu Glu
           195                 200                 205
   gaa atc aag gtc cag gcc cgc tac gtg gaa ctg cac caa cat ctc aag      672
   Glu Ile Lys Val Gln Ala Arg Tyr Val Glu Leu His Gln His Leu Lys
       210                 215                 220
   aag ttc cgg cag tac ttg aaa aat gaa ggg aaa aca aac gcc att gtc      720
   Lys Phe Arg Gln Tyr Leu Lys Asn Glu Gly Lys Thr Asn Ala Ile Val
   225                 230                 235                 240
   aag caa aac atg ggc gat atg cgg cga acg att aag aaa tgc gtt gga       768
   Lys Gln Asn Met Gly Asp Met Arg Arg Thr Ile Lys Lys Cys Val Gly
                   245                 250                 255
   caa cta cgt gaa ggc aag gga gcc aac aaa gcc cag ctt caa gag att       816
   Gln Leu Arg Glu Gly Lys Gly Ala Asn Lys Ala Gln Leu Gln Glu Ile
               260                 265                 270
   cgc gcg acg ctt gag aaa gcc gcc tcg att gct gaa ccg tcg gtg gat       864
   Arg Ala Thr Leu Glu Lys Ala Ala Ser Ile Ala Glu Pro Ser Val Asp
           275                 280                 285
   gtt cgc caa ttc atc gct ttc ccg ccc gag aat att gcc aat tcc gat       912
   Val Arg Gln Phe Ile Ala Phe Pro Pro Glu Asn Ile Ala Asn Ser Asp
       290                 295                 300
   gac aac aag gtg cct gcc ctt ttg atc tac gcc ctg aac atc ttc tcc       960
   Asp Asn Lys Val Pro Ala Leu Leu Ile Tyr Ala Leu Asn Ile Phe Ser
   305                 310                 315                 320
   aag tgt atg atc tct tcc ctc att acg gag gcg tcc atc aac ctg ggc      1008
   Lys Cys Met Ile Ser Ser Leu Ile Thr Glu Ala Ser Ile Asn Leu Gly
                   325                 330                 335
   cat gcg gag ccg gtc ggg atc gtc gcg gcc cag ata ttc tcc gcc gat      1056
   His Ala Glu Pro Val Gly Ile Val Ala Ala Gln Ile Phe Ser Ala Asp
               340                 345                 350
   gcc ttc atc tac aag ggc tgc cac atg gtg gac att cta ttg gcc aag      1104
   Ala Phe Ile Tyr Lys Gly Cys His Met Val Asp Ile Leu Leu Ala Lys
           355                 360                 365
   tac cgt gtt gtt tgc cca gcg ctg tgg gga ttc cat ggc aat gag aag      1152
   Tyr Arg Val Val Cys Pro Ala Leu Trp Gly Phe His Gly Asn Glu Lys
       370                 375                 380
   acc gag ggt ggc cga cgt gcc ctg ggt tgg tgg cgc gaa gga ccg gac      1200
   Thr Glu Gly Gly Arg Arg Ala Leu Gly Trp Trp Arg Glu Gly Pro Asp
   385                 390                 395                 400
   ggg ccg tac atc agc gaa cag gcc cac gcc gac cga atg aca gct ctg      1248
   Gly Pro Tyr Ile Ser Glu Gln Ala His Ala Asp Arg Met Thr Ala Leu
                   405                 410                 415
   ggt gcg ggt tat gcg gcc ttg acc ctg cgc aat ttt ggc aaa acc gcg      1296
   Gly Ala Gly Tyr Ala Ala Leu Thr Leu Arg Asn Phe Gly Lys Thr Ala
               420                 425                 430
   cgc aag aac cct ttc ccc aac acc atg ttt tgg tat acc atg cat aaa      1344
   Arg Lys Asn Pro Phe Pro Asn Thr Met Phe Trp Tyr Thr Met His Lys
           435                 440                 445
   atc ctg aat atc ccg ccg gca gag atc cag gat act cat gtc acc ctg      1392
   Ile Leu Asn Ile Pro Pro Ala Glu Ile Gln Asp Thr His Val Thr Leu
       450                 455                 460
   ctt tcg gca atg ctg aaa tcg tca gcc gaa aga att gtt ggt ttc ttc      1440
   Leu Ser Ala Met Leu Lys Ser Ser Ala Glu Arg Ile Val Gly Phe Phe
   465                 470                 475                 480
   ggg cat atc gga ctg gcg ctg atg agg aag gct ata gtc gaa tta ccc      1488
   Gly His Ile Gly Leu Ala Leu Met Arg Lys Ala Ile Val Glu Leu Pro
                   485                 490                 495
   gac agc ctg ccc cgg cag agt atg agc gtt aac caa ctc aag ctc ctc      1536
   Asp Ser Leu Pro Arg Gln Ser Met Ser Val Asn Gln Leu Lys Leu Leu
               500                 505                 510
   aag gat ctg tat aaa cgg gag aag aac att ttg ctc tga                1575
   Lys Asp Leu Tyr Lys Arg Glu Lys Asn Ile Leu Leu
           515                 520
<210> 109
   <211> 524
   <212> PRT
   <213> Aspergillus niger
<400> 109
   Met Gly His Met Ser Tyr Pro Ser Gln Met Asp Ser Pro Ser Arg Gln
   1               5                   10                  15
   Leu Val Leu Asp Leu Ala Arg Asp Leu Glu Gln Leu Arg Met His Asn
               20                  25                  30
   Thr Glu Leu Lys Lys Val Lys Val Tyr Glu Arg Arg Ser Phe Tyr Glu
           35                  40                  45
   Asn Leu Asp Arg Ile Asp Ser Glu Leu Glu Ala Gln His Asn Ala Ala
       50                  55                  60
   Leu Asp Lys Ala Ala Ala Leu His Asp Arg Val Leu Val Glu Ala Glu
   65                  70                  75                  80
   Glu Thr Leu Arg Ala His Gln Arg Ala Val Glu Glu Glu Leu Arg Arg
                   85                  90                  95
   Arg Glu Glu Glu Ala Arg Lys Glu Ala Glu Arg Ile Glu Arg Glu Lys
               100                 105                 110
   Ala Glu Lys Leu Arg Arg Glu Gln Glu Glu Ala Ala Arg Arg Glu Ala
           115                 120                 125
   Glu Arg Arg Ala Ala Glu Glu Ala Lys Lys Lys Ala Glu Glu Glu Ala
       130                 135                 140
   Glu Arg Lys Lys Lys Ala Ala Gln Glu Glu Gln Glu Arg Lys Glu Arg
   145                 150                 155                 160
   Glu Arg Leu Glu Glu Glu Asn Arg Lys Arg Gln Glu Glu Thr Gln Lys
                   165                 170                 175
   Ala Glu Gln Glu Ala Ser Gln Arg Gln Ala Glu Ala Ala Gln Lys Ala
               180                 185                 190
   Gln Ala Glu Arg Gln Arg Gln Val Gly Gly Ala Arg Leu Thr Glu Glu
           195                 200                 205
   Glu Ile Lys Val Gln Ala Arg Tyr Val Glu Leu His Gln His Leu Lys
   210 215 220
   Lys Phe Arg Gln Tyr Leu Lys Asn Glu Gly Lys Thr Asn Ala Ile Val
   225 230 235 240
   Lys Gln Asn Met Gly Asp Met Arg Arg Thr Ile Lys Lys Cys Val Gly
   245 250 255
   Gln Leu Arg Glu Gly Lys Gly Ala Asn Lys Ala Gln Leu Gln Glu Ile
   260 265 270
   Arg Ala Thr Leu Glu Lys Ala Ala Ser Ile Ala Glu Pro Ser Val Asp
   275 280 285
   Val Arg Gln Phe Ile Ala Phe Pro Pro Glu Asn Ile Ala Asn Ser Asp
   290 295 300
   Asp Asn Lys Val Pro Ala Leu Leu Ile Tyr Ala Leu Asn Ile Phe Ser
   305 310 315 320
   Lys Cys Met Ile Ser Ser Leu Ile Thr Glu Ala Ser Ile Asn Leu Gly
   325 330 335
   His Ala Glu Pro Val Gly Ile Val Ala Ala Gln Ile Phe Ser Ala Asp
   340 345 350
   Ala Phe Ile Tyr Lys Gly Cys His Met Val Asp Ile Leu Leu Ala Lys
   355 360 365
   Tyr Arg Val Val Cys Pro Ala Leu Trp Gly Phe His Gly Asn Glu Lys
   370 375 380
   Thr Glu Gly Gly Arg Arg Ala Leu Gly Trp Trp Arg Glu Gly Pro Asp
   385 390 395 400
   Gly Pro Tyr Ile Ser Glu Gln Ala His Ala Asp Arg Met Thr Ala Leu
   405 410 415
   Gly Ala Gly Tyr Ala Ala Leu Thr Leu Arg Asn Phe Gly Lys Thr Ala
   420 425 430
   Arg Lys Asn Pro Phe Pro Asn Thr Met Phe Trp Tyr Thr Met His Lys
   435 440 445
   Ile Leu Asn Ile Pro Pro Ala Glu Ile Gln Asp Thr His Val Thr Leu
   450 455 460
   Leu Ser Ala Met Leu Lys Ser Ser Ala Glu Arg Ile Val Gly Phe Phe
   465                 470                 475                 480
   Gly His Ile Gly Leu Ala Leu Met Arg Lys Ala Ile Val Glu Leu Pro
                   485                 490                 495
   Asp Ser Leu Pro Arg Gln Ser Met Ser Val Asn Gln Leu Lys Leu Leu
               500                 505                 510
   Lys Asp Leu Tyr Lys Arg Glu Lys Asn Ile Leu Leu
           515                 520
<210> 110
   <211> 2751
   <212> DNA
   <213> Aspergillus niger
<400> 110
   tcgccctggt tctacaccaa tctaccccag tggagcaaga actggctctg gcgcggggac      60
   gatctgtggc atatacggtg ggagcaagtc atgagcttgc agccggcctt tgtccaggta     120
   tggcattcct cggatgatgc tgggcactgc tgacaatgat ttagatcatc agttggaatg     180
   attacggaga atctcactac ataggaccaa tctacgaatc tgggatgccc gatggatctt     240
   ccaagtacgt cttggattgc ccgcacgatg cgtggcgggc actgctgccc cactacattg     300
   ccgcctaccg aggatgcagt ataggcggtc tgtaccaggc gaatcagacc ctacctttgg     360
   aggataagat ggtgtactgg tatcgcctga atccgagtta cgctgggagc gccaatggca     420
   cgacgggcaa tgatcctggc gtgggccagg aggtgatgcc accgagtgaa ctcgcccagg     480
   accgagtatt tgtcagtgct actgtgaaag atgcctgtga gatgtacgtt cagattggat     540
   attcggaacc tacgggaatt caggcgtcgg gtccgggaat tcatcacttt tttgtgccgt     600
   tcaatgggca aacggggccg gtgcgaatag tgatggtccg gaatggctat gaggttgccg     660
   ttgcggttgg accggcaatc acggatgatt gcaaggatgg caatgtcaac tggaacgcct     720
   atgtgggaac gagcgactag gaggagtgta tgggactaca gtagaaagat ggagagatag     780
   gagataaata gtggttcatc ctcggctcgg gccaagtggg acgcagctta tcagtgacgc     840
   agctcccgat caacctggct ggtacataga ccctgggagc tatgcacgcc ctcgtatatt     900
   actgtgaatc ttgagcaatt gctgttgtga ctattccagt taatttcaat gaccataggt     960
   tccttcttgt ttacaatgaa gcccactttt tgccctcgcc cgacggagct ccgggcctca    1020
   gcaacaaaac tggtcacggc gcttatcgga atgatcagcg aagctccgct cagctgacct    1080
   cgtcccttct ccgctcttct ccaattccct ccctctcctc gtcctcctcc tcctcctcaa    1140
   ttgaccactt gactattcca ctacttcatc aaccacctct actaccttct atacagctcc    1200
   atggccaaga agaacatcat gggcctccgt gaggctgcca tggcctatcg tcccagctcg    1260
   tccggccacc tccccgaccc ggtccaggac tactccccag tctctcctgc tcccaccgcg 1320
   caacctacgg cggaaccggg ctccgttaac ccagatgact atgcgaaacc atactgcgaa 1380
   ttcatgactg cgaatccgac catcttccat gctgttgacg gcttcaccaa gcagctggaa 1440
   agcaaaggat acaagcgcct gcccgagcgc gaagcgtgga cctccaagct cgaacgcggc 1500
   gggaaatact attgcactcg gaactccagc gcgtttatcg cgttttccat tggcaaggat 1560
   taccagagtg ggaacggtat ggccatcgtg gccggtcaca tcgacgccct caccgccaag 1620
   ttgaagcctg tgtccaagct ccccacgaag gccggctttc tgcagctcgg ggttgctcca 1680
   tatgctggtg ctctgaacga gacctggtgg gatcgggatc tgtctattgg tggccgtgtc 1740
   ttggtccggg accctagcag cggaaaggtc gagtccaagt tggtcaagct ggactggccg      1800
   atcgcccgta ttcccacctt ggcaccgcac ttcggcgcgc catcccaggg ccccttcaat      1860
   aaggagacac agatggtccc cgttattggt gtggataact ccgatctctt ccagcagcaa 1920
   acgtcatcca ccactagccc gtttgcagca ggctcatttg cctctacgca gccggagaag 1980
   ctcgtcaagg tgatctccaa ggaattggga attacggact acaacaccat tctcaactgg 2040
   gagctggaac tctatgacag tcagccagcg cagctgggtg gtctggagaa ggacctgatc 2100
   tttgcgggac ggattgacga taagctgtgc tgctacgcgg ctcaggaggc tctgatcgcc 2160
   tcgccggaca gcacgtcccc ggggtcagtg aagatggtcg gcatgtttga tgacgaggag 2220
   attggcagtt tgctccgcca gggtgctcgg tccaacttta tgagcagcgt gatggagcgc 2280
   atcgccgagg ccttcgcccc gagctatggt cccaacgtgc tggcgcagac cgtggccaac 2340
   agcttcttcg tctcttcgga cgtcatccat gccgtcaacc ccaacttcct caacgtgtac 2400
   ctggagaacc acgcaccgcg gctgaacgtt ggcgtgtccg tatccgccga ctcgaacggg 2460
   cacatgacga ctgacagtgt gagtcatgct ttcatgaagc gtgttgctga gcgctgcggt 2520
   tcgaccctgc aggtcttcca gatccgcaat gattcccgca gcggcggcac gatcggaccc 2580
   atgaccagct cccggattgg catgcgggcg atcgatgtgg gcattcccca gctcagtatg 2640
   catagtatcc gtgcgacgac gggcagtcgg gaccctggtt tgggagtgca gctgttcaag 2700
   gggttctttg attactttga ggagattgac aaggagtttg ctgactttta g 2751
<210> 111
   <211> 1551
   <212> DNA
   <213> Aspergillus niger
<220>
   <221> CDS
   <222> (1) .. (1551)
<400> 111
   atg gcc aag aag aac atc atg ggc ctc cgt gag gct gcc atg gcc tat         48
   Met Ala Lys Lys Asn Ile Met Gly Leu Arg Glu Ala Ala Met Ala Tyr
   1               5                   10                  15
   cgt ccc agc tcg tcc ggc cac ctc ccc gac ccg gtc cag gac tac tcc      96
   Arg Pro Ser Ser Ser Gly His Leu Pro Asp Pro Val Gln Asp Tyr Ser
           20                  25                  30
   cca gtc tct cct gct ccc acc gcg caa cct acg gcg gaa ccg ggc tcc      144
   Pro Val Ser Pro Ala Pro Thr Ala Gln Pro Thr Ala Glu Pro Gly Ser
           35                  40                  45
   gtt aac cca gat gac tat gcg aaa cca tac tgc gaa ttc atg act gcg      192
   Val Asn Pro Asp Asp Tyr Ala Lys Pro Tyr Cys Glu Phe Met Thr Ala
       50                  55                  60
   aat ccg acc atc ttc cat gct gtt gac ggc ttc acc aag cag ctg gaa      240
   Asn Pro Thr Ile Phe His Ala Val Asp Gly Phe Thr Lys Gln Leu Glu
   65                  70                  75                  80
   agc aaa gga tac aag cgc ctg ccc gag cgc gaa gcg tgg acc tcc aag      288
   Ser Lys Gly Tyr Lys Arg Leu Pro Glu Arg Glu Ala Trp Thr Ser Lys
                   85                  90                  95
   ctc gaa cgc ggc ggg aaa tac tat tgc act cgg aac tcc agc gcg ttt      336
   Leu Glu Arg Gly Gly Lys Tyr Tyr Cys Thr Arg Asn Ser Ser Ala Phe
               100                 105                 110
   atc gcg ttt tcc att ggc aag gat tac cag agt ggg aac ggt atg gcc      384
   Ile Ala Phe Ser Ile Gly Lys Asp Tyr Gln Ser Gly Asn Gly Met Ala
           115                 120                 125
   atc gtg gcc ggt cac atc gac gcc ctc acc gcc aag ttg aag cct gtg      432
   Ile Val Ala Gly His Ile Asp Ala Leu Thr Ala Lys Leu Lys Pro Val
       130                 135                 140
   tcc aag ctc ccc acg aag gcc ggc ttt ctg cag ctc ggg gtt gct cca      480
   Ser Lys Leu Pro Thr Lys Ala Gly Phe Leu Gln Leu Gly Val Ala Pro
   145                 150                 155                 160
   tat gct ggt gct ctg aac gag acc tgg tgg gat cgg gat ctg tct att      528
   Tyr Ala Gly Ala Leu Asn Glu Thr Trp Trp Asp Arg Asp Leu Ser Ile
                   165                 170                 175
   ggt ggc cgt gtc ttg gtc cgg gac cct agc agc gga aag gtc gag tcc      576
   Gly Gly Arg Val Leu Val Arg Asp Pro Ser Ser Gly Lys Val Glu Ser
               180                 185                 190
   aag ttg gtc aag ctg gac tgg ccg atc gcc cgt att ccc acc ttg gca      624
   Lys Leu Val Lys Leu Asp Trp Pro Ile Ala Arg Ile Pro Thr Leu Ala
           195                 200                 205
   ccg cac ttc ggc gcg cca tcc cag ggc ccc ttc aat aag gag aca cag      672
   Pro His Phe Gly Ala Pro Ser Gln Gly Pro Phe Asn Lys Glu Thr Gln
       210                 215                 220
   atg gtc ccc gtt att ggt gtg gat aac tcc gat ctc ttc cag cag caa      720
   Met Val Pro Val Ile Gly Val Asp Asn Ser Asp Leu Phe Gln Gln Gln
   225                 230                 235                 240
   acg tca tcc acc act agc ccg ttt gca gca ggc tca ttt gcc tct acg      768
   Thr Ser Ser Thr Thr Ser Pro Phe Ala Ala Gly Ser Phe Ala Ser Thr
                   245                 250                 255
   cag ccg gag aag ctc gtc aag gtg atc tcc aag gaa ttg gga att acg      816
   Gln Pro Glu Lys Leu Val Lys Val Ile Ser Lys Glu Leu Gly Ile Thr
               260                 265                 270
   gac tac aac acc att ctc aac tgg gag ctg gaa ctc tat gac agt cag        864
   Asp Tyr Asn Thr Ile Leu Asn Trp Glu Leu Glu Leu Tyr Asp Ser Gln
           275                 280                 285
   cca gcg cag ctg ggt ggt ctg gag aag gac ctg atc ttt gcg gga cgg        912
   Pro Ala Gln Leu Gly Gly Leu Glu Lys Asp Leu Ile Phe Ala Gly Arg
       290                 295                 300
   att gac gat aag ctg tgc tgc tac gcg gct cag gag gct ctg atc gcc        960
   Ile Asp Asp Lys Leu Cys Cys Tyr Ala Ala Gln Glu Ala Leu Ile Ala
   305                 310                 315                 320
   tcg ccg gac agc acg tcc ccg ggg tca gtg aag atg gtc ggc atg ttt       1008
   Ser Pro Asp Ser Thr Ser Pro Gly Ser Val Lys Met Val Gly Met Phe
                   325                 330                 335
   gat gac gag gag att ggc agt ttg ctc cgc cag ggt gct cgg tcc aac       1056
   Asp Asp Glu Glu Ile Gly Ser Leu Leu Arg Gln Gly Ala Arg Ser Asn
               340                 345                 350
   ttt atg agc agc gtg atg gag cgc atc gcc gag gcc ttc gcc ccg agc       1104
   Phe Met Ser Ser Val Met Glu Arg Ile Ala Glu Ala Phe Ala Pro Ser
           355                 360                 365
   tat ggt ccc aac gtg ctg gcg cag acc gtg gcc aac agc ttc ttc gtc       1152
   Tyr Gly Pro Asn Val Leu Ala Gln Thr Val Ala Asn Ser Phe Phe Val
       370                 375                 380
   tct tcg gac gtc atc cat gcc gtc aac ccc aac ttc ctc aac gtg tac       1200
   Ser Ser Asp Val Ile His Ala Val Asn Pro Asn Phe Leu Asn Val Tyr
   385                 390                 395                 400
   ctg gag aac cac gca ccg cgg ctg aac gtt ggc gtg tcc gta tcc gcc       1248
   Leu Glu Asn His Ala Pro Arg Leu Asn Val Gly Val Ser Val Ser Ala
                   405                 410                 415
   gac tcg aac ggg cac atg acg act gac agt gtg agt cat gct ttc atg       1296
   Asp Ser Asn Gly His Met Thr Thr Asp Ser Val Ser His Ala Phe Met
               420                 425                 430
   aag cgt gtt gct gag cgc tgc ggt tcg acc ctg cag gtc ttc cag atc       1344
   Lys Arg Val Ala Glu Arg Cys Gly Ser Thr Leu Gln Val Phe Gln Ile
           435                 440                 445
   cgc aat gat tcc cgc agc ggc ggc acg atc gga ccc atg acc agc tcc       1392
   Arg Asn Asp Ser Arg Ser Gly Gly Thr Ile Gly Pro Met Thr Ser Ser
       450                 455                 460
   cgg att ggc atg cgg gcg atc gat gtg ggc att ccc cag ctc agt atg       1440
   Arg Ile Gly Met Arg Ala Ile Asp Val Gly Ile Pro Gln Leu Ser Met
   465                 470                 475                 480
   cat agt atc cgt gcg acg acg ggc agt cgg gac cct ggt ttg gga gtg       1488
   His Ser Ile Arg Ala Thr Thr Gly Ser Arg Asp Pro Gly Leu Gly Val
                   485                 490                 495
   cag ctg ttc aag ggg ttc ttt gat tac ttt gag gag att gac aag gag       1536
   Gln Leu Phe Lys Gly Phe Phe Asp Tyr Phe Glu Glu Ile Asp Lys Glu
               500                 505                 510
   ttt gct gac ttt tag                                                   1551
   Phe Ala Asp Phe
           515
<210> 112
   <211> 516
   <212> PRT
   <213> Aspergillus niger
<400> 112
   Met Ala Lys Lys Asn Ile Met Gly Leu Arg Glu Ala Ala Met Ala Tyr
   1               5                   10                  15
   Arg Pro Ser Ser Ser Gly His Leu Pro Asp Pro Val Gln Asp Tyr Ser
               20                  25                  30
   Pro Val Ser Pro Ala Pro Thr Ala Gln Pro Thr Ala Glu Pro Gly Ser
           35                  40                  45
   Val Asn Pro Asp Asp Tyr Ala Lys Pro Tyr Cys Glu Phe Met Thr Ala
       50                  55                  60
   Asn Pro Thr Ile Phe His Ala Val Asp Gly Phe Thr Lys Gln Leu Glu
   65                  70                  75                  80
   Ser Lys Gly Tyr Lys Arg Leu Pro Glu Arg Glu Ala Trp Thr Ser Lys
                   85                  90                  95
   Leu Glu Arg Gly Gly Lys Tyr Tyr Cys Thr Arg Asn Ser Ser Ala Phe
               100                 105                 110
   Ile Ala Phe Ser Ile Gly Lys Asp Tyr Gln Ser Gly Asn Gly Met Ala
           115                 120                 125
   Ile Val Ala Gly His Ile Asp Ala Leu Thr Ala Lys Leu Lys Pro Val
       130                 135                 140
   Ser Lys Leu Pro Thr Lys Ala Gly Phe Leu Gln Leu Gly Val Ala Pro
   145                 150                 155                 160
   Tyr Ala Gly Ala Leu Asn Glu Thr Trp Trp Asp Arg Asp Leu Ser Ile
                   165                 170                 175
   Gly Gly Arg Val Leu Val Arg Asp Pro Ser Ser Gly Lys Val Glu Ser
               180                 185                 190
   Lys Leu Val Lys Leu Asp Trp Pro Ile Ala Arg Ile Pro Thr Leu Ala
           195                 200                 205
   Pro His Phe Gly Ala Pro Ser Gln Gly Pro Phe Asn Lys Glu Thr Gln
       210                 215                 220
   Met Val Pro Val Ile Gly Val Asp Asn Ser Asp Leu Phe Gln Gln Gln
   225                 230                 235                 240
   Thr Ser Ser Thr Thr Ser Pro Phe Ala Ala Gly Ser Phe Ala Ser Thr
   245 250 255
   Gln Pro Glu Lys Leu Val Lys Val Ile Ser Lys Glu Leu Gly Ile Thr
   260 265 270
   Asp Tyr Asn Thr Ile Leu Asn Trp Glu Leu Glu Leu Tyr Asp Ser Gln
   275 280 285
   Pro Ala Gln Leu Gly Gly Leu Glu Lys Asp Leu Ile Phe Ala Gly Arg
   290 295 300
   Ile Asp Asp Lys Leu Cys Cys Tyr Ala Ala Gln Glu Ala Leu Ile Ala
   305 310 315 320
   Ser Pro Asp Ser Thr Ser Pro Gly Ser Val Lys Met Val Gly Met Phe
   325 330 335
   Asp Asp Glu Glu Ile Gly Ser Leu Leu Arg Gln Gly Ala Arg Ser Asn
   340 345 350
   Phe Met Ser Ser Val Met Glu Arg Ile Ala Glu Ala Phe Ala Pro Ser
   355 360 365
   Tyr Gly Pro Asn Val Leu Ala Gln Thr Val Ala Asn Ser Phe Phe Val
   370 375 380
   Ser Ser Asp Val Ile His Ala Val Asn Pro Asn Phe Leu Asn Val Tyr
   385 390 395 400
   Leu Glu Asn His Ala Pro Arg Leu Asn Val Gly Val Ser Val Ser Ala
   405 410 415
   Asp Ser Asn Gly His Met Thr Thr Asp Ser Val Ser His Ala Phe Met
   420 425 430
   Lys Arg Val Ala Glu Arg Cys Gly Ser Thr Leu Gln Val Phe Gln Ile
   435 440 445
   Arg Asn Asp Ser Arg Ser Gly Gly Thr Ile Gly Pro Met Thr Ser Ser
   450 455 460
   Arg Ile Gly Met Arg Ala Ile Asp Val Gly Ile Pro Gln Leu Ser Met
   465 470 475 480
   His Ser Ile Arg Ala Thr Thr Gly Ser Arg Asp Pro Gly Leu Gly Val
   485 490 495
   Gln Leu Phe Lys Gly Phe Phe Asp Tyr Phe Glu Glu Ile Asp Lys Glu
   500 505 510
   Phe Ala Asp Phe
   515
<210> 113
   <211> 2501
   <212> DNA
   <213> Aspergillus niger
<400> 113
   tcctggtaag ttctctcttc atcttgagct ttctctacaa tgcggggtaa agcgactact 60
   gttatgatgg catatgtcta actccctccg cagttcagcg cctccatgtg gtccgccgag 120
   caggccaacg agatccactc cattgcccgt tcgatcaagc ccgacatcaa gctgcacgct 180
   atccccgagg gtctccaggt cgagcgcggc cccgacgcca ttgttgaata cctttgcgag 240
   aaggtccctc ctctcctgga cgcctaaata tgaggtgtca gtgtaccaac tttaggaata 300
   agcgggggat atcacggtgg atatgacgac aatatctatg tcctgtatat acatttactt 360
   caatgaagct cccagaatag acgatttacg attttgattg ttgaatgtat aaaaggagat 420
   ttacagtaga gtgtgtattc tgacatggca agtatatttg tagaacaacc gagctccctc 480
   ataaaaatca gtggacttga gctagattgg tgcgacgtgg gagtaacagc caagctggcc 540
   cttgcggtgt agaaaaatgt acttccaata aatattgttc tgactgaata accatgttgg 600
   cggcgatgtt catctcagtt tgcagatagt aattaattca cccggattta gtttcccggc 660
   tgttttcgag tcgtacgatc attcagaggc ctaagcgtaa gtagatagta agctgttaca 720
   gcgagagatt ccgatcaata tccgaggtgg aaggtccaac atgaaccggg tggattggta 780
   gtactatcag tagtactaga aacgatgatg caattaatcg atatatattt tgaaccgaga 840
   tcagataaga tccaattact caagccccta caaaccatta ccacaatagc ccaccatcaa 900
   tgcaaggcca gcagtacaac gcagcacaaa ccaaaggcta cctttcagac caaagcaaac 960
   gacaattcac ctccgacata tatacccctg tcaagccaag acacccggat attgctccaa 1020
   gcatgacaat cccattaagc cgacgggaca acggcaaaac cacttgccag ctgcttagtc 1080
   atatacccaa ggcagctgct gatcttcaat gcttaaatgc accaaactca aggtaaaccc 1140
   tcagatcaat cgcattctca gatagcatcg tcatcactat cataatcgcc actcacaatc 1200
   atgctccaac caatccgtac aaccttccga agcgctgccc aaagcgcccg cttattctcc 1260
   acatcacgag gcctccgaac agacctgtcc taccaggtgt ttggccccga gaaaggcgat 1320
   ctggttgatc ggagtcctat catcttcctt catgggctgt ttggctcgaa gcagaataac 1380
   cggggtatta gcaagtatgc atcccttttc cattcaattg ccctcagaag acaaacaccc 1440
   agtgaattgg aaagcgacta accatcatgc agagcattgg cacgtgatct gaaacgtgag 1500
   atcttcacag tggtgtgtaa ctagtgatga agaaaaagaa tttggaatca aagtagtgat 1560
   tcaaatcgtg tttgttctcc aggacctccg caaccatggc caatccttcc acgcccaaga 1620
   acacaactac tccgtcatgg ccgaagacgt gatcaagttc ctccagcagc tgaagctaga 1680
   caaggcggtt ctgatcggcc actcgatgta cgtcatatcc tactactatg tacatgtctc 1740
   tgattcttcc ctctacacac aataccttca gaaccaacag taaagacaaa acatgattaa 1800
   ggcggctaaa ctaacggaaa ataattaggg gcgctaaaac cgccatgaca gttgcccttg 1860
   attcacccaa gcttgtctcg gcccttgtac cggttgacaa cgcccccgtg aatgctcccc 1920
   tgaagagcga tttcggtctc tacgtccgtg gcatgcagca tgttgaggct gcgaatgtga 1980
   cgaagcagtc tgaggctgat gagatcttga agggatatga ggaggtgagt acgagttgtg 2040
   aattgcaaac cgagctcgat ctgtacatat cgatcgacta agctaatatg tgacagtccc 2100
   tccccatccg ccagttcctc ctcaccaacc tggtccgctc gactgaagac caaaccatga 2160
   agttccgtgt cccgctcgcc gtcctcggcg aagccattcc cggtatggct gatttcccat 2220
   accgggagcc gggatctgtc tcgtatgatg gccctacgct gtttgtgagg ggcaccaaga 2280
   gcagatatgt gagcgatgag tctgtgccaa cgattaagaa gttcttcccc aatgctcgca 2340
   tagcggatgt ggaggccggg cattggttga tttcagagaa ccctgaggga tttaggcaag 2400
   gtgagcagtt ctgatctatt tggattgagt aggatggttt gctgacgcgt atgcttttgc 2460
   ttcttgcagc tgttctaaag ttcctccagg aggctccctg a 2501
<210> 114
   <211> 891
   <212> DNA
   <213> Aspergillus niger
<220>
   <221> CDS
   <222> (1) .. (891)
<400> 114
   atg ctc caa cca atc cgt aca acc ttc cga agc gct gcc caa agc gcc      48
   Met Leu Gln Pro Ile Arg Thr Thr Phe Arg Ser Ala Ala Gln Ser Ala
   1               5                   10                  15
   cgc tta ttc tcc aca tca cga ggc ctc cga aca gac ctg tcc tac cag      96
   Arg Leu Phe Ser Thr Ser Arg Gly Leu Arg Thr Asp Leu Ser Tyr Gln
               20                  25                  30
   gtg ttt ggc ccc gag aaa ggc gat ctg gtt gat cgg agt cct atc atc     144
   Val Phe Gly Pro Glu Lys Gly Asp Leu Val Asp Arg Ser Pro Ile Ile
           35                  40                  45
   ttc ctt cat ggg ctg ttt ggc tcg aag cag aat aac cgg ggt att agc     192
   Phe Leu His Gly Leu Phe Gly Ser Lys Gln Asn Asn Arg Gly Ile Ser
       50                  55                  60
   aaa gca ttg gca cgt gat ctg aaa cgt gag atc ttc aca gtg gac ctc     240
   Lys Ala Leu Ala Arg Asp Leu Lys Arg Glu Ile Phe Thr Val Asp Leu
   65                  70                  75                  80
   cgc aac cat ggc caa tcc ttc cac gcc caa gaa cac aac tac tcc gtc     288
   Arg Asn His Gly Gln Ser Phe His Ala Gln Glu His Asn Tyr Ser Val
                   85                  90                  95
   atg gcc gaa gac gtg atc aag ttc ctc cag cag ctg aag cta gac aag     336
   Met Ala Glu Asp Val Ile Lys Phe Leu Gln Gln Leu Lys Leu Asp Lys
               100                 105                 110
   gcg gtt ctg atc ggc cac tcg atg ggc gct aaa acc gcc atg aca gtt     384
   Ala Val Leu Ile Gly His Ser Met Gly Ala Lys Thr Ala Met Thr Val
           115                 120                 125
   gcc ctt gat tca ccc aag ctt gtc tcg gcc ctt gta ccg gtt gac aac     432
   Ala Leu Asp Ser Pro Lys Leu Val Ser Ala Leu Val Pro Val Asp Asn
       130                 135                 140
   gcc ccc gtg aat gct ccc ctg aag agc gat ttc ggt ctc tac gtc cgt     480
   Ala Pro Val Asn Ala Pro Leu Lys Ser Asp Phe Gly Leu Tyr Val Arg
   145                 150                 155                 160
   ggc atg cag cat gtt gag gct gcg aat gtg acg aag cag tct gag gct     528
   Gly Met Gln His Val Glu Ala Ala Asn Val Thr Lys Gln Ser Glu Ala
                   165                 170                 175
   gat gag atc ttg aag gga tat gag gag tcc ctc ccc atc cgc cag ttc     576
   Asp Glu Ile Leu Lys Gly Tyr Glu Glu Ser Leu Pro Ile Arg Gln Phe
               180                 185                 190
   ctc ctc acc aac ctg gtc cgc tcg act gaa gac caa acc atg aag ttc     624
   Leu Leu Thr Asn Leu Val Arg Ser Thr Glu Asp Gln Thr Met Lys Phe
           195                 200                 205
   cgt gtc ccg ctc gcc gtc ctc ggc gaa gcc att ccc ggt atg gct gat     672
   Arg Val Pro Leu Ala Val Leu Gly Glu Ala Ile Pro Gly Met Ala Asp
       210                 215                 220
   ttc cca tac cgg gag ccg gga tct gtc tcg tat gat ggc cct acg ctg     720
   Phe Pro Tyr Arg Glu Pro Gly Ser Val Ser Tyr Asp Gly Pro Thr Leu
   225                 230                 235                 240
   ttt gtg agg ggc acc aag agc aga tat gtg agc gat gag tct gtg cca     768
   Phe Val Arg Gly Thr Lys Ser Arg Tyr Val Ser Asp Glu Ser Val Pro
                   245                 250                 255
   acg att aag aag ttc ttc ccc aat gct cgc ata gcg gat gtg gag gcc     816
   Thr Ile Lys Lys Phe Phe Pro Asn Ala Arg Ile Ala Asp Val Glu Ala
               260                 265                 270
   ggg cat tgg ttg att tca gag aac cct gag gga ttt agg caa gct gtt     864
   Gly His Trp Leu Ile Ser Glu Asn Pro Glu Gly Phe Arg Gln Ala Val
           275                 280                 285
   cta aag ttc ctc cag gag gct ccc tga                                 891
   Leu Lys Phe Leu Gln Glu Ala Pro
       290                 295
<210> 115
   <211> 296
   <212> PRT
   <213> Aspergillus niger
<400> 115
   Met Leu Gln Pro Ile Arg Thr Thr Phe Arg Ser Ala Ala Gln Ser Ala
   1               5                   10                  15
   Arg Leu Phe Ser Thr Ser Arg Gly Leu Arg Thr Asp Leu Ser Tyr Gln
               20                  25                  30
   Val Phe Gly Pro Glu Lys Gly Asp Leu Val Asp Arg Ser Pro Ile Ile
           35                  40                  45
   Phe Leu His Gly Leu Phe Gly Ser Lys Gln Asn Asn Arg Gly Ile Ser
       50                  55                  60
   Lys Ala Leu Ala Arg Asp Leu Lys Arg Glu Ile Phe Thr Val Asp Leu
   65                  70                  75                  80
   Arg Asn His Gly Gln Ser Phe His Ala Gln Glu His Asn Tyr Ser Val
                   85                  90                  95
   Met Ala Glu Asp Val Ile Lys Phe Leu Gln Gln Leu Lys Leu Asp Lys
               100                 105                 110
   Ala Val Leu Ile Gly His Ser Met Gly Ala Lys Thr Ala Met Thr Val
           115                 120                 125
   Ala Leu Asp Ser Pro Lys Leu Val Ser Ala Leu Val Pro Val Asp Asn
       130                 135                 140
   Ala Pro Val Asn Ala Pro Leu Lys Ser Asp Phe Gly Leu Tyr Val Arg
   145                 150                 155                 160
   Gly Met Gln His Val Glu Ala Ala Asn Val Thr Lys Gln Ser Glu Ala
                   165                 170                 175
   Asp Glu Ile Leu Lys Gly Tyr Glu Glu Ser Leu Pro Ile Arg Gln Phe
               180                 185                 190
   Leu Leu Thr Asn Leu Val Arg Ser Thr Glu Asp Gln Thr Met Lys Phe
           195                 200                 205
   Arg Val Pro Leu Ala Val Leu Gly Glu Ala Ile Pro Gly Met Ala Asp
       210                 215                 220
   Phe Pro Tyr Arg Glu Pro Gly Ser Val Ser Tyr Asp Gly Pro Thr Leu
   225                 230                 235                 240
   Phe Val Arg Gly Thr Lys Ser Arg Tyr Val Ser Asp Glu Ser Val Pro
                   245                 250                 255
   Thr Ile Lys Lys Phe Phe Pro Asn Ala Arg Ile Ala Asp Val Glu Ala
   260 265 270
   Gly His Trp Leu Ile Ser Glu Asn Pro Glu Gly Phe Arg Gln Ala Val
   275 280 285
   Leu Lys Phe Leu Gln Glu Ala Pro
   290 295
<210> 116
   <211> 2643
   <212> DNA
   <213> Aspergillus niger
<400> 116
   agcgcattcc catccgcgtg gttcgcatga tggctgagac aaataaagtt ggaggccgaa 60
   ggcggaaggc aggctgaaga gacactcccc caaaacaatg gaacggcggg aatcatttcc 120
   aggggccaaa caaacagcgg atgatctttt catttttctt gtgatgtccg acccgacgga 180
   attgtgggta atatcactga aaatgcacct caatagcata ctaagcgacg acagtccctc 240
   gccactgttc aagtgagcca taggagagaa ttgtcagaag gtaaacgttc gagaatttta 300
   ctcgatgttt ttgcgaggaa aagctcaatc tcattccgag acctgccatt cctggaaggt 360
   caggcacatt cagtgacctt tctagcttct ctggaagaaa gctcactgtc acagttcgat 420
   gcagcatcga tgctcgagaa caattgagcg gtaacagcta tttccactat tgaggaagga 480
   cattcctatt gtatacaatt ggtatgtata gccgcatcac aagcgacacc gatcctacta 540
   gtaccatggt cagtgtttgt tataatgatt ctgtatgtgt gttggcttac gtgacgtaca 600
   ccgcttcatc atgcgcagac atgcgatggc ttgaaatgcc gagatggttg gtgggcgtca 660
   gagcatccga tacatttagc tccgacagac aggatattac ccaagcaagg tagcttgtta 720
   cagaagagaa ccgcaatgaa gcacgcggta gaaaggccta gcagttgagc agatcgatcg 780
   cctgcttgtt cacgactgtt ttcaggccat tgcaagcctc atcagccgat tcgactcgtc 840
   accacgtagg gagaatcatg agtcagctac tcagcaccat aagcgcttaa tggcaactga 900
   gcgctaatca cactcagctc tgcatagggg agagaatagg gattaaacct tacgccgtct 960
   caggcgggat aatgtggctt aggcctcgac acccgagttg gtctagctta ccggctaaaa 1020
   tctcacatac tatcaactga taacgatagc aaaggcagga ttgtgatggc cccgacggac 1080
   tccgggatat gacaaacacc tgctgacagc catttgtccc aacccctctg gggttgatat 1140
   ttggcagctt ccccagtttt tctgctccta ttgacgaagg cccttgttac attcatcatc 1200
   atggtccgcc gaattgcttc agctacacct atggtgcaat cgcccatgtc gccattgggc 1260
   acaacatact gcgtccgtcc taatcctgtt tcactgaatc ttcaaagaag acctctcgtg 1320
   atcgcatcaa cagacgaggc caaggtcact ataatatatg ccggactatt aatccctggc 1380
   gacggagaac ctctgcgcaa tgctgcccta gtcatcagcg ataagatcat cgcgttcgtt 1440
   ggatccgaag ccgacatccc taagaaatac ctccggtcca cgcagtctac tcatcgtgtc 1500
   cccgtgctca tgcctggttt gtgggattgc cacatgcatt ttggcgggga tgacgattat 1560
   tacaacgatt atacatctgg tctggccact catccagcat catcaggtgc tcgactagcc 1620
   cgtggttgct gggaagcatt gcagaatggg tatacatcct accgcgacct agccggatac 1680
   gggtgcgagg tcgcaaaggc gatcaatgat ggcactatcg ttggtccaaa cgtgtactcg 1740
   tctggcgctg cactcagtca gacagctgga cacggcgata tcttcgctct tccagcaggc 1800
   gaagtactgg ggagttatgg agtaatgaac ccacgccctg ggtactgggg ggcagggccg 1860
   ctatgtatcg ccgatggcgt agaggaggtc cgacgagcag tgaggttgca gatccgtcgc 1920
   ggtgcaaagg ttatcaaagt gatggcctct gggggtgtca tgtcgcgaga cgataatccc 1980
   aactttgcac agttctctcc agaagaactg aaggtgatag tggaagaggc ggctcgacag 2040
   aaccggatcg tttctgcaca tgtgcatggc aaggcgggga ttatggctgc tatcaaagca 2100
   ggctgcaaga gtctggagca tgtgtcttat gctgacgagg aggtctggga gctcatgaaa 2160
   gagaagggaa ttttgtatgt ggccacacgc tcggttattg aaatctttct ggctagtaat 2220
   ggagaggggt tggtgaaaga gtcgtgggcc aagttgcagg cccttgccga ttcgcatttg 2280
   aaagcttatc agggagctat taaggcgggt gttaccattg cgttgggaac ggataccgcc 2340
   cccggtggtc ctaccgcact tgagttgcag tttgccgtcg agagaggagg tatgacgccg 2400
   ttggaggcca tcaaagccgc aactgcgaac gctcccctgt cagttggtcc acaagcaccg 2460
   ttgacgggtc agcttcgcga ggggtatgag gcagatgtga ttgcgttgga ggagaatcca 2520
   ttggaggaca tcaaagtctt tcaggagccg aaggcagtta cccacgtctg gaagggaggg 2580
   aaactgttca aaggtccagg tattggtccg tggggagaag atgcacgtaa tccttttctg 2640
   tag 2643
<210> 117
   <211> 1443
   <212> DNA
   <213> Aspergillus niger
<220>
   <221> CDS
   <222> (1) .. (1443)
<400> 117
   atg gtc cgc cga att gct tca gct aca cct atg gtg caa tcg ccc atg        48
   Met Val Arg Arg Ile Ala Ser Ala Thr Pro Met Val Gln Ser Pro Met
   1               5                   10                  15
   tcg cca ttg ggc aca aca tac tgc gtc cgt cct aat cct gtt tca ctg        96
   Ser Pro Leu Gly Thr Thr Tyr Cys Val Arg Pro Asn Pro Val Ser Leu
               20                  25                  30
   aat ctt caa aga aga cct ctc gtg atc gca tca aca gac gag gcc aag       144
   Asn Leu Gln Arg Arg Pro Leu Val Ile Ala Ser Thr Asp Glu Ala Lys
           35                  40                  45
   gtc act ata ata tat gcc gga cta tta atc cct ggc gac gga gaa cct      192
   Val Thr Ile Ile Tyr Ala Gly Leu Leu Ile Pro Gly Asp Gly Glu Pro
       50                  55                  60
   ctg cgc aat gct gcc cta gtc atc agc gat aag atc atc gcg ttc gtt      240
   Leu Arg Asn Ala Ala Leu Val Ile Ser Asp Lys Ile Ile Ala Phe Val
   65                  70                  75                  80
   gga tcc gaa gcc gac atc cct aag aaa tac ctc cgg tcc acg cag tct      288
   Gly Ser Glu Ala Asp Ile Pro Lys Lys Tyr Leu Arg Ser Thr Gln Ser
                   85                  90                  95
   act cat cgt gtc ccc gtg ctc atg cct ggt ttg tgg gat tgc cac atg      336
   Thr His Arg Val Pro Val Leu Met Pro Gly Leu Trp Asp Cys His Met
               100                 105                 110
   cat ttt ggc ggg gat gac gat tat tac aac gat tat aca tct ggt ctg      384
   His Phe Gly Gly Asp Asp Asp Tyr Tyr Asn Asp Tyr Thr Ser Gly Leu
           115                 120                 125
   gcc act cat cca gca tca tca ggt gct cga cta gcc cgt ggt tgc tgg      432
   Ala Thr His Pro Ala Ser Ser Gly Ala Arg Leu Ala Arg Gly Cys Trp
       130                 135                 140
   gaa gca ttg cag aat ggg tat aca tcc tac cgc gac cta gcc gga tac      480
   Glu Ala Leu Gln Asn Gly Tyr Thr Ser Tyr Arg Asp Leu Ala Gly Tyr
   145                 150                 155                 160
   ggg tgc gag gtc gca aag gcg atc aat gat ggc act atc gtt ggt cca      528
   Gly Cys Glu Val Ala Lys Ala Ile Asn Asp Gly Thr Ile Val Gly Pro
                   165                 170                 175
   aac gtg tac tcg tct ggc gct gca ctc agt cag aca gct gga cac ggc      576
   Asn Val Tyr Ser Ser Gly Ala Ala Leu Ser Gln Thr Ala Gly His Gly
               180                 185                 190
   gat atc ttc gct ctt cca gca ggc gaa gta ctg ggg agt tat gga gta      624
   Asp Ile Phe Ala Leu Pro Ala Gly Glu Val Leu Gly Ser Tyr Gly Val
           195                 200                 205
   atg aac cca cgc cct ggg tac tgg ggg gca ggg ccg cta tgt atc gcc      672
   Met Asn Pro Arg Pro Gly Tyr Trp Gly Ala Gly Pro Leu Cys Ile Ala
       210                 215                 220
   gat ggc gta gag gag gtc cga cga gca gtg agg ttg cag atc cgt cgc      720
   Asp Gly Val Glu Glu Val Arg Arg Ala Val Arg Leu Gln Ile Arg Arg
   225                 230                 235                 240
   ggt gca aag gtt atc aaa gtg atg gcc tct ggg ggt gtc atg tcg cga      768
   Gly Ala Lys Val Ile Lys Val Met Ala Ser Gly Gly Val Met Ser Arg
                   245                 250                 255
   gac gat aat ccc aac ttt gca cag ttc tct cca gaa gaa ctg aag gtg      816
   Asp Asp Asn Pro Asn Phe Ala Gln Phe Ser Pro Glu Glu Leu Lys Val
               260                 265                 270
   ata gtg gaa gag gcg gct cga cag aac cgg atc gtt tct gca cat gtg      864
   Ile Val Glu Glu Ala Ala Arg Gln Asn Arg Ile Val Ser Ala His Val
           275                 280                 285
   cat ggc aag gcg ggg att atg gct gct atc aaa gca ggc tgc aag agt      912
   His Gly Lys Ala Gly Ile Met Ala Ala Ile Lys Ala Gly Cys Lys Ser
       290                 295                 300
   ctg gag cat gtg tct tat gct gac gag gag gtc tgg gag ctc atg aaa      960
   Leu Glu His Val Ser Tyr Ala Asp Glu Glu Val Trp Glu Leu Met Lys
   305                 310                 315                 320
   gag aag gga att ttg tat gtg gcc aca cgc tcg gtt att gaa atc ttt     1008
   Glu Lys Gly Ile Leu Tyr Val Ala Thr Arg Ser Val Ile Glu Ile Phe
                   325                 330                 335
   ctg gct agt aat gga gag ggg ttg gtg aaa gag tcg tgg gcc aag ttg     1056
   Leu Ala Ser Asn Gly Glu Gly Leu Val Lys Glu Ser Trp Ala Lys Leu
               340                 345                 350
   cag gcc ctt gcc gat tcg cat ttg aaa gct tat cag gga gct att aag     1104
   Gln Ala Leu Ala Asp Ser His Leu Lys Ala Tyr Gln Gly Ala Ile Lys
           355                 360                 365
   gcg ggt gtt acc att gcg ttg gga acg gat acc gcc ccc ggt ggt cct     1152
   Ala Gly Val Thr Ile Ala Leu Gly Thr Asp Thr Ala Pro Gly Gly Pro
       370                 375                 380
   acc gca ctt gag ttg cag ttt gcc gtc gag aga gga ggt atg acg ccg     1200
   Thr Ala Leu Glu Leu Gln Phe Ala Val Glu Arg Gly Gly Met Thr Pro
   385                 390                 395                 400
   ttg gag gcc atc aaa gcc gca act gcg aac gct ccc ctg tca gtt ggt     1248
   Leu Glu Ala Ile Lys Ala Ala Thr Ala Asn Ala Pro Leu Ser Val Gly
                   405                 410                 415
   cca caa gca ccg ttg acg ggt cag ctt cgc gag ggg tat gag gca gat     1296
   Pro Gln Ala Pro Leu Thr Gly Gln Leu Arg Glu Gly Tyr Glu Ala Asp
               420                 425                 430
   gtg att gcg ttg gag gag aat cca ttg gag gac atc aaa gtc ttt cag     1344
   Val Ile Ala Leu Glu Glu Asn Pro Leu Glu Asp Ile Lys Val Phe Gln
           435                 440                 445
   gag ccg aag gca gtt acc cac gtc tgg aag gga ggg aaa ctg ttc aaa     1392
   Glu Pro Lys Ala Val Thr His Val Trp Lys Gly Gly Lys Leu Phe Lys
       450                 455                 460
   ggt cca ggt att ggt ccg tgg gga gaa gat gca cgt aat cct ttt ctg     1440
   Gly Pro Gly Ile Gly Pro Trp Gly Glu Asp Ala Arg Asn Pro Phe Leu
   465                 470                 475                 480
   tag                                                                 1443
<210> 118
   <211> 480
   <212> PRT
   <213> Aspergillus niger
<400> 118
   Met Val Arg Arg Ile Ala Ser Ala Thr Pro Met Val Gln Ser Pro Met
   1               5                   10                  15
   Ser Pro Leu Gly Thr Thr Tyr Cys Val Arg Pro Asn Pro Val Ser Leu
               20                  25                  30
   Asn Leu Gln Arg Arg Pro Leu Val Ile Ala Ser Thr Asp Glu Ala Lys
           35                  40                  45
   Val Thr Ile Ile Tyr Ala Gly Leu Leu Ile Pro Gly Asp Gly Glu Pro
   50 55 60
   Leu Arg Asn Ala Ala Leu Val Ile Ser Asp Lys Ile Ile Ala Phe Val
   65 70 75 80
   Gly Ser Glu Ala Asp Ile Pro Lys Lys Tyr Leu Arg Ser Thr Gln Ser
   85 90 95
   Thr His Arg Val Pro Val Leu Met Pro Gly Leu Trp Asp Cys His Met
   100 105 110
   His Phe Gly Gly Asp Asp Asp Tyr Tyr Asn Asp Tyr Thr Ser Gly Leu
   115 120 125
   Ala Thr His Pro Ala Ser Ser Gly Ala Arg Leu Ala Arg Gly Cys Trp
   130 135 140
   Glu Ala Leu Gln Asn Gly Tyr Thr Ser Tyr Arg Asp Leu Ala Gly Tyr
   145 150 155 160
   Gly Cys Glu Val Ala Lys Ala Ile Asn Asp Gly Thr Ile Val Gly Pro
   165 170 175
   Asn Val Tyr Ser Ser Gly Ala Ala Leu Ser Gln Thr Ala Gly His Gly
   180 185 190
   Asp Ile Phe Ala Leu Pro Ala Gly Glu Val Leu Gly Ser Tyr Gly Val
   195 200 205
   Met Asn Pro Arg Pro Gly Tyr Trp Gly Ala Gly Pro Leu Cys Ile Ala
   210 215 220
   Asp Gly Val Glu Glu Val Arg Arg Ala Val Arg Leu Gln Ile Arg Arg
   225 230 235 240
   Gly Ala Lys Val Ile Lys Val Met Ala Ser Gly Gly Val Met Ser Arg
   245 250 255
   Asp Asp Asn Pro Asn Phe Ala Gln Phe Ser Pro Glu Glu Leu Lys Val
   260 265 270
   Ile Val Glu Glu Ala Ala Arg Gln Asn Arg Ile Val Ser Ala His Val
   275 280 285
   His Gly Lys Ala Gly Ile Met Ala Ala Ile Lys Ala Gly Cys Lys Ser
   290 295 300
   Leu Glu His Val Ser Tyr Ala Asp Glu Glu Val Trp Glu Leu Met Lys
   305 310 315 320
   Glu Lys Gly Ile Leu Tyr Val Ala Thr Arg Ser Val Ile Glu Ile Phe
   325 330 335
   Leu Ala Ser Asn Gly Glu Gly Leu Val Lys Glu Ser Trp Ala Lys Leu
   340 345 350
   Gln Ala Leu Ala Asp Ser His Leu Lys Ala Tyr Gln Gly Ala Ile Lys
   355 360 365
   Ala Gly Val Thr Ile Ala Leu Gly Thr Asp Thr Ala Pro Gly Gly Pro
   370 375 380
   Thr Ala Leu Glu Leu Gln Phe Ala Val Glu Arg Gly Gly Met Thr Pro
   385 390 395 400
   Leu Glu Ala Ile Lys Ala Ala Thr Ala Asn Ala Pro Leu Ser Val Gly
   405 410 415
   Pro Gln Ala Pro Leu Thr Gly Gln Leu Arg Glu Gly Tyr Glu Ala Asp
   420 425 430
   Val Ile Ala Leu Glu Glu Asn Pro Leu Glu Asp Ile Lys Val Phe Gln
   435 440 445
   Glu Pro Lys Ala Val Thr His Val Trp Lys Gly Gly Lys Leu Phe Lys
   450 455 460
   Gly Pro Gly Ile Gly Pro Trp Gly Glu Asp Ala Arg Asn Pro Phe Leu
   465 470 475 480
<210> 119
   <211> 2796
   <212> DNA
   <213> Aspergillus niger
<400> 119
   tcgaggattt cgtagtaggt tggttcgcgc gccgaggagt agggtttggg ggatgatgag     60
   gtgaatgtgc ggtatagtgg tttggtatag ggtgtggtgg tggtcttggt ggtgatggtg    120
   gtgtgtcgga ggcgggtggc cgtgggccgt agacgtggga gggtcatgat tctttttgac    180
   ggttattttg tgttggtttc tttgggaatg atgtgttaca tttaggtaga gggtgaaatt    240
   aaggggaaat ggagggtgcg attgaatgga gggagagttg ttggaggtgc gactttatcg    300
   ggataagatg agaggaagga aatccgccgc tcttcggtaa ggcggactgc gtcatcggtc    360
   ggggtttatc tgatctgatc tggtggtgga cctctgcttc tgagtacttc gtaggatata     420
   ctttgtacta cgttgtatgg tgttatgctt gggtgtagat tgtgtatctt gatctcggaa    480
   taatgatgag atgatctata tagtggtttt atcatttaat ttcttttctt tcttgttctg 540
   gtagtttatt ggactggtgt gcatgtatcg acattggttc attaatggtg gttttggaca 600
   tcattgtatg aatgtactgc ttagcccatc tcactagtcc attgggtatt tcgtatgcat 660
   atttctcttt cttattatct tatacatttg aatggtccag ttaggcaggt tcctgcaggc 720
   ttcatggtgc acgcgaccat gtgcaatgtt agcgcacact gatacaacct atacatatag 780
   gtctcctttc ataactggtt tatagtgatt agtactttga aggcactcga tcaggagctg 840
   caagttgtac ctgtacctga agcttctcca cactacatcg aattagacaa tcagcatggg 900
   ctaagatact aaccagttgg cgccgcggat ccctgaaagc agccaaagtc aacctcaggc 960
   ttggcattga ccgtcttttc cccgacggtt gccgcttgcc ctcatttttc tttcccctcc 1020
   atcagcccac caccaaccca ccccggtttg ccagcttttc aacatggctg cccaggctag 1080
   cgagaagctg cagaagctcg accgtaagtc actgaacgat cccgttgctg atctccgaac 1140
   cctcctttgc catccgcttg cagtaagctg ataagatttc cccgctcgct ctctagtcaa 1200
   cggtcaaagc ggcgacgcgg aggcggacgc tcctgccgct ggtcaaacac aagccggaga 1260
   ggcggaggac gactccgatg acgatgaagt agaggatgga aatgctgccg gcgagggtgc 1320
   agccagcgga ggtgcgcttt ctccacggat acctacccct cgggcaaatt ccagaattct 1380
   ccccgcactg accaaatccg cggattttca gccgccaaga aaaagaagaa gcgcaagtcc 1440
   aagaagaaga agaagggtgg cgccaaggtc caatcctctc ctccgcgcgt tcccctgtcg 1500
   actctgttcg ccggaaagga ataccccgag ggtgagatcg tcgagtacca gaatgagaat 1560
   tcgtaccgca ccaccaacga ggagaagcgc tacctcgacc gcatgaagaa tgacttcctg 1620
   caggagtacc gtcaggctgc tgaggtgcac cgtcaggttc gtcagtatgc gcagaagacc 1680
   attaagcctg gtcagaccct caccgagatc gcggagggca ttgaagaatc cgtccgtgcc 1740
   ttgactggtc accagggtct tgaggaaggt gataacctca agggtggtat gggtttcccc 1800
   tgtggtctca gcattaacca ctgcgctgcc cactacaccc ccaacgctgg caacaagatg 1860
   gtgttgcagc agggtgatgt catgaaggtc gatttcggtg cccacatcaa cggccgcatc 1920
   gttgacagtg ctttcaccgt ggcttttgac ccggtgtatg accctctctt ggcggccgtc 1980
   aaggatgcta ccaacactgg tatccgcgta tgttctacct ttctgtccta atactgggtt 2040
   tctggttact aacagctgca tgtaggaagc gggtattgac gtccgcatga gcgacattgg 2100
   tgccgctatc caggaagcca tggagagtta cgaagttgaa atcaacggga ctatgtatcc 2160
   cgtcaagtgt atccggaacc tcaacggtca caacattgac cagcacatta tccatggtgg 2220
   caagagcgtt ccgattgtca agggtggcga tcagaccaag atggaggagg gtgaagtgtt 2280
   tgccattgag accttcggca gtaccggaaa gggctacgtg agagaggatg taagttgttg 2340
   acatcgattg gccaatatgt tcaatttgct aattgtgata tgaagatgga aacctctcac 2400
   tacgctttga tccctgatca cagccaggtc cccctgcgtc tctcttcggc gaagaacttg 2460
   ttgaacgtca tcaacaagaa cttcggtact cttcccttct gtcgccgtta cctcgaccgg 2520
   ttgggccagg acaagtacct ccttggggtg agtggagcct ctgcgtcgta aagtgtcaat 2580
   gctaatcagc ttgtgcagtt gaacaacctg gtttcgtccg gaattgtcca ggactacccg 2640
   cctctttgcg atatcaaggg ctcctacact gcccagtatg aacatgtagg ttatctcaca 2700
   atctcaatct ccatggccac gcagctaacc agatgacaga cgattgtcct tcgacccaat 2760
   gtaaaggaag tcatcagtcg cggcgacgac tactaa 2796
<210> 120
   <211> 1296
   <212> DNA
   <213> Aspergillus niger
<220>
   <221> CDS
   <222> (1) .. (1296)
<400> 120
   atg gct gcc cag gct agc gag aag ctg cag aag ctc gac ctc aac ggt        48
   Met Ala Ala Gln Ala Ser Glu Lys Leu Gln Lys Leu Asp Leu Asn Gly
   1               5                   10                  15
   caa agc ggc gac gcg gag gcg gac gct cct gcc gct ggt caa aca caa        96
   Gln Ser Gly Asp Ala Glu Ala Asp Ala Pro Ala Ala Gly Gln Thr Gln
               20                  25                  30
   gcc gga gag gcg gag gac gac tcc gat gac gat gaa gta gag gat gga       144
   Ala Gly Glu Ala Glu Asp Asp Ser Asp Asp Asp Glu Val Glu Asp Gly
           35                  40                  45
   aat gct gcc ggc gag ggt gca gcc agc gga ggt ggc gcc aag gtc caa       192
   Asn Ala Ala Gly Glu Gly Ala Ala Ser Gly Gly Gly Ala Lys Val Gln
       50                  55                  60
   tcc tct cct ccg cgc gtt ccc ctg tcg act ctg ttc gcc gga aag gaa       240
   Ser Ser Pro Pro Arg Val Pro Leu Ser Thr Leu Phe Ala Gly Lys Glu
   65                  70                  75                  80
   tac ccc gag ggt gag atc gtc gag tac cag aat gag aat tcg tac cgc       288
   Tyr Pro Glu Gly Glu Ile Val Glu Tyr Gln Asn Glu Asn Ser Tyr Arg
                   85                  90                  95
   acc acc aac gag gag aag cgc tac ctc gac cgc atg aag aat gac ttc       336
   Thr Thr Asn Glu Glu Lys Arg Tyr Leu Asp Arg Met Lys Asn Asp Phe
               100                 105                 110
   ctg cag gag tac cgt cag gct gct gag gtg cac cgt cag gtt cgt cag       384
   Leu Gln Glu Tyr Arg Gln Ala Ala Glu Val His Arg Gln Val Arg Gln
           115                 120                 125
   tat gcg cag aag acc att aag cct ggt cag acc ctc acc gag atc gcg       432
   Tyr Ala Gln Lys Thr Ile Lys Pro Gly Gln Thr Leu Thr Glu Ile Ala
       130                 135                 140
   gag ggc att gaa gaa tcc gtc cgt gcc ttg act ggt cac cag ggt ctt       480
   Glu Gly Ile Glu Glu Ser Val Arg Ala Leu Thr Gly His Gln Gly Leu
   145                 150                 155                 160
   gag gaa ggt gat aac ctc aag ggt ggt atg ggt ttc ccc tgt ggt ctc      528
   Glu Glu Gly Asp Asn Leu Lys Gly Gly Met Gly Phe Pro Cys Gly Leu
                   165                 170                 175
   agc att aac cac tgc gct gcc cac tac acc ccc aac gct ggc aac aag      576
   Ser Ile Asn His Cys Ala Ala His Tyr Thr Pro Asn Ala Gly Asn Lys
               180                 185                 190
   atg gtg ttg cag cag ggt gat gtc atg aag gtc gat ttc ggt gcc cac      624
   Met Val Leu Gln Gln Gly Asp Val Met Lys Val Asp Phe Gly Ala His
           195                 200                 205
   atc aac ggc cgc atc gtt gac agt gct ttc acc gtg gct ttt gac ccg      672
   Ile Asn Gly Arg Ile Val Asp Ser Ala Phe Thr Val Ala Phe Asp Pro
       210                 215                 220
   gtg tat gac cct ctc ttg gcg gcc gtc aag gat gct acc aac act ggt      720
   Val Tyr Asp Pro Leu Leu Ala Ala Val Lys Asp Ala Thr Asn Thr Gly
   225                 230                 235                 240
   atc cgc gaa gcg ggt att gac gtc cgc atg agc gac att ggt gcc gct      768
   Ile Arg Glu Ala Gly Ile Asp Val Arg Met Ser Asp Ile Gly Ala Ala
                   245                 250                 255
   atc cag gaa gcc atg gag agt tac gaa gtt gaa atc aac ggg act atg      816
   Ile Gln Glu Ala Met Glu Ser Tyr Glu Val Glu Ile Asn Gly Thr Met
               260                 265                 270
   tat ccc gtc aag tgt atc cgg aac ctc aac ggt cac aac att gac cag      864
   Tyr Pro Val Lys Cys Ile Arg Asn Leu Asn Gly His Asn Ile Asp Gln
           275                 280                 285
   cac att atc cat ggt ggc aag agc gtt ccg att gtc aag ggt ggc gat      912
   His Ile Ile His Gly Gly Lys Ser Val Pro Ile Val Lys Gly Gly Asp
       290                 295                 300
   cag acc aag atg gag gag ggt gaa gtg ttt gcc att gag acc ttc ggc      960
   Gln Thr Lys Met Glu Glu Gly Glu Val Phe Ala Ile Glu Thr Phe Gly
   305                 310                 315                 320
   agt acc gga aag ggc tac gtg aga gag gat atg gaa acc tct cac tac     1008
   Ser Thr Gly Lys Gly Tyr Val Arg Glu Asp Met Glu Thr Ser His Tyr
                   325                 330                 335
   gct ttg atc cct gat cac agc cag gtc ccc ctg cgt ctc tct tcg gcg     1056
   Ala Leu Ile Pro Asp His Ser Gln Val Pro Leu Arg Leu Ser Ser Ala
               340                 345                 350
   aag aac ttg ttg aac gtc atc aac aag aac ttc ggt act ctt ccc ttc     1104
   Lys Asn Leu Leu Asn Val Ile Asn Lys Asn Phe Gly Thr Leu Pro Phe
           355                 360                 365
   tgt cgc cgt tac ctc gac cgg ttg ggc cag gac aag tac ctc ctt ggg     1152
   Cys Arg Arg Tyr Leu Asp Arg Leu Gly Gln Asp Lys Tyr Leu Leu Gly
       370                 375                 380
   ttg aac aac ctg gtt tcg tcc gga att gtc cag gac tac ccg cct ctt     1200
   Leu Asn Asn Leu Val Ser Ser Gly Ile Val Gln Asp Tyr Pro Pro Leu
   385                 390                 395                 400
   tgc gat atc aag ggc tcc tac act gcc cag tat gaa cat acg att gtc     1248
   Cys Asp Ile Lys Gly Ser Tyr Thr Ala Gln Tyr Glu His Thr Ile Val
                   405                 410                 415
   ctt cga ccc aat gta aag gaa gtc atc agt cgc ggc gac gac tac taa        1296
   Leu Arg Pro Asn Val Lys Glu Val Ile Ser Arg Gly Asp Asp Tyr
               420                 425                 430
<210> 121
   <211> 431
   <212> PRT
   <213> Aspergillus niger
<400> 121
   Met Ala Ala Gln Ala Ser Glu Lys Leu Gln Lys Leu Asp Leu Asn Gly
   1               5                   10                  15
   Gln Ser Gly Asp Ala Glu Ala Asp Ala Pro Ala Ala Gly Gln Thr Gln
               20                  25                  30
   Ala Gly Glu Ala Glu Asp Asp Ser Asp Asp Asp Glu Val Glu Asp Gly
           35                  40                  45
   Asn Ala Ala Gly Glu Gly Ala Ala Ser Gly Gly Gly Ala Lys Val Gln
       50                  55                  60
   Ser Ser Pro Pro Arg Val Pro Leu Ser Thr Leu Phe Ala Gly Lys Glu
   65                  70                  75                  80
   Tyr Pro Glu Gly Glu Ile Val Glu Tyr Gln Asn Glu Asn Ser Tyr Arg
                   85                  90                  95
   Thr Thr Asn Glu Glu Lys Arg Tyr Leu Asp Arg Met Lys Asn Asp Phe
               100                 105                 110
   Leu Gln Glu Tyr Arg Gln Ala Ala Glu Val His Arg Gln Val Arg Gln
           115                 120                 125
   Tyr Ala Gln Lys Thr Ile Lys Pro Gly Gln Thr Leu Thr Glu Ile Ala
       130                 135                 140
   Glu Gly Ile Glu Glu Ser Val Arg Ala Leu Thr Gly His Gln Gly Leu
   145                 150                 155                 160
   Glu Glu Gly Asp Asn Leu Lys Gly Gly Met Gly Phe Pro Cys Gly Leu
                   165                 170                 175
   Ser Ile Asn His Cys Ala Ala His Tyr Thr Pro Asn Ala Gly Asn Lys
               180                 185                 190
   Met Val Leu Gln Gln Gly Asp Val Met Lys Val Asp Phe Gly Ala His
           195                 200                 205
   Ile Asn Gly Arg Ile Val Asp Ser Ala Phe Thr Val Ala Phe Asp Pro
   210 215 220
   Val Tyr Asp Pro Leu Leu Ala Ala Val Lys Asp Ala Thr Asn Thr Gly
   225 230 235 240
   Ile Arg Glu Ala Gly Ile Asp Val Arg Met Ser Asp Ile Gly Ala Ala
   245 250 255
   Ile Gln Glu Ala Met Glu Ser Tyr Glu Val Glu Ile Asn Gly Thr Met
   260 265 270
   Tyr Pro Val Lys Cys Ile Arg Asn Leu Asn Gly His Asn Ile Asp Gln
   275 280 285
   His Ile Ile His Gly Gly Lys Ser Val Pro Ile Val Lys Gly Gly Asp
   290 295 300
   Gln Thr Lys Met Glu Glu Gly Glu Val Phe Ala Ile Glu Thr Phe Gly
   305 310 315 320
   Ser Thr Gly Lys Gly Tyr Val Arg Glu Asp Met Glu Thr Ser His Tyr
   325 330 335
   Ala Leu Ile Pro Asp His Ser Gln Val Pro Leu Arg Leu Ser Ser Ala
   340 345 350
   Lys Asn Leu Leu Asn Val Ile Asn Lys Asn Phe Gly Thr Leu Pro Phe
   355 360 365
   Cys Arg Arg Tyr Leu Asp Arg Leu Gly Gln Asp Lys Tyr Leu Leu Gly
   370 375 380
   Leu Asn Asn Leu Val Ser Ser Gly Ile Val Gln Asp Tyr Pro Pro Leu
   385 390 395 400
   Cys Asp Ile Lys Gly Ser Tyr Thr Ala Gln Tyr Glu His Thr Ile Val
   405 410 415
   Leu Arg Pro Asn Val Lys Glu Val Ile Ser Arg Gly Asp Asp Tyr
   420 425 430
<210> 122
   <211> 2968
   <212> DNA
   <213> Aspergillus niger
<400> 122
   tcttcgcgcg ggcggagaaa gagcgcgtag gcaaagtagg cgatccaggc ggcaagcaag 60
   agaaggaaaa acgtattttg tcggcggcgt tcccgaagag cgaggtactg agctcgcagg 120
   gagctctcga gaatgaggag attgaggtat atctggggtg gggaagaggg gagggtcgac 180
   agtggatcgg gggttgacga ccgtggcggg gaagagggat cgctagaggg aggatgggag 240
   tgaaatgcag gcgccaatga ttcggaggag gatgtcgcag aagatgcaag gcgactgttg 300
   ggtgctggag atcccttaac cagttggtct aggcttgctg aagccatggt gagctttgga 360
   gtggaagcca gagaagaaga aaggcgaggc agggagatcg ggttcgtctg gacggaaaag 420
   aatagaggga gcaagaatta acctcggagg ttcaaatcgc aggtgaaatt caaaaacaca 480
   gtggtagatt ttggcctttg cggcccggcg ttatctcatc gcatggaaag agaagaggtt 540
   ttgaggaaga tagagaggca tggagagaga gggagacgga gagggagaag aagagacaag 600
   aaagagtgaa gcatcgtcat ccattccatc ggccaacact tggttgaggc tccacttccg 660
   ctggactttc agcttttctc aactttcatc tctccccccc gttcaatcct ctgcctctct 720
   tttccaagta tatactgttt ttgacagtca gggtcttcaa ttgtgcaggt taggaactga 780
   acattctttt ctgaggtttt tctggtcctc accttcagcg tcatcaacat tggttgcctg 840
   ttgcttaact ccatctcgga tcggggttaa cggtctttgg cgttgcctca accgtttctc 900
   attttgttcc actgacctgt ttcttactct ttccactgcg cccccctaag agagaattta 960
   atctatttcc ccttcgctgt cttttaaata ctcccttctt ttccgttgca gccgagagag 1020
   tgacagggct cttccctctg tcactccgat tttcctaacg acaaggtgtc gaccgcgcga 1080
   ctcccttcgc tttcttttca ggtcccagtc tcacgttcct ggacctcgtt cgaaagcgct 1140
   cgatatattt cctcttcttc ccgaccgacg gattctagac gctcgtcgtc tacatctacg 1200
   atggcatccg agtcgttcaa ctcgcccagc ggcgaatttc ggggccgcag ccaggaaccc 1260
   gtggcttctc gccgagagag tacgccacac gcagctcgcg gagccagcgc aactcctgcc 1320
   cccttggcct ggtttcccct aggctacagg gaaggcttca gtcaatgggt acgtttcttt 1380
   actgaggaac aaagtcaagt gtcgcatggg tgactgagac tgggttttct tccagtgggc 1440
   gtctttaccc gccgccacag ccgaacacag ggttctttcc cacctccctt acctccagca 1500
   tcaaccccca acccatctac aaaccggtaa aacgagcagt gtctctggcg ggacgggcag 1560
   tctccagtcc gccgatcaaa cgcaacaggg tgaggtatca gccagttcta ccaatgatcc 1620
   atacggcccc cgaatgtggc gctccagtat ggtggagctg agcgggaaga atcgggctct 1680
   gaacgagttc tctgtcgagc ggattggtga agaagcagat caacatctgg tcatgcttca 1740
   tggatacggt gctggtttgg ggttcttcta taagaatttc gagcctctta gtcgcctgaa 1800
   agggtggcaa ctccatgcgc tggacctcct gggcatgggc cgcagtacgc gaccacccct 1860
   tcgtatcaaa gcgaaggagc gtgaggaggc catcaaagaa gcagaagcct ggttcgtgga 1920
   tgcgctggaa gagtggcgtg tcaagcgcaa catcgaccgt ttcacactac ttggccatag 1980
   tttgggaggc tacatggccg tggcatacgc tctcaaatat ccgggacgac tcaacaagct 2040
   tatccttgct tcccccgtcg gaatcccaga ggacccgtac gctgtgcggg cagagatgcc 2100
   tgcgccaaac gagtcgacca tggcgaatga gctcactcag aaccagcgca atatcgccga 2160
   atctgcatct tcggttcccc caactgagct tcagaaaggc gataacaata ttttgctaaa 2220
   gggttcccct acagatagtg gcgcttccca agatcggccg ccccggcgca tggttcccaa      2280
   gtggttcgcc tatctttggg atgccaacat ctcccccttc agcctggtgc gatgggcggg      2340
   accactcggt ccgcgtctgg tctccgggtg gacatcccgg cgtttctccc atcttccggc 2400
   agaggaggcc aaatctctgc atgactattc gtattcgatc ttcagcatgc gcggcagcgg 2460
   cgagtatgct ctatcgtaca ttctcgcccc gggcgcattc gcccgcagtc cgctaatccg 2520
   acgcatcgag ggcgtcggtc gtcaattcat ccagcaaaat ccgtcacccg catctgctgt 2580
   cctcgagtct gcgaaagccg cagctgtagc taccacatcc caatctactc ttgaatcttc 2640
   ctcggccaac tcggccccct ctcagacccc ctcaacgaca cccaagcgcg agaacggtct 2700
   ccccataatc ttcatgtacg gtgaccacga ttggatggac gtgaagggcg ggctagccgc 2760
   aaaggctaag ttggatcaag agaaggagcg tatcttgcaa aacgcaacag tggaggaacg 2820
   cgaagcggac aatggctcag ctaaggtggt cattatcaag aggtccggcc atcatatcta 2880
   tctagacgga tgggaagact tcaaccgcat cgttttggct gagatggagg aggtggctca 2940
   gaaggagaga gctaatcgcg cctcatag 2968
<210> 123
   <211> 1542
   <212> DNA
   <213> Aspergillus niger
<220>
   <221> CDS
   <222> (1)..(1542)
<400> 123
   atg gca tcc gag tcg ttc aac tcg ccc agc ggc gaa ttt cgg ggc cgc        48
   Met Ala Ser Glu Ser Phe Asn Ser Pro Ser Gly Glu Phe Arg Gly Arg
   1               5                   10                  15
   agc cag gaa ccc gtg gct tct cgc cga gag agt acg cca cac gca gct        96
   Ser Gln Glu Pro Val Ala Ser Arg Arg Glu Ser Thr Pro His Ala Ala
               20                  25                  30
   cgc gga gcc agc gca act cct gcc ccc ttg gcc tgg ttt ccc cta ggc       144
   Arg Gly Ala Ser Ala Thr Pro Ala Pro Leu Ala Trp Phe Pro Leu Gly
           35                  40                  45
   tac agg gaa ggc ttc agt caa tgg tgg gcg tct tta ccc gcc gcc aca       192
   Tyr Arg Glu Gly Phe Ser Gln Trp Trp Ala Ser Leu Pro Ala Ala Thr
       50                  55                  60
   gcc gaa cac agg gtt ctt tcc cac ctc cct tac ctc cag cat caa ccc       240
   Ala Glu His Arg Val Leu Ser His Leu Pro Tyr Leu Gln His Gln Pro
   65                  70                  75                  80
   cca acc cat cta caa acc ggt aaa acg agc agt gtc tct ggc ggg acg       288
   Pro Thr His Leu Gln Thr Gly Lys Thr Ser Ser Val Ser Gly Gly Thr
   85 90 95
   ggc agt ctc cag tcc gcc gat caa acg caa cag ggt gag gta tca gcc 336
   Gly Ser Leu Gln Ser Ala Asp Gln Thr Gln Gln Gly Glu Val Ser Ala
   100 105 110
   agt tct acc aat gat cca tac ggc ccc cga atg tgg cgc tcc agt atg 384
   Ser Ser Thr Asn Asp Pro Tyr Gly Pro Arg Met Trp Arg Ser Ser Met
   115 120 125
   gtg gag ctg agc ggg aag aat cgg gct ctg aac gag ttc tct gtc gag 432
   Val Glu Leu Ser Gly Lys Asn Arg Ala Leu Asn Glu Phe Ser Val Glu
   130 135 140
   cgg att ggt gaa gaa gca gat caa cat ctg gtc atg ctt cat gga tac 480
   Arg Ile Gly Glu Glu Ala Asp Gln His Leu Val Met Leu His Gly Tyr
   145 150 155 160
   ggt gct ggt ttg ggg ttc ttc tat aag aat ttc gag cct ctt agt cgc 528
   Gly Ala Gly Leu Gly Phe Phe Tyr Lys Asn Phe Glu Pro Leu Ser Arg
   165 170 175
   ctg aaa ggg tgg caa ctc cat gcg ctg gac ctc ctg ggc atg ggc cgc 576
   Leu Lys Gly Trp Gln Leu His Ala Leu Asp Leu Leu Gly Met Gly Arg
   180 185 190
   agt acg cga cca ccc ttt cgt atc aaa gcg aag gag cgt gag gag gcc 624
   Ser Thr Arg Pro Pro Phe Arg Ile Lys Ala Lys Glu Arg Glu Glu Ala
   195 200 205
   atc aaa gaa gca gaa gcc tgg ttc gtg gat gcg ctg gaa gag tgg cgt 672
   Ile Lys Glu Ala Glu Ala Trp Phe Val Asp Ala Leu Glu Glu Trp Arg
   210 215 220
   gtc aag cgc aac atc gac cgt ttc aca cta ctt ggc cat agt ttg gga 720
   Val Lys Arg Asn Ile Asp Arg Phe Thr Leu Leu Gly His Ser Leu Gly
   225 230 235 240
   ggc tac atg gcc gtg gca tac gct ctc aaa tat ccg gga cga ctc aac 768
   Gly Tyr Met Ala Val Ala Tyr Ala Leu Lys Tyr Pro Gly Arg Leu Asn
   245 250 255
   aag ctt atc ctt gct tcc ccc gtc gga atc cca gag gac ccg tac gct 816
   Lys Leu Ile Leu Ala Ser Pro Val Gly Ile Pro Glu Asp Pro Tyr Ala
   260 265 270
   gtg cgg gca gag atg cct gcg cca aac gag tcg acc atg gcg aat gag 864
   Val Arg Ala Glu Met Pro Ala Pro Asn Glu Ser Thr Met Ala Asn Glu
   275 280 285
   ctc act cag aac cag cgc aat atc gcc gaa tct gca tct tcg gtt ccc 912
   Leu Thr Gln Asn Gln Arg Asn Ile Ala Glu Ser Ala Ser Ser Val Pro
   290 295 300
   cca act gag ctt cag aaa ggc gat aac aat att ttg cta aag ggt tcc 960
   Pro Thr Glu Leu Gln Lys Gly Asp Asn Asn Ile Leu Leu Lys Gly Ser
   305 310 315 320
   cct aca gat agt ggc gct tcc caa gat cgg ccg ccc cgg cgc atg gtt 1008
   Pro Thr Asp Ser Gly Ala Ser Gln Asp Arg Pro Pro Arg Arg Met Val
   325 330 335
   ccc aag tgg ttc gcc tat ctt tgg gat gcc aac atc tcc ccc ttc agc 1056
   Pro Lys Trp Phe Ala Tyr Leu Trp Asp Ala Asn Ile Ser Pro Phe Ser
   340 345 350
   ctg gtg cga tgg gcg gga cca ctc ggt ccg cgt ctg gtc tcc ggg tgg        1104
   Leu Val Arg Trp Ala Gly Pro Leu Gly Pro Arg Leu Val Ser Gly Trp
           355                 360                 365
   aca tcc cgg cgt ttc tcc cat ctt ccg gca gag gag gcc aaa tct ctg        1152
   Thr Ser Arg Arg Phe Ser His Leu Pro Ala Glu Glu Ala Lys Ser Leu
       370                 375                 380
   cat gac tat tcg tat tcg atc ttc agc atg cgc ggc agc ggc gag tat        1200
   His Asp Tyr Ser Tyr Ser Ile Phe Ser Met Arg Gly Ser Gly Glu Tyr
   385                 390                 395                 400
   gct cta tcg tac att ctc gcc ccg ggc gca ttc gcc cgc agt ccg cta        1248
   Ala Leu Ser Tyr Ile Leu Ala Pro Gly Ala Phe Ala Arg Ser Pro Leu
                   405                 410                 415
   atc cga cgc atc gag ggc aac ggt ctc ccc ata atc ttc atg tac ggt        1296
   Ile Arg Arg Ile Glu Gly Asn Gly Leu Pro Ile Ile Phe Met Tyr Gly
               420                 425                 430
   gac cac gat tgg atg gac gtg aag ggc ggg cta gcc gca aag gct aag        1344
   Asp His Asp Trp Met Asp Val Lys Gly Gly Leu Ala Ala Lys Ala Lys
           435                 440                 445
   ttg gat caa gag aag gag cgt atc ttg caa aac gca aca gtg gag gaa        1392
   Leu Asp Gln Glu Lys Glu Arg Ile Leu Gln Asn Ala Thr Val Glu Glu
       450                 455                 460
   cgc gaa gcg gac aat ggc tca gct aag gtg gtc att atc aag agg tcc        1440
   Arg Glu Ala Asp Asn Gly Ser Ala Lys Val Val Ile Ile Lys Arg Ser
   465                 470                 475                 480
   ggc cat cat atc tat cta gac gga tgg gaa gac ttc aac cgc atc gtt        1488
   Gly His His Ile Tyr Leu Asp Gly Trp Glu Asp Phe Asn Arg Ile Val
                   485                 490                 495
   ttg gct gag atg gag gag gtg gct cag aag gag aga gct aat cgc gcc        1536
   Leu Ala Glu Met Glu Glu Val Ala Gln Lys Glu Arg Ala Asn Arg Ala
               500                 505                 510
   tca tag                                                                1542
   Ser
<210> 124
   <211> 513
   <212> PRT
   <213> Aspergillus niger
<400> 124
   Met Ala Ser Glu Ser Phe Asn Ser Pro Ser Gly Glu Phe Arg Gly Arg
   1               5                   10                  15
   Ser Gln Glu Pro Val Ala Ser Arg Arg Glu Ser Thr Pro His Ala Ala
               20                  25                  30
   Arg Gly Ala Ser Ala Thr Pro Ala Pro Leu Ala Trp Phe Pro Leu Gly
           35                  40                  45
   Tyr Arg Glu Gly Phe Ser Gln Trp Trp Ala Ser Leu Pro Ala Ala Thr
   50 55 60
   Ala Glu His Arg Val Leu Ser His Leu Pro Tyr Leu Gln His Gln Pro
   65 70 75 80
   Pro Thr His Leu Gln Thr Gly Lys Thr Ser Ser Val Ser Gly Gly Thr
   85 90 95
   Gly Ser Leu Gln Ser Ala Asp Gln Thr Gln Gln Gly Glu Val Ser Ala
   100 105 110
   Ser Ser Thr Asn Asp Pro Tyr Gly Pro Arg Met Trp Arg Ser Ser Met
   115 120 125
   Val Glu Leu Ser Gly Lys Asn Arg Ala Leu Asn Glu Phe Ser Val Glu
   130 135 140
   Arg Ile Gly Glu Glu Ala Asp Gln His Leu Val Met Leu His Gly Tyr
   145 150 155 160
   Gly Ala Gly Leu Gly Phe Phe Tyr Lys Asn Phe Glu Pro Leu Ser Arg
   165 170 175
   Leu Lys Gly Trp Gln Leu His Ala Leu Asp Leu Leu Gly Met Gly Arg
   180 185 190
   Ser Thr Arg Pro Pro Phe Arg Ile Lys Ala Lys Glu Arg Glu Glu Ala
   195 200 205
   Ile Lys Glu Ala Glu Ala Trp Phe Val Asp Ala Leu Glu Glu Trp Arg
   210 215 220
   Val Lys Arg Asn Ile Asp Arg Phe Thr Leu Leu Gly His Ser Leu Gly
   225 230 235 240
   Gly Tyr Met Ala Val Ala Tyr Ala Leu Lys Tyr Pro Gly Arg Leu Asn
   245 250 255
   Lys Leu Ile Leu Ala Ser Pro Val Gly Ile Pro Glu Asp Pro Tyr Ala
   260 265 270
   Val Arg Ala Glu Met Pro Ala Pro Asn Glu Ser Thr Met Ala Asn Glu
   275 280 285
   Leu Thr Gln Asn Gln Arg Asn Ile Ala Glu Ser Ala Ser Ser Val Pro
   290 295 300
   Pro Thr Glu Leu Gln Lys Gly Asp Asn Asn Ile Leu Leu Lys Gly Ser
   305 310 315 320
   Pro Thr Asp Ser Gly Ala Ser Gln Asp Arg Pro Pro Arg Arg Met Val
   325 330 335
   Pro Lys Trp Phe Ala Tyr Leu Trp Asp Ala Asn Ile Ser Pro Phe Ser
   340 345 350
   Leu Val Arg Trp Ala Gly Pro Leu Gly Pro Arg Leu Val Ser Gly Trp
   355 360 365
   Thr Ser Arg Arg Phe Ser His Leu Pro Ala Glu Glu Ala Lys Ser Leu
   370 375 380
   His Asp Tyr Ser Tyr Ser Ile Phe Ser Met Arg Gly Ser Gly Glu Tyr
   385 390 395 400
   Ala Leu Ser Tyr Ile Leu Ala Pro Gly Ala Phe Ala Arg Ser Pro Leu
   405 410 415
   Ile Arg Arg Ile Glu Gly Asn Gly Leu Pro Ile Ile Phe Met Tyr Gly
   420 425 430
   Asp His Asp Trp Met Asp Val Lys Gly Gly Leu Ala Ala Lys Ala Lys
   435 440 445
   Leu Asp Gln Glu Lys Glu Arg Ile Leu Gln Asn Ala Thr Val Glu Glu
   450 455 460
   Arg Glu Ala Asp Asn Gly Ser Ala Lys Val Val Ile Ile Lys Arg Ser
   465 470 475 480
   Gly His His Ile Tyr Leu Asp Gly Trp Glu Asp Phe Asn Arg Ile Val
   485 490 495
   Leu Ala Glu Met Glu Glu Val Ala Gln Lys Glu Arg Ala Asn Arg Ala
   500 505 510
   Ser
<210> 125
   <211> 3175
   <212> DNA
   <213> Aspergillus niger
<400> 125
   cgctatgtca agggcgatga tggcgagcca atcatgccca aggtacgtca tggaagggag 60
   aactactcac cacagagtca tcatgtctca tctcacgata tcactgacat atctcagggc 120
   atggtggact tgattaaaaa ggacgcggat cagggcatcg atgatcttgt ttaatccggt 180
   ataccagcga cggaagtagc ttgtccgaaa ataaatctct gtacaggata gttaaagagg 240
   aaatataata agaagctatg agccctagac ccgctagatg aagaagggcc aagtcatgga 300
   catgaaggac tgaccggagt cgggcgaact cttggctatc attacccgcg agagctacat 360
   cacggatagc ttcggaaaag aaaagcaaga tccattgcca cgaggacatt gtggcgttct 420
   gcttggccga ttcaaaatgc atgagagaca cacagtcaaa gaagccatag aaccagatac 480
   tccgacggga ttcgagtctt tctatagaag caacctatag agcccccaaa taggcaacga 540
   tgggctggga cggtctggaa acggtatcag aatggtttca tcccttccat cgggctgctt 600
   ctcacgcctc ttccgggtgc gtaataatcg taacgggatc gcccagatac cggctgatac 660
   ggggccatac cgagcgccgc cccgtctggc cacgccatcc tcttatgctc tctctatcca 720
   ctctctccct cagcttctct caaccacttc cttacccatc tttcaccctc tcttcccctt 780
   tccccctttc aaatctcttg accttcaagt ctggcgcgcg tctggacagc ggctcttgtg 840
   ctagaggtct taactcctac cccgccttcc tctggccttg accttctctg ttcctgaacc 900
   aggttccctc ccggtcctct ccgccgacgt tatccccgac attgtgctgc tcattacctc 960
   cgcccccaag gtaacgggta agcgaaaagt gcaaggccca gttggctaaa tttcgatgac 1020
   tccttgagcg cgactccaag tccgcttggg accgtttcct ccgctccgtc ttcggcaata 1080
   tctactacta ctactattac tattactacc ccggtcagcc gccgccgccg tattacaagt 1140
   acccatcgcc tactgatggt ctccttatca ttccgcgggg agaccacttc cattccctcc 1200
   atggcgtcct ggttgctctc gacgctcctt tttctgagcc cgtccttggt gtcagccaaa 1260
   tcggccgcag actattatgt tcactccttg cccggtgccc ccgaggggcc cttgctgaag 1320
   atgcatgccg ggtaagcttc gctgtcccgg aacggtccgc ttagcaccta tatactgact 1380
   tattgcctcc acgcgcagcc atattgaggt ggatccacag aacaatggaa atcttttctt 1440
   ctggcactac cagaatcgcc atattgccaa ccgccagcgg actgtgatct ggttgaacgg 1500
   tggtcccgga tgtagttcca tggacggcgc gttgatggag gtcggtccgt atcgcctgaa 1560
   ggacaatgaa accttgacct ataatgaggg ttcctgggac gaattcgcca atttgttgtt 1620
   cgtcgatcag ccagtcggaa ccgggttcag ttatgtcaac acggacagct atcttcatga 1680
   gctcgatgag atgtcggctc agttcattgt ctttctggaa gagtggttca gattatttcc 1740
   ggagtatgaa cgcgatgatg tatgctgcaa tcacctatgc tgccctagtc ctgcaccttc 1800
   acggattctg ttctaacatc tgcgacagat ctacattgcc ggcgagtctt acgccggtca 1860
   gcatattcca tacatcgcca aagccatcca ggaacggaac aagaacgttc aagggaagac 1920
   catcgcttcg tggaatctaa aaggcctatt gattggcaat ggttggattt ctcctaatga 1980
   acagtacatg tcctacttgc cctacgcata tgaagaaggc cttatcaagg aaggcagccg 2040
   gaccgcgaag gaactcgaag ttttacagtc agtctgtaag tccaggctgg aaactggcaa 2100
   gaacaaggtc cacctcaacg actgcgagaa ggtcatgaat gctctgttgg ataagacggt 2160
   cgaagacaac aaatgtctca acatgtatga catccgcctt cgtgacacca ccgatgcatg 2220
   cggtatgaac tggcccaccg acctggagga cgtgaagccc tatctgcagc gggaagatgt 2280
   ggttaaagcg cttaacatca atccggagaa gaagtctggc tgggtggagt gttcaggtgc 2340
   agtgagcagc gctttcaatc cgcaaaagtc cccgccctcg gttcaactac ttcccggctt 2400
   gctggaatcg ggacttcaaa tcctcctttt cagcggagac aaggacctga tttgcaacca 2460
   tgttggaacg gaacagctca tcaataacat gaagtggaac ggaggcacgg gtttcgagac 2520
   ctcacctggc gtctgggctc ctcgacacga ctggagtttc gaaggcgagc cggcgggtat 2580
   ctatcaatat gccagaaacc tgacttacgt gctcatctac aacgcaagcc atatggttcc 2640
   ctacgacctt cctcgtcaga gccgggacat gctagatcgc ttcatgaatg tcgatatcgc 2700
   gagcatcgga ggcagccccg ccgactcgcg cattgacggc gagaagctgc cccagacgtc 2760
   ggtgggcggc catcccaaca gcaccgcggc ggaggagcag gagaaggaga ggatcaagga 2820
   gacggaatgg aaagcctacg ccaagtcagg cgaagccgtt ctcctcgtcg tcattatcgg 2880
   tgtattagtt tggggcttct tcatctggcg cagccgccgg cgtcaccagg gataccgggg 2940
   cgtctggcat aaggacatga gcggaagctc tgttctcgag cggttccaca acaagcgcac 3000
   gggaggcgca gacgtcgaag cgggggattt cgacgaggcg gagctcgatg accttcattc 3060
   tccagacctc gaaagagaac actacgccgt gggcgaggac agcgacgagg atgatatttc 3120
   acgacagcat tctcaacagg cctcccgagc cgggggcagt cataatctat cctag 3175
<210> 126
   <211> 1839
   <212> DNA
   <213> Aspergillus niger
<220>
   <221> CDS
   <222> (1) .. (1839)
<400> 126
   atg gcg tcc tgg ttg ctc tcg acg ctc ctt ttt ctg agc ccg tcc ttg          48
   Met Ala Ser Trp Leu Leu Ser Thr Leu Leu Phe Leu Ser Pro Ser Leu
   1               5                   10                  15
   gtg tca gcc aaa tcg gcc gca gac tat tat gtt cac tcc ttg ccc ggt          96
   Val Ser Ala Lys Ser Ala Ala Asp Tyr Tyr Val His Ser Leu Pro Gly
               20                  25                  30
   gcc ccc gag ggg ccc ttg ctg aag atg cat gcc ggc cat att gag gtg         144
   Ala Pro Glu Gly Pro Leu Leu Lys Met His Ala Gly His Ile Glu Val
           35                  40                  45
   gat cca cag aac aat gga aat ctt ttc ttc tgg cac tac cag aat cgc         192
   Asp Pro Gln Asn Asn Gly Asn Leu Phe Phe Trp His Tyr Gln Asn Arg
       50                  55                  60
   cat att gcc aac cgc cag cgg act gtg atc tgg ttg aac ggt ggt ccc        240
   His Ile Ala Asn Arg Gln Arg Thr Val Ile Trp Leu Asn Gly Gly Pro
   65                  70                  75                  80
   gga tgt agt tcc atg gac ggc gcg ttg atg gag gtc ggt ccg tat cgc        288
   Gly Cys Ser Ser Met Asp Gly Ala Leu Met Glu Val Gly Pro Tyr Arg
                   85                  90                  95
   ctg aag gac aat gaa acc ttg acc tat aat gag ggt tcc tgg gac gaa        336
   Leu Lys Asp Asn Glu Thr Leu Thr Tyr Asn Glu Gly Ser Trp Asp Glu
               100                 105                 110
   ttc gcc aat ttg ttg ttc gtc gat cag cca gtc gga acc ggg ttc agt        384
   Phe Ala Asn Leu Leu Phe Val Asp Gln Pro Val Gly Thr Gly Phe Ser
           115                 120                 125
   tat gtc aac acg gac agc tat ctt cat gag ctc gat gag atg tcg gct        432
   Tyr Val Asn Thr Asp Ser Tyr Leu His Glu Leu Asp Glu Met Ser Ala
       130                 135                 140
   cag ttc att gtc ttt ctg gaa gag tgg ttc aga tta ttt ccg gag tat        480
   Gln Phe Ile Val Phe Leu Glu Glu Trp Phe Arg Leu Phe Pro Glu Tyr
   145                 150                 155                 160
   gaa cgc gat gat atc tac att gcc ggc gag tct tac gcc ggt cag cat        528
   Glu Arg Asp Asp Ile Tyr Ile Ala Gly Glu Ser Tyr Ala Gly Gln His
                   165                 170                 175
   att cca tac atc gcc aaa gcc atc cag gaa cgg aac aag aac gtt caa        576
   Ile Pro Tyr Ile Ala Lys Ala Ile Gln Glu Arg Asn Lys Asn Val Gln
               180                 185                 190
   ggg aag acc atc gct tcg tgg aat cta aaa ggc cta ttg att ggc aat        624
   Gly Lys Thr Ile Ala Ser Trp Asn Leu Lys Gly Leu Leu Ile Gly Asn
           195                 200                 205
   ggt tgg att tct cct aat gaa cag tac atg tcc tac ttg ccc tac gca        672
   Gly Trp Ile Ser Pro Asn Glu Gln Tyr Met Ser Tyr Leu Pro Tyr Ala
       210                 215                 220
   tat gaa gaa ggc ctt atc aag gaa ggc agc cgg acc gcg aag gaa ctc        720
   Tyr Glu Glu Gly Leu Ile Lys Glu Gly Ser Arg Thr Ala Lys Glu Leu
   225                 230                 235                 240
   gaa gtt tta cag tca gtc tgt aag tcc agg ctg gaa act ggc aag aac        768
   Glu Val Leu Gln Ser Val Cys Lys Ser Arg Leu Glu Thr Gly Lys Asn
                   245                 250                 255
   aag gtc cac ctc aac gac tgc gag aag gtc atg aat gct ctg ttg gat        816
   Lys Val His Leu Asn Asp Cys Glu Lys Val Met Asn Ala Leu Leu Asp
               260                 265                 270
   aag acg gtc gaa gac aac aaa tgt ctc aac atg tat gac atc cgc ctt        864
   Lys Thr Val Glu Asp Asn Lys Cys Leu Asn Met Tyr Asp Ile Arg Leu
           275                 280                 285
   cgt gac acc acc gat gca tgc ggt atg aac tgg ccc acc gac ctg gag        912
   Arg Asp Thr Thr Asp Ala Cys Gly Met Asn Trp Pro Thr Asp Leu Glu
       290                 295                 300
   gac gtg aag ccc tat ctg cag cgg gaa gat gtg gtt aaa gcg ctt aac        960
   Asp Val Lys Pro Tyr Leu Gln Arg Glu Asp Val Val Lys Ala Leu Asn
   305                 310                 315                 320
   atc aat ccg gag aag aag tct ggc tgg gtg gag tgt tca ggt gca gtg        1008
   Ile Asn Pro Glu Lys Lys Ser Gly Trp Val Glu Cys Ser Gly Ala Val
                   325                 330                 335
   agc agc gct ttc aat ccg caa aag tcc ccg ccc tcg gtt caa cta ctt      1056
   Ser Ser Ala Phe Asn Pro Gln Lys Ser Pro Pro Ser Val Gln Leu Leu
               340                 345                 350
   ccc ggc ttg ctg gaa tcg gga ctt caa atc ctc ctt ttc agc gga gac      1104
   Pro Gly Leu Leu Glu Ser Gly Leu Gln Ile Leu Leu Phe Ser Gly Asp
           355                 360                 365
   aag gac ctg att tgc aac cat gtt gga acg gaa cag ctc atc aat aac      1152
   Lys Asp Leu Ile Cys Asn His Val Gly Thr Glu Gln Leu Ile Asn Asn
       370                 375                 380
   atg aag tgg aac gga ggc acg ggt ttc gag acc tca cct ggc gtc tgg      1200
   Met Lys Trp Asn Gly Gly Thr Gly Phe Glu Thr Ser Pro Gly Val Trp
   385                 390                 395                 400
   gct cct cga cac gac tgg agt ttc gaa ggc gag ccg gcg ggt atc tat      1248
   Ala Pro Arg His Asp Trp Ser Phe Glu Gly Glu Pro Ala Gly Ile Tyr
                   405                 410                 415
   caa tat gcc aga aac ctg act tac gtg ctc atc tac aac gca agc cat      1296
   Gln Tyr Ala Arg Asn Leu Thr Tyr Val Leu Ile Tyr Asn Ala Ser His
               420                 425                 430
   atg gtt ccc tac gac ctt cct cgt cag agc cgg gac atg cta gat cgc      1344
   Met Val Pro Tyr Asp Leu Pro Arg Gln Ser Arg Asp Met Leu Asp Arg
           435                 440                 445
   ttc atg aat gtc gat atc gcg agc atc gga ggc agc ccc gcc gac tcg      1392
   Phe Met Asn Val Asp Ile Ala Ser Ile Gly Gly Ser Pro Ala Asp Ser
       450                 455                 460
   cgc att gac ggc gag aag ctg ccc cag acg tcg gtg ggc ggc cat ccc      1440
   Arg Ile Asp Gly Glu Lys Leu Pro Gln Thr Ser Val Gly Gly His Pro
   465                 470                 475                 480
   aac agc acc gcg gcg gag gag cag gag aag gag agg atc aag gag acg      1488
   Asn Ser Thr Ala Ala Glu Glu Gln Glu Lys Glu Arg Ile Lys Glu Thr
                   485                 490                 495
   gaa tgg aaa gcc tac gcc aag tca ggc gaa gcc gtt ctc ctc gtc gtc      1536
   Glu Trp Lys Ala Tyr Ala Lys Ser Gly Glu Ala Val Leu Leu Val Val
               500                 505                 510
   att atc ggt gta tta gtt tgg ggc ttc ttc atc tgg cgc agc cgc cgg      1584
   Ile Ile Gly Val Leu Val Trp Gly Phe Phe Ile Trp Arg Ser Arg Arg
           515                 520                 525
   cgt cac cag gga tac cgg ggc gtc tgg cat aag gac atg agc gga agc      1632
   Arg His Gln Gly Tyr Arg Gly Val Trp His Lys Asp Met Ser Gly Ser
       530                 535                 540
   tct gtt ctc gag cgg ttc cac aac aag cgc acg gga ggc gca gac gtc      1680
   Ser Val Leu Glu Arg Phe His Asn Lys Arg Thr Gly Gly Ala Asp Val
   545                 550                 555                 560
   gaa gcg ggg gat ttc gac gag gcg gag ctc gat gac ctt cat tct cca      1728
   Glu Ala Gly Asp Phe Asp Glu Ala Glu Leu Asp Asp Leu His Ser Pro
                   565                 570                 575
   gac ctc gaa aga gaa cac tac gcc gtg ggc gag gac agc gac gag gat      1776
   Asp Leu Glu Arg Glu His Tyr Ala Val Gly Glu Asp Ser Asp Glu Asp
               580                 585                 590
   gat att tca cga cag cat tct caa cag gcc tcc cga gcc ggg ggc agt          1824
   Asp Ile Ser Arg Gln His Ser Gln Gln Ala Ser Arg Ala Gly Gly Ser
           595                 600                 605
   cat aat cta tcc tag                                                      1839
   His Asn Leu Ser
       610
<210> 127
   <211> 612
   <212> PRT
   <213> Aspergillus niger
<400> 127
   Met Ala Ser Trp Leu Leu Ser Thr Leu Leu Phe Leu Ser Pro Ser Leu
   1               5                   10                  15
   Val Ser Ala Lys Ser Ala Ala Asp Tyr Tyr Val His Ser Leu Pro Gly
               20                  25                  30
   Ala Pro Glu Gly Pro Leu Leu Lys Met His Ala Gly His Ile Glu Val
           35                  40                  45
   Asp Pro Gln Asn Asn Gly Asn Leu Phe Phe Trp His Tyr Gln Asn Arg
       50                  55                  60
   His Ile Ala Asn Arg Gln Arg Thr Val Ile Trp Leu Asn Gly Gly Pro
   65                  70                  75                  80
   Gly Cys Ser Ser Met Asp Gly Ala Leu Met Glu Val Gly Pro Tyr Arg
                   85                  90                  95
   Leu Lys Asp Asn Glu Thr Leu Thr Tyr Asn Glu Gly Ser Trp Asp Glu
               100                 105                 110
   Phe Ala Asn Leu Leu Phe Val Asp Gln Pro Val Gly Thr Gly Phe Ser
           115                 120                 125
   Tyr Val Asn Thr Asp Ser Tyr Leu His Glu Leu Asp Glu Met Ser Ala
       130                 135                 140
   Gln Phe Ile Val Phe Leu Glu Glu Trp Phe Arg Leu Phe Pro Glu Tyr
   145                 150                 155                 160
   Glu Arg Asp Asp Ile Tyr Ile Ala Gly Glu Ser Tyr Ala Gly Gln His
                   165                 170                 175
   Ile Pro Tyr Ile Ala Lys Ala Ile Gln Glu Arg Asn Lys Asn Val Gln
               180                 185                 190
   Gly Lys Thr Ile Ala Ser Trp Asn Leu Lys Gly Leu Leu Ile Gly Asn
   195 200 205
   Gly Trp Ile Ser Pro Asn Glu Gln Tyr Met Ser Tyr Leu Pro Tyr Ala
   210 215 220
   Tyr Glu Glu Gly Leu Ile Lys Glu Gly Ser Arg Thr Ala Lys Glu Leu
   225 230 235 240
   Glu Val Leu Gln Ser Val Cys Lys Ser Arg Leu Glu Thr Gly Lys Asn
   245 250 255
   Lys Val His Leu Asn Asp Cys Glu Lys Val Met Asn Ala Leu Leu Asp
   260 265 270
   Lys Thr Val Glu Asp Asn Lys Cys Leu Asn Met Tyr Asp Ile Arg Leu
   275 280 285
   Arg Asp Thr Thr Asp Ala Cys Gly Met Asn Trp Pro Thr Asp Leu Glu
   290 295 300
   Asp Val Lys Pro Tyr Leu Gln Arg Glu Asp Val Val Lys Ala Leu Asn
   305 310 315 320
   Ile Asn Pro Glu Lys Lys Ser Gly Trp Val Glu Cys Ser Gly Ala Val
   325 330 335
   Ser Ser Ala Phe Asn Pro Gln Lys Ser Pro Pro Ser Val Gln Leu Leu
   340 345 350
   Pro Gly Leu Leu Glu Ser Gly Leu Gln Ile Leu Leu Phe Ser Gly Asp
   355 360 365
   Lys Asp Leu Ile Cys Asn His Val Gly Thr Glu Gln Leu Ile Asn Asn
   370 375 380
   Met Lys Trp Asn Gly Gly Thr Gly Phe Glu Thr Ser Pro Gly Val Trp
   385 390 395 400
   Ala Pro Arg His Asp Trp Ser Phe Glu Gly Glu Pro Ala Gly Ile Tyr
   405 410 415
   Gln Tyr Ala Arg Asn Leu Thr Tyr Val Leu Ile Tyr Asn Ala Ser His
   420 425 430
   Met Val Pro Tyr Asp Leu Pro Arg Gln Ser Arg Asp Met Leu Asp Arg
   435 440 445
   Phe Met Asn Val Asp Ile Ala Ser Ile Gly Gly Ser Pro Ala Asp Ser
   450 455 460
   Arg Ile Asp Gly Glu Lys Leu Pro Gln Thr Ser Val Gly Gly His Pro
   465 470 475 480
   Asn Ser Thr Ala Ala Glu Glu Gln Glu Lys Glu Arg Ile Lys Glu Thr
   485 490 495
   Glu Trp Lys Ala Tyr Ala Lys Ser Gly Glu Ala Val Leu Leu Val Val
   500 505 510
   Ile Ile Gly Val Leu Val Trp Gly Phe Phe Ile Trp Arg Ser Arg Arg
   515 520 525
   Arg His Gln Gly Tyr Arg Gly Val Trp His Lys Asp Met Ser Gly Ser
   530 535 540
   Ser Val Leu Glu Arg Phe His Asn Lys Arg Thr Gly Gly Ala Asp Val
   545 550 555 560
   Glu Ala Gly Asp Phe Asp Glu Ala Glu Leu Asp Asp Leu His Ser Pro
   565 570 575
   Asp Leu Glu Arg Glu His Tyr Ala Val Gly Glu Asp Ser Asp Glu Asp
   580 585 590
   Asp Ile Ser Arg Gln His Ser Gln Gln Ala Ser Arg Ala Gly Gly Ser
   595 600 605
   His Asn Leu Ser
   610
<210> 128
   <211> 2936
   <212> DNA
   <213> Aspergillus niger
<400> 128
   gagctgagct ggtccaaggc cttggaaatc attccggggg tatagactcg cttcgtcgta 60
   ggatcaacaa gccgtcctga tacgatatcc aacacctcct tctccacgcg ctcgatgatt 120
   tccttgcgct cacgctcgcc aacttgcact tcaccctttc gtagaatttc ttgccggatc 180
   tcatctgcag gcgtgtcggc gccgaaggcc tttgttaact cggcggatgg ggctgtttgc 240
   gctttggaaa ccgacaggaa gatcgtgggg acttggagga cattgtcgag atccttctct 300
   gctccggagc ggtactcgag gagtttgttc ttgtagcagg cgagttcgaa gcgcttttta 360
   cctgttggtg aaacggttag cggggttgcg gggccgactg gaagtttcgc aggcagtttt 420
   tctttctacg ctgaatttgc gcatgatctc cccacattat tgacagacat gccacgcaag 480
   acgatcgcaa atgaagggcc acacatgcat atcaatacgt accctttttt agcctgacga 540
   ctgatacgtt ggtaaattta atctgatttg atggttggtt gatgggcatg ttgctggaag 600
   cctctgcgac aaagcaagag gctcttgtgt atacacagag tcaattcagg taatatatgg 660
   aatcggtata ctcgcaggtg cgtcagtgag gaggattatg tttcgaatgc gctgaaaaaa 720
   atgctgttga tagcctaagg tagtcacccg acgggcttgt gattggagga aaccgcaatg 780
   acgctggtcc cgtgccatga gctgactcag ctggctgact caacgcgtga ctttctgcaa 840
   agggaaggca ccggcatgtg attggtcgcc ttccgttcct gctatcgagg cgtcatcgcc 900
   tacagcccat cgagtacacc tcctcttctc tcctctctcc ttccttcctt cctctccatc 960
   ctcttctggg tttccattga tcacccagta gctggtattt tctgagctac ttgctttttc 1020
   tattttgata cttttgtgtc cgtaagttca ctttatgccc tctgaccccc cccttgataa 1080
   cccgtgctga taagctccct gcccaggtac cgtaccttcc agaccgcaag gtacccatcc 1140
   tctgcctact catcccatca ccatctcaat tcataccggc cccgtagggt ttcagcaaca 1200
   atgagagtcc ttccagctgc tatgctggtt ggagcggcca cggcggccgt tcctcccttc 1260
   cagcaggtcc ttggaggtaa cggtgccaag cacggtgccg accatgcggc cgaggtccct 1320
   gcggatcaca gtgccgacgg gttctccaag ccgctgcacg cattccagga ggaactgaag 1380
   tctctctctg acgaggctcg taagctttgg gatgaggtgg ccagcttctt cccggagagc 1440
   atggatcaga accctctctt ttccctcccc aagaagcaca accgccgtcc cgactcgcac 1500
   tgggaccaca tcgtccgcgg ctccgacgtt cagagcgtct gggtcactgg tgagaacggt 1560
   gagaaggagc gcgaggtcga tggcaagctg gaagcctatg atctcagggt caagaagacc 1620
   gatcctggct ctcttggcat cgaccccggc gtgaagcagt acaccggtta tctcgatgac 1680
   aacgagaatg ataagcattt gttctactgt aagcacacct tggttcaaga tcacgctttt 1740
   tatatgctct ggatatctaa cgcaacttag ggttcttcga gtctcgcaat gaccccgaga 1800
   atgatcccgt tgttctgtgg ctgaacggtg gccctgggtg ctcttccctc accggtctct 1860
   tcatggagct tggccctagc agcatcaaca agaagatcca gccggtctac aatgactacg 1920
   cttggaactc caacgcgtcc gtgatcttcc ttgaccagcc tgtcaatgtc ggttactcct 1980
   acagtaactc tgctgtcagc gacacggtcg ctgctggcaa ggacgtctat gccttgctta 2040
   ccctcttctt caaacaattc cccgagtatg ctaagcagga cttccacatt gccggtgaat 2100
   cttatgctgg tcactatatc cccgtcttcg cttcggagat cctgtctcac aagaagcgca 2160
   acatcaacct gcagtccgtt ctcattggca acggtctcac cgacggatac acccagtacg 2220
   agtactaccg tcccatggcc tgcggtgacg gcggttaccc agctgtcttg gacgagagct 2280
   cctgccagtc catggacaac gctcttcctc gctgccagtc tatgattgag tcttgctaca 2340
   gttccgagag cgcttgggtt tgtgtcccgg cctccatcta ctgtaacaac gccctccttg 2400
   ccccttacca gcgcactggg cagaacgtct atgatgtccg tggtaagtgc gaggatagct     2460
   ctaacctttg ctactcggct atgggctacg tcagcgacta cctgaacaag cccgaagtca     2520
   tcgaggctgt tggcgctgag gtcaacggct acgactcgtg caactttgac atcaaccgca     2580
   acttcctctt ccacggtgac tggatgaagc cctaccaccg cctcgttccg ggactcctgg     2640
   agcagatccc tgtcttgatc tatgccggtg atgctgattt catttgcaac tggctgggca     2700
   acaaggcctg gactgaagcc ctggagtggc ccggacaggc tgaatatgcc tccgctgagc     2760
   tggaggatct ggtcattgtc gacaatgagc acacgggcaa gaagattggc caggttaagt     2820
   cccatggcaa cttcaccttc atgcgtctct atggtggtgg ccacatggtc ccgatggacc     2880
   agcccgagtc gagtctcgag ttcttcaacc gctggttggg aggtgaatgg ttctaa         2936
<210> 129
   <211> 1674
   <212> DNA
   <213> Aspergillus niger
<220>
   <221> CDS
   <222> (1) .. (1674)
<400> 129
   atg aga gtc ctt cca gct gct atg ctg gtt gga gcg gcc acg gcg gcc       48
   Met Arg Val Leu Pro Ala Ala Met Leu Val Gly Ala Ala Thr Ala Ala
   1               5                   10                  15
   gtt cct ccc ttc cag cag gtc ctt gga ggt aac ggt gcc aag cac ggt       96
   Val Pro Pro Phe Gln Gln Val Leu Gly Gly Asn Gly Ala Lys His Gly
               20                  25                  30
   gcc gac cat gcg gcc gag gtc cct gcg gat cac agt gcc gac ggg ttc      144
   Ala Asp His Ala Ala Glu Val Pro Ala Asp His Ser Ala Asp Gly Phe
           35                  40                  45
   tcc aag ccg ctg cac gca ttc cag gag gaa ctg aag tct ctc tct gac      192
   Ser Lys Pro Leu His Ala Phe Gln Glu Glu Leu Lys Ser Leu Ser Asp
       50                  55                  60
   gag gct cgt aag ctt tgg gat gag gtg gcc agc ttc ttc ccg gag agc      240
   Glu Ala Arg Lys Leu Trp Asp Glu Val Ala Ser Phe Phe Pro Glu Ser
   65                  70                  75                  80
   atg gat cag aac cct ctc ttt tcc ctc ccc aag aag cac aac cgc cgt      288
   Met Asp Gln Asn Pro Leu Phe Ser Leu Pro Lys Lys His Asn Arg Arg
                   85                  90                  95
   ccc gac tcg cac tgg gac cac atc gtc cgc ggc tcc gac gtt cag agc      336
   Pro Asp Ser His Trp Asp His Ile Val Arg Gly Ser Asp Val Gln Ser
               100                 105                 110
   gtc tgg gtc act ggt gag aac ggt gag aag gag cgc gag gtc gat ggc      384
   Val Trp Val Thr Gly Glu Asn Gly Glu Lys Glu Arg Glu Val Asp Gly
           115                 120                 125
   aag ctg gaa gcc tat gat ctc agg gtc aag aag acc gat cct ggc tct      432
   Lys Leu Glu Ala Tyr Asp Leu Arg Val Lys Lys Thr Asp Pro Gly Ser
       130                 135                 140
   ctt ggc atc gac ccc ggc gtg aag cag tac acc ggt tat ctc gat gac        480
   Leu Gly Ile Asp Pro Gly Val Lys Gln Tyr Thr Gly Tyr Leu Asp Asp
   145                 150                 155                 160
   aac gag aat gat aag cat ttg ttc tac tgg ttc ttc gag tct cgc aat        528
   Asn Glu Asn Asp Lys His Leu Phe Tyr Trp Phe Phe Glu Ser Arg Asn
                   165                 170                 175
   gac ccc gag aat gat ccc gtt gtt ctg tgg ctg aac ggt ggc cct ggg        576
   Asp Pro Glu Asn Asp Pro Val Val Leu Trp Leu Asn Gly Gly Pro Gly
               180                 185                 190
   tgc tct tcc ctc acc ggt ctc ttc atg gag ctt ggc cct agc agc atc        624
   Cys Ser Ser Leu Thr Gly Leu Phe Met Glu Leu Gly Pro Ser Ser Ile
           195                 200                 205
   aac aag aag atc cag ccg gtc tac aat gac tac gct tgg aac tcc aac        672
   Asn Lys Lys Ile Gln Pro Val Tyr Asn Asp Tyr Ala Trp Asn Ser Asn
       210                 215                 220
   gcg tcc gtg atc ttc ctt gac cag cct gtc aat gtc ggt tac tcc tac        720
   Ala Ser Val Ile Phe Leu Asp Gln Pro Val Asn Val Gly Tyr Ser Tyr
   225                 230                 235                 240
   agt aac tct gct gtc agc gac acg gtc gct gct ggc aag gac gtc tat        768
   Ser Asn Ser Ala Val Ser Asp Thr Val Ala Ala Gly Lys Asp Val Tyr
                   245                 250                 255
   gcc ttg ctt acc ctc ttc ttc aaa caa ttc ccc gag tat gct aag cag        816
   Ala Leu Leu Thr Leu Phe Phe Lys Gln Phe Pro Glu Tyr Ala Lys Gln
               260                 265                 270
   gac ttc cac att gcc ggt gaa tct tat gct ggt cac tat atc ccc gtc        864
   Asp Phe His Ile Ala Gly Glu Ser Tyr Ala Gly His Tyr Ile Pro Val
           275                 280                 285
   ttc gct tcg gag atc ctg tct cac aag aag cgc aac atc aac ctg cag        912
   Phe Ala Ser Glu Ile Leu Ser His Lys Lys Arg Asn Ile Asn Leu Gln
       290                 295                 300
   tcc gtt ctc att ggc aac ggt ctc acc gac gga tac acc cag tac gag        960
   Ser Val Leu Ile Gly Asn Gly Leu Thr Asp Gly Tyr Thr Gln Tyr Glu
   305                 310                 315                 320
   tac tac cgt ccc atg gcc tgc ggt gac ggc ggt tac cca gct gtc ttg       1008
   Tyr Tyr Arg Pro Met Ala Cys Gly Asp Gly Gly Tyr Pro Ala Val Leu
                   325                 330                 335
   gac gag agc tcc tgc cag tcc atg gac aac gct ctt cct cgc tgc cag       1056
   Asp Glu Ser Ser Cys Gln Ser Met Asp Asn Ala Leu Pro Arg Cys Gln
               340                 345                 350
   tct atg att gag tct tgc tac agt tcc gag agc gct tgg gtt tgt gtc       1104
   Ser Met Ile Glu Ser Cys Tyr Ser Ser Glu Ser Ala Trp Val Cys Val
           355                 360                 365
   ccg gcc tcc atc tac tgt aac aac gcc ctc ctt gcc cct tac cag cgc       1152
   Pro Ala Ser Ile Tyr Cys Asn Asn Ala Leu Leu Ala Pro Tyr Gln Arg
       370                 375                 380
   act ggg cag aac gtc tat gat gtc cgt ggt aag tgc gag gat agc tct       1200
   Thr Gly Gln Asn Val Tyr Asp Val Arg Gly Lys Cys Glu Asp Ser Ser
   385                 390                 395                 400
   aac ctt tgc tac tcg gct atg ggc tac gtc agc gac tac ctg aac aag        1248
   Asn Leu Cys Tyr Ser Ala Met Gly Tyr Val Ser Asp Tyr Leu Asn Lys
                   405                 410                 415
   ccc gaa gtc atc gag gct gtt ggc gct gag gtc aac ggc tac gac tcg        1296
   Pro Glu Val Ile Glu Ala Val Gly Ala Glu Val Asn Gly Tyr Asp Ser
               420                 425                 430
   tgc aac ttt gac atc aac cgc aac ttc ctc ttc cac ggt gac tgg atg        1344
   Cys Asn Phe Asp Ile Asn Arg Asn Phe Leu Phe His Gly Asp Trp Met
           435                 440                 445
   aag ccc tac cac cgc ctc gtt ccg gga ctc ctg gag cag atc cct gtc        1392
   Lys Pro Tyr His Arg Leu Val Pro Gly Leu Leu Glu Gln Ile Pro Val
       450                 455                 460
   ttg atc tat gcc ggt gat gct gat ttc att tgc aac tgg ctg ggc aac        1440
   Leu Ile Tyr Ala Gly Asp Ala Asp Phe Ile Cys Asn Trp Leu Gly Asn
   465                 470                 475                 480
   aag gcc tgg act gaa gcc ctg gag tgg ccc gga cag gct gaa tat gcc        1488
   Lys Ala Trp Thr Glu Ala Leu Glu Trp Pro Gly Gln Ala Glu Tyr Ala
                   485                 490                 495
   tcc gct gag ctg gag gat ctg gtc att gtc gac aat gag cac acg ggc        1536
   Ser Ala Glu Leu Glu Asp Leu Val Ile Val Asp Asn Glu His Thr Gly
               500                 505                 510
   aag aag att ggc cag gtt aag tcc cat ggc aac ttc acc ttc atg cgt        1584
   Lys Lys Ile Gly Gln Val Lys Ser His Gly Asn Phe Thr Phe Met Arg
           515                 520                 525
   ctc tat ggt ggt ggc cac atg gtc ccg atg gac cag ccc gag tcg agt        1632
   Leu Tyr Gly Gly Gly His Met Val Pro Met Asp Gln Pro Glu Ser Ser
       530                 535                 540
   ctc gag ttc ttc aac cgc tgg ttg gga ggt gaa tgg ttc taa                1674
   Leu Glu Phe Phe Asn Arg Trp Leu Gly Gly Glu Trp Phe
   545                 550                 555
<210> 130
   <211> 557
   <212> PRT
   <213> Aspergillus niger
<400> 130
   Met Arg Val Leu Pro Ala Ala Met Leu Val Gly Ala Ala Thr Ala Ala
   1 5 10 15
   Val Pro Pro Phe Gln Gln Val Leu Gly Gly Asn Gly Ala Lys His Gly
   20 25 30
   Ala Asp His Ala Ala Glu Val Pro Ala Asp His Ser Ala Asp Gly Phe
   35 40 45
   Ser Lys Pro Leu His Ala Phe Gln Glu Glu Leu Lys Ser Leu Ser Asp
   50 55 60
   Glu Ala Arg Lys Leu Trp Asp Glu Val Ala Ser Phe Phe Pro Glu Ser
   65 70 75 80
   Met Asp Gln Asn Pro Leu Phe Ser Leu Pro Lys Lys His Asn Arg Arg
   85 90 95
   Pro Asp Ser His Trp Asp His Ile Val Arg Gly Ser Asp Val Gln Ser
   100 105 110
   Val Trp Val Thr Gly Glu Asn Gly Glu Lys Glu Arg Glu Val Asp Gly
   115 120 125
   Lys Leu Glu Ala Tyr Asp Leu Arg Val Lys Lys Thr Asp Pro Gly Ser
   130 135 140
   Leu Gly Ile Asp Pro Gly Val Lys Gln Tyr Thr Gly Tyr Leu Asp Asp
   145 150 155 160
   Asn Glu Asn Asp Lys His Leu Phe Tyr Trp Phe Phe Glu Ser Arg Asn
   165 170 175
   Asp Pro Glu Asn Asp Pro Val Val Leu Trp Leu Asn Gly Gly Pro Gly
   180 185 190
   Cys Ser Ser Leu Thr Gly Leu Phe Met Glu Leu Gly Pro Ser Ser Ile
   195 200 205
   Asn Lys Lys Ile Gln Pro Val Tyr Asn Asp Tyr Ala Trp Asn Ser Asn
   210 215 220
   Ala Ser Val Ile Phe Leu Asp Gln Pro Val Asn Val Gly Tyr Ser Tyr
   225 230 235 240
   Ser Asn Ser Ala Val Ser Asp Thr Val Ala Ala Gly Lys Asp Val Tyr
   245 250 255
   Ala Leu Leu Thr Leu Phe Phe Lys Gln Phe Pro Glu Tyr Ala Lys Gln
   260 265 270
   Asp Phe His Ile Ala Gly Glu Ser Tyr Ala Gly His Tyr Ile Pro Val
   275 280 285
   Phe Ala Ser Glu Ile Leu Ser His Lys Lys Arg Asn Ile Asn Leu Gln
   290 295 300
   Ser Val Leu Ile Gly Asn Gly Leu Thr Asp Gly Tyr Thr Gln Tyr Glu
   305 310 315 320
   Tyr Tyr Arg Pro Met Ala Cys Gly Asp Gly Gly Tyr Pro Ala Val Leu
   325 330 335
   Asp Glu Ser Ser Cys Gln Ser Met Asp Asn Ala Leu Pro Arg Cys Gln
   340 345 350
   Ser Met Ile Glu Ser Cys Tyr Ser Ser Glu Ser Ala Trp Val Cys Val
   355 360 365
   Pro Ala Ser Ile Tyr Cys Asn Asn Ala Leu Leu Ala Pro Tyr Gln Arg
   370 375 380
   Thr Gly Gln Asn Val Tyr Asp Val Arg Gly Lys Cys Glu Asp Ser Ser
   385 390 395 400
   Asn Leu Cys Tyr Ser Ala Met Gly Tyr Val Ser Asp Tyr Leu Asn Lys
   405 410 415
   Pro Glu Val Ile Glu Ala Val Gly Ala Glu Val Asn Gly Tyr Asp Ser
   420 425 430
   Cys Asn Phe Asp Ile Asn Arg Asn Phe Leu Phe His Gly Asp Trp Met
   435 440 445
   Lys Pro Tyr His Arg Leu Val Pro Gly Leu Leu Glu Gln Ile Pro Val
   450 455 460
   Leu Ile Tyr Ala Gly Asp Ala Asp Phe Ile Cys Asn Trp Leu Gly Asn
   465 470 475 480
   Lys Ala Trp Thr Glu Ala Leu Glu Trp Pro Gly Gln Ala Glu Tyr Ala
   485 490 495
   Ser Ala Glu Leu Glu Asp Leu Val Ile Val Asp Asn Glu His Thr Gly
   500 505 510
   Lys Lys Ile Gly Gln Val Lys Ser His Gly Asn Phe Thr Phe Met Arg
   515 520 525
   Leu Tyr Gly Gly Gly His Met Val Pro Met Asp Gln Pro Glu Ser Ser
   530 535 540
   Leu Glu Phe Phe Asn Arg Trp Leu Gly Gly Glu Trp Phe
   545 550 555
<210> 131
   <211> 32
   <212> DNA
   <213> Artificial sequence
<220>
   <223> Primer
<400> 131
   tctcctgagg cccgatcgga ccctgcgcga tc 32
<210> 132
   <211> 32
   <212> DNA
   <213> Artificial sequence
<220>
   <223> Primer
<400> 132
   gatcgcgcag ggtccgatcg ggcctcagga ga 32
<210> 133
   <211> 31
   <212> DNA
   <213> Artificial sequence
<220>
   <223> Primer
<400> 133
   gcgtctcctg aggccagacg gaccctgcgc g 31
<210> 134
   <211> 31
   <212> DNA
   <213> Artificial sequence
<220>
   <223> Primer
<400> 134
   cgcgcagggt ccgtctggcc tcaggagacg c 31
<210> 135
   <211> 35
   <212> DNA
   <213> Artificial sequence
<220>
   <223> Primer
<400> 135
   ctaacgtatg gcgtctccag aggcccgacg gaccc 35
<210> 136
   <211> 35
   <212> DNA
   <213> Artificial sequence
<220>
   <223> Primer
<400> 136
   gggtccgtcg ggcctctgga gacgccatac gttag 35
<210> 137
   <211> 32
   <212> DNA
   <213> Artificial sequence
<220>
   <223> Primer
<400> 137
   ctgcgcgatc ggcggtagag cgcaatctaa gg 32
<210> 138
   <211> 32
   <212> DNA
   <213> Artificial sequence
<220>
   <223> Primer
<400> 138
   ccttagattg cgctctaccg ccgatcgcgc ag 32
<210> 139
   <211> 1002
   <212> DNA
   <213> Aspergillus niger
<400> 139
   gcttttggct tgtgatcttg attgctagag atgtatatcc tcacggatac cgccggagtg      60
   cgccatttct ggttaccttc tctttccctt tttgtctcga tcgtgaggcg gaacgcagga     120
   tgaagacacg gcttctccat cgcggcccac caaccaacaa tgtccttgga cgcccaactc     180
   tccatctact ggtcattggt ccaatgcaga gactccgtcg agctcaaatg ggccggccaa     240
   ccccgagtcg tcaggggcag cggcagcgac gagctaaatt agaccactga taagacgcga     300
   tagtccaaag tctgaccgtc acattgtgcc aggcagataa gttgaatcgt gtgactggat     360
   gttggctaac gtatggcgtc tcctgaggcc cgatcggacc ctgcgcgatc ggcggtggag     420
   cgcaatctaa ggacatccgc gcctaagata tctacccttc agcagttcag cctagccctg     480
   cagacttgtc ggaccagtgc tatcgtgatc ggcccccacg gtcgaatgag ctcttgtctc     540
   tttccgtcag accctgccag ttaatctgct atctactccg cggtaacatc gtgcctgtct     600
   ccactaaggc agggtccagg gctgtatgtc ttactttgca ccgagtcggc cgccggttgg     660
   ctctgtcttg gcaattgcga atatcctcac gggcgacgga cgacacggat ttggacggac     720
   atgcggagat cttcgtcggt ttattcctgg aagggacatc atctccttcc atcatgacgg     780
   ctgccatagc ggggactctg agacattttt gctctgaaga gcatggtcga cttggatgat     840
   ggaggagttg atcgaggtca atgaggagag gcttgcaagt ataagaagag actgctcgac     900
   cagcagaatg gatcttcttg ttcatcaacc aagagtccaa ggcttctttg tctggttcta     960
   tctcttctcc gaactctctt gcttgacatt ctcgtggtca aa                      1002

## Description

### Field of the invention

The present invention relates to polynucleotides having a functional, a mutated, non-functional and/or a mutated, enhanced DNA binding site which is specifically recognized by PrtT, a transcriptional activator for protease genes, and their use in regulating gene expression.

### Background of the invention

Fungal transcriptional activators named PrtT have been recently described in WO 00/20596, WO 01/68864 and WO 06/040312. These transcriptional activators were isolated from *Aspergillus niger (A. niger)* and *Aspergillus oryzae (A. oryzae).* They globally activate transcription from the 5.'-upstream promoters of fungal protease genes. Until recently, modulation of PrtT activated transcription was only possible on a global level by altering the transcriptional activator itself.
We now present conserved DNA binding sites in promoters of protease genes that are necessary and sufficient to confer PrtT-dependent transcriptional activation on a downstream nucleotide sequence.

The present invention discloses a novel DNA binding site located in a promoter region and recognized by PrtT transcriptional activators, which has improved properties for regulating transcription of genes in fungi as compared to sequences that have been previously described. Other advantages and improvements are described below or would be apparent from the disclosure herein.

### Summary of the invention

It is an object of the invention to control, by genetic engineering, PrtT responsiveness, enhanced responsiveness or non-responsiveness of a native fungal gene or other heterologous nucleotide sequence through recognition or non-recognition of the promoter's functional or mutated DNA binding site in fungal cells.

Herewith polynucleotides are disclosed that comprise one or more DNA binding sites, wherein at least one site is recognized by PrtT and confers PrtT-dependent transcriptional activation.

Herewith polynucleotides are disclosed that comprise one or more mutated, non-functional DNA binding sites, wherein at least one site is not recognized by PrtT and/or does not confer PrtT-dependent transcriptional activation.

Polynucleotides are disclosed that comprise one or more mutated, enhanced DNA binding sites, wherein at least one site is recognized by PrtT and confers enhanced PrtT-dependent transcriptional activation.

Expression vectors comprising at least one of either of the aforementioned non-mutated and /or mutated, enhanced DNA binding sites are disclosed. The vector may further comprise a downstream nucleotide sequence which is transcribed by the promoter which contains a functional or functional, enhanced DNA binding site and is activated by PrtT. In addition, cells comprising an aforementioned DNA binding site (e.g. non-mutated, mutated, non-functional and mutated, enhanced) and/or an aforementioned vector are disclosed.

Cells in which at least one endogenous gene is mutated such that its promoter is no longer bound or transcriptionally activated by PrtT, or cells comprising the mutated, non-functional aforementioned polynucleotides or respective expression vector are provided. Such cells with at least reduced protease activity are also disclosed.

Processes for identifying protease genes are disclosed. One or more differentially expressed genes are detected between fungal cells either with or without a PrtT transcriptional activator (e.g., deleting *prt*T or otherwise inactivating PrtT). Those differentially expressed genes that encode proteases (e.g., as determined by sequence similarity to known proteases, proteolytic activity of the gene product, and possession of a DNA binding site for PrtT) are identified as protease genes controlled by PrtT. Novel proteases so identified and polynucleotides encoding them are also disclosed.

In one embodiment of the invention, processes for producing a cell with at least one mutated, non-functional DNA binding site in a promoter are provided. The mutation may be introduced into a host chromosome (*i.e.,* an endogenous gene, preferably a protease gene) by mutagenesis or recombination techniques. Introduction of the mutation into the host genome may be confirmed by reduced binding of PrtT to the mutated DNA binding site or reduced PrtT-dependent transcriptional activation from the promoter. Inactivation of the endogenous promoters of at least 13 experimentally identified, more preferably all native protease genes by mutating a like number of DNA binding sites is preferred.

In another embodiment of the invention, processes for producing a polypeptide are provided. One or more polypeptides are expressed in a cell from an expression vector or an endogenous promoter of a native fungal gene. For protease-sensitive polypeptides, the cell is reduced in protease activity. For polypeptides (such as many proteases) that are secreted out of the cell, they may be recovered from the nutrient medium. Otherwise, polypeptides are recovered from the cells (preferably a cell paste or pellet): (i) soluble polypeptides from a cell lysate and (ii) insoluble polypeptides or those inserted into or associated with cell membranes from a cell fraction.

Other processes for using and making the aforementioned polynucleotides, expression vectors, novel proteases and the polynucleotides encoding them, and cells are also disclosed. Further aspects of the invention will be apparent from the following description and claims, and generalizations thereto.

### Brief Description of the Drawings and Tables

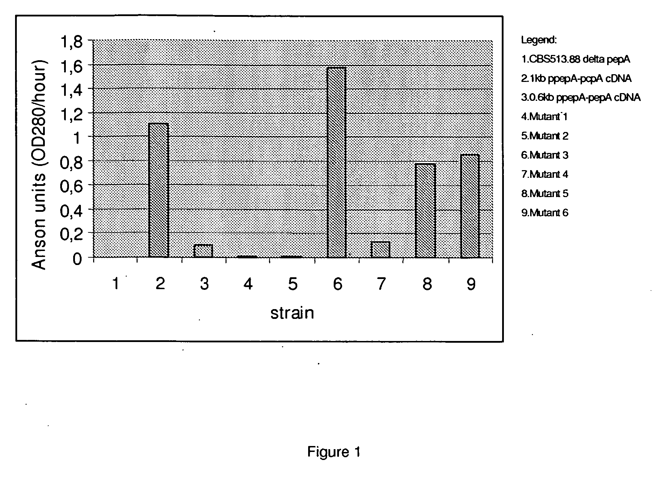

Figure 1 shows the measurement of protease activity in culture supernatants; Results of the Anson assay *(*J. Gen. Physiol. 22:79-89, 1938) were determined using supernatant after 10 days fermentation in a shaker flask. Protease activity measurements in CBS 513.88 delta pepA transformants carrying constructs with differently modified pepA promoters (see Table 5) are shown in lanes 4 to 9. The lanes 2 and 3 correspond to transformants carrying the wild type 1.0kb pepA promoter (containing the PrtT binding site) and the wild type 0.6kb pepA promoter (without the PrtT binding site) constructs, respectively. The background was determined as the protease activity of non-transformed *Aspergillus niger* CBS 513.88 delta pepA (lane 1). Each bar represents a mean value of protease activity of two independent transformants.

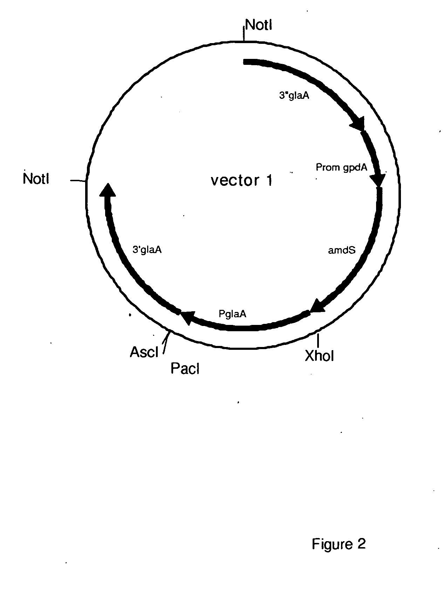

Figure 2 shows a physical plasmid map of vector 1, which illustrates recognition sites for restriction enzymes, the locations of genes, and their orientation. It is used for recloning *Asc*I*lXho*I ppepA-pepA constructs.

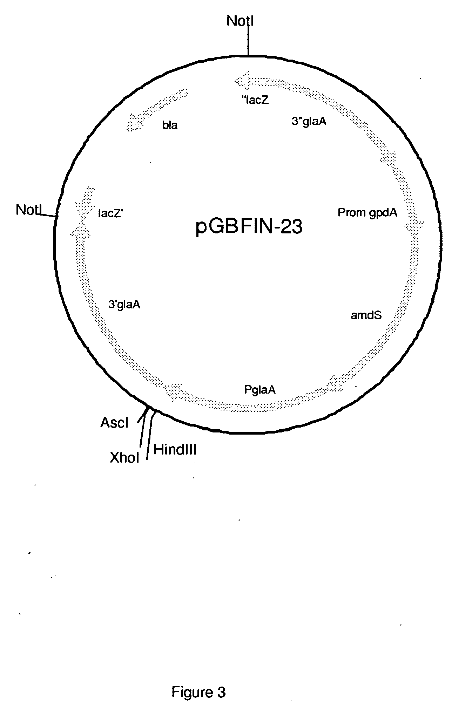

Figure 3 shows a physical plasmid map of the expression vector pGBFIN-23, which illustrates recognition sites for restriction enzymes, the locations of genes, and their orientation. Indicated are the *glaA* flanking regions relative to the *glaA* promoter and the *Hin*dIII*-Xho*I cloning site. The *E. coli* DNA can be removed by digestion with restriction enzyme *Not*I prior to transformation of an *A. niger* strain.

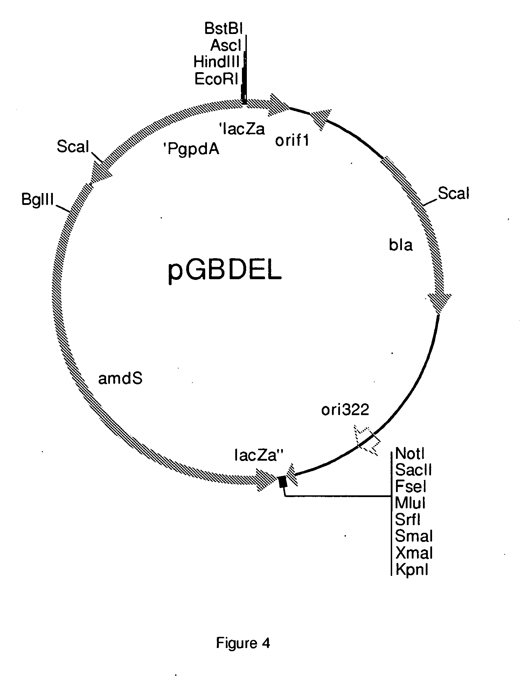

Figure 4 shows a physical plasmid map of the replacement vector pGBDEL, which illustrates recognition sites for restriction enzymes, the locations of genes, and their orientation. Indicated are the multiple cloning sites for cloning the flanking regions relative to the *amd*S marker.

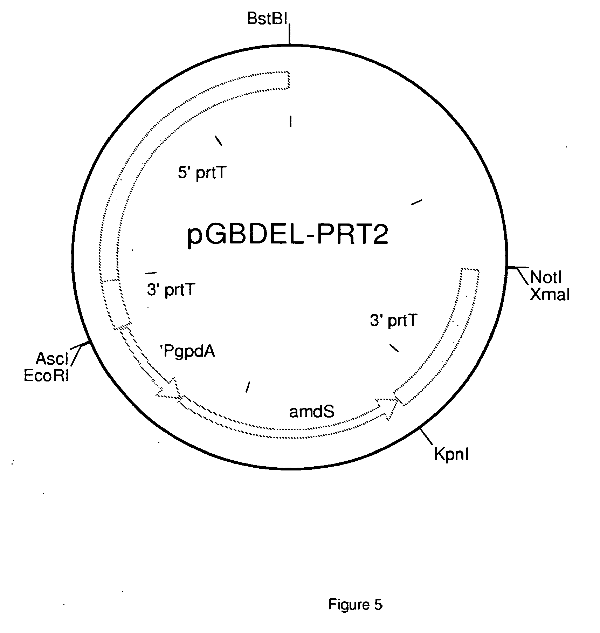

Figure 5 shows a physical plasmid map of the replacement vector pGBDEL-PRT2, which illustrates recognition sites for restriction enzymes, the locations of genes, and their orientation. Indicated are the 5' *prt*T flanking region and the 3' *prt*T flanking regions relative to the *amd*S marker. The sequences of the 3' *prt*T flanking regions overlap by at least a few hundred basepairs. E. *coli* DNA was removed by digestion with restriction enzymes *Bst*BI and *Xma*I*,* and subsequent recircularization prior to transformation of an *A. niger* strain.

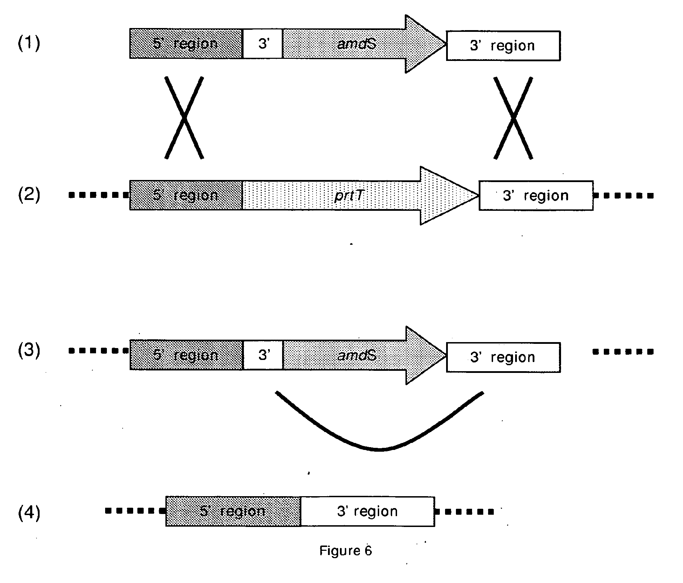

Figure 6 illustrates a procedure for deletion of the chromosomal *prt*T gene. A linear DNA construct of pGBDEL-PRT2 comprising the *amd*S selection marker flanked by homologous regions (5' and 3') of the *prtT gene* (1), integrates through double-crossover homologous recombination (X) in the genome at the *prtT* genetic locus (2), and replaces the chromosomal *prtT* gene (3). Subsequently, recombination between the direct repeats (3', 3' region) removes the *amd*S marker and results in precise excision of the *prtT* gene (4).

### Detailed description of the Invention

The DNA binding site can be defined structurally by a consensus nucleotide sequence and functionally by PrtT's ability to regulate transcriptional activation through that sequence. The DNA binding site was identified as a conserved nucleotide sequence (SEQ ID NO: 22 is the extended sequence) in endogenous promoters of native fungal protease genes. Both PrtT and its cognate DNA binding site (*i.e*., the nucleotide sequence in each promoter that is recognized by PrtT and confers to the gene ability to be transcriptionally controlled by PrtT) can be used in a gene expression system either (i) to improve a method for producing polypeptides in a PrtT-containing cell or (ii) to improve a method for producing a protease-sensitive polypeptide in a PrtT-containing cell. Possession of only a PrtT transcriptional activator is insufficient, because locating its cognate DNA binding site in the promoter is necessary for recognition by PrtT (*i.e.,* binding to the site and activating transcription from the promoter under appropriate conditions). A functional site, such as one obtained from a wild-type fungal protease gene, will confer PrtT-dependent transcriptional activation on 3'-downstream sequences. Conversely, mutation of a wild-type promoter that results in a non-functional site will abolish PrtT-dependent transcriptional activation of 3'-downstream sequences. In the sequence listing of the present invention SEQ ID NO: 22 depicts the PrtT DNA binding site as [snnnnnccgw cggnnnnnnn nnnnnnnnnn nns]. For clarity reasons, in the description of the present invention SEQ ID NO: 22 will be depicted as 5'-G/C (N)₅ C C G A/T C G G (N)₁₉ G/C-3', i_{.}e. G/C for S, and A/T for W.

A polynucleotide is disclosed that comprises one or more double-stranded DNA binding sites. At least 32 bases of a first strand of a site are identical in sequence to 5'-G/C (N)₅ C C G A/T C G G (N)₁₉ G/C-3' (SEQ ID NO:22). Since 24 bases in the extended DNA binding site may be degenerate, there can be changes in the sequence at only nine positions to alter binding affinity of PrtT and/or to alter PrtT activated transcription of a downstream nucleotide sequence. A second strand of the site is complementary to the first strand. The site is specifically bound by a PrtT transcriptional activator under binding conditions and such binding will activate transcription of a downstream nucleotide sequence. The asymmetry of the extended sequence above or the shorter 5'-C C G A/T C G G-3' is helpful in defining the direction of transcription as being in the 5' → 3' direction. It is preferred that a functional DNA binding site conserve the imperfect palindromic sequence of seven contiguous bases. The double-stranded DNA binding site may be obtained from a eukaryote (e.g., fungus).

A polynucleotide is disclosed comprising one or more double-stranded mutated, non-functional DNA binding sites. A double-stranded DNA binding site (*e.g*., at least 32 bases of a first strand of the non-mutated site are identical in sequence to 5'-G/C (N)₅ C C G A/T C G G (N)₁₉ G/C-3', SEQ ID NO:22) is obtained from a eukaryote (*e*.*g*., fungus), wherein the non-mutated, functional site is specifically bound by a PrtT transcriptional activator under binding conditions and such binding will activate transcription of a downstream nucleotide sequence. But at least one (e.g., one, two, three, four, five, six, seven, eight, or nine) bases of a first strand of the mutated site are changed as compared to the sequence of the non-mutated site (e.g., by addition, deletion, transition, transversion, or any combination thereof of bases in the extended sequence) such that PrtT no longer specifically recognizes the mutated, non-functional site or that PrtT no longer activates transcription. Since 24 bases in the extended DNA binding site may be degenerate, there can be changes in the sequence at a maximum of nine positions to alter binding affinity of PrtT and/or to alter PrtT activated transcription of a downstream nucleotide sequence. A second strand of the mutated, non-functional site is complementary to the first strand. The non-mutated site was specifically bound by a PrtT transcriptional activator under binding conditions and such binding would have activated transcription downstream. It is preferred that a mutated, non-functional DNA binding site has one or more changes in the imperfect palindromic sequence of seven contiguous bases.

A polynucleotide is disclosed that comprises one or more double-stranded mutated, enhanced DNA binding sites that demonstrate enhanced transcription of a downstream nucleotide sequence as compared to the non-mutated DNA binding site. A double-stranded DNA binding site (*e.g*., at least 32 bases of a first strand of the non-mutated site are identical in sequence to 5'-G/C (N)₅ C C G A/T C G G (N)₁₉ G/C-3', SEQ ID NO:22) is obtained from a eukaryote (e.g., fungus), wherein the non-mutated and mutated, enhanced sites are specifically bound by a PrtT transcriptional activator under binding conditions and such binding will activate transcription of a downstream nucleotide sequence. But at least one (*e.g*., one, two, three, four, five, six, seven, eight, or nine) bases of a first strand of the mutated site are changed as compared to the sequence of the non-mutated site (e.g., by addition, deletion, transition, transversion, or any combination thereof of bases in the extended sequence) such that PrtT specifically recognizes the site and activates and enhances transcription of the downstream nucleotide sequence. Since 24 bases in the extended DNA binding site may be degenerate, there can be changes in the sequence at a maximum of nine positions to alter binding affinity of PrtT and/or to alter PrtT activated transcription of a downstream nucleotide sequence. A second strand of the mutated site is complementary to the first strand. The non-mutated and mutated, enhanced sites are specifically bound by a PrtT transcriptional activator under binding conditions and such binding would have activated transcription downstream. It is preferred that a mutated, enhanced DNA binding site has one or more changes in both the imperfect palindrome sequence of seven contiguous bases and in the extended sequence.

The term "enhanced transcription of a nucleotide sequence downstream of a mutated DNA binding site" in the context of the present invention is defined as at least 10% more, at least 20% more, at least 30% more, at least 40% more, at least 80% more, at least 100% more, at least 200% more, or at least 300% more transcription product than a corresponding nucleotide sequence downstream of a non-mutated DNA binding site when expressed under the same conditions using the same assay. Assays to monitor the amount of expression product are known to the person skilled in the art. Examples of such assays to monitor the amount of expression product are: Northern Blot, Quantititive Polymerase Chain Reaction (PCR), Real Time PCR, Gene Array analysis.

A polynucleotide may be isolated from a eukaryotic source (e.g., fungus). For example, it may be cloned by screening a source library of recombinant sequences or amplifying a fragmentary sequence from source DNA. Thus, it may be purified to any desired degree from the eukaryote (e.g., at least 90% of solutes in a composition are the desired nucleic acid molecules). A promoter sequence from a non-fungal source that does not possess a PrtT transcriptional activator may fortuitously be identical to SEQ ID NO:22 or similar enough to be corrected to SEQ ID NO:22 and, therefore, regulated by PrtT in a cell. It may be recombinant if DNA which is not colinear in the genome are joined together to construct a recombinant polynucleotide. At least some parts of the polynucleotide are likely to have been obtained from sources other than the cell in which the polynucleotide is replicated.

In an expression vector, the base contacts, orientation, and spacing of the one or more DNA binding sites is typical for a zinc finger-containing transcription factor such as PrtT. The DNA binding site may be inserted into the expression vector for optimal or suboptimal transcriptional activation of a downstream nucleotide sequence (e.g., protease gene or other gene, either native or heterologous) located within 1200 bases, 800 bases, or between 100 and 1200 bases of the site of transcriptional initiation. The downstream nucleotide sequence (e.g. coding sequence) may be a non-protease gene, a non-fungal gene, or both. Other control sequences of the promoter may or may not be derived from a fungal gene.

Two different types of.PrtT-expressing fungal cells may be engineered using the above embodiments of the invention. Fungal cells are engineered by inserting functional DNA binding sites into the chromosome or on an episome to confer PrtT-dependent transcriptional activation on a nucleotide sequence downstream of the site. This type of fungus will coordinately transcribe such sequences with protease genes. Alternatively, fungal cells are engineered by replacing chromosomal or episomal DNA binding sites with mutated, non-functional sites to abolish PrtT-responsiveness because a promoter comprising the mutated, non-functional site is no longer recognized by PrtT. Native protease genes may be inactivated in this manner; PrtT-dependent transcriptional activation of a nucleotide sequence downstream of a functional site may also be inactivated. This type of fungus is suitable for expressing native or heterologous polypeptides that are sensitive to protease degradation. The polynucleotide herewith disclosed may be a recombination vector comprising a functional or non-functional DNA binding site flanked by homologous sequences of the target locus and a selectable marker to detect recombination, but does not require the presence of a downstream coding sequence for expression by the promoter. Preferably the homologous flanking sequences comprise at least 30 bases, more preferably at least 50 bases, more preferably at least 100 bases, at least 200 bases, more preferably at least 500 bases, even more preferably at least 1 kb, most preferably at least 2 kb of the target locus of the host cell. By rendering a PrtT binding site non-functional, specific proteases can be inactivated, depending on the protein of interest to be produced (e.g. inactivation of proline specific proteases when the protein of interest is rich in prolines, or inactivate carboxypeptidases when the protein of interest is sensitive to degradation by carboxypeptidases). In contrast, global inactivation of proteases by deletion of the PrtT gene has the disadvantage that those proteases necessary for intracellular processes and protein regulation will be inactivated as well, leading to an impaired expression host cell. Furthermore, other genes than proteases (e.g. genes encoding transporters) controlled by PrtT can be essential for the cell, and inactivation of PrtT may be lethal for those cells.

PrtT polypeptides are transcriptional activators of protease genes acting at DNA binding sites in their promoters. The term "transcriptional activator" as used herein refers to a polypeptide which has the ability to activate transcription from amongst others: a specific protease promoter or a set of protease promoters. PrtT is necessary for the initiation of transcription of the protease gene (or other heterologous nucleotide sequence) to which the promoter is operably linked.

The biological activity of a DNA binding site which is contained in a protease promoter may be determined by measuring the protease's activity (e.g., proteolysis) as described herein for determination of acidic endo-protease activity using bovine serum albumin (BSA) as the substrate. This method is also described by van den Hombergh et al. (Curr. Genet. 28:299-308, 1995). Other methods for measuring a protease's activity can be found in WO 02/068623. Alternatively, the biological activity of the DNA binding site can be determined by measuring the mRNA level of the protease transcripts. The mRNA levels can, for example, be quantitated by hybridization (e.g., Northern or slot blotting) or RT-PCR. A filter binding assay, protein crosslinking, and chromatin immunoprecipitation may be used to identify polypeptide binding to the DNA binding site.

A reporter gene under the control of a promoter comprising the DNA binding site can be used as a surrogate for the protease or its activity. Measuring the biological activity of β₋galactosidase (lacZ) or green fluorescent protein (GFP) reporter has been described (Luo, Gene 163:127-131, 1995; Henriksen et al., Microbiol. 145:729-734, 1999). Alternatively, one or more specific protease reporter genes such as the pepstatin-sensitive extracellular aspartic protease encoding pepA gene can be used for measuring the activity of a DNA binding site. One or more sites regulate transcription of the reporter gene's promoter.

At least one mutation may be introduced in a DNA binding site by standard techniques, such as site-directed mutagenesis and PCR-mediated mutagenesis. Example of mutagenesis procedures are the QuickChange™ site-directed mutagenesis kit (Stratagerie), the Altered Sites^{®} II *in vitro* mutagenesis system (Promega), sequence overlap extension (SOE-PCR as described by Ho et al., Gene 77:51-59, 1989), or other PCR techniques (Griffin & Griffin, eds., Molecular Biology: Current Innovations and Future Trends, Horizon Scientific Press, Norfolk, UK). Random mutagenesis (*e.g.,* chemical or radiation damage) or error-prone DNA replication (e.g., nucleotide misincorporation) may also be used to introduce mutations in the DNA binding site.

The DNA binding site herewith disclosed may be obtained from any filamentous fungus. "Filamentous fungi" include all filamentous forms of the subdivision Eumycota and Oomycota. The filamentous fungi are characterized by a mycelia wall composed of chitin, cellulose, glucan, chitosan, mannan, and other complex polysaccharides. Vegetative growth is by hyphal elongation and carbon catabolism is obligately aerobic. Filamentous fungal species include, but are not limited to, those of the genus *Acremonium, Aspergillus, Aureobasidium, Cryptococcus,Filibasidium, Fusarium, Humicola, Magnaporthe, Mucor, Myceliophthora, Neocallimastix, Neurospora, Paecilomyces, Penicillium, Piromyces, Schizophyllum, Talaromyces, Thermoascus, Thielavia, Tolypocladium,* and *Trichoderma.*

The DNA binding site may be obtained from a species of *Aspergillus,* such as A. *awamori, A. nidulans, A. niger, A. oryzae, A. sojae,* or *A. fumigatus.* Preferably, it is obtained from a strain of A. *niger, A. oryzae,* or *A. fumigatus.* Alternatively, it is obtained from a species of *Penicillium,* such as *P. chrysogenum,* or a species of *Fusarium,* such as F. *oxysporum* or *F. venenatum.*

It will be understood that for the aforementioned species, the invention encompasses both the perfect and imperfect states, and other taxonomic equivalents, e.g., anamorphs, regardless of the species name by which they are known. Those skilled in the art will readily recognize the identity of appropriate equivalents. For example, the polypeptides may be obtained from microorganisms, which are taxonomic equivalents of *Aspergillus* as defined by Raper & Fennel (The Genus Aspergillus, Wilkins Company, Baltimore MD, 1965) regardless of the species name by which they are known.

*Aspergilli* are mitosporic fungi characterized by an aspergillum comprising a conidiospore stipe with no known teleomorphic states terminating in a vesicle, which in turn bears one or two layers of synchronously formed specialized cells, variously referred to as sterigmata or phialides, and asexually formed spores referred to as conidia. Known teleomorphs of *Aspergillus* include *Eurotium, Neosartorya,* and *Emericella.* Strains of *Aspergillus* and teleomorphs thereof are readily accessible to the public in a number of culture collections.

DNA binding sites may be obtained from strains of filamentous fungus such as for example *Aspergillus niger* CBS 513.88, *Aspergillus oryzae* ATCC 20423, IFO 4177, ATCC 1011, ATCC 9576, ATCC14488-14491, ATCC 11601, ATCC12892. *Aspergillus fumigatus* AF293 (CBS101355), *P. chrysogenum* CBS 455.95, *Penicillium citrinum* ATCC 38065, *Penicillium chrysogenum* P2, *Acremonium chrysogenum* ATCC 36225 or ATCC 48272, *Trichoderma reesei* ATCC 26921 or ATCC 56765 or ATCC 26921, *Aspergillus sojae* ATCC11906, *Chrysosporium lucknowense* ATCC44006, and derivatives thereof.

Furthermore, DNA binding sites may be identified and obtained from other sources including microorganisms isolated from nature *(e.g.,* soil, composts, water, *etc.).* Techniques for isolating microorganisms from the environment are known in the art. The nucleotide sequence may be derived with a labeled probe by screening a genomic library of another microorganism. Once a nucleotide sequence of at least one DNA binding site has been detected with the probe, the sequence may be isolated or cloned by utilizing known techniques (see, *e.g.,* Sambrook & Russell, Molecular Cloning: A Laboratory Manual, 3rd Ed., CSHL Press, Cold Spring Harbor, NY, 2001; and Ausubel et al., Current Protocols in Molecular Biology, Wiley InterScience, NY, 1995).

Low- to medium- to high-stringency conditions means prehybridization and hybridization at 42°C in 5x SSPE, 0.3% SDS, 200 pg/ml sheared and denatured salmon sperm DNA, and one of 25%, 35% or 50% formamide for low to medium to high stringencies, respectively. Subsequently, the hybridization reaction is washed three times for 30 min each using 2x SSC, 0.2% SDS and one of 55°C, 65°C or 75°C for low to medium to high stringencies, respectively. Oligonucleotide probes may be used. They are typically labeled for detecting corresponding DNA binding sites (e.g., with ³²P, ³³P, ³H, ³⁵S, biotin, avidin, or a fluorescent/luminescent marker). For example, molecules to which a ³²P-, ³³P-, ³H- or ³⁵S-labeled oligonucleotide probe hybridizes may be detected by use of X-ray film or Phospho-Image™ analysis.

A variant of the nucleotide sequence may also be a homologous or paralogous DNA binding site in a protease promoter. In the context of the invention, homologous or paralogous means nucleotide sequence identical or similar to 5'-C C G A/T C G G-3' and obtained from A. *niger, A. oryzae, A. fumigatus, P. chrysogenum, F. oxysporum,* or F. *venenatum.* For example, *Aspergillus* strains can be screened for a homologous or paralogous DNA binding site in a protease promoter by hybridization. Upon detection of a homologous or paralogous nucleotide sequence according to the present invention, a genomic DNA library can be screened using a probe that hybridizes to 5'-C C G A/T C G G-3' or more extended versions thereof, e.g. the extend versions as depicted Table 2 and 3.

The techniques used to isolate or clone a nucleotide sequence are known in the art and include direct isolation from genomic DNA. The cloning of a nucleotide sequence of the present invention from such genomic DNA can be accomplished by using known hybridization techniques.

"Polynucleotide" is defined herein as a double-stranded nucleic acid molecule, which is isolated from a naturally occurring gene or which has been modified to contain segments of nucleic acid which are combined and juxtaposed in a manner which would not otherwise exist in nature. "Expression vector" is defined as a double-stranded nucleic acid molecule, which contains control sequences required for expression of a coding sequence and maintenance (at least temporarily) in a cell. The term "coding sequence" as defined herein is a sequence, which is transcribed into mRNA and translated into a polypeptide. The boundaries of the coding sequence are generally determined by the ATG start codon at the 5' end of the mRNA and a translation stop codon sequence terminating the open reading frame at the 3' end of the mRNA. A coding sequence can include, but is not limited to, DNA, cDNA, and recombinant nucleotide sequences. Expression will be understood to include any step involved in the production of the polypeptide including, but not limited to, transcription, splicing, posttranscriptional modification, translation, posttranslational modification, secretion, and proteolytic processing.

The term "control sequences" is defined herein to include all components, which are necessary or advantageous for the expression of a polypeptide. Each control sequence may be native or foreign to the nucleotide sequence encoding the polypeptide. Such control sequences include, but are not limited to, a leader, optimal translation initiation sequences (as described in Kozak, J. Biol. Chem. 266:19867-19870, 1991), polyadenylation sequence, propeptide sequence, prepropeptide sequence, promoter, signal sequence, and transcription terminator. At a minimum, the control sequences include a promoter, and transcriptional and translational stop signals.

Manipulation of the nucleotide sequence encoding a polypeptide prior to its insertion into an expression vector may be desirable or necessary depending on the vector. The techniques for modifying nucleotide sequences utilizing cloning methods are known in the art. The control sequences may be provided with linkers for the purpose of introducing specific restriction sites facilitating ligation of the control sequences with the coding region of the nucleotide sequence encoding a polypeptide. The term "operably linked" is defined herein as a configuration in which a control sequence is appropriately placed at a position relative to the coding sequence such that the control sequence directs expression of a polypeptide or transcription of other downstream sequences.

The control sequence may be a promoter, which is recognized by the cellular machinery for expression of a downstream nucleotide sequence. The promoter contains transcriptional control sequences (e.g., one or more DNA binding sites of the present invention) that regulate the expression of the polypeptide or transcription of other downstream sequences. The promoter may be any nucleotide sequence, which shows transcriptional activity in the cell including mutant, truncated, and hybrid promoters, and may be obtained from genes encoding extracellular or intracellular polypeptides either native or heterologous to the cell.

The control sequence may also be a suitable transcription terminator sequence, a nucleotide sequence recognized by the cellular machinery to terminate transcription. The terminator sequence is operably linked to the 3' terminus of the nucleotide sequence encoding the polypeptide. Any terminator, which is functional in the cell, may be used in the present invention. Preferred terminators for filamentous fungal cells are obtained from the genes encoding A. *oryzae* TAKA amylase, A. *niger* glucoamylase *(glaA),* A. *nidulans* anthranilate synthase, *A. niger* alpha-glucosidase, *trp*C gene, and *Fusarium oxysporum* trypsin-like protease.

The control sequence may also be a suitable leader sequence, a non-translated region of a mRNA which is important for translation by the cell. The leader sequence is operably linked to the 5' terminus of the nucleotide sequence encoding the polypeptide. Any leader sequence, which is functional in the cell, may be used in the present invention. Preferred leaders for filamentous fungal cells are obtained from the genes encoding A. *oryzae* TAKA amylase and A. *nidulans* triose phosphate isomerase and A. *niger glaA.* Other control sequences may be isolated from the *Penicillium* IPNS gene, or *pcb*C gene, the beta tubulin gene (cited in WO 01/21779). .

The control sequence may also be a polyadenylation sequence, a sequence which is operably linked to the 3' terminus of the nucleotide sequence and which, when transcribed, is recognized by the filamentous fungal cell as a signal to add polyadenosine residues to transcribed mRNA. Any polyadenylation sequence, which is functional in the cell, may be used in the present invention. Preferred polyadenylation sequences for filamentous fungal cells are obtained from the genes encoding *A. oryzae* TAKA amylase, *A. niger* glucoamylase, *A. nidulans* anthranilate synthase, *Fusarium oxysporum* trypsin-like protease, and *A. niger* alpha-glucosidase.

### Expression Vectors

Herewith disclosed are expression vectors comprising a DNA binding site within the promoter, a downstream transcribed sequence, a transcriptional stop signal, a translational stop signal, and other control sequences for maintenance of the vector in a cell produced by recombinant technology. The various nucleotide and control sequences described above may be joined together to produce a recombinant expression vector which may include one or more convenient restriction sites to allow for insertion or substitution of the nucleotide sequence encoding the polypeptide at such sites.

A nucleotide sequence encoding a desired polypeptide may be expressed by inserting the nucleotide sequence or a polynucleotide comprising the nucleotide sequence into an appropriate vector for expression. In creating the expression vector, the coding sequence is located in the vector so that the coding sequence is operably linked with the appropriate control sequences (e.g., one or more DNA binding sites) for expression, and possibly secretion.

The expression vector may be any vector (e.g., a plasmid or virus), which can be conveniently engineered by recombinant technology and can bring about the expression of the nucleotide sequence encoding a desired polypeptide. The choice of the vector will typically depend on the compatibility of the vector with the cell (e.g., filamentous fungus) into which the vector is to be introduced. The vectors may be linear or a closed circle (i.e., episome). The vector may be an autonomously replicating vector, i.e., a vector, which exists as an extrachromosomal entity, the replication of which is independent of chromosomal replication, e.g., plasmid, extrachromosomal element, minichromosome, or artificial chromosome. An autonomously maintained cloning vector may comprise the AMA1-sequence (see, e.g., Aleksenko & Clutterbuck, Fungal Genet. Biol. 21:373-397, 1997). Positive and negative selectable markers in the vector may be used for genetic engineering. Maintenance functions and an origin of replication are essential for episomes. Engineered chromosomes may require a centromere, telomeres, and origins of replication for maintenance and segregation.

Alternatively, the vector may be one which, when introduced into the cell *(e.g.,* a filamentous fungus), is integrated into the genome and replicated together with the chromosome into which it has been integrated. The integrative cloning vector may integrate at random or at a predetermined target locus in the chromosome of the cell. In an embodiment of the present invention, the integrative cloning vector comprises a fragmentary region, which is homologous to a nucleotide sequence at a predetermined target locus in the genome of the cell for targeting integration of the cloning vector at the locus. In order to promote targeted integration, the cloning vector is preferably linearized prior to transformation of the cell. Linearization is preferably performed such that at least one but preferably either end.of the cloning vector is flanked by sequences homologous to the target locus to allow integration by homologous recombination. The skilled person will know the optimal length of a flanking sequence for a specific host cell to allow integration by homologous recombination. The length of the homologous sequences flanking the target locus is preferably at least 30 bp, preferably at least 50 bp, preferably at least 0.1 kb, even preferably at least 0.2 kb, more preferably at least 0.5 kb, even more preferably at least 1 kb, most preferably at least 2 kb. Preferably, the efficiency of targeted integration into the genome of the host cell, i.e. integration in a predetermined target locus, is increased by augmented homologous recombination abilities of the host cell. Such phenotype of the cell preferably involves a deficient ku70 gene as described in W02005/095624. W02005/095624, discloses a preferred method to obtain a filamentous fungal cell comprising increased efficiency of targeted integration. Preferably, a DNA sequence in the cloning vector, which is homologous to the target locus is derived from a highly expressed locus meaning that it is derived from a gene, which is capable of high expression level in the filamentous fungal host cell. A gene capable of high expression level, *i.e*. a highly expressed gene, is herein defined as a gene whose mRNA can make up at least 0.5% (w/w) of the total cellular mRNA, e.g. under induced conditions, or alternatively, a gene whose gene product can make up at least 1% (w/w) of the total cellular protein, or, in case of a secreted gene product, can be secreted to a level of at least 0.1 g/l (as described in EP 357127). A number of preferred highly expressed fungal genes are given by way of example: the amylase, glucoamylase, alcohol dehydrogenase, xylanase, glyceraldehyde-phosphate dehydrogenase or cellobiohydrolase *(cbh)* genes from *Aspergilli* or *Trichoderma.* Most preferred highly expressed genes for these purposes are a glucoamylase gene, preferably an *A. niger* glucoamylase. gene, an A. *oryzae* TAKA-amylase gene, an A. *nidulans gpdA* gene, a *Trichoderma reesei cbh* gene, preferably *cbh*1*.* They may be fungal genes regulated by the DNA binding sites of the present invention. More than one copy of a nucleotide sequence encoding a polypeptide may be inserted into the cell to increase production of the gene product. This can be done, preferably by integrating into the cell's chromosome of the nucleotide sequence, more preferably by targeting the integration of the nucleotide sequence at one of the highly expressed loci listed immediately above. Integration may be enhanced by a recombinase. Alternatively, this can be done by including an amplifiable selectable marker gene with the nucleotide sequence where cells containing amplified copies of the selectable marker gene, and thereby additional copies of the nucleotide sequence, can be selected for by cultivating the cells in the presence of the appropriate selectable agent. To increase even more the number of copies of the DNA sequence to be over expressed, the technique of gene conversion as described in WO 98/46772 may be used.

The vectors preferably contain one or more selectable markers, which permit easy selection of transformed cells. A selectable marker is a gene the product of which provides for biocide or viral resistance, resistance to heavy metals, prototrophy to auxotrophs, and the like. A selectable marker for use in a filamentous fungal cell may be selected from the group including, but not limited to, *amd*S (acetamidase), *arg*B (ornithine carbamoyltransferase), *bar* (phosphinothricinacetyltransferase), *ble*A (phleomycin binding), *hyg*B (hygromycinphosphotransferase), *nia*D (nitrate reductase), *pyr*G (orotidine-5'-phosphate decarboxylase), sC (sulfate adenyltransferase), and *trp*C (anthranilate synthase), as well as equivalents from other species. Preferred for use in an *Aspergillus* and *Penicillium* cell are the *amd*S (EP 635574, WO 97106261) and *pyr*G genes of *A. nidulans* or A. *oryzae* and the *bar* gene of *Streptomyces hygroscopicus.* More preferably an *amd*S gene is used, even more preferably an *amd*S gene from *A. nidulans* or *A. niger.* A most preferred selection marker gene is the *A.nidulans amdS* coding sequence fused to the *A.nidulans gpdA* promoter as disclosed in EP 635574, which is herein enclosed by reference. *Amd*S genes from other fungi may also be used, e.g. the ones disclosed in WO 97/06261.

The procedures used to join the above-described elements to construct an expression vector of the present invention are well-known in the art (e.g., Sambrook & Russell, Molecular Cloning: A Laboratory Manual, 3rd Ed., CSHL Press, 2001; Ausubel et al., Current Protocols in Molecular Biology, Wiley InterScience,1995).

### Host Cells and Other Engineered Cells

DNA binding sites herewith disclosed are preferably used for engineering one of two types of cells:
(i) a first type of cell that would be highly suited for producing a desired polypeptide, the desired polypeptide being under the control of one or more DNA binding sites and
(ii) a second type of cell that would be highly suited for producing a desired polypeptide, the desired polypeptide being sensitive to protease degradation. In this type of cell, at least one DNA binding site in the host cell's genome is mutated in accordance with the second aspect of the invention such that PrtT no longer specifically recognizes the mutated, non-functional site or that PrtT no longer activates transcription.

Optionally, both types of cells additionally comprise a polynucleotide or an expression vector comprising at least one functional or mutated (*i.e.,* non-functional, or enhanced) DNA binding site, which is operably linked upstream of a transcribed nucleotide sequence (e.g:, encoding a polypeptide sensitive to protease degradation, a polypeptide under the control of a PrtT transcriptional activator, the PrtT transcriptional activator itself, or another polypeptide to be produced).

Optionally, the host cell comprises an elevated unfolded protein response (UPR) to enhance production abilities of a polypeptide of interest. UPR may be increased by techniques described in US 2004/0186070 and/or US 2001/0034045 and/or WO 01/72783. More specifically, the protein level of HAC1 and/or IRE1 and/or PTC2 has been modulated in order to obtain a host cell having an elevated UPR. Alternatively, or in combination with an elevated UPR and/or a phenotype displaying lower protease expression and/or protease secretion, the host cell displays an oxalate deficient phenotype in order to enhance the yield of production of a polypeptide of interest. An oxalate deficient phenotype may be obtained by techniques described in WO2004/070022A2.
Alternatively, or in combination with an elevated UPR and/or oxalate deficiency, the host cell displays a combination of phenotypic differences compared to the wild cell to enhance the yield of production of the polypeptide of interest. These differences may include, but are not limited to, lowered expression of glucoamylase and/or neutral alpha-amylase A and/or neutral alpha-amylase B, protease, and oxalic acid hydrolase. Said phenotypic differences displayed by the host cell may be obtained by genetic modification according to the techniques described in US2004/0191864A1.

The choice of a host cell will to a large extent depend upon the source of the nucleotide sequence encoding the desired polypeptide to be produced. Preferably, the host cell is a filamentous fungal cell as defined earlier in the description as a source where the DNA binding site may be obtained from. The host cell may also be a host cell as disclosed in WO 01/68864 or WO 00/20596. The introduction of an expression vector or other polynucleotide into a filamentous fungal cell may involve a process consisting of protoplast formation, transformation of the protoplasts, and regeneration of the cell wall in a known manner. Suitable procedures for transformation of *Aspergillus* cells are described in EP 238023 and Yelton et al. (Proc. Natl. Acad. Sci. USA 81:1470-1474, 1984). A suitable method of transforming *Fusarium* species is described by Malardier et al. (Gene 78:147-156, 1989) or in WO 96/00787. The expression vector or nucleic acid construct that can be used were already described under the corresponding sections.

### Production of a Polypeptide Sensitive to Protease Degradation

It is herewith disclosed a host cell with a reduced protease phenotype, which cell is a mutant of a parent cell useful for the production of a polypeptide sensitive to protease degradation, in which the parent cell comprises one or more nucleotide sequences encoding proteases, the transcription of which is activated by a PrtT transcriptional activator, and the mutant cell transcribes fewer protease genes than the parent cell when cultured under the same conditions because the protease promoters comprise one or more mutated, non-functional PrtT binding sites.

A preferred method for measurement of protease activity in a host cell is described in the example section herein for determination of the acidic endo-protease activity using bovine serum albumin (BSA) as substrate. A detailed description of this method is also described by van den Hombergh et al. (Curr. Genet. 28:299-308, 1995). Measurement of protease(s) also may be assayed using other known methods. In one such method, an aliquot of a 48 hr culture media is incubated with ³H-labeled sperm whale myoglobin at pH 4.0 and the radioactivity in the TCA-soluble fraction is measured (van Noort et al., Anal. Biochem. 198:385-390, 1991). Other methods have been described for identifying, e.g., aspartic proteinase A of *A. niger* (Takahashi, Meth. Enzymol. 248:146-155, 1991), endopeptidases (Morihara, Meth. Enzymol. 248:242-253, 1995), carboxypeptidases (Reminton & Breddam, Meth. Enzymol. 244:231-248, 1994), dipeptidyl peptidase (Ikehara et al., Meth. Enzymol. 244:215-227, 1994), and aminopeptidases (Little et al., Meth. Enzymol. 45:495-503, 1976). Alternatively other protease assays may be used such as the one described in WO 02/068623. Alternatively, the assay used may be Northern blotting, the use of a reporter gene under the control of a protease promoter, or a western blotting or a DNA array analysis (Eisen & Brown, Meth. Enzymol. 303:179-205, 1999) as also described herein.

A mutant cell may produce fewer proteases and less protease activity than the parental cell that is used as a reference when measured by any one of the given assays. A mutant *A.niger* may produce fewer proteases and less protease activity than the deposited *A.niger* CBS 513.88. A mutant *A. oryzae* may produce fewer proteases and less protease activity than the deposited A. *oryzae.* A mutant *P. chrysogenum* may produce fewer proteases and less protease activity than the deposited *P.chrysogenum* CBS 455.95. A mutant A. *fumigatus* may produce fewer proteases and less protease activity than *A. fumigatus* AF293 (CBS 101355).

A mutant cell may be obtained by genetic engineering using recombinant genetic manipulation techniques, submitting the filamentous fungus to mutagenesis, or both. Using genetic manipulation techniques, it is preferred to obtain a recombinant fungus: preferably by deleting a DNA binding site recognized by a PrtT transcriptional activator, more preferably the deleted DNA binding site is replaced by a non-functional variant thereof, and most preferably the deletion and replacement are made as described in EP 357127.

In one embodiment the present invention provides a method for the production of a mutant of a parent filamentous fungal host cell, said mutant producing fewer proteases and less protease activity than the parent host cell, comprising:
- provision of a parent filamentous fungal host cell,
- modification or inactivation of a prtT binding site according to SEQ ID NO: 22 rendering said prtT binding site non-functional.
Preferably the modification or inactivation comprises introducing one or more changes in the imperfect palindrome site C C G A/T C G G.
Preferably the filamentous fungal host cell is an *Aspergillus* cell.

The mutant cell may be obtained by modification or inactivation of a DNA binding site recognized by a PrtT transcriptional activator present in the cell and necessary for expression of a downstream sequence. Expression of proteases in the mutant cell may thereby be reduced or eliminated.

Modification or inactivation of the DNA binding site of the present invention may result from subjecting the parent cell to mutagenesis and selecting for mutant cells in which the ability to express proteases has been reduced by comparison to the parental cell. The mutagenesis, which may be specific or random, may be performed, for example, by use of a suitable physical or chemical mutagenizing agent, by use of a suitable oligonucleotide, or by subjecting the DNA sequence to PCR-generated mutagenesis. Furthermore, the mutagenesis may be performed by use of any combination of these mutagenizing agents.

Examples of a physical or chemical mutagenizing agent suitable for the present purpose include gamma or ultraviolet (UV) radiation, hydroxylamine, N-methyl-N'-nitro-N-nitrosoguanidine (MNNG), O-methyl hydroxylamine, nitrous acid, ethyl methane sulphonate (EMS), sodium bisulphite_{,} formic acid, and nucleotide analogs. When such agents are used, the mutagenesis is typically performed by incubating the parent cell to be mutagenized in the presence of the mutagenizing agent of choice under suitable conditions, and selecting for mutant cells exhibiting reduced expression of the gene.

The filamentous fungus obtained may be subsequently selected by monitoring the expression level of the desired polypeptide and/or any protease known to be under control of the PrtT transcriptional activation. Optionally, the filamentous fungus is subsequently selected by measuring the expression level of a given gene of interest to be expressed in the host cell.

The mutant cell, which has been modified or inactivated by any of the methods described above and produces fewer proteases and less protease activity than the parent cell when cultured under identical conditions as measured using the same assays as defined before, may harbor another nucleotide sequence. The mutant cell produces preferably at least 25% less, more preferably at least 50% less, even more preferably at least 75% less, and most preferably at least 95% less protease activity than the parent cell when cultured under identical conditions using the same assays as defined before. The filamentous fungus *Aspergillus niger, Aspergillus oryzae, Aspergillus fumigatus, Penicillium chrysogenum, F. oxysporum,* or *F. venenatum* mutant cell may produce fewer proteases and less protease activity than the corresponding deposited filamentous fungus cited earlier when cultured under identical conditions using the same assays as defined before.

Polypeptides are consequently produced in a host cell of the present invention with a reduced protease phenotype, which cell is a mutant of a parent cell useful for the production of a polypeptide sensitive to protease degradation, in which the parent cell comprises one or more nucleotide sequences encoding proteases, the transcription of which is activated by a PrtT transcriptional activator, and the mutant cell transcribes fewer protease genes than the parent cell when cultured under the same conditions because the protease promoters comprise one or more mutated, non-functional DNA binding sites.

A polypeptide may be produced by a method comprising:
(a) cultivating the host cell with reduced protease phenotype in a nutrient medium, under conditions conducive to expression of the polypeptide
(b) expressing the polypeptide in said host cell, and
(c) optionally recovering the polypeptide from the nutrient medium or from the host cell.

A polypeptide may be produced by a method comprising:
(a) transforming the host cell with reduced protease phenotype with an expression vector, wherein the vector expresses the polypeptide,
(b) cultivating the host cell in a nutrient medium, under conditions conducive to expression of the polypeptide
(c) expressing the polypeptide in the host cell, and
(d) optionally recovering the polypeptide from the nutrient medium or from the host cell.

In another embodiment the present invention provides a method for the production of a polypeptide comprising:
- provision of a parent filamentous fungal host cell,
- modification or inactivation of a prtT binding site according to SEQ ID NO: 22 rendering said prtT binding site non-functional,
- cultivating under conditions conducive to expression of the polypeptide, the mutant filamentous fungal host cell,
- expressing the polypeptide in said cell, and
- optionally recovering the polypeptide from the nutrient medium or from the host cell.
Preferably the method additionally comprises providing the host cell with a polynucleotide encoding a polypeptide of interest.
Preferably in the method according to the invention the modification or inactivation comprises introducing one or more changes in the imperfect palindrome site C C G A/T C G G.
Preferably in the method according to the invention the filamentous fungal host cell is an *Aspergillus* cell.

### Production of Other Native or Heterologous Polypeptides and Other Sequences

Herewith are disclosed methods of transcribing a downstream nucleotide sequence using a PrtT transcriptional activator in a host cell, wherein the transcribed sequence encodes a desired polypeptide or is a functional nucleic acid molecule, comprising:
(a) cultivating, in a nutrient medium, a host cell comprising (i) a promoter, (ii) a transcriptional stop signal, a (iii) translational stop signal and (iv) a non-mutated and/or a mutated, enhanced DNA binding site of the present invention, further comprising a downstream nucleotide sequence which encodes a polypeptide, wherein transcription of the downstream nucleotide sequence is activated by PrtT,
(b) expressing the polypeptide in the host cell, and
(d) optionally, recovering the polypeptide from the nutrient medium or from the host cell.
The polypeptide produced may be sensitive to protease degradation. In this case, a mutant host cell which is protease deficient will be used. The protease deficient host cell is preferably produced according to the method of the present invention. Fungi may be grown or maintained in a nutrient medium suitable for production of the desired polypeptide using methods known in the art. For example, cells may be plated on a solid substrate, shaken in a flask, cultivated in small-scale or large-scale fermentation (including continuous, batch, fedbatch, or solid-state fermentation) in laboratory or industrial fermentors in a suitable medium and under conditions allowing the polypeptide to be expressed and/or isolated. Cultivation takes place in a suitable nutrient medium comprising carbon and nitrogen sources and inorganic salts, using procedures known in the art (see, *e.g*., Bennett & LaSure, eds., More Gene Manipulations in Fungi, Academic Press, CA, 1991). Suitable media are available from commercial suppliers or may be prepared using published compositions (*e.g*., in catalogues of the American Type Culture Collection). If the polypeptide is secreted into the nutrient medium, the polypeptide can be recovered directly from the medium. If the polypeptide is not secreted, it can be recovered from cell lysates.

The resulting polypeptide may be isolated by methods known in the art. For example, the polypeptide may be isolated from the nutrient medium by conventional procedures including, but not limited to, centrifugation, filtration, extraction, spray drying, evaporation, or precipitation. The isolated polypeptide may then be further purified by a variety of procedures known in the art including, but not limited to, chromatography (*e.g*., ion exchange, affinity, hydrophobic, chromatofocusing, or size exclusion), electrophoresis *(e.g.,* preparative isoelectric focusing), differential solubility *(e.g.,* acetone or ammonium sulfate precipitation), or extraction (*e.g*., chaotrope, salt, or pH). See, *e.g.,* Janson & Ryden, eds., Protein Purification, VCH Publishers, New York, 1989.

The polypeptide may be detected using methods known in the art that are specific for the polypeptide. These detection methods may include use of specific antibodies, formation of an enzyme product, disappearance of an enzyme substrate, or SDS-PAGE. For example, an enzyme assay may be used to determine the activity of the polypeptide. Procedures for determining enzyme activity are known in the art for many enzymes.

Cells may produce at least 20% more, at least 50% more, at least 100% more, at least 200% more, or at least 300% more of the polypeptide than a corresponding parent cell when cultivated under the same conditions using one of the given assays. Preferably, the parent cell is one of the deposited strains cited earlier as host cell or as source of the DNA binding site sequence.

The polypeptide may be any polypeptide whether native or heterologous to the filamentous fungal cell. The term "heterologous polypeptide" is defined herein as a polypeptide, which is not produced by a wild-type filamentous fungal cell. The term "polypeptide" is not meant herein to refer to a specific length of the encoded produce and therefore encompasses peptides, oligopeptides and proteins. The nucleotide sequence encoding a heterologous polypeptide may be obtained from any prokaryote, eukaryote, or other source and may be a synthetic gene. The term "obtained from" as used herein in connection with a given source shall mean that the polypeptide is produced by the source or by a cell in which a gene from the source has been inserted.

The desired polypeptide may be an antibody or antigen-binding portion thereof, antigen, clotting factor, enzyme, peptide hormone or variant thereof, receptor or ligand-binding portion thereof, regulatory protein, structural protein, reporter, transport protein, intracellular protein, protein involved in a secretory process, protein involved in a folding process, chaperone, peptide amino acid transporter, glycosylation factor, or transcription factor. The polypeptide may be secreted extracellularly into culture medium.

Enzymes include oxidoreductases, transferases, hydrolases, lyases, isomerases, ligases, catalases, cellulases, chitinases, cutinase, deoxyribonuclease, dextranases, and esterases. The polypeptide may be a carbohydrase, *e.g*. cellulases such as endoglucanases, β-glucanases, cellobiohydrolases, and β-glucosidases, hemicellulases or pectinolytic enzymes such as xylanases, xylosidases, mannanases, galactanases, galactosidases, pectin methyl esterases, pectin lyases, pectate lyases, endo polygalacturonases, exopolygalacturonases rhamnogalacturonases, arabanases, arabinofuranosidases, arabinoxylan hydrolases, galacturonases, lyases, and amylolytic enzymes; hydrolase, isomerase, or ligase, phosphatases such as phytases, esterases such as lipases, phospholipases, galactolipases, proteolytic enzymes, carboxypeptidase, endo-protease, metallo-protease, serine-protease, amino peptidase, oxidoreductases such as oxidases, transferases, and isomerases. More preferably, the desired polypeptide is a porcine phospholipase. The polypeptide may be an amylase, carbohydrase, catalase, chitinase, cutinase, cyclodextrin glycosyltransferase, deoxyribonuclease, esterase, alpha-galactosidase, beta-galactosidase, glucoamylase, alpha-glucosidase, beta-glucosidase, haloperoxidase, proteolytic enzyme, invertase, laccase, lipase, mannosidase, mutanase, oxidase, pectinolytic enzyme, peroxidase, phospholipase, polyphenoloxidase, ribonuclease, transglutaminase, glucose oxidase, hexose oxidase, or monooxygenase.

The polypeptide may be human insulin or an analog thereof, human growth hormone, human erythropoietin, human tissue plasminogen activator (tPA), or human insulinotropin.

Alternatively the polypeptide may be an intracellular protein or enzyme such as, for example, a chaperone, protease, or transcription factor. An example of this is described by Punt et al. (Appl. Microbiol. Biotechnol. 50:447-454, 1998). This can be used for example to improve the efficiency of a host cell as protein producer if this polypeptide, such as a chaperone, protease, or transcription factor, is known to be a limiting factor in protein production.

In the methods of the present invention, the filamentous fungal cells may also be used for the recombinant production of polypeptides, which are native to the cell. The native polypeptides may be recombinantly produced by, *e.g.,* placing a gene encoding the polypeptide under the control of a different promoter to enhance expression of the polypeptide, to expedite export of a native polypeptide of interest outside the cell by use of a signal sequence, and to increase the copy number of a gene encoding the polypeptide normally produced by the cell. The present invention also encompasses, within the scope of the term "heterologous polypeptide", such recombinant production of polypeptides native to the cell, to the extent that such expression involves the use of genetic elements not endogenous to the cell, or use of endogenous sequence elements which have been manipulated to function in a manner that do not normally occur in the filamentous fungal cell. The techniques used to isolate or clone a nucleotide sequence encoding a heterologous polypeptide are known in the art and include isolation from genomic DNA, preparation from cDNA, or a combination thereof.

In the methods of the present invention, heterologous polypeptides may also include a fused or hybrid polypeptide in which another polypeptide is fused at the N-terminus or the C-terminus of the polypeptide or fragment thereof. A fused polypeptide is produced by fusing a nucleotide sequence (or a portion thereof) encoding one polypeptide to a nucleotide sequence (or a portion thereof) encoding another polypeptide.

Techniques for producing fusion polypeptides are known in the art, and include, ligating the coding sequences encoding the polypeptides so that they are in frame and expression of the fused polypeptide is under control of the same promoter (s) and terminator. The hybrid polypeptides may comprise a combination of partial or complete polypeptide sequences obtained from at least two different polypeptides wherein one or more may be heterologous to the mutant fungal cell. An isolated nucleotide sequence encoding a heterologous polypeptide of interest may be manipulated in a variety of ways to provide for expression of the polypeptide. Expression will be understood to include any step involved in the production of the polypeptide including, but not limited to, transcription, posttranscriptional modification, translation, posttranslational modification, and secretion. Manipulation of the nucleotide sequence encoding a polypeptide prior to its insertion into a vector may be desirable or necessary depending on the expression vector. The techniques for modifying nucleotide sequences utilizing cloning methods are well known in the art.

Alternatively, the downstream transcribed sequence does not encode any polypeptide but is a functional nucleic acid molecule instead. Splicing or endonucleolytic/exonucleolytic processing of nascent transcripts may be required posttranscriptionally. The nucleic acid molecule may be an antisense or siRNA molecule.

Modification or inactivation of a host gene may be performed by established antisense techniques using a nucleotide sequence complementary to the nucleotide sequence of the gene. More specifically, expression of the gene by a filamentous fungal cell may be reduced or eliminated by introducing a nucleotide sequence complementary to the nucleotide sequence of the fungal gene, which may be transcribed in the cell and is capable of hybridizing to the mRNA produced in the cell. Under conditions allowing the complementary antisense nucleotide sequence to hybridize to the mRNA, the amount of protein translated is thus reduced or eliminated. Examples of expressing an antisense RNA is provided by Ngiam et al. (Appl. Environ. Microbiol. 66:775-782, 2000) and Zrenner et al. (Planta 190:247-252, 1993).

Modification, downregulation, or inactivation of a host gene may be obtained via RNA interference (RNAi) techniques *(*FEMS Microb. Lett. 237:317-324, 2004). More specifically, expression of the gene by a filamentous fungal cell may be reduced or eliminated by cloning identical sense and antisense portions of the nucleotide sequence, which expression is to be affected, behind each other with a nucleotide spacer in between, inserting into an expression vector, and introducing the expression vector into the cell where double-stranded RNA (dsRNA) may be transcribed and then processed to shorter siRNA that is able to hybridize to target mRNA. After dsRNA is transcribed, formation of small (21-23) nucleotide siRNA fragments will lead to a targeted degradation of the mRNA, which is to be affected. The elimination of the specific mRNA can be to various extents. The RNA interference techniques described in WO 2005/05672 and WO 2005/026356 may be used for modification, downregulation, or inactivation of the host gene.

### Processes for the identification of protease genes.

Processes for identifying protease genes are disclosed. One or more differentially expressed genes are detected in corresponding fungal cells either with or without a PrtT transcriptional activator *(e.g.,* deleting *prt*T or otherwise inactivating PrtT). Gene expression can be determined by methods known to the person skilled in the art. Examples of such expression analyses are aforementioned in the description and include: Northern Blot, Real Time PCR and RT-PCR. Those differentially expressed genes that encode proteases (e.g., as determined by sequence similarity to known proteases, proteolytic activity of the gene product, and possession of a DNA binding site for PrtT) are identified as protease genes controlled by PrtT.

The present invention is further described by the following examples, which should not be construed as limiting the scope of the invention.

### Examples

In the examples described herein, standard molecular cloning techniques such as isolation and purification of nucleic acids, electrophoresis of nucleic acids, enzymatic modification, cleavage and/or amplification of nucleic acids, transformation of *E.coli,* etc., were performed as described in the literature (Sambrook et al. (2000) "Molecular Cloning: a laboratory manual", third edition, Cold Spring Harbor Laboratories, Cold Spring Harbor, New York; Innis et al. (eds.) (1990) "PCR protocols, a guide to methods and applications" Academic Press, San Diego). The *Aspergillus niger* strain (CBS 513.88) used was deposited at the Centraalbureau voor Schimmelcultures Institute (CBS) under the deposit number CBS 513.88.

### Construction of a prtT knock-out of Aspergillus niger CBS 513.88 CBS 513.88 delta prtT:

This *A. niger* strain contains a deletion of the gene encoding the protease regulator PrtT. CBS 513.88 delta *prt*T was constructed by using the "MARKER-GENE FREE" approach as described in EP 635574. In this patent it is extensively described how to delete *gla*A specific DNA sequences in the genome of CBS 513.88. The procedure resulted in a MARKER-GENE FREE recombinant *A*. *niger* CBS 513.88 delta *prt*T, which does not contain any foreign DNA sequences.

A gene replacement vector for the *prt*T gene encoding the protease regulator was designed according to known principles and constructed according to routine cloning procedures. In essence, these vectors comprise approximately 1000-3000 bp flanking regions of a *prt*T ORF for homologous recombination at the predestined genomic locus. In addition, it contains a bi-directional amdS selection marker, in-between direct repeats. The general design of these deletion vectors is disclosed in EP 635574 and WO 98/46772, which are herein incorporated by reference.

Using oligonucleotides of SEQ ID NO:15 and SEQ ID NO: 16 as primers and genomic DNA of CBS 513.88 as template, PCR was used to amplify a 1.5 kb *prt*T downstream flanking region and introduce *Kpn*I and *Xma*I restriction sites at the ends, to allow cloning in pGBDEL (Fig. 4). This 1.5 kb *prt*T downstream flanking fragment was digested with *Kpn*I and *Xma*l and introduced in a *Kpn*I and Xmal digested vector pGBDEL, generating pGBDEL-PRT1.

Using oligonucleotides of SEQ ID NO: 11 and SEQ ID NO: 12 as primers and genomic DNA of CBS 513.88 as template, a 3 kb *prt*T upstream flanking region, identified as a fragment A, was amplified by PCR. Additionally, a *Bst*BI restriction site was attached to the 5'-end and an overlapping sequence of the *prt*T downstream region at the 3'-end of the fragment A. Using oligonucleotides of SEQ ID NO: 13 and SEQ ID NO:14 as primers and genomic DNA of CBS 513.88 as template, a 500 bp *prt*T downstream flanking region, identified as a fragment B, was amplified by PCR. Both resulting fragments, A and B, were fused by sequence overlap extension (SOE-PCR as described by Ho et al., Gene 77:51-59, 1989) using PCR, oligonucleotides of SEQ ID NO:11 and SEQ ID NO:14 as primers and fragments A and B; generating a 3.5 kb fragment C. This fragment C was digested with *BstB*I and *Asc*I and introduced in a *BstB*I and *Asc*I digested vector pGBDEL-PRT1, generating pGBDEL-PRT2 (Fig. 5). The sequence of the introduced PCR fragments comprising the upstream and downstream regions of the *prt*T gene were confirmed by sequence analysis.

Linear DNA from *Bst*BI/*Xma*I-digested deletion vector pGBDEL-PRT2 was isolated and used to transform CBS 513.88. This linear DNA can integrate into the genome at the *prtT* locus, thus substituting the *prt*T coding sequence with the construct containing *amd*S (see Fig. 6). Transformants were selected on acetamide media and colony purified according to standard procedures. Growing colonies were diagnosed by PCR for integration at the *prt*T locus. Deletion of the *prt*T gene was detectable by amplification of a band, with a size specific for the pGBDEL-PRT2 insert and loss of a band specific for the wild-type *prt*T locus. Spores were plated on fluoro-acetamide media to select strains, which lost the *amd*S marker. Candidate strains were tested using Southern analysis for proper deletion of the *prt*T gene. Strains delta *prt*T were selected as representative strains with the *prt*T gene inactivated (see Fig. 6).

### Construction of a pepA knock-out of Aspergillus niger CBS 513.88 CBS 513.88 delta pepA:

A pepA deficient *Aspergillus niger* CBS 513.88 was generated as described by van den Hombergh et al. (Eur. J. Biochem. 247:605-613, 1997)

### Generation of a prtT knock-out of CBS 513.88 delta pepA CBS 513.88 delta prtT delta pepA:

The method described for the construction of CBS 513.88 delta *prt*T was used to analogously construct a *prt*T knockout of CBS 513.88 delta *pepA,* to result in CBS 513.88 delta *prt*T delta *pepA.*

### A. niger shake flask fermentations

*A. niger* strains were precultured in 20 ml preculture medium as described in WO 99/32617. After overnight growth, 10 ml of this culture was transferred to fermentation medium 1 (FM1) with 7% glucose as described in WO 99/32617. This FM1 contains per liter: 25 g casein hydrolysate, 12.5 g yeast extract, 1 g KH₂PO₄ 2 g K₂SO₄ 0.5 g MgSO₄·7H₂O, 0.03 g ZnCl₂, 0.02 g CaCl₂, 0.01 g MnSO₄·4H₂O, 0.3 g FeSO₄·7H₂O, 10 ml pen-strep (5000 IU/ml penicillin and 5 mg/ml streptomycin), adjusted to pH 5.6 with 4N H₂SO₄. Fermentation is performed in 500 ml flasks with baffle bottoms containing 100 ml fermentation broth at 34°C and 170 rpm for the number of days indicated.

For protease induction, mycelia were harvested after culturing for 16-24 hr in FM1, washed at room temperature with Induction Medium (IM) and transferred to IM with C-source as indicated.

### Induction medium (IM) contains per liter:

6 g NaNO₃, 0.5 g KCl, 1.5 g KH₂PO₄, 1.13 ml of 4M KOH, 0.5 g MgSO₄·7H₂O, 0.01% (w/v) casamino acids, 0.1% (w/v) yeast extract, 1 ml of stock trace elements (stock trace elements per liter: 22 g ZnSO₄·7H₂O, 11 g H₃BO₃, 5 g FeSO₄·7H₂O, 1.7 g CoCl₂·6H₂O, 1.6 g Cu₂SO₄·5H₂O, 5 g MnCl₂·4H₂O, 1.5 g Na₂MoO₄·2H₂O, 50 g EDTA, adjust the pH to 6.5 with 4M KOH, filter sterilize and store in the dark at 4°C), 10 ml of stock vitamins (stock vitamins per liter: 200 mg riboflavin, 200 mg thiamine.HCl, 200 mg nicotinamide, 100 mg pyridoxine-HCl, 20 mg panthotenic acid, 0.4 mg biotin, adjusted to pH 6 with 4M NaOH, filter sterilize and store in the dark at 4°C), and adjusted to pH 5.6, containing 1% (w/v) collagen or 2% (w/v) defatted soy flour.

### Construction of a collagen induced cDNA library

*A*. *niger* strain CBS 513.88 was cultivated in 100 ml of medium as described herein at 34°C and 170 rpm in an incubator shaker using a 500 ml baffled shaker flask. *A. niger* CBS 513.88 was precultured overnight and subsequently the mycelium was transferred to Fermentation Medium 1 (FM1 contains per liter: 25 g casein hydrolysate, 12.5 g yeast extract, 1 g KH₂PO₄, 2 g K₂SO₄, 0.5 g MgSO₄·7H₂O, 0.03 g ZnCl₂, 0.02 g CaCl₂, 0.01 g MnSO₄·4H₂O, 0.3 g FeSO₄·7H₂O, 10 ml pen-strep (5000 IU/ml penicillin and 5 mg/ml streptomycin), adjusted to pH 5.6 with 4N H₂SO₄). After 20 hr of growth, the mycelium was shifted to Induction Medium.

Mycelia harvested 18 hr, 28 hr, or 48 hr after the shift to IM containing 1% (w/v) collagen or 2% (w/v) defatted soy flour were used for RNA extractions. The RNA extractions and mRNA isolations were performed as described in detail in WO 99/32617. The construction of a cDNA expression library comprising a.o. the cDNA synthesis, the ligation of linkers and E. *coli* transformation is described as well in WO 99/32617. Linkers used for the cDNA reactions consisted of a *Hin*dIII and *Xho*I restriction sites. The resulting cDNA pools were ligated in the *Hin*dIII - *Xhol* digested pGBFIN-23 vector, which construction and use is described in WO 99/32617. A physical map of pGBFIN-23 can be found in Fig. 3. The ligation mixtures were used to transform DH10B electrocompetent cells (Invitrogen) resulting in the generation of over 10⁵ colonies per cDNA library obtained from both the soy flour and the collagen induced mycelium. Random sequencing of 96 clones of each of the two libraries indicated a low percentage of vectors without insert. The insert sizes for the clones sequenced were between 0.5 kb to 4.7 kb with an average of 1.7 kb. To enable an efficient screening format, the library was constructed in pools of 10³ clones. For each of these pools, glycerol stocks were made and stored for later use.

### Protease activity assays

Total acidic endoprotease activities in culture supernatants were determined as the amount of degraded bovine serum albumin (BSA). 450 µl of 1% (w/v) BSA in 0.1M NaOAc pH 4.0 was incubated with 50 µl culture supernatant at 37°C for different time intervals. At the end of the incubation period, the remainder of the BSA was precipitated with 500 µl of 10% (w/v) trichloracetic acid (TCA) and followed by incubation on ice for 10 min. The precipitate was centrifuged for 10 min at 13000 rpm in an Eppendorf centrifuge. The absorbance of the supernatant was measured at 280 nm. One unit of protease activity was defined in the Anson assay *(*J. Gen. Physiol. 22:79-89, 1938) as the change in absorbance units at 280 nm per hour. A more detailed description and references for this method is also described by van den Hombergh et al. (Curr. Genet. 28:299-308, 1995).

### Example 1: Detection of the PrtT-binding site

### Detection of differentially expressed genes in CBS 513.88 delta prtT

PrtT is a protease transcriptional regulator, an activator of several proteases WO 00/20596, WO 01/68864 and WO 06/040312. By the comparison of gene expression in the CBS 513.88 and CBS 513.88 delta *prt*T strains grown under the identical conditions the genes that expression is affected by the deletion of the PrtT transcriptional regulator can be detected. For the detection of differentially expressed genes several approaches, which are known in the art, can be used:
(i) Northern blot hybridization of RNA samples isolated from the CBS 513.88 and CBS 513.88 delta PrtT-1 grown under the identical conditions using specific probes for known protease-encoding genes (for the specific probes see van den Hombergh, An analysis of the proteolytic system in Aspergillus in order to improve protein production, Ph.D. thesis, 1996; ISBN 90-5485-545-2; van Wijk-Basten, Aminopeptidases from Aspergillus niger, Ph.D. thesis, 2004; ISBN 90-5808-968-1);
(ii) construction of subtraction cDNA library (methods available from companies such as Invitrogen, Genomax, and others) and identification of differentially expressed genes by sequencing of the cDNA clones; and
(iii) microarray analysis of the whole transcriptome with microarray such as the Affymetrix GeneChip® arrays.
We used a custom-made *A.niger* Affymetrix GeneChip® array to identify differentially expressed genes in CBS 513.88 and CBS 513.88 delta *prt*T strains grown under the FM1 conditions. Genes encoding proteases that showed significantly lower expression levels in the CBS 513.88 delta *prt*T strain compared to CBS 513.88 are listed in the Table 1.

### Identification of putative PrtT-binding sites

The 1.0 kb promoter region of the protease-encoding genes in Table 1 was obtained from the nucleotide sequence of the CBS 513.88 strain and contains the sequence immediately upstream from the translation initiation start point of the corresponding gene. The 1.0 kb promoter regions were analyzed using the Multiple Alignment Construction and Analysis Workbench (MACAW) program (version 2.0.5 for Macintosh). This software uses the multiple alignment algorithm (Karlin & Altschul, Proc. Natl. Acad. Sci. USA 87:2264-2268, 1990; Schuler et al., Proteins: Structure, Function, and Genetics 9:180-190, 1991; Lawrence et al., Science 262:208-214, 1993). The parameters were set up as follows: minimum pattern width: 6, maximum pattern width: 25, random seed: 12345, number of trials: 3, iterations per trial: 50.

This search resulted in the identification of a 7 nucleotide long imperfect palindrome sequence 5'-C C G A/T C G G-3' in all the protease-encoding genes experimentally detected (Table 1). Further searching upstream or downstream of the palindrome sequence in the promoters led to refinement of the sequence to a 33 nucleotide long sequence 5'-G/C (N)₅ C C G A/T C G G (N)₁₉ G/C-3' (SEQ ID NO: 22), where N = C or G or A or T (see Table 2). PrtT contains the zinc binuclear cluster Zn(II)₂-Cys₆ DNA binding domain. For a number of yeast and fungal Zn(II)₂-Cysₛ type of transcription factors the binding site has been detected and most of these sites contain two CGG half-sites aligned in inverted, everted (a sequence oriented in opposite direction, for instance target sequence is CGG, everted is CCG), or direct repeats, and separated by a fixed number of nucleotides characteristic of the transcription factor (Kim et al., Mol. Cel. Biol. 23:5208-5216, 2003; Cahuzac et al., Structure 9:827-836, 2001; Le Crom et al., Mol. Cell. Biol. 22:2642-2649, 2002 The putative binding site identified above contains the everted repeat, CCGA/TCGG, that suggest that this site is a part of the sequence for PrtT binding.

### Search of the entire A. niger genome for PrtT-binding sites

Having identified the putative PrtT-binding site in the experimentally detected set of proteases we have searched the 1.0 kb region of all predicted open reading frames (ORF's) in the genome of the CBS 513.88 for other protease-encoding genes that could be regulated via the 33 nucleotide long sequence 5'-G/C (N)₅ CCGA/TCGG (N)₁₉ G/C-3' (SEQ ID NO: 22). This search let to the identification of more than 400 ORF's. In order to select from this set of genes the possible protease encoding genes we have applied the following method:
(i) we have created a file of all Pfam domains that are identified in the MEROPS peptidase database (release 7.20, Rawlings et al., Nucl. Acids Res. 32:D160-D164, 2004) as the Pfam domains present in peptidases/proteases;
(ii) we used the Pfam domain list to search in the genome of the CBS 513.88 for all the ORF's that encode a protein containing a Pfam domain identified in (i).

By the overlap of the list of more than 400 ORF's that contained in the 1.0 kb promoter region the 33 nucleotide long sequence 5'-G/C (N)₅ C C G A/T C G G (N)₁₉ G/C-3' (SEQ ID NO: 22), the extended putative binding site of PrtT, and the list obtained in (ii) we have *in silico* identified additional 16 proteases that could be the potential target of the PrtT regulator. In order to confirm the identity of the gene as being a peptidase/protease, we performed the MEROPS Blast search (http://merops.sanger.ac.uk/) with the identified sequence. Out of the 16 sequences (Table 3), eight had the E-value of the MEROPS Blast search lower than e⁻²⁵ that we had set up as the cut-off value for a gene to be identified as a protease/peptidase.

Similarly, as described above for the search with the extended PrtT-binding site, we performed a search using only the imperfect palindromic sequence, 5'-C C G T/A C G G-3'. The results are described in Table 4. Additional 15 putative peptidase/protease encoding genes were identified. By applying the MEROPS Blast search E-value restriction described above, 11 genes could be specified as a peptidase/protease encoding gene.

The *in silico* search identified additionally 8 peptidase/protease-encoding genes that contain in their 1.0 kb promoter region the extended putative PrtT-binding site, 5'-G/C (N)₅ C C G A/T C G G (N)₁₉-G/C-3' (SEQ ID NO: 22), and 11 peptidase/protease-encoding genes that contain in their 1.0 kb promoter region the putative PrtT-binding site, the imperfect palindrome sequence, 5'-C C G T/A C G G-3'.

**Table 1**

| Protease encoding genes downregulated in the CBS 513.88 delta prtT strain | | |
|---|---|---|
| | | |

| | SEQ ID NO: | Description |
|---|---|---|
| 1. | 23, 24, 25 | strong similarity to protein PR0304 from patent WO200104311-A1 - Homo sapiens |
| 2. | 26, 27, 28 | aspartic proteinase aspergillopepsin I pepA - Aspergillus niger |
| 3. | 29, 30, 31 | proteinase aspergillopepsin II - Aspergillus niger |
| 4. | 32, 33, 34 | strong similarity to lysosomal pepstatin insensitive protease CLN2 - Homo sapiens |
| 5. | 35, 36, 37 | strong similarity to serine-type carboxypeptidase I cdpS - Aspergillus saitoi |
| 6. | 38, 39, 40 | similarity to putative serine peptidase - Oryza sativa |
| 7. | 41, 42, 43 | strong similarity to carboxypeptidase S1 - Penicillium janthinellum |
| 8. | 44, 45, 46 | strong similarity to hypothetical lysosomal pepstatin insensitive protease CLN2 - Canis lupus |
| 9. | 47, 48, 49 | similarity to lysosomal protease CLN2 - Rattus norvegicus |
| 10. | 50, 51, 52 | strong similarity to dipeptidyl peptidase III - Rattus norvegicus |
| 11. | 53, 54, 55 | lysine aminopeptidase apsA - Aspergillus niger |
| 12. | 56, 57, 58 | strong similarity to dipeptidyl peptidase II DPPII - Rattus norvegicus |
| 13. | 59, 60, 61 | strong similarity to hypothetical beta-lactamase XF1621 - Xylella fastidiosa |

Protease-encoding genes that are significantly downregulated in the *Aspergillus niger* CBS 513.88 delta*prt*T strain as compared to the parental *Aspergillus niger* CBS513.88 strain. The three SEQ ID NO's depicted for each gene represent: genomic DNA, cDNA and protein, respectively. The genomic DNA sequence additionally comprises a 1200bp fragment upstream of the transcription start.

**Table 2**

| | Extended binding site in the experimentally determined protease-encoding genes | | | |
|---|---|---|---|---|
| | SEQ ID NO: | Description | **Extended binding site** | |
| | | | C/G (N)₅ CCGA/TCGG (N)₁₉ C/G nucleotide | N=any |
| 1. | 23, 24, 25 | strong similarity to protein PR0304 from patent WO200104311-A1 - Homo sapiens | CTACACCCGACGGAGAGCCGGGGAGAGCATCGG | |
| 2. | 26, 27, 28 | aspartic proteinase aspergillopepsin I pepA - Aspergillus niger | GGAGGCCCGACGGACCCTGCGCGATCGGCGGTG | |
| 3. | 29, 30, 31 | proteinase aspergillopepsin II - Aspergillus niger | GCTGCCCCGACGGTGAACCTTTCTGCATCCCCG GCCAAGCCGTCGGACGTCCGTCCCCCCTCTTTC | |
| 4. | 32, 33, 34 | strong similarity to lysosomal pepstatin insensitive protease CLN2 - Homo sapiens | CGATCGCCGTCGGACTCCGGGTGGGAATCAGGG | |
| 5. | 35, 36, 37 | strong similarity to serine-type carboxypeptidase I cdpS - Aspergillus saitoi | GGCTTTCCGACGGCCTCCTCTGCATCCCCTCAC | |
| 6. | 38, 39, 40 | similarity to putative serine peptidase - Oryza sativa | CGCTCTCCGACGGTGCACAACAATCAATTCTGC | |
| 7. | 41, 42, 43 | strong similarity to carboxypeptidase S1 - Penicillium janthinellum | CGGACTCCGTCGGCTCTGCCAGCGATCGGCAGG | |
| 8. | 44, 45, 46 | strong similarity to hypothetical lysosomal pepstatin insensitive protease CLN2 - Canis lupus | CGCCCTCCGACGGCCCAAACCCACTGCAGAATG | |
| 9. | 47, 48, 49 | similarity to lysosomal protease CLN2 - Rattus norvegicus | GCAACCCCGTCGGAGTGTATACGGAGATGCGTC | |
| 10. | 50, 51, 52 | strong similarity to dipeptidyl peptidase III - Rattus norvegicus | CTTTCCCCGACGGTGACCCGACGTAGCAGTGAC CCGCCTCCGTCGGGCCACAGCTCCGTTGGATCG | |
| 11. | 53, 54, 55 | lysine aminopeptidase apsA - Aspergillus niger | GGACGACCGACGGAATTCCCGCGGCAAAAAGGG | |
| 12. | 56, 57, 58 | strong similarity to dipeptidyl peptidase II DPPII - Rattus norvegicus | GGGAGACCGACGGATAACGCGACGATCGCCGTC | |
| 13. | 59, 60, 61 | strong similarity to hypothetical beta-lactamase XF1621 - Xylella fastidiosa | GGAGCTCCGACGGGGAAACTCAGCATGTCAGCC | |

List of the extended nucleotide sequences 5'-C/G (N)₅ C C G A/T C G G (N)₁₉ C/G-3' (SEQ ID NO:22) of putative PrtT-binding sites for the protease-encoding genes of Table 1. The imperfect palindromic nucleotide sequence defined by the MACAW program is underlined. The three SEQ ID NO's depicted for each gene represent: genomic DNA, cDNA and protein, respectively. The genomic DNA sequence additionally comprises a 1200bp fragment upstream of the transcription start.

**Table 3**

| | Extended binding site in the protease-encoding genes detected in *silico* using palindromic CCGA/TCGG sequence | | |
|---|---|---|---|
| | SEQ ID NO: | Description | **Extended binding site** |
| | | | C/G (N) ₅CCGA/TCGG N=any (N) ₁₉C/G nucleotide |
| 14. | 62, 63, 64 | strong similarity to aspartic proteinase Yps3 - Saccharomyces cerevisiae | GGAGGCCCGACGGCCATCAACCGCCGAACATCC |
| 15. | 65, 66, 67 | strong similarity to aspergillopepsin II precursor (acid proteinase A) - Aspergillus niger | CAGCCACCGTCGGTCCTATCATCATCGCCCTGG |
| 16. | 68, 69, 70 | strong similarity to glutamate carboxypeptidase II - Rattus norvegicus | CCGGGCCCGTCGGATATGCGCAGGCGGTGCTGG |
| 17. | 71, 72, 73 | strong similarity to extracellular protease precursor Bar1 - Saccharomyces cerevisiae | GTAAAACCGACGGAGGTAAAACCCCGGTCATTC |
| 18. | 74, 75, 76 | similarity to axin-associating molecule Axam - Rattus norvegicus | GTGGGTCCGACGGCCATCACCCATTTCGAATTC |
| 19. | 77, 78, 79 | strong similarity to constitutive photomorphogenic COP9 complex chain AJH2 - Arabidopsis thaliana | GTGACCCCGTCGGCCCGGTAACCGCTGACTCAG |
| 20. | 80, 81, 82 | similarity to indole-3-acetyl-L-aspartic acid hydrolase IAA-asp-Enterobacter agglomerans | GGGAAGCCGTCGGCAGATGCGCCAATACGAAGC |
| 21. | 83, 84, 85 | strong similarity to glutamine-fructose-6-phosphate transaminase Gfa1 - Saccharomyces cerevisiae | CGGAGTCCGACGGAGGCACTAAAAGCGCCCCAC |

List of the extended nucleotide sequences 5'-C/G (N)₅ C C G A/T C G G (N)₁₉ C/G-3' (SEQ ID NO:22) of putative PrtT-binding sites detected in protease-encoding genes by *in silico* analysis of the CBS513.88 nucleotide sequence. The imperfect palindromic nucleotide sequence defined by the MACAW program is underlined. The three SEQ ID NO's depicted for each gene represent: genomic DNA, cDNA and protein, respectively. The genomic DNA sequence additionally comprises a 1200bp fragment upstream of the transcription start.

**Table 4**

| | Palindrome binding site in the *in silico* determined protease | | | |
|---|---|---|---|---|
| | SEQ ID NO: | E value /family | Description | Palindrome binding site |
| | | | | CCGA/TCGG |
| 22. | 86, 87, 88 | 1.10 e-70 S10 | similarity to carboxypeptidase D - Penicillium janthinellum | *CCGACGG* |
| 23. | 89, 90, 91 | 1.1 e-111 M1 | strong similarity to leucyl aminopeptidase Ape2 - Saccharomyces cerevisiae | CCGTCGG |
| 24. | 92, 93, 94 | 1.8 e-04 S8A | weak similarity to S-layer protein - Clostridium thermocellum | CCGACGG |
| 25. | 95, 96, 97 | 1.9 e+00 S24 | similarity to vacuolar carboxypeptidase Y Cpy - Saccharomyces cerevisiae | CCGTCGG |
| 26. | 98, 99, 100 | 2.20 e-78 C19 | strong similarity to ubiquitin specific protease Ubp2 - Saccharomyces cerevisiae | CCGACGG |
| 27. | 101, 103 102, | 5.20 e-02 M22 | strong similarity to proteasome 19S regulatory particle subunit Rpn2 - Saccharomyces cerevisiae | CCGTCGG |
| 28. | 104, 106 105, | 6.60 e-46 M24B | similarity to aminopeptidase P pepP - Lactococcus lactis | CCGTCGG |
| 29. | 107, 109 108, | 4.5 e-0 I2 | similarity to hypothetical RNA export mediator like protein CAD21423.1 - Neurospora crassa | CCGACGG |
| 30. | 110, 112 111, | 1.80 e-155 M18 | strong similarity to vacuolar aminopeptidase Ysci - Saccharomyces cerevisiae | CCGACGG |
| 31. | 113, 115 114, | 1.10-128 S33 | similarity to lactone-specific esterase estf1 - Pseudomonas fluorescens | CCGACGG |
| 32. | 116, 118 117, | 8.20 e-48 M38 | strong similarity to prolidase -Aureobacterium esteraromaticum | CCGACGG |
| 33. | 119, 121 120, | 4.10 e-140 M24A | strong similarity to methionyl aminopeptidase P67EIF2 - Homo sapiens | CCGACGG |
| 34. | 122, 124 123, | 1.10 e-128 S33 | strong similarity to hypothetical protein SPAC6G10.03c - Schizosaccharomvces pombe | CCGACGG |
| 35. | 125, 127 126, | 2.10 e-193 S10 | strong similarity to precursor of carboxypeptidase Kex1 - Saccharomyces cerevisiae | CCGACGG |
| 36. | 128, 130 129, | 6.40 e-222 S10 | carboxypeptidase Y cpy from patent WO9609397-A1 - Aspergillus niger | CCGACGG |

List of protease-encoding genes identified *in silico* that contain the sequence 5'-C C G A/T C G G-3' in their 1.2 kb promoter regions. The imperfect palindromic nucleotide sequence defined by the MACAW program is underlined. The E-value of the Blast MEROPS search is shown in the column E-value/family together with the protease family to which the encoded protein likely belongs. The three SEQ ID NO's depicted for each gene represent: genomic DNA, cDNA and protein, respectively. The genomic DNA sequence additionally comprises a 1200bp fragment upstream of the transcription start.

### Example 2: Functionality of the PrtT-binding site

### Construction and analysis of different variants of the pepA promoter containing distinct mutations of the PrtT-binding site

A 1 kb promoter region of p*pepA* was amplified by PCR using oligonucleotides of SEQ ID NO: 1 and SEQ ID NO: 2 as primers and genomic DNA of CBS 513.88 as template, resulting in an amplification product containing SEQ ID NO: 17.

A 0.6 kb promoter region of *pep*A was amplified by PCR using oligonucleotides of SEQ ID NO: 3 and SEQ ID NO: 2 as primers and genomic DNA of CBS 513.88 as template, resulting in an amplification product containing SEQ ID NO: 18. The pepA coding sequence was amplified by PCR using oligonucleotides of SEQ ID NO: 5 and SEQ ID NO: 6 as primers and the cDNA library (collagen) as template, resulting in an amplification product containing SEQ ID NO: 21. PCR fragments were purified through PCR column (QIAGEN) and used in fusion PCR to obtain 1 kb *ppepA-pepA* cDNA fragment and 0.6 kb p*pepA-pepA* cDNA fragment. These were cloned and sequenced. 1 kb *ppep*A-*pep*A cDNA pCR-Blunt-Topo™ plasmid was used as a template to perform site-directed mutagenesis using a QuickChange™ site-directed mutagenesis kit (Stratagene). Table 5 gives the overview of the mutations that have been introduced in the extended 33 bp long PrtT binding sequence, 5'-G/C (N)₅ C C G A/T C G G (N)₁₉ G/C-3', of the *pep*A promoter. The mutations cover the imperfect palindrome sequence CCGACGG and/or the conserved terminal C/G positions. Oligonucleotides used for introducing these mutations are listed in Table 5. After mutagenesis the complete 1kb *ppep*A-*pep*A cDNA instert in pCR-Blunt-Topo™ plasmid was re-sequenced in order to confirm the presence of the desired mutation and to exclude the presence of other mutations, possibly introduced by PCR.
One good clone for each mutant or variant was selected and used to prepare the final expression vector using *Xho*I/*Asc*I as cloning sites (Fig. 2). The obtained constructs were transformed into CBS 513.88 delta pepA (see Fig. 1) and in CBS 513.88 delta *prt*T delta *pep*A strain. Obtained transformants were checked by colony PCR using SEQ ID NO: 1 and SEQ ID NO: 2 (for all constructs carrying 1 kb *ppep*A promoter) or using SEQ ID NO: 2 and SEQ ID NO: 3 as primers (for the construct carrying 0.6kb *ppep*A promoter). The integration of the constructs was targeted to the glucoamylase locus (*g*/*a*A, see Fig. 2) using the flanks present in the vector. Correct transformants were grown for 10 days in 100 ml CSM/MES medium (150 g/kg maltose·H₂O, 60 g/kg Bacto soyton, 1 g/kg NaH₂PO₄, 15 g/kg (NH₄)₂SO₄, 1 g/k MgSO₄·7H₂O, 0.08 g/kg Tween-80, 0.02 g/kg Basildon, 20 g/kg MES, 1 g/kg L-arginine, pH 6.2) in 500 ml baffled shake flasks at 30°C at 250 rpm. After 10 days, the supernatant was harvested and protease activity was measured using the Anson assay *(*J. Gen. Physiol. 22:79-89, 1938). The results are shown in Fig. 1.

The results depicted in Fig. 1 clearly show that extracellular protease activity is strongly reduced when the palindrome CCGACGG is mutated or not present in the *ppepA-pepA* construct in a CBS 513.88 delta *pep*A background, indicating reduced transcriptional activity from the mutated p*pepA* promoter. The mutations in the other two conserved positions (the terminal G/C residues) in the 33bp long extended PrtT binding site affect the transcription efficiency from the *pepA* promoter by about 25%. Surprisingly, the combination of the mutation in the 5' end of the binding site (G to T) and insertion of one extra nucleotide in the imperfect palindrom (CCGACGG to CCGATCGG) created the *pepA* mutant promoter with increased transcriptional efficiency by about 40%. The sequence of the stronger *pepA* promoter is mentioned under the SEQ ID NO:139.
The dependency of the transcription of *pep*A on PrtT is further demonstrated below. When a non-modified 1.0 kb *ppepA-pepA* cDNA construct was transformed to CBS 513.88 delta *pepA,* a strong increase in extracellular protease activity was observed. However, when this construct was transformed into CBS 513.88 delta *prt*T delta *pepA* (data not shown) no extracellular activity was observed, indicating that pepA activity is highly dependent on PrtT mediated transcription. In more detail, the palindrome CCGACGG is very important for PrtT mediated transcription of *pepA.* This leads to the conclusion that CCGACGG is the PrtT-binding site in the *pepA* promoter. The effect of the two terminal conserved residues of the extended PrtT binding site might be in stabilization of the protein-DNA complex.

**Table 5**

| | Extended PrtT binding site | | |
|---|---|---|---|
| | C/G (N) ₅CCGA/TCGG (N) ₁₉C/G N=any nucleotide | | |
| Mutant no. | p*pep*A PrtT binding site | Forward primer | Reverse primer |
| | GGAGGCCCGACGGACCCTGCGCGATCGGCGGTG | | |
| 1. | GGAGGCCAGACTGACCCTGCGCGATCGGCGGTG | SEQ ID NO. 7 | SEQ ID NO. 8 |
| 2. | GGAGGCAAGACTTACCCTGCGCGATCGGCGGTG | SEQ ID NO. 9 | SEQ ID NO. 10 |
| 3. | TGAGGCCCGAtCGGACCCTGCGCGATCGGCGGTG | SEQ ID NO. 131 | SEQ ID NO. 132 |
| 4. | TGAGGCCAGACGGACCCTGCGCGATCGGCGGTG | SEQ ID NO. 133 | SEQ ID NO. 134 |
| 5. | AGAGGCCCGACGGACCCTGCGCGATCGGCGGTG | SEQ ID NO. 135 | SEQ ID NO. 136 |
| 6. | GGAGGCCCGACGGACCCTGCGCGATCGGCGGTA | SEQ ID NO. 137 | SEQ ID NO. 138 |

Over-view of mutations created in the pepA promoter. The mutated nucleotides are depicted in bold and the PrtT binding site is underlined. The oligonucleotide primer sequences that were used to introduce these mutations are listed in the right-hand two columns.

## Claims

1. Method for the production of a mutant of a parent filamentous fungal host cell, said mutant producing fewer proteases and less protease activity than the parent host cell, comprising:
- provision of a parent filamentous fungal host cell,
- modification or inactivation of a prtT binding site according to SEQ ID NO: 22 rendering said prtT binding site non-functional.

2. The method according to claim 1, wherein the modification or inactivation comprises introducing one or more changes in the imperfect palindrome site C C G A/T C G G.

3. Method according to any one of claims 1 and 2, wherein the filamentous fungal host cell is an *Aspergillus* cell.

4. Method for the production of a polypeptide comprising:
- provision of a parent filamentous fungal host cell,
- modification or inactivation of a prtT binding site according to SEQ ID NO: 22 rendering said prtT binding site non-functional,
- cultivating under conditions conducive to expression of the polypeptide, the mutant filamentous fungal host cell,
- expressing the polypeptide in said cell, and
- optionally recovering the polypeptide from the nutrient medium or from the host cell.

5. The method according to claim 4, additionally comprising providing the host cell with a polynucleotide encoding a polypeptide of interest.

6. The method according to any one of claims 4 and 5, wherein the modification or inactivation comprises introducing one or more changes in the imperfect palindrome site C C G A/T C G G.

7. Method according to any one of claims 4 to 6, wherein the filamentous fungal host cell is an *Aspergillus* cell.

## Patentansprüche

1. Verfahren zur Herstellung einer Mutante einer Fadenpilz-Elternwirtszelle, wobei die Mutante weniger Proteasen und weniger Proteaseaktivität als die Elternwirtszelle produziert, wobei das Verfahren Folgendes umfasst:
- Bereitstellen einer Fadenpilz-Elternwirtszelle,
- Modifizieren oder Inaktivieren einer prtT-Bindungsstelle gemäß SEQ ID NO: 22, wodurch die prtT-Bindungsstelle nicht funktionell gemacht wird.

2. Verfahren nach Anspruch 1, wobei das Modifizieren oder Inaktivieren das Einführen von einer oder mehreren Veränderungen in die imperfekte Palindromstelle C C G A/T C G G umfasst.

3. Verfahren nach einem der Ansprüche 1 und 2, wobei es sich bei der Fadenpilzwirtszelle um eine *Aspergillus*-Zelle handelt.

4. Verfahren zur Produktion eines Polypeptids, das Folgendes umfasst:
- Bereitstellen einer Fadenpilz-Elternwirtszelle,
- Modifizieren oder Inaktivieren einer prtT-Bindungsstelle gemäß SEQ ID NO: 22, wodurch die prtT-Bindungsstelle nicht funktionell gemacht wird,
- Kultivieren der Fadenpilz-Wirtszellenmutante unter Bedingungen, die der Expression des Polypeptids förderlich sind,
- Exprimieren des Polypeptids in der Zelle, und
- gegebenenfalls Gewinnen des Polypeptids aus dem Nährmedium oder aus der Wirtszelle.

5. Verfahren nach Anspruch 4, das weiterhin das Bereitstellen eines Polynukleotids, das für ein interessierendes Polypeptid codiert, an die Wirtszelle umfasst.

6. Verfahren nach einem der Ansprüche 4 und 5, wobei das Modifizieren oder Inaktivieren das Einführen von einer oder mehreren Veränderungen in die imperfekte Palindromstelle C C G A/T C G G umfasst.

7. Verfahren nach einem der Ansprüche 4 bis 6, wobei es sich bei der Fadenpilzwirtszelle um eine *Aspergillus*-Zelle handelt.

## Revendications

1. Procédé de production d'un mutant d'une cellule hôte fongique filamenteuse parente, ledit mutant produisant moins de protéase et une plus faible activité de protéase que la cellule hôte parente, comprenant :
- la mise à disposition d'une cellule hôte fongique filamenteuse parente,
- la modification ou l'inactivation d'un site de liaison au prtT selon SEQ ID NO:22, rendant non fonctionnel ledit site de liaison au prtT.

2. Procédé selon la revendication 1, dans lequel la modification ou l'inactivation comprend l'introduction d'un ou plusieurs changements dans le site palindromique imparfait C C G A/T C G G.

3. Procédé selon l'une quelconque des revendications 1 et 2, dans lequel la cellule hôte fongique filamenteuse est une cellule *d'Aspergillus.*

4. Procédé de production d'un polypeptide, comprenant :
- la mise à disposition d'une cellule hôte fongique filamenteuse parente,
- la modification ou l'inactivation d'un site de liaison au prtT selon SEQ ID NO:22, rendant non fonctionnel ledit site de liaison au prtT,
- la culture, dans des conditions conduisant à l'expression du polypeptide, de la cellule hôte fongique filamenteuse mutante,
- l'expression du polypeptide dans ladite cellule, et
- en option, la récupération du polypeptide à partir du milieu nutritif ou de la cellule hôte.

5. Procédé selon la revendication 4, comprenant en outre la fourniture, à la cellule hôte, d'un polynucléotide codant pour un polypeptide d'intérêt.

6. Procédé selon l'une quelconque des revendications 4 et 5, dans lequel la modification ou l'inactivation comprend l'introduction d'un ou plusieurs changements dans le site palindromique imparfait C C G A/T C G G.

7. Procédé selon l'une quelconque des revendications 4 à 6, dans lequel la cellule hôte fongique filamenteuse est une cellule *d'Aspergillus.*
